# EUROPEAN PATENT APPLICATION

(11) **EP 2 390 359 A1**
(43) Date of publication of application: **30.11.2011**
(21) Application number: 11163263.4
(22) Date of filing: 31.05.2007
(51) Int. Cl.: C12Q 1/68

(54) **Method for identifying whether a patient will be responder or not to immunotherapy based on the differential expression of the ICOS gene**

(30) Priority: 02.06.2006 GB 0610949; 15.01.2007 GB 0700761
(62) Divisional of application: 07725789.7
(71) Applicant: GlaxoSmithKline Biologicals S.A., 1330 Rixensart (BE)
(72) Inventor: Brichard, Vincent, B-1330, Rixensart (BE); Clark, James, Scott, B-1330, Rixensart (BE); Coche, Thierry, B-1330, Rixensart (BE); Gaulis, Swann, Romain, Jean-Thomas, B-1330, Rixensart (BE); Gruselle, Olivier, B-1330, Rixensart (BE); Lehmann, Frederic, B-1330, Rixensart (BE); Louahed, Jamila, B-1330, Rixensart (BE)
(74) Representative: O'Farrell, Damien John

(57) **Abstract**

The present invention relates to gene expression profiles, microarrays comprising nucleic acid sequences representing gene expression profiles and new diagnostic kits and methods. The invention further relates to treatment of specific populations of, for example, cancer patients, as characterised by their gene expression profile, suffering from Mage expressing tumours.

SEQUENCE LISTING

<110> GlaxoSmithKline Biologicals S.A.

<120> Method

<130> VB61997

<160> 163

<170> FastSEQ for Windows Version 4.0

<210> 1
<211> 4039
<212> DNA
<213> Homo sapiens

<400> 1
atacttacaa ttacgagatt tatatttgca ttagtctctt tggctggtgg gtaggggtga 60
gaggctcttc ctggatccct tattttctac aggagaggag gaaaacacct gggatgctcc 120
agtgctctta cgcagataat gatcattaac atcagcctct ctgatcaaag agctactcca 180
ccccactctg gctgtagtgt gacattcctg cctgcctgag gaagaaatgg tgagcagggg 240
ctgcaattgc agacaagtgt cacccagaag ccacaagttt ctgtgagcac caggtctaca 300
aactacccaa ggcatagcaa tggcattatc actggaagaa ttcgtccact cccttgacct 360
caggacccta cccagggttc tagaaatcca ggcaggcatc tatcttgaag gctctattta 420
tgaaatgttt ggaaatgaat gctgtttttc aacaggagaa gtgattaaaa ttactggtct 480
caaagttaag aagatcatag ctgaaatttg tgagcagatt gaaggttgtg agtctctaca 540
gccatttgaa ctgcctatga attttccagg tctttttaag attgtggctg ataaaactcc 600
ataccttact atggaagaaa tcacaaggac cattcatatt ggaccaagta gactagggca 660
tccttgcttc tatcatcaga aggatataaa actagagaac ctcatcataa agcagggtga 720
gcaaatcatg ctcaactcag ttgaagagat tgatggagaa ataatggtga gctgtgcagt 780
agcaaggaat catcaaactc actcatttaa tttgcctttg tcacaagaag gagaattcta 840
cgagtgtgaa gatgaacgta tttacactct aaaggagatt gttgaatgga agattcctaa 900
gaacagaaca agaactgtaa accttacaga tttttcaaat aagtgggact caacgaatcc 960
atttcctaaa gacttttatg gtaccctgat tctcaagcct gtttatgaaa ttcaaggtgt 1020
gatgaaattt cgaaaagata taatccgcat cctccccagt ctagatgtcg aagtcaaaga 1080
catcactgat tcttacgatg ctaactggtt tcttcagctg ttatcaacag aagatctttt 1140
tgaaatgact agtaaagagt tccccatagt gactgaagtc atagaagcac ctgaaggaaa 1200
ccacctgccc caaagcattt tacagcctgg gaaaaccatt gtgatccaca aaaagtacca 1260
ggcatcaaga atcttagctt cagaaattag aagcaatttt cctaaaagac acttcttgat 1320
ccccactagc tataaaggca agttcaagcg gcgaccgagg gagttcccaa cggcctatga 1380
cctagagatc gctaagagtg aaaaggagcc tcttcacgtg gtggccacca aagcgtttca 1440
ttcccctcat gacaagctgt catccgtatc tgttggggac cagtttctgg tgcatcagtc 1500
agagacgact gaagtcctct gtgagggaat aaaaaaagtg gtgaatgttc tggcctgtga 1560
aaaaatcctc aaaaagtcct atgaggctgc gctgctccct ttgtacatgg aaggaggttt 1620
tgtagaggtg attcatgata agaaacagta cccgatttct gagctctgta aacagttccg 1680
tttgcccttc aatgtgaagg tgtctgtcag ggatctttcc attgaagagg acgtgttggc 1740
tgccacacca ggactgcagt tggaggagga cattacagac tcttacctac tcataagtga 1800
ctttgccaac cccacggagt gctgggaaat tcctgtgggc cgcttgaata tgactgttca 1860
gttagttagt aatttctcta gggatgcaga accatttcta gtcaggactc tggtagaaga 1920
gatcactgaa gagcaatatt acatgatgcg gagatatgaa agctcagcct cacatccccc 1980
acctcgccct ccgaaacacc cctcagtaga ggaaacaaag ttaaccctgc taaccttagc 2040
agaagaaagg acggtagacc tgcccaagtc tcccaagcgt catcacgtag acataaccaa 2100
gaaacttcac ccaaatcaag ctggcctgga ttcaaaagta ctgattggta gtcagaatga 2160
tttggtggat gaagagaaag aaaggagcaa ccgtggggcc acagcaatag cagaaacatt 2220
caaaaatgaa aaacatcaaa aataacaaga tgtgacagaa gccacttagg cagcaaacat 2280

aaatgttgca gtgaaaaaag aagctagcct tctagctgaa aaacgagtat tccccaatgg 2340
actccagaag aaacttgatt catcgctgca aaggaaagaa caaccttaaa acttttaaca 2400
gataaaactt acagaaacct atgatataga attcatatag tctattctgt tgtgtctaaa 2460
tctgtaggca ttgtgttgtt gttctttagg acgtatttat ttaacttgca cattttttca 2520
gattcttatt tctactacca acaactaagt aattgggaaa taattctgta tttcagtttc 2580
tgagtaaaac cagtctgaaa taggataaaa gccaccaaat attttctttt ttttccagaa 2640
tttgttttgc cattttttag tgctatcatc attcctaaca agactaactt acagaaaaat 2700
aattatatct gactgattta aaatgttcag gtttcttatc caaatccctt ggaactatgg 2760
aaaggagttt gatttcacat tcacagtgta tttacaaaat acgctgtgtc ataaatatgt 2820
ttgaattcca acagccaaag ccattgagag tcataggagt tttccataac cttctcttct 2880
atgacccaac aacaagctca tgactgaaat ttcaccagat ttctgagacg atgtcttaat 2940
attctatgtg ctatgtacca gataattctt tagatgaatg tttcttagga ttgtaggaaa 3000
attatctagt taatcataat atttgatgga aagaaaaaga caataaaatt gtaatataat 3060
aaatttggct gacaagaaac caaagtgatt cttaattagt atacatcaga atgatgctct 3120
tatagttgta ccatctataa aaattacttt aagggctctc acattttaat aatttatctt 3180
attatgtatt aagtatacag gaacaatatt atttttcctt taacaaaatg aagagacagg 3240
ctatctggtt aatgttacat aggaatttaa tagtaatgct tgaacttcat ccatagatca 3300
tactctgtac aaaatttgtt agctaacatc ctatctcata attattttat gttttgtgga 3360
gaaatttgtt gattttgtac caaagtgttt ctgaagacaa taaattgtga gtcaacttta 3420
gaacaaaaaa aattagagtt ttttcaatgt ttatattctg attaagctta ctttacctta 3480
cattttttct aagtaacaat gaatcctgat ttctagtgtc ctaaaaattg cttagtgatt 3540
tgattgtggt aatatcattt cttatctaca atgtctaaag ttttatggga cagtttttct 3600
tttatttatt ttgcctgttt gtgcagataa gaacagaaac ttttctaaga cccacatttg 3660
gttattgaag gccacagcga atcttaacct aacagccttg acaaactgca ccataggtgt 3720
ttttagactc atataatttg ttatttttca aacaatagtg aataattaat atttttgttt 3780
ggaatttgag aacaattaaa tttgtacttt tagtaactac cattctttga ttagaaaatt 3840
aagagaatgc atatcttact ttggttgtaa attatcaagg gctttctaat agaaatcata 3900
tataacattt ctaaatataa gtcctttcac atactgtgtt tccagttgtc ttgatattga 3960
aaagtgtaat aaacttcatg ctcacctatt ggagatttgg gaaggttgaa aataaacttc 4020
ctaattttta aaaaaaaaa 4039

<210> 2
<211> 2029
<212> DNA
<213> Homo sapiens

<400> 2
gtggacgcga ggagccgggc gcttagaaca gaggcttgca caggtggaga tgtggaagtc 60
tgtagtgggc catgatgtgt ctgtttccgt ggagacccag ggtgatgatt gggacacaga 120
tcctgacttt gtgaatgaca tctctgaaaa ggagcaacga tggggagcca agaccatcga 180
ggggtctgga cgcacagaac acatcaacat ccaccagctg aggaacaaag tatcagagga 240
gcatgatgtt ctcaggaaga aagagatgga gtcagggccc aaagcatccc atggctatgg 300
aggtcggttt ggagtagaaa gagaccgaat ggacaagagt gcagtgggcc atgagtatgt 360
tgccgaggtg gagaagcact cttctcagac ggatgctgcc aaaggctttg ggggcaagta 420
cggagttgag agggacaggg cagacaagtc agcagtcggc tttgattata aaggagaagt 480
ggagaagcat acatctcaga aagattactc tcgtggcttt ggtggccggt acggggtgga 540
gaaggataaa tgggacaaag cagctctggg atatgactac aagggagaga cggagaaaca 600
cgagtcccag agagattatg ccaagggctt tggtggccag tatggaatcc agaaggaccg 660
agtggataag agcgctgtcg gcttcaatga aatggaggcc ccgaccacag cttataagaa 720
gacgacgccc atagaagccg cttctagtgg tgcccgtggg ctgaaggcga aatttgagtc 780
catggctgag gagaagagga agcgagagga agaggagaag gcacagcagg tggccaggag 840
gcaacaggag cgaaaggctg tgacaaagag gagccctgag gctccacagc cagtgatagc 900
tatggaagag ccagcagtac cggccccact gcccaagaaa atctcctcag aggcctggcc 960
tccagttggg actcctccat catcagagtc tgagcctgtg agaaccagca gggaacaccc 1020
agtgcccttg ctgcccatta ggcagactct cccggaggac aatgaggagc ccccagctct 1080
gccccctagg actctggaag gcctccaggt ggaggaagag ccagtgtacg aagcagagcc 1140
tgagcctgag cccgagcctg agcccgagcc tgagaatgac tatgaggacg ttgaggagat 1200
ggacaggcat gagcaggagg atgaaccaga gggggactat gaggaggtgc tcgagcctga 1260

agattcttct ttttcttctg ctctggctgg atcatcaggc tgcccggctg gggctggggc 1320
tggggctgtg gctctgggga tctcagctgt ggctctatat gattaccaag gagagggaag 1380
tgatgagctt tcctttgatc cggacgacgt aatcactgac attgagatgg tggacgaggg 1440
ctggtggcgg ggacgttgcc atggccactt tggactcttc cctgcaaatt atgtcaagct 1500
tctggagtga ctagagctca ctgtctactg caactgtgat ttcccatgtc caaagtggct 1560
ctgcctccac cccctcccta ttcctgatgc aaatgtctaa ccagatgagt ttctggacag 1620
acttccctct cctgcttcat taagggcttg gggcagagac agcatgggga aggaggtccc 1680
cttccccaag agtcctctct atcctggatg agctcatgaa catttctctt gtgttcctga 1740
ctccttccca atgaacacct ctctgccacc ccaagctctg ctctcctcct ctgtgagctc 1800
tgggcttccc agtttgttta cccgggaaag tacgtctaga ttgtgtggtt tgcctcattg 1860
tgctatttgc ccactttcct tccctgaaga aatatctgtg aaccttcttt ctgttcagtc 1920
ctaaaattcg aaataaagtg agactatggt tcacctgtaa aaaaaaaaaa aaaaaaaaaa 1980
aaaaaaaaaa aaaaaaaaaa aaaaaaaaaa aaaaaaaaaa aaaaaaaaa 2029

<210> 3
<211> 1451
<212> DNA
<213> Homo sapiens

<400> 3
gaagagcaag cgccatgttg aagccatcat taccattcac atccctctta ttcctgcagc 60
tgcccctgct gggagtgggg ctgaacacga caattctgac gcccaatggg aatgaagaca 120
ccacagctga tttcttcctg accactatgc ccactgactc cctcagtgtt tccactctgc 180
ccctcccaga ggttcagtgt tttgtgttca atgtcgagta catgaattgc acttggaaca 240
gcagctctga gccccagcct accaacctca ctctgcatta ttggtacaag aactcggata 300
atgataaagt ccagaagtgc agccactatc tattctctga agaaatcact tctggctgtc 360
agttgcaaaa aaaggagatc cacctctacc aaacatttgt tgttcagctc caggacccac 420
gggaacccag gagacaggcc acacagatgc taaaactgca gaatctggtg atcccctggg 480
ctccagagaa cctaacactt cacaaactga gtgaatccca gctagaactg aactggaaca 540
acagattctt gaaccactgt ttggagcact tggtgcagta ccggactgac tgggaccaca 600
gctggactga acaatcagtg gattatagac ataagttctc cttgcctagt gtggatgggc 660
agaaacgcta cacgtttcgt gttcggagcc gctttaaccc actctgtgga agtgctcagc 720
attggagtga atggagccac ccaatccact gggggagcaa tacttcaaaa gagaatcctt 780
tcctgtttgc attggaagcc gtggttatct ctgttggctc catgggattg attatcagcc 840
ttctctgtgt gtatttctgg ctggaacgga cgatgccccg aattcccacc ctgaagaacc 900
tagaggatct tgttactgaa taccacggga acttttcggc ctggagtggt gtgtctaagg 960
gactggctga gagtctgcag ccagactaca gtgaacgact ctgcctcgtc agtgagattc 1020
ccccaaaagg aggggccctt ggggaggggc ctggggcctc cccatgcaac cagcatagcc 1080
cctactgggc ccccccatgt tacaccctaa agcctgaaac ctgaacccca atcctctgac 1140
agaagaaccc cagggtcctg tagccctaag tggtactaac tttccttcat tcaacccacc 1200
tgcgtctcat actcacctca ccccactgtg gctgatttgg aattttgtgc ccccatgtaa 1260
gcaccccttc atttggcatt ccccacttga gaattaccct tttgccccga acatgttttt 1320
cttctccctc agtctggccc ttccttttcg caggattctt cctccctccc tctttccctc 1380
ccttcctctt tccatctacc ctccgattgt tcctgaaccg atgagaaata aagtttctgt 1440
tgataatcat c 1451

<210> 4
<211> 523
<212> DNA
<213> Homo sapiens

<400> 4
ctcctggttc aaaagcagct aaaccaaaag aagcctccag acagccctga gatcacctaa 60
aaagctgcta ccaagacagc cacgaagatc ctaccaaaat gaagcgcttc ctcttcctcc 120
tactcaccat cagcctcctg gttatggtac agatacaaac tggactctca ggacaaaacg 180
acaccagcca aaccagcagc ccctcagcat ccagcaacat aagcggaggc attttccttt 240
tcttcgtggc caatgccata atccacctct tctgcttcag ttgaggtgac acgtctcagc 300
cttagccctg tgccccctga aacagctgcc accatcactc gcaagagaat cccctccatc 360

tttgggaggg gttgatgcca gacatcacca ggttgtagaa gttgacaggc agtgccatgg 420
gggcaacagc caaaataggg gggtaatgat gtaggggcca agcagtgccc agctgggggt 480
caataaagtt acccttgtac ttgcaaaaaa aaaaaaaaaa aaa 523

<210> 5
<211> 1579
<212> DNA
<213> Homo sapiens

<400> 5
accaacccct aagatgagct ttccatgtaa atttgtagcc agcttccttc tgattttcaa 60
tgtttcttcc aaaggtgcag tctccaaaga gattacgaat gccttggaaa cctggggtgc 120
cttgggtcag gacatcaact tggacattcc tagttttcaa atgagtgatg atattgacga 180
tataaaatgg gaaaaaactt cagacaagaa aaagattgca caattcagaa aagagaaaga 240
gactttcaag gaaaaagata catataagct atttaaaaat ggaactctga aaattaagca 300
tctgaagacc gatgatcagg atatctacaa ggtatcaata tatgatacaa aaggaaaaaa 360
tgtgttggaa aaaatatttg atttgaagat tcaagagagg gtctcaaaac caaagatctc 420
ctggacttgt atcaacacaa ccctgacctg tgaggtaatg aatggaactg accccgaatt 480
aaacctgtat caagatggga aacatctaaa actttctcag agggtcatca cacacaagtg 540
gaccaccagc ctgagtgcaa aattcaagtg cacagcaggg aacaaagtca gcaaggaatc 600
cagtgtcgag cctgtcagct gtccagagaa aggtctggac atctatctca tcattggcat 660
atgtggagga ggcagcctct tgatggtctt tgtggcactg ctcgttttct atatcaccaa 720
aaggaaaaaa cagaggagtc ggagaaatga tgaggagctg gagacaagag cccacagagt 780
agctactgaa gaaaggggcc ggaagcccca acaaattcca gcttcaaccc ctcagaatcc 840
agcaacttcc caacatcctc ctccaccacc tggtcatcgt tcccaggcac ctagtcatcg 900
tcccccgcct cctggacacc gtgttcagca ccagcctcag aagaggcctc ctgctccgtc 960
gggcacacaa gttcaccagc agaaaggccc gcccctcccc agacctcgag ttcagccaaa 1020
acctccccat ggggcagcag aaaactcatt gtccccttcc tctaattaaa aaagatagaa 1080
actgtctttt tcaataaaaa gcactgtgga tttctgccct cctgatgtgc atatccgtac 1140
ttccatgagg tgttttctgt gtgcagaaca ttgtcacctc ctgaggctgt gggccacagc 1200
cacctctgca tcttcgaact cagccatgtg gtcaacatct ggagtttttg gtctcctcag 1260
agagctccat cacaccagta aggagaagca atataagtgt gattgcaaga atggtagagg 1320
accgagcaca gaaatcttag agatttcttg tcccctctca ggtcatgtgt agatgcgata 1380
aatcaagtga ttggtgtgcc tgggtctcac tacaagcagc ctatctgctt aagagactct 1440
ggagtttctt atgtgccctg gtggacactt gcccaccatc ctgtgagtaa aagtgaaata 1500
aaagctttga ctagaaaaaa aaaaaaaaaa aaaaaaaaaa aaaaaaaaaa aaaaaaaaaa 1560
aaaaaaaaaa aaaaaaaaa 1579

<210> 6
<211> 971
<212> DNA
<213> Homo sapiens

<400> 6
gattgcttga ggagagaagt atgtgatcag aaagcattct ttgtctatta actcctgccc 60
agcaaaagtg aaagaaaatt catgggagca tgcaagaaca aagagcacag caaagctgga 120
caaacacagc aatccaggca ggggatttcc aactcaactc tggtatgtaa gctgcatgca 180
aagtcctttt tctgtctctg gtttctggcc ccttgtctgc agagatggct cccaatgctt 240
cctgcctctg tgtgcatgtc cgttccgagg aatgggattt aatgaccttt gatgccaacc 300
catatgacag cgtgaaaaaa atcaaagaac atgtccggtc taagaccaag gttcctgtgc 360
aggaccaggt tcttttgctg ggctccaaga tcttaaagcc acggagaagc ctctcatctt 420
acggcattga caaagagaag accatccacc ttaccctgaa agtggtgaag cccagtgatg 480
aggagctgcc cttgtttctt gtggagtcag gtgatgaggc aaagaggcac ctcctccagg 540
tgcgaaggtc cagctcagtg gcacaagtga aagcaatgat cgagactaag acgggtataa 600
tccctgagac ccagattgtg acttgcaatg gaaagagact ggaagatggg aagatgatgg 660
cagattacgg catcagaaag ggcaacttac tcttcctggc atcttattgt attggagggt 720
gaccaccctg ggcatggggt gttggcaggg gtcaaaaagc ttatttcttt taatctctta 780
ctcaacgaac acatcttctg atgatttccc aaaattaatg agaatgagat gagtagagta 840

agatttgggt gggatgggta ggatgaagta tattgcccaa ctctatgttt ctttgattct 900
aacacaatta attaagtgac atgattttta ctaatgtatt actgagacta gtaaataaat 960
ttttaagcca a 971

<210> 7
<211> 2761
<212> DNA
<213> Homo sapiens

<400> 7
agaggacgcc cggtgaaggg gctccagcct ggcagtttct gcgtgttagc atttctagaa 60
tagagtgggt gggaactgac ccaagtaaag tcccagagac tcgaacactg acgcacagga 120
aagcctcaag tgggaggaga aatgcaaatc ccctactgat gatggcgtca gcggctttct 180
cctagggact gtgaggggcg cttctgactt tggacttgag cactgcctgg gacctgtgct 240
gagagagcgc tagcatgtct cagtggaatc aagtccaaca gttagaaatc aagtttttgg 300
agcaggtgga tcaattctat gatgacaact ttcccatgga aattcggcat ctgttggccc 360
aatggattga aaatcaagac tgggaggcag cttctaacaa tgaaaccatg gcaacgattc 420
ttcttcaaaa cttgttaata caactggatg aacagttagg tcgtgtttcc aaagagaaaa 480
acctactctt gatacacaat ctaaaaagaa ttaggaaggt ccttcaggga aaatttcatg 540
gaaatccaat gcatgtagct gtggttattt caaactgttt aagggaagag aggagaatat 600
tggctgcagc caacatgcct gtccaggggc ctctagagaa atccttacaa agttcttcag 660
tttcagaaag acagaggaat gtggagcaca aagtggctgc cattaaaaac agtgtgcaga 720
tgacagaaca agataccaaa tacttagaag atctgcaaga cgaatttgac tacaggtata 780
aaacaattca gacaatggat cagagtgaca agaatagtgc catggtgaat caggaagttt 840
tgacactgca ggaaatgctt aacagcctcg atttcaagag aaaggaggct ctcagtaaaa 900
tgacccaaat catccatgag acagacctgt taatgaacac catgctcata gaagagctgc 960
aagactggaa gcggcggcag caaatcgcct gcatcggggg tccactccac aatgggctcg 1020
accagcttca gaactgcttt acactattgg cagaaagtct tttccaactg agaaggcaat 1080
tggagaaact agaggagcaa tctaccaaaa tgacatatga aggtgatccc attccaatgc 1140
aaagaactca catgctagaa agagtcacct tcttgatcta caaccttttc aagaactcat 1200
ttgtggttga gcgacagcca tgtatgccaa cccaccctca gaggccgttg gtacttaaaa 1260
ccctaattca gttcactgta aaactaaggc tactaataaa attgccagaa ctaaactatc 1320
aggtaaaggt taaggcatca attgacaaga atgtttcaac tctaagcaac cgaagatttg 1380
tactttgtgg aactaatgtc aaagccatgt ctattgaaga atcttccaat gggagtctct 1440
cagtagaatt tcgacatttg caaccaaagg aaatgaagtc cagtgctgga ggtaaaggaa 1500
atgagggctg tcacatggtg actgaagaac ttcattccat aacgtttgaa acacagatct 1560
gcctctatgg cctgaccata gatttggaga ccagctcatt gcctgtggtg atgatttcca 1620
atgtcagtca gttacctaat gcttgggcat ccatcatttg gtacaacgtg tcaaccaacg 1680
attcccagaa cttggttttc tttaataatc ctccacctgc cacattgagt caactactgg 1740
aggtgatgag ctggcagttt tcatcgtacg ttggtcgtgg tcttaactca gatcaactcc 1800
atatgctggc agagaagctt acagtccaat ctagctacag tgatggtcac ctcacctggg 1860
ccaagttctg caaggaacat ttacctggta aatcatttac cttttggaca tggcttgaag 1920
caatattgga tctaattaag aaacacattc ttcccctttg gattgatggg tatgtcatgg 1980
gctttgttag caaagagaag gaacggctgt tgctaaagga taaaatgcct ggcacctttt 2040
tattaagatt cagtgaaagc catctcggag gaataacttt cacctgggtg gaccattctg 2100
aaagtgggga agtgagattc cactctgtag aaccctacaa taaaggccgg ttgtctgctc 2160
tgccattcgc tgacatcctg cgagactaca aagttattat ggctgaaaac attcctgaaa 2220
accctctgaa gtacctatat cctgacattc ccaaagacaa agccttcggt aaacactaca 2280
gctctcagcc ttgcgaagtt tcaagaccaa cagaaagggg tgacaaaggt tatgttcctt 2340
ctgtttttat ccccatctca acaatccgaa gtgattcaac agagccacat tctccatcag 2400
accttcttcc catgtctcca agtgtgtatg cggtgttgag agaaaacctg agtcccacaa 2460
caattgaaac tgcaatgaag tctccttatt ctgctgaatg acaggataaa ctctgacgca 2520
ccaagaaagg aagcaaatga aaaagtttaa agactgttct ttgcccaata accacatttt 2580
atttcttcag ctttgtaaat accaggttct aggaaatgtt tgacatctga agctctcttc 2640
acactcccgt ggcactcctc aattgggagt gttgtgactg aaatgcttga aaccaaagct 2700
tcagataaac ttgcaagata agacaacttt aagaaaccag tgttaataac aatattaaca 2760
g 2761

<210> 8
<211> 1074
<212> DNA
<213> Homo sapiens

<400> 8
gatcaacaca tttcatctgg gcttcttaaa tctaaatctt taaaatgact aagttttctt 60
ccttttctct gtttttccta atagttgggg cttatatgac tcatgtgtgt ttcaatatgg 120
aaattattgg agggaaagaa gtgtcacctc attccaggcc atttatggcc tccatccagt 180
atggcggaca tcacgtttgt ggaggtgttc tgattgatcc acagtgggtg ctgacagcag 240
cccactgcca atatcggttt accaaaggcc agtctcccac tgtggtttta ggcgcacact 300
ctctctcaaa gaatgaggcc tccaaacaaa cactggagat caaaaaattt ataccattct 360
caagagttac atcagatcct caatcaaatg atatcatgct ggttaagctt caaacagccg 420
caaaactcaa taaacatgtc aagatgctcc acataagatc caaaacctct cttagatctg 480
gaaccaaatg caaggttact ggctggggag ccaccgatcc agattcatta agaccttctg 540
acaccctgcg agaagtcact gttactgtcc taagtcgaaa actttgcaac agccaaagtt 600
actacaacgg cgaccctttt atcaccaaag acatggtctg tgcaggagat gccaaaggcc 660
agaaggattc ctgtaagggt gactcagggg gccccttgat ctgtaaaggt gtcttccacg 720
ctatagtctc tggaggtcat gaatgtggtg ttgccacaaa gcctggaatc tacaccctgt 780
taaccaagaa ataccagact tggatcaaaa gcaaccttgt cccgcctcat acaaattaag 840
ttacaaataa ttttattgga tgcacttgct tcttttttcc taatatgctc gcaggttaga 900
gttgggtgta agtaaagcag agcacatatg gggtccattt ttgcacttgt aagtcatttt 960
attaaggaat caagttcttt ttcacttgta tcactgatgt atttctacca tgctggtttt 1020
attctaaata aaatttagaa gactcaaaaa aaaaaaaaaa aaaaaaaaaa aaaa 1074

<210> 9
<211> 822
<212> DNA
<213> Homo sapiens

<400> 9
gggctgctcc acgcttttgc cggagacaga gactgacatg gaacagggga agggcctggc 60
tgtcctcatc ctggctatca ttcttcttca aggtactttg gcccagtcaa tcaaaggaaa 120
ccacttggtt aaggtgtatg actatcaaga agatggttcg gtacttctga cttgtgatgc 180
agaagccaaa aatatcacat ggtttaaaga tgggaagatg atcggcttcc taactgaaga 240
taaaaaaaaa tggaatctgg gaagtaatgc caaggaccct cgagggatgt atcagtgtaa 300
aggatcacag aacaagtcaa aaccactcca agtgtattac agaatgtgtc agaactgcat 360
tgaactaaat gcagccacca tatctggctt tctctttgct gaaatcgtca gcattttcgt 420
ccttgctgtt ggggtctact tcattgctgg acaggatgga gttcgccagt cgagagcttc 480
agacaagcag actctgttgc ccaatgacca gctctaccag cccctcaagg atcgagaaga 540
tgaccagtac agccaccttc aaggaaacca gttgaggagg aattgaactc aggactcaga 600
gtagtccagg tgttctcctc ctattcagtt cccagaatca aagcaatgca ttttggaaag 660
ctcctagcag agagactttc agccctaaat ctagactcaa ggttcccaga gatgacaaat 720
ggagaagaaa ggccatcaga gcaaatttgg gggtttctca aataaaataa aaataaaaac 780
aaatactgtg tttcagaagc gccacctatt ggggaaaatt gt 822

<210> 10
<211> 1855
<212> DNA
<213> Homo sapiens

<400> 10
ggttgctact gctgcggcta accaaacagc tcatgcttct ctgaagactt gcagcaaggt 60
ttgctgaggc tcacagaaga tagccccagt gttttggagt ggttttgaat gtgattctga 120
gatcagactg actgagctgg aatcctggct ttatatctta ccagctacac aaccttggag 180
tcttagaaat tttttctttt caataagcag tcatccttac tttccctcaa gatgacaaac 240
agttcgttct tctgcccagt ttataaagat ctggagccat tcacgtattt tttttattta 300
gttttccttg ttggaattat tggaagttgt tttgcaacct gggcttttat acagaagaat 360

acgaatcaca ggtgtgtgag catctactta attaatttgc ttacagccga tttcctgctt 420
actctggcat taccagtgaa aattgttgtt gacttgggtg tggcaccttg gaagctgaag 480
atattccact gccaagtaac agcctgcctc atctatatca atatgtattt atcaattatc 540
ttcttagcat ttgtcagcat tgaccgctgt cttcagctga cacacagctg caagatctac 600
cgaatacaag aacccggatt tgccaaaatg atatcaaccg ttgtgtggct aatggtcctt 660
cttataatgg tgccaaatat gatgattccc atcaaagaca tcaaggaaaa gtcaaatgtg 720
ggttgtatgg agtttaaaaa ggaatttgga agaaattggc atttgctgac aaatttcata 780
tgtgtagcaa tatttttaaa tttctcagcc atcattttaa tatccaattg ccttgtaatt 840
cgacagctct acagaaacaa agataatgaa aattacccaa atgtgaaaaa ggctctcatc 900
aacatacttt tagtgaccac gggctacatc atatgctttg ttccttacca cattgtccga 960
atcccgtata ccctcagcca gacagaagtc ataactgatt gctcaaccag gatttcactc 1020
ttcaaagcca aagaggctac actgctcctg gctgtgtcga acctgtgctt tgatcctgtc 1080
ctgtactatc acctctcaaa agcattccgc tcaaaggtca ctgagacttt tgcctcacct 1140
aaagagacca aggctcagaa agaaaaatta agatgtgaaa ataatgcata aaagacagga 1200
tttttgtgct accaattctg gccttactgg accataaagt taattatagc tttgaaagat 1260
aaaaaaaaaa aaaaaaacaa aaaaaaactc agtatgaaaa aatacagtta gctagcaaat 1320
atggacaggt ttacttagaa atcctgtttc taaatgcaag tcaagcttta ttgttaggct 1380
tgctgctact cattaaccca aatatttgta caaaaaacta aagagtctca ttgaacgaat 1440
gtaaaatcct gcaatatcct tgaaatccaa aagaggtcca tgacatagac ccaaaggtat 1500
tcatgagtta ttcatttaaa tgcctggaac tgacttcttg ataaaaatat aaaaaataat 1560
ttccatgtaa gttaccagaa agcccaccag caacataatt ttaaagcctt tcggattact 1620
tttaaaaaat gcagcttaca tataacaact tgtgcctatt ttatttctaa tctatcactt 1680
caaaagatgg taatctttca actcattatt cctccaattt ttaatgtcga atttttttct 1740
aacacaataa ccaaaagctt ttatttataa aaaggcttga aaaatataaa aaaaaaaaaa 1800
aaaaaaaaaa aaaaaaaaaa aaaaaaaaaa aaaaaaaaaa aaaaaaaaaa aaaaa 1855

<210> 11
<211> 2109
<212> DNA
<213> Homo sapiens

<400> 11
gggggcaacg gtcacctgat ctgcggctgt cgaggccgct gaggcagtgg aggctgaggc 60
tatgatggcg gccatggcga cggctcgagt gcggatgggg ccgcgatgcg cccaggcgct 120
ctggcgcatg ccgtggctgc cggtgttttt gtcgttggcg gcggcggcgg cggcggcagc 180
ggcggagcag caggtcccgc tggtgctgtg gtcgagtgac cgggacttgt gggctcctgc 240
ggccgacact catgaaggcc acatcaccag cgacttgcag ctctctacct acttagatcc 300
cgccctggag ctgggtccca ggaatgtgct gctgttcctg caggacaagc tgagcattga 360
ggatttcaca gcatatggcg gtgtgtttgg aaacaagcag gacagcgcct tttctaacct 420
agagaatgcc ctggacctgg ccccctcctc actggtgctt cctgccgtcg actggtatgc 480
agtcagcact ctgaccactt acctgcagga gaagctcggg gccagcccct tgcatgtgga 540
cctggccacc ctgcgggagc tgaagctcaa tgccagcctc cctgctctgc tgctcattcg 600
cctgccctac acagccagct ctggtctgat ggcacccagg gaagtcctca caggcaacga 660
tgaggtcatc gggcaggtcc tgagcacact caagtccgaa gatgtcccat acacagcggc 720
cctcacagcg gtccgccctt ccagggtggc ccgtgatgta gccgtggtgg ccggagggct 780
aggtcgccag ctgctacaaa aacagccagt atcacctgtg atccatcctc ctgtgagtta 840
caatgacacc gctccccgga tcctgttctg ggcccaaaac ttctctgtgg cgtacaagga 900
ccagtgggag gacctgactc ccctcacctt tggggtgcag gaactcaacc tgactggctc 960
cttctggaat gactcctttg ccaggctctc actgacctat gaacgactct ttggtaccac 1020
agtgacattc aagttcattc tggccaaccg cctctaccca gtgtctgccc ggcactggtt 1080
taccatggag cgcctcgaag tccacagcaa tggctccgtc gcctacttca atgcttccca 1140
ggtcacaggg cccagcatct actccttcca ctgcgagtat gtcagcagcc tgagcaagaa 1200
gggtagtctc ctcgtggccc gcacgcagcc ctctccctgg cagatgatgc ttcaggactt 1260
ccagatccag gctttcaacg taatggggga gcagttctcc tacgccagcg actgtgccag 1320
cttcttctcc cccggcatct ggatggggct gctcacctcc ctgttcatgc tcttcatctt 1380
cacctatggc ctgcacatga tcctcagcct caagaccatg gatcgctttg atgaccacaa 1440
gggccccact atttctttga cccagattgt gtgaccctgt gccagtgggg gggttgaggg 1500
tgggacggtg tccgtgttgt tgctttccca ccctgcagcg cactggactg aagagcttcc 1560

ctcttcctac tgcagcatga actgcaagct cccctcagcc catcttgctc cctcttcagc 1620
ccgctgagga gctttcttgg gctgccccca tctctcccaa caaggtgtac atattctgcg 1680
tagatgctag accaaccagc ttcccagggt tcgtcgctgt gaggcgtaag ggacatgaat 1740
tctagggtct cctttctcct tatttattct tgtggctaca tcatccctgg ctgtggatag 1800
tgcttttgtg tagcaaatgc tccctcctta aggttatagg gctccctgag tttgggagtg 1860
tggaagtact acttaactgt ctgtcctgct tggctgtcgt tatcgttttc tggtgatgtt 1920
gtgctaacaa taagaagtac acgggtttat ttctgtggcc tgagaaggaa gggacctcca 1980
cgacaggtgg gctgggtgcg atcgccggct gtttggcatg ttcccaccgg gagtgccggg 2040
caggagcatg gggtgcttgg ttgtttcctt cctaataaaa taaacgcggg tcgccatgca 2100
aaaaaaaaa 2109

<210> 12
<211> 1267
<212> DNA
<213> Homo sapiens

<400> 12
acattctctt ttcttttatt cttgtctgtt ctgcctcact cccgagctct actgactccc 60
aacagagcgc ccaagaagaa aatggccata agtggagtcc ctgtgctagg atttttcatc 120
atagctgtgc tgatgagcgc tcaggaatca tgggctatca aagaagaaca tgtgatcatc 180
caggccgagt tctatctgaa tcctgaccaa tcaggcgagt ttatgtttga ctttgatggt 240
gatgagattt tccatgtgga tatggcaaag aaggagacgg tctggcggct tgaagaattt 300
ggacgatttg ccagctttga ggctcaaggt gcattggcca acatagctgt ggacaaagcc 360
aacctggaaa tcatgacaaa gcgctccaac tatactccga tcaccaatgt acctccagag 420
gtaactgtgc tcacgaacag ccctgtggaa ctgagagagc ccaacgtcct catctgtttc 480
atagacaagt tcaccccacc agtggtcaat gtcacgtggc ttcgaaatgg aaaacctgtc 540
accacaggag tgtcagagac agtcttcctg cccagggaag accacctttt ccgcaagttc 600
cactatctcc ccttcctgcc ctcaactgag gacgtttacg actgcagggt ggagcactgg 660
ggcttggatg agcctcttct caagcactgg gagtttgatg ctccaagccc tctcccagag 720
actacagaga acgtggtgtg tgccctgggc ctgactgtgg gtctggtggg catcattatt 780
gggaccatct tcatcatcaa gggattgcgc aaaagcaatg cagcagaacg cagggggcct 840
ctgtaaggca catggaggtg atggtgtttc ttagagagaa gatcactgaa gaaacttctg 900
ctttaatggc tttacaaagc tggcaatatt acaatccttg acctcagtga aagcagtcat 960
cttcagcatt ttccagccct atagccaccc caagtgtgga tatgcctctt cgattgctcc 1020
gtactctaac atctagctgg cttccctgtc tattgccttt tcctgtatct attttcctct 1080
atttcctatc attttattat caccatgcaa tgcctctgga ataaaacata caggagtctg 1140
tctctgctat ggaatgcccc atggggcatc tcttgtgtac ttattgttta aggtttcctc 1200
aaactgtgat ttttctgaac acaataaact attttgatga tcttgggtgg aaaaaaaaaa 1260
aaaaaaa 1267

<210> 13
<211> 1190
<212> DNA
<213> Homo sapiens

<400> 13
cagatccatc aggtccgagc tgtgttgact accacttttc ccttcgtctc aattatgtct 60
tggaaaaagg ctttgcggat ccccggaggc cttcgggcag caactgtgac cttgatgctg 120
tcgatgctga gcaccccagt ggctgagggc agagactctc ccgaggattt cgtgtaccag 180
tttaagggca tgtgctactt caccaacggg acagagcgcg tgcgtcttgt gagcagaagc 240
atctataacc gagaagagat cgtgcgcttc gacagcgacg tgggggagtt ccgggcggtg 300
acgctgctgg ggctgcctgc cgccgagtac tggaacagcc agaaggacat cctggagagg 360
aaacgggcgg cggtggacag ggtgtgcaga cacaactacc agttggagct ccgcacgacc 420
ttgcagcggc gagtggagcc cacagtgacc atctccccat ccaggacaga ggccctcaac 480
caccacaacc tgctggtctg ctcggtgaca gatttctatc cagcccagat caaagtccgg 540
tggtttcgga atgaccagga ggagacagct ggcgttgtgt ccacccccct tattaggaat 600
ggtgactgga ccttccagat cctggtgatg ctggaaatga ctccccagcg tggagacgtc 660
tacacctgcc acgtggagca ccccagcctc cagagcccca tcaccgtgga gtggcgggct 720

caatctgaat ctgcccagag caagatgctg agtggcattg gaggcttcgt gctggggctg 780
atcttcctcg ggctgggcct tatcatccat cacaggagtc agaaagggct cctgcactga 840
ctcctgagac tattttaact gggattggtt atcacttttc tgtaacgcct gcttgtccct 900
gcccagaatt cccagctgtc tgtgtcagcc tgtccccctg agatcagagt cctacagtgg 960
ctgtcacgca gccaccaggt catctccttt catccccacc ttgaggcgga tggctgtgac 1020
cctacttcct gcactgaccc acagcctctg cctgtgcacg gccagctgca tctactcagg 1080
ccccaagggg tttctgtttc tattctctcc tcagactgct caagagaagc acatgaaaac 1140
cattacctga ctttagagct tttttacata attaaacatg atcctgagtt 1190

<210> 14
<211> 2666
<212> DNA
<213> Homo sapiens

<400> 14
atgcactcaa gcagagaaga aatccacaaa gactcacagt ctgctggtgg gcagagaaga 60
cagaaacgac atgagcacag caggaaaagt aatcaaatgc aaagcagctg tgctatggga 120
ggtaaagaaa cccttttcca ttgaggatgt ggaggttgca cctcctaagg cttatgaagt 180
tcgcattaag atggtggctg taggaatctg tcacacagat gaccacgtgg ttagtggcaa 240
cctggtgacc ccccttcctg tgattttagg ccatgaggca gccggcatcg tggagagtgt 300
tggagaaggg gtgactacag tcaaaccagg tgataaagtc atcccgctct ttactcctca 360
gtgtggaaaa tgcagagttt gtaaaaaccc ggagagcaac tactgcttga aaaatgatct 420
aggcaatcct cgggggaccc tgcaggatgg caccaggagg ttcacctgca gggggaagcc 480
cattcaccac ttccttggca ccagcacctt ctcccagtac acggtggtgg atgagaatgc 540
agtggccaaa attgatgcag cctcgcccct ggagaaagtc tgcctcattg gctgtggatt 600
ctcgactggt tatgggtctg cagttaacgt tgccaaggtc accccaggct ctacctgtgc 660
tgtgtttggc ctgggagggg tcggcctatc tgctgttatg ggctgtaaag cagctggagc 720
agccagaatc attgcggtgg acatcaacaa ggacaaattt gcaaaggcca aagagttggg 780
tgccactgaa tgcatcaacc ctcaagacta caagaaaccc atccaggaag tgctaaagga 840
aatgactgat ggaggtgtgg atttttcgtt tgaagtcatc ggtcggcttg acaccatgat 900
ggcttccctg ttatgttgtc atgaggcatg tggcacaagc gtcatcgtag gggtacctcc 960
tgcttcccag aacctctcaa taaaccctat gctgctactg actggacgca cctggaaggg 1020
ggctgtttat ggtggcttta agagtaaaga aggtatccca aaacttgtgg ctgattttat 1080
ggctaagaag ttttcactgg atgcgttaat aacccatgtt ttaccttttg aaaaaataaa 1140
tgaaggattt gacctgcttc actctgggaa aagtatccgt accgtcctga cgttttgagg 1200
caatagagat gccttcccct gtagcagtct tcagcctcct ctaccctaca agatctggag 1260
caacagctag gaaatatcat taattcagct cttcagagat gttatcaata aattacacat 1320
gggggctttc caaagaaatg gaaattgatg ggaaattatt tttcaggaaa atttaaaatt 1380
caagtgagaa gtaaataaag tgttgaacat cagctgggga attgaagcca acaaaccttc 1440
cttcttaacc attctactgt gtcacctttg ccattgagga aaaatattcc tgtgacttct 1500
tgcatttttg gtatcttcat aatctttagt catcgaatcc cagtggaggg gaccctttta 1560
cttgccctga acatacacat gctgggccat tgtgattgaa gtcttctaac tctgtctcag 1620
ttttcactgt cgacattttc ctttttctaa taaaaatgta ccaaatccct ggggtaaaag 1680
ctagggtaag gtaaaggata gactcacatt tacaagtagt gaaggtccaa gagttctaaa 1740
tacaggaaat ttcttaggaa ctcaaataaa atgccccaca ttttactaca gtaaatggca 1800
gtgtttttat gacttttata ctatttcttt atggtcgata tacaattgat tttttaaaat 1860
aatagcagat ttcttgcttc atatgacaaa gcctcaatta ctaattgtaa aaactgaact 1920
attcccagaa tcatgttcaa aaaatctgta atttttgctg atgaaagtgc ttcattgact 1980
aaacagtatt agtttgtggc tataaatgat tatttagatg atgactgaaa atgtgtataa 2040
agtaattaaa agtaatatgg tggctttaag tgtagagatg ggatggcaaa tgctgtgaat 2100
gcagaatgta aaattggtaa ctaagaaatg gcacaaacac cttaagcaat atattttcct 2160
agtagatata tatatacaca tacatatata cacatataca aatgtatatt tttgcaaaat 2220
tgttttcaat ctagaacttt tctattaact accatgtctt aaaatcaagt ctataatcct 2280
agcattagtt taatattttg aatatgtaaa gacctgtgtt aatgctttgt taatgctttt 2340
cccactctca tttgttaatg ctttcccact ctcaggggaa ggatttgcat tttgagcttt 2400
atctctaaat gtgacatgca aagattattc ctggtaaagg aggtagctgt ctccaaaaat 2460
gctattgttg caatatctac attctatttc atattatgaa agaccttaga cataaagtaa 2520
aatagtttat catttactgt gtgatcttca gtaagtctct caggctctct gagcttgttc 2580

atcctttgtt ttgaaaaaat tactcaacca atccattaca gcttaaccaa gattaaatgg 2640
gatgatgtta atgaaagagc ttcgcc 2666

<210> 15
<211> 1508
<212> DNA
<213> Homo sapiens

<400> 15
ttttgaaacc cttcaaaggc agagacttgt ccagcctaac ctgcctgctg ctcctagctc 60
ctgaggctca gggcccttgg cttctgtccg ctctgctcag ggccctccag cgtggccact 120
gctcagccat gctcctgctg ctcgtcccag tgctcgaggt gatttttacc ctgggaggaa 180
ccagagccca gtcggtgacc cagcttggca gccacgtctc tgtctctgaa ggagccctgg 240
ttctgctgag gtgcaactac tcatcgtctg ttccaccata tctcttctgg tatgtgcaat 300
accccaacca aggactccag cttctcctga agtacacatc agcggccacc ctggttaaag 360
gcatcaacgg ttttgaggct gaatttaaga agagtgaaac ctccttccac ctgacgaaac 420
cctcagccca tatgagcgac gcggctgagt acttctgtgc tgtgagtgat ctcgaaccga 480
acagcagtgc ttccaagata atctttggat cagggaccag actcagcatc cggccaaata 540
tccagaaccc tgaccctgcc gtgtaccagc tgagagactc taaatccagt gacaagtctg 600
tctgcctatt caccgatttt gattctcaaa caaatgtgtc acaaagtaag gattctgatg 660
tgtatatcac agacaaaact gtgctagaca tgaggtctat ggacttcaag agcaacagtg 720
ctgtggcctg gagcaacaaa tctgactttg catgtgcaaa cgccttcaac aacagcatta 780
ttccagaaga caccttcttc cccagcccag aaagttcctg tgatgtcaag ctggtcgaga 840
aaagctttga aacagatacg aacctaaact ttcaaaacct gtcagtgatt gggttccgaa 900
tcctcctcct gaaagtggcc gggtttaatc tgctcatgac gctgcggctg tggtccagct 960
gagatctgca agattgtaag acagcctgtg ctccctcgct ccttcctctg cattgcccct 1020
cttctccctc tccaaacaga gggaactctc ctacccccaa ggaggtgaaa gctgctacca 1080
cctctgtgcc cccccggtaa tgccaccaac tggatcctac ccgaatttat gattaagatt 1140
gctgaagagc tgccaaacac tgctgccacc ccctctgttc ccttattgct gcttgtcact 1200
gcctgacatt cacggcagag gcaaggctgc tgcagcctcc cctggctgtg cacattccct 1260
cctgctcccc agagactgcc tccgccatcc cacagatgat ggatcttcag tgggttctct 1320
tgggctctag gtcctggaga atgttgtgag gggtttattt ttttttaata gtgttcataa 1380
agaaatacat agtattcttc ttctcaagac gtggggggaa attatctcat tatcgaggcc 1440
ctgctatgct gtgtgtctgg gcgtgttgta tgtcctgctg ccgatgcctt cattaaaatg 1500
atttggaa 1508

<210> 16
<211> 1702
<212> DNA
<213> Homo sapiens

<400> 16
agactcaaca agagctccag caaagacttt cactgtagct tgacttgacc tgagattaac 60
tagggaatct tgagaataaa gatgagctct gaaaattgtt tcgtagcaga gaacagctct 120
ttgcatccgg agagtggaca agaaaatgat gccaccagtc cccatttctc aacacgtcat 180
gaagggtcct tccaagttcc tgtcctgtgt gctgtaatga atgtggtctt catcaccatt 240
ttaatcatag ctctcattgc cttatcagtg ggccaataca attgtccagg ccaatacaca 300
ttctcaatgc catcagacag ccatgtttct tcatgctctg aggactgggt tggctaccag 360
aggaaatgct actttatttc tactgtgaag aggagctgga cttcagccca aaatgcttgt 420
tctgaacatg gtgctactct tgctgtcatt gattctgaaa aggacatgaa ctttctaaaa 480
cgatacgcag gtagagagga acactgggtt ggactgaaaa aggaacctgg tcacccatgg 540
aagtggtcaa atggcaaaga atttaacaac tggttcaacg ttacagggtc tgacaagtgt 600
gtttttctga aaaacacaga ggtcagcagc atggaatgtg agaagaattt atactggata 660
tgtaacaaac cttacaaata ataaggaaac atgttcactt attgactatt atagaatgga 720
actcaaggaa atctgtgtca gtggatgctg ctctgtggtc cgaagtcttc catagagact 780
ttgtgaaaaa aaattttata gtgtcttggg aattttcttc caaacagaac tatggaaaaa 840
aaggaagaaa ttccaggaaa atctgcactg tgggctttta ttgccatgag ctagaagcat 900
cacaggttga ccaataacca tgcccaagaa tgagaagaat gactatgcaa cctttggatg 960

cactttatat tattttgaat ccagaaataa tgaaataact aggcgtggac ttactattta 1020
ttgctgaatg actaccaaca gtgagagccc ttcatgcatt tgcactactg gaaggagtta 1080
gatgttggta ctagatactg aatgtaaaca aaggaattat ggctggtaac ataggttttt 1140
agtctaattg aatcccttaa actcagggag catttataaa tggacaaatg cttatgaaac 1200
taagatttgt aatatttctc tctttttaga gaaatttgcc aatttacttt gttatttttc 1260
cccaaaaaga atgggatgat cgtgtattta tttttttact tcctcagctg tagacaggtc 1320
cttttcgatg gtacatattt ctttgccttt ataatctttt atacagtgtc ttacagagaa 1380
aagacataag caaagactat gaggaatatt tgcaagacat agaatagtgt tggaaaatgt 1440
gcaatatgtg atgtggcaaa tctctattag gaaatattct gtaatcttca gacctagaat 1500
aatactagtc ttataatagg tttgtgactt tcctaaatca attctattac gtgcaatact 1560
tcaatacttc atttaaaata tttttatgtg caataaaatg tatttgtttg tattttgtgt 1620
tcagtacaat tataagctgt ttttatatat gtgaaataaa agtagaataa acacaaaaaa 1680
aaaaaaaaaa aaaaaaaaaa aa 1702

<210> 17
<211> 3273
<212> DNA
<213> Homo sapiens

<400> 17
agtccccgcg cagtccccgc gcagtcccag cgccaccggg cagcagcggc gccgtgctcg 60
ctccagggcg caaccatgtc gccatttctt cggattggct tgtccaactt tgactgcggg 120
tcctgccagt cttgtcaggg cgaggctgtt aacccttact gtgctgtgct cgtcaaagag 180
tatgtcgaat cagagaacgg gcagatgtat atccagaaaa agcctaccat gtacccaccc 240
tgggacagca cttttgatgc ccatatcaac aagggaagag tcatgcagat cattgtgaaa 300
ggcaaaaacg tggacctcat ctctgaaacc accgtggagc tctactcgct ggctgagagg 360
tgcaggaaga acaacgggaa gacagaaata tggttagagc tgaaacctca aggccgaatg 420
ctaatgaatg caagatactt tctggaaatg agtgacacaa aggacatgaa tgaatttgag 480
acggaaggct tctttgcttt gcatcagcgc cggggtgcca tcaagcaggc aaaggtccac 540
cacgtcaagt gccacgagtt cactgccacc ttcttcccac agcccacatt ttgctctgtc 600
tgccacgagt ttgtctgggg cctgaacaaa cagggctacc agtgccgaca atgcaatgca 660
gcaattcaca agaagtgtat tgataaagtt atagcaaagt gcacaggatc agctatcaat 720
agccgagaaa ccatgttcca caaggagaga ttcaaaattg acatgccaca cagatttaaa 780
gtctacaatt acaagagccc gaccttctgt gaacactgtg ggaccctgct gtggggactg 840
gcacggcaag gactcaagtg tgatgcatgt ggcatgaatg tgcatcatag atgccagaca 900
aaggtggcca acctttgtgg cataaaccag aagctaatgg ctgaagcgct ggccatgatt 960
gagagcactc aacaggctcg ctgcttaaga gatactgaac agatcttcag agaaggtccg 1020
gttgaaattg gtctcccatg ctccatcaaa aatgaagcaa ggccgccatg tttaccgaca 1080
ccgggaaaaa gagagcctca gggcatttcc tgggagtctc cgttggatga ggtggataaa 1140
atgtgccatc ttccagaacc tgaactgaac aaagaaagac catctctgca gattaaacta 1200
aaaattgagg attttatctt gcacaaaatg ttggggaaag gaagttttgg caaggtcttc 1260
ctggcagaat tcaagaaaac caatcaattt ttcgcaataa aggccttaaa gaaagatgtg 1320
gtcttgatgg acgatgatgt tgagtgcacg atggtagaga agagagttct ttccttggcc 1380
tgggagcatc cgtttctgac gcacatgttt tgtacattcc agaccaagga aaacctcttt 1440
tttgtgatgg agtacctcaa cggaggggac ttaatgtacc acatccaaag ctgccacaag 1500
ttcgaccttt ccagagcgac gttttatgct gctgaaatca ttcttggtct gcagttcctt 1560
cattccaaag gaatagtcta cagggacctg aagctagata acatcctgtt agacaaagat 1620
ggacatatca agatcgcgga ttttggaatg tgcaaggaga acatgttagg agatgccaag 1680
acgaatacct tctgtgggac acctgactac atcgccccag agatcttgct gggtcagaaa 1740
tacaaccact ctgtggactg gtggtccttc ggggttctcc tttatgaaat gctgattggt 1800
cagtcgcctt tccacgggca ggatgaggag gagctcttcc actccatccg catggacaat 1860
ccctttaccc cacggtggct ggagaaggaa gcaaaggacc ttctggtgaa gctcttcgtg 1920
cgagaacctg agaagaggct gggcgtgagg ggagacatcc gccagcaccc tttgtttcgg 1980
gagatcaact gggaggaact tgaacggaag gagattgacc caccgttccg gccgaaagtg 2040
aaatcaccat ttgactgcag caatttcgac aaagaattct taaacgagaa gccccggctg 2100
tcatttgccg acagagcact gatcaacagc atggaccaga atatgttcag gaacttttcc 2160
ttcatgaacc ccgggatgga gcggctgata tcctgaatct tgcccctcca gagacaggaa 2220
agaatttgcc ttctccctgg gaactggttc aagagacact gcttgggttc ctttttcaac 2280

ttggaaaaag aaagaaacac tcaacaataa agactgagac ccgttcgccc ccatgtgact 2340
tttatctgta gcagaaacca agtctacttc actaatgacg atgccgtgtg tctcgtctcc 2400
tgacatgtct cacagacgct cctgaagtta ggtcattact aaccatagtt atttacttga 2460
aagatgggtc tccgcacttg gaaaggtttc aagacttgat actgcaataa attatggctc 2520
ttcacctggg cgccaactgc tgatcaatga aatgcttgtt gaatcagggg caaacggagt 2580
acagacgtct caagactgaa acggccccat tgcctggtct agtagcggat ctcactcagc 2640
cgcagacaag taatcactaa cccgttttat tctattccta tctgtggatg tgtaaatggc 2700
tgggggccca gccctggata ggtttttatg ggaattcttt acaataaaca tagcttgtaa 2760
cttgagatct acaaatccat tcatcctgat tgggcatgaa atccatggtc aagaggacaa 2820
gtggaaagtg agagggaagg tttgctagac accttcgctt gttatcttgt caagatagaa 2880
aagatagtat catttcaccc ttgccagtaa aaacctttcc atccacccat tctcagcaga 2940
ctccagtatt ggcacagtca ctcactgcca ttctcacact ataacaagaa aagaaatgaa 3000
gtgcataagt ctcctgggaa aagaacctta accccttctc gtgccatgac tggtgatttc 3060
atgactcata agcccctccg taggcatcat tcaagatcaa tggcccatgc atgctgtttg 3120
cagcagtcaa ttgagttgaa ttagaattcc aaccatacat tttaaaggta tttgtgctgt 3180
gtgtatattt tgataaaatg ttgtgacttc atggcaaaca ggtggatgtg taaaaatgga 3240
ataaaaaaaa aaaaagagtc aaaaaaaaaa aaa 3273

<210> 18
<211> 1074
<212> DNA
<213> Homo sapiens

<400> 18
gtaaagctcc catcctgccc tgaccctgcc atgggcacca gcctcctctg ctggatggcc 60
ctgtgtctcc tgggggcaga tcacgcagat actggagtct cccagaaccc cagacacaac 120
atcacaaaga ggggacagaa tgtaactttc aggtgtgatc caatttctga acacaaccgc 180
ctttattggt accgacagac cctggggcag ggcccagagt ttctgactta cttccagaat 240
gaagctcaac tagaaaaatc aaggctgctc agtgatcggt tctctgcaga gaggcctaag 300
ggatctttct ccaccttgga gatccagcgc acagagcagg gggactcggc catgtatctc 360
tgtgccagca gcttagcagg gttgaatcag ccccagcatt ttggtgatgg gactcgactc 420
tccatcctag aggacctgaa caaggtgttc ccacccgagg tcgctgtgtt tgagccatca 480
gaagcagaga tctcccacac ccaaaaggcc acactggtgt gcctggccac aggtatcttc 540
cctgaccacg tggagctgag ctggtgggtg aatgggaagg aggtgcacag tggggtcagc 600
acggacccgc agcccctcaa ggagcagccc gccctcaatg actccagata ctgcctgagc 660
agccgcctga gggtctcggc caccttctgg cagaaccccc gcaaccactt ccgctgtcaa 720
gtccagttct acgggctctc ggagaatgac gagtggaccc aggatagggc caaacccgtc 780
acccagatcg tcagcgccga ggcctggggt agagcagact gtggctttac ctcggtgtcc 840
taccagcaag gggtcctgtc tgccaccatc ctctatgaga tcctgctagg gaaggccacc 900
atgtatgctg tgctggtcag cgcccttgtg ttgatggcca tggtcaagag aaaggatttc 960
tgaaggcagc cctggaagtg gagttaggag cttctaaccc gtcatggttt caatacacat 1020
tcttcttttg ccagcgcttc tgaagagctg ctctcacctc tctgcatccc aata 1074

<210> 19
<211> 1080
<212> DNA
<213> Homo sapiens

<400> 19
tccaaaatga agggtctgtg gaaggacatg aataaagcac aggaggttga agtcagattt 60
gcagctttct aggcaggaga caagacaatc tgcatcttca caggagggat ggccatgctc 120
ctgggggcat cagtgctgat tctgtggctt cagccagact gggtaaacag tcaacagaag 180
aatgatgacc agcaagttaa gcaaaattca ccatccctga gcgtccagga aggaagaatt 240
tctattctga actgtgacta tactaacagc atgtttgatt atttcctatg gtacaaaaaa 300
taccctgctg aaggtcctac attcctgata tctataagtt ccattaagga taaaaatgaa 360
gatggaagat tcactgtctt cttaaacaaa agtgccaagc acctctctct gcacattgtg 420
ccctcccagc ctggagactc tgcagtgtac ttctgtgcag caaagggggc cggcactgcc 480
agtaaactca cctttgggac tggaacaaga cttcaggtca cgctcgatat ccagaaccct 540

gaccctgccg tgtaccagct gagagactct aaatccagtg acaagtctgt ctgcctattc 600
accgattttg attctcaaac aaatgtgtca caaagtaagg attctgatgt gtatatcaca 660
gacaaaactg tgctagacat gaggtctatg gacttcaaga gcaacagtgc tgtggcctgg 720
agcaacaaat ctgactttgc atgtgcaaac gccttcaaca acagcattat tccagaagac 780
accttcttcc ccagcccaga aagttcctgt gatgtcaagc tggtcgagaa aagctttgaa 840
acagatacga acctaaactt tcaaaacctg tcagtgattg ggttccgaat cctcctcctg 900
aaagtggccg ggtttaatct gctcatgacg ctgcggctgt ggtccagctg agatctgcaa 960
gattgtaaga cagcctgtgc tccctcgctc cttcctctgc attgcccctc ttctccctct 1020
ccaaacagag ggaactctcc cacccccaag gaggtgaaag ctgctaccac cctctgtgcc 1080

<210> 20
<211> 1586
<212> DNA
<213> Homo sapiens

<400> 20
gaatcaggaa gaccagctcc tcctactgtc ttctgtgtta cgggatcagc gttccttgtt 60
gagtgggacc tgagttttga gagggtcttc tgctcctctt ggtctggtcc cttacttcca 120
agagccccag agaggaaggc atgctgttgg ctctagctct gcttctagct ttcctgcctc 180
ctgccagtca gaaatcttcc aacttggaag ggagaacaaa gtcagtcacc aggccaactg 240
ggtcatcagc tgtaatcact tgtgatcttc ctgtagaaaa tgccgtctac acccactggt 300
acctacacca ggaggggaag gccccacagc gtcttctgta ctatgactcc tacaactcca 360
gggttgtgtt ggaatcagga atcagtcgag aaaagtatca tacttatgca agcacaggga 420
agagccttaa atttatactg gaaaatctaa ttgaacgtga ctctggggtc tattactgtg 480
ccacctggaa ggattattat aagaaactct ttggcagtgg aacaacactt gttgtcacag 540
ataaacaact tgatgcagat gtttccccca agcccactat ttttcttcct tcgattgctg 600
aaacaaaact ccagaaggct ggaacatatc tttgtcttct tgagaaattt ttcccagata 660
ttattaagat acattggcaa gaaaagaaga gcaacacgat tctgggatcc caggagggga 720
acaccatgaa gactaacgac acatacatga aatttagctg gttaacggtg ccagaagagt 780
cactggacaa agaacacaga tgtatcgtca gacatgagaa taataaaaac ggaattgatc 840
aagaaattat ctttcctcca ataaagacag atgtcaccac agtggatccc aaagacagtt 900
attcaaaaga tgcaaatgat gtcaccacag tggatcccaa atacaattat tcaaaggatg 960
caaatgatgt catcacaatg gatcccaaag acaattggtc aaaagatgca aatgatacac 1020
tactgctgca gctcacaaac acctctgcat attacatgta cctcctcctg ctcctcaaga 1080
gtgtggtcta ttttgccatc atcacctgct gtctgcttgg aagaacggct ttctgctgca 1140
atggagagaa atcataacag acggtggcac aaggaggcca tcttttcctc atcggttatt 1200
gtccctagaa gcgtcttctg aggatctagt tgggctttct ttctgggttt gggccatttc 1260
agttctcatg tgtgtactat tctatcatta ttgtataatg gttttcaaac cagtgggcac 1320
acagagaacc tcagtctgta ataacaatga ggaatagcca tggcgatctc cagcaccaat 1380
ctctccatgt tttccacagc tcctccagcc aacccaaata gcgcctgcta tagtgtagac 1440
agcctgcggc ttctagcctt gtccctctct tagtgttctt taatcagata actgcctgga 1500
agcctttcat tttacacgcc ctgaagcagt cttctttgct agttgaatta tgtggtgtgt 1560
ttttccgtaa taagcaaaat aaattt 1586

<210> 21
<211> 942
<212> DNA
<213> Homo sapiens

<400> 21
atgagcatcg gcctcctgtg ctgtgcagcc ttgtctctcc tgtgggcagg tccagtgaat 60
gctggtgtca ctcagacccc aaaattccag gtcctgaaga caggacagag catgacactg 120
cagtgtgccc aggatatgaa ccatgaatac atgtcctggt atcgacaaga cccaggcatg 180
gggctgaggc tgattcatta ctcagttggt gctggtatca ctgaccaagg agaagtcccc 240
aatggctaca atgtctccag atcaaccaca gaggatttcc cgctcaggct gctgtcggct 300
gctccctccc agacatctgt gtacttctgt gccagcagtt tcccccggca gccgtcctac 360
aatgagcagt tcttcgggcc agggacacgg ctcaccgtgc tagaggacct gaaaaacgtg 420

ttcccacccg aggtcgctgt gtttgagcca tcagaagcag agatctccca cacccaaaag 480
gccacactgg tgtgcctggc cacaggtttc taccccgacc acgtggagct gagctggtgg 540
gtgaatggga aggaggtgca cagtggggtc agcacagacc cgcagcccct caaggagcag 600
cccgccctca atgactccag atactgcctg agcagccgcc tgagggtctc ggccaccttc 660
tggcagaacc cccgcaacca cttccgctgt caagtccagt tctacgggct ctcggagaat 720
gacgagtgga cccaggatag ggccaaacct gtcacccaga tcgtcagcgc cgaggcctgg 780
ggtagagcag actgtggctt cacctccgag tcttaccagc aaggggtcct gtctgccacc 840
atcctctatg agatcttgct agggaaggcc accttgtatg ctgtgctggt cagtgccctc 900
gtgctgatgg ccatggtcaa gagaaaggat tccagaggct ga 942

<210> 22
<211> 840
<212> DNA
<213> Homo sapiens

<400> 22
atggcatgcc ctggcttcct gtgggcactt gtgatctcca cctgtcttga atttagcatg 60
gctcagacag tcactcagtc tcaaccagag atgtctgtgc aggaggcaga gaccgtgacc 120
ctgagctgca catatgacac cagtgagagt gattattatt tattctggta caagcagcct 180
cccagcaggc agatgattct cgttattcgc caagaagctt ataagcaaca gaatgcaaca 240
gagaatcgtt tctctgtgaa cttccagaaa gcagccaaat ccttcagtct caagatctca 300
gactcacagc tgggggatgc cgcgatgtat ttctgtgctt ataggagtgc atactctggg 360
gctgggagtt accaactcac tttcgggaag gggaccaaac tctcggtcat accaaatatc 420
cagaaccctg accctgccgt gtaccagctg agagactcta aatccagtga caagtctgtc 480
tgcctattca ccgattttga ttctcaaaca aatgtgtcac aaagtaagga ttctgatgtg 540
tatatcacag acaaaactgt gctagacatg aggtctatgg acttcaagag caacagtgct 600
gtggcctgga gcaacaaatc tgactttgca tgtgcaaacg ccttcaacaa cagcattatt 660
ccagaagaca ccttcttccc cagcccagaa agttcctgtg atgtcaagct ggtcgagaaa 720
agctttgaaa cagatacgaa cctaaacttt caaaacctgt cagtgattgg gttccgaatc 780
ctcctcctga aagtggccgg gtttaatctg ctcatgacgc tgcggttgtg gtccagctga 840

<210> 23
<211> 671
<212> DNA
<213> Homo sapiens

<400> 23
tgcctggcca caggcttctt ccccgaccac gtggagctga gctggtgggt gaatgggaag 60
gaggtgcaca atggggtcaa cacagacccg cagcccctca aagaacagcc cgccctcaat 120
gactccaaat amtgcctgag cagccgcctg agggtctcgg ccaccttctg gcagaacccc 180
cgcaaccact tccgctgtca agtccagttc tacgggctct cggagaatga cgagtggacc 240
caggataggg ccaaacccgt cacccaaatc gtcagcgccg aggcctgggg taragcaram 300
tgtggcttta cctcggtgtc ctaccaacaa ggggtcctgt ctgccaccat cctctatgar 360
atcctgctag ggaaggccac cctgtatgct gtgctggtca gcgcccttgt gttgatggcc 420
atggtcaaga gaaaggattt ctgaaggcag ccctggaagt ggaattaaga acttctaacc 480
cgtcatggtt tcaatacaca ttcttctttt gccagcgctt ctgaagagct gctctcacct 540
ctctgcatcc caatagatat ccccctatgt gcatgcacac ctgcacactc acggctgaaa 600
tctccctaac ccagggggac cttagcatgc ctaagtgact aaaccaataa aaatgttctg 660
gtctggcctg a 671

<210> 24
<211> 771
<212> DNA
<213> Homo sapiens

<400> 24
agagaagcag acatcttcta gttcctcccc cactctcctc tttccggtac ctgtgagtca 60

gctaggggag ggcagctctc acccaggctg atagttcggt gacctggctt tatctactgg 120
atgagttccg ctgggagatg gaacatagca cgtttctctc tggcctggta ctggctaccc 180
ttctctcgca agtgagcccc ttcaagatac ctatagagga acttgaggac agagtgtttg 240
tgaattgcaa taccagcatc acatgggtag agggaacggt gggaacactg ctctcagaca 300
ttacaagact ggacctggga aaacgcatcc tggacccacg aggaatatat aggtgtaatg 360
ggacagatat atacaaggac aaagaatcta ccgtgcaagt tcattatcga atgtgccaga 420
gctgtgtgga gctggatcca gccaccgtgg ctggcatcat tgtcactgat gtcattgcca 480
ctctgctcct tgctttggga gtcttctgct ttgctggaca tgagactgga aggctgtctg 540
gggctgccga cacacaagct ctgttgagga atgaccaggt ctatcagccc ctccgagatc 600
gagatgatgc tcagtacagc caccttggag gaaactgggc tcggaacaag tgaacctgag 660
actggtggct tctagaagca gccattacca actgtacctt cccttcttgc tcagccaata 720
aatatatcct ctttcactca gaaaaaaaaa aaaaaaaaaa aaaaaaaaaa a 771

<210> 25
<211> 1421
<212> DNA
<213> Homo sapiens

<400> 25
ggcatgcggt gggccctact ggtgcttcta gctttcctgt ctcctgccag tcagaaatct 60
tccaacttgg aagggagaac gaagtcagtc accaggcaga ctgggtcatc tgctgaaatc 120
acttgcgatc ttactgtaac aaataccttc tacatccact ggtacctaca ccaggagggg 180
aaggccccac agcgtcttct gtactatgac gtctccactg caagggatgt gttggaatca 240
ggactcagtc caggaaagta ttatactcat acacccagga ggtggagctg gatattgaga 300
ctgcaaaatc taattgaaaa tgattctggg gtctattact gtgccacctg ggacaggcaa 360
aaattattat aagaaactct ttggcagtgg aacaacactt gttgtcacag ataaacaact 420
tgatgcagat gtttccccca agcccactat ttttcttcct tcaattgctg aaacaaaact 480
ccagaaggct ggaacatacc tttgtcttct tgagaaattt ttcccagata ttattaagat 540
acattggcaa gaaaagaaga gcaacacgat tctgggatcc caggagggga acaccatgaa 600
gactaacgac acatacatga aatttagctg gttaacggtg ccagaagagt cactggacaa 660
agaacacaga tgtatcgtca gacatgagaa taataaaaac ggaattgatc aagaaattat 720
ctttcctcca ataaagacag atgtcaccac agtggatccc aaagacagtt attcaaaaga 780
tgcaaatgat gtcatcacaa tggatcccaa agacaattgg tcaaaagatg caaatgatac 840
actactgctg cagctcacaa acacctctgc atattacatg tacctcctcc tgctcctcaa 900
gagtgtggtc tattttgcca tcatcacctg ctgtctgctt ggaagaacgg ctttctgctg 960
caatggagag aaatcataac agacggtggc acaaggaggc catcttttcc tcatcggtta 1020
ttgtccctag aagcgtcttc tgaggatcta gttgggcttt ctttctgggt ttgggccatt 1080
tcagttctca tgtgtgtact attctatcat tattgtataa tggttttcaa accagtgggc 1140
acacagagaa cctcagtctg taataacaat gaggaatagc catggcgatc tccagcacca 1200
atctctccat gttttccaca gctcctccag ccaacccaaa tagcgcctgc tatagtgtag 1260
acagcctgcg gcttctagcc ttgtccctct cttagtgttc tttaatcaga taactgcctg 1320
gaagcctttc attttacacg ccctgaagca gtcttctttg ctagttgaat tatgtggtgt 1380
gtttttccgt aataagcaaa ataaatttaa aaaaatgaaa a 1421

<210> 26
<211> 1834
<212> DNA
<213> Homo sapiens

<400> 26
gtcaaaggaa taatcccatc taaaaagacg aaacagaaag aagtgtatcc tgctacacct 60
gcatgcaccc caagcaaccg tctcacagct aaaggagcag aggagactct tggacctcag 120
gtaagcttca ggaagaggag caggcttcaa gtctcacagt ggaagctctg ctgtggctgt 180
tccactcaat ctgtccagca ggcagttatt tcttcatatg tttcccatca agtttcagat 240
ttatcaaatt acataataat tgatcatctt tctgcaaggc aacaagttaa acgctttagt 300
aaacataatg taaatataca taaaataaat ataatatttt catctccaat agagaaggat 360
gttaacttga gagtcagata aaaaaacgtt tgcctatgtt tacaaaagcc tagtttctta 420
actgcaagtc agcatatccc aaaacacaag taattaagga atgatgtgtg ttactttctc 480

tgctcccttt ttaaaaatga aaccatctat gccatgttct ttcaattggc ctggggatgt 540
acttaagttt ccaagaaaaa caatttatat acaataaata tattaccttg taatgaaaat 600
gtgctctgct tcatttgaca ctgaaagtaa ttaacaagaa aaataaacta cttgtagaaa 660
agaaaaaaac catctgaaga agagactgga accaaaagga gtaagatgtc caaagagcag 720
actcggcctt cctgctctgc aggagccagc acgtccacag ccatgggccg ttccccacct 780
ccccagacct catcatcagc tccacccaac acttcctcaa ctgaggtaca ctcttcctgg 840
tccccttttg attcattttc ttcaacccaa aatgtaggaa tctgatttca tcttctactg 900
aaaaatgaca tcaatcatca gccagtaaat caaatgtata gactgagaat taactgcatt 960
ttaatctttt gcttccacag gcatatttga tgaacttgac attatctctg actgcaggaa 1020
gttttctgtc ctgtgctgtt tggggaagag acagagaact gcggaatctg gaactttcag 1080
caacagactc actgtctact gcccccatct attatacacc cattcccttt gctcactaat 1140
ttgttcaagt ttctctgaca tacaccatgc tcctttttcc tttaggattt tcacacacca 1200
tatttctttc acctttaaac tcttacctgg ccaaccctat ccaccctctg gatcccaata 1260
ttgagatctt atcctcaggg aatcctcact tagacccctg taacaggtta aatcttcatg 1320
gtgttctgtt tcctaggaac ttctttcttt tctactgttt atgacaactg aagttaataa 1380
gtgtttatct ttcccaccta ctcaaagtag ttccaagatt agggctagtt tgtaattctg 1440
tggaccactg taaacgaggg cctagttcag tgtctgcctc atgggaagct tccaataaat 1500
acctttgctc aacgaaaaaa tgaaaaccca gtggctcacg cctgtaatcc cagcactttg 1560
ggaggccgag gcaggtggat tgcctgaggt caggagtttg agactagtct ggccaacatg 1620
gtgaaacact atctctacta aaaatacaaa aaaattagct gggtatggtg gcttacgcct 1680
ataatcccag ctactcagga ggctgaggca ggggaattgc ttgaaccagg gaggtggagg 1740
ttgcagtgag ctgagatcgc accactgcac tccagcctgg gtgacagagc gagactccat 1800
ctcaaaataa aaaagtaaaa aaaaaaaaaa aaaa 1834

<210> 27
<211> 1695
<212> DNA
<213> Homo sapiens

<400> 27
gaggcacaga taaagataag ttttactgtc atgctgcttt taacataaca gagcaacatc 60
acctaggaaa aaagtttgta ggaggatttt taatccatat atttgtctta tggctagata 120
aagatttctc tgaaaaaaag aagcatgtca ggaatctctg ggtgcccctt tttcctctgg 180
ggacttctag cattgttggg cttggctttg gttatatcac tgatcttcaa tatttcccac 240
tatgtggaaa agcaacgaca agataaaatg tacagctact ccagtgacca caccagggtt 300
gatgagtatt atattgaaga cacaccaatt tatggtaact tagatgatat gatttcagaa 360
ccaatggatg aaaattgcta tgaacaaatg aaagcccgac cagagaaatc tgtaaataag 420
atgcaggaag ccaccccatc tgcacaggca accaatgaaa cacagatgtg ctacgcctca 480
cttgatcaca gcgttaaggg gaagcgtaga aagcccagga aacagaatac tcatttctca 540
gacaaggatg gagatgagca actacatgca atagatgcca gcgtttctaa gaccacctta 600
gtagacagtt tctccccaga aagccaggca gtagaggaaa acattcatga tgatcccatc 660
agactgtttg gattgatccg tgctaagaga gaacctataa actagctgga ccatgatcta 720
gttcaatgat ttggctccta ttgaagatgg cttctaagaa aacaagatgc acagaggaca 780
cagaaggact tggcagcagg gtgatgacct gatcatttgt tgatgggatg gtggcttacc 840
tcttattcac agcttacact tatgcatgcc aaatgtaagg ccatgaaaat cagtatttca 900
aataacttaa aaaatgcttt actactaaaa tgtaaaaaat taatgtgctc acctcggcag 960
cacatatact aaaaattaat aagacccagc ttgaaaattg agcctgataa caagattaca 1020
aattcacaat acctaatact tagggaaata taaaaattta agcatgaatg cgttctggaa 1080
cacgttagaa gaaaaataaa agccaatgag ttttttttta attctccttt ctcaccaatg 1140
ggcaatagcc cataattgaa ataaatttct gattgaaagg tataggaaac attaaaatgc 1200
attactaaga gaagtaatat aattttctta caaagtattt ttcccaaaga tagctttact 1260
atttcaaaaa ttgtcaaatt aatgcatgct ccttacaaca aacaaatatc aaaaagagtt 1320
taggaattct actagccaga gatagtcact tggagaaact ttctatatat ccttctaaat 1380
atttttctgg gcatgcttat gtatgtacat cagttgtttc tttttatttt gaaccaaaaa 1440
tgtggtttct tttgtacaca ttacttaaac tttctttcca gtcaacaata tattgtggat 1500
ttattttcac tgttatattt aactatatat aaatacgcat atattgtaat tttaatgtct 1560
gcttagcacc ccactgataa ccaaatcaca gtttatttaa ataattttaa tgacttttca 1620
aaaacaattt attgatgcaa aaagcaaggt tgagatgaca atgtttcttt caataattaa 1680

aaaatactgc ttcac 1695

<210> 28
<211> 2672
<212> DNA
<213> Homo sapiens

<400> 28
cttccagaga gcaatatggc tggttcccca acatgcctca ccctcatcta tatcctttgg 60
cagctcacag ggtcagcagc ctctggaccc gtgaaagagc tggtcggttc cgttggtggg 120
gccgtgactt tccccctgaa gtccaaagta aagcaagttg actctattgt ctggaccttc 180
aacacaaccc ctcttgtcac catacagcca gaagggggca ctatcatagt gacccaaaat 240
cgtaataggg agagagtaga cttcccagat ggaggctact ccctgaagct cagcaaactg 300
aagaagaatg actcagggat ctactatgtg gggatataca gctcatcact ccagcagccc 360
tccacccagg agtacgtgct gcatgtctac gagcacctgt caaagcctaa agtcaccatg 420
ggtctgcaga gcaataagaa tggcacctgt gtgaccaatc tgacatgctg catggaacat 480
ggggaagagg atgtgattta tacctggaag gccctggggc aagcagccaa tgagtcccat 540
aatgggtcca tcctccccat ctcctggaga tggggagaaa gtgatatgac cttcatctgc 600
gttgccagga accctgtcag cagaaacttc tcaagcccca tccttgccag gaagctctgt 660
gaaggtgctg ctgatgaccc agattcctcc atggtcctcc tgtgtctcct gttggtgccc 720
ctcctgctca gtctctttgt actggggcta tttctttggt ttctgaagag agagagacaa 780
gaagagtaca ttgaagagaa gaagagagtg gacatttgtc gggaaactcc taacatatgc 840
ccccattctg gagagaacac agagtacgac acaatccctc acactaatag aacaatccta 900
aaggaagatc cagcaaatac ggtttactcc actgtggaaa taccgaaaaa gatggaaaat 960
ccccactcac tgctcacgat gccagacaca ccaaggctat ttgcctatga gaatgttatc 1020
tagacagcag tgcactcccc taagtctctg ctcaaaaaaa aaacaattct cggcccaaag 1080
aaaacaatca gaagaattca ctgatttgac tagaaacatc aaggaagaat gaagaacgtt 1140
gacttttttc caggataaat tatctctgat gcttctttag atttaagagt tcataattcc 1200
atccactgct gagaaatctc ctcaaaccca gaaggtttaa tcacttcatc ccaaaaatgg 1260
gattgtgaat gtcagcaaac cataaaaaaa gtgcttagaa gtattcctat agaaatgtaa 1320
atgcaaggtc acacatatta atgacagcct gttgtattaa tgatggctcc aggtcagtgt 1380
ctggagtttc attccatccc agggcttgga tgtaaggatt ataccaagag tcttgctacc 1440
aggagggcaa gaagaccaaa acagacagac aagtccagca gaagcagatg cacctgacaa 1500
aaatggatgt attaattggc tctataaact atgtgcccag cactatgctg agcttacact 1560
aattggtcag acgtgctgtc tgccctcatg aaattggctc caaatgaatg aactactttc 1620
atgagcagtt gtagcaggcc tgaccacaga ttcccagagg gccaggtgtg gatccacagg 1680
acttgaaggt caaagttcac aaagatgaag aatcagggta gctgaccatg tttggcagat 1740
actataatgg agacacagaa gtgtgcatgg cccaaggaca aggacctcca gccaggcttc 1800
atttatgcac ttgtgctgca aaagaaaagt ctaggtttta aggctgtgcc agaacccatc 1860
ccaataaaga gaccgagtct gaagtcacat tgtaaatcta gtgtaggaga cttggagtca 1920
ggcagtgaga ctggtggggc acggggggca gtgggtactt gtaaaccttt aaagatggtt 1980
aattcattca atagatattt attaagaacc tatgcggccc ggcatggtgg ctcacacctg 2040
taatcccagc actttgggag gccaaggtgg gtgggtcatc tgaggtcagg agttcaagac 2100
cagcctggcc aacatggtga aaccccatct ctactaaaga tacaaaaatt tgctgagcgt 2160
ggtggtgtgc acctgtaatc ccagctactc gagaggccaa ggcatgagaa tcgcttgaac 2220
ctgggaggtg gaggttgcag tgagctgaga tggcaccact gcactccggc ctaggcaacg 2280
agagcaaaac tccaatacaa acaaacaaac aaacacctgt gctaggtcag tctggcacgt 2340
aagatgaaca tccctaccaa cacagagctc accatctctt atacttaagt gaaaaacatg 2400
ggaaggggga aggggaatgg gctgcttttg atatgttccc tgacacatat cttgaatgga 2460
gacctcccta ccaagtgatg aaagtgttga aaaacttaat aacaaatgct tgttgggcaa 2520
gaatgggatt gaggattatc ttctctcaga aaggcattgt gaaggaattg agccagatct 2580
ctctccctac tgcaaaaccc tattgtagta aaaaagtctt ctttactatc ttaataaaac 2640
agatattgtg agattcaaaa aaaaaaaaaa aa 2672

<210> 29
<211> 539
<212> DNA
<213> Homo sapiens

<400> 29
taaaacagct acaatattcc agggccagtc acttgccatt tctcataaca gcgtcagaga 60
gaaagaactg actgaaacgt ttgagatgaa gaaagttctc ctcctgatca cagccatctt 120
ggcagtggct gttggtttcc cagtctctca agaccaggaa cgagaaaaaa gaagtatcag 180
tgacagcgat gaattagctt cagggttttt tgtgttccct tacccatatc catttcgccc 240
acttccacca attccatttc caagatttcc atggtttaga cgtaattttc ctattccaat 300
acctgaatct gcccctacaa ctccccttcc tagcgaaaag taaacaagaa ggaaaagtca 360
cgataaacct ggtcacctga aattgaaatt gagccacttc cttgaagaat caaaattcct 420
gttaataaaa gaaaaacaaa tgtaattgaa atagcacaca gcattctcta gtcaatatct 480
ttagtgatct tctttaataa acttgaaagc aaagattttg gtttcttaat ttccacaaa 539

<210> 30
<211> 1542
<212> DNA
<213> Homo sapiens

<400> 30
acaattactc tacagctcag aacaccaact gctgaggctg ccttgggaag aggatgatcc 60
taaacaaagc tctgctgctg ggggccctcg ctctgaccac cgtgatgagc ccctgtggag 120
gtgaagacat tgtggctgac cacgttgcct cttgtggtgt aaacttgtac cagttttacg 180
gtccctctgg ccagtacacc catgaatttg atggagatga gcagttctac gtggacctgg 240
agaggaagga gactgcctgg cggtggcctg agttcagcaa atttggaggt tttgacccgc 300
agggtgcact gagaaacatg gctgtggcaa aacacaactt gaacatcatg attaaacgct 360
acaactctac cgctgctacc aatgaggttc ctgaggtcac agtgttttcc aagtctcccg 420
tgacactggg tcagcccaac accctcattt gtcttgtgga caacatcttt cctcctgtgg 480
tcaacatcac atggctgagc aatgggcagt cagtcacaga aggtgtttct gagaccagct 540
tcctctccaa gagtgatcat tccttcttca agatcagtta cctcaccttc ctcccttctg 600
ctgatgagat ttatgactgc aaggtggagc actggggcct ggaccagcct cttctgaaac 660
actgggagcc tgagattcca gcccctatgt cagagctcac agagactgtg gtctgtgccc 720
tggggttgtc tgtgggcctc atgggcattg tggtgggcac tgtcttcatc atccaaggcc 780
tgcgttcagt tggtgcttcc agacaccaag ggccattgtg aatcccatcc tggaagggaa 840
ggtgcatcgc catctacagg agcagaagaa tggacttgct aaatgaccta gcactattct 900
ctggcccgat ttatcatatc ccttttctcc tccaaatatt tctcctctca ccttttctct 960
gggacttaag ctgctatatc ccctcagagc tcacaaatgc ctttacattc tttccctgac 1020
ctcctgattt tttttttctt ttctcaaatg ttacctacaa agacatgcct ggggtaagcc 1080
acccggctac ctaattcctc agtaacctcc atctaaaatc tccaaggaag caataaattc 1140
cttttatgag atctatgtca aatttttcca tctttcatcc agggctgact gaaactatgg 1200
ctaataattg gggtactctt atgtttcaat ccaatttaac ctcatttccc agatcatttt 1260
tcatgtccag taacacagaa gccaccaagt acagtatagc ctgataatat gttgatttct 1320
tagctgacat taatatttct tgcttccttg tgttcccacc cttggcactg ccacccaccc 1380
ctcaattcag gcaacaatga aattaatgga taccgtctgc ccttggccca gaattgttat 1440
agcaaaaatt ttagaaccaa aaaataagtc tgtactaatt tcaatgtggc ttttaaaagt 1500
atgacagaga aataagttag gataaaggaa atttgaatct ca 1542

<210> 31
<211> 399
<212> DNA
<213> Homo sapiens

<400> 31
agcggccgcc cttttttttt ttttttgaat gtttttatat ttttatgttt ttctctctca 60
ccacattaga gattcacatg tcaaaaaaaa agttcaaaga ttccacttaa atggtagtca 120
aaattaaaag catgttgttt aatttgccaa ctattccata aatcctgtgc ttgaagcact 180
accttttatc aaccacttga aaggaaggaa tatgaagtgt gactcttgag gattgcagtt 240
ccatttctcc acagacatag cagggtgtgt agttccaaat gggtttcttc caaagtcatc 300
tcctcagagt aggactgtct gagtagtcat tctgataatg ttgccattaa ctacaagcac 360
atctgagaac cctcgtaagg aagtgccatt agattcaga 399

<210> 32
<211> 426
<212> DNA
<213> Homo sapiens

<400> 32
ggcccagccc taggaggggg cagtcccaca gcaggctcac accctgccct tcagagttgc 60
agcagctaat gctgatgcca ggaccttgtc ctgccaggaa gcctctgatt agggctgtga 120
accaacttcc ttttcacatc tgcaaggact ctgctctaag ctcaaatttt gcagtttctg 180
ctgtcatcac agctgggaat gagaaaggga aggggctcca gttggtctcc ctctgcctct 240
ggagcacacg tggaggacat gaggcttccg caggaacagc tctgaaagct gagttctcgg 300
gtccctgtga aggagggaag cttgcctgcc ttggagcgtg aaaaagtctt gctggacaga 360
agtctcacgg tttaaaactg ggaagggaga ggattgagat cacactagaa tgcacagaaa 420
attctt 426

<210> 33
<211> 649
<212> DNA
<213> Homo sapiens

<400> 33
atggttagta atttcttcca tgtcatacaa gtattcgaga aatctgctac cttgattagt 60
aagactgaac acattggttt tgtcatttat tcatggagga agtccaccac ccacttgggg 120
agcagaagga aatttgccat ctcaatttac ttatcagaag tttctttgca gaaatatgat 180
tgtcccttca gtgggacatc atttgtggtc ttctctctct ttttgatctg tgcaatggct 240
ggagatgtag tctacgctga catcaaaact gttcggactt ccccgttaga actcgcgttt 300
ccacttcaga gatctgtttc tttcaacttt tctactgtcc ataaatcatg tcctgccaaa 360
gactggaagg tgcataaggg aaaatgttac tggattgctg aaactaagaa atcttggaac 420
aaaagtcaaa atgactgtgc cataaacaat tcatatctca tggtgattca agacattact 480
gctatggtga gatttaacat ttagaggtga cagcatcccc cacactggca gtgaattttt 540
tgtgctacaa acttggcaaa agtctgtgaa aagaagtttc aacttcatgt gttattaact 600
atacaaatat tagttgaatg aattgttgaa ttacaaaaaa aaaaaaaaa 649

<210> 34
<211> 1353
<212> DNA
<213> Artifical Sequence

<220>
<223> Nucleotide sequence encoding fusion protein of Lipoprotein D
fragment, Mage3 fragment, and histidine tail

<400> 34
atggatccaa aaactttagc cctttcttta ttagcagctg gcgtactagc aggttgtagc 60
agccattcat caaatatggc gaatacccaa atgaaatcag acaaaatcat tattgctcac 120
cgtggtgcta gcggttattt accagagcat acgttagaat ctaaagcact tgcgtttgca 180
caacaggctg attatttaga gcaagattta gcaatgacta aggatggtcg tttagtggtt 240
attcacgatc actttttaga tggcttgact gatgttgcga aaaaattccc acatcgtcat 300
cgtaaagatg gccgttacta tgtcatcgac tttaccttaa aagaaattca aagtttagaa 360
atgacagaaa actttgaaac catggatctg gaacagcgta gtcagcactg caagcctgaa 420
gaaggccttg aggcccgagg agaggccctg ggcctggtgg gtgcgcaggc tcctgctact 480
gaggagcagg aggctgcctc ctcctcttct actctagttg aagtcaccct gggggaggtg 540
cctgctgccg agtcaccaga tcctccccag agtcctcagg gagcctccag cctccccact 600
accatgaact accctctctg gagccaatcc tatgaggact ccagcaacca agaagaggag 660
gggccaagca ccttccctga cctggagtcc gagttccaag cagcactcag taggaaggtg 720
gccgaattgg ttcattttct gctcctcaag tatcgagcca gggagccggt cacaaaggca 780
gaaatgctgg ggagtgtcgt cggaaattgg cagtatttct ttcctgtgat cttcagcaaa 840

gcttccagtt ccttgcagct ggtctttggc atcgagctga tggaagtgga ccccatcggc 900
cacttgtaca tctttgccac ctgcctgggc ctctcctacg atggcctgct gggtgacaat 960
cagatcatgc ccaaggcagg cctcctgata atcgtcctgg ccataatcgc aagagagggc 1020
gactgtgccc ctgaggagaa aatctgggag gagctgagtg tgttagaggt gtttgagggg 1080
agggaagaca gtatcttggg ggatcccaag aagctgctca cccaacattt cgtgcaggaa 1140
aactacctgg agtaccggca ggtccccggc agtgatcctg catgttatga attcctgtgg 1200
ggtccaaggg ccctcgttga aaccagctat gtgaaagtcc tgcaccatat ggtaaagatc 1260
agtggaggac ctcacatttc ctacccaccc ctgcatgagt gggttttgag agagggggaa 1320
gagggcggtc atcaccatca ccatcaccat taa 1353

<210> 35
<211> 450
<212> PRT
<213> Artificial Sequence

<220>
<223> Amino acid sequence of the fusion protein of Lipoprotein D
fragment, Mage3 fragment and histidine tails

<400> 35
Met Asp Pro Lys Thr Leu Ala Leu Ser Leu Leu Ala Ala Gly Val Leu
1               5                   10                  15
Ala Gly Cys Ser Ser His Ser Ser Asn Met Ala Asn Thr Gln Met Lys
            20                  25                  30
Ser Asp Lys Ile Ile Ile Ala His Arg Gly Ala Ser Gly Tyr Leu Pro
        35                  40                  45
Glu His Thr Leu Glu Ser Lys Ala Leu Ala Phe Ala Gln Gln Ala Asp
    50                  55                  60
Tyr Leu Glu Gln Asp Leu Ala Met Thr Lys Asp Gly Arg Leu Val Val
65                  70                  75                  80
Ile His Asp His Phe Leu Asp Gly Leu Thr Asp Val Ala Lys Lys Phe
                85                  90                  95
Pro His Arg His Arg Lys Asp Gly Arg Tyr Tyr Val Ile Asp Phe Thr
            100                 105                 110
Leu Lys Glu Ile Gln Ser Leu Glu Met Thr Glu Asn Phe Glu Thr Met
        115                 120                 125
Asp Leu Glu Gln Arg Ser Gln His Cys Lys Pro Glu Glu Gly Leu Glu
    130                 135                 140
Ala Arg Gly Glu Ala Leu Gly Leu Val Gly Ala Gln Ala Pro Ala Thr
145                 150                 155                 160
Glu Glu Gln Glu Ala Ala Ser Ser Ser Ser Thr Leu Val Glu Val Thr
                165                 170                 175
Leu Gly Glu Val Pro Ala Ala Glu Ser Pro Asp Pro Pro Gln Ser Pro
            180                 185                 190
Gln Gly Ala Ser Ser Leu Pro Thr Thr Met Asn Tyr Pro Leu Trp Ser
        195                 200                 205
Gln Ser Tyr Glu Asp Ser Ser Asn Gln Glu Glu Glu Gly Pro Ser Thr
    210                 215                 220
Phe Pro Asp Leu Glu Ser Glu Phe Gln Ala Ala Leu Ser Arg Lys Val
225                 230                 235                 240
Ala Glu Leu Val His Phe Leu Leu Leu Lys Tyr Arg Ala Arg Glu Pro
                245                 250                 255
Val Thr Lys Ala Glu Met Leu Gly Ser Val Val Gly Asn Trp Gln Tyr
            260                 265                 270
Phe Phe Pro Val Ile Phe Ser Lys Ala Ser Ser Ser Leu Gln Leu Val
        275                 280                 285
Phe Gly Ile Glu Leu Met Glu Val Asp Pro Ile Gly His Leu Tyr Ile

290                 295                 300
Phe Ala Thr Cys Leu Gly Leu Ser Tyr Asp Gly Leu Leu Gly Asp Asn
305                 310                 315                 320
Gln Ile Met Pro Lys Ala Gly Leu Leu Ile Ile Val Leu Ala Ile Ile
                325                 330                 335
Ala Arg Glu Gly Asp Cys Ala Pro Glu Glu Lys Ile Trp Glu Glu Leu
            340                 345                 350
Ser Val Leu Glu Val Phe Glu Gly Arg Glu Asp Ser Ile Leu Gly Asp
        355                 360                 365
Pro Lys Lys Leu Leu Thr Gln His Phe Val Gln Glu Asn Tyr Leu Glu
    370                 375                 380
Tyr Arg Gln Val Pro Gly Ser Asp Pro Ala Cys Tyr Glu Phe Leu Trp
385                 390                 395                 400
Gly Pro Arg Ala Leu Val Glu Thr Ser Tyr Val Lys Val Leu His His
                405                 410                 415
Met Val Lys Ile Ser Gly Gly Pro His Ile Ser Tyr Pro Pro Leu His
            420                 425                 430
Glu Trp Val Leu Arg Glu Gly Glu Glu Gly Gly His His His His His
        435                 440                 445
His His
    450

<210> 36
<211> 9
<212> PRT
<213> Homo sapiens

<400> 36
Glu Val Asp Pro Ile Gly His Leu Tyr
1               5

<210> 37
<211> 9
<212> PRT
<213> Homo sapiens

<400> 37
Phe Leu Trp Gly Pro Arg Ala Leu Val
1               5

<210> 38
<211> 10
<212> PRT
<213> Homo sapiens

<400> 38
Met Glu Val Asp Pro Ile Gly His Leu Tyr
1               5                   10

<210> 39
<211> 10
<212> PRT
<213> Homo sapiens

<400> 39
Val His Phe Leu Leu Leu Lys Tyr Arg Ala
1               5                   10


<210> 40
<211> 10
<212> PRT
<213> Homo sapiens

<400> 40
Leu Val His Phe Leu Leu Leu Lys Tyr Arg
1               5                   10


<210> 41
<211> 10
<212> PRT
<213> Homo sapiens

<400> 41
Leu Lys Tyr Arg Ala Arg Glu Pro Val Thr
1               5                   10


<210> 42
<211> 16
<212> PRT
<213> Homo sapiens

<400> 42
Ala Cys Tyr Glu Phe Leu Trp Gly Pro Arg Ala Leu Val Glu Thr Ser
1               5                   10                  15


<210> 43
<211> 12
<212> PRT
<213> Homo sapiens

<400> 43
Thr Gln His Phe Val Gln Glu Asn Tyr Leu Glu Tyr
1               5                   10


<210> 44
<211> 20
<212> DNA
<213> Artificial Sequence

<220>
<223> CpG containing oligonucleotide CpG 1826

<400> 44
tccatgacgt tcctgacgtt                                                   20

<210> 45
<211> 18

<212> DNA
<213> Artificial Sequence

<220>
<223> CpG containing oligonucleotide CpG 1758

<400> 45
tctcccagcg tgcgccat                                                    18

<210> 46
<211> 30
<212> DNA
<213> Artificial Sequence

<220>
<223> CpG containing oligonucleotide

<400> 46
accgatgacg tcgccggtga cggcaccacg                                       30

<210> 47
<211> 24
<212> DNA
<213> Artificial Sequence

<220>
<223> CpG containing oligonucleotide CpG 2006/7909

<400> 47
tcgtcgtttt gtcgttttgt cgtt                                             24

<210> 48
<211> 20
<212> DNA
<213> Artificial Sequence

<220>
<223> CpG containing oligonucleotide CpG 1668

<400> 48
tccatgacgt tcctgatgct                                                  20

<210> 49
<211> 347
<212> DNA
<213> Homo sapiens

<400> 49
ttctaagacc cacatttggt tattgaaggc cacagcgaat cttaacctaa cagccttgac 60
aaactgcacc ataggtgttt ttagactcat ataatttgtt atttttcaaa caatagtgaa 120
taattaatat tnttgtttgg aatttgagna caattaaatt tgtactttta gtaactacca 180
ttctttgatt agaaaattaa gagaatgcat atcttacttt ggttgtaaat tatcaagggc 240
tttctaatag aaatcatata taacatttct aaatataagt cctttcacat actgtgtttc 300
cagttgtctt gatattgaaa agtgtaataa acttcatgct cacctat                   347

<210> 50
<211> 388
<212> DNA

<213> Homo sapiens

<400> 50
ttgctggctt ctcttatact aacccagagt ttgtcattaa tgtgtaggtg aatgcaaact 60
ccatcgttga gcctggggtg taagacttca agccaagcgt atgtatcaat tctagtcttc 120
caggattcac ggtgcacatg ctggcattca acatgtggaa agcttgtctt agaggccttt 180
cttgtatgtg tagcttgcta gtttgttttc tacatttgaa aatgtttagt ttagaataag 240
cgcattatcc aattatagag gtacaatttt ccaaacttcc agaaactcat caaatgaaca 300
gacaatgtca aaactactgt gtctgatacc aaaatgcttc agtatttgta atttttcaag 360
tcagaagctg atgttcctgg taaaagtt 388

<210> 51
<211> 411
<212> DNA
<213> Homo sapiens

<400> 51
ttctggctgg aacggacgat gccccgaatt cccaccctga agaacctaga ggatcttgtt 60
actgaatacc acgggaactt ttcggcctgg agtggtgtgt ctaagggact ggctgagagt 120
ctgcagccag actacagtga acgactctgc ctcgtcagtg agattccccc aaaaggaggg 180
gcccttgggg aggggcctgg ggcctcccca tgcaaccagc atagccccta ctgggccccc 240
ccatgttaca ccctaaagcc tgaaacctga accccaatcc tctgacagaa gaaccccagg 300
gtcctgtagc cctaagtggt actaactttc cttcattcaa cccacctgcg tctcatactc 360
acctcacccc actgtggctg atttggaatt ttgtgccccc atgtaagcac c 411

<210> 52
<211> 388
<212> DNA
<213> Homo sapiens

<400> 52
   acagccacga agatcctacc aaaatgaagc gcttcctctt cctcctactc accatcagcc 60
   tcctggttat ggtacagata caaactggac tctcaggaca aaacgacacc agccaaacca 120
   gcagcccctc agcatccagc agcatgagcg gaggcatttt ccttttcttc gtggccaatg 180
   ccataatcca cctcttctgc ttcagttgag gtgacacgtc tcagccttag ccctgtgccc 240
   cctgaaacag ctgccaccat cactcgcaag agaatcccct ccatctttgg gaggggttga 300
   tgccagacat caccaggttg tagaagttga caggcagtgc catgggggca acagccaaaa 360
   taggggggta atgatgtagg ggccaagc 388

<210> 53
<211> 428
<212> DNA
<213> Homo sapiens

<400> 53
agacctcgag ttcagccaaa acctccccat ggggcagcag aaaactcatt gtccccttcc 60
tctaattaaa aaagatagaa actgtctttt tcaataaaaa gcactgtgga tttctgccct 120
cctgatgtgc atatccgtac ttccatgagg tgttttctgt gtgcagaaca ttgtcacctc 180
ctgaggctgt gggccacagc cacctctgca tcttcgaact cagccatgtg gtcaacatct 240
ggagtttttg gtctcctcag agagctccat cacaccagta aggagaagca atataagtgt 300
gattgcaaga atggtagagg accgagcaca gaaatcttag agatttcttg tcccctctca 360
ggtcatgtgt agatgcgata aatcaagtga ttggtgtgcc tgggtctcac tacaagcagc 420
ctatctgc 428

<210> 54
<211> 508
<212> DNA
<213> Homo sapiens

<400> 54
gatcttaaag ccacggagaa gcctctcatc ttatggcatt gacaaagaga agaccatcca 60
ccttaccctg aaagtggtga agcccagtga tgaggagctg cccttgtttc ttgtggagtc 120
aggtgatgag gcaaagaggc acctcctcca ggtgcgaagg tccagctcag tggcacaagt 180
gaaagcaatg atcgagacta agacgggtat aatccctgag acccagattg tgacttgcaa 240
tggaaagaga ctggaagatg ggaagatgat ggcagattac ggcatcagaa agggcaactt 300
actcttcctg gcatcttatt gtattggagg gtgaccaccc tggggatggg gtgttggcag 360
gggtcaaaaa gcttatttct tttaatctct tactcaacga acacatcttc tgatgatttc 420
ccaaaattaa tgagaatgag atgagtagag taagatttgg gtgggatggg taggatgaag 480
tatattgccc aactctatgt ttctttga 508

<210> 55
<211> 501
<212> DNA
<213> Homo sapiens

<400> 55
gctgacatcc tgcgagacta caaagttatt atggctgaaa acattcctga aaaccctctg 60
aagtacctat atcctgacat tcccaaagac aaagccttcg gtaaacacta cagctctcag 120
ccttgcgaag tttcaagacc aacagaaagg ggtgacaaag gttatgttcc ttctgttttt 180
atccccatct caacaatccg aagtgattca acagagccac attctccatc agaccttctt 240
cccatgtctc caagtgtgta tgcggtgttg agagaaaacc tgagtcccac aacaattgaa 300
actgcaatga agtctcctta ttctgctgaa tgacaggata aactctgacg caccaagaaa 360
ggaagcaaat gaaaaagttt aaagactgtt ctttgcccaa taaccacatt ttatttcttc 420
agctttgtaa ataccaggtt ctaggaaatg tttgacatct gaagctctct tcacactccc 480
gtggcactcc tcaattggga g 501

<210> 56
<211> 489
<212> DNA
<213> Homo sapiens

<400> 56
aaacctctct tagatctgga accaaatgca aggttactgg ctggggagcc accgatccag 60
attcattaag accttctgac accctgcgag aagtcactgt tactgtccta agtcgaaaac 120
tttgcaacag ccaaagttac tacaacggcg acccttttat caccaaagac atggtctgtg 180
caggagatgc caaaggccag aaggattcct gtaagggtga ctcagggggc cccttgatct 240
gtaaaggtgt cttccacgct atagtctctg gaggtcatga atgtggtgtt gccacaaagc 300
ctggaatcta caccctgtta accaagaaat accagacttg gatcaaaagc aaccttgtcc 360
cgcctcatac aaattaagtt acaaataatt ttattggatg cacttgcttc tttttcccta 420
atatgctcgc aggttagagt tgggtgtaag taaagcagag cacatatggg gtccattttt 480
gcacttgta 489

<210> 57
<211> 453
<212> DNA
<213> Homo sapiens

<400> 57
gtaatgccaa ggaccctcga gggatgtatc agtgtaaagg atcacagaac aagtcaaaac 60
cactccaagt gtattacaga atgtgtcaga actgcattga actaaatgca gccaccatat 120
ctggctttct ctttgctgaa atcgtcagca ttttcgtcct tgctgttggg gtctacttca 180
ttgctggaca ggatggagtt cgccagtcga gagcttcaga caagcagact ctgttgccca 240
atgaccagct ctaccagccc ctcaaggatc gagaagatga ccagtacagc caccttcaag 300
gaaaccagtt gaggaggaat tgaactcagg actcagagta gtccaggtgt tctcctccta 360
ttcagttccc agaatcaaag caatgcattt tggaaagctc ctagcagaga gactttcagc 420
cctaaatcta gactcaaggt tcccagagat gac 453

<210> 58
<211> 405
<212> DNA
<213> Homo sapiens

<400> 58
ttgccttgta attcgacagc tctacagaaa caaagataat gaaaattacc caaatgtgaa 60
aaaggctctc atcaacatac ttttagtgac cacgggctac atcatatgct ttgttcctta 120
ccacattgtc cgaatcccgt ataccctcag ccagacagaa gtcataactg attgctcaac 180
caggatttca ctcttcaaag ccaaagaggc tacactgctc ctggctgtgt cgaacctgtg 240
ctttgatcct atcctgtact atcacctctc aaaagcattc cgctcaaagg tcactgagac 300
ttttgcctca cctaaagaga ccaaggctca gaaagaaaaa ttaagatgtg aaaataatgc 360
ataaaagaca ggattttttg tgctaccaat tctggcctta ctgga 405

<210> 59
<211> 542
<212> DNA
<213> Homo sapiens

<400> 59
tgatgagctt tcctttgatc cggacgacgt aatcactgac attgagatgg tggacgaggg 60
ctggtggcgg ggacgttgcc atggccactt tggactcttc cctgcaaatt atgtcaagct 120
tctggagtga ctagagctca ctgtctactg caactgtgat ttcccatgtc caaagtggct 180
ctgctccacc ccctccctat tcctgatgca aatgtctaac cagatgagtt tctggacaga 240
cttccctctc ctgcttcatt aagggcttgg ggcagagaca gcatggggaa ggaggtcccc 300
ttccccaaga gtcctctcta tcctggatga gctcatgaac atttctcttg tgttcctgac 360
tccttcccaa tgaacacctc tctgccaccc caagctctgc tctcctcctc tgtgagctct 420
gggcttccca gtttgtttac ccgggaaagt acgtctagat tgtgtggttt gcctcattgt 480
gctatttgcc cactttcctt ccctgaagaa atatctgtga accttctttc tgttcagtcc 540
ta 542

<210> 60
<211> 101
<212> DNA
<213> Homo sapiens

<400> 60
cgatcaccaa tgtacctcca gaggtaactg tgctcacgaa cagccctgtg gaactgagag 60
agcccaacgt cctcatctgt ttcatagaca agttcacccc a 101

<210> 61
<211> 342
<212> DNA
<213> Homo sapiens

<400> 61
aaaaaaggaa atcgctgcag aatgaaggaa tatcccttga ggtgacccag ccaacctgtg 60
gccagaagga gggttgtacc ttgaaaagac cactgaaagc attttggggt gtcaagtaag 120
ggtgggcaga ggaggtagaa aatcaattca attgtcgcat cattcatggt tctttaatat 180
tgatgctcag tgcattggcc ttagaatatc ccagcctctc ttctggtttg gtgagtgctg 240
tgtaagtaag catggtagaa ttgtttggag acatatatag tgatccttgg tcactggtgt 300
ttcaaacatt ctggaaagtc acatcgatca agaatatttt tt 342

<210> 62
<211> 555
<212> DNA
<213> Homo sapiens

<400> 62
gcagatttct tgcttcatat gacaaagcct caattactaa ttgtaaaaac tgaactattc 60
ccagaatcat gttcaaaaaa tctgtaattt ttgctgatcg aaagtgcttc attgactaaa 120
cagtattagt ttgtggctat aaatgattat ttagatgatg actgaaaatg tgtataaagt 180
aattaaaagt aatatggtgg ctttaagtgt agagatggga tggcaaatgc tgtgaatgca 240
gaatgtaaaa ttggtaacta agaaatggca caaacacctt aagcaatata ttttcctagt 300
agatatatat atacacatac atatatacac atatacaaat gtatattttt gcaaaattgt 360
tttcaatcta gaacttttct attaactacc atgtcttaaa atcaagtcta taatcctagc 420
attagtttaa tattttgaat atgtaaacac ctgtgttaat gctttgttaa tgcttttccc 480
actctcattt gttaatgctt tcccactctc gggaaggatt tgcattttga gctttatctc 540
taaatgtgac atgca 555

<210> 63
<211> 372
<212> DNA
<213> Homo sapiens

<400> 63
caactggatc ctacccgaat ttatgattaa gattgctgaa gagctgccaa acactgctgc 60
caccccctct gttcccttat tgctgcttgt cactgcctga cattcacggc agaggcaagg 120
ctgctgcagc ctcccctggc tgtgcacatt ccctcctgct ccccagagac tgcctccgcc 180
atcccacaga tgatggatct tcagtgggtt ctcttgggct ctaggtcctg gagaatgttg 240
tgaggggttt attttttttt aatagtgttc ataaagaaat acatagtatt cttcttctca 300
agacgtgggg ggaaattatc tcattatcga ggccctgcta tgctgtgtgt ctgggcgtgt 360
tgtatgtcct gc 372

<210> 64
<211> 432
<212> DNA
<213> Homo sapiens

<400> 64
tagtctaatt gaatccctta aactcaggga gcatttataa atggcaaatg cttatgaaac 60
taagatttgt aatatttctc tctttttaga gaaatttgcc aatttacttt gttatttttc 120
cccaaaaaga atgggatgat cgtgtattta tttttttact tcctcagctg tagacaggtc 180
cttttcgatg gtacatattt ctttgccttt ataatctttt atacagtgtc ttacagagaa 240
aagacataag caaagactat gaggaatatt tgcaagacat agaatagtgt tggaaaatgt 300
gcaatatgtg atgtggcaaa tctctattag gaaatattct gtaatcttca gacctagaat 360
aatactagtc ttataatagg tttgtgactt tcctaaatca attctattac gtgcaatact 420
tcaatacttc at 432

<210> 65
<211> 352
<212> DNA
<213> Homo sapiens

<400> 65
aatccattca tcctgattgg gcatgaaatc catggtcaag aggacaagtg gaaagtgaga 60
gggaaggttt gctagacacc ttcgcttgtt atcttgtcaa gatagaaaag atagtatcat 120
ttcacccttg ccagtaaaaa cctttccatc cacccattct cagcagactc cagtattggc 180
acagtcactc actgccattc tcacactata acaagaaaag aaatgaagtg cataagtctc 240
ctgggaaaag aaccttaacc ccttctcgtg ccatgactgg tgatttcatg actcataagc 300
ccctccgtag gcatcattca agatcaatgg cccatgcatg ctgtttgcag ca 352

<210> 66
<211> 532
<212> DNA

<213> Homo sapiens

<400> 66
aaaggccaca ctggtgtgcc tggccacagg tatcttccct gaccacgtgg agctgagctg 60
gtgggtgaat gggaaggagg tgcacagtgg ggtcagcacg gacccgcagc ccctcaagga 120
gcagcccgcc ctcaatgact ccagatactg cctgagcagc cgcctgaggg tctcggccac 180
cttctggcag aacccccgca accacttccg ctgtcaagtc cagttctacg ggctctcgga 240
gaatgacgag tggacccagg atagggccaa acccgtcacc cagatcgtca gcgccgaggc 300
ctggggtaga gcagactgtg gctttacctc ggtgtcctac cagcaagggg tcctgtctgc 360
caccatcctc tatgagatcc tgctagggaa ggccaccatg tatgctgtgc tggtcagcgc 420
ccttgtgttg atggccatgg tcaagagaaa ggatttctga aggcagccct ggaagtggag 480
ttaggagctt ctaacccgtc atggtttcaa tacacattct tcttttgcca gc 532

<210> 67
<211> 484
<212> DNA
<213> Homo sapiens

<400> 67
ggaacaagac ttcaggtcac gctcgatatc cagaaccctg accctgccgt gtaccagctg 60
agagactcta aatccagtga caagtctgtc tgcctattca ccgattttga ttctcaaaca 120
aatgtgtcac aaagtaagga ttctgatgtg tatatcacag acaaaactgt gctagacatg 180
aggtctatgg acttcaagag caacagtgct gtggcctgga gcaacaaatc tgactttgca 240
tgtgcaaacg ccttcaacaa cagcattatt ccagaagaca ccttcttccc cagcccagaa 300
agttcctgtg atgtcaagct ggtcgagaaa agctttgaaa cagatacgaa cctaaacttt 360
caaaacctgt cagtgattgg gttccgaatc ctcctcctga aagtggccgg gtttaatctg 420
ctcatgacgc tgcggctgtg gtccagctga gatctgcaag attgtaagac agcctgtgct 480
ccct 484

<210> 68
<211> 512
<212> DNA
<213> Homo sapiens

<400> 68
aaatgataca ctactgctgc agctcacaaa cacctctgca tattacatgt acctcctcct 60
gctcctcaag agtgtggtct attttgccat catcacctgc tgtctgcttg gaagaacggc 120
tttctgctgc aatggagaga aatcataaca gacggtggca caaggaggcc atcttttcct 180
catcggttat tgtccctaga agcgtcttct gaggatctag ttgggctttc tttctgggtt 240
tgggccattt cagttctcat gtgtgtacta ttctatcatt attgtataat ggttttcaaa 300
ccagtgggca cacagagaac ctcagtctgt aataacaatg aggaatagcc atggcgatct 360
ccagcaccaa tctctccatg ttttccacag ctcctccagc caacccaaat agcgcctgct 420
atagtgtaga cagcctgcgg cttctagcct tgtccctctc ttagtgttct ttaatcagat 480
aactgcctgg aagcctttca ttttacacgc cc 512

<210> 69
<211> 483
<212> DNA
<213> Homo sapiens

<400> 69
gccatcagaa gcagagatct cccacaccca aaaggccaca ctggtgtgcc tggccacagg 60
tttctacccc gaccacgtgg agctgagctg gtgggtgaat gggaaggagg tgcacagtgg 120
ggtcagcaca gacccgcagc ccctcaagga gcagcccgcc ctcaatgact ccagatactg 180
cctgagcagc cgcctgaggg tctcggccac cttctggcag aacccccgca accacttccg 240
ctgtcaagtc cagttctacg ggctctcgga gaatgacgag tggacccagg atagggccaa 300
acctgtcacc cagatcgtca gcgccgaggc ctggggtaga gcagactgtg gcttcacctc 360
cgagtcttac cagcaagggg tcctgtctgc caccatcctc tatgagatct tgctagggaa 420

ggccaccttg tatgctgtgc tggtcagtgc cctcgtgctg atggccatgg tcaagagaaa 480
gga 483

<210> 70
<211> 592
<212> DNA
<213> Homo sapiens

<400> 70
gaatcgtttc tctgtgaact tccagaaagc agccaaatcc ttcagtctca agatctcaga 60
ctcacagctg ggggatgccg cgatgtattt ctgtgcttat aggagtgcat actctggggc 120
tgggagttac caactcactt tcgggaaggg gaccaaactc tcggtcatac caaatatcca 180
gaaccctgac cctgccgtgt accagctgag agactctaaa tccagtgaca agtctgtctg 240
cctattcacc gattttgatt ctcaaacaaa tgtgtcacaa agtaaggatt ctgatgtgta 300
tatcacagac aaaactgtgc tagacatgag gtctatggac ttcaagagca acagtgctgt 360
ggcctggagc aacaaatctg actttgcatg tgcaaacgcc ttcaacaaca gcattattcc 420
agaagacacc ttcttcccca gcccagaaag ttcctgtgat gtcaagctgg tcgagaaaag 480
ctttgaaaca gatacgaacc taaactttca aaacctgtca gtgattgggt tccgaatcct 540
cctcctgaaa gtggccgggt ttaatctgct catgacgctg cggttgtggt cc 592

<210> 71
<211> 358
<212> DNA
<213> Homo sapiens

<400> 71
cactgcagaa tgaaggaaca tcccttgagg tgacccagcc aacctgtggc cagaaggagg 60
nttgtacctt gaaaagacac tgaaagcatt ttggngtgtn aagtaagggt gggcagagga 120
ggtagaaaat caattcaatt gtcgcatcat tcatggttct ttaatattga tgctcagtgc 180
antggcctna gaatatccca gcctctcttc tggtttgntg agtgctntnt aagtaagcat 240
ggtngaattg tttggggnca natatagtga nccttggtca ctggtgtttc aaacattctg 300
gnaagtcaca tcnatcaaga atanttttta nttttaagaa agcataacca gcaataaa 358

<210> 72
<211> 519
<212> DNA
<213> Homo sapiens

<400> 72
tgactccaga tactgcctga gcagccgcct gagggtctcg gccaccttct ggcagaaccc 60
ccgcaaccac ttccgctgtc aagtccagtt ctacgggctc tcggagaatg acgagtggac 120
ccaggatagg gccaaacccg tcacccagat cgtcagcgcc gaggcctggg gtagagcaga 180
ctgtggcttt acctcggtgt cctaccagca aggggtcctg tctgccacca tcctctatga 240
gatcctgcta gggaaggcca ccctgtatgc tgtgctggtc agcncccttg tgttgatggc 300
catggtcaag agaaaggatt tctgaaggca gccctggaag tggagttagg agcttctaac 360
ccgtcatggt ttcaatacac attcttcttt tgccagcgct tctgaagagc tgctctcacc 420
tctctgcatc ccaatagata tccccctatg tgcatgcaca cctgcacact cacggctgaa 480
atctccctaa cccaggggga ccttagcatg cctaagtga 519

<210> 73
<211> 419
<212> DNA
<213> Homo sapiens

<400> 73
gggaacactg ctctcagaca ttacaagact ggacctggga aaacgcatcc tggacccacg 60
aggaatatat aggtgtaatg ggacagatat atacaaggac aaagaatcta ccgtgcaagt 120
tcattatcga atgtgccaga gctgtgtgga gctggatcca gccaccgtgg ctggcatcat 180

tgtcactgat gtcattgcca ctctgctcct tgctttggga gtcttctgct ttgctggaca 240
tgagactgga aggctgtctg gggctgccga cacacaagct ctgttgagga atgaccaggt 300
ctatcagccc ctccgagatc gagatgatgc tcagtacagc caccttggag gaaactgggc 360
tcggaacaag tgaacctgag actggtggct tctagaagca gccattacca actgtacct 419

<210> 74
<211> 532
<212> DNA
<213> Homo sapiens

<400> 74
aaatgataca ctactgctgc agctcacaaa cacctctgca tattacatgt acctcctcct 60
gctcctcaag agtgtggtct attttgccat catcacctgc tgtctgcntg naagaacggc 120
nnnctgctgc aatggagaga antcataaca gacggtggca caaggaggcc nncntntcct 180
catcggnnat tgtccctaga agcgtcttct gaggatctag ttgggctttc tttctgggtt 240
tgggccattt cagttctcat gtgtgtacta ttctatcatt attgtataat ggttttcaaa 300
ccagtgggca cacagagaac ctcagtctgt aataacaatg aggaatagcc atggcgatct 360
ccagcaccaa tctctccatg ttttccacag ctcctccagc caacccaaat agcgcctgct 420
atagtgtaga nannctgcgg cttctagcct tgtccctctc ttagtgttct ttaatcagat 480
aactgcctgg aagcctttca ttttacacgc cctgaagcag tcttctttgc ta 532

<210> 75
<211> 267
<212> DNA
<213> Homo sapiens

<400> 75
ccaccctctg gatcccaata ttgagatctt atcctcaggg aatcctcact tagacccctg 60
taacaggtta aatcttcatg gtgttctgtt tcctaggaac ttctttcttt tctactgttt 120
atgacaactg aagttaataa gtgtttatct ttcccaccta ctcaaagtag ttccaagatt 180
agggctagtt tgtaattctg tggaccactg taaacgaggg cctagttcag tgtctgcctc 240
atgggaagct tccaataaat acctttg 267

<210> 76
<211> 469
<212> DNA
<213> Homo sapiens

<400> 76
tctcctttct caccaatggg caatagccca taattgaaat aaatttctga ttgaaaggta 60
taggaaacat taaaatgcat tactaagaga agtaatataa ttttcttaca aagtattttt 120
cccaaagata gctttactat ttcaaaaatt gtcaaattaa tgcatgctcc ttacaacaaa 180
caaatatcaa aaagagttta ggaattctac tagccagaga tagtcacttg gagaaacttt 240
ctatatatcc ttctaaatat ttttctgggc atgctcatgt atgtacatca gttgtttctt 300
tttattttga accaaaaatg tggtttcttt tgtacacatt acttaaactt tctttccagt 360
caacaatata ttgtggattt attttcactg ttatatttaa ctatatataa atacgcatat 420
attgtaattt taatgtctgc ttagcacccc actgataacc aaatcacag 469

<210> 77
<211> 97
<212> DNA
<213> Homo sapiens

<400> 77
gagagtggac atttgtcggg aaactcctaa catatgcccc cattctggag agaacacaga 60
gtacgacaca atccctcaca ctaatagaac aatccta 97

<210> 78

<211> 299
<212> DNA
<213> Homo sapiens

<400> 78
aacacctgtg ctaggtcagt ctggcacgta agatgaacat ccctaccaac acagagctca 60
ccatctctta tacttaagtg aaaaacatgg ggaaggggaa aggggaatgg ctgcttttga 120
tatgttccct gacacatatc ttgaatggag acctccctac caagtgatga aagtgttgaa 180
aaacttaata acaaatgctt gttgggcaag aatgggattg aggattatct tctctcagaa 240
aggcattgtg aaggaattga gccagatctc tctccctact gcaaaaccct attgtagta 299

<210> 79
<211> 413
<212> DNA
<213> Homo sapiens

<400> 79
tgaagaaagt tctcctcctg atcacagcca tcttggcagt ggctgttggt ttcccagtct 60
ctcaagacca ggaacgagaa aaaagaagta tcagtgacag cgatgaatta gcttcagggt 120
tttttgtgtt cccttaccca tatccatttc gcccacttcc accaattcca tttccaagat 180
ttccatggtt tagacgtaat tttcctattc caatacctga atctgcccct acaactcccc 240
ttcctagcga aaagtaaaca agaaggaaaa gtcacgataa acctggtcac ctgaaattga 300
aattgagcca cttccttgaa gaatcaaaat tcctgttaat aaaagaaaaa caaatgtaat 360
tgaaatagca cacagcattc tctagtcaat atctttagtg atcttcttta ata 413

<210> 80
<211> 405
<212> DNA
<213> Homo sapiens

<400> 80
atgtcaggtt tgtacctacc acatttaaaa tagggacttg aagaattaaa cattttatta 60
caaatgaagc acttcatgca cagactggca catagtaagt agtcgatagg tgttaacaat 120
ttgtgttatt gttattttct ggagtccaac taacaaatcc cacagtgaat gacatcacag 180
ggatgcaacc aacaagatcc agaatatgga aacttctact agataaacaa ctccatttct 240
tcagcaacaa ttcaagagag agagagaaga gaagctatac attttaaaag gctgaagaaa 300
tatatgaacc aaatttgtat gaggcaatca gaaaaactga caccgactgt attaaggaat 360
tatctaattt tagtgtggta atgagattgc tgttatgttt tctaa 405

<210> 81
<211> 365
<212> DNA
<213> Homo sapiens

<400> 81
aaaagatctc tcactgggaa aagaaaaagt tatgcattta taaagtaatt aaactggttt 60
tccttgtact ttattaatct gaatctaatg gcacttcctt acgagggttt tcagatgtgc 120
ttgtagttaa tggcaacatt atcagaatga ctacacagac agtcctactc tgaggagatg 180
actttggaag aaacccattt ggaactacac accctgctat gtctgtggag aaatggaact 240
gcaatcctca agagtcacac ttcatattcc ttcctttcaa gtggttgata aaaggtagtg 300
cttcaagcac aggatttatg gaatagttgg caaattaaac aacatgcttt ttattttgac 360
tacca 365

<210> 82
<211> 321
<212> DNA
<213> Homo sapiens

<400> 82
ttatcaccat taatccatgc cagttatgta cagttttgca tgtttgtttt ttattttact 60
cttttctctc cttcattccc tattcctgtt cccccatagg tgcccagtct aatgtatttg 120
atatctgtcc ttagagcctc cgtatctgtg aagactagag tcatgcactt catcacattt 180
cagtaacgat gggccgcatg taccacagtc ccatgagatg atagaggtgc agaaaaattc 240
ctgtcatcta gtgacatcgt agccatcata acatnncaac acgactctca tttgtggtga 300
ccctggtgta cacaaaccta c 321

<210> 83
<211> 366
<212> DNA
<213> Homo sapiens

<400> 83
atggctggag atgtagtcta cgctgacatc aaaactgttc ggacttcccc gttagaactc 60
gcgtttccac ttcagagatc tgtttctttc aacttttcta ctgtccataa atcatgtcct 120
gccaaagact ggaaggtgca taagggaaaa tgttactgga ttgctgaaac taagaaatct 180
tggaacaaaa gtcaaaatga ctgtgccata aacaattcat atctcatggt gattcaagac 240
attactgcta tggtgagatt taacatttag aggtgacagc atcccccaca ctggcagtga 300
attttttgtg ctacaaactt ggcaaaagtc tgtgaaaaga agtttcaact tcatgtgtta 360
ttaact 366

<210> 84
<211> 548
<212> DNA
<213> Homo sapiens

<400> 84
taatcggctc actataggaa tttgcntcga ggccaagatt cgnacgagnn ngttcaaaag 60
cagctaaacc aaaagaagcc tccagacagc cctgagatca cctaaaaagc tgctaccaag 120
acagccacga agatcctacc aaaatgaagc gcttcctctt cctcctactc accatcagcc 180
tcctggttat ggtacagata caaactggac tctcaggaca aaacgacacc agccaaacca 240
gcagcccctc agcatccagc agcatgagcg gaggcatttt ccttttcttc gtggccaatg 300
ccataatcca cctcttctgc ttcagttgag gtgacacgtc tcagccttag ccctgtgccc 360
cctgaaacan nnnnnnnnnn nnnnnnnnag agaatcccct ccatctttgg gaggggttga 420
tgccagacat caccaggttg tagaagttga caggcagtgc catggggnca acagccaaaa 480
taggggggta atgatgtagg ggccaagcag tgcccagctg ggggtcaata aagttaccct 540
tgtacttg 548

<210> 85
<211> 388
<212> DNA
<213> Homo sapiens

<400> 85
   acagccacga agatcctacc aaaatgaagc gcttcctctt cctcctactc accatcagcc 60
   tcctggttat ggtacagata caaactggac tctcaggaca aaacgacacc agccaaacca 120
   gcagcccctc agcatccagc agcatgagcg gaggcatttt ccttttcttc gtggccaatg 180
   ccataatcca cctcttctgc ttcagttgag gtgacacgtc tcagccttag ccctgtgccc 240
   cctgaaacag ctgccaccat cactcgcaag agaatcccct ccatctttgg gaggggttga 300
   tgccagacat caccaggttg tagaagttga caggcagtgc catgggggca acagccaaaa 360
   taggggggta atgatgtagg ggccaagc 388

<210> 86
<211> 508
<212> DNA
<213> Homo sapiens

<400> 86
gatcttaaag ccacggagaa gcctctcatc ttatggcatt gacaaagaga agaccatcca 60
ccttaccctg aaagtggtga agcccagtga tgaggagctg cccttgtttc ttgtggagtc 120
aggtgatgag gcaaagaggc acctcctcca ggtgcgaagg tccagctcag tggcacaagt 180
gaaagcaatg atcgagacta agacgggtat aatccctgag acccagattg tgacttgcaa 240
tggaaagaga ctggaagatg ggaagatgat ggcagattac ggcatcagaa agggcaactt 300
actcttcctg gcatcttatt gtattggagg gtgaccaccc tggggatggg gtgttggcag 360
gggtcaaaaa gcttatttct tttaatctct tactcaacga acacatcttc tgatgatttc 420
ccaaaattaa tgagaatgag atgagtagag taagatttgg gtgggatggg taggatgaag 480
tatattgccc aactctatgt ttctttga 508

<210> 87
<211> 501
<212> DNA
<213> Homo sapiens

<400> 87
gctgacatcc tgcgagacta caaagttatt atggctgaaa acattcctga aaaccctctg 60
aagtacctat atcctgacat tcccaaagac aaagccttcg gtaaacacta cagctctcag 120
ccttgcgaag tttcaagacc aacagaaagg ggtgacaaag gttatgttcc ttctgttttt 180
atccccatct caacaatccg aagtgattca acagagccac attctccatc agaccttctt 240
cccatgtctc caagtgtgta tgcggtgttg agagaaaacc tgagtcccac aacaattgaa 300
actgcaatga agtctcctta ttctgctgaa tgacaggata aactctgacg caccaagaaa 360
ggaagcaaat gaaaaagttt aaagactgtt ctttgcccaa taaccacatt ttatttcttc 420
agctttgtaa ataccaggtt ctaggaaatg tttgacatct gaagctctct tcacactccc 480
gtggcactcc tcaattggga g 501

<210> 88
<211> 489
<212> DNA
<213> Homo sapiens

<400> 88
aaacctctct tagatctgga accaaatgca aggttactgg ctggggagcc accgatccag 60
attcattaag accttctgac accctgcgag aagtcactgt tactgtccta agtcgaaaac 120
tttgcaacag ccaaagttac tacaacggcg acccttttat caccaaagac atggtctgtg 180
caggagatgc caaaggccag aaggattcct gtaagggtga ctcagggggc cccttgatct 240
gtaaaggtgt cttccacgct atagtctctg gaggtcatga atgtggtgtt gccacaaagc 300
ctggaatcta caccctgtta accaagaaat accagacttg gatcaaaagc aaccttgtcc 360
cgcctcatac aaattaagtt acaaataatt ttattggatg cacttgcttc tttttcctaa 420
atatgctcgc aggttagagt tgggtgtaag taaagcagag cacatatggg gtccattttt 480
gcacttgta 489

<210> 89
<211> 405
<212> DNA
<213> Homo sapiens

<400> 89
ttgccttgta attcgacagc tctacagaaa caaagataat gaaaattacc caaatgtgaa 60
aaaggctctc atcaacatac ttttagtgac cacgggctac atcatatgct ttgttcctta 120
ccacattgtc cgaatcccgt ataccctcag ccagacagaa gtcataactg attgctcaac 180
caggatttca ctcttcaaag ccaaagaggc tacactgctc ctggctgtgt cgaacctgtg 240
ctttgatcct atcctgtact atcacctctc aaaagcattc cgctcaaagg tcactgagac 300
ttttgcctca cctaaagaga ccaaggctca gaaagaaaaa ttaagatgtg aaaataatgc 360
ataaaagaca ggattttttg tgctaccaat tctggcctta ctgga 405

<210> 90

<211> 352
<212> DNA
<213> Homo sapiens

<400> 90
aatccattca tcctgattgg gcatgaaatc catggtcaag aggacaagtg gaaagtgaga 60
gggaaggttt gctagacacc ttcgcttgtt atcttgtcaa gatagaaaag atagtatcat 120
ttcacccttg ccagtaaaaa cctttccatc cacccattct cagcagactc cagtattggc 180
acagtcactc actgccattc tcacactata acaagaaaag aaatgaagtg cataagtctc 240
ctgggaaaag aaccttaacc ccttctcgtg ccatgactgg tgatttcatg actcataagc 300
ccctccgtag gcatcattca agatcaatgg cccatgcatg ctgtttgcag ca 352

<210> 91
<211> 484
<212> DNA
<213> Homo sapiens

<400> 91
ggaacaagac ttcaggtcac gctcgatatc cagaaccctg accctgccgt gtaccagctg 60
agagactcta aatccagtga caagtctgtc tgcctattca ccgattttga ttctcaaaca 120
aatgtgtcac aaagtaagga ttctgatgtg tatatcacag acaaaactgt gctagacatg 180
aggtctatgg acttcaagag caacagtgct gtggcctgga gcaacaaatc tgactttgca 240
tgtgcaaacg ccttcaacaa cagcattatt ccagaagaca ccttcttccc cagcccagaa 300
agttcctgtg atgtcaagct ggtcgagaaa agctttgaaa cagatacgaa cctaaacttt 360
caaaacctgt cagtgattgg gttccgaatc ctcctcctga aagtggccgg gtttaatctg 420
ctcatgacgc tgcggctgtg gtccagctga gatctgcaag attgtaagac agcctgtgct 480
ccct 484

<210> 92
<211> 512
<212> DNA
<213> Homo sapiens

<400> 92
aaatgataca ctactgctgc agctcacaaa cacctctgca tattacatgt acctcctcct 60
gctcctcaag agtgtggtct attttgccat catcacctgc tgtctgcttg gaagaacggc 120
tttctgctgc aatggagaga aatcataaca gacggtggca caaggaggcc atcttttcct 180
catcggttat tgtccctaga agcgtcttct gaggatctag ttgggctttc tttctgggtt 240
tgggccattt cagttctcat gtgtgtacta ttctatcatt attgtataat ggttttcaaa 300
ccagtgggca cacagagaac ctcagtctgt aataacaatg aggaatagcc atggcgatct 360
ccagcaccaa tctctccatg ttttccacag ctcctccagc caacccaaat agcgcctgct 420
atagtgtaga cagcctgcgg cttctagcct tgtccctctc ttagtgttct ttaatcagat 480
aactgcctgg aagcctttca ttttacacgc cc 512

<210> 93
<211> 483
<212> DNA
<213> Homo sapiens

<400> 93
gccatcagaa gcagagatct cccacaccca aaaggccaca ctggtgtgcc tggccacagg 60
tttctacccc gaccacgtgg agctgagctg gtgggtgaat gggaaggagg tgcacagtgg 120
ggtcagcaca gacccgcagc ccctcaagga gcagcccgcc ctcaatgact ccagatactg 180
cctgagcagc cgcctgaggg tctcggccac cttctggcag aacccccgca accacttccg 240
ctgtcaagtc cagttctacg ggctctcgga gaatgacgag tggacccagg atagggccaa 300
acctgtcacc cagatcgtca gcgccgaggc ctggggtaga gcagactgtg gcttcacctc 360
cgagtcttac cagcaagggg tcctgtctgc caccatcctc tatgagatct tgctagggaa 420
ggccaccttg tatgctgtgc tggtcagtgc cctcgtgctg atggccatgg tcaagagaaa 480

gga                                                                 483

<210> 94
<211> 519
<212> DNA
<213> Homo sapiens

<400> 94
tgactccaga tactgcctga gcagccgcct gagggtctcg gccaccttct ggcagaaccc 60
ccgcaaccac ttccgctgtc aagtccagtt ctacgggctc tcggagaatg acgagtggac 120
ccaggatagg gccaaacccg tcacccagat cgtcagcgcc gaggcctggg gtagagcaga 180
ctgtggcttt acctcggtgt cctaccagca aggggtcctg tctgccacca tcctctatga 240
gatcctgcta gggaaggcca ccctgtatgc tgtgctggtc agcncccttg tgttgatggc 300
catggtcaag agaaaggatt tctgaaggca gccctggaag tggagttagg agcttctaac 360
ccgtcatggt ttcaatacac attcttcttt tgccagcgct tctgaagagc tgctctcacc 420
tctctgcatc ccaatagata tccccctatg tgcatgcaca cctgcacact cacggctgaa 480
atctccctaa cccaggggga ccttagcatg cctaagtga                          519

<210> 95
<211> 419
<212> DNA
<213> Homo sapiens

<400> 95
gggaacactg ctctcagaca ttacaagact ggacctggga aaacgcatcc tggacccacg 60
aggaatatat aggtgtaatg ggacagatat atacaaggac aaagaatcta ccgtgcaagt 120
tcattatcga atgtgccaga gctgtgtgga gctggatcca gccaccgtgg ctggcatcat 180
tgtcactgat gtcattgcca ctctgctcct tgctttggga gtcttctgct ttgctggaca 240
tgagactgga aggctgtctg gggctgccga cacacaagct ctgttgagga atgaccaggt 300
ctatcagccc ctccgagatc gagatgatgc tcagtacagc caccttggag gaaactgggc 360
tcggaacaag tgaacctgag actggtggct tctagaagca gccattacca actgtacct  419

<210> 96
<211> 469
<212> DNA
<213> Homo sapiens

<400> 96
tctcctttct caccaatggg caatagccca taattgaaat aaatttctga ttgaaaggta 60
taggaaacat taaaatgcat tactaagaga agtaatataa ttttcttaca aagtattttt 120
cccaaagata gctttactat ttcaaaaatt gtcaaattaa tgcatgctcc ttacaacaaa 180
caaatatcaa aaagagttta ggaattctac tagccagaga tagtcacttg gagaaacttt 240
ctatatatcc ttctaaatat ttttctgggc atgctcatgt atgtacatca gttgtttctt 300
tttattttga accaaaaatg tggtttcttt tgtacacatt acttaaactt tctttccagt 360
caacaatata ttgtggattt attttcactg ttatatttaa ctatatataa atacgcatat 420
attgtaattt taatgtctgc ttagcacccc actgataacc aaatcacag               469

<210> 97
<211> 548
<212> DNA
<213> Homo sapiens

<400> 97
taatcggctc actataggaa tttgcntcga ggccaagatt cgnacgagnn ngttcaaaag 60
cagctaaacc aaaagaagcc tccagacagc cctgagatca cctaaaaagc tgctaccaag 120
acagccacga agatcctacc aaaatgaagc gcttcctctt cctcctactc accatcagcc 180
tcctggttat ggtacagata caaactggac tctcaggaca aaacgacacc agccaaacca 240
gcagcccctc agcatccagc agcatgagcg gaggcatttt ccttttcttc gtggccaatg 300

ccataatcca cctcttctgc ttcagttgag gtgacacgtc tcagccttag ccctgtgccc 360
cctgaaacan nnnnnnnnnn nnnnnnnnag agaatcccct ccatctttgg gaggggttga 420
tgccagacat caccaggttg tagaagttga caggcagtgc catggggnca acagccaaaa 480
taggggggta atgatgtagg ggccaagcag tgcccagctg ggggtcaata aagttaccct 540
tgtacttg 548

<210> 98
<211> 21
<212> DNA
<213> Homo sapiens

<400> 98
tcctaccagc ctttgcctct t 21

<210> 99
<211> 23
<212> DNA
<213> Artificial Sequence

<220>
<223> IL7R Reverse primer

<400> 99
ccatacttgg cttttctgaa gca 23

<210> 100
<211> 21
<212> DNA
<213> Homo sapiens

<400> 100
cttcaatgtg gtttccatgg g 21

<210> 101
<211> 25
<212> DNA
<213> Homo sapiens

<400> 101
gttgaggaat gaccaggtct atcag 25

<210> 102
<211> 22
<212> DNA
<213> Artificial Sequence

<220>
<223> CD3D Reverse primer

<400> 102
aaggtggctg tactgagcat ca 22

<210> 103
<211> 14
<212> DNA
<213> Homo sapiens

<400> 103

cctccgagat cgag                                                    14

<210> 104
<211> 23
<212> DNA
<213> Homo sapiens

<400> 104
tcagcctcct ggttatggta cag                                          23

<210> 105
<211> 19
<212> DNA
<213> Artificial Sequence

<220>
<223> CD52 Reverse primer

<400> 105
tggtttggct ggtgtcgtt                                               19

<210> 106
<211> 19
<212> DNA
<213> Homo sapiens

<400> 106
caaactggac tctcaggac                                               19

<210> 107
<211> 20
<212> DNA
<213> Homo sapiens

<400> 107
tgaggagctg cccttgtttc                                              20

<210> 108
<211> 20
<212> DNA
<213> Artificial Sequence

<220>
<223> UBD Reverse primer

<400> 108
ctggaggagg tgcctctttg                                              20

<210> 109
<211> 17
<212> DNA
<213> Homo sapiens

<400> 109
catcacctga ctccaca                                                 17

<210> 110
<211> 25

<212> DNA
<213> Homo sapiens

<400> 110
gaagtcataa ctgattgctc aacca                                          25

<210> 111
<211> 22
<212> DNA
<213> Artificial Sequence

<220>
<223> GPR171 Reverse primer

<400> 111
gacacagcca ggagcagtgt ag                                             22

<210> 112
<211> 20
<212> DNA
<213> Homo sapiens

<400> 112
tcactcttca aagccaaaga                                                20

<210> 113
<211> 20
<212> DNA
<213> Homo sapiens

<400> 113
gtctgtgcag gagatgccaa                                                20

<210> 114
<211> 17
<212> DNA
<213> Artificial Sequence

<220>
<223> GMZK Reverse primer

<400> 114
gggcccctg agtcacc                                                    17

<210> 115
<211> 19
<212> DNA
<213> Homo sapiens

<400> 115
ttacaggaat ccttctggc                                                 19

<210> 116
<211> 25
<212> DNA
<213> Homo sapiens

<400> 116

atccatggtc aagaggacaa gtgga                                          25

<210> 117
<211> 24
<212> DNA
<213> Artificial Sequence

<220>
<223> PRKCQ Reverse primer

<400> 117
gacaagataa caagcgaagg tgtc                                           24

<210> 118
<211> 18
<212> DNA
<213> Homo sapiens

<400> 118
caaaccttcc ctctcact                                                  18

<210> 119
<211> 22
<212> DNA
<213> Homo sapiens

<400> 119
gtcccacaac aattgaaact gc                                             22

<210> 120
<211> 24
<212> DNA
<213> Artificial Sequence

<220>
<223> STAT4 Reverse primer

<400> 120
gtgcgtcaga gtttatcctg tcat                                           24

<210> 121
<211> 20
<212> DNA
<213> Homo sapiens

<400> 121
tgaagtctcc ttattctgct                                                20

<210> 122
<211> 23
<212> DNA
<213> Homo sapiens

<400> 122
ggtcgagaaa agctttgaaa cag                                            23

<210> 123
<211> 22

<212> DNA
<213> Artificial Sequence

<220>
<223> TRDV2 Reverse primer

<400> 123
ggaacccaat cactgacagg tt                                                22

<210> 124
<211> 16
<212> DNA
<213> Homo sapiens

<400> 124
acgaacctaa actttc                                                       16

<210> 125
<211> 18
<212> DNA
<213> Homo sapiens

<400> 125
aagccacccc atctgcac                                                     18

<210> 126
<211> 21
<212> DNA
<213> Artificial Sequence

<220>
<223> TRAT1 Reverse primer

<400> 126
ctgtgatcaa gtgaggcgta g                                                 21

<210> 127
<211> 20
<212> DNA
<213> Homo sapiens

<400> 127
caaccaatga aacacagatg                                                   20

<210> 128
<211> 19
<212> DNA
<213> Homo sapiens

<400> 128
gaggcctggg gtagagcag                                                    19

<210> 129
<211> 20
<212> DNA
<213> Artificial Sequence

<220>

<223> TRBV19 Reverse primer

<400> 129
agaggatggt ggcagacagg                                                    20

<210> 130
<211> 17
<212> DNA
<213> Homo sapiens

<400> 130
tgtcctacca gcaaggg                                                       17

<210> 131
<211> 26
<212> DNA
<213> Homo sapiens

<400> 131
aatcatagct ctcattgcct tatcag                                             26

<210> 132
<211> 23
<212> DNA
<213> Artificial Sequence

<220>
<223> CD69 Reverse primer

<400> 132
tgtctgatgg cattgagaat gtg                                                23

<210> 133
<211> 20
<212> DNA
<213> Homo sapiens

<400> 133
ccaatacaat tgtccaggcc                                                    20

<210> 134
<211> 19
<212> DNA
<213> Homo sapiens

<400> 134
ccagcagact gctggtgga                                                     19

<210> 135
<211> 25
<212> DNA
<213> Artificial Sequence

<220>
<223> INDO1 Reverse primer

<400> 135
ggcatatatc ttctcatgtc ctgga                                              25

<210> 136
<211> 16
<212> DNA
<213> Homo sapiens

<400> 136
acatgctgct cagttc                                                        16

<210> 137
<211> 20
<212> DNA
<213> Homo sapiens

<400> 137
cagctatcgg tgctttgcag                                                    20

<210> 138
<211> 24
<212> DNA
<213> Artificial Sequence

<220>
<223> H3F3A Reverse primer

<400> 138
gatagcacac aggttggtgt cttc                                               24

<210> 139
<211> 16
<212> DNA
<213> Homo sapiens

<400> 139
caagtgaggc ctatct                                                        16

<210> 140
<211> 25
<212> DNA
<213> Homo sapiens

<400> 140
gagatgcctg atggttcaag tagag                                              25

<210> 141
<211> 25
<212> DNA
<213> Artificial Sequence

<220>
<223> CD45R Reverse primer

<400> 141
ttcttcaaac tgattgtatt ccacc                                              25

<210> 142
<211> 19
<212> DNA

<213> Homo sapiens

<400> 142
cagtacatct tgatccatc                                                    19

<210> 143
<211> 19
<212> DNA
<213> Homo sapiens

<400> 143
ggcaaagccc aacaccttc                                                    19

<210> 144
<211> 22
<212> DNA
<213> Artificial Sequence

<220>
<223> CD45RO Reverse primer

<400> 144
cagaagggct cagagtggtt gt                                                22

<210> 145
<211> 16
<212> DNA
<213> Homo sapiens

<400> 145
tgcctacctt aatgcc                                                       16

<210> 146
<211> 19
<212> DNA
<213> Homo sapiens

<400> 146
cacctggctg ggaaaatgg                                                    19

<210> 147
<211> 19
<212> DNA
<213> Artificial Sequence

<220>
<223> FoxP3 Reverse primer

<400> 147
ggagcccttg tcggatgat                                                    19

<210> 148
<211> 16
<212> DNA
<213> Homo sapiens

<400> 148
caaggcttca tctgtg                                                       16

<210> 149
<211> 25
<212> DNA
<213> Homo sapiens

<400> 149
catacaatct ctgttcttgg gcatt                                             25

<210> 150
<211> 22
<212> DNA
<213> Artificial Sequence

<220>
<223> CD20 Reverse primer

<400> 150
tcattctcaa cgatgccagc ta                                                22

<210> 151
<211> 17
<212> DNA
<213> Homo sapiens

<400> 151
tcagtgatgc tgatctt                                                      17

<210> 152
<211> 19
<212> DNA
<213> Homo sapiens

<400> 152
cgccagaccc tctggagaa                                                    19

<210> 153
<211> 19
<212> DNA
<213> Artificial Sequence

<220>
<223> TLR9 Reverse primer

<400> 153
gcctgcacca ggagagaca                                                    19

<210> 154
<211> 13
<212> DNA
<213> Homo sapiens

<400> 154
ttctgccgca gcg                                                          13

<210> 155
<211> 27
<212> DNA

<213> Homo sapiens

<400> 155
aagtccatcc tgtatgtggt agaagag                                          27

<210> 156
<211> 20
<212> DNA
<213> Artificial Sequence

<220>
<223> ITGB3 Reverse primer

<400> 156
gagcaggacc accaggatgt                                                  20

<210> 157
<211> 14
<212> DNA
<213> Homo sapiens

<400> 157
agtgtcccaa gggc                                                        14

<210> 158
<211> 21
<212> DNA
<213> Homo sapiens

<400> 158
ggaaaggcta gcagtcatcc a                                                21

<210> 159
<211> 20
<212> DNA
<213> Artificial Sequence

<220>
<223> TLR3 Reverse primer

<400> 159
gtggaggatg cacacagcat                                                  20

<210> 160
<211> 17
<212> DNA
<213> Homo sapiens

<400> 160
atgagacaga ctttgcc                                                     17

<210> 161
<211> 19
<212> DNA
<213> Homo sapiens

<400> 161
ccagaactgc aggtgctgg                                                   19

<210> 162
<211> 22
<212> DNA
<213> Artificial Sequence

<220>
<223> TLR4 Reverse primer

<400> 162
ggtggcttag gctctgatat gc                                                22

<210> 163
<211> 20
<212> DNA
<213> Homo sapiens

<400> 163
ccaggtgtga aatccagaca                                                   20

## Description

### Field of the Invention

The present invention relates to gene expression profiles; microarrays comprising nucleic acid sequences for identifying said profiles, for example, by analysing patient derived samples; new diagnostic kits and methods for identification of same. The invention further relates to treatment of specific populations of patients, for example cancer patients, characterised as a responder by their gene expression profile, such as patients suffering from Mage expressing tumours. The invention further includes methods of inducing a responder's profile in a patient initially or originally designated as a non-responder.

### Background

Melanomas are tumors originating from melanocyte cells in the epidermis. Patients with malignant melanoma in distant metastasis (stage IV according to the American Joint Commission on Cancer (AJCC) classification) have a median survival time of one year, with a long-term survival rate of only 5%. Even the standard chemotherapy for stage IV melanoma has therapeutic response rates of only 8-25%, but with no effect on overall survival. Patients with regional metastases (stage III) have a median survival of two to three years with very low chance of long-term survival, even after an adequate surgical control of the primary and regional metastases (Balch *et al.,* 1992). Most Patients with stage I to III melanoma have their tumour removed surgically, but these patients maintain a substantial risk of relapse. Thus there remains a need to prevent melanoma progression, and to have improved treatment regimes for metastatic melanoma and adjuvant treatments for patients having had a primary tumour removed.

There are two types of lung cancer: non-small cell lung cancer (NSCLC) and small cell lung cancer (SCLC). The names simply describe the type of cell found in the tumours. NSCLC includes squamous-cell carcinoma, adenocarcinoma, and large-cell carcinoma and accounts for around 80% of lung cancers. NSCLC is hard to cure and treatments available tend to have the aim of prolonging life, as far as possible, and relieving symptoms of disease. NSCLC is the most common type of lung cancer and is associated with poor outcomes (Gatzmeier *et al.,* 1994). Of all NSCLC patients, only about 25% have loco-regional disease at the time of diagnosis and are still amenable to surgical excision (stages IB, IIA or IIB according to the AJCC classification). However, more than 50% of these patients will relapse within the two years following the complete surgical resection. There is therefore a need to provide better treatment for these patients.

Traditional chemotherapy is based on administering toxic substances to the patient and relying, in part, on the aggressive uptake of the toxic agent by the tumour/cancer cells. These toxic substances adversely affect the patient's immune system, leaving the individual physically weakened and susceptible to infection.

It is known that not all patients with cancer respond to current cancer treatments. It is thought that only 30% or less of persons suffering from a cancer will respond to any given treatment. The cancers that do not respond to treatment are described as resistant. In many instances there have not been reliable methods for establishing if the patients will respond to treatment. However, administering treatment to patients who are both responders and non-responders because they cannot be differentiated is an inefficient use of resources and, even worse, can be damaging to the patient because, as discussed already, many cancer treatments have significant side effects, such as severe immunosuppression, emesis and/or alopecia. It is thought that in a number of cases patients receive treatment, when it is not necessary or when it will not be effective.

Cells including cancer/tumour cells express many hundreds even thousands of genes.

A large amount of work has been done in recent times to assist in the diagnosis and prognosis of cancer patients, for example to identify those patients who do not require further treatment because they have no risk of metastasis, recurrence or progression of the disease.

WO 2006/124836 identifies certain gene expression signatures over several oncogenic pathways, thereby defining the prognosis of the patient and sensitivity to therapeutic agents that target these pathways. The specific oncogenes are; Myc, Ras, E2, S3, Src and beta-catenin.

US 2006/0265138 discloses a method of generating a genetic profile, generally for identifying the primary tumour so that appropriate treatment can be given.

US 2006/0240441 and US 2006/0252057 describe methods of diagnosing lung cancer based on the differential expression of certain genes.

US 2006/0234259 relates to the identification and use of certain gene expression profiles of relevance to prostate cancer.

WO 2006/103442 describes gene expression profiles expressed in a subset of estrogen receptor (ER) positive tumours, which act, as a predictive signature for response to certain hormone therapies such as tamoxifen and also certain chemotherapies.

WO 2006/093507 describes a gene profile useful for characterising a patient with colorectal cancer as having a good prognosis or a bad prognosis, wherein patients with a good prognosis are suitable for chemotherapy.

WO 2006/092610 describes a method for monitoring melanoma progression based on differential expression of certain genes and novel markers for the disease, in particular TSBY1, CYBA and MT2A.

WO 2005/049829 describes an isolated set of marker genes that may be employed to predict the sensitivity of certain cancers to a chemotherapeutic agent, which is an erbB receptor kinase inhibitor, such as gefitinib.

Generally, these cases relate to markers for one or more cancers based on biological markers for the identification and/or progression of the cancer. In some instances these pathways are modulated by so-called oncogenes. Diagnosis employing the above techniques allows those patients who are likely to relapse and/or suffer metastasis to be identified and targeted for further therapy. In other instances a specific marker relevant to resistance to a specific treatment is identified.

A new generation of cancer treatments based on antigens, peptides, DNA and the like is currently under investigation by a number of groups. The strategy behind many of these therapies, often referred to as cancer immunotherapy, is to stimulate the patient's immune system into fighting the cancer. These therapies are likely to be advantageous because the side effects, of taking such treatments, are expected to be minimal in comparison to the side effects currently encountered by patients undergoing cancer treatment. An antigen used in a cancer immunotherapy may be referred to as an ASCI, that is antigen-specific cancer immunotherapeutic.

In the early 1980s, Van Pel and Boon published the discovery of cytolytic T cells directed against an antigen presented on tumour cells. This led to the characterization of the first tumour-specific, shared antigen: Melanoma AGE-1 (MAGE-1, subsequently renamed MAGE-A1). It was followed by the identification of a large number of genes sharing the same expression pattern: they are expressed in a wide range of tumour types such as, melanoma, lung, bladder, breast, head and neck cancers. They are not expressed in normal cells, except testis. However, this expression in the testis does not normally lead to antigen expression, as these germ line cells do not express MHC class I molecules. From their peculiar expression profile, the name of Cancer Testis (CT) genes was proposed for these genes.

MAGE antigens are antigens encoded by the family of Melanoma- associated antigen genes (MAGE). MAGE genes are predominately expressed on melanoma cells (including malignant melanoma) and some other cancers including NSCLC (non small cell lung cancer), head and neck squamous cell carcinoma, bladder transitional cell carcinoma and oesophagus carcinoma, but are not detectable on normal tissues except in the testis and the placenta (Gaugler et al Human gene MAGE-3 codes for an antigen recognized on a melanoma by autologous cytolytic T lymphocytes J Exp Med. 1994 Mar 1;179(3):921-930); Weynants et al Expression of mage genes by non-small-cell lung carcinomas Int. J Cancer. 1994 Mar 15;56(6):826-829, Patard et al Int J. Cancer 64: 60, 1995). MAGE-A3 is expressed in 69% of melanomas (Gaugler, 1994), and can also be detected in 44% of NSCLC (Yoshimatsu 1988), 48% of head and neck squamous cell carcinoma, 34% of bladder transitional cell carcinoma, 57% of oesophageal carcinoma, 32% of colon cancers and 24% of breast cancers (Van Pel, et al Genes coding for tumor antigens recognized by cytolytic T lymphocytes Immunological Reviews 145, 229-250, 1995, 1995.); Inoue 1995; Fujie 1997; Nishimura 1997). Cancers expressing MAGE proteins are known as Mage associated tumours.

### Summary

In one aspect the invention provides a method for detection of a gene signature, indicative of a responder or non-responder to immunotherapy, such as cancer immunotherapy, in a biological sample. Thus the invention provides a method of screening patients to establish if they are suitable for treatment, for example with a cancer immunotherapy.

In one aspect the invention provides a diagnostic kit comprising one or more nucleotide probes capable of hybridising to the mRNA or cDNA of one or more immune activation genes relevant to the profile.

In one aspect the invention provides one or more probes for identifying said one or more immune activation genes relevant to the profile.

In one aspect the invention provides a microarray of said probes suitable for the detection of said gene(s)/profile.

In another aspect the invention provides use of a microarray, including known microarrays, for the identification of said immune gene(s)/profile.

In one aspect the invention provides use of PCR (or other known techniques) for identification of differential expression (such as upregulation) of one or more of said genes.

In one aspect the invention provides a method of treating a patient with an appropriate immunotherapy after screening to identify the patient as a responder to said treatment.

In another aspect the invention provides a method of increasing the efficacy of an immunotherapy in a patient population comprising the step of first screening the population for the differential expression of one or more immune genes/said profile, and a second step of characterising the patient as a responder or non-responder.

In a further aspect the invention provides a method of generating a list of diffentially expressed immune genes (ie a profile of immune genes) indicative of a responder or non-responder to immunotherapy.

### Brief Description of the Figures, Sequences & Tables

**Figure 1** is a diagrammatic representation of hierarchical clustering using Spotfire analysis linking the clinical outcome of 31 patients found to be responders (defined herein to include responder, mixed responder and stable disease) or non-responders, to Mage immunotherapy in the MAGE008 clinical trial, with the gene profile identified by microarray analysis and employing the 148 top probe sets.
**Figure 2** is a diagrammatic representation of the same analysis as Figure 1, wherein the hierarchical clustering was performed using the BaldiBH analysis using 100 probes sets.
**Figure 3** is a diagrammatic representation of the same analysis as Figure 1, wherein the hierarchical clustering was performed using Arrayminer Classmaker gene list.
**Figure 4** is a Venn diagram representing the comparison of gene lists used in Figure 1, Figure 2 and Figure 3.
**Figure 5** is a visual representation of the expression profile of various genes (36 probe sets) for 30 different patients (patients are along the X-axis and the various probe sets extend along the Y-axis).
**Figure 5a** is a visual representation of the expression of 2 genes for 30 different patients (patients along the X-axis).
**Figure 6** shows the Principal Component Analysis using PRF1, GZMB, GNLY, CD8A, PRKCQ, FOXP3, IFNG, CCL5, GPR171 and TRBV19 genes.
**Figure 7** is a visual representation of the expression profile of various genes (41 probe sets) for 33 different patients (patient identification numbers are along the X-axis) from the MAGE008 clinical trial using the adjuvant AS15.
**Figure 8** is a diagrammatic representation of hierarchical clustering for patients in the AS15 arm of the MAGE008 clinical trial.
**Figure 9** is a visual representation of the expression profile of various genes (41 probe sets) for 33 different patients (patient identification numbers are along the X-axis) from the MAGE008 clinical trial using the adjuvant AS02b.
**Figure 10** is a diagrammatic representation of hierarchical clustering for patients in the AS02b arm of the MAGE008 clinical trial.

In the heat maps herein upregulated genes are represented in red which in greyscale tends to be presented by darker shades. Lighter shades in greyscale tend to present green on the heat map (genes which are not upregulated).
- **Seq ID No 1**: Chromosome 6 open reading frame 190
- **Seq ID No 2**: Hematopoietic cell-specific Lyn substrate 1
- **Seq ID No 3**: Interleukin 2 receptor, gamma (severe combined immunodeficiency)
- **Seq ID No 4**: CD52 antigen (CAMPATH-1 antigen) /// CD52 antigen (CAMPATH-1 antigen)
- **Seq ID No 5**: CD2 antigen (p50), sheep red blood cell receptor /// CD2 antigen (p50), sheep red blood cell receptor
- **Seq ID No 6**: Ubiquitin D
- **Seq ID No 7**: Signal transducer and activator of transcription 4
- **Seq ID No 8**: Granzyme K (granzyme 3; tryptase II) /// granzyme K (granzyme 3; tryptase II)
- **Seq ID No 9**: CD3G antigen, gamma polypeptide (TiT3 complex)
- **Seq ID No10**: G protein-coupled receptor 171
- **Seq ID No 11**: Protein kinase C, beta 1
- **Seq ID No 12**: Major histocompatibility complex, class II, DR alpha /// major histocompatibility complex, class II, DR alpha
- **Seq ID No 13**: Major histocompatibility complex, class II, DQ beta 1 /// Major histocompatibility complex, class II, DQ beta 1
- **Seq ID No 14**: Alcohol dehydrogenase IB (class I), beta polypeptide
- **Seq ID No 15**: T cell receptor alpha constant /// T cell receptor alpha constant
- **Seq ID No 16**: CD69 antigen (p60, early T-cell activation antigen)
- **Seq ID No 17**: Protein kinase C, theta
- **Seq ID No 18**: T cell receptor beta variable 19 /// T cell receptor beta constant 1
- **Seq ID No 19**: T cell receptor alpha locus /// T cell receptor delta variable 2 /// T cell receptor alpha variable 20 /// T cell receptor alpha joining 17 /// T cell receptor alpha constant
- **Seq ID No 20**: T cell receptor gamma constant 2
- **Seq ID No 21**: T cell receptor beta variable 21-1 /// T cell receptor beta variable 19 /// T cell receptor beta variable 5-4 /// T cell receptor beta variable 3-1 /// T cell receptor beta constant 1
- **Seq ID No 22**: T cell receptor alpha locus
- **Seq ID No 23**: T cell receptor beta variable 19 /// T cell receptor beta variable 19 /// T cell receptor beta constant 1 /// T cell receptor beta constant 1
- **Seq ID No 24**: CD3D antigen, delta polypeptide (TiT3 complex)
- **Seq ID No 25**: T cell receptor gamma constant 2 /// T cell receptor gamma variable 9 /// similar to T-cell receptor gamma chain C region PT-gamma-1/2 /// similar to T-cell receptor gamma chain V region PT-gamma-1/2 precursor /// TCR gamma alternate reading frame protein
- **Seq ID No 26**: Pyrin and HIN domain family, member 1
- **Seq ID No 27**: T cell receptor associated transmembrane adaptor 1
- **Seq ID No 28**: SLAM family member 7
- **Seq ID No 29**: Chromosome 4 open reading frame 7
- **Seq ID No 30**: Major histocompatibility complex, class II, DQ alpha 1
- **Seq ID No 31**: Transcribed locus
- **Seq ID No 32**: Amphiphysin (Stiff-Man syndrome with breast cancer 128kDa autoantigen)
- **Seq ID No 33**: Dendritic cell-associated lectin-1

Each sequences above is given in Table 1A below.
- **Seq ID No 34**: Nucleotide sequence encoding fusion protein of Lipoprotein D fragment, Mage3 fragment, and histidine tail
- **Seq ID No 35**: Amino acid sequence of the fusion protein of Lipoprotein D fragment, Mage3 fragment and histidine tails
- **Seq ID No.s 36 to 43**: (found in the description) are peptide sequences relevant to MAGE A3.
- **Seq ID No.s 44 to 48**: are examples of oliogionucleotide sequences containing a CpG motif.
- **Seq ID No.s 49 to 84**: (listed in Table 1B) are probes sets suitable for hybridising to the genes listed in Table 1.
- **Seq ID No.s 85 to 97**: (listed in Table 3A) are sequences for the genes listed in Table 3.

**Tables 1 to 4, 11 and 12** provide lists of genes that are differentially regulated according to the present invention.
**Table 1A** provides the nucleotide sequence listing for each of the genes listed in Table 1.
**Table 1B** provides the probe set identifier number (a unique reference number for the probe) of probes (and sequence thereof) wherein each probe is suitable for identifying particular genes listed in Table 1.
**Table 3A** links the genes (and sequences thereof) of Table 3 and probes suitable for identifying said genes.
**Table 5** provides a list of genes, primers and probes suitable for use in PCR analysis.
**Table 6** is a list of gene included in the TaqMan® (Q-PCR) Immune Profiling Array.
**Table 7** provides a list of genes according to one aspect of the invention.
**Table 7A** provides the geomean ratio between responders and non-responder groups for the genes listed in Table 7.
**Table 8** provides a correlation matrix for 30 genes.
**Table 9** provides a list of genes according to one aspect of the invention.
**Table 9A** logistical regression results for certain genes listed in Table 9
**Table 10** shows the percentage of correct classification using a logistical regression model for the genes listed in Table 9.
**Table 11** provides a list of genes according to one aspect of the invention.
**Tables 11A & 11B** show the level of gene expression (based on the results of 41 probe sets) for various patients.
**Tables 12 and 13** show gene lists that form further aspects of the invention.
**Table 14** provides a correlation between the gene expression levels given in Tables 11A & 11B with the clinical outcome for said patients.

**Annexes A to C** are computer code for assisting with stastical analysis of samples.

The MAGE-1 gene belongs to a family of 12 closely related genes, MAGE 1, MAGE 2, MAGE 3, MAGE 4, MAGE 5, MAGE 6, MAGE 7 , MAGE 8, MAGE 9, MAGE 10, MAGE 11, MAGE 12, located on chromosome X and sharing with each other 64 to 85% homology in their coding sequence (De Plaen, 1994). These are sometimes known as MAGE A1, MAGE A2, MAGE A3, MAGE A4, MAGE A5, MAGE A6, MAGE A7, MAGE A8, MAGE A9, MAGE A 10, MAGE A11, MAGE A12 (The MAGE A family).

Two other groups of proteins are also part of the MAGE family although more distantly related. These are the MAGE B and MAGE C group. The MAGE B family includes MAGE B1 (also known as MAGE Xp1, and DAM 10), MAGE B2 (also known as MAGE Xp2 and DAM 6) MAGE B3 and MAGE B4 - the Mage C family currently includes MAGE C1 and MAGE C2.

In general terms, a MAGE A protein can be defined as containing a core sequence signature located towards the C-terminal end of the protein (for example with respect to MAGE A1 a 309 amino acid protein, the core signature corresponds to amino acid 195-279).

It does not simply follow that if the tumour expresses, for example, Mage that the patient will respond to immunotherapy based on a Mage antigen.

### Statement of Invention

Analysis performed on cancers/tumours from patients prior to receiving immunotherapy, such as Mage immunotherapy, identified that certain genes were differentially expressed in patients that responded well to the treatment, in comparison to those patients who did not respond to the immunotherapy. The present inventors have discovered a gene signature/profile that is predictive of the likely response of the patient to an appropriate immunotherapy. More specifically the present inventors have discovered a gene signature that is predictive of improved survival after treatment with Mage antigen specific immunotherapy.

Thus the invention provides a gene profile, from a patient derived sample, which is indicative of an increased likelihood that the patient will be a responder (or alternatively a non-responder) to an immunotherapy, for example a cancer immunotherapy, such as Mage immunotherapy, wherein the profile comprises differential expression of at least one immune activation gene. The invention provides a gene profile, from a patient derived sample, which is indicative of an increased likelihood that the patient will be a responder to an immunotherapy, for example a cancer immunotherapy, such as Mage immunotherapy, wherein the profile comprises upregulation of at least one immune activation gene.

The present invention provides a predictive profile in constrast to a diagnostic or prognostic profile.

Whilst not wishing to be bound by theory it is hypothesised that the gene signature identified is in fact indicative of an immune/inflammatory, such as a T cell infiltration/activation response in the patients who are designated as responders, for example, the signature may represent a T-cell activation marker. The presence of this response is thought to assist the patient's body to fight the disease, such as cancer, after administration of the immunotherapy thereby rendering a patient more responsive to said immunotherapy.

Thus the signature of the present invention does not focus on markers/genes specifically associated with the diagnosis and/or prognosis of the relevant disease, for example cancer such as oncogenes, but rather is predictive of whether the patient will respond to an appropriate immunotherapy, such as cancer immunotherpay.

The gene profile identified herein for cancer and methods for identifying the same are thought to be indicative of the microenvironment of the tumor. The correct microenvironment of the tumor seems to be key to whether the patient responds to appropriate cancer immunotherapy. Immunotherapy in the context of the invention means therapy based on stimulating an immune response, generally to an antigen, wherein the response results in the treatment, amelioration and/or retardation of the progression of a disease associated therewith. Treatment in this context would not usually include prophylactic treatment.

Cancer immunotherapy in the context of this specification means immunotherapy for the treatment of cancer. In one aspect the immunotherapy is based on a cancer testis antigen, such as Mage (discussed in more detail below).

This invention may be used for identifying cancer patients that are likely to respond to appropriate immunotherapy, for example patients with melanoma, breast, bladder, lung, NSCLC, head and neck cancer, squamous cell carcinoma, colon carcinoma and oesophageal carcinoma, such as in patients with MAGE-expressing cancers. In an embodiment, the invention may be used in an adjuvant (post-operative, for example disease-free) setting in such cancers, particularly lung and melanoma. The invention also finds utility in the treatment of cancers in the metastatic setting.

Thus in a first aspect the invention provides a signature indicative of a patient, such as a cancer patient, designated a responder or non-responder to treatment with an appropriate immunotherapy, the signature comprising differential expression of one or more genes selected from immune activation/immune response/inflammatory response genes, for example, the group of genes indicative of T cell infiltration/activation, such as a gene listed (or a gene list comprising or consisting) those listed in Table 1, 2, 3, 4, 7, 9, 11, 12 and/or 13 such as Table 1 or 2. Differential expression in the context of the present invention means the gene is upregulated or downregulated in comparison to its normal expression. Statistical methods for calculating gene differentiation are discussed below.

Immune activation gene is intended to mean a gene that facilitates, increases or stimulates an appropriate immune response. Immune response gene and immune activation gene are used interchangeably herein.

An important technique for the analysis of the genes expressed by cells, such as cancer/tumour cells, is DNA microarray (also known as gene chip technology), where hundreds or more probe sequences (such as 55, 000 probe sets) are attached to a glass surface. The probe sequences are generally all 25 mers or 60 mers and are sequences from known genes. These probes are generally arranged in a set of 11 individual probes (a probe set) and are fixed in a predefined pattern on the glass surface. Once exposed to an appropriate biological sample these probes hybridise to the relevant RNA or DNA of a particular gene. After washing, the chip is "read" by an appropriate method and a quantity such as colour intensity recorded. The differential expression of a particular gene is proportional to the measure/intensity recorded. This technology is discussed in more detail below.

Once a target gene/profile has been identified there are several analytical methods to measure whether the gene(s) is/are differentially expressed. These analytical techniques include real-time polymerase chain reaction, also called quantitative real time polymerase chain reaction (QRT-PCR or Q-PCR), which is used to simultaneously quantify and amplify a specific part of a given DNA molecule present in the sample.

The procedure follows the general pattern of polymerase chain reaction, but the DNA is quantified after each round of amplification (the "real-time" aspect). Two common methods of quantification are the use of fluorescent dyes that intercalate with double-strand DNA, and modified DNA oligonucleotide probes that fluoresce when hybridized with a complementary DNA.

The basic idea behind real-time polymerase chain reaction is that the more abundant a particular cDNA (and thus mRNA) is in a sample, the earlier it will be detected during repeated cycles of amplification. Various systems exist which allow the amplification of DNA to be followed and they often involve the use of a fluorescent dye which is incorporated into newly synthesised DNA molecules during real-time amplification. Real-time polymerase chain reaction machines, which control the thermocycling process, can then detect the abundance of fluorescent DNA and thus the amplification progress of a given sample. Typically, amplification of a given cDNA over time follows a curve, with an initial flat-phase, followed by an exponential phase. Finally, as the experiment reagents are used up, DNA synthesis slows and the exponential curve flattens into a plateau.

Alternatively the mRNA or protein product of the target gene(s) may be measured by Northern Blot analysis,Western Blot and/or immunohistochemistry.

In one aspect the analysis to identify the profile/signature is performed on a patient sample wherein a cancer testis antigen is expressed.

If a gene is always upregulated or always down regulated in patients that are deemed to be responders (or alternatively non-responders) then this single gene can be used to establish if the patient is a responder or a non-responder once a threshold is established and provided the separation of the two groups is adequate.

When a single gene is analysed, for example, by Q-PCR then the gene expression can be normalised by reference to a gene that remains constant, for example genes with the symbol H3F3A, GAPDH, TFRC, GUSB or PGK1. The normalisation can be performed by substracting the value obtained for the constant gene from the value obtained for the gene under consideration. A threshold may be established by plotting a measure of the expression of the relevant gene for each patient. Generally the responders and the non-responders will be clustered about a different axis/focal point. A threshold can be established in the gap between the clusters by classical statistical methods or simply plotting a "best fit line" to establish the middle ground between the two groups. Values, for example, above the pre-defined threshold can be designated as responders and values, for example below the pre-designated threshold can be designated as non-responders.

In one aspect the invention provides a gene profile for identifying a responder comprising one or more of said genes wherein 50, 60, 70, 75, 80, 85, 90, 95, 99 or 100% of the genes are upregulated.

The genes listed in Table 1, 2, 3, 4, 7, 9, 11, 12 and/or 13 such as Table 1, 2 and/or 3 are generally upregulated in responders.

The robustness of the predictive method of the invention can be further improved for larger sample sizes by employing 2, 3, 4, 5, 6, etc genes from Table 1, 2, 3, 4, 7, 9, 11, 12 and/or 13 such as Table 1 or 2.

Furthermore, once at least two differential expressed genes are included in the signature then statistical clustering methods can be used to differentiate the responders and non-responders. Methods for statistical clustering and software for the same are discussed below.

One parameter used in quantifying the differential expression of genes is the fold change, which is a metric for comparing a gene's mRNA-expression level between two distinct experimental conditions. Its arithmetic definition differs between investigators. However, the higher the fold change the more likely that the differential expression of the relevant genes will be adequately separated, rendering it easier to decide which category (responder or non-responder) the patient falls into.

The fold change may, for example be at least 10, at least 15, at least 20 or 30.

Another parameter also used to quantify differential expression is the "p" value. It is thought that the lower the p value the more differentially expressed the gene is likely to be, which renders it a good candidate for use in profiles of the invention. P values may for example include 0.1 or less, such as 0.05 or less, in particular 0.01 or less. P values as used herein include corrected "P" values and/or also uncorrected "P" values.

The invention provides a method for the detection of a gene signature in a biological sample, the method comprising the analysis of the expression of at least 5 genes as set forth in Table 1. Alternatively one or more genes may be selected from Table 1, 2, 3, 4, 7, 9, 11, 12 and/or 13 such as Table 1 or 2.

Thus in one aspect the invention provides a method of identifying whether a cancer patient will be a responder or non-responder to immunotherapy, the method comprising:
1. analysing a sample comprising mRNA or fragments thereof expressed by genes of cancerous cells or DNA or fragments thereof from cancerous cells, for differential expression of one or more genes indicative of T-cell infiltration/activation, for example selected from the group comprising or consisting of genes listed in Table 1, 2, 3, 4, 7, 9, 11, 12 and/or 13 such as Table 1 or 2, and
2. characterising a patient as a responder or a non-responder based on the results of step 1.

Responder in the context of the present invention includes persons where the cancer/tumor(s) is irradicated, reduced or improved (mixed responder or partial responder) or simply stabilised such that the disease is not progressing. In responders where the cancer is stabilised then the period of stabilisation is such that the quality of life and/or patients life expectancy is increased (for example stable disease for more than 6 months) in comparison to a patient that does not receive treatment.

Partial clinical response in respect of cancer is wherein all of the tumors/cancers respond to treatment to some extent, for example where said cancer is reduced by 30, 40, 50, 60% or more. Mixed clinical responder in respect of cancer is defined as wherein some of the tumors/cancers respond to treatment and others remain unchanged or progress.

Standard definitions are available for responders, partial responders and mixed responders to cancer treatment. These standard definitions apply herein unless from the context it is clear that they do not apply.

As used herein, methods to predict a favorable clinical response or to identify subjects more likely to respond to therapy, is not meant to imply a 100% predictive ability, but to indicate that subjects with certain characteristics are more likely to experience a favorable clinical response to a specified therapy than subjects who lack such characteristics. However, as will be apparent to one skilled in the art, some individuals identified as more likely to experience a favorable clinical response may nonetheless fail to demonstrate measurable clinical response to the treatment. Similarly, some individuals predicted as non-responders may nonetheless exhibit a favorable clinical response to the treatment.

As used herein, a 'favorable response' (or 'favorable clinical response') to, for example, an anticancer treatment refers to a biological or physical response that is recognized by those skilled in the art as indicating a decreased rate of tumor growth, compared to tumor growth that would occur with an alternate treatment or the absence of any treatment. "Favorable clinical response" as used herein is not synonymous with a cure, but includes Partial Response, Mixed Response or Stable Disease. A favorable clinical response to therapy may include a lessening of symptoms experienced by the subj ect, an increase in the expected or achieved survival time, a decreased rate of tumor growth, cessation of tumor growth (stable disease), regression in the number or mass of metastatic lesions, and/or regression of the overall tumor mass (each as compared to that which would occur in the absence of therapy, or in response to an alternate therapy).

Patients in need of treatment for, for example, a Mage-expressing tumor, whose tumor cells have a gene signature described herein as a "Responder" signature are more likely to have a favorable clinical response, compared to patients whose tumor cells show a gene signature described herein as a "Non-Responder" signature, when treated with Mage specific immunotherapy. Non-responder in the context of this invention includes persons whose symptoms ie cancers/tumors are not improved or stabilised.

Optionally the characterisation of the patient as a responder or non-responder can be performed by reference to a "standard" or a training set. The standard may be the profile of a person/patient who is known to be a responder or non-responder or alternatively may be a numerical value. Such pre-determined standards may be provided in any suitable form, such as a printed list or diagram, computer software program, or other media.

Training set in the context of the present specification is intended to refer to a group of samples for which the clinical results can be correlated with the gene profile and can be employed for training an appropriate stastical model/programme to identify responders and/or non-responser for new samples. Tables 11A, 11B and 14 contain the training set information relevant to the 41 probe set model described in Example 4.

In one aspect a mathematical model/algorithm/statical method is employed to characterise the patient as responder or non-responder.

In one apect the invention provides a profile based one or more immune activation genes, such as 5 or more such genes.

In one aspect the invention provides a profile based on the genes in Table 1, 2, 3, 4, 7, 9, 11, 12 and/or 13 such as Table 1 or 2.

In one aspect the invention provides a profile based on 489 probes (listed in Table 4A) and/or approximately 480 genes as listed in Table 4.

According to one aspect the present invention provides a gene signature indicative of improved survival of patients with Mage expressing cancers following treatment with Mage specific immunotherapy. This gene signature, of at least 1, 2, 3, 4, 5, 6, 7, 8, 9, 10, 11, 12, 13, 14, 15, 16,17, 18, 19, 20 21, 22, 23, 24, 25, 26, 27, 28 , 29, 30, or 31, genes from the genes disclosed in Table 1, is characterised by differential expression compared to the gene signature of MAGE-expressing tumour patients who do not respond to Mage antigen specific cancer immunotherapy. Improved survival in likely to be a corollary of a response to the immunotherapy administered, if the patient is in fact a responder.

### TABLES WITH GENE LISTS

In one aspect the invention relates to one or more genes listed in Table 1

**Table 1**

| | **Gene Title** | **Seq Id No** |
|---|---|---|
| 1.1 | chromosome 6 open reading frame 190 | 1 |
| 1.2 | hematopoietic cell-specific Lyn substrate 1 | 2 |
| 1.3 | interleukin 2 receptor, gamma (severe combined immunodeficiency) | 3 |
| 1.4 | CD52 antigen (CAMPATH-1 antigen) /// CD52 antigen (CAMPATH-1 antigen) | 4 |
| 1.5 | CD2 antigen (p50), sheep red blood cell receptor /// CD2 antigen (p50), sheep red blood cell receptor | 5 |
| 1.6 | ubiquitin D | 6 |
| 1.7 | signal transducer and activator of transcription 4 | 7 |
| 1.8 | granzyme K (granzyme 3; tryptase II)///granzyme K (granzyme 3; tryptase II) | 8 |
| 1.9 | CD3G antigen, gamma polypeptide (TiT3 complex) | 9 |
| 1.10 | G protein-coupled receptor 171 | 10 |
| 1.11 | Protein kinase C, beta 1 | 11 |
| 1.12 | major histocompatibility complex, class II, DR alpha /// major histocompatibility complex, class II, DR alpha | 12 |
| 1.13 | Major histocompatibility complex, class II, DQ beta 1 /// Major histocompatibility complex, class II, DQ beta 1 | 13 |
| 1.14 | alcohol dehydrogenase IB (class I), beta polypeptide | 14 |
| 1.15 | T cell receptor alpha constant /// T cell receptor alpha constant | 15 |
| 1.16 | CD69 antigen (p60, early T-cell activation antigen) | 16 |
| 1.17 | protein kinase C, theta | 17 |
| 1.18 | T cell receptor beta variable 19 /// T cell receptor beta constant 1 | 18 |
| 1.19 | T cell receptor alpha locus /// T cell receptor delta variable 2 /// T cell receptor alpha variable 20 /// T cell receptor alpha joining 17 /// T cell receptor alpha constant | 19 |
| 1.20 | T cell receptor gamma constant 2 | 20 |
| 1.21 | T cell receptor beta variable 21-1 /// T cell receptor beta variable 19 /// T cell receptor beta variable 5-4 /// T cell receptor beta variable 3-1 /// T cell receptor beta constant 1 | 21 |
| 1.22 | T cell receptor alpha locus | 22 |
| 1.23 | T cell receptor beta variable 19 /// T cell receptor beta variable 19 /// T cell receptor beta constant 1 /// T cell receptor beta constant 1 | 23 |
| 1.24 | CD3D antigen, delta polypeptide (TiT3 complex) | 24 |
| 1.25 | T cell receptor gamma constant 2 /// T cell receptor gamma variable 9 /// similar to T-cell receptor gamma chain C region PT-gamma-1/2 /// similar to T-cell receptor gamma chain V region PT-gamma-1/2 precursor /// TCR gamma alternate reading frame protein | 25 |
| 1.26 | pyrin and HIN domain family, member 1 | 26 |
| 1.27 | T cell receptor associated transmembrane adaptor 1 | 27 |
| 1.28 | SLAM family member 7 | 28 |
| 1.29 | chromosome 4 open reading frame 7 | 29 |
| 1.30 | Major histocompatibility complex, class II, DQ alpha 1 | 30 |
| 1.31 | Transcribed locus | 31 |
| 1.32 | Amphiphysin (Stiff-Man syndrome with breast cancer 128kDa autoantigen) | 32 |
| 1.33 | dendritic cell-associated lectin-1 | 33 |

Table 1A at the end of this specification gives the full nucleotide sequence for each of the above genes.

In one aspect the invention provides a profile based on differential expression of 1 or more of 62 genes, identified in the literature, that relate to immune infiltration and activation.

In one aspect the invention provides a profile based on the genes listed in Table 2.

**Table 2:**

| | **GeneSymbol** | **Gene Title** | **Seq Id No** |
|---|---|---|---|
| 2.1 | HLA-DQA1 | major histocompatibility complex, class II, DQ alpha 1 | 30 |
| 2.2 | TRBV3-1 | T cell receptor beta variable 3-1 | 21 |
| 2.3 | IL2RG | interleukin 2 receptor, gamma (severe combined immunodeficiency | 3 |
| 2.4 | CD2 | CD2 antigen (p50), sheep red blood cell receptor | 5 |
| 2.5 | GPR171 | G protein-coupled receptor 171 | 10 |
| 2.6 | ADH1B | alcohol dehydrogenase IB (class I), beta polypeptide | 14 |
| 2.7 | CD69 | CD69 antigen (p60, early T-cell activation antigen) | 16 |
| 2.8 | TRBV19 | T cell receptor beta variable 19 | 18 |
| 2.9 | TRAT1 | T cell receptor associated transmembrane adaptor 1 | 27 |
| 2.10 | C4orf7 | chromosome 4 open reading frame 7 | 29 |
| 2.11 | PRKCB1 | protein kinase C, beta 1 | 11 |
| 2.12 | CD3D | CD3D antigen, delta polypeptide (TiT3 complex) | 24 |
| 2.13 | UBD | ubiquitin D | 6 |

In one aspect the invention provides the list of genes in Table 3.

**Table 3**

| | **Gene Symbol** | **Gene Title** |
|---|---|---|
| 3.1 | CD52 | CD52 molecule /// CD52 molecule |
| 3.2 | UBD | gamma-aminobutyric acid (GABA) B receptor, 1 /// ubiquitin D |
| 3.3 | STAT4 | signal transducer and activator of transcription 4 |
| 3.4 | GZMK | granzyme K (granzyme 3; tryptase II) /// granzyme K (granzyme 3; tryptase II) |
| 3.5 | GPR171 | G protein-coupled receptor 171 |
| 3.6 | PRKCQ | protein kinase C, theta |
| 3.7 | TRA@ /// TRDV2 /// TRAV20 /// TRAC | T cell receptor alpha locus /// T cell receptor delta variable 2 /// T cell receptor alpha variable 20 /// T cell receptor alpha constant |
| 3.8 | TRGC2 /// TRGV2 /// TRGV9 /// TARP /// LOC642083 | T cell receptor gamma constant 2 /// T cell receptor gamma variable 2 /// T cell receptor gamma variable 9 /// TCR gamma alternate reading frame protein /// hypothetical protein LOC642083 |
| 3.9 | TRBV21-1 /// TRBV19 /// TRBV5-4 /// TRBV3-1 /// TRBC1 | T cell receptor beta variable 21-1 /// T cell receptor beta variable 19 /// T cell receptor beta variable 5-4 /// T cell receptor beta variable 3-1 /// T cell receptor beta constant 1 /// similar to T-cell receptor beta chain V region CTL-L17 precursor |
| 3.10 | TRBV19 /// TRBC1 | T cell receptor beta variable 19 /// T cell receptor beta variable 19 /// T cell receptor beta constant 1 /// T cell receptor beta constant 1 |
| 3.11 | CD3D | CD3d molecule, delta (CD3-TCR complex) |
| 3.12 | TRAT1 | T cell receptor associated transmembrane adaptor 1 |

In one aspect the invention provides a profile based on one or more genes of Table 4

**Table 4**

| | **GeneSymbol** | **Gene Title** |
|---|---|---|
| 4.1 | FLJ20647 | NA |
| 4,2 | NAV1 | neuron navigator 1 |
| 4.3 | GPR171 | G protein-coupled receptor 171 |
| 4.4 | CCL14 | chemokine (C-C motif) ligand 14 |
| 4.5 | C1S | complement component 1, s subcomponent |
| 4.6 | CXCL2 | chemokine (C-X-C motif) ligand 2 |
| 4.7 | TRBV3-1 | T cell receptor beta variable 3-1 |
| 4.8 | TRDV2 | T cell receptor delta variable 2 |
| 4.9 | RUFY3 | RUN and FYVE domain containing 3 |
| 4.10 | DOCK8 | dedicator of cytokinesis 8 |
| 4.11 | GCH1 | GTP cyclohydrolase 1 (dopa-responsive dystonia) |
| 4.12 | CENTD3 | centaurin, delta 3 |
| 4.13 | ACSL5 | acyl-CoA synthetase long-chain family member 5 |
| 4.14 | AMICA1 | adhesion molecule, interacts with CXADR antigen 1 |
| 4.15 | IL2RG | interleukin 2 receptor, gamma (severe combined immunodeficiency) |
| 4.16 | TNFAIP3 | tumor necrosis factor, alpha-induced protein 3 |
| 4.17 | PSCDBP | pleckstrin homology, Sec7 and coiled-coil domains, binding protein |
| 4.18 | ESR1 | estrogen receptor 1 |
| 4.19 | TRBC1 | T cell receptor beta constant 1 |
| 4.20 | CD52 | CD52 antigen (CAMPATH-1 antigen) |
| 4.21 | LOC442535 | NA |
| 4.22 | TRBV19 | T cell receptor beta variable 19 |
| 4.23 | IL7R | interleukin 7 receptor |
| 4.24 | TRAC | T cell receptor alpha constant |
| 4.25 | NCF2 | neutrophil cytosolic factor 2 (65kDa, chronic granulomatous disease, autosomal 2) |
| 4.26 | LOC92689 | NA |
| 4.27 | GZMK | c("granzyme K (granzyme 3", " tryptase II)") |
| 4.28 | NA | NA (gene identified by probe 235831_at) |
| 4.29 | RAB34 | RAB34, member RAS oncogene family |
| 4.30 | DPT | dermatopontin |
| 4.31 | PVT1 | Pvt1 oncogene homolog, MYC activator (mouse) |
| 4.32 | TRGC2 | T cell receptor gamma constant 2 |
| 4.33 | GIMAP5 | GTPase, IMAP family member 5 |
| 4.34 | CD52 | CD52 antigen (CAMPATH-1 antigen) |
| 4.35 | CD3D | CD3d antigen, delta polypeptide (TiT3 complex) |
| 4.36 | TMEM132C | transmembrane protein 132C |
| 4.37 | NFKBIA | nuclear factor of kappa light polypeptide gene enhancer in B-cells inhibitor, alpha |
| 4.38 | TRA@ | T cell receptor alpha locus |
| 4.39 | TRAT1 | T cell receptor associated transmembrane adaptor 1 |
| 4.40 | LOC442535 | NA |
| 4.41 | RAB34 | RAB34, member RAS oncogene family |
| 4.42 | CD69 | CD69 antigen (p60, early T-cell activation antigen) |
| 4.43 | DOCK8 | dedicator of cytokinesis 8 |
| 4.44 | GIMAP5 | GTPase, IMAP family member 5 |
| 4.45 | IRF8 | interferon regulatory factor 8 |
| 4.46 | KLRB1 | killer cell lectin-like receptor subfamily B, member 1 |
| 4.47 | NA | NA |
| 4.48 | ITGA3 | integrin, alpha 3 (antigen CD49C, alpha 3 subunit of VLA-3 receptor) |
| 4.49 | MAP3K8 | mitogen-activated protein kinase kinase kinase 8 |
| 4.50 | C1orf162 | chromosome 1 open reading frame 162 |
| 4.51 | UBD | ubiquitin D |
| 4.52 | TRGV9 | T cell receptor gamma variable 9 |
| 4.53 | NAV1 | neuron navigator 1 |
| 4.54 | ARHGAP9 | Rho GTPase activating protein 9 |
| 4.55 | TIFA | NA |
| 4.56 | DPT | dermatopontin |
| 4.57 | NA | NA (gene identified by probe 1569942_at) |
| 4.58 | GIMAP4 | GTPase, IMAP family member 4 |
| 4.59 | HCLS1 | hematopoietic cell-specific Lyn substrate 1 |
| 4.60 | PRKCH | protein kinase C, eta |
| 4.61 | STAT4 | signal transducer and activator of transcription 4 |
| 4.62 | HLA-DQA1 | major histocompatibility complex, class II, DQ alpha 1 |
| 4.63 | ADRB2 | adrenergic, beta-2-, receptor, surface |
| 4.64 | NA | NA |
| 4.65 | CTSW | cathepsin W (lymphopain) |
| 4.66 | MYH11 | myosin, heavy polypeptide 11, smooth muscle |
| 4.67 | GIMAP6 | GTPase, IMAP family member 6 |
| 4.68 | HLA-DQB1 | major histocompatibility complex, class II, DQ beta 1 |
| 4.69 | CD8A | CD8 antigen, alpha polypeptide (p32) |
| 4.70 | TNFAIP3 | tumor necrosis factor, alpha-induced protein 3 |
| 4.71 | CP | ceruloplasmin (ferroxidase) |
| 4.72 | SMOC2 | SPARC related modular calcium binding 2 |
| 4.73 | C20orf24 | chromosome 20 open reading frame 24 |
| 4.74 | C16orf54 | chromosome 16 open reading frame 54 |
| 4.75 | CD2 | CD2 antigen (p50), sheep red blood cell receptor |
| 4.76 | SLIT3 | slit homolog 3 (Drosophila) |
| 4.77 | BAALC | brain and acute leukemia, cytoplasmic |
| 4.78 | TRIB3 | tribbles homolog 3 (Drosophila) |
| 4.79 | LOC440160 | NA |
| 4.80 | C6orf190 | chromosome 6 open reading frame 190 |
| 4.81 | TAGAP | T-cell activation GTPase activating protein |
| 4.82 | FAM92A1 | family with sequence similarity 92, member A1 |
| 4.83 | PSTPIP2 | proline-serine-threonine phosphatase interacting protein 2 |
| 4.84 | PTPRC | protein tyrosine phosphatase, receptor type, C |
| 4.85 | HLA-DRA | major histocompatibility complex, class II, DR alpha |
| 4.86 | EFCAB2 | EF-hand calcium binding domain 2 |
| 4.87 | TNFAIP8 | tumor necrosis factor, alpha-induced protein 8 |
| 4.88 | SLIC1 | NA |
| 4.89 | CD1C | CD1c antigen |
| 4.90 | TRAF3IP3 | TRAF3 interacting protein 3 |
| 4.91 | HLA-DQA1 | major histocompatibility complex, class II, DQ alpha 1 |
| 4.92 | NAV1 | neuron navigator 1 |
| 4.93 | HLA-DQB1 | major histocompatibility complex, class II, DQ beta 1 |
| 4.94 | PTPRC | protein tyrosine phosphatase, receptor type, C |
| 4.95 | IGJ | immunoglobulin J polypeptide, linker protein for immunoglobulin alpha and mu polypeptides |
| 4.96 | PLEK | pleckstrin |
| 4.97 | TRA@ | T cell receptor alpha locus |
| 4.98 | TMEM44 | transmembrane protein 44 |
| 4.99 | TRA@ | T cell receptor alpha locus |
| 4.100 | EBI2 | Epstein-Barr virus induced gene 2 (lymphocyte-specific G protein-coupled receptor) |
| 4.101 | SAMSN1 | SAM domain, SH3 domain and nuclear localisation signals, 1 |
| 4.102 | KIAA1794 | KIAA1794 |
| 4.103 | ALDH2 | aldehyde dehydrogenase 2 family (mitochondrial) |
| 4.104 | CDC42SE2 | CDC42 small effector 2 |
| 4.105 | GFRA1 | GDNF family receptor alpha 1 |
| 4.106 | ITK | IL2-inducible T-cell kinase |
| 4.107 | HLA-DRA | major histocompatibility complex, class II, DR alpha |
| 4.108 | GIMAP7 | GTPase, IMAP family member 7 |
| 4.109 | FLJ20273 | NA |
| 4.110 | PTPN6 | protein tyrosine phosphatase, non-receptor type 6 |
| 4.111 | PTGER3 | prostaglandin E receptor 3 (subtype EP3) |
| 4.112 | RAI2 | retinoic acid induced 2 |
| 4.113 | LGALS2 | lectin, galactoside-binding, soluble, 2 (galectin 2) |
| 4.114 | HMOX1 | heme oxygenase (decycling) 1 |
| 4.115 | NA | NA (gene identified by probe 227995_at) |
| 4.116 | ZNFN1A1 | zinc finger protein, subfamily 1 A, 1 (Ikaros) |
| 4.117 | CSF2RB | colony stimulating factor 2 receptor, beta, low-affinity (granulocyte-macrophage) |
| 4.118 | PCSK5 | proprotein convertase subtilisin/kexin type 5 |
| 4.119 | CCDC69 | coiled-coil domain containing 69 |
| 4.120 | CDC42SE2 | CDC42 small effector 2 |
| 4.121 | GZMA | granzyme A (granzyme 1, cytotoxic T-lymphocyte-associated serine esterase 3) |
| 4.122 | C3 | complement component 3 |
| 4.123 | TNFAIP8 | tumor necrosis factor, alpha-induced protein 8 |
| 4.124 | C15orf48 | chromosome 15 open reading frame 48 |
| 4.125 | RARRES3 | retinoic acid receptor responder (tazarotene induced) 3 |
| 4.126 | LOC283537 | NA |
| 4.127 | CXCL12 | chemokine (C-X-C motif) ligand 12 (stromal cell-derived factor 1) |
| 4.128 | NAV1 | neuron navigator 1 |
| 4.129 | NA | NA (gene identified by probe set 231882_at) |
| 4.130 | SOD2 | superoxide dismutase 2, mitochondrial |
| 4.131 | CTSS | cathepsin S |
| 4.132 | CTBP2 | C-terminal binding protein 2 |
| 4.133 | BCL11B | B-cell CLL/lymphoma 11B (zinc finger protein) |
| 4.134 | CCL22 | chemokine (C-C motif) ligand 22 |
| 4.135 | ACSL5 | acyl-CoA synthetase long-chain family member 5 |
| 4.136 | DOC1 | NA |
| 4.137 | SLC31A2 | solute carrier family 31 (copper transporters), member 2 |
| 4.138 | POPDC3 | popeye domain containing 3 |
| 4.139 | DOC1 | NA |
| 4.140 | SQRDL | sulfide quinone reductase-like (yeast) |
| 4.141 | RASGEF1B | RasGEF domain family, member 1B |
| 4.142 | FGL2 | fibrinogen-like 2 |
| 4.143 | C10orf128 | chromosome 10 open reading frame 128 |
| 4.144 | IL10RA | interleukin 10 receptor, alpha |
| 4.145 | EGFL6 | EGF-like-domain, multiple 6 |
| 4.146 | IL18 | interleukin 18 (interferon-gamma-inducing factor) |
| 4.147 | ARHGAP30 | Rho GTPase activating protein 30 |
| 4.148 | PALMD | palmdelphin |
| 4.149 | RASSF5 | Ras association (RalGDS/AF-6) domain family 5 |
| 4.150 | GATA3 | GATA binding protein 3 |
| 4.151 | DKFZP564O0823 | NA |
| 4.152 | BCL11B | B-cell CLL/lymphoma 11B (zinc finger protein) |
| 4.153 | TXNIP | thioredoxin interacting protein |
| 4.154 | DTX4 | deltex 4 homolog (Drosophila) |
| 4.155 | DARC | Duffy blood group, chemokine receptor |
| 4.156 | RNASE6 | ribonuclease, RNase A family, k6 |
| 4.157 | CD86 | CD86 antigen (CD28 antigen ligand 2, B7-2 antigen) |
| 4.158 | ZFP36 | zinc finger protein 36, C3H type, homolog (mouse) |
| 4.159 | BASP1 | brain abundant, membrane attached signal protein 1 |
| 4.160 | CKAP1 | cytoskeleton associated protein 1 |
| 4.161 | HCP5 | HLA complex P5 |
| 4.162 | GRB14 | growth factor receptor-bound protein 14 |
| 4.163 | GJA7 | gap junction protein, alpha 7, 45kDa (connexin 45) |
| 4.164 | FLJ14054 | NA |
| 4.165 | VNN1 | vanin 1 |
| 4.166 | ADCY7 | adenylate cyclase 7 |
| 4.167 | MS4A6A | membrane-spanning 4-domains, subfamily A, member 6A |
| 4.168 | CPA3 | carboxypeptidase A3 (mast cell) |
| 4.169 | PIM1 | pim-1 oncogene |
| 4.170 | CCL19 | chemokine (C-C motif) ligand 19 |
| 4.171 | SYK | spleen tyrosine kinase |
| 4.172 | NAV1 | neuron navigator 1 |
| 4.173 | SIT1 | signaling threshold regulating transmembrane adaptor 1 |
| 4.174 | NA | NA (gene identified by probe set 228812_at) |
| 4.175 | NAP1L2 | nucleosome assembly protein 1-like 2 |
| 4.176 | CCL13 | chemokine (C-C motif) ligand 13 |
| 4.177 | SLA | Src-like-adaptor |
| 4.178 | NOD3 | NA |
| 4.179 | PRKCH | protein kinase C, eta |
| 4.180 | TRD@ | T cell receptor delta locus |
| 4.181 | BAALC | brain and acute leukemia, cytoplasmic |
| 4.182 | RP1-93H18.5 | NA |
| 4.183 | FLJ20701 | NA |
| 4.184 | SH3TC2 | SH3 domain and tetratricopeptide repeats 2 |
| 4.185 | CCR2 | chemokine (C-C motif) receptor 2 |
| 4.186 | CCL5 | chemokine (C-C motif) ligand 5 |
| 4.187 | HLA-DPA1 | major histocompatibility complex, class II, DP alpha 1 |
| 4.188 | MS4A6A | membrane-spanning 4-domains, subfamily A, member 6A |
| 4.189 | PECAM1 | platelet/endothelial cell adhesion molecule (CD31 antigen) |
| 4.190 | AMIGO2 | adhesion molecule with Ig-like domain 2 |
| 4.191 | CCDC69 | coiled-coil domain containing 69 |
| 4.192 | CLEC7A | C-type lectin domain family 7, member A |
| 4.193 | P2RY14 | purinergic receptor P2Y, G-protein coupled, 14 |
| 4.194 | PIK3API | phosphoinositide-3-kinase adaptor protein 1 |
| 4.195 | ADH1B | alcohol dehydrogenase IB (class I), beta polypeptide |
| 4.196 | TOP1MT | topoisomerase (DNA) I, mitochondrial |
| 4.197 | CD276 | CD276 antigen |
| 4.198 | HLA-DQB1 | major histocompatibility complex, class II, DQ beta 1 |
| 4.199 | JAM2 | junctional adhesion molecule 2 |
| 4.200 | C1S | complement component 1, s subcomponent |
| 4.201 | MS4A6A | membrane-spanning 4-domains, subfamily A, member 6A |
| 4.202 | TGFBR3 | transforming growth factor, beta receptor III (betaglycan, 300kDa) |
| 4.203 | ITGAL | c(" integrin, alpha L (antigen CD11A (p180), lymphocyte function-associated antigen 1", "alpha polypeptide)") |
| 4.204 | IL1R1 | interleukin 1 receptor, type I |
| 4.205 | MS4A6A | membrane-spanning 4-domains, subfamily A, member 6A |
| 4.206 | HLA-DRB1 | major histocompatibility complex, class II, DR beta 1 |
| 4.207 | GIMAP2 | GTPase, IMAP family member 2 |
| 4.208 | ZC3H12D | zinc finger CCCH-type containing 12D |
| 4.209 | PCDH9 | protocadherin 9 |
| 4.210 | SLAMF7 | SLAM family member 7 |
| 4.211 | MGC7036 | NA |
| 4.212 | RGS18 | regulator of G-protein signalling 18 |
| 4.213 | HLA-DQA1 | major histocompatibility complex, class II, DQ alpha 1 |
| 4.214 | CD53 | CD53 antigen |
| 4.215 | MPEG1 | NA |
| 4.216 | SSBP4 | single stranded DNA binding protein 4 |
| 4.217 | NA | NA (gene identified by probe set 231262_at) |
| 4.218 | CDH19 | cadherin 19, type 2 |
| 4.219 | CTBP2 | C-terminal binding protein 2 |
| 4.220 | NAV1 | neuron navigator 1 |
| 4.221 | FAM107B | family with sequence similarity 107, member B |
| 4.222 | IGKC | immunoglobulin kappa constant |
| 4.223 | ITGAM | integrin, alpha M (complement component 3 receptor 3 subunit) |
| 4.224 | CKAP1 | cytoskeleton associated protein 1 |
| 4.225 | HLA-DRB1 | major histocompatibility complex, class II, DR beta 1 |
| 4,226 | CDH19 | cadherin 19, type 2 |
| 4.227 | MGC16291 | NA |
| 4.228 | DDEF2 | development and differentiation enhancing factor 2 |
| 4.229 | TNFAIP2 | tumor necrosis factor, alpha-induced protein 2 |
| 4.230 | CXCL14 | chemokine (C-X-C motif) ligand 14 |
| 4.231 | CD209 | CD209 antigen |
| 4.232 | COL9A3 | collagen, type IX, alpha 3 |
| 4.233 | ANKRD22 | ankyrin repeat domain 22 |
| 4.234 | NCKAP1L | NCK-associated protein 1-like |
| 4.235 | CMKOR1 | chemokine orphan receptor 1 |
| 4.236 | HLA-DRB5 | major histocompatibility complex, class II, DR beta 5 |
| 4.237 | LCP1 | lymphocyte cytosolic protein 1 (L-plastin) |
| 4.238 | ADH1B | alcohol dehydrogenase IB (class I), beta polypeptide |
| 4.239 | CXXC5 | CXXC finger 5 |
| 4.240 | GJA7 | gap junction protein, alpha 7, 45kDa (connexin 45) |
| 4.241 | FGD2 | FYVE, RhoGEF and PH domain containing 2 |
| 4.242 | MAN1A1 | mannosidase, alpha, class 1A, member 1 |
| 4.243 | C6orf115 | chromosome 6 open reading frame 115 |
| 4.244 | RP1-93H18.5 | NA |
| 4.245 | CXCL9 | chemokine (C-X-C motif) ligand 9 |
| 4.246 | FAM107B | family with sequence similarity 107, member B |
| 4.247 | NPR3 | natriuretic peptide receptor C/guanylate cyclase C (atrionatriuretic peptide receptor C) |
| 4.248 | FYB | FYN binding protein (FYB-120/130) |
| 4.249 | VCAM1 | vascular cell adhesion molecule 1 |
| 4.250 | FLI1 | Friend leukemia virus integration 1 |
| 4.251 | CXXC5 | CXXC finger 5 |
| 4.252 | TRAM2 | translocation associated membrane protein 2 |
| 4.253 | IGKC | immunoglobulin kappa constant |
| 4.254 | SHC4 | SHC (Src homology 2 domain containing) family, member 4 |
| 4.255 | SLC9A9 | solute carrier family 9 (sodium/hydrogen exchanger), member 9 |
| 4.256 | PTPRC | protein tyrosine phosphatase, receptor type, C |
| 4.257 | PTGER4 | prostaglandin E receptor 4 (subtype EP4) |
| 4.258 | LILRB1 | leukocyte immunoglobulin-like receptor, subfamily B (with TM and ITIM domains), member 1 |
| 4.259 | PRDM1 | PR domain containing 1, with ZNF domain |
| 4.260 | RP1-93H18.5 | NA |
| 4.261 | ARHGAP15 | Rho GTPase activating protein 15 |
| 4.262 | SLC5A3 | solute carrier family 5 (inositol transporters), member 3 |
| 4.263 | DOCK9 | dedicator of cytokinesis 9 |
| 4.264 | GPSM1 | G-protein signalling modulator 1 (AGS3-like, C. elegans) |
| 4.265 | CCL5 | chemokine (C-C motif) ligand 5 |
| 4.266 | GLIPR1 | GLI pathogenesis-related 1 (glioma) |
| 4.267 | APOL3 | apolipoprotein L, 3 |
| 4.268 | HLA-DM | major histocompatibility complex, class II, DM beta |
| 4.269 | SYNPO2 | synaptopodin 2 |
| 4.270 | NA | NA (gene identified by probe set 221651_x_at) |
| 4.271 | NA | NA (gene identified by probe set 231929_at) |
| 4.272 | RP1-93H18.5 | NA |
| 4.273 | CASP1 | caspase 1, apoptosis-related cysteine peptidase (interleukin 1, beta, convertase) |
| 4.274 | PRKCQ | protein kinase C, theta |
| 4.275 | IL1R2 | interleukin 1 receptor, type II |
| 4.276 | CARD15 | caspase recruitment domain family, member 15 |
| 4.277 | ARHGDIB | Rho GDP dissociation inhibitor (GDI) beta |
| 4.278 | HLA-DRB4 | major histocompatibility complex, class II, DR beta 4 |
| 4.279 | SART2 | squamous cell carcinoma antigen recognized by T cells 2 |
| 4.280 | LSP1 | lymphocyte-specific protein 1 |
| 4.281 | AMPD3 | adenosine monophosphate deaminase (isoform E) |
| 4.282 | SEMA4F | sema domain, immunoglobulin domain (Ig), transmembrane domain (TM) and short cytoplasmic domain, (semaphorin) 4F |
| 4.283 | ISOC1 | isochorismatase domain containing 1 |
| 4.284 | CCL5 | chemokine (C-C motif) ligand 5 |
| 4.285 | HPS3 | Hermansky-Pudlak syndrome 3 |
| 4.286 | HLA-DPA1 | major histocompatibility complex, class II, DP alpha 1 |
| 4.287 | HLA-DQB1 | major histocompatibility complex, class II, DQ beta 1 |
| 4.288 | HOXB7 | homeobox B7 |
| 4.289 | FGL2 | fibrinogen-like 2 |
| 4.290 | ZNFN1A1 | zinc finger protein, subfamily 1 A, 1 (Ikaros) |
| 4.291 | ARHGAP9 | Rho GTPase activating protein 9 |
| 4.292 | GATA2 | GATA binding protein 2 |
| 4.293 | AP2B1 | adaptor-related protein complex 2, beta 1 subunit |
| 4.294 | CTSC | cathepsin C |
| 4.295 | PLK2 | polo-like kinase 2 (Drosophila) |
| 4.296 | CD4 | CD4 antigen (p55) |
| 4.297 | GGTA1 | glycoprotein, alpha-galactosyltransferase 1 |
| 4.298 | GADD45B | growth arrest and DNA-damage-inducible, beta |
| 4.299 | GADD45B | growth arrest and DNA-damage-inducible, beta |
| 4.300 | FLJ10847 | NA |
| 4.301 | KIF21B | kinesin family member 21B |
| 4.302 | CCND2 | cyclin D2 |
| 4.303 | PRG1 | proteoglycan 1, secretory granule |
| 4.304 | SLC40A1 | solute carrier family 40 (iron-regulated transporter), member 1 |
| 4.305 | HLA-DPA1 | major histocompatibility complex, class II, DP alpha 1 |
| 4.306 | SOD2 | superoxide dismutase 2, mitochondrial |
| 4.307 | CRIP1 | cysteine-rich protein 1 (intestinal) |
| 4.308 | LOC283070 | NA |
| 4.309 | SIGLEC1 | sialic acid binding Ig-like lectin 1, sialoadhesin |
| 4.310 | ZNF11B | zinc finger protein 11B |
| 4.311 | CXCR4 | chemokine (C-X-C motif) receptor 4 |
| 4.312 | HLA-DNA | major histocompatibility complex, class II, DM alpha |
| 4.313 | MRC1 | mannose receptor, C type 1 |
| 4.314 | CASP1 | caspase 1, apoptosis-related cysteine peptidase (interleukin 1, beta, convertase) |
| 4.315 | LMO2 | LIM domain only 2 (rhombotin-like 1) |
| 4.315a | DENND2D | DENN/MADD domain containing 2D |
| 4.316 | CCL18 | chemokine (C-C motif) ligand 18 (pulmonary and activation-regulated) |
| 4.317 | P2RY13 | purinergic receptor P2Y, G-protein coupled, 13 |
| 4.318 | CCL18 | chemokine (C-C motif) ligand 18 (pulmonary and activation-regulated) |
| 4.319 | ANGPTL1 | angiopoietin-like 1 |
| 4.320 | NA | NA (gene identified by probe set 230391_at) |
| 4.321 | ITGAL | c(" integrin, alpha L (antigen CD11A (p180), lymphocyte function-associated antigen 1", " alpha polypeptide)") |
| 4.322 | C8orf51 | chromosome 8 open reading frame 51 |
| 4.323 | GIMAP8 | GTPase, IMAP family member 8 |
| 4.324 | NA | NA (gene identified by probe set 227780_s_at) |
| 4.325 | JAK2 | Janus kinase 2 (a protein tyrosine kinase) |
| 4.326 | TNFSF10 | tumor necrosis factor (ligand) superfamily, member 10 |
| 4.327 | C1R | complement component 1, r subcomponent |
| 4.328 | ACPL2 | acid phosphatase-like 2 |
| 4.329 | TNFRSF19 | tumor necrosis factor receptor superfamily, member 19 |
| 4.330 | PCSK5 | proprotein convertase subtilisin/kexin type 5 |
| 4.331 | LRP12 | low density lipoprotein-related protein 12 |
| 4.332 | NA | NA (gene identified by probe set 1557116_at) |
| 4.333 | PECAM1 | platelet/endothelial cell adhesion molecule (CD31 antigen) |
| 4.334 | PRKCB1 | protein kinase C, beta 1 |
| 4.335 | IPO11 | importin 11 |
| 4.336 | DLGAP1 | discs, large (Drosophila) homolog-associated protein 1 |
| 4.337 | PRKAR2B | protein kinase, cAMP-dependent, regulatory, type II, beta |
| 4.338 | MAP3K8 | mitogen-activated protein kinase kinase kinase 8 |
| 4.339 | EVI2B | ecotropic viral integration site 2B |
| 4.340 | GBP1 | guanylate binding protein 1, interferon-inducible, 67kDa |
| 4.341 | CXCL10 | chemokine (C-X-C motif) ligand 10 |
| 4.342 | CAMK2N1 | calcium/calmodulin-dependent protein kinase II inhibitor 1 |
| 4.343 | MED12L | mediator of RNA polymerase II transcription, subunit 12 homolog (yeast)-like |
| 4.344 | ID2 | inhibitor of DNA binding 2, dominant negative helix-loop-helix protein |
| 4.345 | CTBP2 | C-terminal binding protein 2 |
| 4.346 | IGLJ3 | immunoglobulin lambda joining 3 |
| 4.347 | GBP4 | guanylate binding protein 4 |
| 4.348 | LOC439949 | NA |
| 4.349 | FBXO16 | F-box protein 16 |
| 4.350 | PRF1 | perforin 1 (pore forming protein) |
| 4.351 | TRAM2 | translocation associated membrane protein 2 |
| 4.352 | LYN | v-yes-1 Yamaguchi sarcoma viral related oncogene homolog |
| 4.353 | CENTD1 | centaurin, delta 1 |
| 4.354 | CASP1 | caspase 1, apoptosis-related cysteine peptidase (interleukin 1, beta, convertase) |
| 4.355 | FLJ20273 | NA |
| 4.356 | TFEC | transcription factor EC |
| 4.357 | PPP1R16B | protein phosphatase 1, regulatory (inhibitor) subunit 16B |
| 4.358 | CD48 | CD48 antigen (B-cell membrane protein) |
| 4.359 | HLA-DPB1 | major histocompatibility complex, class II, DP beta 1 |
| 4.360 | SHC4 | SHC (Src homology 2 domain containing) family, member 4 |
| 4.361 | GTPBP5 | GTP binding protein 5 (putative) |
| 4.362 | GBP5 | guanylate binding protein 5 |
| 4.363 | MAP1B | microtubule-associated protein 1B |
| 4.364 | EXTL3 | exostoses (multiple)-like 3 |
| 4.365 | CORO1A | coronin, actin binding protein, 1A |
| 4.366 | PDGFRL | platelet-derived growth factor receptor-like |
| 4.367 | RP9 | retinitis pigmentosa 9 (autosomal dominant) |
| 4.368 | RHOU | ras homolog gene family, member U |
| 4.369 | MTAC2D1 | membrane targeting (tandem) C2 domain containing 1 |
| 4.370 | CCL8 | chemokine (C-C motif) ligand 8 |
| 4.371 | CECR1 | cat eye syndrome chromosome region, candidate 1 |
| 4.372 | IGKC | immunoglobulin kappa constant |
| 4.373 | SLC40A1 | solute carrier family 40 (iron-regulated transporter), member 1 |
| 4.374 | ADCY6 | adenylate cyclase 6 |
| 4.375 | CP | ceruloplasmin (ferroxidase) |
| 4.376 | EDG1 | endothelial differentiation, sphingolipid G-protein-coupled receptor, 1 |
| 4.377 | RGS3 | regulator of G-protein signalling 3 |
| 4.378 | CD28 | CD28 antigen (Tp44) |
| 4.379 | NA | NA (gene identified by probe set 228339_at) |
| 4.380 | ABHD5 | abhydrolase domain containing 5 |
| 4.381 | MS4A7 | membrane-spanning 4-domains, subfamily A, member 7 |
| 4.382 | PRKCH | protein kinase C, eta |
| 4.383 | GBP1 | guanylate binding protein 1, interferon-inducible, 67kDa |
| 4.384 | LOC286071 | NA |
| 4.385 | BLNK | B-cell linker |
| 4.386 | NA | NA (gene identified by probe set 242546_at) |
| 4.387 | PCDHGC3 | protocadherin gamma subfamily C, 3 |
| 4.388 | CCL13 | chemokine (C-C motif) ligand 13 |
| 4.389 | JAK2 | Janus kinase 2 (a protein tyrosine kinase) |
| 4.390 | CAMSAP1L1 | calmodulin regulated spectrin-associated protein 1-like 1 |
| 4.391 | NPY1R | neuropeptide Y receptor Y1 |
| 4.392 | CD274 | CD274 antigen |
| 4.393 | PGM5 | phosphoglucomutase 5 |
| 4.394 | PLCG2 | phospholipase C, gamma 2 (phosphatidylinositol-specific) |
| 4.395 | TNFSF10 | tumor necrosis factor (ligand) superfamily, member 10 |
| 4.396 | SOD2 | superoxide dismutase 2, mitochondrial |
| 4.397 | BTG2 | BTG family, member 2 |
| 4.398 | LAMP3 | lysosomal-associated membrane protein 3 |
| 4.399 | IGLC1 | immunoglobulin lambda constant 1 (Mcg marker) |
| 4.400 | SIPA1L1 | signal-induced proliferation-associated 1 like 1 |
| 4.401 | AIF1 | allograft inflammatory factor 1 |
| 4.402 | IGLC2 | immunoglobulin lambda constant 2 (Kern-Oz- marker) |
| 4.403 | B2M | beta-2-microglobulin |
| 4.404 | CLEC7A | C-type lectin domain family 7, member A |
| 4.405 | MGC17330 | NA |
| 4.406 | IGF1R | insulin-like growth factor 1 receptor |
| 4.407 | HIVEP1 | human immunodeficiency virus type I enhancer binding protein 1 |
| 4.408 | FKBP14 | FK506 binding protein 14, 22 kDa |
| 4.409 | LAPTM5 | lysosomal associated multispanning membrane protein 5 |
| 4.410 | ABI3BP | ABI gene family, member 3 (NESH) binding protein |
| 4.411 | HLA-E | major histocompatibility complex, class I, E |
| 4.412 | ARL4C | ADP-ribosylation factor-like 4C |
| 4.413 | ASS | argininosuccinate synthetase |
| 4.414 | CASP1 | caspase 1, apoptosis-related cysteine peptidase (interleukin 1, beta, convertase) |
| 4.415 | ITGB3 | integrin, beta 3 (platelet glycoprotein IIIa, antigen CD61) |
| 4.416 | SYK | spleen tyrosine kinase |
| 4.417 | RAC2 | ras-related C3 botulinum toxin substrate 2 (rho family, small GTP binding protein Rac2) |
| 4.418 | NA | NA (gene identified by probe set 1557222_at) |
| 4.419 | CD3G | CD3g antigen, gamma polypeptide (TiT3 complex) |
| 4.420 | IGF1 | insulin-like growth factor 1 (somatomedin C) |
| 4.421 | NA | NA (gene identified by probe set 228858_at) |
| 4.422 | CYB5A | cytochrome b5 type A (microsomal) |
| 4.423 | TTC25 | tetratricopeptide repeat domain 25 |
| 4.424 | SLAMF6 | SLAM family member 6 |
| 4.425 | ARHGAP21 | Rho GTPase activating protein 21 |
| 4.426 | FLOT1 | flotillin 1 |
| 4.427 | AIF1 | allograft inflammatory factor 1 |
| 4.428 | IBRDC2 | IBR domain containing 2 |
| 4.429 | KIAA1794 | KIAA1794 |
| 4.430 | OLFML1 | olfactomedin-like 1 |
| 4.431 | GMFG | glia maturation factor, gamma |
| 4.432 | TNFRSF1B | tumor necrosis factor receptor superfamily, member 1B |
| 4.433 | NA | NA (gene identified by probe set 217629_at) |
| 4.434 | DEF6 | differentially expressed in FDCP 6 homolog (mouse) |
| 4.435 | HLA-E | major histocompatibility complex, class I, E |
| 4.436 | MAP4K4 | mitogen-activated protein kinase kinase kinase kinase 4 |
| 4.437 | CMKOR1 | chemokine orphan receptor 1 |
| 4.438 | NA | NA (gene identified by probe set 1563461_at) |
| 4.439 | CHKA | choline kinase alpha |
| 4.440 | NA | NA (gene identified by probe set 226865_at) |
| 4.441 | HS3ST3B1 | heparan sulfate (glucosamine) 3-O-sulfotransferase 3B1 |
| 4.442 | CXorf9 | chromosome X open reading frame 9 |
| 4.443 | EVI2A | ecotropic viral integration site 2A |
| 4.444 | GBP1 | guanylate binding protein 1, interferon-inducible, 67kDa |
| 4.445 | NFAM1 | NFAT activating protein with ITAM motif 1 |
| 4.446 | NA | NA (gene identified by probe set 242874_at) |
| 4.447 | ATP5J | ATP synthase, H+ transporting, mitochondrial F0 complex, subunit F6 |
| 4.448 | NAV1 | neuron navigator 1 |
| 4.449 | IGLC2 | immunoglobulin lambda constant 2 (Kern-Oz- marker) |
| 4.450 | CYLD | cylindromatosis (turban tumor syndrome) |
| 4.451 | GIMAP6 | GTPase, IMAP family member 6 |
| 4.452 | MFAP4 | microfibrillar-associated protein 4 |
| 4.453 | TUBB2B | tubulin, beta 2B |
| 4.454 | NELL2 | NEL-like 2 (chicken) |
| 4.455 | NA | NA |
| 4.456 | IL1RN | interleukin 1 receptor antagonist |
| 4.457 | KIAA1211 | NA |
| 4.458 | SYK | spleen tyrosine kinase |
| 4.459 | ADAMDEC1 | ADAM-like, decysin 1 |
| 4.460 | AOC3 | amine oxidase, copper containing 3 (vascular adhesion protein 1) |
| 4.461 | SAMHD1 | SAM domain and HD domain 1 |
| 4.462 | CXCL14 | chemokine (C-X-C motif) ligand 14 |
| 4.463 | SLC22A3 | solute carrier family 22 (extraneuronal monoamine transporter), member 3 |
| 4.464 | IL1R1 | interleukin 1 receptor, type I |
| 4.465 | IGLV3-25 | immunoglobulin lambda variable 3-25 |
| 4.466 | NA | NA (gene identified by probe set 1556185_a_at) |
| 4.467 | RAB11FIP1 | RAB 11 family interacting protein 1 (class I) |
| 4.468 | PER2 | period homolog 2 (Drosophila) |
| 4.469 | TTL | tubulin tyrosine ligase |
| 4.470 | SIAHBP1 | NA |
| 4.471 | LAPTM5 | lysosomal associated multispanning membrane protein 5 |
| 4.472 | FLJ22536 | NA |
| 4.473 | RP6-213H19.1 | NA |
| 4.474 | NA | NA (gene identified by probe set 235804_at) |
| 4.475 | NCF4 | neutrophil cytosolic factor 4, 40kDa |
| 4.476 | EPSTI1 | epithelial stromal interaction 1 (breast) |
| 4.477 | LOC441212 | NA |
| 4.478 | ANK3 | ankyrin 3, node of Ranvier (ankyrin G) |
| 4.479 | PCDH9 | protocadherin 9 |
| 4.480 | C21orf86 | chromosome 21 open reading frame 86 |
| 4.481 | DHRS9 | dehydrogenase/reductase (SDR family) member 9 |
| 4.482 | ARHGAP25 | Rho GTPase activating protein 25 |
| 4.483 | TRAF4 | TNF receptor-associated factor 4 |
| 4.484 | LST1 | leukocyte specific transcript 1 |
| 4.485 | PALMD | palmdelphin |
| 4.486 | TAP1 | transporter 1, ATP-binding cassette, sub-family B (MDR/TAP) |
| 4.487 | MSX2 | msh homeobox homolog 2 (Drosophila) |
| 4.488 | SIRPG | signal-regulatory protein gamma |

| | | |
|---|---|---|
| NA = not applicable | | |

In the tables listed herein the given gene may be listed more than once, for example as a specific gene or as a gene cluster. Table 4 above is generated from the probe sets listed in Table 4A below. There are often multiple probe sets for each gene and thus where more than one probe set has been used to indentify a particular gene then the gene appears more than once in Table 4. An attempt has been made to remove the duplication by scoring through genes that appear more than once in Table 4.

In one aspect the invention provides across the various embodiments herein one or more genes selected from Table 1, 2, 3, 4, 7, 9, 11, 12 and/or 13 including combinations thereof. In a further aspect the invention provides all the genes of Table 1, 2, 3, 4, 7, 9, 11, 12 or 13 or indeed combinations thereof.

In a further aspect the invention provides at least 10% of the genes listed in Table 1, 2, 3, 4, 7, 9, 11, 12 and/or 13, for example at least 40%, 50%, 60% or 70% such as 80%, 90% or 100% thereof.

One or more genes includes 1-5, 6-10, 11-15, 16-20, 21-25, 26-30, 31-35, 36-40, 41-45, 46-50, 51-55, 56-60, 61-65, 66-70. 71-75, 76-80, 81-85, 86-90, 91-95, 96-100, 101-105, 106-110, 111-115. 116-120, 121-125, 126-130, 131-135, 136-140, 141-145, 146-150, 151-155, 156-160, 161-165, 166-170, 171-175, 176-180, 181-185, 186-190, 191-195, 196-200, 201-205, 206-210, 211-215, 216-220, 221-225, 226-230, 231-235, 236-240, 241-245, 246-250, 251-255, 256-260, 261-265, 266-270, 271-275, 276-280, 281-285, 286-290, 291-295, 296-300, 301-305, 306-310, 311-315, 316-320, 321-325, 326-330, 331-335, 336-340, 341-345, 346-350, 351-355, 356-360, 361-365, 366-370, 371-375, 376-380, 381-385, 386-390, 391-395, 396-400, 401-405, 406-410, 411-415, 416-420, 421-425, 426-430, 431-435, 436-440, 441-445, 446-450, 451-455, 456-460, 461-465, 466-470, 471-475, 476-480, as appropriate and combinations thereof.

In one or more aspects the invention provides an embodiment as described in any one of paragraphs 1 to 430 below.
1) Thus the invention may employ one or more genes from Table 4.
2) In another aspect the invention employs one or more genes according to paragraph 1, wherein the gene has the symbol FLJ20647, optionally in combination with one or more genes labeled as 4.2 to 4.488 identified in Table 4.
3) In another aspect the invention employs one or more genes according to paragraph 1 or 2, wherein the gene has the NAV1, optionally in combination with one or more genes labeled as 4.3 to 4.488 identified in Table 4.
4) In another aspect the invention employs one or more genes according to any one one of paragraphs 1-3, wherein the gene has the symbol GPR171, optionally in combination with one or more genes labeled as 4.4 to 4.488 identified in Table 4.
5) In another aspect the invention employs one or more genes according to any one one of paragraphs 1-4, wherein the gene has the symbol CCL14, optionally in combination with one or more genes labeled as 4.5 to 4.488 identified in Table 4.
6) In another aspect the invention employs one or more genes according to any one one of paragraphs 1-5, wherein the gene has the symbol C1S, optionally in combination with one or more genes labeled as 4.6 to 4.488 identified in Table 4.
7) In another aspect the invention employs one or more genes according to any one one of paragraphs 1-6, wherein the gene has the symbol CXCL2, optionally in combination with one or more genes labeled as 4.7 to 4.488 identified in Table 4.
8) In another aspect the invention employs one or more genes according to any one one of paragraphs 1-7, wherein the gene has the symbol TRBV3-1, optionally in combination with one or more genes labeled as 4.8 to 4.488 identified in Table 4.
9) In another aspect the invention employs one or more genes according to any one one of paragraphs 1-8, wherein the gene has the symbol TRDV2, optionally in combination with one or more genes labeled as 4.9 to 4.488 identified in Table 4.
10) In another aspect the invention employs one or more genes according to any one one of paragraphs 1-9, wherein the gene has the symbol RUFY3, optionally in combination with one or more genes labeled as 4.10 to 4.488 identified in Table 4.
11) In another aspect the invention employs one or more genes according to any one one of paragraphs 1-10, wherein the gene has the symbol DOCK8, optionally in combination with one or more genes labeled as 4.11 to 4.488 identified in Table 4.
12) In another aspect the invention employs one or more genes according to any one one of paragraphs 1-11, wherein the gene has the symbol GCH1, optionally in combination with one or more genes labeled as 4.12 to 4.488 identified in Table 4.
13) In another aspect the invention employs one or more genes according to any one one of paragraphs 1-12, wherein the gene has the symbol CENTD3, optionally in combination with one or more genes labeled as 4.13 to 4.488 identified in Table 4.
14) In another aspect the invention employs one or more genes according to any one of paragraphs 1-13, wherein the gene has the symbol ACSL5, optionally in combination with one or more genes labeled as 4.14 to 4.488 identified in Table 4.
15) In another aspect the invention employs one or more genes according to any one of paragraphs 1-14, wherein the gene has the symbol AMICA1, optionally in combination with one or more genes labeled as 4.15 to 4.488 identified in Table 4.
16) In another aspect the invention employs one or more genes according to any one of paragraphs 1-15, wherein the gene has the symbol IL2RG, optionally in combination with one or more genes labeled as 4.16 to 4.488 identified in Table 4.
17) In another aspect the invention employs one or more genes according to any one of paragraphs 1-16, wherein the gene has the symbol TNFAIP3, optionally in combination with one or more genes labeled as 4.17 to 4.488 identified in Table 4.
18) In another aspect the invention employs one or more genes according to any one of paragraphs 1-17, wherein the gene has the symbol PSCDBP, optionally in combination with one or more genes labeled as 4.18 to 4.488 identified in Table 4.
19) In another aspect the invention employs one or more genes according to any one of paragraphs 1-18, wherein the gene has the symbol ESR1, optionally in combination with one or more genes labeled as 4.19 to 4.488 identified in Table 4.
20) In another aspect the invention employs one or more genes according to any one of paragraphs 1-19, wherein the gene has the symbol TRBC1, optionally in combination with one or more genes labeled as 4.20 to 4.488 identified in Table 4.
21) In another aspect the invention employs one or more genes according to any one of paragraphs 1-20, wherein the gene has the symbol CD52, optionally in combination with one or more genes labeled as 4.21 to 4.488 identified in Table 4.
22) In another aspect the invention employs one or more genes according to any one of paragraphs 1-21, wherein the gene has the symbol LOC442535, optionally in combination with one or more genes labeled as 4.22 to 4.488 identified in Table 4.
23) In another aspect the invention employs one or more genes according to any one of paragraphs 1-22, wherein the gene has the symbol TRBV19, optionally in combination with one or more genes labeled as 4.23 to 4.488 identified in Table 4.
24) In another aspect the invention employs one or more genes according to any one of paragraphs 1-23, wherein the gene has the symbol IL7R, optionally in combination with one or more genes labeled as 4.24 to 4.488 identified in Table 4.
25) In another aspect the invention employs one or more genes according to any one of paragraphs 1-24, wherein the gene has the symbol TRAC, optionally in combination with one or more genes labeled as 4.25 to 4.488 identified in Table 4.
26) In another aspect the invention employs one or more genes according to any one of paragraphs 1-25, wherein the gene has the symbol NCF2, optionally in combination with one or more genes labeled as 4.26 to 4.488 identified in Table 4.
27) In another aspect the invention employs one or more genes according to any one of paragraphs 1-26, wherein the gene has the symbol LOC92689, optionally in combination with one or more genes labeled as 4.27 to 4.488 identified in Table 4.
28) In another aspect the invention employs one or more genes according to any one of paragraphs 1-27, wherein the gene has the symbol GZMK, optionally in combination with one or more genes labeled as 4.28 to 4.488 identified in Table 4.
29) In another aspect the invention employs one or more genes according to any one of paragraphs 1-28, wherein the gene is the one identified by probe set 235831, optionally in combination with one or more genes labeled as 4.29 to 4.488 identified in Table 4.
30) In another aspect the invention employs one or more genes according to any one of paragraphs 1-29, wherein the gene has the symbol RAB34, optionally in combination with one or more genes labeled as 4.30 to 4.488 identified in Table 4.
31) In another aspect the invention employs one or more genes according to any one of paragraphs 1-30, wherein the gene has the symbol DPT, optionally in combination with one or more genes labeled as 4.31 to 4.488 identified in Table 4.
32) In another aspect the invention employs one or more genes according to any one of paragraphs 1-31, wherein the gene has the symbol PVT1, optionally in combination with one or more genes labeled as 4.32 to 4.488 identified in Table 4.
33) In another aspect the invention employs one or more genes according to any one of paragraphs 1-32, wherein the gene has the symbol TRGC2, optionally in combination with one or more genes labeled as 4.33 to 4.488 identified in Table 4.
34) In another aspect the invention employs one or more genes according to any one of paragraphs 1-33, wherein the gene has the symbol GIMAP5, optionally in combination with one or more genes labeled as 4.34 to 4.488 identified in Table 4.
35) In another aspect the invention employs one or more genes according to any one of paragraphs 1-34, wherein the gene has the symbol CD52, optionally in combination with one or more genes labeled as 4.35 to 4.488 identified in Table 4.
36) In another aspect the invention employs one or more genes according to any one of paragraphs 1-35, wherein the gene has the symbol CD3D, optionally in combination with one or more genes labeled as 4.36 to 4.488 identified in Table 4.
37) In another aspect the invention employs one or more genes according to any one of paragraphs 1-36, wherein the gene has the symbol TMEM132C, optionally in combination with one or more genes labeled as 4.37 to 4.488 identified in Table 4.
38) In another aspect the invention employs one or more genes according to any one of paragraphs 1-37, wherein the gene has the symbol NFKBIA, optionally in combination with one or more genes labeled as 4.38 to 4.488 identified in Table 4.
39) In another aspect the invention employs one or more genes according to any one of paragraphs 1-38, wherein the gene has the symbol TRA@, optionally in combination with one or more genes labeled as 4.39 to 4.488 identified in Table 4.
40) In another aspect the invention employs one or more genes according to any one of paragraphs 1-39, wherein the gene has the symbol TRAT1, optionally in combination with one or more genes labeled as 4.41 to 4.488 identified in Table 4.
41) In another aspect the invention employs one or more genes according to any one of paragraphs 1-40, wherein the gene has the symbol RAB34, optionally in combination with one or more genes labeled as 4.42 to 4.488 identified in Table 4.
42) In another aspect the invention employs one or more genes according to any one of paragraphs 1-41, wherein the gene has the symbol CD69, optionally in combination with one or more genes labeled as 4.43 to 4.488 identified in Table 4.
43) In another aspect the invention employs one or more genes according to any one of paragraphs 1-42, wherein the gene has the symbol DOCK8 optionally in combination with one or more genes labeled as 4.44 to 4.488 identified in Table 4.
44) In another aspect the invention employs one or more genes according to any one of paragraphs 1-43, wherein the gene has the symbol IRF8 optionally in combination with one or more genes labeled as 4.46 to 4.488 identified in Table 4.
45) In another aspect the invention employs one or more genes according to any one of paragraphs 1-44, wherein the gene has the symbol KLRB1 optionally in combination with one or more genes labeled as 4.47 to 4.488 identified in Table 4.
46) In another aspect the invention employs one or more genes according to any one of paragraphs 1-45, wherein the gene is identifiable by probe set number 236280_at, optionally in combination with one or more genes labeled as 4.48 to 4.488 identified in Table 4.
47) In another aspect the invention employs one or more genes according to any one of paragraphs 1-46, wherein the gene has the symbol ITGA3, optionally in combination with one or more genes labeled as 4.49 to 4.488 identified in Table 4.
48) In another aspect the invention employs one or more genes according to any one of paragraphs 1-47, wherein the gene has the symbol MAP3K8, optionally in combination with one or more genes labeled as 4.50 to 4.488 identified in Table 4.
49) In another aspect the invention employs one or more genes according to any one of paragraphs 1-48, wherein the gene has the symbol Clorf162, optionally in combination with one or more genes labeled as 4.51 to 4.488 identified in Table 4.
50) In another aspect the invention employs one or more genes according to any one of paragraphs 1-49, wherein the gene has the symbol UBD, optionally in combination with one or more genes labeled as 4.52 to 4.488 identified in Table 4.
51) In another aspect the invention employs one or more genes according to any one of paragraphs 1-50, wherein the gene has the symbol TRGV9, optionally in combination with one or more genes labeled as 4.54 to 4.488 identified in Table 4.
52) In another aspect the invention employs one or more genes according to any one of paragraphs 1-51, wherein the gene has the symbol optionally in combination with one or more genes labeled as 4.54 to 4.488 identified in Table 4.
53) In another aspect the invention employs one or more genes according to any one of paragraphs 1-51, wherein the gene has the symbol ARHGAP9, optionally in combination with one or more genes labeled as 4.55 to 4.488 identified in Table 4.
54) In another aspect the invention employs one or more genes according to any one of paragraphs 1-53, wherein the gene has the symbol TIFA, optionally in combination with one or more genes labeled as 4.56 to 4.488 identified in Table 4.
55) In another aspect the invention employs one or more genes according to any one of paragraphs 1-54, wherein the gene is identified by probe number 1569942_at, optionally in combination with one or more genes labeled as 4.58 to 4.488 identified in Table 4.
56) In another aspect the invention employs one or more genes according to any one of paragraphs 1-55, wherein the gene has the symbol GIMAP4, optionally in combination with one or more genes labeled as 4.59 to 4.488 identified in Table 4.
57) In another aspect the invention employs one or more genes according to any one of paragraphs 1-56, wherein the gene has the symbol HCLS1, optionally in combination with one or more genes labeled as 4.60 to 4.488 identified in Table 4.
58) In another aspect the invention employs one or more genes according to any one of paragraphs 1-57, wherein the gene has the symbol PRKCH, optionally in combination with one or more genes labeled as 4.61 to 4.488 identified in Table 4.
59) In another aspect the invention employs one or more genes according to any one of paragraphs 1-58, wherein the gene has the symbol STAT4, optionally in combination with one or more genes labeled as 4.62 to 4.488 identified in Table 4.
60) In another aspect the invention employs one or more genes according to any one of paragraphs 1-59, wherein the gene has the symbol HLA-DQA1, optionally in combination with one or more genes labeled as 4.63 to 4.488 identified in Table 4.
61) In another aspect the invention employs one or more genes according to any one of paragraphs 1-60, wherein the gene has the symbol ADRB2, optionally in combination with one or more genes labeled as 4.64 to 4.488 identified in Table 4.
62) In another aspect the invention employs one or more genes according to any one of paragraphs 1-61, wherein the gene is identifiable by probe set number 239237_at, optionally in combination with one or more genes labeled as 4.65 to 4.488 identified in Table 4.
63) In another aspect the invention employs one or more genes according to any one of paragraphs 1-62, wherein the gene has the symbol CTSW, optionally in combination with one or more genes labeled as 4.66 to 4.488 identified in Table 4.
64) In another aspect the invention employs one or more genes according to any one of paragraphs 1-63, wherein the gene has the symbol MYH11, optionally in combination with one or more genes labeled as 4.67 to 4.488 identified in Table 4.
65) In another aspect the invention employs one or more genes according to any one of paragraphs 1-64, wherein the gene has the symbol GIMAP6, optionally in combination with one or more genes labeled as 4.68 to 4.488 identified in Table 4.
66) In another aspect the invention employs one or more genes according to any one of paragraphs 1-65, wherein the gene has the symbol HLA-DQB1,, optionally in combination with one or more genes labeled as 4.69 to 4.488 identified in Table 4.
67) In another aspect the invention employs one or more genes according to any one of paragraphs 1-66, wherein the gene has the symbol CD8A,, optionally in combination with one or more genes labeled as 4.70 to 4.488 identified in Table 4.
68) In another aspect the invention employs one or more genes according to any one of paragraphs 1-67, wherein the gene has the symbol TNFAIP3, optionally in combination with one or more genes labeled as 4.71 to 4.488 identified in Table 4.
69) In another aspect the invention employs one or more genes according to any one of paragraphs 1-68, wherein the gene has the symbol CP, optionally in combination with one or more genes labeled as 4.72 to 4.488 identified in Table 4.
70) In another aspect the invention employs one or more genes according to any one of paragraphs 1-69, wherein the gene has the symbol SMOC2, optionally in combination with one or more genes labeled as 4.73 to 4.488 identified in Table 4.
71) In another aspect the invention employs one or more genes according to any one of paragraphs 1-70, wherein the gene has the symbol C20orf24, optionally in combination with one or more genes labeled as 4.74 to 4.488 identified in Table 4.
72) In another aspect the invention employs one or more genes according to any one of paragraphs 1-71, wherein the gene has the symbol C16orf54, optionally in combination with one or more genes labeled as 4.75 to 4.488 identified in Table 4.
73) In another aspect the invention employs one or more genes according to any one of paragraphs 1-72, wherein the gene has the symbol CD2, optionally in combination with one or more genes labeled as 4.76 to 4.488 identified in Table 4.
74) In another aspect the invention employs one or more genes according to any one of paragraphs 1-73, wherein the gene has the symbol SLIT3, optionally in combination with one or more genes labeled as 4.77 to 4.488 identified in Table 4.
75) In another aspect the invention employs one or more genes according to any one of paragraphs 1-74, wherein the gene has the symbol BAALC, optionally in combination with one or more genes labeled as 4.78 to 4.488 identified in Table 4.
76) In another aspect the invention employs one or more genes according to any one of paragraphs 1-75, wherein the gene has the symbol TRIB3, optionally in combination with one or more genes labeled as 4.79 to 4.488 identified in Table 4.
77) In another aspect the invention employs one or more genes according to any one of paragraphs 1-76, wherein the gene has the symbol LOC440160, optionally in combination with one or more genes labeled as 4.80 to 4.488 identified in Table 4.
78) In another aspect the invention employs one or more genes according to any one of paragraphs 1-77, wherein the gene has the symbol C6orf190, optionally in combination with one or more genes labeled as 4.81 to 4.488 identified in Table 4.
79) In another aspect the invention employs one or more genes according to any one of paragraphs 1-78, wherein the gene has the symbol TAGAP, optionally in combination with one or more genes labeled as 4.82 to 4.488 identified in Table 4.
80) In another aspect the invention employs one or more genes according to any one of paragraphs 1-79, wherein the gene has the symbol FAM92A1, optionally in combination with one or more genes labeled as 4.83 to 4.488 identified in Table 4.
81) In another aspect the invention employs one or more genes according to any one of paragraphs 1-80, wherein the gene has the symbol PSTPIP2, optionally in combination with one or more genes labeled as 4.84 to 4.488 identified in Table 4.
82) In another aspect the invention employs one or more genes according to any one of paragraphs 1-81, wherein the gene has the symbol PTPRC, optionally in combination with one or more genes labeled as 4.85 to 4.488 identified in Table 4.
83) In another aspect the invention employs one or more genes according to any one of paragraphs 1-82, wherein the gene has the symbol HLA-DRA, optionally in combination with one or more genes labeled as 4.86 to 4.488 identified in Table 4.
84) In another aspect the invention employs one or more genes according to any one of paragraphs 1-83, wherein the gene has the symbol EFCAB2, optionally in combination with one or more genes labeled as 4.87 to 4.488 identified in Table 4.
85) In another aspect the invention employs one or more genes according to any one of paragraphs 1-84, wherein the gene has the symbol TNFAIP8, optionally in combination with one or more genes labeled as 4.88 to 4.488 identified in Table 4.
86) In another aspect the invention employs one or more genes according to any one of paragraphs 1-85, wherein the gene has the symbol SLIC1, optionally in combination with one or more genes labeled as 4.89 to 4.488 identified in Table 4.
87) In another aspect the invention employs one or more genes according to any one of paragraphs 1-86, wherein the gene has the symbol CD1C, optionally in combination with one or more genes labeled as 4.90 to 4.488 identified in Table 4.
88) In another aspect the invention employs one or more genes according to any one of paragraphs 1-87, wherein the gene has the symbol TRAF3IP3, optionally in combination with one or more genes labeled as 4.91 to 4.488 identified in Table 4.
89) In another aspect the invention employs one or more genes according to any one of paragraphs 1-88, wherein the gene has the symbol IGJ, optionally in combination with one or more genes labeled as 4.96 to 4.488 identified in Table 4.
90) In another aspect the invention employs one or more genes according to any one of paragraphs 1-89, wherein the gene has the symbol PLEK, optionally in combination with one or more genes labeled as 4.98 to 4.488 identified in Table 4.
91) In another aspect the invention employs one or more genes according to any one of paragraphs 1-90, wherein the gene has the symbol TMEM44, optionally in combination with one or more genes labeled as 4.100 to 4.488 identified in Table 4.
92) In another aspect the invention employs one or more genes according to any one of paragraphs 1-91, wherein the gene has the symbol EBI2, optionally in combination with one or more genes labeled as 4.101 to 4.488 identified in Table 4.
93) In another aspect the invention employs one or more genes according to any one of paragraphs 1-92, wherein the gene has the symbol SAMSN1, optionally in combination with one or more genes labeled as 4.102 to 4.488 identified in Table 4.
94) In another aspect the invention employs one or more genes according to any one of paragraphs 1-93, wherein the gene has the symbol KIAA1794, optionally in combination with one or more genes labeled as 4.103 to 4.488 identified in Table 4.
95) In another aspect the invention employs one or more genes according to any one of paragraphs 1-94, wherein the gene has the symbol ALDH2, optionally in combination with one or more genes labeled as 4.104 to 4.488 identified in Table 4.
96) In another aspect the invention employs one or more genes according to any one of paragraphs 1-95, wherein the gene has the symbol CDC42SE2, optionally in combination with one or more genes labeled as 4.105 to 4.488 identified in Table 4.
97) In another aspect the invention employs one or more genes according to any one of paragraphs 1-96, wherein the gene has the symbol GFRA1, optionally in combination with one or more genes labeled as 4.106 to 4.488 identified in Table 4.
98) In another aspect the invention employs one or more genes according to any one of paragraphs 1-97,wherein the gene has the symbol ITK, optionally in combination with one or more genes labeled as 4.107 to 4.488 identified in Table 4.
99) In another aspect the invention employs one or more genes according to any one of paragraphs 1-98, wherein the gene has the symbol GIMAP7, optionally in combination with one or more genes labeled as 4.109 to 4.488 identified in Table 4.
100) In another aspect the invention employs one or more genes according to any one of paragraphs 1-99, wherein the gene has the symbol FLJ20273, optionally in combination with one or more genes labeled as 4.110 to 4.488 identified in Table 4.
101) In another aspect the invention employs one or more genes according to any one of paragraphs 1-100, wherein the gene has the symbol PTPN6, optionally in combination with one or more genes labeled as 4.111 to 4.488 identified in Table 4.
102) In another aspect the invention employs one or more genes according to any one of paragraphs 1-101, wherein the gene has the symbol PTGER3, optionally in combination with one or more genes labeled as 4.112 to 4.488 identified in Table 4.
103) In another aspect the invention employs one or more genes according to any one of paragraphs 1-102, wherein the gene has the symbol RAI2, optionally in combination with one or more genes labeled as 4.113 to 4.488 identified in Table 4.
104) In another aspect the invention employs one or more genes according to any one of paragraphs 1-103, wherein the gene has the symbol LGALS2, optionally in combination with one or more genes labeled as 4.114 to 4.488 identified in Table 4.
105) In another aspect the invention employs one or more genes according to any one of paragraphs 1-104, wherein the gene has the symbol HMOX1, optionally in combination with one or more genes labeled as 4.115 to 4.488 identified in Table 4.
106) In another aspect the invention employs one or more genes according to any one of paragraphs 1-105, wherein the gene is identifiable by probe set number 227995_at, optionally in combination with one or more genes labeled as 4.116 to 4.488 identified in Table 4.
107) In another aspect the invention employs one or more genes according to any one of paragraphs 1-106, wherein the gene has the symbol ZNFN1A1, optionally in combination with one or more genes labeled as 4.117 to 4.488 identified in Table 4.
108) In another aspect the invention employs one or more genes according to any one of paragraphs 1-107, wherein the gene has the symbol CSF2RB, optionally in combination with one or more genes labeled as 4.118 to 4.488 identified in Table 4.
109) In another aspect the invention employs one or more genes according to any one of paragraphs 1-108, wherein the gene has the symbol PCSK5, optionally in combination with one or more genes labeled as 4.119 to 4.488 identified in Table 4.
110) In another aspect the invention employs one or more genes according to any one of paragraphs 1-109, wherein the gene has the symbol CCDC69, optionally in combination with one or more genes labeled as 4.120 to 4.488 identified in Table 4.
111) In another aspect the invention employs one or more genes according to any one of paragraphs 1-110, wherein the gene has the symbol CDC42SE2, optionally in combination with one or more genes labeled as 4.121 to 4.488 identified in Table 4.
112) In another aspect the invention employs one or more genes according to any one of paragraphs 1-111, wherein the gene has the symbol GZMA, optionally in combination with one or more genes labeled as 4.122 to 4.488 identified in Table 4.
113) In another aspect the invention employs one or more genes according to any one of paragraphs 1-112, wherein the gene has the symbol C3, optionally in combination with one or more genes labeled as 4.123 to 4.488 identified in Table 4.
114) In another aspect the invention employs one or more genes according to any one of paragraphs 1-113, wherein the gene has the symbol C15orf48, optionally in combination with one or more genes labeled as 4.125 to 4.488 identified in Table 4.
115) In another aspect the invention employs one or more genes according to any one of paragraphs 1-114, wherein the gene has the symbol RARRES3, optionally in combination with one or more genes labeled as 4.126 to 4.488 identified in Table 4.
116) In another aspect the invention employs one or more genes according to any one of paragraphs 1-115, wherein the gene has the symbol LOC283537, optionally in combination with one or more genes labeled as 4.127 to 4.488 identified in Table 4.
117) In another aspect the invention employs one or more genes according to any one of paragraphs 1-116, wherein the gene has the symbol CXCL12, optionally in combination with one or more genes labeled as 4.129 to 4.488 identified in Table 4.
118) In another aspect the invention employs one or more genes according to any one of paragraphs 1-117, wherein the gene is identifiable by probe set number 231882_at, optionally in combination with one or more genes labeled as 4.130 to 4.488 identified in Table 4.
119) In another aspect the invention employs one or more genes according to any one of paragraphs 1-118, wherein the gene has the symbol SOD2, optionally in combination with one or more genes labeled as 4.131 to 4.488 identified in Table 4.
120) In another aspect the invention employs one or more genes according to any one of paragraphs 1-119, wherein the gene has the symbol CTSS, optionally in combination with one or more genes labeled as 4.132 to 4.488 identified in Table 4.
121) In another aspect the invention employs one or more genes according to any one of paragraphs 1-120, wherein the gene has the symbol CTBP2, optionally in combination with one or more genes labeled as 4.133 to 4.488 identified in Table 4.
122) In another aspect the invention employs one or more genes according to any one of paragraphs 1-121, wherein the gene has the symbol BCL11B, optionally in combination with one or more genes labeled as 4.134 to 4.488 identified in Table 4.
123) In another aspect the invention employs one or more genes according to any one of paragraphs 1-122, wherein the gene has the symbol CCL22, optionally in combination with one or more genes labeled as 4.135 to 4.488 identified in Table 4.
124) In another aspect the invention employs one or more genes according to any one of paragraphs 1-123, wherein the gene has the symbol ACSL5, optionally in combination with one or more genes labeled as 4.136 to 4.488 identified in Table 4.
125) In another aspect the invention employs one or more genes according to any one of paragraphs 1-124, wherein the gene has the symbol DOC1, optionally in combination with one or more genes labeled as 4.137 to 4.488 identified in Table 4.
126) In another aspect the invention employs one or more genes according to any one of paragraphs 1-125, wherein the gene has the symbol SLC31A2, optionally in combination with one or more genes labeled as 4.138 to 4.488 identified in Table 4.
127) In another aspect the invention employs one or more genes according to any one of paragraphs 1-126, wherein the gene has the symbol POPDC3, optionally in combination with one or more genes labeled as 4.139 to 4.488 identified in Table 4.
128) In another aspect the invention employs one or more genes according to any one of paragraphs 1-127, wherein the gene has the symbol SQRDL, optionally in combination with one or more genes labeled as 4.141 to 4.488 identified in Table 4.
129) In another aspect the invention employs one or more genes according to any one of paragraphs 1-128, wherein the gene has the symbol RASGEF1B, optionally in combination with one or more genes labeled as 4.142 to 4.488 identified in Table 4.
130) In another aspect the invention employs one or more genes according to any one of paragraphs 1-129, wherein the gene has the symbol FGL2, optionally in combination with one or more genes labeled as 4.143 to 4.488 identified in Table 4.
131) In another aspect the invention employs one or more genes according to any one of paragraphs 1-130, wherein the gene has the symbol C10orf128, optionally in combination with one or more genes labeled as 4.144 to 4.488 identified in Table 4.
132) In another aspect the invention employs one or more genes according to any one of paragraphs 1-131, wherein the gene has the symbol IL10RA, optionally in combination with one or more genes labeled as 4.145 to 4.488 identified in Table 4.
133) In another aspect the invention employs one or more genes according to any one of paragraphs 1-132, wherein the gene has the symbol EGFL6, optionally in combination with one or more genes labeled as 4.146 to 4.488 identified in Table 4.
134) In another aspect the invention employs one or more genes according to any one of paragraphs 1-133, wherein the gene has the symbol IL18, optionally in combination with one or more genes labeled as 4.147 to 4.488 identified in Table 4.
135) In another aspect the invention employs one or more genes according to any one of paragraphs 1-134, wherein the gene has the symbol ARHGAP30, optionally in combination with one or more genes labeled as 4.148 to 4.488 identified in Table 4.
136) In another aspect the invention employs one or more genes according to any one of paragraphs 1-135, wherein the gene has the symbol PALMD, optionally in combination with one or more genes labeled as 4.149 to 4.488 identified in Table 4.
137) In another aspect the invention employs one or more genes according to any one of paragraphs 1-136, wherein the gene has the symbol RASSF5, optionally in combination with one or more genes labeled as 4.150 to 4.488 identified in Table 4.
138) In another aspect the invention employs one or more genes according to any one of paragraphs 1-137, wherein the gene has the symbol GATA3, optionally in combination with one or more genes labeled as 4.151 to 4.488 identified in Table 4.
139) In another aspect the invention employs one or more genes according to any one of paragraphs 1-138, wherein the gene has the symbol DKFZP564O0823, optionally in combination with one or more genes labeled as 4.152 to 4.488 identified in Table 4.
140) In another aspect the invention employs one or more genes according to any one of paragraphs 1-139, wherein the gene has the symbol TXNIP, optionally in combination with one or more genes labeled as 4.154 to 4.488 identified in Table 4.
141) In another aspect the invention employs one or more genes according to any one of paragraphs 1-140, wherein the gene has the symbol DTX4, optionally in combination with one or more genes labeled as 4.155 to 4.488 identified in Table 4.
142) In another aspect the invention employs one or more genes according to any one of paragraphs 1-141, wherein the gene has the symbol DARC, optionally in combination with one or more genes labeled as 4.156 to 4.488 identified in Table 4.
143) In another aspect the invention employs one or more genes according to any one of paragraphs 1-142, wherein the gene has the symbol RNASE6, optionally in combination with one or more genes labeled as 4.157 to 4.488 identified in Table 4.
144) In another aspect the invention employs one or more genes according to any one of paragraphs 1-143, wherein the gene has the symbol CD86, optionally in combination with one or more genes labeled as 4.158 to 4.488 identified in Table 4.
145) In another aspect the invention employs one or more genes according to any one of paragraphs 1-144, wherein the gene has the symbol ZFP36, optionally in combination with one or more genes labeled as 4.159 to 4.488 identified in Table 4.
146) In another aspect the invention employs one or more genes according to any one of paragraphs 1-145, wherein the gene has the symbol BASP1, optionally in combination with one or more genes labeled as 4.160 to 4.488 identified in Table 4.
147) In another aspect the invention employs one or more genes according to any one of paragraphs 1-146, wherein the gene has the symbol CKAP1, optionally in combination with one or more genes labeled as 4.161 to 4.488 identified in Table 4.
148) In another aspect the invention employs one or more genes according to any one of paragraphs 1-147, wherein the gene has the symbol HCP5, optionally in combination with one or more genes labeled as 4.162 to 4.488 identified in Table 4.
149) In another aspect the invention employs one or more genes according to any one of paragraphs 1-148, wherein the gene has the symbol GRB14, optionally in combination with one or more genes labeled as 4.163 to 4.488 identified in Table 4.
150) In another aspect the invention employs one or more genes according to any one of paragraphs 1-149, wherein the gene has the symbol GJA7, optionally in combination with one or more genes labeled as 4.164 to 4.488 identified in Table 4.
151) In another aspect the invention employs one or more genes according to any one of paragraphs 1-150, wherein the gene has the symbol FLJ14054, optionally in combination with one or more genes labeled as 4.165 to 4.488 identified in Table 4.
152) In another aspect the invention employs one or more genes according to any one of paragraphs 1-151, wherein the gene has the symbol VNN1, optionally in combination with one or more genes labeled as 4.166 to 4.488 identified in Table 4.
153) In another aspect the invention employs one or more genes according to any one of paragraphs 1-152, wherein the gene has the symbol ADCY7, optionally in combination with one or more genes labeled as 4.167 to 4.488 identified in Table 4.
154) In another aspect the invention employs one or more genes according to any one of paragraphs 1-153, wherein the gene has the symbol MS4A6A, optionally in combination with one or more genes labeled as 4.168 to 4.488 identified in Table 4.
155) In another aspect the invention employs one or more genes according to any one of paragraphs 1-154, wherein the gene has the symbol CPA3, optionally in combination with one or more genes labeled as 4.169 to 4.488 identified in Table 4.
156) In another aspect the invention employs one or more genes according to any one of paragraphs 1-155, wherein the gene has the symbol PIM1, optionally in combination with one or more genes labeled as 4.170 to 4.488 identified in Table 4.
157) In another aspect the invention employs one or more genes according to any one of paragraphs 1-156, wherein the gene has the symbol CCL19, optionally in combination with one or more genes labeled as 4.171 to 4.488 identified in Table 4.
158) In another aspect the invention employs one or more genes according to any one of paragraphs 1-157, wherein the gene has the symbol SYK, optionally in combination with one or more genes labeled as 4.172 to 4.488 identified in Table 4.
159) In another aspect the invention employs one or more genes according to any one of paragraphs 1-158, wherein the gene has the symbol SIT1, optionally in combination with one or more genes labeled as 4.174 to 4.488 identified in Table 4.
160) In another aspect the invention employs one or more genes according to any one of paragraphs 1-159, wherein the gene is identifiable by probe set number 228812_at, optionally in combination with one or more genes labeled as 4.175 to 4.488 identified in Table 4.
161) In another aspect the invention employs one or more genes according to any one of paragraphs 1-160, wherein the gene has the symbol NAP1L2 , optionally in combination with one or more genes labeled as 4.176 to 4.488 identified in Table 4.
162) In another aspect the invention employs one or more genes according to any one of paragraphs 1-161, wherein the gene has the symbol CCL13, optionally in combination with one or more genes labeled as 4.177 to 4.488 identified in Table 4.
163) In another aspect the invention employs one or more genes according to any one of paragraphs 1-162, wherein the gene has the symbol SLA, optionally in combination with one or more genes labeled as 4.178 to 4.488 identified in Table 4.
164) In another aspect the invention employs one or more genes according to any one of paragraphs 1-163, wherein the gene has the symbol NOD3, optionally in combination with one or more genes labeled as 4.179 to 4.488 identified in Table 4.
165) In another aspect the invention employs one or more genes according to any one of paragraphs 1-164, wherein the gene has the symbol PRKCH , optionally in combination with one or more genes labeled as 4.180 to 4.488 identified in Table 4.
166) In another aspect the invention employs one or more genes according to any one of paragraphs 1-165, wherein the gene has the symbol TRD@, optionally in combination with one or more genes labeled as 4.181 to 4.488 identified in Table 4.
167) In another aspect the invention employs one or more genes according to any one of paragraphs 1-166, wherein the gene has the symbol BAALC, optionally in combination with one or more genes labeled as 4.182 to 4.488 identified in Table 4.
168) In another aspect the invention employs one or more genes according to any one of paragraphs 1-167, wherein the gene has the symbol RP1-93H18.5, optionally in combination with one or more genes labeled as 4.183 to 4.488 identified in Table 4.
169) In another aspect the invention employs one or more genes according to any one of paragraphs 1-168, wherein the gene has the symbol FLJ20701, optionally in combination with one or more genes labeled as 4.184 to 4.488 identified in Table 4.
170) In another aspect the invention employs one or more genes according to any one of paragraphs 1-169, wherein the gene has the symbol SH3TC2, optionally in combination with one or more genes labeled as 4.185 to 4.488 identified in Table 4.
171) In another aspect the invention employs one or more genes according to any one of paragraphs 1-170, wherein the gene has the symbol CCR2, optionally in combination with one or more genes labeled as 4.186 to 4.488 identified in Table 4.
172) In another aspect the invention employs one or more genes according to any one of paragraphs 1-171, wherein the gene has the symbol CCL5, optionally in combination with one or more genes labeled as 4.187 to 4.488 identified in Table 4.
173) In another aspect the invention employs one or more genes according to any one of paragraphs 1-172, wherein the gene has the symbol HLA-DPA1, optionally in combination with one or more genes labeled as 4.189 to 4.488 identified in Table 4.
174) In another aspect the invention employs one or more genes according to any one of paragraphs 1-173, wherein the gene has the symbol PECAM1, optionally in combination with one or more genes labeled as 4.190 to 4.488 identified in Table 4.
175) In another aspect the invention employs one or more genes according to any one of paragraphs 1-174, wherein the gene has the symbol AMIGO2, optionally in combination with one or more genes labeled as 4.192 to 4.488 identified in Table 4.
176) In another aspect the invention employs one or more genes according to any one of paragraphs 1-175, wherein the gene has the symbol CLEC7A, optionally in combination with one or more genes labeled as 4.193 to 4.488 identified in Table 4.
177) In another aspect the invention employs one or more genes according to any one of paragraphs 1-176, wherein the gene has the symbol P2RY14, optionally in combination with one or more genes labeled as 4.194 to 4.488 identified in Table 4.
178) In another aspect the invention employs one or more genes according to any one of paragraphs 1-177, wherein the gene has the symbol PIK3AP1, optionally in combination with one or more genes labeled as 4.195 to 4.488 identified in Table 4.
179) In another aspect the invention employs one or more genes according to any one of paragraphs 1-178, wherein the gene has the symbol ADH1B, optionally in combination with one or more genes labeled as 4.196 to 4.488 identified in Table 4.
180) In another aspect the invention employs one or more genes according to any one of paragraphs 1-179, wherein the gene has the symbol TOP1MT, optionally in combination with one or more genes labeled as 4.197 to 4.488 identified in Table 4.
181) In another aspect the invention employs one or more genes according to any one of paragraphs 1-180, wherein the gene has the symbol CD276, optionally in combination with one or more genes labeled as 4.199 to 4.488 identified in Table 4.
182) In another aspect the invention employs one or more genes according to any one of paragraphs 1-181, wherein the gene has the symbol JAM2, optionally in combination with one or more genes labeled as 4.200 to 4.488 identified in Table 4.
183) In another aspect the invention employs one or more genes according to any one of paragraphs 1-182, wherein the gene has the symbol C1S, optionally in combination with one or more genes labeled as 4.202 to 4.488 identified in Table 4.
184) In another aspect the invention employs one or more genes according to any one of paragraphs 1-183, wherein the gene has the symbol TGFBR3, optionally in combination with one or more genes labeled as 4.203 to 4.488 identified in Table 4.
185) In another aspect the invention employs one or more genes according to any one of paragraphs 1-184, wherein the gene has the symbol ITGAL, optionally in combination with one or more genes labeled as 4.204 to 4.488 identified in Table 4.
186) In another aspect the invention employs one or more genes according to any one of paragraphs 1-185, wherein the gene has the symbol IL1R1, optionally in combination with one or more genes labeled as 4.206 to 4.488 identified in Table 4.
187) In another aspect the invention employs one or more genes according to any one of paragraphs 1-186, wherein the gene has the symbol HLA-DRB1, optionally in combination with one or more genes labeled as 4.207 to 4.488 identified in Table 4.
188) In another aspect the invention employs one or more genes according to any one of paragraphs 1-187, wherein the gene has the symbol GIMAP2, optionally in combination with one or more genes labeled as 4.208 to 4.488 identified in Table 4.
189) In another aspect the invention employs one or more genes according to any one of paragraphs 1-188, wherein the gene has the symbol ZC3H12D, optionally in combination with one or more genes labeled as 4.209 to 4.488 identified in Table 4.
190) In another aspect the invention employs one or more genes according to any one of paragraphs 1-189, wherein the gene has the symbol PCDH9, optionally in combination with one or more genes labeled as 4.210 to 4.488 identified in Table 4.
191) In another aspect the invention employs one or more genes according to any one of paragraphs 1-190, wherein the gene has the symbol SLAMF7, optionally in combination with one or more genes labeled as 4.211 to 4.488 identified in Table 4.
192) In another aspect the invention employs one or more genes according to any one of paragraphs 1-191, wherein the gene has the symbol MGC7036, optionally in combination with one or more genes labeled as 4.212 to 4.488 identified in Table 4.
193) In another aspect the invention employs one or more genes according to any one of paragraphs 1-192, wherein the gene has the symbol RGS18, optionally in combination with one or more genes labeled as 4.214 to 4.488 identified in Table 4.
194) In another aspect the invention employs one or more genes according to any one of paragraphs 1-193, wherein the gene has the symbol CD53, optionally in combination with one or more genes labeled as 4.215 to 4.488 identified in Table 4.
195) In another aspect the invention employs one or more genes according to any one of paragraphs 1-194, wherein the gene has the symbol MPEG1, optionally in combination with one or more genes labeled as 4.216 to 4.488 identified in Table 4.
196) In another aspect the invention employs one or more genes according to any one of paragraphs 1-195, wherein the gene has the symbol SSBP4, optionally in combination with one or more genes labeled as 4.217 to 4.488 identified in Table 4.
197) In another aspect the invention employs one or more genes according to any one of paragraphs 1-196, wherein the gene is identifiable by probe set number 231262_at, optionally in combination with one or more genes labeled as 4.218 to 4.488 identified in Table 4.
198) In another aspect the invention employs one or more genes according to any one of paragraphs 1-197, wherein the gene has the symbol CDH19, optionally in combination with one or more genes labeled as 4.219 to 4.488 identified in Table 4.
199) In another aspect the invention employs one or more genes according to any one of paragraphs 1-198, wherein the gene has the symbol CTBP2, optionally in combination with one or more genes labeled as 4.221 to 4.488 identified in Table 4.
200) In another aspect the invention employs one or more genes according to any one of paragraphs 1-199, wherein the gene has the symbol FAM107B, optionally in combination with one or more genes labeled as 4.222 to 4.488 identified in Table 4.
201) In another aspect the invention employs one or more genes according to any one of paragraphs 1-200, wherein the gene has the symbol IGKC, optionally in combination with one or more genes labeled as 4.223 to 4.488 identified in Table 4.
202) In another aspect the invention employs one or more genes according to any one of paragraphs 1-201, wherein the gene has the symbol ITGAM, optionally in combination with one or more genes labeled as 4.224 to 4.488 identified in Table 4.
203) In another aspect the invention employs one or more genes according to any one of paragraphs 1-202, wherein the gene has the symbol CKAP1, optionally in combination with one or more genes labeled as 4.227 to 4.488 identified in Table 4.
204) In another aspect the invention employs one or more genes according to any one of paragraphs 1-203, wherein the gene has the symbol MGC16291, optionally in combination with one or more genes labeled as 4.228 to 4.488 identified in Table 4.
205) In another aspect the invention employs one or more genes according to any one of paragraphs 1-204, wherein the gene has the symbol DDEF2, optionally in combination with one or more genes labeled as 4.229 to 4.488 identified in Table 4.
206) In another aspect the invention employs one or more genes according to any one of paragraphs 1-205, wherein the gene has the symbol TNFAIP2, optionally in combination with one or more genes labeled as 4.230 to 4.488 identified in Table 4.
207) In another aspect the invention employs one or more genes according to any one of paragraphs 1-206, wherein the gene has the symbol CXCL14, optionally in combination with one or more genes labeled as 4.231 to 4.488 identified in Table 4.
208) In another aspect the invention employs one or more genes according to any one of paragraphs 1-207, wherein the gene has the symbol CD209, optionally in combination with one or more genes labeled as 4.232 to 4.488 identified in Table 4.
209) In another aspect the invention employs one or more genes according to any one of paragraphs 1-208, wherein the gene has the symbol COL9A3, optionally in combination with one or more genes labeled as 4.233 to 4.488 identified in Table 4.
210) In another aspect the invention employs one or more genes according to any one of paragraphs 1-209, wherein the gene has the symbol ANKRD22, optionally in combination with one or more genes labeled as 4.234 to 4.488 identified in Table 4.
211) In another aspect the invention employs one or more genes according to any one of paragraphs 1-210, wherein the gene has the symbol NCKAP1L, optionally in combination with one or more genes labeled as 4.235 to 4.488 identified in Table 4.
212) In another aspect the invention employs one or more genes according to any one of paragraphs 1-211, wherein the gene has the symbol CMKOR1, optionally in combination with one or more genes labeled as 4.236 to 4.488 identified in Table 4.
213) In another aspect the invention employs one or more genes according to any one of paragraphs 1-212, wherein the gene has the symbol HLA-DRB5, optionally in combination with one or more genes labeled as 4.237 to 4.488 identified in Table 4.
214) In another aspect the invention employs one or more genes according to any one of paragraphs 1-213, wherein the gene has the symbol LCP1, optionally in combination with one or more genes labeled as 4.239 to 4.488 identified in Table 4.
215) In another aspect the invention employs one or more genes according to any one of paragraphs 1-214, wherein the gene has the symbol CXXC5, optionally in combination with one or more genes labeled as 4.240 to 4.488 identified in Table 4.
216) In another aspect the invention employs one or more genes according to any one of paragraphs 1-215, wherein the gene has the symbol GJA7, optionally in combination with one or more genes labeled as 4.241 to 4.488 identified in Table 4.
217) In another aspect the invention employs one or more genes according to any one of paragraphs 1-216, wherein the gene has the symbol FGD2, optionally in combination with one or more genes labeled as 4.242 to 4.488 identified in Table 4.
218) In another aspect the invention employs one or more genes according to any one of paragraphs 1-217, wherein the gene has the symbol MAN1A1, optionally in combination with one or more genes labeled as 4.243 to 4.488 identified in Table 4.
219) In another aspect the invention employs one or more genes according to any one of paragraphs 1-218, wherein the gene has the symbol C6orf115, optionally in combination with one or more genes labeled as 4.245 to 4.488 identified in Table 4.
220) In another aspect the invention employs one or more genes according to any one of paragraphs 1-219, wherein the gene has the symbol CXCL9, optionally in combination with one or more genes labeled as 4.247 to 4.488 identified in Table 4.
221) In another aspect the invention employs one or more genes according to any one of paragraphs 1-220, wherein the gene has the symbol NPR3, optionally in combination with one or more genes labeled as 4.248 to 4.488 identified in Table 4.
222) In another aspect the invention employs one or more genes according to any one of paragraphs 1-221, wherein the gene has the symbol FYB, optionally in combination with one or more genes labeled as 4.249 to 4.488 identified in Table 4.
223) In another aspect the invention employs one or more genes according to any one of paragraphs 1-222, wherein the gene has the symbol VCAM1, optionally in combination with one or more genes labeled as 4.250 to 4.488 identified in Table 4.
224) In another aspect the invention employs one or more genes according to any one of paragraphs 1-223, wherein the gene has the symbol FLI1, optionally in combination with one or more genes labeled as 4.251 to 4.488 identified in Table 4.
225) In another aspect the invention employs one or more genes according to any one of paragraphs 1-224, wherein the gene has the symbol CXXC5, optionally in combination with one or more genes labeled as 4.252 to 4.488 identified in Table 4.
226) In another aspect the invention employs one or more genes according to any one of paragraphs 1-225, wherein the gene has the symbol TRAM2, optionally in combination with one or more genes labeled as 4.254 to 4.488 identified in Table 4.
227) In another aspect the invention employs one or more genes according to any one of paragraphs 1-226, wherein the gene has the symbol SHC4, optionally in combination with one or more genes labeled as 4.255 to 4.488 identified in Table 4.
228) In another aspect the invention employs one or more genes according to any one of paragraphs 1-227, wherein the gene has the symbol SLC9A9, optionally in combination with one or more genes labeled as 4.256 to 4.488 identified in Table 4.
229) In another aspect the invention employs one or more genes according to any one of paragraphs 1-228, wherein the gene has the symbol PTPRC, optionally in combination with one or more genes labeled as 4.257 to 4.488 identified in Table 4.
230) In another aspect the invention employs one or more genes according to any one of paragraphs 1-229, wherein the gene has the symbol PTGER4, optionally in combination with one or more genes labeled as 4.258 to 4.488 identified in Table 4.
231) In another aspect the invention employs one or more genes according to any one of paragraphs 1-230, wherein the gene has the symbol LILRB1, optionally in combination with one or more genes labeled as 4.259 to 4.488 identified in Table 4.
232) In another aspect the invention employs one or more genes according to any one of paragraphs 1-231, wherein the gene has the symbol PRDM1, optionally in combination with one or more genes labeled as 4.261 to 4.488 identified in Table 4.
233) In another aspect the invention employs one or more genes according to any one of paragraphs 1-232, wherein the gene has the symbol ARHGAP15, optionally in combination with one or more genes labeled as 4.262 to 4.488 identified in Table 4.
234) In another aspect the invention employs one or more genes according to any one of paragraphs 1-233, wherein the gene has the symbol SLC5A3, optionally in combination with one or more genes labeled as 4.263 to 4.488 identified in Table 4.
235) In another aspect the invention employs one or more genes according to any one of paragraphs 1-234, wherein the gene has the symbol DOCK9, optionally in combination with one or more genes labeled as 4.264 to 4.488 identified in Table 4.
236) In another aspect the invention employs one or more genes according to any one of paragraphs 1-235, wherein the gene has the symbol GPSM1, optionally in combination with one or more genes labeled as 4.265 to 4.488 identified in Table 4.
237) In another aspect the invention employs one or more genes according to any one of paragraphs 1-236, wherein the gene has the symbol CCL5, optionally in combination with one or more genes labeled as 4.266 to 4.488 identified in Table 4.
238) In another aspect the invention employs one or more genes according to any one of paragraphs 1-237, wherein the gene has the symbol GLIPR1, optionally in combination with one or more genes labeled as 4.267 to 4.488 identified in Table 4.
239) In another aspect the invention employs one or more genes according to any one of paragraphs 1-238, wherein the gene has the symbol APOL3, optionally in combination with one or more genes labeled as 4.268 to 4.488 identified in Table 4.
240) In another aspect the invention employs one or more genes according to any one of paragraphs 1-239, wherein the gene has the symbol HLA-DMB, optionally in combination with one or more genes labeled as 4.269 to 4.488 identified in Table 4.
241) In another aspect the invention employs one or more genes according to any one of paragraphs 1-240, wherein the gene has the symbol SYNPO2, optionally in combination with one or more genes labeled as 4.270 to 4.488 identified in Table 4.
242) In another aspect the invention employs one or more genes according to any one of paragraphs 1-241, wherein the gene is identifiable by probe set number 221651_x_at, optionally in combination with one or more genes labeled as 4.271 to 4.488 identified in Table 4.
243) In another aspect the invention employs one or more genes according to any one of paragraphs 1-242, wherein the gene is identifiable by probe set number 231929_at, optionally in combination with one or more genes labeled as 4.273 to 4.488 identified in Table 4.
244) In another aspect the invention employs one or more genes according to any one of paragraphs 1-243, wherein the gene has the symbol CASP1, optionally in combination with one or more genes labeled as 4.274 to 4.488 identified in Table 4.
245) In another aspect the invention employs one or more genes according to any one of paragraphs 1-244, wherein the gene has the symbol PRKCQ, optionally in combination with one or more genes labeled as 4.275 to 4.488 identified in Table 4.
246) In another aspect the invention employs one or more genes according to any one of paragraphs 1-245, wherein the gene has the symbol IL1R2, optionally in combination with one or more genes labeled as 4.276 to 4.488 identified in Table 4.
247) In another aspect the invention employs one or more genes according to any one of paragraphs 1-246, wherein the gene has the symbol CARD15, optionally in combination with one or more genes labeled as 4.277 to 4.488 identified in Table 4.
248) In another aspect the invention employs one or more genes according to any one of paragraphs 1-247, wherein the gene has the symbol ARHGDIB, optionally in combination with one or more genes labeled as 4.278 to 4.488 identified in Table 4.
249) In another aspect the invention employs one or more genes according to any one of paragraphs 1-248, wherein the gene has the symbol HLA-DRB4, optionally in combination with one or more genes labeled as 4.279 to 4.488 identified in Table 4.
250) In another aspect the invention employs one or more genes according to any one of paragraphs 1-249, wherein the gene has the symbol SART2, optionally in combination with one or more genes labeled as 4.280 to 4.488 identified in Table 4.
251) In another aspect the invention employs one or more genes according to any one of paragraphs 1-250, wherein the gene has the symbol LSP1, optionally in combination with one or more genes labeled as 4.281 to 4.488 identified in Table 4.
252) In another aspect the invention employs one or more genes according to any one of paragraphs 1-251, wherein the gene has the symbol AMPD3, optionally in combination with one or more genes labeled as 4.282 to 4.488 identified in Table 4.
253) In another aspect the invention employs one or more genes according to any one of paragraphs 1-252, wherein the gene has the symbol SEMA4F, optionally in combination with one or more genes labeled as 4.283 to 4.488 identified in Table 4.
254) In another aspect the invention employs one or more genes according to any one of paragraphs 1-253, wherein the gene has the symbol ISOC1, optionally in combination with one or more genes labeled as 4.285 to 4.488 identified in Table 4.
255) In another aspect the invention employs one or more genes according to any one of paragraphs 1-254, wherein the gene has the symbol HPS3, optionally in combination with one or more genes labeled as 4.288 to 4.488 identified in Table 4.
256) In another aspect the invention employs one or more genes according to any one of paragraphs 1-255, wherein the gene has the symbol HOXB7, optionally in combination with one or more genes labeled as 4.290 to 4.488 identified in Table 4.
257) In another aspect the invention employs one or more genes according to any one of paragraphs 1-256, wherein the gene has the symbol ZNFN1A1, optionally in combination with one or more genes labeled as 4.291 to 4.488 identified in Table 4.
258) In another aspect the invention employs one or more genes according to any one of paragraphs 1-257, wherein the gene has the symbol ARHGAP9, optionally in combination with one or more genes labeled as 4.292 to 4.488 identified in Table 4.
259) In another aspect the invention employs one or more genes according to any one of paragraphs 1-258, wherein the gene has the symbol GATA2, optionally in combination with one or more genes labeled as 4.293 to 4.488 identified in Table 4.
260) In another aspect the invention employs one or more genes according to any one of paragraphs 1-259, wherein the gene has the symbol AP2B1, optionally in combination with one or more genes labeled as 4.294 to 4.488 identified in Table 4.
261) In another aspect the invention employs one or more genes according to any one of paragraphs 1-260, wherein the gene has the symbol CTSC, optionally in combination with one or more genes labeled as 4.295 to 4.488 identified in Table 4.
262) In another aspect the invention employs one or more genes according to any one of paragraphs 1-261, wherein the gene has the symbol PLK2, optionally in combination with one or more genes labeled as 4.296 to 4.488 identified in Table 4.
263) In another aspect the invention employs one or more genes according to any one of paragraphs 1-262, wherein the gene has the symbol CD4, optionally in combination with one or more genes labeled as 4.297 to 4.488 identified in Table 4.
264) In another aspect the invention employs one or more genes according to any one of paragraphs 1-263, wherein the gene has the symbol GGTA1, optionally in combination with one or more genes labeled as 4.298 to 4.488 identified in Table 4.
265) In another aspect the invention employs one or more genes according to any one of paragraphs 1-264, wherein the gene has the symbol GADD45B, optionally in combination with one or more genes labeled as 4.300 to 4.488 identified in Table 4.
266) In another aspect the invention employs one or more genes according to any one of paragraphs 1-265, wherein the gene has the symbol FLJ10847, optionally in combination with one or more genes labeled as 4.301 to 4.488 identified in Table 4.
267) In another aspect the invention employs one or more genes according to any one of paragraphs 1-266, wherein the gene has the symbol KIF21B, optionally in combination with one or more genes labeled as 4.302 to 4.488 identified in Table 4.
268) In another aspect the invention employs one or more genes according to any one of paragraphs 1-267, wherein the gene has the symbol CCND2, optionally in combination with one or more genes labeled as 4.303 to 4.488 identified in Table 4.
269) In another aspect the invention employs one or more genes according to any one of paragraphs 1-268, wherein the gene has the symbol PRG1, optionally in combination with one or more genes labeled as 4.304 to 4.488 identified in Table 4.
270) In another aspect the invention employs one or more genes according to any one of paragraphs 1-269, wherein the gene has the symbol SLC40A1, optionally in combination with one or more genes labeled as 4.307 to 4.488 identified in Table 4.
271) In another aspect the invention employs one or more genes according to any one of paragraphs 1-270, wherein the gene has the symbol CRIP1, optionally in combination with one or more genes labeled as 4.308 to 4.488 identified in Table 4.
272) In another aspect the invention employs one or more genes according to any one of paragraphs 1-271, wherein the gene has the symbol LOC283070, optionally in combination with one or more genes labeled as 4.309 to 4.488 identified in Table 4.
273) In another aspect the invention employs one or more genes according to any one of paragraphs 1-272, wherein the gene has the symbol SIGLEC1, optionally in combination with one or more genes labeled as 4.310 to 4.488 identified in Table 4.
274) In another aspect the invention employs one or more genes according to any one of paragraphs 1-273, wherein the gene has the symbol ZNF11B, optionally in combination with one or more genes labeled as 4.311 to 4.488 identified in Table 4.
275) In another aspect the invention employs one or more genes according to any one of paragraphs 1-274, wherein the gene has the symbol CXCR4, optionally in combination with one or more genes labeled as 4.312 to 4.488 identified in Table 4.
276) In another aspect the invention employs one or more genes according to any one of paragraphs 1-275, wherein the gene has the symbol HLA-DMA, optionally in combination with one or more genes labeled as 4.313 to 4.488 identified in Table 4
277) In another aspect the invention employs one or more genes according to any one of paragraphs 1-276, wherein the gene has the symbol MRC1, optionally in combination with one or more genes labeled as 4.315 to 4.488 identified in Table 4.
278) In another aspect the invention employs one or more genes according to any one of paragraphs 1-277, wherein the gene has the symbol LMO2, optionally in combination with one or more genes labeled as 4.315a to 4.488 identified in Table 4.
279) In another aspect the invention employs one or more genes according to any one of paragraphs 1-278, wherein the gene has the symbol DENND2D, optionally in combination with one or more genes labeled as 4.316 to 4.488 identified in Table 4.
280) In another aspect the invention employs one or more genes according to any one of paragraphs 1-279, wherein the gene has the symbol CCL18, optionally in combination with one or more genes labeled as 4.317 to 4.488 identified in Table 4.
281) In another aspect the invention employs one or more genes according to any one of paragraphs 1-280, wherein the gene has the symbol P2RY13, optionally in combination with one or more genes labeled as 4.319 to 4.488 identified in Table 4.
282) In another aspect the invention employs one or more genes according to any one of paragraphs 1-281, wherein the gene has the symbol ANGPTL1, optionally in combination with one or more genes labeled as 4.320 to 4.488 identified in Table 4.
283) In another aspect the invention employs one or more genes according to any one of paragraphs 1-282, wherein the gene is identifiable by probe set number 230391_at, optionally in combination with one or more genes labeled as 4.322 to 4.488 identified in Table 4.
284) In another aspect the invention employs one or more genes according to any one of paragraphs 1-283, wherein the gene has the symbol C8orf51, optionally in combination with one or more genes labeled as 4.323 to 4.488 identified in Table 4.
285) In another aspect the invention employs one or more genes according to any one of paragraphs 1-284, wherein the gene has the symbol GIMAP8, optionally in combination with one or more genes labeled as 4.324 to 4.488 identified in Table 4.
286) In another aspect the invention employs one or more genes according to any one of paragraphs 1-285, wherein the gene is identifiable by probe set number 2277880_s_at, optionally in combination with one or more genes labeled as 4.325 to 4.488 identified in Table 4.
287) In another aspect the invention employs one or more genes according to any one of paragraphs 1-286, wherein the gene has the symbol JAK2, optionally in combination with one or more genes labeled as 4.326 to 4.488 identified in Table 4.
288) In another aspect the invention employs one or more genes according to any one of paragraphs 1-287, wherein the gene has the symbol TNFSF10, optionally in combination with one or more genes labeled as 4.327 to 4.488 identified in Table 4.
289) In another aspect the invention employs one or more genes according to any one of paragraphs 1-288, wherein the gene has the symbol C1R, optionally in combination with one or more genes labeled as 4.328 to 4.488 identified in Table 4.
290) In another aspect the invention employs one or more genes according to any one of paragraphs 1-289, wherein the gene has the symbol ACPL2, optionally in combination with one or more genes labeled as 4.329 to 4.488 identified in Table 4.
291) In another aspect the invention employs one or more genes according to any one of paragraphs 1-290, wherein the gene has the symbol TNFRSF19, optionally in combination with one or more genes labeled as 4.331 to 4.488 identified in Table 4.
292) In another aspect the invention employs one or more genes according to any one of paragraphs 1-291, wherein the gene has the symbol LRP12, optionally in combination with one or more genes labeled as 4.332 to 4.488 identified in Table 4.
293) In another aspect the invention employs one or more genes according to any one of paragraphs 1-292, wherein the gene is identifiable by probe set number 1557116_at, optionally in combination with one or more genes labeled as 4.334 to 4.488 identified in Table 4.
294) In another aspect the invention employs one or more genes according to any one of paragraphs 1-293, wherein the gene has the symbol PRKCB1, optionally in combination with one or more genes labeled as 4.335 to 4.488 identified in Table 4.
295) In another aspect the invention employs one or more genes according to any one of paragraphs 1-294, wherein the gene has the symbol IPO11, optionally in combination with one or more genes labeled as 4.336 to 4.488 identified in Table 4.
296) In another aspect the invention employs one or more genes according to any one of paragraphs 1-295, wherein the gene has the symbol DLGAP1, optionally in combination with one or more genes labeled as 4.337 to 4.488 identified in Table 4.
297) In another aspect the invention employs one or more genes according to any one of paragraphs 1-296, wherein the gene has the symbol PRKAR2B, optionally in combination with one or more genes labeled as 4.338 to 4.488 identified in Table 4.
298) In another aspect the invention employs one or more genes according to any one of paragraphs 1-297, wherein the gene has the symbol MAP3K8, optionally in combination with one or more genes labeled as 4.339 to 4.488 identified in Table 4.
299) In another aspect the invention employs one or more genes according to any one of paragraphs 1-298, wherein the gene has the symbol EVI2B, optionally in combination with one or more genes labeled as 4.340 to 4.488 identified in Table 4.
300) In another aspect the invention employs one or more genes according to any one of paragraphs 1-299, wherein the gene has the symbol GBP1, optionally in combination with one or more genes labeled as 4.341 to 4.488 identified in Table 4.
301) In another aspect the invention employs one or more genes according to any one of paragraphs 1-300, wherein the gene has the symbol CXCL10, optionally in combination with one or more genes labeled as 4.342 to 4.488 identified in Table 4.
302) In another aspect the invention employs one or more genes according to any one of paragraphs 1-301, wherein the gene has the symbol CAMK2N1, optionally in combination with one or more genes labeled as 4.343 to 4.488 identified in Table 4
303) In another aspect the invention employs one or more genes according to any one of paragraphs 1-302, wherein the gene has the symbol MED12L, optionally in combination with one or more genes labeled as 4.344 to 4.488 identified in Table 4.
304) In another aspect the invention employs one or more genes according to any one of paragraphs 1-303, wherein the gene has the symbol ID2, optionally in combination with one or more genes labeled as 4.345 to 4.488 identified in Table 4.
305) In another aspect the invention employs one or more genes according to any one of paragraphs 1-304, wherein the gene has the symbol CTBP2, optionally in combination with one or more genes labeled as 4.346 to 4.488 identified in Table 4.
306) In another aspect the invention employs one or more genes according to any one of paragraphs 1-305, wherein the gene has the symbol IGLJ3, optionally in combination with one or more genes labeled as 4.347 to 4.488 identified in Table 4.
307) In another aspect the invention employs one or more genes according to any one of paragraphs 1-306, wherein the gene has the symbol GBP4, optionally in combination with one or more genes labeled as 4.348 to 4.488 identified in Table 4.
308) In another aspect the invention employs one or more genes according to any one of paragraphs 1-307, wherein the gene has the symbol LOC439949, optionally in combination with one or more genes labeled as 4.349 to 4.488 identified in Table 4.
309) In another aspect the invention employs one or more genes according to any one of paragraphs 1-308, wherein the gene has the symbol FBXO16 optionally in combination with one or more genes labeled as 4.350 to 4.488 identified in Table 4.
310) In another aspect the invention employs one or more genes according to any one of paragraphs 1-309, wherein the gene has the symbol PRF1, optionally in combination with one or more genes labeled as 4.351 to 4.488 identified in Table 4.
311) In another aspect the invention employs one or more genes according to any one of paragraphs 1-310, wherein the gene has the symbol TRAM2, optionally in combination with one or more genes labeled as 4.352 to 4.488 identified in Table 4.
312) In another aspect the invention employs one or more genes according to any one of paragraphs 1-311, wherein the gene has the symbol LYN, optionally in combination with one or more genes labeled as 4.353 to 4.488 identified in Table 4.
313) In another aspect the invention employs one or more genes according to any one of paragraphs 1-312, wherein the gene has the symbol CENTD1, optionally in combination with one or more genes labeled as 4.355 to 4.488 identified in Table 4.
314) In another aspect the invention employs one or more genes according to any one of paragraphs 1-313, wherein the gene has the symbol FLJ20273, optionally in combination with one or more genes labeled as 4.356 to 4.488 identified in Table 4.
315) In another aspect the invention employs one or more genes according to any one of paragraphs 1-314, wherein the gene has the symbol TFEC, optionally in combination with one or more genes labeled as 4.357 to 4.488 identified in Table 4.
316) In another aspect the invention employs one or more genes according to any one of paragraphs 1-315, wherein the gene has the symbol PPP1R16B, optionally in combination with one or more genes labeled as 4.358 to 4.488 identified in Table 4.
317) In another aspect the invention employs one or more genes according to any one of paragraphs 1-316, wherein the gene has the symbol CD48, optionally in combination with one or more genes labeled as 4.359 to 4.488 identified in Table 4.
318) In another aspect the invention employs one or more genes according to any one of paragraphs 1-317, wherein the gene has the symbol HLA-DPB1, optionally in combination with one or more genes labeled as 4.361 to 4.488 identified in Table 4.
319) In another aspect the invention employs one or more genes according to any one of paragraphs 1-318, wherein the gene has the symbol GTPBP5, optionally in combination with one or more genes labeled as 4.362 to 4.488 identified in Table 4.
320) In another aspect the invention employs one or more genes according to any one of paragraphs 1-319, wherein the gene has the symbol GBP5, optionally in combination with one or more genes labeled as 4.363 to 4.488 identified in Table 4.
321) In another aspect the invention employs one or more genes according to any one of paragraphs 1-320, wherein the gene has the symbol MAP1B, optionally in combination with one or more genes labeled as 4.364 to 4.488 identified in Table 4.
322) In another aspect the invention employs one or more genes according to any one of paragraphs 1-321, wherein the gene has the symbol EXTL3, optionally in combination with one or more genes labeled as 4.365 to 4.488 identified in Table 4.
323) In another aspect the invention employs one or more genes according to any one of paragraphs 1-322, wherein the gene has the symbol CORO1A, optionally in combination with one or more genes labeled as 4.366 to 4.488 identified in Table 4.
324) In another aspect the invention employs one or more genes according to any one of paragraphs 1-323, wherein the gene has the symbol PDGFRL, optionally in combination with one or more genes labeled as 4.367 to 4.488 identified in Table 4.
325) In another aspect the invention employs one or more genes according to any one of paragraphs 1-324, wherein the gene has the symbol RP9, optionally in combination with one or more genes labeled as 4.368 to 4.488 identified in Table 4.
326) In another aspect the invention employs one or more genes according to any one of paragraphs 1-325, wherein the gene has the symbol RHOU, optionally in combination with one or more genes labeled as 4.369 to 4.488 identified in Table 4.
327) In another aspect the invention employs one or more genes according to any one of paragraphs 1-326, wherein the gene has the symbol MTAC2D1, optionally in combination with one or more genes labeled as 4.370 to 4.488 identified in Table 4.
328) In another aspect the invention employs one or more genes according to any one of paragraphs 1-327, wherein the gene has the symbol CCL8, optionally in combination with one or more genes labeled as 4.371 to 4.488 identified in Table 4.
329) In another aspect the invention employs one or more genes according to any one of paragraphs 1-328, wherein the gene has the symbol CECR1, optionally in combination with one or more genes labeled as 4.373 to 4.488 identified in Table 4.
330) In another aspect the invention employs one or more genes according to any one of paragraphs 1-329, wherein the gene has the symbol SLC40A1, optionally in combination with one or more genes labeled as 4.374 to 4.488 identified in Table 4.
331) In another aspect the invention employs one or more genes according to any one of paragraphs 1-330, wherein the gene has the symbol ADCY6, optionally in combination with one or more genes labeled as 4.375 to 4.488 identified in Table 4.
332) In another aspect the invention employs one or more genes according to any one of paragraphs 1-331, wherein the gene has the symbol CP, optionally in combination with one or more genes labeled as 4.376 to 4.488 identified in Table 4.
333) In another aspect the invention employs one or more genes according to any one of paragraphs 1-332, wherein the gene has the symbol EDG1, optionally in combination with one or more genes labeled as 4.377 to 4.488 identified in Table 4.
334) In another aspect the invention employs one or more genes according to any one of paragraphs 1-333, wherein the gene has the symbol RGS3, optionally in combination with one or more genes labeled as 4.379 to 4.488 identified in Table 4.
335) In another aspect the invention employs one or more genes according to any one of paragraphs 1-334, wherein the gene is identifiable by probe set number 228339_at, optionally in combination with one or more genes labeled as 4.380 to 4.488 identified in Table 4.
336) In another aspect the invention employs one or more genes according to any one of paragraphs 1-335, wherein the gene has the symbol ABHD5, optionally in combination with one or more genes labeled as 4.381 to 4.488 identified in Table 4.
337) In another aspect the invention employs one or more genes according to any one of paragraphs 1-336, wherein the gene has the symbol MS4A7, optionally in combination with one or more genes labeled as 4.382 to 4.488 identified in Table 4.
338) In another aspect the invention employs one or more genes according to any one of paragraphs 1-337, wherein the gene has the symbol PRKCH, optionally in combination with one or more genes labeled as 4.384 to 4.488 identified in Table 4.
339) In another aspect the invention employs one or more genes according to any one of paragraphs 1-338, wherein the gene has the symbol LOC286071, optionally in combination with one or more genes labeled as 4.385 to 4.488 identified in Table 4.
340) In another aspect the invention employs one or more genes according to any one of paragraphs 1-339, wherein the gene has the symbol BLNK, optionally in combination with one or more genes labeled as 4.386 to 4.488 identified in Table 4.
341) In another aspect the invention employs one or more genes according to any one of paragraphs 1-340, wherein the gene is identifiable by the probe set number 242546_at, optionally in combination with one or more genes labeled as 4.387 to 4.488 identified in Table 4.
342) In another aspect the invention employs one or more genes according to any one of paragraphs 1-341, wherein the gene has the symbol PCDHGC3, optionally in combination with one or more genes labeled as 4.390 to 4.488 identified in Table 4.
343) In another aspect the invention employs one or more genes according to any one of paragraphs 1-342, wherein the gene has the symbol CAMSAP1L1, optionally in combination with one or more genes labeled as 4.391 to 4.488 identified in Table 4.
344) In another aspect the invention employs one or more genes according to any one of paragraphs 1-343, wherein the gene has the symbol NPY1R, optionally in combination with one or more genes labeled as 4.392 to 4.488 identified in Table 4.
345) In another aspect the invention employs one or more genes according to any one of paragraphs 1-344, wherein the gene has the symbol CD274, optionally in combination with one or more genes labeled as 4.393 to 4.488 identified in Table 4.
346) In another aspect the invention employs one or more genes according to any one of paragraphs 1-345, wherein the gene has the symbol PGM5, optionally in combination with one or more genes labeled as 4.394 to 4.488 identified in Table 4.
347) In another aspect the invention employs one or more genes according to any one of paragraphs 1-346, wherein the gene has the symbol PLCG2, optionally in combination with one or more genes labeled as 4.395 to 4.488 identified in Table 4.
348) In another aspect the invention employs one or more genes according to any one of paragraphs 1-347, wherein the gene has the symbol TNFSF10, optionally in combination with one or more genes labeled as 4.397 to 4.488 identified in Table 4.
349) In another aspect the invention employs one or more genes according to any one of paragraphs 1-348, wherein the gene has the symbol BTG2, optionally in combination with one or more genes labeled as 4.398 to 4.488 identified in Table 4.
350) In another aspect the invention employs one or more genes according to any one of paragraphs 1-349, wherein the gene has the symbol LAMP3, optionally in combination with one or more genes labeled as 4.399 to 4.488 identified in Table 4.
351) In another aspect the invention employs one or more genes according to any one of paragraphs 1-350, wherein the gene has the symbol IGLC1, optionally in combination with one or more genes labeled as 4.400 to 4.488 identified in Table 4.
352) In another aspect the invention employs one or more genes according to any one of paragraphs 1-351, wherein the gene has the symbol SIPA1L1, optionally in combination with one or more genes labeled as 4.401 to 4.488 identified in Table 4.
353) In another aspect the invention employs one or more genes according to any one of paragraphs 1-352 wherein the gene has the symbol AIF1, optionally in combination with one or more genes labeled as 4.402 to 4.488 identified in Table 4.
354) In another aspect the invention employs one or more genes according to any one of paragraphs 1-353, wherein the gene has the symbol IGLC2, optionally in combination with one or more genes labeled as 4.403 to 4.488 identified in Table 4.
355) In another aspect the invention employs one or more genes according to any one of paragraphs 1-354, wherein the gene has the symbol B2M, optionally in combination with one or more genes labeled as 4.404 to 4.488 identified in Table 4.
356) In another aspect the invention employs one or more genes according to any one of paragraphs 1-355, wherein the gene has the symbol CLEC7A, optionally in combination with one or more genes labeled as 4.405 to 4.488 identified in Table 4.
357) In another aspect the invention employs one or more genes according to any one of paragraphs 1-356, wherein the gene has the symbol MGC17330, optionally in combination with one or more genes labeled as 4.406 to 4.488 identified in Table 4.
358) In another aspect the invention employs one or more genes according to any one of paragraphs 1-357, wherein the gene has the symbol IGF1R, optionally in combination with one or more genes labeled as 4.407 to 4.488 identified in Table 4.
359) In another aspect the invention employs one or more genes according to any one of paragraphs 1-358, wherein the gene has the symbol HIVEP1, optionally in combination with one or more genes labeled as 4.408 to 4.488 identified in Table 4.
360) In another aspect the invention employs one or more genes according to any one of paragraphs 1-359, wherein the gene has the symbol FKBP14, optionally in combination with one or more genes labeled as 4.409 to 4.488 identified in Table 4.
361) In another aspect the invention employs one or more genes according to any one of paragraphs 1-360, wherein the gene has the symbol LAPTM5, optionally in combination with one or more genes labeled as 4.410 to 4.488 identified in Table 4.
362) In another aspect the invention employs one or more genes according to any one of paragraphs 1-361, wherein the gene has the symbol AB13BP, optionally in combination with one or more genes labeled as 4.411 to 4.488 identified in Table 4.
363) In another aspect the invention employs one or more genes according to any one of paragraphs 1-362, wherein the gene has the symbol HLA-E, optionally in combination with one or more genes labeled as 4.412 to 4.488 identified in Table 4.
364) In another aspect the invention employs one or more genes according to any one of paragraphs 1-363, wherein the gene has the symbol ARL4C, optionally in combination with one or more genes labeled as 4.413 to 4.488 identified in Table 4.
365) In another aspect the invention employs one or more genes according to any one of paragraphs 1-364, wherein the gene has the symbol ASS, optionally in combination with one or more genes labeled as 4.415 to 4.488 identified in Table 4.
366) In another aspect the invention employs one or more genes according to any one of paragraphs 1-365, wherein the gene has the symbol ITGB3, optionally in combination with one or more genes labeled as 4.417 to 4.488 identified in Table 4.
367) In another aspect the invention employs one or more genes according to any one of paragraphs 1-366, wherein the gene has the symbol optionally in combination with one or more genes labeled as 4.417 to 4.488 identified in Table 4.
368) In another aspect the invention employs one or more genes according to any one of paragraphs 1-366, wherein the gene has the symbol RAC2, optionally in combination with one or more genes labeled as 4.418 to 4.488 identified in Table 4.
369) In another aspect the invention employs one or more genes according to any one of paragraphs 1-368, wherein the gene is identifiable by probe set number 1557222_at, optionally in combination with one or more genes labeled as 4.419 to 4.488 identified in Table 4.
370) In another aspect the invention employs one or more genes according to any one of paragraphs 1-369, wherein the gene has the symbol CD3G, optionally in combination with one or more genes labeled as 4.420 to 4.488 identified in Table 4.
371) In another aspect the invention employs one or more genes according to any one of paragraphs 1-370, wherein the gene has the symbol IGF1, optionally in combination with one or more genes labeled as 4.421 to 4.488 identified in Table 4.
372) In another aspect the invention employs one or more genes according to any one of paragraphs 1-371, wherein the gene is identifiable by probe set number 228858_at, optionally in combination with one or more genes labeled as 4.422 to 4.488 identified in Table 4.
373) In another aspect the invention employs one or more genes according to any one of paragraphs 1-372, wherein the gene is has the symbol CYB5A, optionally in combination with one or more genes labeled as 4.423 to 4.488 identified in Table 4.
374) In another aspect the invention employs one or more genes according to any one of paragraphs 1-373, wherein the gene is has the symbol TTC25, optionally in combination with one or more genes labeled as 4.424 to 4.488 identified in Table 4.
375) In another aspect the invention employs one or more genes according to any one of paragraphs 1-374, wherein the gene is has the symbol SLAMF6, optionally in combination with one or more genes labeled as 4.425 to 4.488 identified in Table 4.
376) In another aspect the invention employs one or more genes according to any one of paragraphs 1-375, wherein the gene is has the symbol ARHGAP21, optionally in combination with one or more genes labeled as 4.426 to 4.488 identified in Table 4.
377) In another aspect the invention employs one or more genes according to any one of paragraphs 1-376, wherein the gene is has the symbol FLOT1, optionally in combination with one or more genes labeled as 4.428 to 4.488 identified in Table 4.
378) In another aspect the invention employs one or more genes according to any one of paragraphs 1-377, wherein the gene is has the symbol IBRDC2 optionally in combination with one or more genes labeled as 4.429 to 4.488 identified in Table 4.
379) In another aspect the invention employs one or more genes according to any one of paragraphs 1-378, wherein the gene is has the symbol KIAA1794, optionally in combination with one or more genes labeled as 4.430 to 4.488 identified in Table 4.
380) In another aspect the invention employs one or more genes according to any one of paragraphs 1 -379, wherein the gene is has the symbol OLFML1, optionally in combination with one or more genes labeled as 4.431 to 4.488 identified in Table 4.
381) In another aspect the invention employs one or more genes according to any one of paragraphs 1-380, wherein the gene is has the symbol GMFG, optionally in combination with one or more genes labeled as 4.432 to 4.488 identified in Table 4.
382) In another aspect the invention employs one or more genes according to any one of paragraphs 1-381, wherein the gene is has the symbol TNFRSF1B, optionally in combination with one or more genes labeled as 4.433 to 4.488 identified in Table 4.
383) In another aspect the invention employs one or more genes according to any one of paragraphs 1-382, wherein the gene is identifiable by probe set number 217629_at, optionally in combination with one or more genes labeled as 4.434 to 4.488 identified in Table 4.
384) In another aspect the invention employs one or more genes according to any one of paragraphs 1-383, wherein the gene is has the symbol DEF6, optionally in combination with one or more genes labeled as 4.436 to 4.488 identified in Table 4.
385) In another aspect the invention employs one or more genes according to any one of paragraphs 1-384, wherein the gene is has the symbol MAP4K4, optionally in combination with one or more genes labeled as 4.437 to 4.488 identified in Table 4.
386) In another aspect the invention employs one or more genes according to any one of paragraphs 1-385, wherein the gene is has the symbol CMKOR1, optionally in combination with one or more genes labeled as 4.438 to 4.488 identified in Table 4.
387) In another aspect the invention employs one or more genes according to any one of paragraphs 1-386, wherein the gene is identifiable by probe set number 1563461_at, optionally in combination with one or more genes labeled as 4.439 to 4.488 identified in Table 4.
388) In another aspect the invention employs one or more genes according to any one of paragraphs 1-387, wherein the gene is has the symbol CHKA, optionally in combination with one or more genes labeled as 4.440 to 4.488 identified in Table 4.
389) In another aspect the invention employs one or more genes according to any one of paragraphs 1-388, wherein the gene is identifiable by probe set number 226865_at, optionally in combination with one or more genes labeled as 4.441 to 4.488 identified in Table 4.
390) In another aspect the invention employs one or more genes according to any one of paragraphs 1-389, wherein the gene has the symbol HS3ST3B1, optionally in combination with one or more genes labeled as 4.442 to 4.488 identified in Table 4.
391) In another aspect the invention employs one or more genes according to any one of paragraphs 1-390, wherein the gene has the symbol CXorf9, optionally in combination with one or more genes labeled as 4.443 to 4.488 identified in Table 4.
392) In another aspect the invention employs one or more genes according to any one of paragraphs 1-391, wherein the gene has the symbol EVI2A, optionally in combination with one or more genes labeled as 4.445 to 4.488 identified in Table 4.
393) In another aspect the invention employs one or more genes according to any one of paragraphs 1-392, wherein the gene has the symbol NFAM1, optionally in combination with one or more genes labeled as 4.446 to 4.488 identified in Table 4.
394) In another aspect the invention employs one or more genes according to any one of paragraphs 1-393, wherein the gene is identifiable by probe set number 242874_at, optionally in combination with one or more genes labeled as 4.447 to 4.488 identified in Table 4.
395) In another aspect the invention employs one or more genes according to any one of paragraphs 1-394, wherein the gene has the symbol ATP5J, optionally in combination with one or more genes labeled as 4.450 to 4.488 identified in Table 4.
396) In another aspect the invention employs one or more genes according to any one of paragraphs 1-395, wherein the gene has the symbol CYLD, optionally in combination with one or more genes labeled as 4.451 to 4.488 identified in Table 4.
397) In another aspect the invention employs one or more genes according to any one of paragraphs 1-396, wherein the gene has the symbol GIMAP6, optionally in combination with one or more genes labeled as 4.452 to 4.488 identified in Table 4.
398) In another aspect the invention employs one or more genes according to any one of paragraphs 1-397, wherein the gene has the symbol MFAP4, optionally in combination with one or more genes labeled as 4.453 to 4.488 identified in Table 4.
399) In another aspect the invention employs one or more genes according to any one of paragraphs 1-398, wherein the gene has the symbol TUBB2B, optionally in combination with one or more genes labeled as 4.454 to 4.488 identified in Table 4.
400) In another aspect the invention employs one or more genes according to any one of paragraphs 1-399, wherein the gene has the symbol NELL2, optionally in combination with one or more genes labeled as 4.455 to 4.488 identified in Table 4.
401) In another aspect the invention employs one or more genes according to any one of paragraphs 1-400, wherein the gene is identifiable by probe set number 236583_at, optionally in combination with one or more genes labeled as 4.456 to 4.488 identified in Table 4.
402) In another aspect the invention employs one or more genes according to any one of paragraphs 1-401, wherein the gene has the symbol IL1RN, optionally in combination with one or more genes labeled as 4.457 to 4.488 identified in Table 4.
403) In another aspect the invention employs one or more genes according to any one of paragraphs 1-402, wherein the gene has the symbol KIAA1211, optionally in combination with one or more genes labeled as 4.459 to 4.488 identified in Table 4.
404) In another aspect the invention employs one or more genes according to any one of paragraphs 1-403, wherein the gene has the symbol ADAMDEC1, optionally in combination with one or more genes labeled as 4.460 to 4.488 identified in Table 4.
405) In another aspect the invention employs one or more genes according to any one of paragraphs 1-404, wherein the gene has the symbol AOC3, optionally in combination with one or more genes labeled as 4.461 to 4.488 identified in Table 4.
406) In another aspect the invention employs one or more genes according to any one of paragraphs 1-405, wherein the gene has the symbol SAMHD1, optionally in combination with one or more genes labeled as 4.463 to 4.488 identified in Table 4.
407) In another aspect the invention employs one or more genes according to any one of paragraphs 1-406, wherein the gene has the symbol SLC22A3, optionally in combination with one or more genes labeled as 4.465 to 4.488 identified in Table 4.
408) In another aspect the invention employs one or more genes according to any one of paragraphs 1-407, wherein the gene has the symbol IGLV3-25, optionally in combination with one or more genes labeled as 4.466 to 4.488 identified in Table 4.
409) In another aspect the invention employs one or more genes according to any one of paragraphs 1-408, wherein the gene is identifiable by probe set number 1556185_a_at, optionally in combination with one or more genes labeled as 4.467 to 4.488 identified in Table 4.
410) In another aspect the invention employs one or more genes according to any one of paragraphs 1-409, wherein the gene has the symbol RAB11FIP1, optionally in combination with one or more genes labeled as 4.468- to 4.488 identified in Table 4.
411) In another aspect the invention employs one or more genes according to any one of paragraphs 1-410, wherein the gene has the symbol PER2, optionally in combination with one or more genes labeled as 4.469 to 4.488 identified in Table 4.
412) In another aspect the invention employs one or more genes according to any one of paragraphs 1-411, wherein the gene has the symbol TTL, optionally in combination with one or more genes labeled as 4.470 to 4.488 identified in Table 4.
413) In another aspect the invention employs one or more genes according to any one of paragraphs 1-412, wherein the gene has the symbol SIAHBP1, optionally in combination with one or more genes labeled as 4.472 to 4.488 identified in Table 4.
414) In another aspect the invention employs one or more genes according to any one of paragraphs 1-413, wherein the gene has the symbol FLJ22536, optionally in combination with one or more genes labeled as 4.473 to 4.488 identified in Table 4.
415) In another aspect the invention employs one or more genes according to any one of paragraphs 1-414, wherein the gene has the symbol RP6-213H19.1, optionally in combination with one or more genes labeled as 4.474 to 4.488 identified in Table 4.
416) In another aspect the invention employs one or more genes according to any one of paragraphs 1-415, wherein the gene is identifiable by probe set number 235804_at, optionally in combination with one or more genes labeled as 4.475 to 4.488 identified in Table 4.
417) In another aspect the invention employs one or more genes according to any one of paragraphs 1-416, wherein the gene has the symbol NCF4, optionally in combination with one or more genes labeled as 4.476 to 4.488 identified in Table 4.
418) In another aspect the invention employs one or more genes according to any one of paragraphs 1-417, wherein the gene has the symbol EPSTI1, optionally in combination with one or more genes labeled as 4.477 to 4.488 identified in Table 4.
419) In another aspect the invention employs one or more genes according to any one of paragraphs 1-418, wherein the gene has the symbol LOC441212, optionally in combination with one or more genes labeled as 4.478 to 4.488 identified in Table 4.
420) In another aspect the invention employs one or more genes according to any one of paragraphs 1-419, wherein the gene has the symbol ANK3, optionally in combination with one or more genes labeled as 4.479 to 4.488 identified in Table 4.
421) In another aspect the invention employs one or more genes according to any one of paragraphs 1-420, wherein the gene has the symbol PCDH9, optionally in combination with one or more genes labeled as 4.480 to 4.488 identified in Table 4.
422) In another aspect the invention employs one or more genes according to any one of paragraphs 1-421, wherein the gene has the symbol C21orf86, optionally in combination with one or more genes labeled as 4.481 to 4.488 identified in Table 4.
423) In another aspect the invention employs one or more genes according to any one of paragraphs 1-422, wherein the gene has the symbol DHRS9, optionally in combination with one or more genes labeled as 4.482 to 4.488 identified in Table 4.
424) In another aspect the invention employs one or more genes according to any one of paragraphs 1-423, wherein the gene has the symbol ARHGAP25, optionally in combination with one or more genes labeled as 4.483 to 4.488 identified in Table 4.
425) In another aspect the invention employs one or more genes according to any one of paragraphs 1-424, wherein the gene has the symbol TRAF4, optionally in combination with one or more genes labeled as 4.484 to 4.488 identified in Table 4.
426) In another aspect the invention employs one or more genes according to any one of paragraphs 1-425, wherein the gene has the symbol LST1, optionally in combination with one or more genes labeled as 4.485 to 4.488 identified in Table 4.
427) In another aspect the invention employs one or more genes according to any one of paragraphs 1-426, wherein the gene has the symbol PALMD, optionally in combination with one or more genes labeled as 4.486 to 4.488 identified in Table 4.
428) In another aspect the invention employs one or more genes according to any one of paragraphs 1-427, wherein the gene has the symbol TAP1, optionally in combination with one or more genes labeled as 4.487 to 4.488 identified in Table 4.
429) In another aspect the invention employs one or more genes according to any one of paragraphs 1-428, wherein the gene has the symbol MSX2, optionally in combination with one or more genes labeled as 4.448 identified in Table 4.
430) In another aspect the invention employs one or more genes according to any one of paragraphs 1-429, wherein the gene has the symbol SIRPG.

In one aspect the invention employs a gene listed in Table 7.

**Table 7**

| **Gene Symbol** |
|---|
| CCL5 |
| FASLG |
| GNLY |
| GZMB |
| PRF1 |
| IFNG |
| ICOS |
| TBX21 |
| CD8A |
| CD3E |
| CXCL11 |
| CXCL10 |
| CXCR3 |
| CD20 |
| FOXP3 |
| INDO |
| CD45Ro |
| CD45R |
| CD69 |
| TRBV19 |
| TRAT1 |
| TRDV2 |
| STAT4 |
| PRKCQ |
| GMZK |
| GPR171 |
| UBD |
| CD52 |
| CD3D |
| IL7R |
| IRF1 |
| TLR7 |

In one aspect the invention employs one or more genes selected from Table 9.

**Table 9**

| **Gene Symbol** |
|---|
| PRF1 |
| IRF1 |
| GZMB |
| GNLY |
| CD8A |
| PRKCQ |
| FOXP3 |
| IFNG |
| CCL5 |
| GPR171 |
| TRBV19 |
| CD3E |
| TBX21 |
| FASLG |
| CXCL10 |
| ICOS |
| CXCR3 |
| CXCL11 |

In one apect the invention employs one or more genes selected from Table 11.

**Table 11**

| **Gene Symbol** | **Gene Title** |
|---|---|
| CCL5 | chemokine (C-C motif) ligand 5 |
| UCHL1 | Ubiquitin carboxyl-terminal esterase L1 (ubiquitin thiolesterase) |
| PVT1 /// LOC441378 | Pvt1 oncogene homolog, MYC activator (mouse) /// LOC441378 |
| - | CDNA clone IMAGE:4796388 |
| CD52 | CD52 antigen (CAMPATH-1 antigen) /// CD52 antigen (CAMPATH-1 antigen) |
| UBD | ubiquitin D |
| STAT4 | signal transducer and activator of transcription 4 |
| GZMK | granzyme K (granzyme 3; tryptase II) /// granzyme K (granzyme 3; tryptase II) |
| IL18RAP | interleukin 18 receptor accessory protein |
| GPR171 | G protein-coupled receptor 171 |
| PSCDBP | pleckstrin homology, Sec7 and coiled-coil domains, binding protein /// pleckstrin homology, Sec7 and coiled-coil domains, binding protein |
| NCF2 | neutrophil cytosolic factor 2 (65kDa, chronic granulomatous disease, autosomal 2) |
| PRKCQ | protein kinase C, theta |
| LST1 | leukocyte specific transcript 1 |
| TRA@ /// TRDV2 /// T TRAV20 /// TRAJ17 /// TRAC | T cell receptor alpha locus /// T cell receptor delta variable 2 /// T cell receptor alpha variable 20 /// T cell receptor alpha joining 17 /// T cell receptor alpha constant |
| TRGC2 | T cell receptor gamma constant 2 |
| TRBV21-1 /// TRBV19 /// TRBV5-4 /// TRBV3-1 /// TRBC1 | T cell receptor beta variable 21-1 /// T cell receptor beta variable 19 /// T cell receptor beta variable 5-4 /// T cell receptor beta variable 3-1 /// T cell receptor beta constant 1 |
| HLA-DQA1 /// HLA-DQA2 | major histocompatibility complex, class II, DQ alpha 1 /// major histocompatibility complex, class II, DQ alpha 2 |
| TRBV19 /// TRBC1 | T cell receptor beta variable 19 /// T cell receptor beta variable 19 /// T cell receptor beta constant 1 /// T cell receptor beta constant 1 |
| CD3D | CD3D antigen, delta polypeptide (TiT3 complex) |
| GADD45B | Growth arrest and DNA-damage-inducible, beta |
| ITGB3 | integrin, beta 3 (platelet glycoprotein IIIa, antigen CD61) |
| TRAT1 | T cell receptor associated transmembrane adaptor 1 |
| HLA-DMA | major histocompatibility complex, class II, DM alpha |
| CENTD3 | centaurin, delta 3 |
| SAMSN1 | SAM domain, SH3 domain and nuclear localisation signals, 1 |
| CLEC4E | C-type lectin domain family 4, member E |
| TNFRSF19 | tumor necrosis factor receptor superfamily, member 19 |
| NAV1 | neuron navigator 1 |
| LOXL4 | lysyl oxidase-like 4 |
| TNFRSF19 | tumor necrosis factor receptor superfamily, member 19 |
| TAGAP | T-cell activation GTPase activating protein |
| GIMAP2 | GTPase, IMAP family member 2 |
| IGF1R | Insulin-like growth factor 1 receptor |
| C17orf63 | Chromosome 17 open reading frame 63 |
| CD52 | CD52 antigen (CAMPATH-1 antigen) |

In one aspect the invention employs one or more genes selected from Table 12.

**Table 12**

| **Gene symbol** | **Gene title** |
|---|---|
| CCL5 | chemokine (C-C motif) ligand 5 |
| TRAT1 | T cell receptor associated transmembrane adaptor 1 |
| STAT4 | signal transducer and activator of transcription 4 |
| PRKCQ | protein kinase C, theta |
| GPR171 | G protein-coupled receptor 171 |
| UBD | ubiquitin D |
| CD52 | CD52 molecule |
| CD3D | CD3d molecule, delta (CD3-TCR complex) |
| PRF1 | perforin 1 (pore forming protein) |
| CD8A | CD8a molecule |
| CXCL10 | chemokine (C-X-C motif) ligand 10 |
| CD69 | CD69 molecule |
| TRBV19 | T cell receptor beta variable 19 |
| TRDV2 | T cell receptor delta variable 2 |
| IL7R | interleukin 7 receptor |
| GZMK | granzyme K PROTEIN-TYROSINE PHOSPHATASE, RECEPTOR-TYPE, C |
| CD45R | (PTPRC) |

In one aspect the invention provides one or more genes selected from Table 13.

**Table 13**

| **Gene symbol** | **Gene title** |
|---|---|
| FASLG | Fas ligand (TNF superfamily, member 6) |
| GNLY | granulysin |
| GZMB | granzyme B (granzyme 2, cytotoxic T-lymphocyte-associated serine esterase 1) |
| IFNG | interferon, gamma |
| ICOS | inducible T-cell co-stimulator |
| TBX21 | T-box 21 |
| CD3E | CD3e molecule, epsilon (CD3-TCR complex) |
| CXCL11 | chemokine (C-X-C motif) ligand 11 |
| CXCR3 | chemokine (C-X-C motif) receptor 3 |
| CD20 | CD20 molecule |
| FOXP3 | forkhead box P3 |
| INDO | indoleamine-pyrrole 2,3 dioxygenase |
| IRF1 | interferon regulatory factor 1 |
| TLR7 | toll-like receptor 7 |

PCR is a more sensitive technique than microarray and therefore can detect lower levels of differentially expressed genes.

In particular the following genes are suitable for PCR analysis: IL7R, CD3D, CD3E, CD52, UBD, GPR171, GMZK, PRKCQ, STAT4, TRDV2, TRAT1, TRBV19, CD69, INDO, CD45R, CD45RO, FOXP3, CD20, CCL5, FASLG, GNLY, GZMB, PRF1, IFNG, ICOS, TBX21, CD8A, CD3E, CXCL10, CXCL11, IRF1, TLR7 and CXCR3.

In one aspect the gene(s) employed are selected from the group comprising or consisiting of: CCL5, TRAT1, STAT4, PRKCQ, GPR171, UBD, CD52, CD3D, PRF1, CD8A, CXCL10, CD69, TRBV19, TRDV2, IL7R, GZMK and CD45R.

In one aspect the gene(s) employed are selected from the comprising or consisting of:
FASLG, GNLY, GZMB, IFNG, ICOS, TBX21, CD3E, CXCL11, CXCR3, CD20, FOXP3, INDO, IRF1 and TLR7.

The gene(s) FOXP3 and/or PRF1 is/are particularly suitable for PCR analysis according to the invention.

Suitable targets for immunohistochemistry include CD3, CD8, CD86, LAMP, CD20, CD45RO, CXCR3, CXL10/11, CD69, granzyme B, IDO, B cells and gene products from one or more genes from the NK family, HLA family, T cell receptor family and/or activated T cell.

In a further aspect of the invention there is provided a gene signature indicative of a non-responder, for example wherein one or more of immune genes such as those listed herein are NOT differentially expressed and/or are down regulated and/or are NOT upregulated.

In one aspect the gene is NOT FOXP3.

According to a further aspect the present invention provides a gene signature indicative of an increased likelihood of a patients responding favourably to appropriate immunotherapy, which in turn is likely to result in an improved survival of patients, for example with Mage expressing cancers following treatment with Mage specific immunotherapy. This gene signature, of at least 5, 6, 7, 8, 9, 10, 11, 12, 13, 14, 15, 16, 17, 18, 19, 20 21, 22, 23, 24, 25, 26, 27, 28, 29, 30 or 31 genes from the genes disclosed in Table 1, is characterised by differential expression compared to the gene signature of Mage-expressing tumour patients who do not respond to Mage antigen specific cancer immunotherapy.

In one aspect the invention provides a profile based on differential expression of 1 or more of 62 known genes that relate to immune infiltration and activation.

The predictive genes correspond mainly to expression of and often upregulation of genes related to immune infiltration and activation. These genes include HLA class II, Interleukin-2 receptor gamma, T cell receptor genes (TRBV19, TRAT1, TRGC2), granzyme, and CD69.

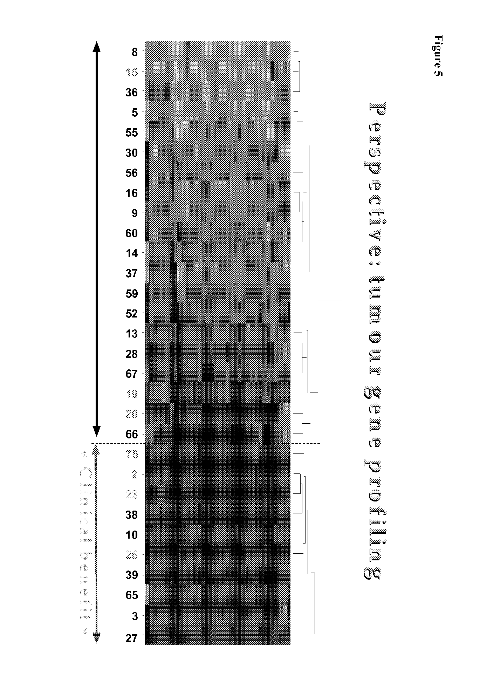

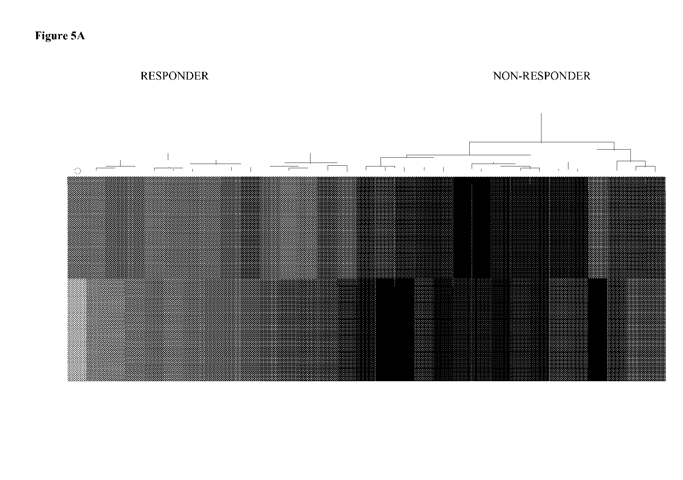

In one embodiment there is provided a gene signature, present in patients, for example with or who have had MAGE-expressing tumours, who respond to treatment, in which one or more, for example at least 5, suitably 6, 7, 8, 9, 10 of the genes of Table 2 are differentially expressed:
As shown in Fig 5a below, characterisation of responders and non-responders may be based on the differential expression on only one or two genes, such as TCR, CD3 and/or IL-7.

Other genes that are thought to be particularly suitable in methods employing only one or two genes include: IL7R, CD3D, CD3E, CD52, UBD, GPR171, GMZK, PRKCQ, STAT4, TRDV2, TRAT1, TRBV19, CD69, INDO, CD45R, CD45RO, FOXP3, CD20, CCL5, FASLG, GNLY, GZMB, PRF1, IFNG, ICOS, TBX21, CD8A, CD3E, CXCL10, CXCL11, TRF1, TLR7 and CXCR3, which may require the use of appropriately sensitive analytical techniques.

The invention herein extends to use of all permutations of the genes listed herein for identification of said signature/profile.

The invention also extends to embodiments according to the invention described herein, which comprise, consist essentially of, or consists of the components/elements described.

The invention extends to the functional equivalents of genes listed herein, for example as characterised by hierarchical classification of genes such as described by Hongwei Wu *et al* 2007(Hierarchical classification of equivalent genes in prokaryotes-*Nucliec Acid Research Advance Access*).

Whilst not wishing to be bound by theory, it is thought that is not necessarily the gene *per se* that is characteristic of the signature but rather it is the gene function which is fundamentally important. Thus a functionally equivalent gene to an immune activation gene such as those listed above, for example in Table 1, 2, 3, 4, 7, 9, 11, 12 or 13 may be employed in the signature, see for example, Journal of the National Cancer Institute Vol 98, No. 7 April 5 2006.

The genes were identified by specific probes and thus a skilled person will understand that the description of the genes above is a description based on current understanding of what hybridises to the probe. However, regardless of the nomenclature used for the genes by repeating the hybridisation to the relevant probe under the prescribed conditions the requisite gene can be identified.

The invention extends to use of the profile(s) according to the invention for predicting or identifying a patient as a responder or non-responder to immunotherapy, such as cancer immunotherapy, for example cancer testis immunotherapy.

Thus the invention includes a method of analyzing a patient derived sample, based on differential expression of the profile/gene(s) according to the invention for the purpose of characterising the patient from which the sample was derived as a responder or non-responder to immunotherapy. In one aspect the invention provides a method of identifying a profile according to the invention comprising the steps:
a) analyzing a patient derived sample for differential expression of one or more immune response genes, and
b) characterising the patient from which the sample was derived as a responder or non-responder to appropriate immunotherapy, based on the results of step (a),
wherein the characterisation step is optionally performed by reference or comparison to a standard.

Suitable standards are described above.

The present invention therefore, generally relates, in one aspect, to a method for
the detection of a gene signature in a biological sample, the method comprising the analysis of the expression of one or more genes of Tables 1, 2, 3, 4, 7, 9, 11, 12 or 13, for example at least 5, 6, 7, 8, 9, 10, 11, 12, 13, 14, 15 ,16,17, 18, 19, 20 21, 22, 23, 24, 25, 26, 27, 28, 29, 30 or 31 genes (eg for genes set forth in Table 1). In an embodiment, the analysis comprises the expression of at least 5, suitably 6, 7, 8, 9 or 10 genes set forth in Table 2.

In one aspect the invention provides a method for measuring expression levels of polynucleotides from immune activation genes such as one or more genes listed in Tables 1, 2, 3, 4, 7, 9, 11, 12 and/or 13 in a sample for the purpose of identifying if the patient, from whom the sample was derived, is likely to be a responder or non-responder to immunotherapy such a cancer immunotherapy comprising the steps:
isolating the RNA from the sample,
optionally amplifying the copies of the cDNA from the sample for said genes, and
quantifying the levels of cDNA in the sample.

In one embodiment, the diagnostic method comprises determining whether a subject expresses any of the gene products of the genes set forth in Table 1 or any other embodiment of the invention described herein by, for example, detecting the level of the corresponding mRNA and/or protein level of the gene products of the genes set forth in Table 1 etc. For example, by using techniques such as by Northern blot analysis, reverse transcription-polymerase chain reaction (RT-PCR), in situ hybridization, immunoprecipitation, Western blot hybridization, or immunohistochemistry. According to the method, cells may be obtained from a subject and the levels of the protein, or mRNA, of the analyzed genes, compared to that of a non-responder patient. Patients who differentially express one or more, for example, 5, 6, 7, 8, 9, 10, 11, 12, 13, 14, 15, 16, 17, 18, 19, 20 21, 22, 23, 24, 25, 26, 27, 28, 29, 30 or 31 genes selected from Table 1 or another embodiment of the invention are those which can be predicted to benefit from immunotherapy, for example cancer immunotherapy, such as Mage antigen specific cancer immunotherapy. For example, a nucleic acid molecule which hybridizes to a given location on a microarray is said to be differentially expressed if the hybridization signal is, for example, higher than the hybridization signal at the same location on an identical array hybridized with a nucleic acid sample obtained from a subject that does not clinically respond to Mage specific immunotherapy.

The results of 30 patients who have been subjected to gene profiling are shown in figure 5. This pictorially demonstrates that the gene signature of the present invention is aligned with clinical response to MAGE-antigen specific cancer immunotherapy.

Alternatively the cancer patient may be characterised as a responder or non-responder to immunotherapy from a visual inspection of a tissue section derived from the cancer or tumour. A responder is likely to be a patient with an infiltration of immune response cells into the cancer/tumour microenvironment.

The invention also provides a method of generating a new gene profile not specifically recited herein, based on differential expression of one or more immune response/activation genes, for indicating whether a patient is likely to be a responder or non-responder to appropriate immunotherapy, for example cancer immunotherapy such as Mage immunotherapy comprising the steps:
a) analysing at least two patient derived samples for differential expression of one or more of immune activation genes, where the sample group comprises both responders and non-responders to appropriate immunotherapy, and
b) correlating same with clinical outcome, after appropriate treatment, of patients from which the samples were derived, and
c) identifying one or more genes which are differentially expressed in responders and/O non-responders.

After the new profile has been identified the invention also extends to analysis of a previously uncharacterised sample, designating same as responders or non-responders (as appropriate) and if desired administering a therapeutically effective amount of an appropriate immunotherapy, for example cancer immunotherapy such as Mage immunotherapy to one or more patients designated as responders.

The invention also provides a method of generating a gene profile based on differential expression of one or more gene sequences recited in Table 1, 2, 3, 4, 7, 9, 11, 12 and/or 13 for indicating whether a patient is likely to be a responder or non-responder to immunotherapy comprising the steps:
a) analyzing at least two patient derived samples for differential expression of
   (i) one or more gene sequences recited in Table 1, 2, 3, 4, 7, 9, 11, 12 and/or 13 and
   (ii) optionally one or more gene sequences not recited inTable 1, 2, 3, 4, 7, 9, 11, 12 and/or 13,
   where the sample group comprises both responders and non-responders to appropriate immunotherapy;
b) correlating differential expression of said one or more gene sequences with clinical outcome, after appropriate treatment, of patients from which the samples were derived, and
c) identifying one or more genes which are differentially expressed in responders and non-responders.

After the profile has been identified the invention also extends to analysis of a previously uncharacterised sample, designating same as responders or non-responders (as appropriate) and if desired administering a therapeutically effective amount of an appropriate immunotherapy, for example cancer immunotherapy such as Mage immunotherapy to one or more patients designated as responders.

The invention provides a diagnostic kit comprising at least one component for performing an analysis on a patient derived sample to identify a profile according to the invention, the results of which may be used to designate a patient from which the sample was derived as a responder or non-responder to immunotherapy.

The kit may comprise materials/reagents for PCR (such as QPCR), microarray analysis, immunohistochemistry or other analytical technique that may be used for accessing differential expression of one or more genes.

The invention also provides a diagnostic kit comprising a set of probes capable of hybridising to the mRNA or cDNA of one or more, such as at least 5 genes as set forth in Table 1 or alternatively Tables 2, 3, 4, 7, 9, 11, 12 and/or 13, for example a diagnostic kit comprising a set of probes capable of hybridising to the mRNA or its cDNA of at least 6, 7, 8, 9, 10, 11, 12, 13, 14,15, 16, 17, 18, 19, 20, 21,22, 23, 24, 25, 26, 27, 28, 29, 30 or 31 genes as set forth in Table 1. In another embodiment this invention relates to diagnostic kits. For example, diagnostic kits containing such microarrays comprising a microarray substrate and probes that are capable of hybridising to mRNA or cDNA expressed from, for example, 5, 6, 7, 8, 9, 10, 11, 12, 13, 14, 15,16,17, 18, 19, 20 21, 22, 23, 24, 25, 26, 27, 28 , 29, 30 or 31 genes from, for example Table 1 or any other embodiment of the invention that are capable of demonstrating the gene signature of the invention.

In one aspect the invention provides microarrays adapted for identification of a signature according to the invention.

The invention also extends to substrates and probes suitable for hybridising to an mRNA or cDNA moiety expressed from one or more genes employed in the invention, for example from Table 1, 2, 3, 4, 7, 9, 11, 12 or 13.

Commercially available microarrays contain many more probes than are required to characterise the differential expression of the genes under consideration at any one time, to aid the accuracy of the analysis. Thus one or more probe sets may recognise the same gene.

Thus in one embodiment multiple probes or probe sets are used to identify if an immune activation gene is differentially expressed, such as upregulated.

The diagnostic kit may, for example comprise probes, which are arrayed in a microarray. Specifically, prepared microarrays, for example, containing one or more probe sets described herein can readily be prepared by companies such as Affimetrix, thereby providing a specific test and optionally reagents for identifying the profile, according to the invention.

In an embodiment the microarrays or diagnostic kits will additionally be able to test for the presence or absence of the relevant cancer testis antigen expressing gene such as the Mage gene. Thus in one aspect the invention provides a probe and/or probe set suitable for said hybridisation, under appropriate conditions. The invention also extends to use of probes, for example as described herein or functional equivalents thereof, for the identification of a gene profile according to the present invention.

The invention herein described extends to use of all permutations of the probes listed herein (or functional analogues thereof) for identification of the said signature.

In one aspect the invention provides use of a probe for the identification of differential expression of at least one gene product of an immune activation gene for establishing if a gene profile according to the present invention is present in a patient derived sample.

Table 1B discloses probe sets from which probes of typically 25 mer in length may be designed and which are suitable for identifying the mRNA (or its cDNA) expressed from, for example the genes of Table 1 (or alternatively Table 2, 3, 4, 7, 9, 11, 12 or 13). Such probes and probe sets are an aspect of the invention. Typically each probe set may comprise about or exactly 11 individual sequences of about or exactly 25 nucleotides, which correspond precisely to a run of sequences from the probe set.

Accordingly, in one aspect, this invention relates to oligonucleotide probes and primers capable of recognising the mRNA (or its cDNA) expressed from the genes from Table 1, (or alternatively

Table 2, 3, 4, 7, 9, 11, 12 or 13) and diagnostic kits based on these probes and primers. Such kits may include probes or kits for the detection of a Mage gene.

In an aspect the invention provides a profile based on the differential expression of one or more of the genes of Table 3 identifiable by one or more of following 13 probes:
204661_at, 205890_s_at, 206118_at, 206666_at, 207651_at, 210038_at, 210972_x_at, 211144_x_at, 211796_s_at, 213193_x_at, 213539_at, 217147_s_at, 34210_at.

Further details of these probes and the target genes of the same are given in Table 3A below. Hybridisation will generally be preformed under stringent conditions, such as 3X SSC, 0.1% SDS, at 50 °C.

Once the target gene(s)/profile has/have been identified then it is well within the skilled person's ability to design alternative probes that hybridise to the same target. Therefore the invention also extends to probes, which under appropriate conditions measure the same differential expression of the gene(s) of the present invention to provide a signature/profile as described.

The invention also extends to use of the relevant probe in analysis of whether a cancer patient will be a responder or non-responder to treatment with an appropriate immunotherapy.

The invention also extends to use (and processes employing same) of known microarrays for identification of said signature.

A nucleic acid probe may be at least 10, 15, 20, 25, 30, 35, 40, 50, 75, 100 or more nucleotides in length and may comprise the full length gene. Probes for use in the invention are those that are able to hybridise specifically to the mRNA (or its cDNA) expressed from the genes listed in Table 1 (or Table 2, 3, 4, 7, 9, 11, 12 or 13) under stringent conditions.

The present invention further relates to a method of screening the effects of a drug on a tissue or cell sample comprising the step of analysing the expression profile of, for example 5, 6, 7, 8, 9, 10, 11, 12, 13, 14, 15, 16, 17, 18, 19, 20 21, 22, 23, 24, 25, 26, 27, 28 , 29, 30 or 31 genes selected from Table 1 or any other embodiment of the invention described herein before and after drug treatment. The invention therefore provides a method for screening for a drug, which would alter the gene profile to that of a patient having improved survival following treatment with, for example, Mage antigen specific cancer immunotherapy (ie. to alter the gene profile to that of a responder), to enable the patient to benefit from, for example, Mage antigen specific cancer immunotherapy.

The present invention further provides a method of patient diagnosis comprising, for example, the step of analysing the expression profile of, for example, 5, 6, 7, 8, 9, 10, 11, 12, 13, 14, 15,16,17, 18, 19, 20 21, 22, 23, 24, 25, 26, 27, 28 , 29, 30 or 31 genes selected from Table 1 or any other embodiment of the invention described herein and comparing it with a standard to diagnose whether the patient would benefit from Mage specific immunotherapy.

The invention includes a method of patient diagnosis comprising the step of analysing the expression profile of at least 5, 6, 7, 8, 9, 10, 11, 12, 13, 14, 15, 16, 17, 18, 19, 20 21, 22, 23, 24, 25, 26, 27, 28, 29, 30 or 31 genes selected from Table 1 or other embodiment of the invention from a tumour tissue sample given by a patient and assessing whether 5 or more of said genes are expressed.

Thus in clinical applications, tissue samples from a human patient may be screened for the presence and/or absence of the expression of, for example 5, 6, 7, 8, 9, 10, 11, 12, 13, 14, 15, 16, 17, 18, 19, 20 21, 22, 23, 24, 25, 26, 27, 28, 29, 30 or 31 genes selected from Table 1 or any other embodiment of the invention described herein.

In the context of the present invention, the sample may be of any biological tissue or fluid derived from a patient potentially in need of treatment. The sample maybe derived from sputum, blood, urine, or from solid tissues such as biopsy from a primary tumour or metastasis, or from sections of previously removed tissues.

Samples could comprise or consist of, for example, needle biopsy cores, surgical resection samples or lymph node tissue. These methods include obtaining a biopsy, which is optionally fractionated by cryostat sectioning to enrich tumour cells to about 80% of the total cell population. In certain embodiments, nucleic acids extracted from these samples may be amplified using techniques well known in the art. The levels of selected markers (eg the gene products of table 1) can be detected and can be compared with statistically valid groups of, for example, Mage positive non responder patients.

For cancer, the biological sample will contain cancer or tumour cells and may, for example, be derived from the cancer or tumour such as a fresh sample (including frozen samples) or a sample that has been preserved in paraffin. Having said this, samples preserved in paraffin can suffer from degradation and the profile observed may be modified. A person working the in field is well able to compensate of these changes observed by recalibrating the parameters of the profile.

### Microarrays

A microarray is an array of discrete regions, typically nucleic acids, which are separate from one another and are typically arrayed at a density of between, about 100/cm² to 1000/cm², but can be arrayed at greater densities such as 10000 /cm². The principle of a microarray experiment, is that mRNA from a given cell line or tissue is used to generate a labeled sample typically labeled cDNA, termed the 'target', which is hybridized in parallel to a large number of, nucleic acid sequences, typically DNA sequences, immobilised on a solid surface in an ordered array.

Tens of thousands of transcript species can be detected and quantified simultaneously. Although many different microarray systems have been developed the most commonly used systems today can be divided into two groups, according to the arrayed material: complementary DNA (cDNA) and oligonucleotide microarrays. The arrayed material has generally been termed the probe since it is equivalent to the probe used in a northern blot analysis. Probes for cDNA arrays are usually products of the polymerase chain reaction (PCR) generated from cDNA libraries or clone collections, using either vector-specific or gene-specific primers, and are printed onto glass slides or nylon membranes as spots at defined locations. Spots are typically 10-300 µm in size and are spaced about the same distance apart. Using this technique, arrays consisting of more than 30,000 cDNAs can be fitted onto the surface of a conventional microscope slide. For oligonucleotide arrays, short 20-25mers are synthesized *in situ,* either by photolithography onto silicon wafers (high-density-oligonucleotide arrays from Affymetrix or by ink-jet technology (developed by Rosetta Inpharmatics, and licensed to Agilent Technologies). Alternatively, presynthesized oligonucleotides can be printed onto glass slides. Methods based on synthetic oligonucleotides offer the advantage that because sequence information alone is sufficient to generate the DNA to be arrayed, no time-consuming handling of cDNA resources is required. Also, probes can be designed to represent the most unique part of a given transcript, making the detection of closely related genes or splice variants possible. Although short oligonucleotides may result in less specific hybridization and reduced sensitivity, the arraying of presynthesized longer oligonucleotides (50-100mers) has recently been developed to counteract these disadvantages. Thus in performing a microarray to ascertain whether a patient presents with a gene signature of the present invention, the following steps are performed: obtain mRNA from the sample and prepare nucleic acids targets, contact the array under conditions, typically as suggested by the manufactures of the microarray (suitably stringent hybridisation conditions such as 3X SSC, 0.1% SDS, at 50 °C) to bind corresponding probes on the array, wash if necessary to remove unbound nucleic acid targets and analyse the results.

It will be appreciated that the mRNA may be enriched for sequences of interest such as those in Table 1 or 2 (or other embodiment of the invention) by methods known in the art, such as primer specific cDNA synthesis. The population may be further amplified, for example, by using PCR technology. The targets or probes are labeled to permit detection of the hybridisation of the target molecule to the microarray. Suitable labels include isotopic or fluorescent labels which can be incorporated into the probe.

In an alternative embodiment, a patient may be diagnosed to ascertain whether his/her tumor expresses the gene signature of the invention utilising a diagnostic kit based on PCR technology, in particular Quantative PCR ( For a review see Ginzinger D Experimental haematology 30 ( 2002) p 503 - 512 and Giuliette et al Methods, 25 p 386 (2001).

In an alternative aspect the invention provides a method further comprising the steps of analyzing a tumour derived sample to determine which antigen(s) are expressed by the tumour and hence enabling administration of an a therapeutically effective amount of an appropriate antigen specific cancer immunotherapeutic, for example where the tumour is found to be MAGE (such as Mage A3) positive, appropriate treatment may, for example, include administration of Mage A3 antigen specific immunotherapy.

A sample such as tumour tissue of a patient is deemed to present the gene signature of the invention if one or more genes of Table 1, 2, 3, 4, 7, 9, 11, 12 and/or 13 are differentially expressed (such as upregulated), for example, 5, 6, 7, 8, 9, 10, 11, 12, 13, 14, 15 ,16,17, 18, 19, 20 21, 22, 23, 24, 25, 26, 27, 28 , 29, 30 or 31 genes from for example Table 1 or other embodiment of the invention are expressed and can be detected by microarray analysis or other appropriate analysis for example as described herein. The tumour tissue preferably shows expression of at least 5 genes selected from Table 1, 2, 3, 4, 7, 9, 11, 12 and/or 13, more preferably 10 genes, particularly 5, 6, 7, 8, 9 or 10 genes from Table 1, 2, 3, 4, 7, 9, 11, 12 or 13.

### Immunotherapeutics

In a further aspect the invention provides a method of treating a responder patient with an appropriate immunotherapy, for example cancer immunotherapy such as cancer testis immunotherapy, after identification of the same as a responder thereto.

Thus the invention provides a method of treating a patient comprising the step of administering a therapeutically effective amount of an appropriate immunotherapy (for example cancer immunotherapy, such as Mage cancer immunotherapy), after first characterising the patient as a responder based on differential expression of at least one immune activation gene, for example as shown by appropriate analysis of a sample derived from the patient. In particular wherein the patient is characterised as a responder based on one or more embodiments described herein.

In one aspect the immunotherapy comprises an appropriate adjuvant (immunostimulant), see description below.

In yet a further embodiment of the invention there is provided a method of treating a patient suffering from, for example, a Mage expressing tumour, the method comprising determining whether the patient expresses the gene signature of the invention and then administering, for example, a Mage specific immunotherapeutic. In a further embodiment, the patient is treated with, for example, the Mage specific immunotherapy to prevent or ameliorate recurrence of disease, after first receiving treatment such as resection by surgery of any tumour or other chemotherapeutic or radiotherapy treatment.

A further aspect of the invention is a method of treating a patient suffering from a Mage expressing tumour, the method comprising determining whether the patient's tumour expresses at least 5, 6, 7, 8, 9, 10, 11, 12, 13, 14, 15 ,16,17, 18, 19, 20 21, 22, 23, 24, 25, 26, 27, 28 , 29, 30 or 31 genes selected from Table 1 (or other embodiment of the invention) from a tumour tissue sample given by a patient and then administering a Mage specific immunotherapeutic to said patient.

Also provided is a method of treating a patient susceptible to recurrence of Mage expressing tumour having been treated to remove/treat a Mage expressing tumour, the method comprising determining whether the patient's tumour expresses at least 5, 6, 7, 8, 9, 10, 11, 12, 13, 14, 15, 16,17, 18, 19, 20 21, 22, 23, 24, 25, 26, 27, 28 , 29, 30 or 31 genes selected from Table 1 (or other embodiment of the invention) from a tumour tissue sample given by a patient and then administering a Mage specific immunotherapeutic.

The invention also provides as method of treatment or use employing:
- MAGE specific immunotherapeutic comprising a MAGE antigen or peptide thereof,
- MAGE antigen comprising a MAGE-A3 protein or peptide,
- MAGE antigen comprising the peptide EVDPIGHLY,
- MAGE antigen or peptide fused or conjugated to a carrier protein, for example in which the carrier protein is selected from protein D, NS 1 or CLytA or fragments thereof, and/or
- MAGE specific immunotherapeutic further comprises an adjuvant, for example in which the adjuvant comprises one or more or combinations of: 3D-MPL; aluminium salts; CpG containing oligonucleotides; saponin-containing adjuvants such as QS21
or ISCOMs; oil-in-water emulsions; and liposomes.

The invention also extends to use of an immunotherapy such as a cancer immunotherapy, in particular Mage immunotherapy in the manufacture of a medicament for the treatment of a patient such as a cancer patient designated as a responder, thereto.

It was observed that one patient initially characterised as a non-responder was subsequently characterised as responder after radiation therapy. Interestingly the inventors also believe that it may be possible to induce a responders profile in at least some non-responders, for example by subjecting the patient to radiation therapy, or administering an inflammatory stimulant such as interferon (for example imiquimod such as administered topically) or a TLR 3 (for example as described in WO 2006/054177), 4, 7, 8 or TLR 9 agonist (for example containing a CpG motif, in particular administering a high dose thereof such as 0.1 to 75 mg per Kg adminstered, for example weekly). See for example Krieg, A. M., Efler, S. M., Wittpoth, M., Al Adhami, M. J. & Davis, H. L. Induction of systemic TH1-like innate immunity in normal volunteers following subcutaneous but not intravenous administration of CPG 7909, a synthetic B-class CpG oligodeoxynucleotide TLR9 agonist. J. Immunother. 27, 460-471 (2004).

The high dose of CpG may, for example be inhaled or given subcutaneously.

Whilst not wishing to be bound by theory, it is hypothesised that the radiation therapy stimulated a systemic inflammatory response/immune response, which once triggered rendered the patient (or tumour tissue therein) more responsive to immunotherapy.

Accordingly, the invention further provides a method of inducing a responders profile by stimulating a systemic immune response, for example by administering an immunostimulant. The invention further provides the use of Mage specific immunotherapy in the manufacture of a medicament for the treatment of patients suffering from Mage expressing tumour or patients who have received treatment (e.g. surgery, chemotherapy or radiotherapy) to remove/treat a Mage expressing tumour, said patient expressing the gene signature of the invention.

The immunotherapy may then be administered once the responders profile has been induced.

In one aspect the invention provides use of Mage specific immunotherapy in the manufacture of a medicament for the treatment of patients suffering from Mage expressing tumour, said patient characterised by their tumour expressing at least 5, 6, 7, 8, 9, 10, 11, 12, 13, 14, 15 ,16,17, 18, 19, 20 21, 22, 23, 24, 25, 26, 27, 28 , 29, 30 or 31 genes selected from Table 1, or alternatively other embodiments of the invention.

The invention also provides use of Mage specific immunotherapy in the manufacture of a medicament for the treatment of patients susceptible to recurrence from Mage expressing tumour said patient characterised by their tumour expressing at least 5, 6, 7, 8, 9, 10, 11, 12, 13, 14, 15, 16, 17, 18, 19, 20 21, 22, 23, 24, 25, 26, 27, 28, 29, 30 or 31 genes selected from Table 1 or alternatively other embodiments of the invention.

Advantageously, the invention may allow medics to target those populations of patients that will obtain a clinical benefit from receiving an appropriate immunotherapy. It is expected that after screening that at least 60% of patients such as 70, 75, 80, 85% or more of patients deemed/characterised as responders will receive a clinical benefit from the immunotherapy, which is a significant increase over the current levels observed with therapy such as cancer therapy generally.

Advantageously if the cancer immunotherapy is given concomitantly or subsequent to chemotherapy it may assist in raising the patient's immune responses, which may have been depleted by the chemotherapy.

Antigen Specific Cancer Immunotherapeutics (ASCIs) suitable for use in the invention may, for example include those capable of raising a Mage specific immune response. Such immunotherapeutics may be capable of raising an immune response to a Mage gene product, for example a Mage-A antigen such as Mage-A3. The immunotherapeutic will generally contain at least one epitope from a Mage gene product. Such an epitope may be present as a peptide antigen optionally linked covalently to a carrier and optionally in the presence of an adjuvant. Alternatively larger protein fragments may be used. For example, the immunotherapeutic for use in the invention may comprise an antigen that corresponds to or comprises amino acids 195-279 of MAGE-A1. The fragments and peptides for use must however, when suitably presented be capable of raising a Mage specific immune response. Examples of peptides that may be used in the present invention include the MAGE-3.A1 nonapeptide EVDPIGHLY [Seq. ID No 36] (see Marchand et al., International Journal of Cancer 80(2), 219-230), and the following MAGE-A3 peptides:

| | |
|---|---|
| FLWGPRALV; | [Seq. ID No 37] |
| MEVDPIGHLY; | [Seq. ID No 38] |
| VHFLLLKYRA; | [Seq. ID No 39] |
| LVHFLLLKYR; | [Seq. ID No 40] |
| LKYRAREPVT; | [Seq. ID No 41] |
| ACYEFLWGPRALVETS; and | [Seq. ID No 42] |
| TQHFVQENYLEY; | [Seq. ID No 43] |

Alternative ASCIs include cancer testis antigens such as PRAME, LAGE 1, LAGE 2, and others, for example details of which can be obtained from www.cancerimmunity.org/CTdatabase.

The cancer immunotherapy may be based, for example on one or more of the antigens discussed below.

In one embodiment of the present invention, the antigen to be used may consist or comprise a

MAGE tumour antigen, for example, MAGE 1, MAGE 2, MAGE 3, MAGE 4, MAGE 5, MAGE 6, MAGE 7, MAGE 8, MAGE 9, MAGE 10, MAGE 11 or MAGE 12. The genes encoding these MAGE antigens are located on chromosome X and share with each other 64 to 85% homology in their coding sequence (De Plaen, 1994). These antigens are sometimes known as MAGE A1, MAGE A2, MAGE A3, MAGE A4, MAGE A5, MAGE A6, MAGE A7, MAGE A8, MAGE A9, MAGE A 10, MAGE A11 and/or MAGE A12 (The MAGE A family). In one embodiment, the antigen is MAGE A3.

In one embodiment, an antigen from one of two further MAGE families may be used: the MAGE B and MAGE C group. The MAGE B family includes MAGE B1 (also known as MAGE Xp1, and DAM 10), MAGE B2 (also known as MAGE Xp2 and DAM 6) MAGE B3 and MAGE B4 - the Mage C family currently includes MAGE C1 and MAGE C2.

In general terms, a MAGE protein can be defined as containing a core sequence signature located towards the C-terminal end of the protein (for example with respect to MAGE A1 a 309 amino acid protein, the core signature corresponds to amino acid 195-279).

The consensus pattern of the core signature is thus described as follows wherein x represents any amino acid, lower case residues are conserved (conservative variants allowed) and upper case residues are perfectly conserved.

### Core sequence signature

LixvL(2x)I(3x)g(2x)apEExiWexl(2x)m(3-4x)Gxe(3-4x)gxp(2x)llt(3x)VqexYLxYxqVPxsxP(2x)**yeFLWGprA**(2x)Et(3x)kv Conservative substitutions are well known and are generally set up as the default scoring matrices in sequence alignment computer programs. These programs include PAM250 (Dayhoft M.O. et al., (1978), "A model of evolutionary changes in proteins", In "Atlas of Protein sequence and structure" 5(3) M.O. Dayhoft (ed.), 345-352), National Biomedical Research Foundation, Washington, and Blosum 62 (Steven Henikoft and Jorja G. Henikoft (1992), "Amino acid substitution matricies from protein blocks"), Proc. Natl. Acad. Sci. USA 89 (Biochemistry): 10915-10919.

In general terms, substitution within the following groups are conservative substitutions, but substitutions between groups are considered non-conserved. The groups are:
i) Aspartate/asparagine/glutamate/glutamine
ii) Serine/threonine
iii) Lysine/arginine
iv) Phenylalanine/tyrosine/tryptophane
v) Leucine/isoleucine/valine/methionine
vi) Glycine/alanine

In general and in the context of this invention, a MAGE protein will be approximately 50% or more identical, such as 70, 80, 90, 95 or 99% identical, in this core region with amino acids 195 to 279 of MAGE A1.

MAGE protein derivatives are also known in the art, see: WO 99/40188. Such derivatives are suitable for use in therapeutic vaccine formulations (Immunotherapeutic) which are suitable for the treatment of a range of tumour types.

Several CTL epitopes have been identified on the MAGE-3 protein. One such epitope, MAGE-3.A1, is a nonapeptide sequence located between amino acids 168 and 176 of the MAGE-3 protein which constitutes an epitope specific for CTLs when presented in association with the MHC class I molecule HLA.A1. Recently two additional CTL epitopes have been identified on the peptide sequence of the MAGE-3 protein by their ability to mount a CTL response in a mixed culture of melanoma cells and autologous lymphocytes. These two epitopes have specific binding motifs for the HLA.A2 (Van der Bruggen, 1994) and HLA.B44 (Herman, 1996) alleles respectively.

In a further embodiment of the invention, the tumour antigen may comprise or consist of one of the following antigens, or an immunogenic portion thereof which is able to direct an immune response to the antigen:
SSX-2; SSX-4; SSX-5; NA17; MELAN-A; Tyrosinase; LAGE-1; NY-ESO-1; PRAME; P790; P510; P835; B305D; B854; CASB618 (as described in WO00/53748); CASB7439 (as described in WO01/62778); C1491; C1584; and C1585.

In one embodiment, the antigen may comprise or consist of P501S (also known as prostein). The P501 S antigen may be a recombinant protein that combines most of the P501 S protein with a bacterial fusion protein comprising the C terminal part of protein LytA of *Streptococcus pneumoniae* in which the P2 universal T helper peptide of tetanus toxoid has been inserted, ie. a fusion comprising **C**LytA-**P**2-**C**Lyta (the "CPC" fusion partner), as described in WO03/104272; In one embodiment, the antigen may comprise or consist of WT-1 expressed by the Wilm's tumor gene, or its N-terminal fragment WT-1F comprising about or approximately amino acids 1-249; the antigen expressed by the Her-2/neu gene, or a fragment thereof. In one embodiment, the Her-2/neu antigen may be one of the following fusion proteins which are described in WO00/44899.

In a further embodiment, the antigen may comprise or consist of "HER-2/neu ECD-ICD fusion protein," also referred to as "ECD-ICD" or "ECD-ICD fusion protein," which refers to a fusion protein (or fragments thereof) comprising the extracellular domain (or fragments thereof) and the intracellular domain (or fragments thereof) of the HER-2/neu protein. In one embodiment, this ECD-ICD fusion protein does not include a substantial portion of the HER-2/neu transmembrane domain, or does not include any of the HER-2/neu transmembrane domain.

In a further embodiment, the antigen may comprise or consist of "HER-2/neu ECD-PD fusion protein," also referred to as "ECD-PD" or "ECD-PD fusion protein," or the "HER-2/neu ECD-ΔPD fusion protein," also referred to as "ECD-ΔPD" or "ECD-ΔPD fusion protein," which refers to fusion proteins (or fragments thereof) comprising the extracellular domain (or fragments thereof) and phosphorylation domain (or fragments thereof, *e.g*., ΔPD) of the HER-2/neu protein. In one embodiment, the ECD-PD and ECD-ΔPD fusion proteins do not include a substantial portion of the HER-2/neu transmembrane domain, or does not include any of the HER-2/neu transmembrane domain.

In one embodiment, the antigen may comprise a Mage or other appropriate protein linked to an immunological fusion or expression enhancer partner. Fusion proteins may include a hybrid protein comprising two or more antigens relevant to a given disease or may be a hybrid of an antigen and an expression enhancer partner.

The antigen and partner may be chemically conjugated, or may be expressed as a recombinant fusion protein. In an embodiment in which the antigen and partner are expressed as a recombinant fusion protein, this may allow increased levels to be produced in an expression system compared to non-fused protein. Thus the fusion partner may assist in providing T helper epitopes (immunological fusion partner), preferably T helper epitopes recognised by humans, and/or assist in expressing the protein (expression enhancer) at higher yields than the native recombinant protein. In one embodiment, the fusion partner may be both an immunological fusion partner and expression enhancing partner.

In one embodiment of the invention, the immunological fusion partner that may be used is derived from protein D, a surface protein of the gram-negative bacterium, *Haemophilus influenza* B (WO 91/18926) or a derivative thereof. The protein D derivative may comprise the first 1/3 of the protein, or approximately or about the first 1/3 of the protein, in particular it may comprise the first N-terminal 100-110 amino acids or approximately the first N-terminal 100-110 amino acids.

In one embodiment the fusion protein comprises the first 109 residues (or 108 residues therefrom) or amino acids 20 to 127 of protein D.

Other fusion partners that may be used include the non-structural protein from *influenzae* virus, NS1 (hemagglutinin). Typically the N terminal 81 amino acids of NS1 may be utilised, although different fragments may be used provided they include T-helper epitopes.

In another embodiment the immunological fusion partner is the protein known as LytA. LytA is derived from *Streptococcus pneumoniae* which synthesise an N-acetyl-L-alanine amidase, amidase LytA, (coded by the LytA gene (Gene, 43 (1986) page 265-272) an autolysin that specifically degrades certain bonds in the peptidoglycan backbone. The C-terminal domain of the LytA protein is responsible for the affinity to the choline or to some choline analogues such as DEAE. This property has been exploited for the development of E.coli C-LytA expressing plasmids useful for expression of fusion proteins. Purification of hybrid proteins containing the C-LytA fragment at its amino terminus has been described (Biotechnology: 10, (1992) page 795-798). In one embodiment, the C terminal portion of the molecule may be used. The embodiment may utilise the repeat portion of the LytA molecule found in the C terminal end starting at residue 178. In one embodiment, the LytA portion may incorporate residues 188 - 305.

In one embodiment of the present invention, the Mage protein may comprise a derivatised free thiol. Such antigens have been described in WO 99/40188. In particular carboxyamidated or carboxymethylated derivatives may be used.

In one embodiment of the present invention, the tumour associated antigen comprises a Mage-A3-protein D molecule. The nucleotide and amino acid sequences for this molecule are shown in seq ID No 35. This antigen and those summarised below are described in more detail in WO 99/40188.

In further embodiments of the present invention, the tumour associated antigen may comprise any of the following fusion proteins:
A fusion protein of Lipoprotein D fragment, MAGE1 fragment, and histidine tail; fusion protein of NS1-MAGE3, and Histidine tail; fusion protein of CLYTA-MAGE1-Histidine; fusion protein of CLYTA-MAGE3-Histidine.

A further embodiment of the present invention comprises utilising a nucleic acid immunotherapeutic, which comprises a nucleic acid molecule encoding a Mage specific tumour associated antigens as described herein. Such sequences may be inserted into a suitable expression vector and used for DNA/RNA vaccination. Microbial vectors expressing the nucleic acid may also be used as vectored delivered immunotherapeutics. Such vectors include for example, poxvirus, adenovirus, alphavirus and listeria.

Conventional recombinant techniques for obtaining nucleic acid sequences, and production of expression vectors of are described in Maniatis et al., Molecular Cloning - A Laboratory Manual; Cold Spring Harbor, 1982-1989.

For protein based immunotherapeutics the proteins of the present invention are provided either in a liquid form or in a lyophilised form.

It is generally expected that each human dose will comprise 1 to 1000 µg of protein, and preferably 30 - 300 µg.

The method(s) as described herein may comprise a composition further comprises a vaccine adjuvant, and/or immunostimulatory cytokine or chemokine.

Suitable vaccine adjuvants for use in the present invention are commercially available such as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminium salts such as aluminium hydroxide gel (alum) or aluminium phosphate; salts of calcium, iron or zinc; an insoluble suspension of acylated tyrosine; acylated sugars; cationically or anionically derivatised polysaccharides; polyphosphazenes; biodegradable microspheres; monophosphoryl lipid A and quil A. Cytokines, such as GM-CSF or interleukin-2, -7, or -12, and chemokines may also be used as adjuvants.

In formulations it may be desirable that the adjuvant composition induces an immune response predominantly of the Th1 type. High levels of Th1-type cytokines (e.g., IFN-γ, TNFα, IL-2 and IL-12) tend to favour the induction of cell mediated immune responses to an administered antigen. According to one embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989. Accordingly, suitable adjuvants that may be used to elicit a predominantly Th1-type response include, for example a combination of monophosphoryl lipid A, such as 3-de-O-acylated monophosphoryl lipid A (3D-MPL) together with an aluminium salt. 3D-MPL or other toll like receptor 4 (TLR4) ligands such as aminoalkyl glucosaminide phosphates as disclosed in WO 98/50399, WO 01/34617 and WO 03/065806 may also be used alone to generate a predominantly Th1-type response.

Other known adjuvants, which may preferentially induce a TH1 type immune response, include TLR9 agonists such as unmethylated CpG containing oligonucleotides. The oligonucleotides are characterised in that the CpG dinucleotide is unmethylated. Such oligonucleotides are well known and are described in, for example WO 96/02555.

Suitable oligionucleotides include:
OLIGO 1: TCC ATG ACG TTC CTG ACG TT (CpG 1826) [Seq ID No 44]
OLIGO 2: TCT CCC AGC GTG CGC CAT (CpG 1758) [Seq ID No 45]
OLIGO 3: ACC GAT GAC GTC GCC GGT GAC GGC ACC ACG [Seq ID No 46]
OLIGO 4 TCG TCG TTT TGT CGT TTT GTC GTT (CpG 2006, CpG 7909) [Seq ID No 47]
OLIGO 5 TCC ATG ACG TTC CTG ATG CT (CpG 1668) [Seq ID No 48]

CpG-containing oligonucleotides may also be used alone or in combination with other adjuvants. For example, an enhanced system involves the combination of a CpG-containing oligonucleotide and a saponin derivative particularly the combination of CpG and QS21 as disclosed in WO 00/09159 and WO 00/62800.

The formulation may additionally comprise an oil in water emulsion and/or tocopherol.

Another suitable adjuvant is a saponin, for example QS21 (Aquila Biopharmaceuticals Inc., Framingham, MA), that may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other suitable formulations comprise an oil-in-water emulsion and tocopherol. A particularly potent adjuvant formulation involving QS21, 3D-MPL and tocopherol in, for example, an oil-in-water emulsion is described in WO 95/17210.

In another embodiment, the adjuvants may be formulated in a liposomal composition.

The amount of 3D-MPL used is generally small, but depending on the immunotherapeutic formulation may be in the region of 1-1000µg per dose, preferably 1-500µg per dose, and more preferably between 1 to 100µg per dose.

In an embodiment, the adjuvant system comprises three immunostimulants: a CpG oligonucleotide, 3D-MPL, & QS21 either presented in a liposomal formulation or an oil in water emulsion such as described in WO 95/17210.

The amount of CpG or immunostimulatory oligonucleotides in the adjuvants or immunotherapeutics of the present invention is generally small, but depending on the immunotherapeutic formulation may be in the region of 1-1000µg per dose, preferably 1-500µg per dose, and more preferably between 1 to 100µg per dose.

The amount of saponin for use in the adjuvants of the present invention may be in the region of 1-1000µg per dose, preferably 1-500µg per dose, more preferably 1-250µg per dose, and most preferably between 1 to 100µg per dose.

Generally, it is expected that each human dose will comprise 0.1-1000 µg of antigen, preferably 0.1-500 µg, preferably 0.1-100 µg, most preferably 0.1 to 50 µg. An optimal amount for a particular immunotherapeutic can be ascertained by standard studies involving observation of appropriate immune responses in vaccinated subjects. Following an initial vaccination, subjects may receive one or several booster immunisation adequately spaced.

Other suitable adjuvants include Montanide ISA 720 (Seppic, France), SAF (Chiron, California, United States), ISCOMS (CSL), MF-59 (Chiron), Ribi Detox, RC-529 (GSK, Hamilton, MT) and other aminoalkyl glucosaminide 4-phosphates (AGPs).

Accordingly there is provided an immunogenic composition for use in the method of the present invention comprising an antigen as disclosed herein and an adjuvant, wherein the adjuvant comprises one or more of 3D-MPL, QS21, a CpG oligonucleotide, a polyethylene ether or ester or a combination of two or more of these adjuvants. The antigen within the immunogenic composition may be presented in an oil in water or a water in oil emulsion vehicle or in a liposomal formulation.

In one embodiment, the adjuvant may comprise one or more of 3D-MPL, QS21 and an immunostimulatory CpG oligonucleotide. In an embodiment all three immunostimulants are present. In another embodiment 3D-MPL and QS21 are presented in an oil in water emulsion, and in the absence of a CpG oligonucleotide.

A composition for use in the method of the present invention may comprise a pharmaceutical composition comprising tumour associated antigen as described herein, or a fusion protein, in a pharmaceutically acceptable excipient.

### EXAMPLES

### EXAMPLE 1

MAGE008 Mage melanoma clinical trial:
In this on-going trial, the recMAGE-A3 protein (recombinant mage fusion protein as shown in Seq Id No 35) is combined with two different immunological adjuvants: either AS02B (QS21, MPL) or AS15 (QS21, MPL and CpG7909). The objectives were to discriminate between the adjuvants in terms of safety profile, clinical response and immunological response.

In this experiment two adjuvant compositions are made up of mixtures of two immunostimulants:
1. QS21 (Purified, naturally occurring saponin molecule from the South-American tree *Quillaja Saponaria* Molina), and
2. MPL (3 de-O-acetylated monophosphoryl lipid A - detoxified derivative of lipid A, derived from *S. minnesota* LPS).

AS02B is an oil-in-water emulsion of QS21 and MPL.

In animal models these adjuvants have been successfully shown to induce both humoral and TH1 types of cellular-mediated immune responses, including CD4 and CD8 T-cells producing IFNα (Moore *et al.,* 1999; Gérard *et al.,* 2001). Moreover, the injection of recombinant protein formulated in this type of adjuvant leads to the induction of a systemic anti-tumor response: indeed, vaccinated animals were shown to be protected against challenges with murine tumor cells genetically engineered to express the tumor antigen, and regressing tumors were shown to be highly infiltrated by CD8, CD4 and NK cells and by macrophages.

The second adjuvant system is AS15: it contains a third immunostimulant, namely CpG7909 (otherwise known as CpG 2006 supra), in addition to MPL and QS21, in a liposome formulation. In animal models (mainly mice), it has been shown that the addition of CpG7909 further improves the induced immune and anti-tumor responses (Krieg and Davis, 2001; Ren *et al.,* 2004). CpG oligodeoxynucleotides (ODNs) directly stimulate dendritic-cell activation through TLR9 triggering. In addition, in mice, the systemic application of CpG7909 greatly increases the infiltration of transferred T-cells into tumors (Meidenbauer *et al.,* 2004).

### Study overview

### 1. Design

The MAGE008 trial is:
- open
- randomized
- two-arm (AS02B vs. AS 15)
- with 68 patients in total.

As described above, the recMAGE-A3 protein is combined with either AS02B or AS15 adjuvant system.

### 2. Patients population

The recMAGE-A3 protein is administered to patients with progressive metastatic melanoma with regional or distant skin and/or lymph-node lesions (unresectable stage III and stage IV M1a). The expression of the MAGE-A3 gene by the tumor was assessed by quantitative PCR. The selected patients did not receive previous treatment for melanoma (recMAGE-A3 is given as first-line treatment) and had no visceral disease.

### 3. Schedule of immunization

### Method of treatment schedules

### Adjuvant setting

The method of treatment schedule for use in disease in an adjuvant (post-operative) setting may comprise administration of an antigen as described herein according to the following schedules: Administration of antigen at three week intervals for the first 5 to 8 vaccinations, followed at 3 month intervals for the next 8, 9 or more vaccinations.

The antigen may be administered at the exact time frame indicated, or the antigen may be given 1, 2, 3 or 4 days before or after the exact interval, as required or as practical. An example of this schedule is shown in the table below:
**Induction**: 5 vaccinations at intervals of 3 weeks for example Weeks 0, 3, 6, 9, 12 or Weeks 0, 6, 9, 12
**Maintenance**: 9 vaccinations at intervals of 3 months

Alternatively, the vaccinations may be given initially at 2 week intervals, for example 6 injections at two week intervals followed by appropriate maintenance therapy.

### Active disease

The method of treatment schedule for use in active or unresectable disease, for example in melanoma cancer, comprising: administration of an antigen as described herein at two or three week intervals for the first six months to one year of treatment. A schedule may comprise the following pattern of injections: the antigen may be given at two week intervals for the first 4 to 10 vaccinations, followed by 3 week intervals for the next 4 to 10 vaccinations, then at 6 week intervals for the next 3 to 7 vaccinations. Long term treatment may then continue with vaccinations at 3 month intervals for 3 to 5 vaccinations, followed by 6 month intervals for the next 3 to 5 vaccinations.

The antigen may be administered at the exact time frame indicated, or the antigen may be given 1, 2, 3 or 4 days before or after the exact interval, as required or as practical.

An example of this schedule is shown in the table below:

| | |
|---|---|
| **Cycle 1:** | 6 vaccinations at intervals of 2 weeks (Weeks 1, 3, 5, 7, 9, 11) |
| **Cycle 2:** | 6 vaccinations at intervals of 3 weeks (Weeks 15, 18, 21, 24, 27, 30) |
| **Cycle 3:** | 4 vaccinations at intervals of 6 weeks (Weeks 34, 40, 46, 52) |
| **Long Term Treatment:** | 4 vaccinations at intervals of 3 months, for example followed by 4 vaccinations at intervals of 6 months |

For both of the above treatment regimes additional vaccinations may be given after treatment, as required.

In order to screen potential participants in the above clinical trial we received biopsies of the tumor, prior to any immunization, as frozen tumor samples. From these samples we extracted RNA for the quantitative PCR. The quality of this purified RNA was extremely high and it was suitable for microarray analysis. We therefore analyzed the tumor samples by microarrays. The goal was to identify a set of genes associated with the clinical response so that patients likely to benefit from this antigen-specific cancer immunotherapeutic are properly identified and selected. Gene profiling has been performed only on biopsies from patients who signed the informed consent for microarray analysis.

### Materials and Methods

### Tumor specimens

30 tumor specimens (pre-vaccination) were used from the Mage008 Mage-3 melanoma clinical trial. These were fresh frozen preserved in the RNA stabilizing solution RNAlater.

### RNA purification

Tumoral total RNA was purified using the Tripure method - Tripure extraction (Roche Cat. No. 1 667 165). The protocols provided were followed subsequently by the use of an RNeasy Mini kit - clean-up protocol with DNAse treatment (Qiagen Cat. No. 74106). http://wwwl.qiagen.com/literature/handbooks/PDF/RNAStabilizationAndPurification/FromAnim alAndPlantTissuesBacteriaYeastAndFungi/RNY_Mini/1035969_HB.pdf. *www.roche-applied-science.com*/*PROD_INF*/*MANUALS*/*napi_man*/*pdf*/*chapter4*/*page_152-158.pdf*

### RNA quality control

Quantification of RNA was initially completed using optical density at 260nm and Quant-IT RiboGreen RNA assay kit (Invitrogen - Molecular probes R11490). http://probes.invitrogen.com/media/pis/mp11490.pdf.

The Quality of the 28s and 18s ribosomal RNA peaks were assessed by use of the Agilent bioanalyser.

### RNA labeling and amplification for microarray analysis

Due to the small biopsy size received during the clinical study an amplification method was used in conjunction with the labeling of the RNA for microarray analysis.

The Nugen 3' ovation biotin kit (Labelling of 50 ng of RNA - Ovation biotin system Cat; 2300-12, 2300-60) http://www.nugeninc.com/html/03_products2.html

A starting input of 50ng of total RNA was used.

### Microarray chips

The Affymetrix HU-U133.Plus 2.0 gene chips were utilized as these were the most up to date chips (based on the human genome project) available from the supplier. These chips cover about 47,000 potential gene transcripts.

### Microarray hybridization and scanning

The hybridized chips were washed and scanned according to the standard Affymetrix protocols: Affymetrix gene chip expression analysis protocols can be downloaded from http://www.affymetrix.com/support/technical/manual/expression_manual.affx.

The scanner that has to be used with the Affymetrix microarray workstation is http://www.affymetrix.com/products/instruments/specific/scanner 3000.affx Data normalization

The fluorescent scanned data was then normalized using RMA to allow comparisons between individual chips. The following reference describes this method.

Irizarry RA, Hobbs B, Collin F, Beazer-Barclay YD, Antonellis KJ, Scherf U, Speed TP. Exploration, normalization, and summaries of high density oligonucleotide array probe level data. Biostatistics. 2003 Apr;4(2):249-64.

### Data analysis

Three independent methods were used to generate gene lists to distinguish responder from non-responding patients. The methods are 1. Spotfire, 2. Baldi BH and 3. Arrayminer.

The Baldi analysis method is available from the following website. http://www.igb.uci.edu/∼pfbaldi/software_and_servers.htm.

The spotfire software can be purchased from this site http://www.spotfire.com. The arrayminer software is available for purchase at http://www.optimaldesign.com/ArrayMiner/ArrayMiner.htm.

All data analysis was performed with the use of the R statistics software (available at www.r-project.org) and various Bioconductor packages for R (available at www.bioconductor.org).

The raw data were pre-processed for background correction, normalisation and probe summarisation by the RMA (Irizarry RA, Hobbs B, Collin F, Beazer-Barclay YD, Antonellis KJ, Scherf U, Speed TP. Exploration, normalization, and summaries of high density oligonucleotide array probe level data. Biostatistics. 2003 Apr;4(2):249-64)

The differential gene expression between any two groups was computed by two different statistical methods, the RankProduct (Breitling, R., Armengaud, P., Amtmann, A., and Herzyk, P.(2004) Rank Products: A simple, yet powerful, new method to detect differentially regulated genes in replicated microarray experiments, FEBS Letter, 57383-92) and the CyberT (P. Baldi and A.D. Long, "A Bayesian Framework for the Analysis of Microarray Expression Data: Regularized t-Test and Statistical Inferences of Gene Changes", Bioinformatics, 17, 6, 509-519, (2001)) methods.

In each of them, a correction for multiple testing was done with the method of Benjamini-Hochberg (Y. Benjamini and Y. Hochberg, Controlling the false discovery rate: a practical and powerful approach to multiple testing, J. Roy. Stat. Soc. B 57 (1995)), and the threshold of 5% was chosen for the false discovery rate; thus, the genes had to have a corrected p-value less or equal to 0.05 to be considered as differentially expressed. Baldi-BH is equivalent to CyberT followed by Benjamini-Hochberg correction.

### 1. Spotfire analysis

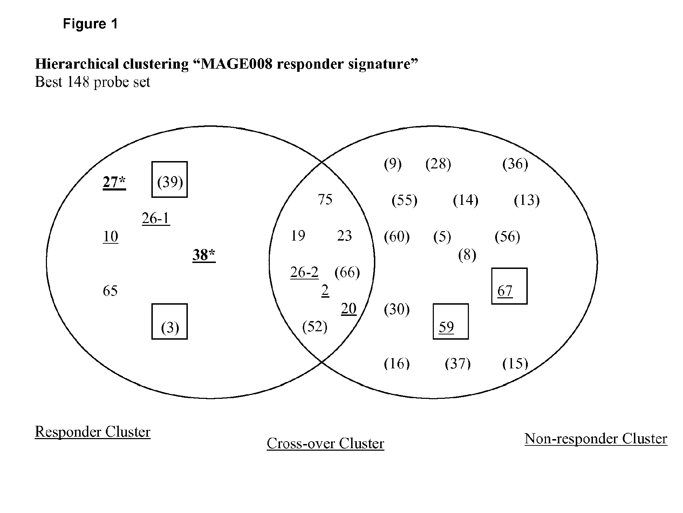

The results of the analysis of patient samples (from the clinical research program named Mage008) are summarized in the figure 1.

As a first step, a supervised comparison was performed in order to find a group of genes able to cluster. Two patients that showed clinical response (patient 67 and 59) fell within the cluster of non-repsonders. Two patients who were non-responders (patient 3 and 39) fell within the cluster designated as responders. The responding patients are also found within cross over cluster (ie the areas of overlap in the figure). However, there are two patients who are non-responders that fall within this area of overlap.

In Figure 1 RESPONDERS were patients 27 and 38 (full/complete clinical responders), patients 2, 10, 20, 26-1, 26-2, 59 & 67 (mixed responders) and patients 19, 23, 65 and 75 (stable disease). NON-RESPONDERS in Figure 1 were patients 3, 5, 8, 9, 13, 14, 15, 16, 28, 30, 36, 37, 39, 52, 55, 56, 60 and 66.

### 2. Baldi BH analysis

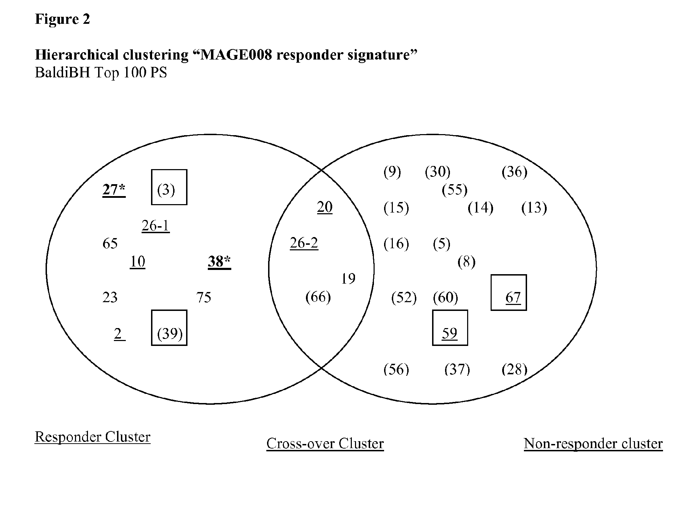

This software is designed for statistical supervised gene classification in microarray experiments. A list of 100 most significant genes were identified that define the two groups. See figure 2 below. The clustering is a more effective than with spotfire with the majority of the responders found in the responder cluster. However we still see the same patients non responding (patients 3, 39), and the responding patients (67 and 59) that are located within the wrong clusters.

In Figure 2 RESPONDERS were patients 27 and 38 (full/complete clinical responders), patients 2, 10, 20, 26-1, 26-2, 59 & 67 (mixed responders) and patients 19, 23, 65 and 75 (stable disease). NON-RESPONDERS in Figure 2 were patients 3, 5, 8, 9, 13, 14, 15, 16, 28, 30, 36, 37, 39, 52, 55, 56, 60 and 66.

### 3. Arrayminer analysis

This software is classifies genes by using a "train and test" method. In this method the software first identifies a discriminant set of markers for the training classes, similar to a classical cross-validation study.

Here due to the analysis there is only the responder or non-responder cluster, with no area of overlap. Here again there is mis-classification of samples, in particular patients 59 and 67 who both responded to treatment but in this analysis were in the cluster of non-responders. Patients 3, 14, 39, 52 and 66 were non-responders but were located in the cluster for responders using this analysis.

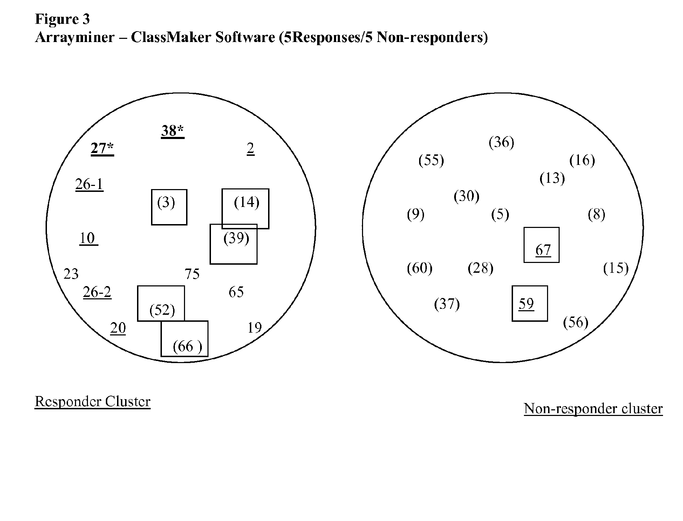

In Figure 3 RESPONDERS were patients 27 and 38 (full/complete clinical responders), patients 10, 20, 26-1, 26-2, 59 & 67 (mixed responders and patients) 19, 23, 65 and 75 (stable disease). NON-RESPONDERS in Figure 3 were patients 3, 5, 8, 9, 13, 14, 15, 16, 28, 30, 36, 37, 39, 52, 55, 56, 60 and 66.

### Combination of the three methods

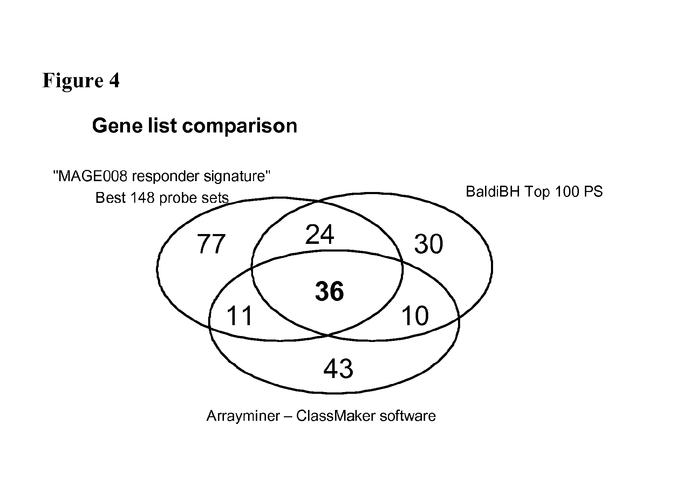

The three methods were used together to find a common set of gene probes that will define the signature with high significance. A venn diagram was created comparing all of the results. See figure 4. A list of 36 probesets was found that are common with all three programs. (Table 1B). These 36 probesets were then used for a supervised clustering of the responding and non-reponding patients. The genes which are identified by the 36 probes sets form a specific aspect of the invention.

### Fold change definition

In programs such as Arrayminer, Fold change of gene expression can be calculated. This is shown in Table 1B and is the change in average gene expression between two groups, for example responder vs non-responder. The statistical value attached to the fold change is calculated and is the more significant in genes where the level of expression is less variable between patients in each group, and difference between groups is larger.

### Gene expression shown on a heat map

Figure 5 is a heat map (a diagrammatic respresentation of the expression level of various genes for a given patient), on which can be seen individual expression of genes represented in the Y-Axes by a coloured box and the patients in the X-axes. The expression values of each gene in all samples are normalized to a mean of zero and a standard deviation of 1, and these normalized values are coded into a gradient of colors.

Affymetrix arrays have 11 probe pairs available to interrogate each gene. Ideally, the signal intensity from all of these 11 probes should be equal as they all interrogate the same gene. However, there are enormous differences between individual probes in a probe set. The pattern of the probe signals in a probe set is called a "probe response pattern". The Robust Multi-array Average" (RMA) software uses, model-based algorithms to incorporate information from multiple microarrays to calculate the expression of a gene. After proper correction for background, and quantile normalisation of the probe intensities, the probe response pattern is fitted over multiple arrays an additive model in RMA software. These algorithms use the fitted models to detect abnormally behaving probes, which are subsequently excluded for calculating gene expression. Therefore, gene expression from these model-based algorithms can be expected to provide more reproducible results. RMA use a stochastic model to estimate gene expression which is potentially a better way of estimating gene expression. Algorithms are implemented in RMA software.

**Figure 4****:** The predictive 36 probesets correspond mainly to upregulation of genes related to immune infiltration and activation. These genes include HLA class II, Interleukin-2 receptor gamma, T cell receptor genes (TRBV19, TRAT1, TRGC2), granzyme, and CD69.

### Increase in treatment efficacy

As described herein, gene profiling has allowed the identification of a subset of patients likely to respond to recMAGE-A3 treatment. This subset corresponds to ∼30% of the patient population. Patients having the gene profile show far higher efficacy levels could be obtained. Indeed, with the early identification of patients susceptible for anti-MAGE-A3 immunization, the efficacy of the treatment will probably increase to levels far higher than other current cancer treatments. This may also lead to a better compliance by the patient. For example, in 100 patients with melanoma, 60 will be found to express MAGE-A3. If these patients all receive recMAGE-A3 injections, 10 out of them will respond; leading to a 15% efficacy rate. However, if gene profile is performed on the 60 MAGE-A3-expressing patients, only the 14 out of them who are predicted to be susceptible to this treatment will receive recMAGE-A3 injections. The other 46 patients will receive another treatment. 10 out of these 14 patients will respond to the treatment, leading to a 71% efficacy rate.

### Gene profile in other tumour types

This gene profiling has also been performed in a NSCLC study. However, as this study is blinded, only preliminary data are available. It is thought that the signature observed in melanoma is also present in NSCLC, stages IB and II. Preliminary data suggests aproximately 30% of the patients present a gene profile signature characteristic of immune response and immune cell infiltration and that suggests they will respond to ASCI treatment.

Figure 5 shows and expression profile for 30 individual patients for a number of genes.

Fig 5a shows the expression profiles for 31 patients of two genes.

### EXAMPLE 2:

### Predicting the clinical outcome of patients using machine learning approaches.

### Materials & Methods.

### Tumor specimens, RNA purification, quality control, labeling and amplification for microarray analysis

Tumor specimens (pre-vaccination) were used from a Mage-3 melanoma clinical trial (MAGE008). These were preserved, RNA prepared, labeled and amplified as in Example 1 above. The quality of the RNA samples was checked prior to hybridization to the genechip. Only data from samples that were deemed to adequately hybridize to the genechip were used in this Example (61 samples in total).

### Microarray chips, hybridizations and scanning.

As in the material and methods section of the first example above (referred to herein as Example 1), Affymetrix HG-U133.Plus2.0 genechips were employed, and hybridized and scanned as described therein.

### Data processing & normalization.

The fluorescent scanned data image was processed and normalized using a R 2.3.1 [1] implementation of GCRMA algorithm [2, 3].

### Unspecific filtering of gene expression matrix

Prior to calculation of differential expression gene expression matrix was filtered in an unspecific (i.e. independently of experimental groups) manner. 4 processes were used:
1. panp filtering: calls indicating whether a gene is expressed in a given sample (present call, P) or not (absent call, A) were derived for all array measures using the panp method implemented in the panp R package [4]. Only probe sets showing at least one P call throughout experimental samples were kept for subsequent calculations.
2. SD filtering: standard deviation (SD) of each probe sets were calculated throughout samples, were only kept probe sets having SDs above the 75th percentile of all SDs.
3. IQR filtering: interquartile range (IQR) of each probe sets were calculated throughout samples, were only kept probe sets having IQRs above the 7th quantile of all IQRs.
4. SH filtering: SD of each probe sets were calculated throughout samples, the midpoint of the SD shorth (the shortest interval containing all of the data) as calculated using the shorth function of genefilter package [5] under R 2.3.1, were only kept probes sets having SD above the shorth for subsequent calculations.

### Calculation of differential expression.

The differential expression between the groups of patients showing a clinical benefit after vaccination or not (subsequently called Responder (R) or Non-Responder (NR) groups) was calculated in R 2.3.1 program according to 2 statistical procedures:
1. Regular t-test, as implemented in the genefilter package, and
2. A modified (regularized, moderated) t-test, to account for poor estimates of gene-specific variance under standard t-test, such as the method of Jain et al [6], implemented the LPE R package [7], or the method of Smyth [8], implemented in the limma R package [9], or the method of Baldi and Long [10], also known as Cyber-T [11].

Correction for multiple testing by controlling the false discovery rate (FDR) at 5% was done under R 2.3.1 with Benjamini-Hochberg method [12] implemented in the multtest package [13].

### Gene profile normalizations

To make the probe sets having low and high levels of expression comparable and to put an emphasis on differential profiles rather than absolute profiles, data was further normalized at the probe set level. Two gene-level normalizations schemes were used:
1. IQR normalization, where each probe set measure is subtracted by its median over samples and divided by its IQR.
2. Z-score normalization, where each probe set measure is subtracted by its mean over samples and divided by its SD.

### Machine learning algorithms

The 14 machine learning algorithms (or predictive rules) interfaced in MLInterfaces package [14] running under R 2.3.1 program were used to train clinical outcome predictive models and to predict the Mage008 patient clinical outcomes, under the reporter lists calculated by the unspecific filtering, differential expression, gene normalization processes. These algorithms were, knn (k-nearest neighbour, kNN, function of class package), nnet (neural network), gbm (generalized boost regression), lvq1 (learning vector quantization 1), naiveBayes (naive bayes classifier), svm (support vector machine), lda (linear discriminant algorithm), rpart (recursive partitioning), stat.diag.da (diagonal discriminant analysis), randomforest (random forest), bagging, ipredknn (k-nearest neighbour function form ipred package, equivalent to knn form class package), slda (stabilized linear discriminant analysis), pamr (partition around medoids, also known as nearest shrunken centroid classifier). Default parameters were used for all algorithms except for kNN rule where even values of k ranging from 1 to 7 were tested. Leave-one-out (LOO) scheme of MLinterfaces coded in the xval function was used for cross-validation when appropriate, without re-calculation of reporter list at each cross-validation loop. The misclassification rate was calculated by averaging the number of wrongly predicted samples to the total number of samples of each patient group or class (either R or NR).

Some of the MLIinterfaces interfaced predictive rules (nnet, lda, naiveBayes) were used outside of the MLInterfaces package as direct calling to make the related classifiers usable on external data sets independently of the data used to train the models.

The MiPP algorithm [15, 16], implemented in the MiPP R library and ran under R 2.3.1, was employed to reduce the number of probe sets involved in the reporter lists. The best reporters out of the submitted list were selected as optimizing the 10-fold cross-validation of linear discriminant analysis predictions of a training set and further reduced to reporters giving an optimized global misclassification error of linear discriminant analysis predictions of a testing set. Global misclassification error or rate was calculated as the ratio of the total number of misclassified patients to the total of set patients.

### Results

### Patient sample stratification for training and testing section definitions.

A 31 sample subpart of the full 61 sample data set was used to train and test the classifying model in a first attempt; the remaining 30 samples were kept for subsequent model evaluations.

Several training and testing sections were defined from the 31 sample data set subpart. The training sections were used to calculate the reporter list (i.e. the differentially expressed genes to be used in clinical outcome prediction of patient samples) and to set the machine learning algorithm weights for clinical outcome prediction. The testing sections allowed evaluation of the model performance independently of the patients used to construct the predictive model. Model evaluations were also undertaken on the training sections according to a cross-validation procedure.

Training and testing sections from the 31 sample data set subpart were defined as follows:
1. A first training section containing 5 Responders and 5 Non-Responders, leaving 21 patients for the testing section;
2. A second training section of 10 Responders and 11 Non-Responders, leaving 10 patients for the testing section;
3. A last training section containing all 31 patients (13 Responders and 18 Non-Responders). In this last stratification there is no patient left for a testing section, classifying model would only be evaluated by a cross-validation procedure.

### Identification of the differential expression calculation process performing best for classification.

Gene expression data was pre-processed and normalized according to Material & Methods taking into account the full data set. Differential expressions (DE) between the Responder and Non-Responder groups were calculated for each training / testing section according to various processes.

The DE calculation framework was constructed as follows:
1. Unspecific filtering of gene expression matrix: panp filtering, or not, followed by either SD, IQR or SH filtering, or none.
2. Calculation of DE : either t-test or a modified (regularized, moderated) t-test, such as the method of Jain *et al,* implemented the LPE R package, or the method of Smyth, implemented in the limma R package, or the method of Baldi and Long (Cyber-T), followed by multiple testing correction, or not.
3. Gene profile normalizations: Either IQR or Z-score normalizations, or none.

The processes minimizing the overall misclassification rates in both training and testing sections in at least two out of the three available training / testing stratifications were selected. The misclassification rate was obtained by comparing the predicted clinical outcome given by a kNN predictive rule to the given clinical outcome.

Seven DE processes were found as minimizing the overall misclassification rate according to the kNN rule:
- t-test without correction for multiple testing and irrespective of panp filtering;
- SH filter, t-test without correction for multiple testing and irrespective of panp filtering;
- SD filter, t-test without correction for multiple testing;
- SD filter, t-test without correction for multiple testing, Z-score normalization;
- IQR filter, t-test without correction for multiple testing, IQR normalization.

The minimized overall misclassification rates for these processes were as follows:
- 0% in the 5 Responders to 5 Non-Responders stratification, as evaluated by leave-one-out cross-validation, all patients were therefore correctly classified.
- 20% in the 21 patients testing section, given by the direct prediction of these patients from the model defined from the 5 Responders to 5 Non-Responders training set. 80% of patients were correctly predicted.
- 12% for the 13 Responders to 18 Non-Responders segregation, as assessed by cross-validation, being 88% of patient correctly predicted.

The performances for the other training/testing stratification was not found to be informative (i.e. no DE process converging to a minimized misclassification rate), hence not mentioned.

The last process, IQR filter, t-test without correction for multiple testing, IQR normalization, was preferred since the minimized misclassification rates were obtained with a higher value of k (k=5) than other six processes (k=3). It was anticipated that working with higher values of k would facilitate the transposition of the predictive model in further settings, since high k values generate simpler models supposed to be less biased towards a training set.

This process generated a reporter list of 489 Affymetrix probe sets. The reporter list is given in Table 4A, as Affymetrix probe set identifications along with gene names and symbols.

### External validation: independent evaluation of the best clinical outcome predicting model.

The best DE calculation and gene expression treatment process (IQR filter, t-test without correction for multiple testing, IQR normalization) was used to build a kNN predictive model (k=5) from 13 Responders/18 Non-responders training section.

The clinical outcome of the independent patients that were previously left away was then directly predicted from this model. Of these 30 independent patients only 15 were informative (i.e. having a characterized clinical outcome). Misclassification rate was calculated on the basis on these 15 informative patients for the model evaluation.

According to this predictive model, misclassification rate was 34%, hence 66% of independent patients had their clinical outcome correctly predicted.

### Improvement of clinical outcome predicting model through other classification rules.

On the basis of the best DE (and expression normalization) process as selected with a kNN rule, further classification (predictive) rules were tried to eventually increase the predictive model performance. Beside kNN, 13 other rules were assessed. For each selected DE process that performed for the kNN rule, each further rule was trained on the 13 Responders/18 Non-responders section of the data set, its predictive performance evaluated on the training set by cross-validation and on the independent set of 30 patient samples by direct prediction.

Further classification rules did not improve the misclassification rate, as assessed by cross-validation on the training set, the lowest remaining unchanged at 12% of samples miss-predicted (88% of patient assigned to their correct clinical outcome).

However, for the above mentioned preferred DE process, the neural network rule did improve the misclassification rate on the independent set of 15 informative patients out of the 30 available, by reducing it from 34%, as given by KNN rule, to 30% (i.e. one additional patient correctly predicted), while keeping it the lowest on the cross-validation evaluation of the training set. Neural network rule then allows the correct prediction of 70% of patient independently of those used to train the model.

Furthermore, in a further evaluation of the predictive modeling performance, when clinical outcomes were available for all 30 external patient samples, i.e. when all the independent patients were informative for misclassification rate calculation, the lowest misclassification error became 26%, which is 74% of patients were correctly classified. This best performance obtained on a wider data set in terms of informative sample number was given by a lda rule. A naiveBayes rule was also able to give a 28% misclassification rate, correctly prediction the clinical outcome of 72% of patients. However, the misclassification rate using kNN and nnet rules became 35% and 41%, respectively. This performance is based on the use of the gene list and probes of Table 4A.

### Implementation of the preferred classifiers, predicting the clinical outcome of further patient biopsies.

To make use on further metastatic melanoma samples of the exemplified neural network classifier, yielding 30% of misclassification on the 15 informative sample external set, the following R code can be used in a R session (a stastical programme):
library(nnet)
set.seed(1234)
predict(nn, data, type="class")
where
- data is a data frame containing the 489 reporter (Table 4A) expression data of the samples to classify (GCRMA sample normalized and IQR gene normalized). Samples are organized in columns and reporters in rows;
- nn is the R object of class nnet shown in **Appendix A**.
the lda (linear discriminant analysis) classifier would be reproduced by programming the following R code chunk :
library(MASS)
predict(lda, data, type="class")
where
- data is as previously;
- lda is the R object of class lda shown in **Appendix B.**

The naïve Bayes classifier works under the following R coding:
library(e1071)
predict(nb, data, type="class")
where
- data is as previously;
- nb is the R object of class naiveBayes shown in **Appendix C.**

### Down-sizing the 489 probe set reporter list while maintaining an identical classification performance.

The MiPP algorithm was used to determine if it was possible to reduce the number of reporters involved in the IQR filter, t-test without correction for multiple testing, IQR normalization, based predictive model while keeping the same misclassification rate. Indeed, working under a shorter reporter list would be more convenient for classifier follow-up purposes for instance such as transposition from a microarray based format to a quantitative RT-PCR format.

The 489 probe set microarray data was submitted to the MiPP process: 11 classifying probe sets were selected on the 13 R / 18 NR training section as being sufficient to achieve a lowest global misclassification error, and 4 probe sets out of these were further identified as minimal on the 30 informative external sample data set to obtain the lowest global misclassification rate for the independent patients.

The 4 probe set reporter list was then used in leave-one-out cross-validation predictions of the 13 R / 18 NR stratification of data as training set and direct prediction the 30 informative patient section as external testing set under all 14 predictive rules. LOO performance was increased for most of the rules (being as high as 3% of misclassification rate for lda and naiveBayes among other), while misclassification rate stayed 28% on the external data set under svm and gbm rules.

The following table depicts the identity of the genes involved in the 4 probe set model :

| **Probe Set ID** | **Gene Symbol** | **Gene Name** |
|---|---|---|
| 207651_at | GPR171 | G protein-coupled receptor 171 |
| 205392_s_at | CCL14 | chemokine (C-C motif) ligand 14 |
| 212233_at | MAP1B | microtubule-associated protein 1B |
| 206204_at | GRB14 | growth factor receptor-bound protein 14. |

### EXAMPLE 3:

**Predicting the clinical outcome of patients using Quantitative Polymerase Chain Reaction**

### (Q-PCR)

### Material & Methods

**Tumor specimens and RNA purification.**

30 tumor specimens (pre-vaccination) were used from MAGE-A3 melanoma clinical trial (MAGE008). These were preserved and RNA extracted as in Example 1 above.

**cDNA synthesis and quantitative PCR amplification**

2 µg of total RNA were retro-transcripted into cDNA using M-MLV reverse transcriptase (Invitrogen) and oligo(dT) or random primers.

The different interesting genes were amplified by quantitative PCR using TaqMan chemistry and 7900 sequence detection system (Applied Biosystems ).

Genes were amplified using standard 96 well plates or the TaqMan® Immune Profiling Array (TaqMan Low density arrays - TLDA - Applied Biosystems). TLDA are ready to use 384 well plates pre-coated with primers and probes.

The 7900 apparatus is a fully integrated system for real-time detection of PCR including a 96-well or a TLDA thermal cycler, a laser to induce fluorescence, a charge-coupled device detector, a computer and a real-time sequence detection software.

In standard 96 well plates, cDNA corresponding to 50 ng of total RNA was amplified by polymerase-chain-reaction using the TF,TAQMAN,PCR REAGENT CORE KIT (Applied Biosystems). Primers and probes are listed in **Table 5.**

For the TaqMan® Immune Profiling Array, cDNA corresponding to only 1 ng of RNA was amplified by polymerase-chain-reaction using the TF,TAQMAN,PCR CORE REAGENT KIT (Applied Biosystems). The genes included in the array are listed in **Table 6.**

### Data processing and normalization

All PCR data were normalized against H3F3A (standard 96 Well plates) or the geometric mean of GUSB and PGK1 (TLDA) house keeping genes (also referred to as constant genes). Expression values were afterwards log transformed.

### Univariate statistical analysis

Analyses of variance (ANOVA1) were performed using the SAS software to significantly select the genes able to differentiate responder from non-responder groups. The first group was composed of 14 responder patients, the second one was composed of 15 non-responder patients. Each gene was analyzed independently from the others.

IL7R, CD3D, CD52, UBD, GPR171, GMZK, PRKCQ, STAT4, TRDV2, TRAT1, TRBV19, CD69, INDO1, CD45R, CD45RO, FoxP3, CD20, CCL5, FASLG, GNLY, GZMB, PRF1, IFNG, ICOS, TBX21, CD8A, CD3E, CXCL10, CXCL11, IRF1, TLR7 and CXCR3 genes were selected for their capacity to differentiate both groups. The p-value was <0.0001 for every gene. For every gene, a significant difference was proven between mean (responder/non- responder).

The Geomean ratios between both groups were also calculated for all genes (Table 7A) and range from 3.4 to 21.2.

### Correlation analysis

A correlation matrix **(Table8)** was calculated between 30 genes showing that all these genes are pretty well correlated.

### Logistic regression model

An analysis by logistic regression was performed using Proc logistic (SAS Sofware). Logistic regression analysis is often used to investigate the relationship between binary responses and a set of explanatory variables.

The number of predictors is too large and the use of all possible regression is not feasible. So, stepwise selection was employed. This procedure gives a model with a good value (high) for a specified criterion: Score Chi-square. This is analogous to the use of SSE or R² in multiple linear regression.

The model information are:
Data: 29 patients and 111 genes.
Response variable: clinical status
Number of response level: 2 (responder - non responder)
Mode: binary logit
Optimization technique: Fisher's scoring

The model giving the best value of score Chi-square (Chi-square = 16 and pvalue <0.0001) is : Logit (p) = 7.69 + 5.07 log(PRF1), with p the probability to be responder

Coefficient B0 (7.69) & B1 (5.07) are significant (p < 0.005)

Other criterion can be used to test goodness of fit

| | | | |
|---|---|---|---|
| Likehood Ratio | : | 23.1429 (chi-square) and | <0.001 (p-value) |
| Wald | : | 6.9250 (chi-sqaure) and | <0.001 (p-value) |

Like in linear regression, a R² is computed: 0.55.

From the 111 genes, 18 (with only one predictor) are listed in the **Table 9.** Higher the R2(%) and chi-square score are the better the model/gene.

Using the PRF1 model, it is possible to classify correctly 86.6 % of patients.

**Table 10** gives the percentage of correct classification calculated using the logistic regression model for some other genes.

Combining more than 1 gene didn't improve the classification performance.

### Genex analysis

The 30 patients were also classified using the PCA (Principal Component Analysis) and neural network analysis from Genex software with the genes PRF1, GZMB, GNLY, CD8A, PRKCQ, FOXP3, IFNG, CCL5, GPR171 and TRBV19.

For the neural network analysis, 5 responder patients (Patient ID No.s 27, 53, 65, 119, 127) and 5 non responder patients (Patient ID No.s 8, 60, 66, 81, 160) were used as training set. The remaining 20 patients were used as testing set.

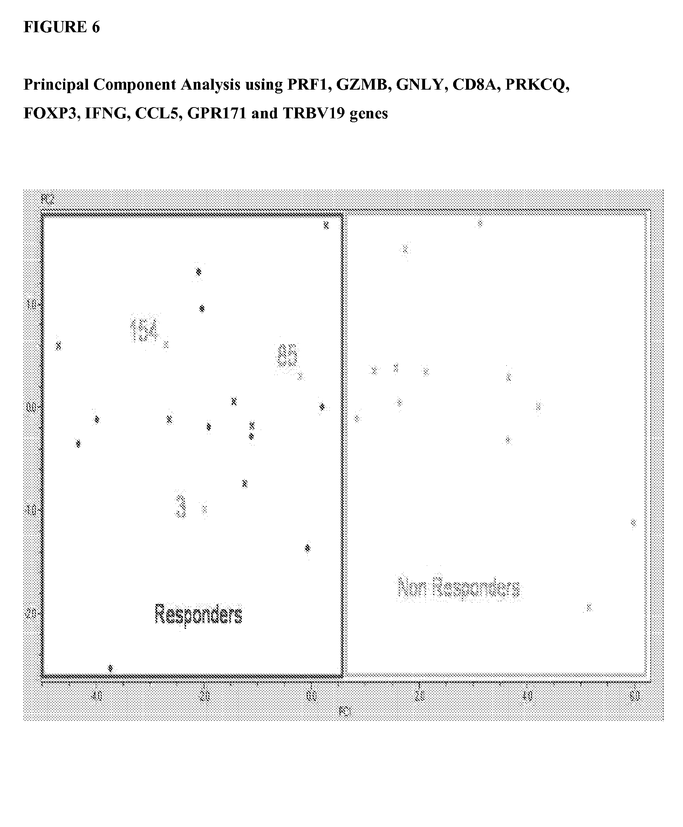

Figure 6 shows the Principal Component Analysis using PRF1, GZMB, GNLY, CD8A, PRKCQ, FOXP3, IFNG, CCL5, GPR171 and TRBV19 genes.

It can be seen the responders are clustered on the left-hand side of Figure 6 and the non-responders are clustered on the right-hand side of the figure. Patients labeled 85, 3 and 154 are miss-classified. Therefore, the percentage of "correct" classification is 85% using the PCA. The neural network approach confirms a correct classification range of 85%.

Comparison: Microarray to Q-PCR data. 17 genes (Table 12) evaluated by the Q-PCR technology were already present in the microarray gene lists. These genes are therefore able to discriminate responder from non responder patients whatever the technology used to detect them. These genes form a specific aspect of the invention.

**Table 12 Genes present in microarray and Q-PCR list.**

| **Gene symbol** | **Gene title** |
|---|---|
| CCL5 | chemokine (C-C motif) ligand 5 |
| TRAT1 | T cell receptor associated transmembrane adaptor 1 |
| STAT4 | signal transducer and activator of transcription 4 |
| PRKCQ | protein kinase C, theta |
| GPR171 | G protein-coupled receptor 171 |
| UBD | ubiquitin D |
| CD52 | CD52 molecule |
| CD3D | CD3d molecule, delta (CD3-TCR complex) |
| PRF1 | perforin 1 (pore forming protein) |
| CD8A | CD8a molecule |
| CXCL10 | chemokine (C-X-C motif) ligand 10 |
| CD69 | CD69 molecule |
| TRBV19 | T cell receptor beta variable 19 |
| TRDV2 | T cell receptor delta variable 2 |
| IL7R | interleukin 7 receptor |
| GZMK | granzyme K PROTEIN-TYROSINE PHOSPHATASE, RECEPTOR-TYPE, C |
| CD45R | (PTPRC) |

Fourteen genes (Table 13) were able to discriminate responder from non-responder patients using Q-PCR technology were not present in the microarray gene lists. This may be due to the increased sensitivity of the Q-PCR technology compared to the microarray.

**Table 13. Gene used to classify patients using Q-PCR technology, absent in Microarray gene lists.**

| **Gene symbol** | **Gene title** |
|---|---|
| FASLG | Fas ligand (TNF superfamily, member 6) |
| GNLY | granulysin granzyme B (granzyme 2, cytotoxic T-lymphocyte-associated serine |
| GZMB | esterase 1) |
| IFNG | interferon, gamma |
| ICOS | inducible T-cell co-stimulator |
| TBX21 | T-box 21 |
| CD3E | CD3e molecule, epsilon (CD3-TCR complex) |
| CXCL11 | chemokine (C-X-C motif) ligand 11 |
| CXCR3 | chemokine (C-X-C motif) receptor 3 |
| CD20 | CD20 molecule |
| FOXP3 | forkhead box P3 |
| INDO | indoleamine-pyrrole 2,3 dioxygenase |
| IRF1 | interferon regulatory factor 1 |
| TLR7 | toll-like receptor 7 |

Some genes absent in the microarray signature but are known to be expressed by immunological cells and thus are also able to predict the clinical outcome of the patients.

The presence of an immune infiltration into the tumoral tissue seems to be the most important biological event to predict the clinical outc ome of the patients rather than only a limited list of genes.

For example, PRF1 and CD8A are present in the microarray gene lists and are expressed by CD8 T lymphocytes. CD8 T lymphocytes express also IFNG that is absent in the microarray gene lists. Nevertheless, a good prediction of the clinical response was achieved using this gene by Q-PCR.

The number of miss-classified patients using the q-PCR data remains in the same range than clustering and KNN approaches using microarray data. Therefore different technologies and **s**oftwares can be used to classify the patients.

### Example 4:

### Predicting the clinical outcome of patients using hierarchical clustering approach. Material & Methods

Tumor specimens and RNA purification. 67 tumor specimens (pre-vaccination) were used from MAGE-A3 melanoma clinical trial (MAGE008). These were preserved, RNA prepared, quality controlled, labeled and amplified as in Example 1 above. The quality of the RNA samples was checked prior to hybridization to the genechip.

### Microarray chips, hybridizations and scanning

As in the material and methods section of the first example above (referred to herein as Example 1), Affymetrix HG-U133.Plus2.0 genechips were employed, and hybridized and scanned as described in Example 1.

### Data processing & normalization.

The fluorescent scanned data image was processed and normalized using a R 2.3.1 implementation of GCRMA algorithm as described in Example 2.

### Selection of the gene list

41 Probe Sets were selected to discriminate responder from non responder patients using Arrayminer software described in Example 1.

Analysis parameters were:
- Train and test evaluation
- Pre-definition of 2 class
   a. Responder class : PID2, PID65, PID75, PID20, PID10, PID19, PID23, PID26, PID27, PID38, PID67
   b. Non Responder class : PID14, PID52, PID66, PID37, PID16, PID13, PID55, PID56, PID5, PID60, PID8
      wherein PID stands for patient identification No (ie the numerical label given to the patient)
- Maximum number of markers per class: 25
- Number of markers per couple: 1
- Allow multi-class markers : Yes
- Use KNN classification: No - Proprietary voting method was used.

A list of 50 probe sets was obtained. 9 Probe sets were removed due the Absence status in all samples. The intensities of the 41 probe sets for the 22 patients listed above are listed in Tables 11A and 11B.

### Prediction of the clinical status

The clinical status of the 67 patients was predicted using the hierarchical clustering approach of Spotfire software.

Data were normalized regarding the genes using a Z-score calculation available in Spotfire before performing the Hierarchical clustering.

Calculation options of the hierarchical clustering were:
- Clustering method: complete linkage
- Similarity measure: Euclidean distance
- Ordering function: average value

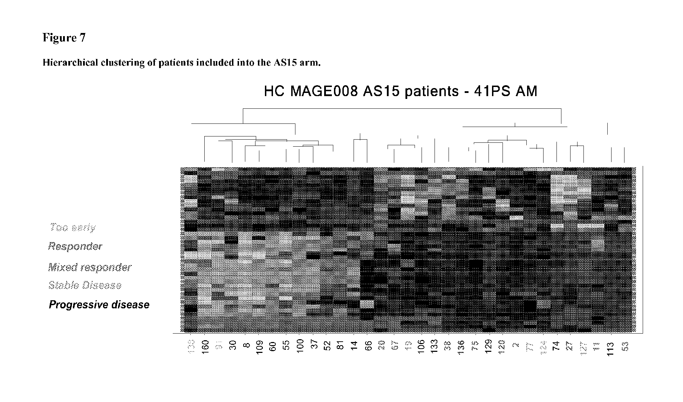

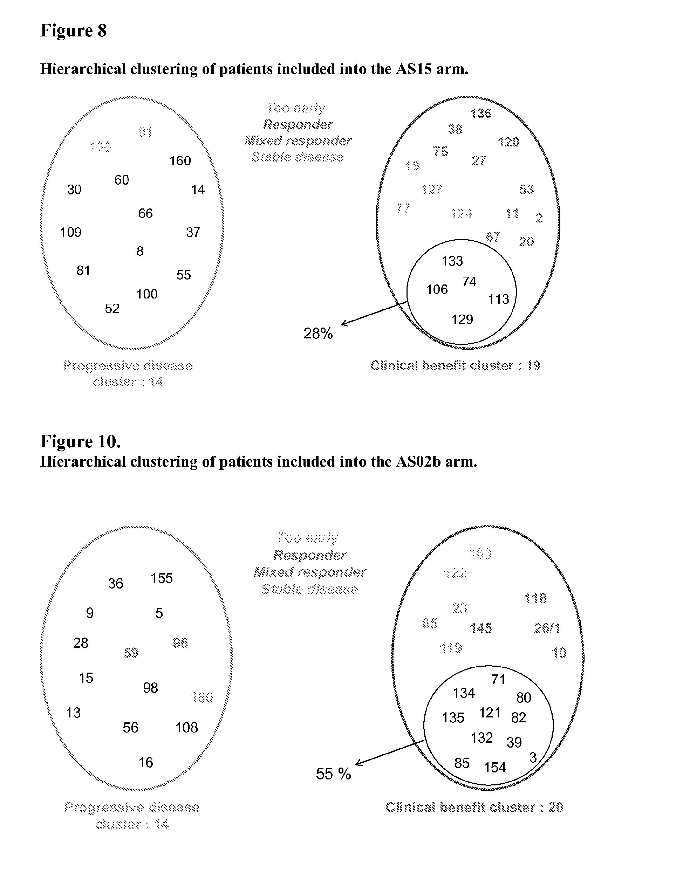

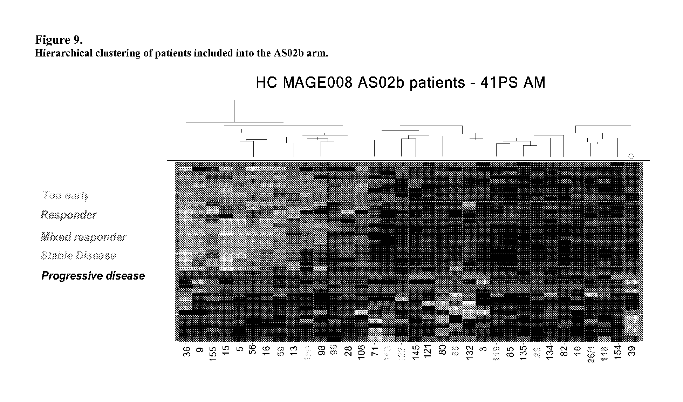

This clustering was performed for patients included into the AS15 (Figure 7 and 8) or the AS02B (Figure 9 and 10) arms.

Using the hierarchical clustering approach, about 58% of patients have the responder signature whatever the adjuvant group.

100% and 87.5% of patients with a clinical benefit are well clustered respectively in AS15 and in AS02b groups. 71 % and 52% of patients with a progressive disease are well clustered respectively in AS15 and in AS02b groups.

In the clinical benefit cluster, 28% and 55% of patients have a progressive disease respectively in AS15 and in AS02b groups. The percentage of patients with a responder gene profile according to the invention who achieved a clinical benefit to the MAGE-A3 vaccination when the AS15 adjuvant was used was larger than the percentage who achieved a clinical benefit to the MAGE-A3 vaccination when AS02b adjuvant was employed in the formulation.

### Example 5

### Inducing a responder's profile.

Initial biopsy (sampled before irradiation) of patient 59 did not have the responder signature while his second biopsy (sampled after irradiation) had the responder signature (data not shown) suggesting that irradiation of lesions may induce the responder signature.
1. R Development Core Team (2006). R: A language and environment for statistical computing. R Foundation for Statistical Computing, Vienna, Austria. ISBN 3-900051-07-0, URL http://www.R-project.org)
2. Jean (ZHIJIN) Wu and Rafael Irizarry with contributions from James MacDonald Jeff Gentry (2005). gcrma: Background Adjustment Using Sequence Information. R package version 2.4.1.
3. Wu Z, Irizarry RA, Gentleman R, Martinez-Murillo F, Spencer F: A model-based background adjustment for oligonucleotide expression arrays. Journal of the American Statistical Association 2004, 99:909-917.
4. Peter Warren (2005). panp: Presence-Absence Calls from Negative Strand Matching Probesets. R package version 1.2.0.
5. R. Gentleman, V. Carey and W. Huber (2006). genefilter: genefilter: filter genes. R package version 1.10.1.
6. Jain N, Thatte J, Braciale T, Ley K, O'Connell M, Lee JK. Local-pooled-error test for identifying differentially expressed genes with a small number of replicated microarrays. Bioinformatics. 2003 Oct 12;19(15):1945-51.
7. Nitin Jain, Michael O'Connell and Jae K. Lee. Includes R source code contributed by HyungJun Cho <hcho@virginia.edu> (2006). LPE: Methods for analyzing microarray data using Local Pooled Error (LPE) method. R package version 1.6.0. http://www.r-project.org.
8. Smyth, G. K. Linear models and empirical Bayes methods for assessing differential expression in microarray experiments. Statistical Applications in Genetics and Molecular Biology (2004) 3, No. 1, Article 3.
9. Smyth, G. K. (2005). Limma: linear models for microarray data. In: 'Bioinformatics and Computational Biology Solutions using R and Bioconductor'. R. Gentleman, V. Carey, S. Dudoit, R. Irizarry, W. Huber (eds), Springer, New York, pages 397--420.
10. Baldi P, Long AD. A Bayesian framework for the analysis of microarray expression data: regularized t -test and statistical inferences of gene changes. Bioinformatics. 2001 Jun;17(6):509-19.
11. http://visitor.ics.uci.edu/genex/cybert/
12. Benjamini,Y. and Hochberg,Y. (1995) Controlling the false discovery rate: a practical and powerful approach to multiple testing. J. Roy. Stat. Soc. B., 57, 289-300.
13. Katherine S. Pollard, Yongchao Ge and Sandrine Dudoit. multtest: Resampling-based multiple hypothesis testing. R package version 1.10.2.
14. Jess Mar, Robert Gentleman and Vince Carey. MLInterfaces: Uniform interfaces to R machine learning procedures for data in Bioconductor containers. R package version 1.4.0.
15. Soukup M, Cho H, and Lee JK (2005). Robust classification modeling on microarray data using misclassification penalized posterior, Bioinformatics, 21 (Suppl): i423-i430.
16. Soukup M and Lee JK (2004). Developing optimal prediction models for cancer classification using gene expression data, Journal of Bioinformatics and Computational Biology, 1(4) 681-694.

**Table 1A Gene sequences for and Seq ID No.s for the genes of Table 1**

| **Seq ID No.** | **Gene name** | **Full gene sequence** |
|---|---|---|
| **1** | chromosome 6 open reading frame 190 | >gi\|58218985\|ref\|MM_001010923.1\| Homo sapiens chromosome 6 open reading frame 190 (C6orf190), mRNA |
| | | ATACTTACAATTACGAGATTTATATTTGCATTAGTCTCTTTGGCTGGTGGGTAGGGGTGAGAGGCTCTTC CTGGATCCCTTATTTTCTACAGGAGAGGAGGAAAACACCTGGGATGCTCCAGTGCTCTTACGCAGATAAT GATCATTAACATCAGCCTCTCTGATCAAAGAGCTACTCCACCCCACTCTGGCTGTAGTGTGACATTCCTG CCTGCCTGAGGAAGAAATGGTGAGCAGGGGCTGCAATTGCAGACAAGTGTCACCCAGAAGCCACAAGTTT CTGTGAGCACCAGGTCTACAAACTACCCAAGGCATAGCAATGGCATTATCACTGGAAGAATTCGTCCACT CCCTTGACCTCAGGACCCTACCCAGGGTTCTAGAAATCCAGGCAGGCATCTATCTTGAAGGCTCTATTTA TGAAATGTTTGGAAATGAATGCTGTTTTTCAACAGGAGAAGTGATTAAAATTACTGGTCTCAAAGTTAAG AAGATCATAGCTGAAATTTGTGAGCAGATTGAAGGTTGTGAGTCTCTACAGCCATTTGAACTGCCTATGA ATTTTCCAGGTCTTTTTAAGATTGTGGCTGATAAAACTCCATACCTTACTATGGAAGAAATCACAAGGAC CATTCATATTGGACCAAGTAGACTAGGGCATCCTTGCTTCTATCATCAGAAGGATATAAAACTAGAGAAC CTCATCATAAAGCAGGGTGAGCAAATCATGCTCAACTCAGTTGAAGAGATTGATGGAGAAATAATGGTGA GCTGTGCAGTAGCAAGGAATCATCAAACTCACTCATTTAATTTGCCTTTGTCACAAGAAGGAGAATTCTA CGAGTGTGAAGATGAACGTATTTACACTCTAAAGGAGATTGTTGAATGGAAGATTCCTAAGAACAGAACA AGAACTGTAAACCTTACAGATTTTTCAAATAAGTGGGACTCAACGAATCCATTTCCTAAAGACTTTTATG GTACCCTGATTCTCAAGCCTGTTTATGAAATTCAAGGTGTGATGAAATTTCGAAAAGATATAATCCGCAT CCTCCCCAGTCTAGATGTCGAAGTCAAAGACATCACTGATTCTTACGATGCTAACTGGTTTCTTCAGCTG TTATCAACAGAAGATCTTTTTGAAATGACTAGTAAAGAGTTCCCCATAGTGACTGAAGTCATAGAAGCAC CTGAAGGAAACCACCTGCCCCAAAGCATTTTACAGCCTGGGAAAACCATTGTGATCCACAAAAAGTACCA GGCATCAAGAATCTTAGCTTCAGAAATTAGAAGCAATTTTCCTAAAAGACACTTCTTGATCCCCACTAGC TATAAAGGCAAGTTCAAGCGGCGACCGAGGGAGTTCCCAACGGCCTATGACCTAGAGATCGCTAAGAGTG AAAAGGAGCCTCTTCACGTGGTGGCCACCAAAGCGTTTCATTCCCCTCATGACAAGCTGTCATCCGTATC TGTTGGGGACCAGTTTCTGGTGCATCAGTCAGAGACGACTGAAGTCCTCTGTGAGGGAATAAAAAAAGTG GTGAATGTTCTGGCCTGTGAAAAAATCCTCAAAAAGTCCTATGAGGCTGCGCTGCTCCCTTTGTACATGG AAGGAGGTTTTGTAGAGGTGATTCATGATAAGAAACAGTACCCGATTTCTGAGCTCTGTAAACAGTTCCG TTTGCCCTTCAATGTGAAGGTGTCTGTCAGGGATCTTTCCATTGAAGAGGACGTGTTGGCTGCCACACCA GGACTGCAGTTGGAGGAGGACATTACAGACTCTTACCTACTCATAAGTGACTTTGCCAACCCCACGGAGT GCTGGGAAATTCCTGTGGGCCGCTTGAATATGACTGTTCAGTTAGTTAGTAATTTCTCTAGGGATGCAGA |
| | | ACCATTTCTAGTCAGGACTCTGGTAGAAGAGATCACTGAAGAGCAATATTACATGATGCGGAGATATGAA AGCTCAGCCTCACATCCCCCACCTCGCCCTCCGAAACACCCCTCAGTAGAGGAAACAAAGTTAACCCTGC TAACCTTAGCAGAAGAAAGGACGGTAGACCTGCCCAAGTCTCCCAAGCGTCATCACGTAGACATAACCAA GAAACTTCACCCAAATCAAGCTGGCCTGGATTCAAAAGTACTGATTGGTAGTCAGAATGATTTGGTGGAT GAAGAGAAAGAAAGGAGCAACCGTGGGGCCACAGCAATAGCAGAAACATTCAAAAATGAAAAACATCAAA AATAACAAGATGTGACAGAAGCCACTTAGGCAGCAAACATAAATGTTGCAGTGAAAAAGAAGCTAGCCT TCTAGCTGAAAAACGAGTATTCCCCAATGGACTCCAGAAGAAACTTGATTCATCGCTGCAAAGGAAAGAA CAACCTTAAAACTTTTAACAGATAAAACTTACAGAAACCTATGATATAGAATTCATATAGTCTATTCTGT TGTGTCTAAATCTGTAGGCATTGTGTTGTTGTTCTTTAGGACGTATTTATTTAACTTGCACATTTTTTCA GATTCTTATTTCTACTACCAACAACTAAGTAATTGGGAAATAATTCTGTATTTCAGTTTCTGAGTAAAAC CAGTCTGAAATAGGATAAAAGCCACCAAATATTTTCTTTTTTTTCCAGAATTTGTTTTGCCATTTTTTAG TGCTATCATCATTCCTAACAAGACTAACTTACAGAAAAATAATTATATCTGACTGATTTAAAATGTTCAG GTTTCTTATCCAAATCCCTTGGAACTATGGAAAGGAGTTTGATTTCACATTCACAGTGTATTTACAAAAT ACGCTGTGTCATAAATATGTTTGAATTCCAACAGCCAAAGCCATTGAGAGTCATAGGAGTTTTCCATAAC CTTCTCTTCTATGACCCAACAACAAGCTCATGACTGAAATTTCACCAGATTTCTGAGACGATGTCTTAAT ATTCTATGTGCTATGTACCAGATAATTCTTTAGATGAATGTTTCTTAGGATTGTAGGAAAATTATCTAGT TAATCATAATATTTGATGGAAAGAAAAAGACAATAAAATTGTAATATAATAAATTTGGCTGACAAGAAAC CAAAGTGATTCTTAATTAGTATACATCAGAATGATGCTCTTATAGTTGTACCATCTATAAAAATTACTTT AAGGGCTCTCACATTTTAATAATTTATCTTATTATGTATTAAGTATACAGGAACAATATTATTTTTCCTT TAACAAAATGAAGAGACAGGCTATCTGGTTAATGTTACATAGGAATTTAATAGTAATGCTTGAACTTCAT CCATAGATCATACTCTGTACAAAATTTGTTAGCTAACATCCTATCTCATAATTATTTTATGTTTTGTGGA GAAATTTGTTGATTTTGTACCAAAGTGTTTCTGAAGACAATAAATTGTGAGTCAACTTTAGAACAAAAAA AATTAGAGTTTTTTCAATGTTTATATTCTGATTAAGCTTACTTTACCTTACATTTTTTCTAAGTAACAAT GAATCCTGATTTCTAGTGTCCTAAAAATTGCTTAGTGATTTGATTGTGGTAATATCATTTCTTATCTACA ATGTCTAAAGTTTTATGGACAGTTTTTCTTTTATTTATTTTGCCTGTTTGTGCAGATAAGAACAGAAAC TTTTCTAAGACCCACATTTGGTTATTGAAGGCCACAGCGAATCTTAACCTAACAGCCTTGACAAACTGCA CCATAGGTGTTTTTAGACTCATATAATTTGTTATTTTTCAAACAATAGTGAATAATTAATATTTTTGTTT GGAATTTGAGAACAATTAAATTTGTACTTTTAGTAACTACCATTCTTTGATTAGAAAATTAAGAGAATGC ATATCTTACTTTGGTTGTAAATTATCAAGGGCTTTCTAATAGAAATCATATATAACATTTCTAAATATAA GTCCTTTCACATACTGTGTTTCCAGTTGTCTTGATATTGAAAAGTGTAATAAACTTCATGCTCACCTATT GGAGATTTGGGAAGGTTGAAAATAAACTTCCTAATTTTTAAAAAAAAAA |
| **2** | hematopoietic cell-specific Lyn substrate 1 | >gi\|37059786\|ref\|NM_005335.3\| Homo sapiens hematopoietic cell-specific Lyn substrate 1 (HCLS1), mRNA |
| | | GTGGACGCGAGGAGCCGGGCGCTTAGAACAGAGGCTTGCACAGGTGGAGATGTGGAAGTCTGTAGTGGGC CATGATGTGTCTGTTTCCGTGGAGACCCAGGGTGATGATTGGACACAGATCCTGACTTTGTGAATGACA TCTCTGAAAAGGAGCAACGATGGGGAGCCAAGACCATCGAGGGGTCTGGACGCACAGAACACATCAACAT CCACCAGCTGAGGAACAAAGTATCAGAGGAGCATGATGTTCTCAGGAAGAAAGAGATGGAGTCAGGGCCC AAAGCATCCCATGGCTATGGAGGTCGGTTTGGAGTAGAAAGAGACCGAATGGACAAGAGTGCAGTGGGCC ATGAGTATGTTGCCGAGGTGGAGAAGCACTCTTCTCAGACGGATGCTGCCAAAGGCTTTGGGGGCAAGTA CGGAGTTGAGAGGGACAGGGCAGACAAGTCAGCAGTCGGCTTTGATTATAAAGGAGAAGTGGAGAAGCAT ACATCTCAGAAAGATTACTCTCGTGGCTTTGGTGGCCGGTACGGGGTGGAGAAGGATAAATGGGACAAAG CAGCTCTGGGATATGACTACAAGGGAGAGACGGAGAAACACGAGTCCCAGAGAGATTATGCCAAGGGCTT TGGTGGCCAGTATGGAATCCAGAAGGACCGAGTGGATAAGAGCGCTGTCGGCTTCAATGAAATGGAGGCC CCGACCACAGCTTATAAGAAGACGACGCCCATAGAAGCCGCTTCTAGTGGTGCCCGTGGGCTGAAGGCGA AATTTGAGTCCATGGCTGAGGAGAAGAGGAAGCGAGAGGAAGAGGAGAAGGCACAGCAGGTGGCCAGGAG GCAACAGGAGCGAAAGGCTGTGACAAAGAGGAGCCCTGAGGCTCCACAGCCAGTGATAGCTATGGAAGAG CCAGCAGTACCGGCCCCACTGCCCAAGAAAATCTCCTCAGAGGCCTGGCCTCCAGTTGGGACTCCTCCAT CATCAGAGTCTGAGCCTGTGAGAACCAGCAGGGAACACCCAGTGCCCTTGCTGCCCATTAGGCAGACTCT CCCGGAGGACAATGAGGAGCCCCCAGCTCTGCCCCCTAGGACTCTGGAAGGCCTCCAGGTGGAGGAAGAG CCAGTGTACGAAGCAGAGCCTGAGCCTGAGCCCGAGCCTGAGCCCGAGCCTGAGAATGACTATGAGGACG TTGAGGAGATGGACAGGCATGAGCAGGAGGATGAACCAGAGGGGGACTATGAGGAGGTGCTCGAGCCTGA AGATTCTTCTTTTTCTTCTGCTCTGGCTGGATCATCAGGCTGCCCGGCTGGGGCTGGGGCTGGGGCTGTG GCTCTGGGGATCTCAGCTGTGGCTCTATATGATTACCAAGGAGAGGGAAGTGATGAGCTTTCCTTTGATC CGGACGACGTAATCACTGACATTGAGATGGTGGACGAGGGCTGGTGGCGGGGACGTTGCCATGGCCACTT TGGACTCTTCCCTGCAAATTATGTCAAGCTTCTGGAGTGACTAGAGCTCACTGTCTACTGCAACTGTGAT TTCCCATGTCCAAAGTGGCTCTGCCTCCACCCCCTCCCTATTCCTGATGCAAATGTCTAACCAGATGAGT TTCTGGACAGACTTCCCTCTCCTGCTTCATTAAGGGCTTGGGGCAGAGACAGCATGGGGAAGGAGGTCCC CTTCCCCAAGAGTCCTCTCTATCCTGGATGAGCTCATGAACATTTCTCTTGTGTTCCTGACTCCTTCCCA ATGAACACCTCTCTGCCACCCCAAGCTCTGCTCTCCTCCTCTGTGAGCTCTGGGCTTCCCAGTTTGTTTA CCCGGGAAAGTACGTCTAGATTGTGTGGTTTGCCTCATTGTGCTATTTGCCCACTTTCCTTCCCTGAAGA AATATCTGTGAACCTTCTTTCTGTTCAGTCCTAAAATTCGAAATAAAGTGAGACTATGGTTCACCTGTAA AAAAAAAAAAAAAAAAAAAAAAAAAAAAAAAAAAAAAAAAAAAAAAAAAAAAAAAAAAAAAAAAAAAA |
| **3** | interleukin 2 receptor, gamma (severe combined immunodeficiency) | >gi\|4557881\|ref\|NM_000206.1\| Homo sapiens interleukin 2 receptor, gamma (severe combined immunodeficiency) (IL2RG), mRNA |
| | | GAAGAGCAAGCGCCATGTTGAAGCCATCATTACCATTCACATCCCTCTTATTCCTGCAGCTGCCCCTGCT GGGAGTGGGGCTGAACACGACAATTCTGACGCCCAATGGGAATGAAGACACCACAGCTGATTTCTTCCTG ACCACTATGCCCACTGACTCCCTCAGTGTTTCCACTCTGCCCCTCCCAGAGGTTCAGTGTTTTGTGTTCA |
| | | ATGTCGAGTACATGAATTGCACTTGGAACAGCAGCTCTGAGCCCCAGCCTACCAACCTCACTCTGCATTA TTGGTACAAGAACTCGGATAATGATAAAGTCCAGAAGTGCAGCCACTATCTATTCTCTGAAGAAATCACT TCTGGCTGTCAGTTGCAAAAAAAGGAGATCCACCTCTACCAAACATTTGTTGTTCAGCTCCAGGACCCAC GGGAACCCAGGAGACAGGCCACACAGATGCTAAAACTGCAGAATCTGGTGATCCCCTGGGCTCCAGAGAA CCTAACACTTCACAAACTGAGTGAATCCCAGCTAGAACTGAACTGGAACAACAGATTCTTGAACCACTGT TTGGAGCACTTGGTGCAGTACCGGACTGACTGGGACCACAGCTGGACTGAACAATCAGTGGATTATAGAC ATAAGTTCTCCTTGCCTAGTGTGGATGGGCAGAAACGCTACACGTTTCGTGTTCGGAGCCGCTTTAACCC ACTCTGTGGAAGTGCTCAGCATTGGAGTGAATGGAGCCACCCAATCCACTGGGGGAGCAATACTTCAAAA GAGAATCCTTTCCTGTTTGCATTGGAAGCCGTGGTTATCTCTGTTGGCTCCATGGGATTGATTATCAGCC TTCTCTGTGTGTATTTCTGGCTGGAACGGACGATGCCCCGAATTCCCACCCTGAAGAACCTAGAGGATCT TGTTACTGAATACCACGGGAACTTTTCGGCCTGGAGTGGTGTGTCTAAGGGACTGGCTGAGAGTCTGCAG CCAGACTACAGTGAACGACTCTGCCTCGTCAGTGAGATTCCCCCAAAAGGAGGGGCCCTTGGGGAGGGGC CTGGGGCCTCCCCATGCAACCAGCATAGCCCCTACTGGGCCCCCCCATGTTACACCCTAAAGCCTGAAAC CTGAACCCCAATCCTCTGACAGAAGAACCCCAGGGTCCTGTAGCCCTAAGTGGTACTAACTTTCCTTCAT TCAACCCACCTGCGTCTCATACTCACCTCACCCCACTGTGGCTGATTTGGAATTTTGTGCCCCCATGTAA GCACCCCTTCATTTGGCATTCCCCACTTGAGAATTACCCTTTTGCCCCGAACATGTTTTTCTTCTCCCTC AGTCTGGCCCTTCCTTTTCGCAGGATTCTTCCTCCCTCCCTCTTTCCCTCCCTTCCTCTTTCCATCTACC CTCCGATTGTTCCTGAACCGATGAGAAATAAAGTTTCTGTTGATAATCATC |
| **4** | CD52 antigen (CAMPATH-1 antigen) /// CD52 antigen (CAMPATH-1 antigen) | >gi\|68342029\|ref\|NM_001803.2\| Homo sapiens CD52 molecule (CD52), mRNA |
| | | CTCCTGGTTCAAAAGCAGCTAAACCAAAAGAAGCCTCCAGACAGCCCTGAGATCACCTAAAAAGCTGCTA CCAAGACAGCCACGAAGATCCTACCAAAATGAAGCGCTTCCTCTTCCTCCTACTCACCATCAGCCTCCTG GTTATGGTACAGATACAAACTGGACTCTCAGGACAAAACGACACCAGCCAAACCAGCAGCCCCTCAGCAT CCAGCAACATAAGCGGAGGCATTTTCCTTTTCTTCGTGGCCAATGCCATAATCCACCTCTTCTGCTTCAG TTGAGGTGACACGTCTCAGCCTTAGCCCTGTGCCCCCTGAAACAGCTGCCACCATCACTCGCAAGAGAAT CCCCTCCATCTTTGGGAGGGGTTGATGCCAGACATCACCAGGTTGTAGAAGTTGACAGGCAGTGCCATGG GGGCAACAGCCAAAATAGGGGGGTAATGATGTAGGGGCCAAGCAGTGCCCAGCTGGGGGTCAATAAAGTT ACCCTTGTACTTGCAAAAAAAAAAAAAAAAAAA |
| **5** | CD2 antigen (p50), sheep red blood cell receptor /// | >gi\|31542293\|ref\|NM_001767.2\| Homo sapiens CD2 molecule (CD2), mRNA |
| | | ACCAACCCCTAAGATGAGCTTTCCATGTAAATTTGTAGCCAGCTTCCTTCTGATTTTCAATGTTTCTTCC AAAGGTGCAGTCTCCAAAGAGATTACGAATGCCTTGGAAACCTGGGGTGCCTTGGGTCAGGACATCAACT TGGACATTCCTAGTTTTCAAATGAGTGATGATATTGACGATATAAAATGGGAAAAAACTTCAGACAAGAA |
| | CD2 antigen (p50), sheep red blood cell receptor | AAAGATTGCACAATTCAGAAAAGAGAAAGAGACTTTCAAGGAAAAAGATACATATAAGCTATTTAAAAAT GGAACTCTGAAAATTAAGCATCTGAAGACCGATGATCAGGATATCTACAAGGTATCAATATATGATACAA AAGGAAAAAATGTGTTGGAAAAAATATTTGATTTGAAGATTCAAGAGAGGGTCTCAAAACCAAAGATCTC CTGGACTTGTATCAACACAACCCTGACCTGTGAGGTAATGAATGGAACTGACCCCGAATTAAACCTGTAT CAAGATGGGAAACATCTAAAACTTTCTCAGAGGGTCATCACACACAAGTGGACCACCAGCCTGAGTGCAA AATTCAAGTGCACAGCAGGGAACAAAGTCAGCAAGGAATCCAGTGTCGAGCCTGTCAGCTGTCCAGAGAA AGGTCTGGACATCTATCTCATCATTGGCATATGTGGAGGAGGCAGCCTCTTGATGGTCTTTGTGGCACTG CTCGTTTTCTATATCACCAAAAGGAAAAAACAGAGGAGTCGGAGAAATGATGAGGAGCTGGAGACAAGAG CCCACAGAGTAGCTACTGAAGAAAGGGGCCGGAAGCCCCAACAAATTCCAGCTTCAACCCCTCAGAATCC AGCAACTTCCCAACATCCTCCTCCACCACCTGGTCATCGTTCCCAGGCACCTAGTCATCGTCCCCCGCCT CCTGGACACCGTGTTCAGCACCAGCCTCAGAAGAGGCCTCCTGCTCCGTCGGGCACACAAGTTCACCAGC AGAAAGGCCCGCCCCTCCCCAGACCTCGAGTTCAGCCAAAACCTCCCCATGGGGCAGCAGAAAACTCATT GTCCCCTTCCTCTAATTAAAAAAGATAGAAACTGTCTTTTTCAATAAAAAGCACTGTGGATTTCTGCCCT CCTGATGTGCATATCCGTACTTCCATGAGGTGTTTTCTGTGTGCAGAACATTGTCACCTCCTGAGGCTGT GGGCCACAGCCACCTCTGCATCTTCGAACTCAGCCATGTGGTCAACATCTGGAGTTTTTGGTCTCCTCAG AGAGCTCCATCACACCAGTAAGGAGAAGCAATATAAGTGTGATTGCAAGAATGGTAGAGGACCGAGCACA GAAATCTTAGAGATTTCTTGTCCCCTCTCAGGTCATGTGTAGATGCGATAAATCAAGTGATTGGTGTGCC TGGGTCTCACTACAAGCAGCCTATCTGCTTAAGAGACTCTGGAGTTTCTTATGTGCCCTGGTGGACACTT GCCCACCATCCTGTGAGTAAAAGTGAAATAAAAGCTTTGACTAGAAAAAAAAAAAAAAAAAAAAAAAAAA AAAAAAAAAAAAAAAAAAAAAAAAAAAAAAAAAAAAAA |
| **6** | ubiquitin D | >gi\|50355987\|ref\|NM_006398.2\| Homo sapiens ubiquitin D (UBD), mRNA |
| | | GATTGCTTGAGGAGAGAAGTATGTGATCAGAAAGCATTCTTTGTCTATTAACTCCTGCCCAGCAAAAGTG AAAGAAAATTCATGGGAGCATGCAAGAACAAAGAGCACAGCAAAGCTGGACAAACACAGCAATCCAGGCA GGGGATTTCCAACTCAACTCTGGTATGTAAGCTGCATGCAAAGTCCTTTTTCTGTCTCTGGTTTCTGGCC CCTTGTCTGCAGAGATGGCTCCCAATGCTTCCTGCCTCTGTGTGCATGTCCGTTCCGAGGAATGGGATTT AATGACCTTTGATGCCAACCCATATGACAGCGTGAAAAAAATCAAAGAACATGTCCGGTCTAAGACCAAG GTTCCTGTGCAGGACCAGGTTCTTTTGCTGGGCTCCAAGATCTTAAAGCCACGGAGAAGCCTCTCATCTT ACGGCATTGACAAAGAGAAGACCATCCACCTTACCCTGAAAGTGGTGAAGCCCAGTGATGAGGAGCTGCC CTTGTTTCTTGTGGAGTCAGGTGATGAGGCAAAGAGGCACCTCCTCCAGGTGCGAAGGTCCAGCTCAGTG GCACAAGTGAAAGCAATGATCGAGACTAAGACGGGTATAATCCCTGAGACCCAGATTGTGACTTGCAATG GAAAGAGACTGGAAGATGGGAAGATGATGGCAGATTACGGCATCAGAAAGGGCAACTTACTCTTCCTGGC ATCTTATTGTATTGGAGGGTGACCACCCTGGGCATGGGGTGTTGGCAGGGGTCAAAAAGCTTATTTCTTT TAATCTCTTACTCAACGAACACATCTTCTGATGATTTCCCAAAATTAATGAGAATGAGATGAGTAGAGTA AGATTTGGGTGGGATGGGTAGGATGAAGTATATTGCCCAACTCTATGTTTCTTTGATTCTAACACAATTA |
| | | ATTAAGTGACATGATTTTTACTAATGTATTACTGAGACTAGTAAATAAATTTTTAAGCCAA |
| **7** | signal transducer and activator of transcription 4 | >gi\|21618332\|ref\|NM_003151.2\| Homo sapiens signal transducer and activator of transcription 4 (STAT4), mRNA |
| | | AGAGGACGCCCGGTGAAGGGGCTCCAGCCTGGCAGTTTCTGCGTGTTAGCATTTCTAGAATAGAGTGGGT GGGAACTGACCCAAGTAAAGTCCCAGAGACTCGAACACTGACGCACAGGAAAGCCTCAAGTGGGAGGAGA AATGCAAATCCCCTACTGATGATGGCGTCAGCGGCTTTCTCCTAGGGACTGTGAGGGGCGCTTCTGACTT TGGACTTGAGCACTGCCTGGGACCTGTGCTGAGAGAGCGCTAGCATGTCTCAGTGGAATCAAGTCCAACA GTTAGAAATCAAGTTTTTGGAGCAGGTGGATCAATTCTATGATGACAACTTTCCCATGGAAATTCGGCAT CTGTTGGCCCAATGGATTGAAAATCAAGACTGGGAGGCAGCTTCTAACAATGAAACCATGGCAACGATTC TTCTTCAAAACTTGTTAATACAACTGGATGAACAGTTAGGTCGTGTTTCCAAAGAGAAAAACCTACTCTT GATACACAATCTAAAAAGAATTAGGAAGGTCCTTCAGGGAAAATTTCATGGAAATCCAATGCATGTAGCT GTGGTTATTTCAAACTGTTTAAGGAAGAGAGGAGAATATTGGCTGCAGCCAACATGCCTGTCCAGGGGC CTCTAGAGAAATCCTTACAAAGTTCTTCAGTTTCAGAAAGACAGAGGAATGTGGAGCACAAAGTGGCTGC CATTAAAAACAGTGTGCAGATGACAGAACAAGATACCAAATACTTAGAAGATCTGCAAGACGAATTTGAC TACAGGTATAAAACAATTCAGACAATGGATCAGAGTGACAAGAATAGTGCCATGGTGAATCAGGAAGTTT TGACACTGCAGGAAATGCTTAACAGCCTCGATTTCAAGAGAAAGGAGGCTCTCAGTAAAATGACCCAAAT CATCCATGAGACAGACCTGTTAATGAACACCATGCTCATAGAAGAGCTGCAAGACTGGAAGCGGCGGCAG CAAATCGCCTGCATCGGGGGTCCACTCCACAATGGGCTCGACCAGCTTCAGAACTGCTTTACACTATTGG CAGAAAGTCTTTTCCAACTGAGAAGGCAATTGGAGAAACTAGAGGAGCAATCTACCAAAATGACATATGA AGGTGATCCCATTCCAATGCAAAGAACTCACATGCTAGAAAGAGTCACCTTCTTGATCTACAACCTTTTC AAGAACTCATTTGTGGTTGAGCGACAGCCATGTATGCCAACCCACCCTCAGAGGCCGTTGGTACTTAAAA CCCTAATTCAGTTCACTGTAAAACTAAGGCTACTAATAAAATTGCCAGAACTAAACTATCAGGTAAAGGT TAAGGCATCAATTGACAAGAATGTTTCAACTCTAAGCAACCGAAGATTTGTACTTTGTGGAACTAATGTC AAAGCCATGTCTATTGAAGAATCTTCCAATGGGAGTCTCTCAGTAGAATTTCGACATTTGCAACCAAAGG AAATGAAGTCCAGTGCTGGAGGTAAAGGAAATGAGGGCTGTCACATGGTGACTGAAGAACTTCATTCCAT AACGTTTGAAACACAGATCTGCCTCTATGGCCTGACCATAGATTTGGAGACCAGCTCATTGCCTGTGGTG ATGATTTCCAATGTCAGTCAGTTACCTAATGCTTGGGCATCCATCATTTGGTACAACGTGTCAACCAACG ATTCCCAGAACTTGGTTTTCTTTAATAATCCTCCACCTGCCACATTGAGTCAACTACTGGAGGTGATGAG CTGGCAGTTTTCATCGTACGTTGGTCGTGGTCTTAACTCAGATCAACTCCATATGCTGGCAGAGAAGCTT ACAGTCCAATCTAGCTACAGTGATGGTCACCTCACCTGGGCCAAGTTCTGCAAGGAACATTTACCTGGTA AATCATTTACCTTTTGGACATGGCTTGAAGCAATATTGGATCTAATTAAGAAACACATTCTTCCCCTTTG GATTGATGGGTATGTCATGGGCTTTGTTAGCAAAGAGAAGGAACGGCTGTTGCTAAAGGATAAAATGCCT GGCACCTTTTTATTAAGATTCAGTGAAAGCCATCTCGGAGGAATAACTTTCACCTGGGTGGACCATTCTG AAAGTGGGGAAGTGAGATTCCACTCTGTAGAACCCTACAATAAAGGCCGGTTGTCTGCTCTGCCATTCGC |
| | | TGACATCCTGCGAGACTACAAAGTTATTATGGCTGAAAACATTCCTGAAAACCCTCTGAAGTACCTATAT CCTGACATTCCCAAAGACAAAGCCTTCGGTAAACACTACAGCTCTCAGCCTTGCGAAGTTTCAAGACCAA CAGAAAGGGGTGACAAAGGTTATGTTCCTTCTGTTTTTATCCCCATCTCAACAATCCGAAGTGATTCAAC AGAGCCACATTCTCCATCAGACCTTCTTCCCATGTCTCCAAGTGTGTATGCGGTGTTGAGAGAAAACCTG AGTCCCACAACAATTGAAACTGCAATGAAGTCTCCTTATTCTGCTGAATGACAGGATAAACTCTGACGCA CCAAGAAAGGAAGCAAATGAAAAAGTTTAAAGACTGTTCTTTGCCCAATAACCACATTTTATTTCTTCAG CTTTGTAAATACCAGGTTCTAGGAAATGTTTGACATCTGAAGCTCTCTTCACACTCCCGTGGCACTCCTC AATTGGGAGTGTTGTGACTGAAATGCTTGAAACCAAAGCTTCAGATAAACTTGCAAGATAAGACAACTTT AAGAAACCAGTGTTAATAACAATATTAACAG |
| **8** | granzyme K (granzyme 3; tryptase II) /// granzyme K (granzyme 3; tryptase II) | >gi\|73747815\|ref\|NM_002104.2\| Homo sapiens granzyme K (granzyme 3; tryptase II) (GZMK), mRNA |
| | | GATCAACACATTTCATCTGGGCTTCTTAAATCTAAATCTTTAAAATGACTAAGTTTTCTTCCTTTTCTCT GTTTTTCCTAATAGTTGGGGCTTATATGACTCATGTGTGTTTCAATATGGAAATTATTGGAGGGAAAGAA GTGTCACCTCATTCCAGGCCATTTATGGCCTCCATCCAGTATGGCGGACATCACGTTTGTGGAGGTGTTC TGATTGATCCACAGTGGGTGCTGACAGCAGCCCACTGCCAATATCGGTTTACCAAAGGCCAGTCTCCCAC TGTGGTTTTAGGCGCACACTCTCTCTCAAAGAATGAGGCCTCCAAACAAACACTGGAGATCAAAAAATTT ATACCATTCTCAAGAGTTACATCAGATCCTCAATCAAATGATATCATGCTGGTTAAGCTTCAAACAGCCG CAAAACTCAATAAACATGTCAAGATGCTCCACATAAGATCCAAAACCTCTCTTAGATCTGGAACCAAATG CAAGGTTACTGGCTGGGGAGCCACCGATCCAGATTCATTAAGACCTTCTGACACCCTGCGAGAAGTCACT GTTACTGTCCTAAGTCGAAAACTTTGCAACAGCCAAAGTTACTACAACGGCGACCCTTTTATCACCAAAG ACATGGTCTGTGCAGGAGATGCCAAAGGCCAGAAGGATTCCTGTAAGGGTGACTCAGGGGGCCCCTTGAT CTGTAAAGGTGTCTTCCACGCTATAGTCTCTGGAGGTCATGAATGTGGTGTTGCCACAAAGCCTGGAATC TACACCCTGTTAACCAAGAAATACCAGACTTGGATCAAAAGCAACCTTGTCCCGCCTCATACAAATTAAG TTACAAATAATTTTATTGGATGCACTTGCTTCTTTTTTCCTAATATGCTCGCAGGTTAGAGTTGGGTGTA AGTAAAGCAGAGCACATATGGGGTCCATTTTTGCACTTGTAAGTCATTTTATTAAGGAATCAAGTTCTTT TTCACTTGTATCACTGATGTATTTCTACCATGCTGGTTTTATTCTAAATAAAATTTAGAAGACTCAAAAA AAAAAAAAAAAAAAAAAAAAA |
| **9** | CD3G antigen, gamma polypeptide (TiT3 complex) | >gi\|4557428\|ref\|NM_000073.1\| Homo sapiens CD3g molecule, gamma (CD3-TCR complex) (CD3G), mRNA |
| | | GGGCTGCTCCACGCTTTTGCCGGAGACAGAGACTGACATGGAACAGGGGAAGGGCCTGGCTGTCCTCATC CTGGCTATCATTCTTCTTCAAGGTACTTTGGCCCAGTCAATCAAAGGAAACCACTTGGTTAAGGTGTATG |
| | | ACTATCAAGAAGATGGTTCGGTACTTCTGACTTGTGATGCAGAAGCCAAAAATATCACATGGTTTAAAGA TGGGAAGATGATCGGCTTCCTAACTGAAGATAAAAAAAAATGGAATCTGGGAAGTAATGCCAAGGACCCT CGAGGGATGTATCAGTGTAAAGGATCACAGAACAAGTCAAAACCACTCCAAGTGTATTACAGAATGTGTC AGAACTGCATTGAACTAAATGCAGCCACCATATCTGGCTTTCTCTTTGCTGAAATCGTCAGCATTTTCGT CCTTGCTGTTGGGGTCTACTTCATTGCTGGACAGGATGGAGTTCGCCAGTCGAGAGCTTCAGACAAGCAG ACTCTGTTGCCCAATGACCAGCTCTACCAGCCCCTCAAGGATCGAGAAGATGACCAGTACAGCCACCTTC AAGGAAACCAGTTGAGGAGGAATTGAACTCAGGACTCAGAGTAGTCCAGGTGTTCTCCTCCTATTCAGTT CCCAGAATCAAAGCAATGCATTTTGGAAAGCTCCTAGCAGAGAGACTTTCAGCCCTAAATCTAGACTCAA GGTTCCCAGAGATGACAAATGGAGAAGAAAGGCCATCAGAGCAAATTTGGGGGTTTCTCAAATAAAATAA AAATAAAAACAAATACTGTGTTTCAGAAGCGCCACCTATTGGGGAAAATTGT |
| **10** | G protein-coupled receptor 171 | >gi\|31377771\|ref\|NM_013308.2\| Homo sapiens G protein-coupled receptor 171 (GPR171), mRNA |
| | | GGTTGCTACTGCTGCGGCTAACCAAACAGCTCATGCTTCTCTGAAGACTTGCAGCAAGGTTTGCTGAGGC TCACAGAAGATAGCCCCAGTGTTTTGGAGTGGTTTTGAATGTGATTCTGAGATCAGACTGACTGAGCTGG AATCCTGGCTTTATATCTTACCAGCTACACAACCTTGGAGTCTTAGAAATTTTTTCTTTTCAATAAGCAG TCATCCTTACTTTCCCTCAAGATGACAAACAGTTCGTTCTTCTGCCCAGTTTATAAAGATCTGGAGCCAT TCACGTATTTTTTTTATTTAGTTTTCCTTGTTGGAATTATTGGAAGTTGTTTTGCAACCTGGGCTTTTAT ACAGAAGAATACGAATCACAGGTGTGTGAGCATCTACTTAATTAATTTGCTTACAGCCGATTTCCTGCTT ACTCTGGCATTACCAGTGAAAATTGTTGTTGACTTGGGTGTGGCACCTTGGAAGCTGAAGATATTCCACT GCCAAGTAACAGCCTGCCTCATCTATATCAATATGTATTTATCAATTATCTTCTTAGCATTTGTCAGCAT TGACCGCTGTCTTCAGCTGACACACAGCTGCAAGATCTACCGAATACAAGAACCCGGATTTGCCAAAATG ATATCAACCGTTGTGTGGCTAATGGTCCTTCTTATAATGGTGCCAAATATGATGATTCCCATCAAAGACA TCAAGGAAAAGTCAAATGTGGGTTGTATGGAGTTTAAAAAGGAATTTGGAAGAAATTGGCATTTGCTGAC AAATTTCATATGTGTAGCAATATTTTTAAATTTCTCAGCCATCATTTTAATATCCAATTGCCTTGTAATT CGACAGCTCTACAGAAACAAAGATAATGAAAATTACCCAAATGTGAAAAAGGCTCTCATCAACATACTTT TAGTGACCACGGGCTACATCATATGCTTTGTTCCTTACCACATTGTCCGAATCCCGTATACCCTCAGCCA GACAGAAGTCATAACTGATTGCTCAACCAGGATTTCACTCTTCAAAGCCAAAGAGGCTACACTGCTCCTG GCTGTGTCGAACCTGTGCTTTGATCCTGTCCTGTACTATCACCTCTCAAAAGCATTCCGCTCAAAGGTCA CTGAGACTTTTGCCTCACCTAAAGAGACCAAGGCTCAGAAAGAAAAATTAAGATGTGAAAATAATGCATA AAAGACAGGATTTTTGTGCTACCAATTCTGGCCTTACTGGACCATAAAGTTAATTATAGCTTTGAAAGAT AAAAAAAAAAAAAAAAACAAAAAAAAAACTCAGTATGAAAAAATACAGTTAGCTAGCAAATATGGACAGGT TTACTTAGAAATCCTGTTTCTAAATGCAAGTCAAGCTTTATTGTTAGGCTTGCTGCTACTCATTAACCCA AATATTTGTACAAAAAACTAAAGAGTCTCATTGAACGAATGTAAAATCCTGCAATATCCTTGAAATCCAA |
| | | AAGAGGTCCATGACATAGACCCAAAGGTATTCATGAGTTATTCATTTAAATGCCTGGAACTGACTTCTTG ATAAAAATATAAAAAATAATTTCCATGTAAGTTACCAGAAAGCCCACCAGCAACATAATTTTAAAGCCTT TCGGATTACTTTTAAAAAATGCAGCTTACATATAACAACTTGTGCCTATTTTATTTCTAATCTATCACTT CAAAAGATGGTAATCTTTCAACTCATTATTCCTCCAATTTTTAATGTCGAATTTTTTTCTAACACAATAA CCAAAAGCTTTTATTTATAAAAAGGCTTGAAAAATATAAAAAAAAAAAAAAAAAAAAAAAAAAAAAAAAA AAAAAAAAAAAAAAAAAAAAAAAAAAAAAAAAAAAA |
| **11** | Protein kinase C, beta 1 | >gi\|89353296\|ref\|NM_001183.4\| Homo sapiens ATPase, H+ transporting, lysosomal accessory protein 1 (ATP6AP1), mRNA |
| | | GGGGGCAACGGTCACCTGATCTGCGGCTGTCGAGGCCGCTGAGGCAGTGGAGGCTGAGGCTATGATGGCG GCCATGGCGACGGCTCGAGTGCGGATGGGGCCGCGATGCGCCCAGGCGCTCTGGCGCATGCCGTGGCTGC CGGTGTTTTTGTCGTTGGCGGCGGCGGCGGCGGCAGCGGCGGAGCAGCAGGTCCCGCTGGTGCTGTG GTCGAGTGACCGGGACTTGTGGGCTCCTGCGGCCGACACTCATGAAGGCCACATCACCAGCGACTTGCAG CTCTCTACCTACTTAGATCCCGCCCTGGAGCTGGGTCCCAGGAATGTGCTGCTGTTCCTGCAGGACAAGC TGAGCATTGAGGATTTCACAGCATATGGCGGTGTGTTTGGAAACAAGCAGGACAGCGCCTTTTCTAACCT AGAGAATGCCCTGGACCTGGCCCCCTCCTCACTGGTGCTTCCTGCCGTCGACTGGTATGCAGTCAGCACT CTGACCACTTACCTGCAGGAGAAGCTCGGGGCCAGCCCCTTGCATGTGGACCTGGCCACCCTGCGGGAGC TGAAGCTCAATGCCAGCCTCCCTGCTCTGCTGCTCATTCGCCTGCCCTACACAGCCAGCTCTGGTCTGAT GGCACCCAGGGAAGTCCTCACAGGCAACGATGAGGTCATCGGGCAGGTCCTGAGCACACTCAAGTCCGAA GATGTCCCATACACAGCGGCCCTCACAGCGGTCCGCCCTTCCAGGGTGGCCCGTGATGTAGCCGTGGTGG CCGGAGGGCTAGGTCGCCAGCTGCTACAAAAACAGCCAGTATCACCTGTGATCCATCCTCCTGTGAGTTA CAATGACACCGCTCCCCGGATCCTGTTCTGGGCCCAAAACTTCTCTGTGGCGTACAAGGACCAGTGGGAG GACCTGACTCCCCTCACCTTTGGGGTGCAGGAACTCAACCTGACTGGCTCCTTCTGGAATGACTCCTTTG CCAGGCTCTCACTGACCTATGAACGACTCTTTGGTACCACAGTGACATTCAAGTTCATTCTGGCCAACCG CCTCTACCCAGTGTCTGCCCGGCACTGGTTTACCATGGAGCGCCTCGAAGTCCACAGCAATGGCTCCGTC GCCTACTTCAATGCTTCCCAGGTCACAGGGCCCAGCATCTACTCCTTCCACTGCGAGTATGTCAGCAGCC TGAGCAAGAAGGGTAGTCTCCTCGTGGCCCGCACGCAGCCCTCTCCCTGGCAGATGATGCTTCAGGACTT CCAGATCCAGGCTTTCAACGTAATGGGGGAGCAGTTCTCCTACGCCAGCGACTGTGCCAGCTTCTTCTCC CCCGGCATCTGGATGGGGCTGCTCACCTCCCTGTTCATGCTCTTCATCTTCACCTATGGCCTGCACATGA TCCTCAGCCTCAAGACCATGGATCGCTTTGATGACCACAAGGGCCCCACTATTTCTTTGACCCAGATTGT GTGACCCTGTGCCAGTGGGGGGGTTGAGGGTGGGACGGTGTCCGTGTTGTTGCTTTCCCACCCTGCAGCG CACTGGACTGAAGAGCTTCCCTCTTCCTACTGCAGCATGAACTGCAAGCTCCCCTCAGCCCATCTTGCTC CCTCTTCAGCCCGCTGAGGAGCTTTCTTGGGCTGCCCCCATCTCTCCCAACAAGGTGTACATATTCTGCG TAGATGCTAGACCAACCAGCTTCCCAGGGTTCGTCGCTGTGAGGCGTAAGGACATGAATTCTAGGGTCT |
| | | CCTTTCTCCTTATTTATTCTTGTGGCTACATCATCCCTGGCTGTGGATAGTGCTTTTGTGTAGCAAATGC TCCCTCCTTAAGGTTATAGGGCTCCCTGAGTTTGGGAGTGTGGAAGTACTACTTAACTGTCTGTCCTGCT TGGCTGTCGTTATCGTTTTCTGGTGATGTTGTGCTAACAATAAGAAGTACACGGGTTTATTCTGTGGCC TGAGAAGGAAGGGACCTCCACGACAGGTGGGCTGGGTGCGATCGCCGGCTGTTTGGCATGTTCCCACCGG GAGTGCCGGGCAGGAGCATGGGGTGCTTGGTTGTTTCCTTCCTAATAAAATAAACGCGGGTCGCCATGCA AAAAAAAAAA |
| **12** | major histocompatibility complex, class II, DR alpha /// major histocompatibility complex, class II, DR alpha | >gi\|52426773\|ref\|NM_019111.3\| Homo sapiens major histocompatibility complex, class II, DR alpha (HLA-DRA), mRNA |
| | | ACATTCTCTTTTCTTTTATTCTTGTCTGTTCTGCCTCACTCCCGAGCTCTACTGACTCCCAACAGAGCGC CCAAGAAGAAAATGGCCATAAGTGGAGTCCCTGTGCTAGGATTTTTCATCATAGCTGTGCTGATGAGCGC TCAGGAATCATGGGCTATCAAAGAAGAACATGTGATCATCCAGGCCGAGTTCTATCTGAATCCTGACCAA TCAGGCGAGTTTATGTTTGACTTTGATGGTGATGAGATTTTCCATGTGGATATGGCAAAGAAGGAGACGG TCTGGCGGCTTGAAGAATTTGGACGATTTGCCAGCTTTGAGGCTCAAGGTGCATTGGCCAACATAGCTGT GGACAAAGCCAACCTGGAAATCATGACAAAGCGCTCCAACTATACTCCGATCACCAATGTACCTCCAGAG GTAACTGTGCTCACGAACAGCCCTGTGGAACTGAGAGAGCCCAACGTCCTCATCTGTTTCATAGACAAGT TCACCCCACCAGTGGTCAATGTCACGTGGCTTCGAAATGGAAAACCTGTCACCACAGGAGTGTCAGAGAC AGTCTTCCTGCCCAGGGAAGACCACCTTTTCCGCAAGTTCCACTATCTCCCCTTCCTGCCCTCAACTGAG GACGTTTACGACTGCAGGGTGGAGCACTGGGGCTTGGATGAGCCTCTTCTCAAGCACTGGGAGTTTGATG CTCCAAGCCCTCTCCCAGAGACTACAGAGAACGTGGTGTGCCCTGGGCCTGACTGTGGGTCTGGTGGG CATCATTATTGGGACCATCTTCATCATCAAGGGATTGCGCAAAAGCAATGCAGCAGAACGCAGGGGGCCT CTGTAAGGCACATGGAGGTGATGGTGTTTCTTAGAGAGAAGATCACTGAAGAAACTTCTGCTTTAATGGC TTTACAAAGCTGGCAATATTACAATCCTTGACCTCAGTGAAAGCAGTCATCTTCAGCATTTTCCAGCCCT ATAGCCACCCCAAGTGTGGATATGCCTCTTCGATTGCTCCGTACTCTAACATCTAGCTGGCTTCCCTGTC TATTGCCTTTTCCTGTATCTATTTTCCTCTATTTCCTATCATTTTATTATCACCATGCAATGCCTCTGGA ATAAAACATACAGGAGTCTGTCTCTGCTATGGAATGCCCCATGGGGCATCTCTTGTGTACTTATTGTTTA AGGTTTCCTCAAACTGTGATTTTTCTGAACACAATAAACTATTTTGATGATCTTGGGTGGAAAAAAAAAA AAAAAAA |
| **Sequence** | Major histocompatibility complex, class II, | >gi\|24797068\|ref\|NM_002123.2\| Homo sapiens major histocompatibility complex, class II, DQ beta 1 (HLA-DQB1), mRNA |
| | | CAGATCCATCAGGTCCGAGCTGTGTTGACTACCACTTTTCCCTTCGTCTCAATTATGTCTTGGAAAAAGG CTTTGCGGATCCCCGGAGGCCTTCGGGCAGCAACTGTGACCTTGATGCTGTCGATGCTGAGCACCCCAGT |
| **13** | DQ beta 1 /// Major histocompatibility complex, class II, DQ beta 1 | GGCTGAGGGCAGAGACTCTCCCGAGGATTTCGTGTACCAGTTTAAGGCATGTGCTACTTCACCAACGGG ACAGAGCGCGTGCGTCTTGTGAGCAGAAGCATCTATAACCGAGAAGAGATCGTGCGCTTCGACAGCGACG TGGGGGAGTTCCGGGCGGTGACGCTGCTGGGGCTGCCTGCCGCCGAGTACTGGAACAGCCAGAAGGACAT CCTGGAGAGGAAACGGGCGGCGGTGGACAGGGTGTGCAGACACAACTACCAGTTGGAGCTCCGCACGACC TTGCAGCGGCGAGTGGAGCCCACAGTGACCATCTCCCCATCCAGGACAGAGGCCCTCAACCACCACAACC TGCTGGTCTGCTCGGTGACAGATTTCTATCCAGCCCAGATCAAAGTCCGGTGGTTTCGGAATGACCAGGA GGAGACAGCTGGCGTTGTGTCCACCCCCCTTATTAGGAATGGTGACTGGACCTTCCAGATCCTGGTGATG CTGGAAATGACTCCCCAGCGTGGAGACGTCTACACCTGCCACGTGGAGCACCCCAGCCTCCAGAGCCCCA TCACCGTGGAGTGGCGGGCTCAATCTGAATCTGCCCAGAGCAAGATGCTGAGTGGCATTGGAGGCTTCGT GCTGGGGCTGATCTTCCTCGGGCTGGGCCTTATCATCCATCACAGGAGTCAGAAAGGGCTCCTGCACTGA CTCCTGAGACTATTTTAACTGGGATTGGTTATCACTTTTCTGTAACGCCTGCTTGTCCCTGCCCAGAATT CCCAGCTGTCTGTGTCAGCCTGTCCCCCTGAGATCAGAGTCCTACAGTGGCTGTCACGCAGCCACCAGGT CATCTCCTTTCATCCCCACCTTGAGGCGGATGGCTGTGACCCTACTTCCTGCACTGACCCACAGCCTCTG CCTGTGCACGGCCAGCTGCATCTACTCAGGCCCCAAGGGGTTTCTGTTTCTATTCTCTCCTCAGACTGCT CAAGAGAAGCACATGAAAACCATTACCTGACTTTAGAGCTTTTTTACATAATTAAACATGATCCTGAGTT |
| **14** | alcohol dehydrogenase IB (class I), beta polypeptide | >gi\|34577060\|ref\|NM_000668.3\| Homo sapiens alcohol dehydrogenase IB (class I), beta polypeptide (ADH1B), mRNA |
| | | ATGCACTCAAGCAGAGAAGAAATCCACAAAGACTCACAGTCTGCTGGTGGGCAGAGAAGACAGAAACGAC ATGAGCACAGCAGGAAAAGTAATCAAATGCAAAGCAGCTGTGCTATGGAGGTAAAGAAACCCTTTTCCA TTGAGGATGTGGAGGTTGCACCTCCTAAGGCTTATGAAGTTCGCATTAAGATGGTGGCTGTAGGAATCTG TCACACAGATGACCACGTGGTTAGTGGCAACCTGGTGACCCCCCTTCCTGTGATTTTAGGCCATGAGGCA GCCGGCATCGTGGAGAGTGTTGGAGAAGGGGTGACTACAGTCAAACCAGGTGATAAAGTCATCCCGCTCT TTACTCCTCAGTGTGGAAAATGCAGAGTTTGTAAAAACCCGGAGAGCAACTACTGCTTGAAAAATGATCT AGGCAATCCTCGGGGGACCCTGCAGGATGGCACCAGGAGGTTCACCTGCAGGGGGAAGCCCATTCACCAC TTCCTTGGCACCAGCACCTTCTCCCAGTACACGGTGGTGGATGAGAATGCAGTGGCCAAAATTGATGCAG CCTCGCCCCTGGAGAAAGTCTGCCTCATTGGCTGTGGATTCTCGACTGGTTATGGGTCTGCAGTTAACGT TGCCAAGGTCACCCCAGGCTCTACCTGTGCTGTGTTTGGCCTGGGAGGGGTCGGCCTATCTGCTGTTATG GGCTGTAAAGCAGCTGGAGCAGCCAGAATCATTGCGGTGGACATCAACAAGGACAAATTTGCAAAGGCCA AAGAGTTGGGTGCCACTGAATGCATCAACCCTCAAGACTACAAGAAACCCATCCAGGAAGTGCTAAAGGA AATGACTGATGGAGGTGTGGATTTTTCGTTTGAAGTCATCGGTCGGCTTGACACCATGATGGCTTCCCTG TTATGTTGTCATGAGGCATGTGGCACAAGCGTCATCGTAGGGGTACCTCCTGCTTCCCAGAACCTCTCAA TAAACCCTATGCTGCTACTGACTGGACGCACCTGGAAGGGGGCTGTTTATGGTGGCTTTAAGAGTAAAGA AGGTATCCCAAAACTTGTGGCTGATTTTATGGCTAAGAAGTTTTCACTGGATGCGTTAATAACCCATGTT |
| | | TTACCTTTTGAAAAAATAAATGAAGGATTTGACCTGCTTCACTCTGGGAAAAGTATCCGTACCGTCCTGA CGTTTTGAGGCAATAGAGATGCCTTCCCCTGTAGCAGTCTTCAGCCTCCTCTACCCTACAAGATCTGGAG CAACAGCTAGGAAATATCATTAATTCAGCTCTTCAGAGATGTTATCAATAAATTACACATGGGGGCTTTC CAAAGAAATGGAAATTGATGGGAAATTATTTTTCAGGAAAATTTAAAATTCAAGTGAGAAGTAAATAAAG TGTTGAACATCAGCTGGGGAATTGAAGCCAACAAACCTTCCTTCTTAACCATTCTACTGTGTCACCTTTG CCATTGAGGAAAAATATTCCTGTGACTTCTTGCATTTTTGGTATCTTCATAATCTTTAGTCATCGAATCC CAGTGGAGGGGACCCTTTTACTTGCCCTGAACATACACATGCTGGGCCATTGTGATTGAAGTCTTCTAAC TCTGTCTCAGTTTTCACTGTCGACATTTTCCTTTTTCTAATAAAAATGTACCAAATCCCTGGGGTAAAAG CTAGGGTAAGGTAAAGGATAGACTCACATTTACAAGTAGTGAAGGTCCAAGAGTTCTAAATACAGGAAAT TTCTTAGGAACTCAAATAAAATGCCCCACATTTTACTACAGTAAATGGCAGTGTTTTTATGACTTTTATA CTATTTCTTTATGGTCGATATACAATTGATTTTTAAAATAATAGCAGATTTCTTGCTTCATATGACAAA GCCTCAATTACTAATTGTAAAAACTGAACTATTCCCAGAATCATGTTCAAAAAATCTGTAATTTTTGCTG ATGAAAGTGCTTCATTGACTAAACAGTATTAGTTTGTGGCTATAAATGATTATTTAGATGATGACTGAAA ATGTGTATAAAGTAATTAAAAGTAATATGGTGGCTTTAAGTGTAGAGATGGGATGGCAAATGCTGTGAAT GCAGAATGTAAAATTGGTAACTAAGAAATGGCACAAACACCTTAAGCAATATATTTTCCTAGTAGATATA TATATACACATACATATATACACATATACAAATGTATATTTTTGCAAAATTGTTTTCAATCTAGAACTTT TCTATTAACTACCATGTCTTAAAATCAAGTCTATAATCCTAGCATTAGTTTAATATTTTGAATATGTAAA GACCTGTGTTAATGCTTTGTTAATGCTTTTCCCACTCTCATTTGTTAATGCTTTCCCACTCTCAGGGGAA GGATTTGCATTTTGAGCTTTATCTCTAAATGTGACATGCAAAGATTATTCCTGGTAAAGGAGGTAGCTGT CTCCAAAAATGCTATTGTTGCAATATCTACATTCTATTTCATATTATGAAAGACCTTAGACATAAAGTAA AATAGTTTATCATTTACTGTGTGATCTTCAGTAAGTCTCTCAGGCTCTCTGAGCTTGTTCATCCTTTGTT TTGAAAAAATTACTCAACCAATCCATTACAGCTTAACCAAGATTAAATGGGATGATGTTAATGAAAGAGC TTCGCC |
| **15** | T cell receptor alpha constant /// T cell receptor alpha constant | >gi\|36944\|emb\|X02592.1\|HSTCRAR Human mRNA for T-cell receptor alpha chain (TCR-alpha) |
| | | TTTTGAAACCCTTCAAAGGCAGAGACTTGTCCAGCCTAACCTGCCTGCTGCTCCTAGCTCCTGAGGCTCA GGGCCCTTGGCTTCTGTCCGCTCTGCTCAGGGCCCTCCAGCGTGGCCACTGCTCAGCCATGCTCCTGCTG CTCGTCCCAGTGCTCGAGGTGATTTTTACCCTGGGAGGAACCAGAGCCCAGTCGGTGACCCAGCTTGGCA GCCACGTCTCTGTCTCTGAAGGAGCCCTGGTTCTGCTGAGGTGCAACTACTCATCGTCTGTTCCACCATA TCTCTTCTGGTATGTGCAATACCCCAACCAAGGACTCCAGCTTCTCCTGAAGTACACATCAGCGGCCACC CTGGTTAAAGGCATCAACGGTTTTGAGGCTGAATTTAAGAAGAGTGAAACCTCCTTCCACCTGACGAAAC CCTCAGCCCATATGAGCGACGCGGCTGAGTACTTCTGTGCTGTGAGTGATCTCGAACCGAACAGCAGTGC TTCCAAGATAATCTTTGGATCAGGGACCAGACTCAGCATCCGGCCAAATATCCAGAACCCTGACCCTGCC |
| | | GTGTACCAGCTGAGAGACTCTAAATCCAGTGACAAGTCTGTCTGCCTATTCACCGATTTTGATTCTCAAA CAAATGTGTCACAAAGTAAGGATTCTGATGTGTATATCACAGACAAAACTGTGCTAGACATGAGGTCTAT GGACTTCAAGAGCAACAGTGCTGTGGCCTGGAGCAACAAATCTGACTTTGCATGTGCAAACGCCTTCAAC AACAGCATTATTCCAGAAGACACCTTCTTCCCCAGCCCAGAAAGTTCCTGTGATGTCAAGCTGGTCGAGA AAAGCTTTGAAACAGATACGAACCTAAACTTTCAAAACCTGTCAGTGATTGGGTTCCGAATCCTCCTCCT GAAAGTGGCCGGGTTTAATCTGCTCATGACGCTGCGGCTGTGGTCCAGCTGAGATCTGCAAGATTGTAAG ACAGCCTGTGCTCCCTCGCTCCTTCCTCTGCATTGCCCCTCTTCTCCCTCTCCAAACAGAGGGAACTCTC CTACCCCCAAGGAGGTGAAAGCTGCTACCACCTCTGTGCCCCCCCGGTAATGCCACCAACTGGATCCTAC CCGAATTTATGATTAAGATTGCTGAAGAGCTGCCAAACACTGCTGCCACCCCCTCTGTTCCCTTATTGCT GCTTGTCACTGCCTGACATTCACGGCAGAGGCAAGGCTGCTGCAGCCTCCCCTGGCTGTGCACATTCCCT CCTGCTCCCCAGAGACTGCCTCCGCCATCCCACAGATGATGGATCTTCAGTGGGTTCTCTTGGGCTCTAG GTCCTGGAGAATGTTGTGAGGGGTTTATTTTTTTTTAATAGTGTTCATAAAGAAATACATAGTATTCTTC TTCTCAAGACGTGGGGGAAATTATCTCATTATCGAGGCCCTGCTATGCTGTGTGTCTGGGCGTGTTGTA TGTCCTGCTGCCGATGCCTTCATTAAAATGATTTGGAA |
| **16** | CD69 antigen (p60, early T-cell activation antigen) | >gi\|4502680\|ref\|NM_001781.1\| Homo sapiens CD69 molecule (CD69), mRNA |
| | | AGACTCAACAAGAGCTCCAGCAAAGACTTTCACTGTAGCTTGACTTGACCTGAGATTAACTAGGGAATCT TGAGAATAAAGATGAGCTCTGAAAATTGTTTCGTAGCAGAGAACAGCTCTTTGCATCCGGAGAGTGGACA AGAAAATGATGCCACCAGTCCCCATTTCTCAACACGTCATGAAGGGTCCTTCCAAGTTCCTGTCCTGTGT GCTGTAATGAATGTGGTCTTCATCACCATTTTAATCATAGCTCTCATTGCCTTATCAGTGGGCCAATACA ATTGTCCAGGCCAATACACATTCTCAATGCCATCAGACAGCCATGTTTCTTCATGCTCTGAGGACTGGGT TGGCTACCAGAGGAAATGCTACTTTATTTCTACTGTGAAGAGGAGCTGGACTTCAGCCCAAAATGCTTGT TCTGAACATGGTGCTACTCTTGCTGTCATTGATTCTGAAAAGGACATGAACTTTCTAAAACGATACGCAG GTAGAGAGGAACACTGGGTTGGACTGAAAAAGGAACCTGGTCACCCATGGAAGTGGTCAAATGGCAAAGA ATTTAACAACTGGTTCAACGTTACAGGGTCTGACAAGTGTGTTTTTCTGAAAAACACAGAGGTCAGCAGC ATGGAATGTGAGAAGAATTTATACTGGATATGTAACAAACCTTACAAATAATAAGGAAACATGTTCACTT ATTGACTATTATAGAATGGAACTCAAGGAAATCTGTGTCAGTGGATGCTGCTCTGTGGTCCGAAGTCTTC CATAGAGACTTTGTGAAAAAAATTTATAGTGTCTTGGGAATTTTCTTCCAAACAGAACTATGGAAAAA AAGGAAGAAATTCCAGGAAAATCTGCACTGTGGGCTTTTATTGCCATGAGCTAGAAGCATCACAGGTTGA CCAATAACCATGCCCAAGAATGAGAAGAATGACTATGCAACCTTTGGATGCACTTTATATTATTTTGAAT CCAGAAATAATGAAATAACTAGGCGTGGACTTACTATTTATTGCTGAATGACTACCAACAGTGAGAGCCC TTCATGCATTTGCACTACTGGAAGGAGTTAGATGTTGGTACTAGATACTGAATGTAAACAAAGGAATTAT GGCTGGTAACATAGGTTTTAGTCTAATTGAATCCCTTAAACTCAGGGAGCATTTATAAATGGACAAATG CTTATGAAACTAAGATTTGTAATATTTCTCTCTTTTTAGAGAAATTTGCCAATTTACTTTGTTATTTTTC CCCAAAAAGAATGGGATGATCGTGTATTTATTTTTTTACTTCCTCAGCTGTAGACAGGTCCTTTTCGATG |
| | | GTACATATTTCTTTGCCTTTATAATCTTTTATACAGTGTCTTACAGAGAAAAGACATAAGCAAAGACTAT GAGGAATATTTGCAAGACATAGAATAGTGTTGGAAAATGTGCAATATGTGATGTGGCAAATCTCTATTAG GAAATATTCTGTAATCTTCAGACCTAGAATAATACTAGTCTTATAATAGGTTTGTGACTTTCCTAAATCA ATTCTATTACGTGCAATACTTCAATACTTCATTTAAAATATTTTTATGTGCAATAAAATGTATTTGTTTG TATTTTGTGTTCAGTACAATTATAAGCTGTTTTTATATATGTGAAATAAAAGTAGAATAAACACAAAAAA AAAAAAAAAAAAAAAAAAAAAA |
| **17** | protein kinase C, theta | >gi\|48255887\|ref\|NM_006257.2\| Homo sapiens protein kinase C, theta (PRKCQ), mRNA |
| | | AGTCCCCGCGCAGTCCCCGCGCAGTCCCAGCGCCACCGGGCAGCAGCGGCGCCGTGCTCGCTCCAGGGCG CAACCATGTCGCCATTTCTTCGGATTGGCTTGTCCAACTTTGACTGCGGGTCCTGCCAGTCTTGTCAGGG CGAGGCTGTTAACCCTTACTGTGCTGTGCTCGTCAAAGAGTATGTCGAATCAGAGAACGGGCAGATGTAT ATCCAGAAAAAGCCTACCATGTACCCACCCTGGGACAGCACTTTTGATGCCCATATCAACAAGGAAGAG TCATGCAGATCATTGTGAAAGGCAAAAACGTGGACCTCATCTCTGAAACCACCGTGGAGCTCTACTCGCT GGCTGAGAGGTGCAGGAAGAACAACGGGAAGACAGAAATATGGTTAGAGCTGAAACCTCAAGGCCGAATG CTAATGAATGCAAGATACTTTCTGGAAATGAGTGACACAAAGGACATGAATGAATTTGAGACGGAAGGCT TCTTTGCTTTGCATCAGCGCCGGGGTGCCATCAAGCAGGCAAAGGTCCACCACGTCAAGTGCCACGAGTT CACTGCCACCTTCTTCCCACAGCCCACATTTTGCTCTGTCTGCCACGAGTTTGTCTGGGGCCTGAACAAA CAGGGCTACCAGTGCCGACAATGCAATGCAGCAATTCACAAGAAGTGTATTGATAAAGTTATAGCAAAGT GCACAGGATCAGCTATCAATAGCCGAGAAACCATGTTCCACAAGGAGAGATTCAAAATTGACATGCCACA CAGATTTAAAGTCTACAATTACAAGAGCCCGACCTTCTGTGAACACTGTGGGACCCTGCTGTGGGGACTG GCACGGCAAGGACTCAAGTGTGATGCATGTGGCATGAATGTGCATCATAGATGCCAGACAAAGGTGGCCA ACCTTTGTGGCATAAACCAGAAGCTAATGGCTGAAGCGCTGGCCATGATTGAGAGCACTCAACAGGCTCG CTGCTTAAGAGATACTGAACAGATCTTCAGAGAAGGTCCGGTTGAAATTGGTCTCCCATGCTCCATCAAA AATGAAGCAAGGCCGCCATGTTTACCGACACCGGGAAAAAGAGAGCCTCAGGGCATTTCCTGGGAGTCTC CGTTGGATGAGGTGGATAAAATGTGCCATCTTCCAGAACCTGAACTGAACAAAGAAAGACCATCTCTGCA GATTAAACTAAAAATTGAGGATTTTATCTTGCACAAAATGTTGGGGAAAGGAAGTTTTGGCAAGGTCTTC CTGGCAGAATTCAAGAAAACCAATCAATTTTTCGCAATAAAGGCCTTAAAGAAAGATGTGGTCTTGATGG ACGATGATGTTGAGTGCACGATGGTAGAGAAGAGAGTTCTTTCCTTGGCCTGGGAGCATCCGTTTCTGAC GCACATGTTTTGTACATTCCAGACCAAGGAAAACCTCTTTTTTGTGATGGAGTACCTCAACGGAGGGGAC TTAATGTACCACATCCAAAGCTGCCACAAGTTCGACCTTTCCAGAGCGACGTTTTATGCTGCTGAAATCA TTCTTGGTCTGCAGTTCCTTCATTCCAAAGGAATAGTCTACAGGGACCTGAAGCTAGATAACATCCTGTT AGACAAAGATGGACATATCAAGATCGCGGATTTTGGAATGTGCAAGGAGAACATGTTAGGAGATGCCAAG ACGAATACCTTCTGTGGGACACCTGACTACATCGCCCCAGAGATCTTGCTGGGTCAGAAATACAACCACT |
| | | CTGTGGACTGGTGGTCCTTCGGGGTTCTCCTTTATGAAATGCTGATTGGTCAGTCGCCTTTCCACGGGCA GGATGAGGAGGAGCTCTTCCACTCCATCCGCATGGACAATCCCTTTTACCCACGGTGGCTGGAGAAGGAA GCAAAGGACCTTCTGGTGAAGCTCTTCGTGCGAGAACCTGAGAAGAGGCTGGGCGTGAGGGGAGACATCC GCCAGCACCCTTTGTTTCGGGAGATCAACTGGGAGGAACTTGAACGGAAGGAGATTGACCCACCGTTCCG GCCGAAAGTGAAATCACCATTTGACTGCAGCAATTTCGACAAAGAATTCTTAAACGAGAAGCCCCGGCTG TCATTTGCCGACAGAGCACTGATCAACAGCATGGACCAGAATATGTTCAGGAACTTTTCCTTCATGAACC CCGGGATGGAGCGGCTGATATCCTGAATCTTGCCCCTCCAGAGACAGGAAAGAATTTGCCTTCTCCCTGG GAACTGGTTCAAGAGACACTGCTTGGGTTCCTTTTTCAACTTGGAAAAAGAAAGAAACACTCAACAATAA AGACTGAGACCCGTTCGCCCCCATGTGACTTTATCTGTAGCAGAAACCAAGTCTACTTCACTAATGACG ATGCCGTGTGTCTCGTCTCCTGACATGTCTCACAGACGCTCCTGAAGTTAGGTCATTACTAACCATAGTT ATTTACTTGAAAGATGGGTCTCCGCACTTGGAAAGGTTTCAAGACTTGATACTGCAATAAATTATGGCTC TTCACCTGGGCGCCAACTGCTGATCAATGAAATGCTTGTTGAATCAGGGGCAAACGGAGTACAGACGTCT CAAGACTGAAACGGCCCCATTGCCTGGTCTAGTAGCGGATCTCACTCAGCCGCAGACAAGTAATCACTAA CCCGTTTTATTCTATTCCTATCTGTGGATGTGTAAATGGCTGGGGGGCCAGCCCTGGATAGGTTTTTATG GGAATTCTTTACAATAAACATAGCTTGTAACTTGAGATCTACAAATCCATTCATCCTGATTGGGCATGAA ATCCATGGTCAAGAGGACAAGTGGAAAGTGAGAGGGAAGGTTTGCTAGACACCTTCGCTTGTTATCTTGT CAAGATAGAAAAGATAGTATCATTTCACCCTTGCCAGTAAAAACCTTTCCATCCACCCATTCTCAGCAGA CTCCAGTATTGGCACAGTCACTCACTGCCATTCTCACACTATAACAAGAAAAGAAATGAAGTGCATAAGT CTCCTGGGAAAAGAACCTTAACCCCTTCTCGTGCCATGACTGGTGATTTCATGACTCATAAGCCCCTCCG TAGGCATCATTCAAGATCAATGGCCCATGCATGCTGTTTGCAGCAGTCAATTGAGTTGAATTAGAATTCC AACCATACATTTTAAAGGTATTTGTGCTGTGTGTATATTTTGATAAAATGTTGTGACTTCATGGCAAACA GGTGGATGTGTAAAAATGGAATAAAAAAAAAAAAAAGAGTCAAAAAAAAAAAAA |
| **18** | T cell receptor beta variable 19 /// T cell receptor beta constant 1 | >gi\|339011\|gb\|M15564.1\|HUMTCBYZ Human T-cell receptor rearranged beta-chain V-region (V-D-J) mRNA, complete cds |
| | | GTAAAGCTCCCATCCTGCCCTGACCCTGCCATGGGCACCAGCCTCCTCTGCTGGATGGCCCTGTGTCTCC TGGGGGCAGATCACGCAGATACTGGAGTCTCCCAGAACCCCAGACACAACATCACAAAGAGGGGACAGAA TGTAACTTTCAGGTGTGATCCAATTTCTGAACACAACCGCCTTTATTGGTACCGACAGACCCTGGGGCAG GGCCCAGAGTTTCTGACTTACTTCCAGAATGAAGCTCAACTAGAAAAATCAAGGCTGCTCAGTGATCGGT TCTCTGCAGAGAGGCCTAAGGGATCTTTCTCCACCTTGGAGATCCAGCGCACAGAGCAGGGGGACTCGGC CATGTATCTCTGTGCCAGCAGCTTAGCAGGGTTGAATCAGCCCCAGCATTTTGGTGATGGGACTCGACTC TCCATCCTAGAGGACCTGAACAAGGTGTTCCCACCCGAGGTCGCTGTGTTTGAGCCATCAGAAGCAGAGA TCTCCCACACCCAAAAGGCCACACTGGTGTGCCTGGCCACAGGTATCTTCCCTGACCACGTGGAGCTGAG CTGGTGGGTGAATGGGAAGGAGGTGCACAGTGGGGTCAGCACGGACCCGCAGCCCCTCAAGGAGCAGCCC |
| | | GCCCTCAATGACTCCAGATACTGCCTGAGCAGCCGCCTGAGGGTCTCGGCCACCTTCTGGCAGAACCCCC GCAACCACTTCCGCTGTCAAGTCCAGTTCTACGGGCTCTCGGAGAATGACGAGTGGACCCAGGATAGGGC CAAACCCGTCACCCAGATCGTCAGCGCCGAGGCCTGGGGTAGAGCAGACTGTGGCTTTACCTCGGTGTCC TACCAGCAAGGGGTCCTGTCTGCCACCATCCTCTATGAGATCCTGCTAGGGAAGGCCACCATGTATGCTG TGCTGGTCAGCGCCCTTGTGTTGATGGCCATGGTCAAGAGAAAGGATTTCTGAAGGCAGCCCTGGAAGTG GAGTTAGGAGCTTCTAACCCGTCATGGTTTCAATACACATTCTTCTTTTGCCAGCGCTTCTGAAGAGCTG CTCTCACCTCTCTGCATCCCAATA |
| **19** | T cell receptor alpha locus /// T cell receptor delta variable 2 /// T cell receptor alpha variable 20 /// T cell receptor alpha joining 17 /// T cell receptor alpha constant | >gi\|338765\|gb\|M15565.1\|HUMTCAYE Human T-cell receptor rearranged alpha-chain V-region (V-D-J) mRNA, complete cds |
| | | TCCAAAATGAAGGGTCTGTGGAAGGACATGAATAAAGCACAGGAGGTTGAAGTCAGATTTGCAGCTTTCT AGGCAGGAGACAAGACAATCTGCATCTTCACAGGAGGGATGGCCATGCTCCTGGGGGCATCAGTGCTGAT TCTGTGGCTTCAGCCAGACTGGGTAAACAGTCAACAGAAGAATGATGACCAGCAAGTTAAGCAAAATTCA CCATCCCTGAGCGTCCAGGAAGGAAGAATTTCTATTCTGAACTGTGACTATACTAACAGCATGTTTGATT ATTTCCTATGGTACAAAAAATACCCTGCTGAAGGTCCTACATTCCTGATATCTATAAGTTCCATTAAGGA TAAAAATGAAGATGGAAGATTCACTGTCTTCTTAAACAAAAGTGCCAAGCACCTCTCTCTGCACATTGTG CCCTCCCAGCCTGGAGACTCTGCAGTGTACTTCTGTGCAGCAAAGGGGGCCGGCACTGCCAGTAAACTCA CCTTTGGGACTGGAACAAGACTTCAGGTCACGCTCGATATCCAGAACCCTGACCCTGCCGTGTACCAGCT GAGAGACTCTAAATCCAGTGACAAGTCTGTCTGCCTATTCACCGATTTTGATTCTCAAACAAATGTGTCA CAAAGTAAGGATTCTGATGTGTATATCACAGACAAAACTGTGCTAGACATGAGGTCTATGGACTTCAAGA GCAACAGTGCTGTGGCCTGGAGCAACAAATCTGACTTTGCATGTGCAAACGCCTTCAACAACAGCATTAT TCCAGAAGACACCTTCTTCCCCAGCCCAGAAAGTTCCTGTGATGTCAAGCTGGTCGAGAAAAGCTTTGAA ACAGATACGAACCTAAACTTTCAAAACCTGTCAGTGATTGGGTTCCGAATCCTCCTCCTGAAAGTGGCCG GGTTTAATCTGCTCATGACGCTGCGGCTGTGGTCCAGCTGAGATCTGCAAGATTGTAAGACAGCCTGTGC TCCCTCGCTCCTTCCTCTGCATTGCCCCTCTTCTCCCTCTCCAAACAGAGGGAACTCTCCCACCCCCAAG GAGGTGAAAGCTGCTACCACCCTCTGTGCC |
| **20** | T cell receptor gamma constant 2 | >gi\|339406\|gb\|M30894.1\|HUMTCRGAD Human T-cell receptor Ti rearranged gamma-chain mRNA V-J-C region, complete cds |
| | | GAATCAGGAAGACCAGCTCCTCCTACTGTCTTCTGTGTTACGGGATCAGCGTTCCTTGTTGAGTGGGACC TGAGTTTTGAGAGGGTCTTCTGCTCCTCTTGGTCTGGTCCCTTACTTCCAAGAGCCCCAGAGAGGAAGGC ATGCTGTTGGCTCTAGCTCTGCTTCTAGCTTTCCTGCCTCCTGCCAGTCAGAAATCTTCCAACTTGGAAG GGAGAACAAAGTCAGTCACCAGGCCAACTGGGTCATCAGCTGTAATCACTTGTGATCTTCCTGTAGAAAA |
| | | TGCCGTCTACACCCACTGGTACCTACACCAGGAGGGAAGGCCCCACAGCGTCTTCTGTACTATGACTCC TACAACTCCAGGGTTGTGTTGGAATCAGGAATCAGTCGAGAAAAGTATCATACTTATGCAAGCACAGGGA AGAGCCTTAAATTTATACTGGAAAATCTAATTGAACGTGACTCTGGGGTCTATTACTGTGCCACCTGGAA GGATTATTATAAGAAACTCTTTGGCAGTGGAACAACACTTGTTGTCACAGATAAACAACTTGATGCAGAT GTTTCCCCCAAGCCCACTATTTTTCTTCCTTCGATTGCTGAAACAAAACTCCAGAAGGCTGGAACATATC TTTGTCTTCTTGAGAAATTTTTCCCAGATATTATTAAGATACATTGGCAAGAAAAGAAGAGCAACACGAT TCTGGGATCCCAGGAGGGGAACACCATGAAGACTAACGACACATACATGAAATTTAGCTGGTTAACGGTG CCAGAAGAGTCACTGGACAAAGAACACAGATGTATCGTCAGACATGAGAATAATAAAAACGGAATTGATC AAGAAATTATCTTTCCTCCAATAAAGACAGATGTCACCACAGTGGATCCCAAAGACAGTTATTCAAAAGA TGCAAATGATGTCACCACAGTGGATCCCAAATACAATTATTCAAAGGATGCAAATGATGTCATCACAATG GATCCCAAAGACAATTGGTCAAAAGATGCAAATGATACACTACTGCTGCAGCTCACAAACACCTCTGCAT ATTACATGTACCTCCTCCTGCTCCTCAAGAGTGTGGTCTATTTTGCCATCATCACCTGCTGTCTGCTTGG AAGAACGGCTTTCTGCTGCAATGGAGAGAAATCATAACAGACGGTGGCACAAGGAGGCCATCTTTTCCTC ATCGGTTATTGTCCCTAGAAGCGTCTTCTGAGGATCTAGTTGGGCTTTCTTTCTGGGTTTGGGCCATTTC AGTTCTCATGTGTGTACTATTCTATCATTATTGTATAATGGTTTTCAAACCAGTGGGCACACAGAGAACC TCAGTCTGTAATAACAATGAGGAATAGCCATGGCGATCTCCAGCACCAATCTCTCCATGTTTTCCACAGC TCCTCCAGCCAACCCAAATAGCGCCTGCTATAGTGTAGACAGCCTGCGGCTTCTAGCCTTGTCCCTCTCT TAGTGTTCTTTAATCAGATAACTGCCTGGAAGCCTTTCATTTTACACGCCCTGAAGCAGTCTTCTTTGCT AGTTGAATTATGTGGTGTGTTTTTCCGTAATAAGCAAAATAAATTT |
| **21** | T cell receptor beta variable 21-1 /// T cell receptor beta variable 19 /// T cell receptor beta variable 5-4 /// T cell receptor beta variable 3-1 /// T cell receptor beta constant 1 | >gi\|3002924\|gb\|AF043179.1\|AF043179 Homo sapiens T cell receptor beta chain (TCRBV13S1-TCRBJ2S1) mRNA, complete cds |
| | | ATGAGCATCGGCCTCCTGTGCTGTGCAGCCTTGTCTCTCCTGTGGGCAGGTCCAGTGAATGCTGGTGTCA CTCAGACCCCAAAATTCCAGGTCCTGAAGACAGGACAGAGCATGACACTGCAGTGTGCCCAGGATATGAA CCATGAATACATGTCCTGGTATCGACAAGACCCAGGCATGGGGCTGAGGCTGATTCATTACTCAGTTGGT GCTGGTATCACTGACCAAGGAGAAGTCCCCAATGGCTACAATGTCTCCAGATCAACCACAGAGGATTTCC CGCTCAGGCTGCTGTCGGCTGCTCCCTCCCAGACATCTGTGTACTTCTGTGCCAGCAGTTTCCCCCGGCA GCCGTCCTACAATGAGCAGTTCTTCGGGCCAGGGACACGGCTCACCGTGCTAGAGGACCTGAAAAACGTG TTCCCACCCGAGGTCGCTGTGTTTGAGCCATCAGAAGCAGAGATCTCCCACACCCAAAAGGCCACACTGG TGTGCCTGGCCACAGGTTTCTACCCCGACCACGTGGAGCTGAGCTGGTGGGTGAATGGGAAGGAGGTGCA CAGTGGGGTCAGCACAGACCCGCAGCCCCTCAAGGAGCAGCCCGCCCTCAATGACTCCAGATACTGCCTG AGCAGCCGCCTGAGGGTCTCGGCCACCTTCTGGCAGAACCCCCGCAACCACTTCCGCTGTCAAGTCCAGT TCTACGGGCTCTCGGAGAATGACGAGTGGACCCAGGATAGGGCCAAACCTGTCACCCAGATCGTCAGCGC CGAGGCCTGGGGTAGAGCAGACTGTGGCTTCACCTCCGAGTCTTACCAGCAAGGGGTCCTGTCTGCCACC ATCCTCTATGAGATCTTGCTAGGGAAGGCCACCTTGTATGCTGTGCTGGTCAGTGCCCTCGTGCTGATGG |
| | | CCATGGTCAAGAGAAAGGATTCCAGAGGCTGA |
| **22** | T cell receptor alpha locus | >gi\|1100165\|gb\|L34703.1\|HUMTCRAZ Homo sapiens T-cell receptor alpha chain (TCRA) mRNA (HLA-A1, 24; B7, 8; DR 1, 3), complete cds |
| | | ATGGCATGCCCTGGCTTCCTGTGGGCACTTGTGATCTCCACCTGTCTTGAATTTAGCATGGCTCAGACAG TCACTCAGTCTCAACCAGAGATGTCTGTGCAGGAGGCAGAGACCGTGACCCTGAGCTGCACATATGACAC CAGTGAGAGTGATTATTATTTATTCTGGTACAAGCAGCCTCCCAGCAGGCAGATGATTCTCGTTATTCGC CAAGAAGCTTATAAGCAACAGAATGCAACAGAGAATCGTTTCTCTGTGAACTTCCAGAAAGCAGCCAAAT CCTTCAGTCTCAAGATCTCAGACTCACAGCTGGGGGATGCCGCGATGTATTTCTGTGCTTATAGGAGTGC ATACTCTGGGGCTGGGAGTTACCAACTCACTTTCGGGAAGGGGACCAAACTCTCGGTCATACCAAATATC CAGAACCCTGACCCTGCCGTGTACCAGCTGAGAGACTCTAAATCCAGTGACAAGTCTGTCTGCCTATTCA CCGATTTTGATTCTCAAACAAATGTGTCACAAAGTAAGGATTCTGATGTGTATATCACAGACAAAACTGT GCTAGACATGAGGTCTATGGACTTCAAGAGCAACAGTGCTGTGGCCTGGAGCAACAAATCTGACTTTGCA TGTGCAAACGCCTTCAACAACAGCATTATTCCAGAAGACACCTTCTTCCCCAGCCCAGAAAGTTCCTGTG ATGTCAAGCTGGTCGAGAAAAGCTTTGAAACAGATACGAACCTAAACTTTCAAAACCTGTCAGTGATTGG GTTCCGAATCCTCCTCCTGAAAGTGGCCGGGTTTAATCTGCTCATGACGCTGCGGTTGTGGTCCAGCTGA |
| **23** | T cell receptor beta variable 19 /// T cell receptor beta variable 19 /// T cell receptor beta constant 1 /// T cell receptor beta constant 1 | >gi\|46184507\|gb\|AL559122.3\|AL559122 AL559122 Homo sapiens T CELLS (JURKAT CELL LINE) COT 10-NORMALIZED Homo sapiens cDNA clone CS0DJ014YE01 5-PRIME, mRNA sequence |
| | | TGCCTGGCCACAGGCTTCTTCCCCGACCACGTGGAGCTGAGCTGGTGGGTGAATGGGAAGGAGGTGCACA ATGGGGTCAACACAGACCCGCAGCCCCTCAAAGAACAGCCCGCCCTCAATGACTCCAAATAMTGCCTGAG CAGCCGCCTGAGGGTCTCGGCCACCTTCTGGCAGAACCCCCGCAACCACTTCCGCTGTCAAGTCCAGTTC TACGGGCTCTCGGAGAATGACGAGTGGACCCAGGATAGGGCCAAACCCGTCACCCAAATCGTCAGCGCCG AGGCCTGGGGTARAGCARAMTGTGGCTTTACCTCGGTGTCCTACCAACAAGGGGTCCTGTCTGCCACCAT CCTCTATGARATCCTGCTAGGGAAGGCCACCCTGTATGCTGTGCTGGTCAGCGCCCTTGTGTTGATGGCC ATGGTCAAGAGAAAGGATTTCTGAAGGCAGCCCTGGAAGTGGAATTAAGAACTTCTAACCCGTCATGGTT TCAATACACATTCTTCTTTTGCCAGCGCTTCTGAAGAGCTGCTCTCACCTCTCTGCATCCCAATAGATAT CCCCCTATGTGCATGCACACCTGCACACTCACGGCTGAAATCTCCCTAACCCAGGGGGACCTTAGCATGC CTAAGTGACTAAACCAATAAAAATGTTCTGGTCTGGCCTGA |
| **24** | CD3D antigen, delta polypeptide (TiT3 complex) | >gi\|98985799\|ref\|NM_000732.4\| Homo sapiens CD3d molecule, delta (CD3-TCR complex) (CD3D), transcript variant 1, mRNA |
| | | AGAGAAGCAGACATCTTCTAGTTCCTCCCCCACTCTCCTCTTTCCGGTACCTGTGAGTCAGCTAGGGGAG GGCAGCTCTCACCCAGGCTGATAGTTCGGTGACCTGGCTTTATCTACTGGATGAGTTCCGCTGGGAGATG GAACATAGCACGTTTCTCTCTGGCCTGGTACTGGCTACCCTTCTCTCGCAAGTGAGCCCCTTCAAGATAC |
| | | CTATAGAGGAACTTGAGGACAGAGTGTTTGTGAATTGCAATACCAGCATCACATGGGTAGAGGGAACGGT GGGAACACTGCTCTCAGACATTACAAGACTGGACCTGGGAAAACGCATCCTGGACCCACGAGGAATATAT AGGTGTAATGGGACAGATATATACAAGGACAAAGAATCTACCGTGCAAGTTCATTATCGAATGTGCCAGA GCTGTGTGGAGCTGGATCCAGCCACCGTGGCTGGCATCATTGTCACTGATGTCATTGCCACTCTGCTCCT TGCTTTGGGAGTCTTCTGCTTTGCTGGACATGAGACTGGAAGGCTGTCTGGGGCTGCCGACACACAAGCT CTGTTGAGGAATGACCAGGTCTATCAGCCCCTCCGAGATCGAGATGATGCTCAGTACAGCCACCTTGGAG GAAACTGGGCTCGGAACAAGTGAACCTGAGACTGGTGGCTTCTAGAAGCAGCCATTACCAACTGTACCTT CCCTTCTTGCTCAGCCAATAAATATATCCTCTTTCACTCAGAAAAAAAAAAAAAAAAAAAAAAAAAAAAA A |
| **25** | T cell receptor gamma constant 2 /// T cell receptor gamma variable 9 /// similar to T-cell receptor gamma chain C region PT-gamma-1/2 /// similar to T-cell receptor gamma chain V region PT-gamma-1/2 precursor /// TCR gamma alternate reading frame protein | >gi\|339168\|gb\|M13231.1\|HUMTCGXH Human T-cell receptor aberrantly rearranged gamma-chain mRNA from cell line HPB-MLT |
| | | GGCATGCGGTGGGCCCTACTGGTGCTTCTAGCTTTCCTGTCTCCTGCCAGTCAGAAATCTTCCAACTTGG AAGGGAGAACGAAGTCAGTCACCAGGCAGACTGGGTCATCTGCTGAAATCACTTGCGATCTTACTGTAAC AAATACCTTCTACATCCACTGGTACCTACACCAGGAGGGAAGGCCCCACAGCGTCTTCTGTACTATGAC GTCTCCACTGCAAGGGATGTGTTGGAATCAGGACTCAGTCCAGGAAAGTATTATACTCATACACCCAGGA GGTGGAGCTGGATATTGAGACTGCAAAATCTAATTGAAAATGATTCTGGGGTCTATTACTGTGCCACCTG GGACAGGCAAAAATTATTATAAGAAACTCTTTGGCAGTGGAACAACACTTGTTGTCACAGATAAACAACT TGATGCAGATGTTTCCCCCAAGCCCACTATTTTTCTTCCTTCAATTGCTGAAACAAAACTCCAGAAGGCT GGAACATACCTTTGTCTTCTTGAGAAATTTTTCCCAGATATTATTAAGATACATTGGCAAGAAAAGAAGA GCAACACGATTCTGGGATCCCAGGAGGGGAACACCATGAAGACTAACGACACATACATGAAATTTAGCTG GTTAACGGTGCCAGAAGAGTCACTGGACAAAGAACACAGATGTATCGTCAGACATGAGAATAATAAAAAC GGAATTGATCAAGAAATTATCTTTCCTCCAATAAAGACAGATGTCACCACAGTGGATCCCAAAGACAGTT ATTCAAAAGATGCAAATGATGTCATCACAATGGATCCCAAAGACAATTGGTCAAAAGATGCAAATGATAC ACTACTGCTGCAGCTCACAAACACCTCTGCATATTACATGTACCTCCTCCTGCTCCTCAAGAGTGTGGTC TATTTTGCCATCATCACCTGCTGTCTGCTTGGAAGAACGGCTTTCTGCTGCAATGGAGAGAAATCATAAC AGACGGTGGCACAAGGAGGCCATCTTTTCCTCATCGGTTATTGTCCCTAGAAGCGTCTTCTGAGGATCTA GTTGGGCTTTCTTTCTGGGTTTGGGCCATTTCAGTTCTCATGTGTGTACTATTCTATCATTATTGTATAA TGGTTTTCAAACCAGTGGGCACACAGAGAACCTCAGTCTGTAATAACAATGAGGAATAGCCATGGCGATC TCCAGCACCAATCTCTCCATGTTTTCCACAGCTCCTCCAGCCAACCCAAATAGCGCCTGCTATAGTGTAG ACAGCCTGCGGCTTCTAGCCTTGTCCCTCTCTTAGTGTTCTTTAATCAGATAACTGCCTGGAAGCCTTTC ATTTTACACGCCCTGAAGCAGTCTTCTTTGCTAGTTGAATTATGTGGTGTGTTTTTCCGTAATAAGCAAA ATAAATTTAAAAAAATGAAAA |
| **26** | pyrin and HIN domain family, member 1 | >gi\|10437303\|dbj\|AK024890.1\| Homo sapiens cDNA: FLJ21237 fis, clone COL01114 |
| | | GTCAAAGGAATAATCCCATCTAAAAAGACGAAACAGAAAGAAGTGTATCCTGCTACACCTGCATGCACCC CAAGCAACCGTCTCACAGCTAAAGGAGCAGAGGAGACTCTTGGACCTCAGGTAAGCTTCAGGAAGAGGAG CAGGCTTCAAGTCTCACAGTGGAAGCTCTGCTGTGGCTGTTCCACTCAATCTGTCCAGCAGGCAGTTATT TCTTCATATGTTTCCCATCAAGTTTCAGATTTATCAAATTACATAATAATTGATCATCTTTCTGCAAGGC AACAAGTTAAACGCTTTAGTAAACATAATGTAAATATACATAAAATAAATATAATATTTTCATCTCCAAT AGAGAAGGATGTTAACTTGAGAGTCAGATAAAAAAACGTTTGCCTATGTTTACAAAAGCCTAGTTTCTTA ACTGCAAGTCAGCATATCCCAAAACACAAGTAATTAAGGAATGATGTGTTACTTTCTCTGCTCCCTTT TTAAAAATGAAACCATCTATGCCATGTTCTTTCAATTGGCCTGGGGATGTACTTAAGTTTCCAAGAAAAA CAATTTATATACAATAAATATATTACCTTGTAATGAAAATGTGCTCTGCTTCATTTGACACTGAAAGTAA TTAACAAGAAAAATAAACTACTTGTAGAAAAGAAAAAAACCATCTGAAGAAGAGACTGGAACCAAAAGGA GTAAGATGTCCAAAGAGCAGACTCGGCCTTCCTGCTCTGCAGGAGCCAGCACGTCCACAGCCATGGGCCG TTCCCCACCTCCCCAGACCTCATCATCAGCTCCACCCAACACTTCCTCAACTGAGGTACACTCTTCCTGG TCCCCTTTTGATTCATTTTCTTCAACCCAAAATGTAGGAATCTGATTTCATCTTCTACTGAAAAATGACA TCAATCATCAGCCAGTAAATCAAATGTATAGACTGAGAATTAACTGCATTTTAATCTTTTGCTTCCACAG GCATATTTGATGAACTTGACATTATCTCTGACTGCAGGAAGTTTTCTGTCCTGTGCTGTTTGGGGAAGAG ACAGAGAACTGCGGAATCTGGAACTTTCAGCAACAGACTCACTGTCTACTGCCCCCATCTATTATACACC CATTCCCTTTGCTCACTAATTTGTTCAAGTTTCTCTGACATACACCATGCTCCTTTTTCCTTTAGGATTT TCACACACCATATTTCTTTCACCTTTAAACTCTTACCTGGCCAACCCTATCCACCCTCTGGATCCCAATA TTGAGATCTTATCCTCAGGGAATCCTCACTTAGACCCCTGTAACAGGTTAAATCTTCATGGTGTTCTGTT TCCTAGGAACTTCTTTCTTTTCTACTGTTTATGACAACTGAAGTTAATAAGTGTTTATCTTTCCCACCTA CTCAAAGTAGTTCCAAGATTAGGGCTAGTTTGTAATTCTGTGGACCACTGTAAACGAGGGCCTAGTTCAG TGTCTGCCTCATGGGAAGCTTCCAATAAATACCTTTGCTCAACGAAAAAATGAAAACCCAGTGGCTCACG CCTGTAATCCCAGCACTTTGGGAGGCCGAGGCAGGTGGATTGCCTGAGGTCAGGAGTTTGAGACTAGTCT GGCCAACATGGTGAAACACTATCTCTACTAAAAATACAAAAAAATTAGCTGGGTATGGTGGCTTACGCCT ATAATCCCAGCTACTCAGGAGGCTGAGGCAGGGGAATTGCTTGAACCAGGGAGGTGGAGGTTGCAGTGAG CTGAGATCGCACCACTGCACTCCAGCCTGGGTGACAGAGCGAGACTCCATCTCAAAATAAAAAGTAAAA AAAAAAAAAAAAAA |
| **27** | T cell receptor associated transmembrane adaptor 1 | >gi\|54607136\|ref\|NM_016388.2\| Homo sapiens T cell receptor associated transmembrane adaptor 1 (TRAT1), mRNA |
| | | GAGGCACAGATAAAGATAAGTTTTACTGTCATGCTGCTTTTAACATAACAGAGCAACATCACCTAGGAAA AAAGTTTGTAGGAGGATTTTTAATCCATATATTTGTCTTATGGCTAGATAAAGATTTCTCTGAAAAAAAG AAGCATGTCAGGAATCTCTGGGTGCCCCTTTTTCCTCTGGGGACTTCTAGCATTGTTGGGCTTGGCTTTG |
| | | GTTATATCACTGATCTTCAATATTTCCCACTATGTGGAAAAGCAACGACAAGATAAAATGTACAGCTACT CCAGTGACCACACCAGGGTTGATGAGTATTATATTGAAGACACACCAATTTATGGTAACTTAGATGATAT GATTTCAGAACCAATGGATGAAAATTGCTATGAACAAATGAAAGCCCGACCAGAGAAATCTGTAAATAAG ATGCAGGAAGCCACCCCATCTGCACAGGCAACCAATGAAACACAGATGTGCTACGCCTCACTTGATCACA GCGTTAAGGGGAAGCGTAGAAAGCCCAGGAAACAGAATACTCATTTCTCAGACAAGGATGGAGATGAGCA ACTACATGCAATAGATGCCAGCGTTTCTAAGACCACCTTAGTAGACAGTTTCTCCCCAGAAAGCCAGGCA GTAGAGGAAAACATTCATGATGATCCCATCAGACTGTTTGGATTGATCCGTGCTAAGAGAGAACCTATAA ACTAGCTGGACCATGATCTAGTTCAATGATTTGGCTCCTATTGAAGATGGCTTCTAAGAAAACAAGATGC ACAGAGGACACAGAAGGACTTGGCAGCAGGGTGATGACCTGATCATTTGTTGATGGGATGGTGGCTTACC TCTTATTCACAGCTTACACTTATGCATGCCAAATGTAAGGCCATGAAAATCAGTATTTCAAATAACTTAA AAAATGCTTTACTACTAAAATGTAAAAAATTAATGTGCTCACCTCGGCAGCACATATACTAAAAATTAAT AAGACCCAGCTTGAAAATTGAGCCTGATAACAAGATTACAAATTCACAATACCTAATACTTAGGGAAATA TAAAAATTTAAGCATGAATGCGTTCTGGAACACGTTAGAAGAAAAATAAAAGCCAATGAGTTTTTTTTTA ATTCTCCTTTCTCACCAATGGGCAATAGCCCATAATTGAAATAAATTTCTGATTGAAAGGTATAGGAAAC ATTAAAATGCATTACTAAGAGAAGTAATATAATTTTCTTACAAAGTATTTTTCCCAAAGATAGCTTTACT ATTTCAAAAATTGTCAATTAATGCATGCTCCTTACAACAAACAAATATCAAAAAGAGTTTAGGAATTCT ACTAGCCAGAGATAGTCACTTGGAGAAACTTTCTATATATCCTTCTAAATATTTTTCTGGGCATGCTTAT GTATGTACATCAGTTGTTTCTTTTTATTTTGAACCAAAAATGTGGTTTCTTTTGTACACATTACTTAAAC TTTCTTTCCAGTCAACAATATATTGTGGATTTATTTTCACTGTTATATTTAACTATATATAAATACGCAT ATATTGTAATTTTAATGTCTGCTTAGCACCCCACTGATAACCAAATCACAGTTTATTTAAATAATTTTAA TGACTTTTCAAAAACAATTTATTGATGCAAAAAGCAAGGTTGAGATGACAATGTTTCTTTCAATAATTAA AAAATACTGCTTCAC |
| **28** | SLAM family member 7 | >gi\|19923571\|ref\|NM_021181.3\| Homo sapiens SLAM family member 7 (SLAMF7), mRNA |
| | | CTTCCAGAGAGCAATATGGCTGGTTCCCCAACATGCCTCACCCTCATCTATATCCTTTGGCAGCTCACAG GGTCAGCAGCCTCTGGACCCGTGAAAGAGCTGGTCGGTTCCGTTGGTGGGGCCGTGACTTTCCCCCTGAA GTCCAAAGTAAAGCAAGTTGACTCTATTGTCTGGACCTTCAACACAACCCCTCTTGTCACCATACAGCCA GAAGGGGGCACTATCATAGTGACCCAAAATCGTAATAGGGAGAGAGTAGACTTCCCAGATGGAGGCTACT CCCTGAAGCTCAGCAAACTGAAGAAGAATGACTCAGGGATCTACTATGTGGGGATATACAGCTCATCACT CCAGCAGCCCTCCACCCAGGAGTACGTGCTGCATGTCTACGAGCACCTGTCAAAGCCTAAAGTCACCATG GGTCTGCAGAGCAATAAGAATGGCACCTGTGTGACCAATCTGACATGCTGCATGGAACATGGGGAAGAGG ATGTGATTTATACCTGGAAGGCCCTGGGGCAAGCAGCCAATGAGTCCCATAATGGGTCCATCCTCCCCAT CTCCTGGAGATGGGGAGAAAGTGATATGACCTTCATCTGCGTTGCCAGGAACCCTGTCAGCAGAAACTTC |
| | | TCAAGCCCCATCCTTGCCAGGAAGCTCTGTGAAGGTGCTGCTGATGACCCAGATTCCTCCATGGTCCTCC TGTGTCTCCTGTTGGTGCCCCTCCTGCTCAGTCTCTTTGTACTGGGGCTATTTCTTTGGTTTCTGAAGAG AGAGAGACAAGAAGAGTACATTGAAGAGAAGAAGAGAGTGGACATTTGTCGGGAAACTCCTAACATATGC CCCCATTCTGGAGAGAACACAGAGTACGACACAATCCCTCACACTAATAGAACAATCCTAAAGGAAGATC CAGCAAATACGGTTTACTCCACTGTGGAAATACCGAAAAAGATGGAAAATCCCCACTCACTGCTCACGAT GCCAGACACACCAAGGCTATTTGCCTATGAGAATGTTATCTAGACAGCAGTGCACTCCCCTAAGTCTCTG CTCAAAAAAAAAACAATTCTCGGCCCAAAGAAAACAATCAGAAGAATTCACTGATTTGACTAGAAACATC AAGGAAGAATGAAGAACGTTGACTTTTTCCAGGATAAATTATCTCTGATGCTTCTTTAGATTTAAGAGT TCATAATTCCATCCACTGCTGAGAAATCTCCTCAAACCCAGAAGGTTTAATCACTTCATCCCAAAAATGG GATTGTGAATGTCAGCAAACCATAAAAAAAGTGCTTAGAAGTATTCCTATAGAAATGTAAATGCAAGGTC ACACATATTAATGACAGCCTGTTGTATTAATGATGGCTCCAGGTCAGTGTCTGGAGTTTCATTCCATCCC AGGGCTTGGATGTAAGGATTATACCAAGAGTCTTGCTACCAGGAGGGCAAGAAGACCAAAACAGACAGAC AAGTCCAGCAGAAGCAGATGCACCTGACAAAAATGGATGTATTAATTGGCTCTATAAACTATGTGCCCAG CACTATGCTGAGCTTACACTAATTGGTCAGACGTGCTGTCTGCCCTCATGAAATTGGCTCCAAATGAATG AACTACTTTCATGAGCAGTTGTAGCAGGCCTGACCACAGATTCCCAGAGGGCCAGGTGTGGATCCACAGG ACTTGAAGGTCAAAGTTCACAAAGATGAAGAATCAGGGTAGCTGACCATGTTTGGCAGATACTATAATGG AGACACAGAAGTGTGCATGGCCCAAGGACAAGGACCTCCAGCCAGGCTTCATTTATGCACTTGTGCTGCA AAAGAAAAGTCTAGGTTTTAAGGCTGTGCCAGAACCCATCCCAATAAAGAGACCGAGTCTGAAGTCACAT TGTAAATCTAGTGTAGGAGACTTGGAGTCAGGCAGTGAGACTGGTGGGCACGGGGGCAGTGGGTACTT GTAAACCTTTAAAGATGGTTAATTCATTCAATAGATATTTATTAAGAACCTATGCGGCCCGGCATGGTGG CTCACACCTGTAATCCCAGCACTTTGGGAGGCCAAGGTGGGTGGGTCATCTGAGGTCAGGAGTTCAAGAC CAGCCTGGCCAACATGGTGAAACCCCATCTCTACTAAAGATACAAAAATTTGCTGAGCGTGGTGGTGTGC ACCTGTAATCCCAGCTACTCGAGAGGCCAAGGCATGAGAATCGCTTGAACCTGGGAGGTGGAGGTTGCAG TGAGCTGAGATGGCACCACTGCACTCCGGCCTAGGCAACGAGAGCAAAACTCCAATACAAACAAACAAAC AAACACCTGTGCTAGGTCAGTCTGGCACGTAAGATGAACATCCCTACCAACACAGAGCTCACCATCTCTT ATACTTAAGTGAAAAACATGGGGAAGGGGAAAGGGGAATGGCTGCTTTTGATATGTTCCCTGACACATAT CTTGAATGGAGACCTCCCTACCAAGTGATGAAAGTGTTGAAAAACTTAATAACAAATGCTTGTTGGGCAA GAATGGGATTGAGGATTATCTTCTCTCAGAAAGGCATTGTGAAGGAATTGAGCCAGATCTCTCTCCCTAC TGCAAAACCCTATTGTAGTAAAAAAGTCTTCTTTACTATCTTAATAAACAGATATTGTGAGATTCAAAA AAAAAAAAAAAA |
| **29** | chromosome 4 open reading frame 7 | >gi\|50428928\|ref\|NM_152997.2\| Homo sapiens chromosome 4 open reading frame 7 (C4orf7), mRNA |
| | | TAAAACAGCTACAATATTCCAGGGCCAGTCACTTGCCATTTCTCATAACAGCGTCAGAGAGAAAGAACTG |
| | | ACTGAAACGTTTGAGATGAAGAAAGTTCTCCTCCTGATCACAGCCATCTTGGCAGTGGCTGTTGGTTTCC CAGTCTCTCAAGACCAGGAACGAGAAAAAAGAAGTATCAGTGACAGCGATGAATTAGCTTCAGGGTTTTT TGTGTTCCCTTACCCATATCCATTTCGCCCACTTCCACCAATTCCATTTCCAAGATTTCCATGGTTTAGA CGTAATTTTCCTATTCCAATACCTGAATCTGCCCCTACAACTCCCCTTCCTAGCGAAAAGTAAACAAGAA GGAAAAGTCACGATAAACCTGGTCACCTGAAATTGAAATTGAGCCACTTCCTTGAAGAATCAAAATTCCT GTTAATAAAAGAAAAACAAATGTAATTGAAATAGCACACAGCATTCTCTAGTCAATATCTTTAGTGATCT TCTTTAATAAACTTGAAAGCAAAGATTTTGGTTTCTTAATTTCCACAAA |
| **30** | Major histocompatibility complex, class II, DQ alpha 1 | >gi\|52426772\|ref\|NM_002122.3\| Homo sapiens major histocompatibility complex, class II, DQ alpha 1 (HLA-DQA1), mRNA |
| | | ACAATTACTCTACAGCTCAGAACACCAACTGCTGAGGCTGCCTTGGGAAGAGGATGATCCTAAACAAAGC TCTGCTGCTGGGGGCCCTCGCTCTGACCACCGTGATGAGCCCCTGTGGAGGTGAAGACATTGTGGCTGAC CACGTTGCCTCTTGTGGTGTAAACTTGTACCAGTTTTACGGTCCCTCTGGCCAGTACACCCATGAATTTG ATGGAGATGAGCAGTTCTACGTGGACCTGGAGAGGAAGGAGACTGCCTGGCGGTGGCCTGAGTTCAGCAA ATTTGGAGGTTTTGACCCGCAGGGTGCACTGAGAAACATGGCTGTGGCAAAACACAACTTGAACATCATG ATTAAACGCTACAACTCTACCGCTGCTACCAATGAGGTTCCTGAGGTCACAGTGTTTTCCAAGTCTCCCG TGACACTGGGTCAGCCCAACACCCTCATTTGTCTTGTGGACAACATCTTTCCTCCTGTGGTCAACATCAC ATGGCTGAGCAATGGGCAGTCAGTCACAGAAGGTGTTTCTGAGACCAGCTTCCTCTCCAAGAGTGATCAT TCCTTCTTCAAGATCAGTTACCTCACCTTCCTCCCTTCTGCTGATGAGATTTATGACTGCAAGGTGGAGC ACTGGGGCCTGGACCAGCCTCTTCTGAAACACTGGGAGCCTGAGATTCCAGCCCCTATGTCAGAGCTCAC AGAGACTGTGGTCTGTGCCCTGGGGTTGTCTGTGGGCCTCATGGGCATTGTGGTGGGCACTGTCTTCATC ATCCAAGGCCTGCGTTCAGTTGGTGCTTCCAGACACCAAGGGCCATTGTGAATCCCATCCTGGAAGGGAA GGTGCATCGCCATCTACAGGAGCAGAAGAATGGACTTGCTAAATGACCTAGCACTATTCTCTGGCCCGAT TTATCATATCCCTTTTCTCCTCCAAATATTTCTCCTCTCACCTTTTCTCTGGGACTTAAGCTGCTATATC CCCTCAGAGCTCACAAATGCCTTTACATTCTTTCCCTGACCTCCTGATTTTTTTTTTCTTTTCTCAAATG TTACCTACAAAGACATGCCTGGGGTAAGCCACCCGGCTACCTAATTCCTCAGTAACCTCCATCTAAAATC TCCAAGGAAGCAATAAATTCCTTTTATGAGATCTATGTCAAATTTTTCCATCTTTCATCCAGGGCTGACT GAAACTATGGCTAATAATTGGGGTACTCTTATGTTTCAATCCAATTTAACCTCATTTCCCAGATCATTTT TCATGTCCAGTAACACAGAAGCCACCAAGTACAGTATAGCCTGATAATATGTTGATTTCTTAGCTGACAT TAATATTTCTTGCTTCCTTGTGTTCCCACCCTTGGCACTGCCACCCACCCCTCAATTCAGGCAACAATGA AATTAATGGATACCGTCTGCCCTTGGCCCAGAATTGTTATAGCAAAAATTTAGAACCAAAAAATAAGTC TGTACTAATTTCAATGTGGCTTTTAAAAGTATGACAGAGAAATAAGTTAGGATAAAGGAAATTTGAATCT CA |
| **31** | Transcribed locus | >gi\|3807951\|gb\|AI225238.1\|AI225238 qx12c04.x1 NCI_CGAP_Lym12 Homo sapiens cDNA clone IMAGE:2001126 3', mRNA sequence |
| | | AGCGGCCGCCCTTTTTTTTTTTTTTTGAATGTTTTTATATTTTTATGTTTTTCTCTCTCACCACATTAGA GATTCACATGTCAAAAAAAAAGTTCAAAGATTCCACTTAAATGGTAGTCAAAATTAAAAGCATGTTGTTT AATTTGCCAACTATTCCATAAATCCTGTGCTTGAAGCACTACCTTTTATCAACCACTTGAAAGGAAGGAA TATGAAGTGTGACTCTTGAGGATTGCAGTTCCATTTCTCCACAGACATAGCAGGGTGTGTAGTTCCAAAT GGGTTTCTTCCAAAGTCATCTCCTCAGAGTAGGACTGTCTGAGTAGTCATTCTGATAATGTTGCCATTAA CTACAAGCACATCTGAGAACCCTCGTAAGGAAGTGCCATTAGATTCAGA |
| **32** | Amphiphysin (Stiff-Man syndrome with breast cancer 128kDa autoantigen) | >gi\|51466133\|ref\|XM_499518.1\| PREDICTED: Homo sapiens LOC442534 (LOC442534), mRNA |
| | | GGCCCAGCCCTAGGAGGGGGCAGTCCCACAGCAGGCTCACACCCTGCCCTTCAGAGTTGCAGCAGCTAAT GCTGATGCCAGGACCTTGTCCTGCCAGGAAGCCTCTGATTAGGGCTGTGAACCAACTTCCTTTTCACATC TGCAAGGACTCTGCTCTAAGCTCAAATTTTGCAGTTTCTGCTGTCATCACAGCTGGGAATGAGAAAGGGA AGGGGCTCCAGTTGGTCTCCCTCTGCCTCTGGAGCACACGTGGAGGACATGAGGCTTCCGCAGGAACAGC TCTGAAAGCTGAGTTCTCGGGTCCCTGTGAAGGAGGGAAGCTTGCCTGCCTTGGAGCGTGAAAAAGTCTT GCTGGACAGAAGTCTCACGGTTTAAAACTGGGAAGGGAGAGGATTGAGATCACACTAGAATGCACAGAAA ATTCTT |
| **33** | dendritic cell-associated lectin-1 | >gi\|40548404\|ref\|NM_172004.2\| Homo sapiens dendritic cell-associated lectin-1 (DCAL1), mRNA |
| | | ATGGTTAGTAATTTCTTCCATGTCATACAAGTATTCGAGAAATCTGCTACCTTGATTAGTAAGACTGAAC ACATTGGTTTTGTCATTTATTCATGGAGGAAGTCCACCACCCACTTGGGGAGCAGAAGGAAATTTGCCAT CTCAATTTACTTATCAGAAGTTTCTTTGCAGAAATATGATTGTCCCTTCAGTGGGACATCATTTGTGGTC TTCTCTCTCTTTTTGATCTGTGCAATGGCTGGAGATGTAGTCTACGCTGACATCAAAACTGTTCGGACTT CCCCGTTAGAACTCGCGTTTCCACTTCAGAGATCTGTTTCTTTCAACTTTTCTACTGTCCATAAATCATG TCCTGCCAAAGACTGGAAGGTGCATAAGGGAAAATGTTACTGGATTGCTGAAACTAAGAAATCTTGGAAC AAAAGTCAAAATGACTGTGCCATAAACAATTCATATCTCATGGTGATTCAAGACATTACTGCTATGGTGA GATTTAACATTTAGAGGTGACAGCATCCCCCACACTGGCAGTGAATTTTTTGTGCTACAAACTTGGCAAA AGTCTGTGAAAAGAAGTTTCAACTTCATGTGTTATTAACTATACAAATATTAGTTGAATGAATTGTTGAA TTACAAAAAAAAAAAAAAAA |

**Table 1B Probes for identifying genes of Table 1 (and their fold upregulation in Mage cancer patients)**

| **Probe set ID** | **Gene name** | **Probeset sequence** | **Full gene sequence** | **Fold change** |
|---|---|---|---|---|
| 155897 2_s_at | chromosome 6 open reading frame 190 | ttctaagacccacatttggttatt gaaggccacagcgaatcttaacct aacagccttgac aaactgcaccataggtgtttttag actcatataatttgttatttttca aacaatagtgaa taattaatattnttgtttggaatt tgagnacaattaaatttgtacttt tagtaactacca ttctttgattagaaaattaagaga atgcatatcttactttggttgtaa attatcaagggc tttctaatagaaatcatatataac atttctaaatataagtcctttcac atactgtgtttc cagttgtcttgatattgaaaagtg taataaacttcatgctcacctat [Seq ID No 49] | >gi\|58218985\|ref\|NM_001010923.1\| Homo sapiens chromosome 6 open reading frame 190 (C6orf190), mRNA | 44.93 |
| | | | ATACTTACAATTACGAGATTTATATTTGCATTAGTCTCTTTGGCTGG TGGGTAGGGGTGAGAGGCTCTTC CTGGATCCCTTATTTTCTACAGGAGAGGAGGAAAACACCTGGGATGC TCCAGTGCTCTTACGCAGATAAT GATCATTAACATCAGCCTCTCTGATCAAAGAGCTACTCCACCCCACT CTGGCTGTAGTGTGACATTCCTG CCTGCCTGAGGAAGAAATGGTGAGCAGGGGCTGCAATTGCAGACAAG TGTCACCCAGAAGCCACAAGTTT CTGTGAGCACCAGGTCTACAAACTACCCAAGGCATAGCAATGGCATT ATCACTGGAAGAATTCGTCCACT CCCTTGACCTCAGGACCCTACCCAGGGTTCTAGAAATCCAGGCAGGC ATCTATCTTGAAGGCTCTATTTA TGAAATGTTTGGAAATGAATGCTGTTTTTCAACAGGAGAAGTGATTA AAATTACTGGTCTCAAAGTTAAG AAGATCATAGCTGAAATTTGTGAGCAGATTGAAGGTTGTGAGTCTCT ACAGCCATTTGAACTGCCTATGA ATTTTCCAGGTCTTTTTAAGATTGTGGCTGATAAAACTCCATACCTT ACTATGGAAGAAATCACAAGGAC CATTCATATTGGACCAAGTAGACTAGGGCATCCTTGCTTCTATCATC AGAAGGATATAAAACTAGAGAAC CTCATCATAAAGCAGGGTGAGCAAATCATGCTCAACTCAGTTGAAGA GATTGATGGAGAAATAATGGTGA GCTGTGCAGTAGCAAGGAATCATCAAACTCACTCATTTAATTTGCCT TTGTCACAAGAAGGAGAATTCTA CGAGTGTGAAGATGAACGTATTTACACTCTAAAGGAGATTGTTGAAT | |
| | | | GGAAGATTCCTAAGAACAGAACA AGAACTGTAAACCTTACAGATTTTTCAAATAAGTGGACTCAACGAA TCCATTTCCTAAAGACTTTTATG GTACCCTGATTCTCAAGCCTGTTTATGAAATTCAAGGTGTGATGAAA TTTCGAAAAGATATAATCCGCAT CCTCCCCAGTCTAGATGTCGAAGTCAAAGACATCACTGATTCTTACG ATGCTAACTGGTTTCTTCAGCTG TTATCAACAGAAGATCTTTTTGAAATGACTAGTAAAGAGTTCCCCAT AGTGACTGAAGTCATAGAAGCAC CTGAAGGAAACCACCTGCCCCAAAGCATTTTACAGCCTGGGAAAACC ATTGTGATCCACAAAAAGTACCA GGCATCAAGAATCTTAGCTTCAGAAATTAGAAGCAATTTTCCTAAAA GACACTTCTTGATCCCCACTAGC TATAAAGGCAAGTTCAAGCGGCGACCGAGGGAGTTCCCAACGGCCTA TGACCTAGAGATCGCTAAGAGTG AAAAGGAGCCTCTTCACGTGGTGGCCACCAAAGCGTTTCATTCCCCT CATGACAAGCTGTCATCCGTATC TGTTGGGGACCAGTTTCTGGTGCATCAGTCAGAGACGACTGAAGTCC TCTGTGAGGGAATAAAAAAAGTG GTGAATGTTCTGGCCTGTGAAAAAATCCTCAAAAAGTCCTATGAGGC TGCGCTGCTCCCTTTGTACATGG AAGGAGGTTTTGTAGAGGTGATTCATGATAAGAAACAGTACCCGATT TCTGAGCTCTGTAAACAGTTCCG TTTGCCCTTCAATGTGAAGGTGTCTGTCAGGGATCTTTCCATTGAAG AGGACGTGTTGGCTGCCACACCA GGACTGCAGTTGGAGGAGGACATTACAGACTCTTACCTACTCATAAG TGACTTTGCCAACCCCACGGAGT GCTGGGAAATTCCTGTGGGCCGCTTGAATATGACTGTTCAGTTAGTT AGTAATTTCTCTAGGGATGCAGA ACCATTTCTAGTCAGGACTCTGGTAGAAGAGATCACTGAAGAGCAAT ATTACATGATGCGGAGATATGAA AGCTCAGCCTCACATCCCCCACCTCGCCCTCCGAAACACCCCTCAGT AGAGGAAACAAAGTTAACCCTGC TAACCTTAGCAGAAGAAAGGACGGTAGACCTGCCCAAGTCTCCCAAG | |
| | | | CGTCATCACGTAGACATAACCAA GAAACTTCACCCAAATCAAGCTGGCCTGGATTCAAAAGTACTGATTG GTAGTCAGAATGATTTGGTGGAT GAAGAGAAAGAAAGGAGCAACCGTGGGGCCACAGCAATAGCAGAAAC ATTCAAAAATGAAAAACATCAAA AATAACAAGATGTGACAGAAGCCACTTAGGCAGCAAACATAAATGTT GCAGTGAAAAAAGAAGCTAGCCT TCTAGCTGAAAAACGAGTATTCCCCAATGGACTCCAGAAGAAACTTG ATTCATCGCTGCAAAGGAAAGAA CAACCTTAAAACTTTTAACAGATAAAACTTACAGAAACCTATGATAT AGAATTCATATAGTCTATTCTGT TGTGTCTAAATCTGTAGGCATTGTGTTGTTGTTCTTTAGGACGTATT TATTTAACTTGCACATTTTTTCA GATTCTTATTTCTACTACCAACAACTAAGTAATTGGGAAATAATTCT GTATTTCAGTTTCTGAGTAAAAC CAGTCTGAAATAGGATAAAAGCCACCAAATATTTTCTTTTTTTTCCA GAATTTGTTTTGCCATTTTTTAG TGCTATCATCATTCCTAACAAGACTAACTTACAGAAAAATAATTATA TCTGACTGATTTAAAATGTTCAG GTTTCTTATCCAAATCCCTTGGAACTATGGAAAGGAGTTTGATTTCA CATTCACAGTGTATTTACAAAAT ACGCTGTGTCATAAATATGTTTGAATTCCAACAGCCAAAGCCATTGA GAGTCATAGGAGTTTTCCATAAC CTTCTCTTCTATGACCCAACAACAAGCTCATGACTGAAATTTCACCA GATTTCTGAGACGATGTCTTAAT ATTCTATGTGCTATGTACCAGATAATTCTTTAGATGAATGTTTCTTA GGATTGTAGGAAAATTATCTAGT TAATCATAATATTTGATGGAAAGAAAAAGACAATAAAATTGTAATAT AATAAATTTGGCTGACAAGAAAC CAAAGTGATTCTTAATTAGTATACATCAGAATGATGCTCTTATAGTT GTACCATCTATAAAAATTACTTT AAGGGCTCTCACATTTTAATAATTTATCTTATTATGTATTAAGTATA CAGGAACAATATTATTTTTCCTT TAACAAAATGAAGAGACAGGCTATCTGGTTAATGTTACATAGGAATT | |
| | | | TAATAGTAATGCTTGAACTTCAT CCATAGATCATACTCTGTACAAAATTTGTTAGCTAACATCCTATCTC ATAATTATTTTATGTTTTGTGGA GAAATTTGTTGATTTTGTACCAAAGTGTTTCTGAAGACAATAAATTG TGAGTCAACTTTAGAACAAAAAA AATTAGAGTTTTTTCAATGTTTATATTCTGATTAAGCTTACTTTACC TTACATTTTTTCTAAGTAACAAT GAATCCTGATTTCTAGTGTCCTAAAAATTGCTTAGTGATTTGATTGT GGTAATATCATTTCTTATCTACA ATGTCTAAAGTTTTATGGGACAGTTTTCTTTTATTTATTTTGCCTG TTTGTGCAGATAAGAACAGAAAC TTTTCTAAGACCCACATTTGGTTATTGAAGGCCACAGCGAATCTTAA CCTAACAGCCTTGACAAACTGCA CCATAGGTGTTTTTAGACTCATATAATTTGTTATTTTTCAAACAATA GTGAATAATTAATATTTTTGTTT GGAATTTGAGAACAATTAAATTTGTACTTTTAGTAACTACCATTCTT TGATTAGAAAATTAAGAGAATGC ATATCTTACTTTGGTTGTAAATTATCAAGGGCTTTCTAATAGAAATC ATATATAACATTTCTAAATATAA GTCCTTTCACATACTGTGTTTCCAGTTGTCTTGATATTGAAAAGTGT AATAAACTTCATGCTCACCTATT GGAGATTTGGGAAGGTTGAAAATAAACTTCCTAATTTTAAAAAAAA AA | |
| 202957 _at | hematopoietic cell-specific Lyn substrate 1 | ttgctggcttctcttatactaacc cagagtttgtcattaatgtgtagg tgaatgcaaact ccatcgttgagcctggggtgtaag acttcaagccaagcgtatgtatca attctagtcttc caggattcacggtgcacatgctgg cattcaacatgtggaaagcttgtc ttagaggccttt cttgtatgtgtagcttgctagttt | >gi\|37059786\|ref\|NM_005335.3 Homo sapiens hematopoietic cell-specific Lyn substrate 1 (HCLS1), mRNA | 19.09 |
| | | | GTGGACGCGAGGAGCCGGGCGCTTAGAACAGAGGCTTGCACAGGTGG AGATGTGGAAGTCTGTAGTGGGC CATGATGTGTCTGTTTCCGTGGAGACCCAGGGTGATGATTGGGACAC AGATCCTGACTTTGTGAATGACA TCTCTGAAAAGGAGCAACGATGGGGAGCCAAGACCATCGAGGGGTCT GGACGCACAGAACACATCAACAT CCACCAGCTGAGGAACAAAGTATCAGAGGAGCATGATGTTCTCAGGA | |
| | | gttttctacatttgaaaatgttta gtttagaataag cgcattatccaattatagaggtac aattttccaaacttccagaaactc atcaaatgaaca gacaatgtcaaaactactgtgtct gataccaaaatgcttcagtatttg taatttttcaag tcagaagctgatgttcctggtaaa agtt [Seq ID No 50] | AGAAAGAGATGGAGTCAGGGCCC AAAGCATCCCATGGCTATGGAGGTCGGTTTGGAGTAGAAAGAGACCG AATGGACAAGAGTGCAGTGGGCC ATGAGTATGTTGCCGAGGTGGAGAAGCACTCTTCTCAGACGGATGCT GCCAAAGGCTTTGGGGGCAAGTA CGGAGTTGAGAGGGACAGGGCAGACAAGTCAGCAGTCGGCTTTGATT ATAAAGGAGAAGTGGAGAAGCAT ACATCTCAGAAAGATTACTCTCGTGGCTTTGGTGGCCGGTACGGGGT GGAGAAGGATAAATGGGACAAAG CAGCTCTGGGATATGACTACAAGGGAGAGACGGAGAAACACGAGTCC CAGAGAGATTATGCCAAGGGCTT TGGTGGCCAGTATGGAATCCAGAAGGACCGAGTGGATAAGAGCGCTG TCGGCTTCAATGAAATGGAGGCC CCGACCACAGCTTATAAGAAGACGACGCCCATAGAAGCCGCTTCTAG TGGTGCCCGTGGGCTGAAGGCGA AATTTGAGTCCATGGCTGAGGAGAAGAGGAAGCGAGAGGAAGAGGAG AAGGCACAGCAGGTGGCCAGGAG GCAACAGGAGCGAAAGGCTGTGACAAAGAGGAGCCCTGAGGCTCCAC AGCCAGTGATAGCTATGGAAGAG CCAGCAGTACCGGCCCCACTGCCCAAGAAAATCTCCTCAGAGGCCTG GCCTCCAGTTGGGACTCCTCCAT CATCAGAGTCTGAGCCTGTGAGAACCAGCAGGGAACACCCAGTGCCC TTGCTGCCCATTAGGCAGACTCT CCCGGAGGACAATGAGGAGCCCCCAGCTCTGCCCCCTAGGACTCTGG AAGGCCTCCAGGTGGAGGAAGAG CCAGTGTACGAAGCAGAGCCTGAGCCTGAGCCCGAGCCTGAGCCCGA GCCTGAGAATGACTATGAGGACG TTGAGGAGATGGACAGGCATGAGCAGGAGGATGAACCAGAGGGGGAC TATGAGGAGGTGCTCGAGCCTGA AGATTCTTCTTTTTCTTCTGCTCTGGCTGGATCATCAGGCTGCCCGG CTGGGGCTGGGGCTGGGGCTGTG GCTCTGGGGATCTCAGCTGTGGCTCTATATGATTACCAAGGAGAGGG AAGTGATGAGCTTTCCTTTGATC CGGACGACGTAATCACTGACATTGAGATGGTGGACGAGGGCTGGTGG | |
| | | | CGGGGACGTTGCCATGGCCACTT TGGACTCTTCCCTGCAAATTATGTCAAGCTTCTGGAGTGACTAGAGC TCACTGTCTACTGCAACTGTGAT TTCCCATGTCCAAAGTGGCTCTGCCTCCACCCCCTCCCTATTCCTGA TGCAAATGTCTAACCAGATGAGT TTCTGGACAGACTTCCCTCTCCTGCTTCATTAAGGGCTTGGGGCAGA GACAGCATGGGGAAGGAGGTCCC CTTCCCCAAGAGTCCTCTCTATCCTGGATGAGCTCATGAACATTTCT CTTGTGTTCCTGACTCCTTCCCA ATGAACACCTCTCTGCCACCCCAAGCTCTGCTCTCCTCCTCTGTGAG CTCTGGGCTTCCCAGTTTGTTTA CCCGGGAAAGTACGTCTAGATTGTGTGGTTTGCCTCATTGTGCTATT TGCCCACTTTCCTTCCCTGAAGA AATATCTGTGAACCTTCTTTCTGTTCAGTCCTAAAATTCGAAATAAA GTGAGACTATGGTTCACCTGTAA AAAAAAAAAAAAAAAAAAAAAAAAAAAAAAAAAAAAAAAAAAAAAAA AAAAAAAAAAAAAAAAAAAAAAA | |
| 204116 _at | interleukin 2 receptor, gamma (severe combined immunodeficiency) | ttctggctggaacggacgatgccc cgaattcccaccctgaagaaccta gaggatcttgtt actgaataccacggaacttttcg gcctggagtggtgtgtctaaggga ctggctgagagtctgcagccagac tacagtgaacgactctgcctcgtc agtgagattcccccaaaggaggg gcccttggggagggcctgggcc tccccatgcaaccagcatagcccc tactgggcccccccatgttacacc ctaaagcctgaaacctgaacccca atcctctgacagaagaaccccagg gtcctgtagccctaagtggtacta actttccttcattcaacccacctg cgtctcatactcacctcaccccac tgtggctgatttggaattttgtgc | >gi\|4557881\|ref\|NM_000206.1\| Homo sapiens interleukin 2 receptor, gamma (severe combined immunodeficiency) (IL2RG), mRNA | 61.98 |
| | | | GAAGAGCAAGCGCCATGTTGAAGCCATCATTACCATTCACATCCCTC TTATTCCTGCAGCTGCCCCTGCT GGGAGTGGGGCTGAACACGACAATTCTGACGCCCAATGGGAATGAAG ACACCACAGCTGATTTCTTCCTG ACCACTATGCCCACTGACTCCCTCAGTGTTTCCACTCTGCCCCTCCC AGAGGTTCAGTGTTTTGTGTTCA ATGTCGAGTACATGAATTGCACTTGGAACAGCAGCTCTGAGCCCCAG CCTACCAACCTCACTCTGCATTA TTGGTACAAGAACTCGGATAATGATAAAGTCCAGAAGTGCAGCCACT ATCTATTCTCTGAAGAAATCACT TCTGGCTGTCAGTTGCAAAAAAAGGAGATCCACCTCTACCAAACATT TGTTGTTCAGCTCCAGGACCCAC GGGAACCCAGGAGACAGGCCACACAGATGCTAAAACTGCAGAATCTG GTGATCCCCTGGGCTCCAGAGAA | |
| | | ccccatgtaagcacc [Seq ID No 51] | CCTAACACTTCACAAACTGAGTGAATCCCAGCTAGAACTGAACTGGA ACAACAGATTCTTGAACCACTGT TTGGAGCACTTGGTGCAGTACCGGACTGACTGGGACCACAGCTGGAC TGAACAATCAGTGGATTATAGAC ATAAGTTCTCCTTGCCTAGTGTGGATGGGCAGAAACGCTACACGTTT CGTGTTCGGAGCCGCTTTAACCC ACTCTGTGGAAGTGCTCAGCATTGGAGTGAATGGAGCCACCCAATCC ACTGGGGGAGCAATACTTCAAAA GAGAATCCTTTCCTGTTTGCATTGGAAGCCGTGGTTATCTCTGTTGG CTCCATGGGATTGATTATCAGCC TTCTCTGTGTGTATTTCTGGCTGGAACGGACGATGCCCCGAATTCCC ACCCTGAAGAACCTAGAGGATCT TGTTACTGAATACCACGGGAACTTTTCGGCCTGGAGTGGTGTGTCTA AGGGACTGGCTGAGAGTCTGCAG CCAGACTACAGTGAACGACTCTGCCTCGTCAGTGAGATTCCCCCAAA AGGAGGGGCCCTTGGGGAGGGGC CTGGGGCCTCCCCATGCAACCAGCATAGCCCCTACTGGGCCCCCCCA TGTTACACCCTAAAGCCTGAAAC CTGAACCCCAATCCTCTGACAGAAGAACCCCAGGGTCCTGTAGCCCT AAGTGGTACTAACTTTCCTTCAT TCAACCCACCTGCGTCTCATACTCACCTCACCCCACTGTGGCTGATT TGGAATTTTGTGCCCCCATGTAA GCACCCCTTCATTTGGCATTCCCCACTTGAGAATTACCCTTTTGCCC CGAACATGTTTTTCTTCTCCCTC AGTCTGGCCCTTCCTTTTCGCAGGATTCTTCCTCCCTCCCTCTTTCC CTCCCTTCCTCTTTCCATCTACC CTCCGATTGTTCCTGAACCGATGAGAAATAAAGTTTCTGTTGATAAT CATC | |
| 204661 _at | CD52 antigen (CAMPATH-1 antigen) | acagccacgaagatcctaccaaaa tgaagcgcttcctcttcctcctac tcaccatcagcc tcctggttatggtacagatacaaa ctggactctcaggacaaaacgaca | >gi\|68342029\|ref\|NM_001803.2\| Homo sapiens CD52 molecule (CD52), mRNA | 30.71 |
| | | | CTCCTGGTTCAAAAGCAGCTAAACCAAAAGAAGCCTCCAGACAGCCC TGAGATCACCTAAAAAGCTGCTA CCAAGACAGCCACGAAGATCCTACCAAAATGAAGCGCTTCCTCTTCC | |
| | /// CD52 antigen (CAMPATH-1 antigen) | ccagccaaaccagcagcccctcag catccagcagcatgagcggaggca ttttccttttcttcgtggccaatg ccataatccacctcttctgcttca gttgaggtgacacgtctcagcctt agccctgtgccc cctgaaacagctgccaccatcact cgcaagagaatcccctccatcttt gggaggggttgatgccagacatca ccaggttgtagaagttgacaggca gtgccatgggggcaacagccaaaa tagggggtaatgatgtaggggcc aagc [Seq ID No 52] | TCCTACTCACCATCAGCCTCCTG GTTATGGTACAGATACAAACTGGACTCTCAGGACAAAACGACACCAG CCAAACCAGCAGCCCCTCAGCAT CCAGCAACATAAGCGGAGGCATTTTCCTTTTCTTCGTGGCCAATGCC ATAATCCACCTCTTCTGCTTCAG TTGAGGTGACACGTCTCAGCCTTAGCCCTGTGCCCCCTGAAACAGCT GCCACCATCACTCGCAAGAGAAT CCCCTCCATCTTTGGGAGGGGTTGATGCCAGACATCACCAGGTTGTA GAAGTTGACAGGCAGTGCCATGG GGGCAACAGCCAAAATAGGGGGGTAATGATGTAGGGGCCAAGCAGTG CCCAGCTGGGGGTCAATAAAGTT ACCCTTGTACTTGCAAAAAAAAAAAAAAAAAA | |
| 205831 _at | CD2 antigen (p50), sheep red blood cell receptor /// CD2 antigen (p50), sheep red blood cell receptor | agacctcgagttcagccaaaacct ccccatgggcagcagaaaactca ttgtccccttcctctaattaaaaa agatagaaactgtctttttcaata aaaagcactgtggatttctgccct cctgatgtgcatatccgtacttcc atgaggtgttttctgtgtgcagaa cattgtcacctc ctgaggctgtgggccacagccacc tctgcatcttcgaactcagccatg tggtcaacatct ggagtttttggtctcctcagagag ctccatcacaccagtaaggagaag caatataagtgt gattgcaagaatggtagaggaccg agcacagaaatcttagagatttct tgtcccctctca ggtcatgtgtagatgcgataaatc | >gi\|31542293\|ref\|NM_001767.2\| Homo sapiens CD2 molecule (CD2), mRNA | 22.66 |
| | | | ACCAACCCCTAAGATGAGCTTTCCATGTAAATTTGTAGCCAGCTTCC TTCTGATTTTCAATGTTTCTTCC AAAGGTGCAGTCTCCAAAGAGATTACGAATGCCTTGGAAACCTGGGG TGCCTTGGGTCAGGACATCAACT TGGACATTCCTAGTTTTCAAATGAGTGATGATATTGACGATATAAAA TGGGAAAAAACTTCAGACAAGAA AAAGATTGCACAATTCAGAAAAGAGAAAGAGACTTTCAAGGAAAAAG ATACATATAAGCTATTTAAAAAT GGAACTCTGAAAATTAAGCATCTGAAGACCGATGATCAGGATATCTA CAAGGTATCAATATATGATACAA AAGGAAAAAATGTGTTGGAAAAAATATTTGATTTGAAGATTCAAGAG AGGGTCTCAAAACCAAAGATCTC CTGGACTTGTATCAACACAACCCTGACCTGTGAGGTAATGAATGGAA CTGACCCCGAATTAAACCTGTAT CAAGATGGGAAACATCTAAAACTTTCTCAGAGGGTCATCACACACAA GTGGACCACCAGCCTGAGTGCAA | |
| | | aagtgattggtgtgcctgggtctc actacaagcagc ctatctgc [Seq ID No 53] | AATTCAAGTGCACAGCAGGGAACAAAGTCAGCAAGGAATCCAGTGTC GAGCCTGTCAGCTGTCCAGAGAA AGGTCTGGACATCTATCTCATCATTGGCATATGTGGAGGAGGCAGCC TCTTGATGGTCTTTGTGGCACTG CTCGTTTTCTATATCACCAAAAGGAAAAAACAGAGGAGTCGGAGAAA TGATGAGGAGCTGGAGACAAGAG CCCACAGAGTAGCTACTGAAGAAAGGGGCCGGAAGCCCCAACAAATT CCAGCTTCAACCCCTCAGAATCC AGCAACTTCCCAACATCCTCCTCCACCACCTGGTCATCGTTCCCAGG CACCTAGTCATCGTCCCCCGCCT CCTGGACACCGTGTTCAGCACCAGCCTCAGAAGAGGCCTCCTGCTCC GTCGGGCACACAAGTTCACCAGC AGAAAGGCCCGCCCCTCCCCAGACCTCGAGTTCAGCCAAAACCTCCC CATGGGGCAGCAGAAAACTCATT GTCCCCTTCCTCTAATTAAAAAAGATAGAAACTGTCTTTTTCAATAA AAAGCACTGTGGATTTCTGCCCT CCTGATGTGCATATCCGTACTTCCATGAGGTGTTTTCTGTGTGCAGA ACATTGTCACCTCCTGAGGCTGT GGGCCACAGCCACCTCTGCATCTTCGAACTCAGCCATGTGGTCAACA TCTGGAGTTTTTGGTCTCCTCAG AGAGCTCCATCACACCAGTAAGGAGAAGCAATATAAGTGTGATTGCA AGAATGGTAGAGGACCGAGCACA GAAATCTTAGAGATTTCTTGTCCCCTCTCAGGTCATGTGTAGATGCG ATAAATCAAGTGATTGGTGTGCC TGGGTCTCACTACAAGCAGCCTATCTGCTTAAGAGACTCTGGAGTTT CTTATGTGCCCTGGTGGACACTT GCCCACCATCCTGTGAGTAAAAGTGAAATAAAAGCTTTGACTAGAAA AAAAAAAAAAAAAAAAAAAAAAA AAAAAAAAAAAAAAAAAAAAAAAAAAAAAAAAAAAAAAAAAA | |
| 205890 _s_at | ubiquitin D | Gatcttaaagccacggagaagcct ctcatcttatggcattgacaaaga gaagaccatccaccttaccctgaa agtggtgaagcccagtgatgagga gctgcccttgtttcttgtggagtc | >gi\|50355987\|ref\|NM_006398.2\| Homo sapiens ubiquitin D (UBD), mRNA | 30.29 |
| | | | GATTGCTTGAGGAGAGAAGTATGTGATCAGAAAGCATTCTTTGTCTA TTAACTCCTGCCCAGCAAAAGTG AAAGAAAATTCATGGGAGCATGCAAGAACAAAGAGCACAGCAAAGCT | |
| | | aggtgatgaggcaaagaggcacct cctccaggtgcgaaggtccagctc agtggcacaagtgaaagcaatgat cgagactaagacgggtataatccc tgagacccagattgtgacttgcaa tggaaagagactggaagatgggaa gatgatggcagattacggcatcag aaagggcaacttactcttcctggc atcttattgtattggaggtgacc accctggggatggggtgttggcag gggtcaaaaagcttatttctttta atctcttactcaacgaacacatct tctgatgatttcccaaaattaatg agaatgagatgagtagagtaagat ttgggtgggatgggtaggatgaag tatattgcccaactctatgtttct ttga [Seq ID No 54] | GGACAAACACAGCAATCCAGGCA GGGGATTTCCAACTCAACTCTGGTATGTAAGCTGCATGCAAAGTCCT TTTTCTGTCTCTGGTTTCTGGCC CCTTGTCTGCAGAGATGGCTCCCAATGCTTCCTGCCTCTGTGTGCAT GTCCGTTCCGAGGAATGGGATTT AATGACCTTTGATGCCAACCCATATGACAGCGTGAAAAAAATCAAAG AACATGTCCGGTCTAAGACCAAG GTTCCTGTGCAGGACCAGGTTCTTTTGCTGGGCTCCAAGATCTTAAA GCCACGGAGAAGCCTCTCATCTT ACGGCATTGACAAAGAGAAGACCATCCACCTTACCCTGAAAGTGGTG AAGCCCAGTGATGAGGAGCTGCC CTTGTTTCTTGTGGAGTCAGGTGATGAGGCAAAGAGGCACCTCCTCC AGGTGCGAAGGTCCAGCTCAGTG GCACAAGTGAAAGCAATGATCGAGACTAAGACGGGTATAATCCCTGA GACCCAGATTGTGACTTGCAATG GAAAGAGACTGGAAGATGGGAAGATGATGGCAGATTACGGCATCAGA AAGGGCAACTTACTCTTCCTGGC ATCTTATTGTATTGGAGGGTGACCACCCTGGGCATGGGGTGTTGGCA GGGGTCAAAAAGCTTATTTCTTT TAATCTCTTACTCAACGAACACATCTTCTGATGATTTCCCAAAATTA ATGAGAATGAGATGAGTAGAGTA AGATTTGGGTGGGATGGGTAGGATGAAGTATATTGCCCAACTCTATG TTTCTTTGATTCTAACACAATTA ATTAAGTGACATGATTTTTACTAATGTATTACTGAGACTAGTAAATA AATTTTTAAGCCAA | |
| 206118 _at | signal transducer and activator of transcription 4 | gctgacatcctgcgagactacaaa gttattatggctgaaaacattcct gaaaaccctctg aagtacctatatcctgacattccc aaagacaaagccttcggtaaacac tacagctctcag ccttgcgaagtttcaagaccaaca gaaaggggtgacaaaggttatgtt ccttctgttttt | >gi\|21618332\|ref\|NM_003151.2\| Homo sapiens signal transducer and activator of transcription 4 (STAT4), mRNA | 13.56 |
| | | | AGAGGACGCCCGGTGAAGGGGCTCCAGCCTGGCAGTTTCTGCGTGTT AGCATTTCTAGAATAGAGTGGGT GGGAACTGACCCAAGTAAAGTCCCAGAGACTCGAACACTGACGCACA GGAAAGCCTCAAGTGGGAGGAGA AATGCAAATCCCCTACTGATGATGGCGTCAGCGGCTTTCTCCTAGGG ACTGTGAGGGGCGCTTCTGACTT | |
| | | atccccatctcaacaatccgaagt gattcaacagagccacattctcca tcagaccttctt cccatgtctccaagtgtgtatgcg gtgttgagagaaaacctgagtccc acaacaattgaa actgcaatgaagtctccttattct gctgaatgacaggataaactctga cgcaccaagaaa ggaagcaaatgaaaaagtttaaag actgttctttgcccaataaccaca ttttatttcttc agctttgtaaataccaggttctag gaaatgtttgacatctgaagctct cttcacactccc gtggcactcctcaattgggag [Seq ID No 55] | TGGACTTGAGCACTGCCTGGGACCTGTGCTGAGAGAGCGCTAGCATG TCTCAGTGGAATCAAGTCCAACA GTTAGAAATCAAGTTTTTGGAGCAGGTGGATCAATTCTATGATGACA ACTTTCCCATGGAAATTCGGCAT CTGTTGGCCCAATGGATTGAAAATCAAGACTGGGAGGCAGCTTCTAA CAATGAAACCATGGCAACGATTC TTCTTCAAAACTTGTTAATACAACTGGATGAACAGTTAGGTCGTGTT TCCAAAGAGAAAAACCTACTCTT GATACACAATCTAAAAAGAATTAGGAAGGTCCTTCAGGGAAAATTTC ATGGAAATCCAATGCATGTAGCT GTGGTTATTTCAAACTGTTTAAGGGAAGAGAGGAGAATATTGGCTGC AGCCAACATGCCTGTCCAGGGGC CTCTAGAGAAATCCTTACAAAGTTCTTCAGTTTCAGAAAGACAGAGG AATGTGGAGCACAAAGTGGCTGC CATTAAAAACAGTGTGCAGATGACAGAACAAGATACCAAATACTTAG AAGATCTGCAAGACGAATTTGAC TACAGGTATAAAACAATTCAGACAATGGATCAGAGTGACAAGAATAG TGCCATGGTGAATCAGGAAGTTT TGACACTGCAGGAAATGCTTAACAGCCTCGATTTCAAGAGAAAGGAG GCTCTCAGTAAAATGACCCAAAT CATCCATGAGACAGACCTGTTAATGAACACCATGCTCATAGAAGAGC TGCAAGACTGGAAGCGGCGGCAG CAAATCGCCTGCATCGGGGGTCCACTCCACAATGGGCTCGACCAGCT TCAGAACTGCTTTACACTATTGG CAGAAAGTCTTTTCCAACTGAGAAGGCAATTGGAGAAACTAGAGGAG CAATCTACCAAAATGACATATGA AGGTGATCCCATTCCAATGCAAAGAACTCACATGCTAGAAAGAGTCA CCTTCTTGATCTACAACCTTTTC AAGAACTCATTTGTGGTTGAGCGACAGCCATGTATGCCAACCCACCC TCAGAGGCCGTTGGTACTTAAAA CCCTAATTCAGTTCACTGTAAAACTAAGGCTACTAATAAAATTGCCA GAACTAAACTATCAGGTAAAGGT TAAGGCATCAATTGACAAGAATGTTTCAACTCTAAGCAACCGAAGAT TTGTACTTTGTGGAACTAATGTC | |
| | | | AAAGCCATGTCTATTGAAGAATCTTCCAATGGGAGTCTCTCAGTAGA ATTTCGACATTTGCAACCAAAGG AAATGAAGTCCAGTGCTGGAGGTAAAGGAAATGAGGGCTGTCACATG GTGACTGAAGAACTTCATTCCAT AACGTTTGAAACACAGATCTGCCTCTATGGCCTGACCATAGATTTGG AGACCAGCTCATTGCCTGTGGTG ATGATTTCCAATGTCAGTCAGTTACCTAATGCTTGGGCATCCATCAT TTGGTACAACGTGTCAACCAACG ATTCCCAGAACTTGGTTTCTTTAATAATCCTCCACCTGCCACATTG AGTCAACTACTGGAGGTGATGAG CTGGCAGTTTTCATCGTACGTTGGTCGTGGTCTTAACTCAGATCAAC TCCATATGCTGGCAGAGAAGCTT ACAGTCCAATCTAGCTACAGTGATGGTCACCTCACCTGGGCCAAGTT CTGCAAGGAACATTTACCTGGTA AATCATTTACCTTTTGGACATGGCTTGAAGCAATATTGGATCTAATT AAGAAACACATTCTTCCCCTTTG GATTGATGGGTATGTCATGGGCTTTGTTAGCAAAGAGAAGGAACGGC TGTTGCTAAAGGATAAAATGCCT GGCACCTTTTTATTAAGATTCAGTGAAAGCCATCTCGGAGGAATAAC TTTCACCTGGGTGGACCATTCTG AAAGTGGGGAAGTGAGATTCCACTCTGTAGAACCCTACAATAAAGGC CGGTTGTCTGCTCTGCCATTCGC TGACATCCTGCGAGACTACAAAGTTATTATGGCTGAAAACATTCCTG AAAACCCTCTGAAGTACCTATAT CCTGACATTCCCAAAGACAAAGCCTTCGGTAAACACTACAGCTCTCA GCCTTGCGAAGTTTCAAGACCAA CAGAAAGGGGTGACAAAGGTTATGTTCCTTCTGTTTTTATCCCCATC TCAACAATCCGAAGTGATTCAAC AGAGCCACATTCTCCATCAGACCTTCTTCCCATGTCTCCAAGTGTGT ATGCGGTGTTGAGAGAAAACCTG AGTCCCACAACAATTGAAACTGCAATGAAGTCTCCTTATTCTGCTGA ATGACAGGATAAACTCTGACGCA CCAAGAAAGGAAGCAAATGAAAAAGTTTAAAGACTGTTCTTTGCCCA ATAACCACATTTTATTTCTTCAG | |
| | | | CTTTGTAAATACCAGGTTCTAGGAAATGTTTGACATCTGAAGCTCTC TTCACACTCCCGTGGCACTCCTC AATTGGGAGTGTTGTGACTGAAATGCTTGAAACCAAAGCTTCAGATA AACTTGCAAGATAAGACAACTTT AAGAAACCAGTGTTAATAACAATATTAACAG | |
| 206666 _at | granzyme K (granzyme 3; tryptase II) /// granzyme K (granzyme 3; tryptase II) | aaacctctcttagatctggaacca aatgcaaggttactggctggggag ccaccgatccagattcattaagac cttctgacaccctgcgagaagtca ctgttactgtcctaagtcgaaaac tttgcaacagccaaagttactaca acggcgacccttttatcaccaaag acatggtctgtg caggagatgccaaaggccagaagg attcctgtaagggtgactcagggg gccccttgatctgtaaaggtgtct tccacgctatagtctctggaggtc atgaatgtggtgttgccacaaagc ctggaatctacaccctgttaacca agaaataccagacttggatcaaaa gcaaccttgtcccgcctcatacaa attaagttacaaataattttattg gatgcacttgcttctttttttccta atatgctcgcaggttagagttggg tgtaagtaaagcagagcacatatg gggtccattttt gcacttgta [Seq ID No 56] | >gi\|73747815\|ref\|NM_002104.2\| Homo sapiens granzyme K (granzyme 3; tryptase II) (GZMK), mRNA | 18.25 |
| | | | GATCAACACATTTCATCTGGGCTTCTTAAATCTAAATCTTTAAAATG ACTAAGTTTTCTTCCTTTTCTCT GTTTTTCCTAATAGTTGGGGCTTATATGACTCATGTGTGTTTCAATA TGGAAATTATTGGAGGGAAAGAA GTGTCACCTCATTCCAGGCCATTTATGGCCTCCATCCAGTATGGCGG ACATCACGTTTGTGGAGGTGTTC TGATTGATCCACAGTGGGTGCTGACAGCAGCCCACTGCCAATATCGG TTTACCAAAGGCCAGTCTCCCAC TGTGGTTTTAGGCGCACACTCTCTCTCAAAGAATGAGGCCTCCAAAC AAACACTGGAGATCAAAAAATTT ATACCATTCTCAAGAGTTACATCAGATCCTCAATCAAATGATATCAT GCTGGTTAAGCTTCAAACAGCCG CAAAACTCAATAAACATGTCAAGATGCTCCACATAAGATCCAAAACC TCTCTTAGATCTGGAACCAAATG CAAGGTTACTGGCTGGGGAGCCACCGATCCAGATTCATTAAGACCTT CTGACACCCTGCGAGAAGTCACT GTTACTGTCCTAAGTCGAAAACTTTGCAACAGCCAAAGTTACTACAA CGGCGACCCTTTTATCACCAAAG ACATGGTCTGTGCAGGAGATGCCAAAGGCCAGAAGGATTCCTGTAAG GGTGACTCAGGGGGCCCCTTGAT CTGTAAAGGTGTCTTCCACGCTATAGTCTCTGGAGGTCATGAATGTG GTGTTGCCACAAAGCCTGGAATC TACACCCTGTTAACCAAGAAATACCAGACTTGGATCAAAAGCAACCT TGTCCCGCCTCATACAAATTAAG TTACAAATAATTTTATTGGATGCACTTGCTTCTTTTTTCCTAATATG | |
| | | | CTCGCAGGTTAGAGTTGGGTGTA AGTAAAGCAGAGCACATATGGGGTCCATTTTTGCACTTGTAAGTCAT TTTATTAAGGAATCAAGTTCTTT TTCACTTGTATCACTGATGTATTTCTACCATGCTGGTTTTATTCTAA ATAAAATTTAGAAGACTCAAAAA AAAAAAAAAAAAAAAAAAAAAAA | |
| 206804 _at | CD3G antigen, gamma polypeptide (TiT3 complex) | gtaatgccaaggaccctcgaggga tgtatcagtgtaaaggatcacaga acaagtcaaaaccactccaagtgt attacagaatgtgtcagaactgca ttgaactaaatgcagccaccatat ctggctttctctttgctgaaatcg tcagcattttcgtccttgctgttg gggtctacttca ttgctggacaggatggagttcgcc agtcgagagcttcagacaagcaga ctctgttgcccaatgaccagctct accagcccctcaaggatcgagaag atgaccagtacagccaccttcaag gaaaccagttgaggaggaattgaa ctcaggactcagagtagtccaggt gttctcctcctattcagttcccag aatcaaagcaatgcattttggaaa gctcctagcagagagactttcagc cctaaatctagactcaaggttccc agagatgac [Seq ID No 57] | >gi\|4557428\|ref\|NM_000073.1\| Homo sapiens CD3g molecule, gamma (CD3-TCR complex) (CD3G), mRNA | 23.86 |
| | | | GGGCTGCTCCACGCTTTTGCCGGAGACAGAGACTGACATGGAACAGG GGAAGGGCCTGGCTGTCCTCATC CTGGCTATCATTCTTCTTCAAGGTACTTTGGCCCAGTCAATCAAAGG AAACCACTTGGTTAAGGTGTATG ACTATCAAGAAGATGGTTCGGTACTTCTGACTTGTGATGCAGAAGCC AAAAATATCACATGGTTTAAAGA TGGGAAGATGATCGGCTTCCTAACTGAAGATAAAAAAAAATGGAATC TGGGAAGTAATGCCAAGGACCCT CGAGGGATGTATCAGTGTAAAGGATCACAGAACAAGTCAAAACCACT CCAAGTGTATTACAGAATGTGTC AGAACTGCATTGAACTAAATGCAGCCACCATATCTGGCTTTCTCTTT GCTGAAATCGTCAGCATTTTCGT CCTTGCTGTTGGGGTCTACTTCATTGCTGGACAGGATGGAGTTCGCC AGTCGAGAGCTTCAGACAAGCAG ACTCTGTTGCCCAATGACCAGCTCTACCAGCCCCTCAAGGATCGAGA AGATGACCAGTACAGCCACCTTC AAGGAAACCAGTTGAGGAGGAATTGAACTCAGGACTCAGAGTAGTCC AGGTGTTCTCCTCCTATTCAGTT CCCAGAATCAAAGCAATGCATTTTGGAAAGCTCCTAGCAGAGAGACT TTCAGCCCTAAATCTAGACTCAA GGTTCCCAGAGATGACAAATGGAGAAGAAAGGCCATCAGAGCAAATT TGGGGGTTTCTCAAATAAAATAA AAATAAAAACAAATACTGTGTTTCAGAAGCGCCACCTATTGGGGAAA ATTGT | |
| 207651 _at | G protein-coupled receptor 171 | ttgccttgtaattcgacagctcta cagaaacaaagataatgaaaatta cccaaatgtgaa aaaggctctcatcaacatactttt agtgaccacgggctacatcatatg ctttgttcctta ccacattgtccgaatcccgtatac cctcagccagacagaagtcataac tgattgctcaac caggatttcactcttcaaagccaa agaggctacactgctcctggctgt gtcgaacctgtg ctttgatcctatcctgtactatca cctctcaaaagcattccgctcaaa ggtcactgagac ttttgcctcacctaaagagaccaa ggctcagaaagaaaaattaagatg tgaaaataatgc ataaaagacaggatttttttgtgct accaattctggccttactgga [Seq ID No 58] | >gi\|31377771\|ref\|NM_013308.2\| Homo sapiens G protein-coupled receptor 171 (GPR171), mRNA | 46.65 |
| | | | GGTTGCTACTGCTGCGGCTAACCAAACAGCTCATGCTTCTCTGAAGA CTTGCAGCAAGGTTTGCTGAGGC TCACAGAAGATAGCCCCAGTGTTTTGGAGTGGTTTTGAATGTGATTC TGAGATCAGACTGACTGAGCTGG AATCCTGGCTTTATATCTTACCAGCTACACAACCTTGGAGTCTTAGA AATTTTTTCTTTTCAATAAGCAG TCATCCTTACTTTCCCTCAAGATGACAAACAGTTCGTTCTTCTGCCC AGTTTATAAAGATCTGGAGCCAT TCACGTATTTTTTTTATTTAGTTTTCCTTGTTGGAATTATTGGAAGT TGTTTTGCAACCTGGGCTTTTAT ACAGAAGAATACGAATCACAGGTGTGTGAGCATCTACTTAATTAATT TGCTTACAGCCGATTTCCTGCTT ACTCTGGCATTACCAGTGAAAATTGTTGTTGACTTGGGTGTGGCACC TTGGAAGCTGAAGATATTCCACT GCCAAGTAACAGCCTGCCTCATCTATATCAATATGTATTTATCAATT ATCTTCTTAGCATTTGTCAGCAT TGACCGCTGTCTTCAGCTGACACACAGCTGCAAGATCTACCGAATAC AAGAACCCGGATTTGCCAAAATG ATATCAACCGTTGTGTGGCTAATGGTCCTTCTTATAATGGTGCCAAA TATGATGATTCCCATCAAAGACA TCAAGGAAAAGTCAAATGTGGGTTGTATGGAGTTTAAAAAGGAATTT GGAAGAAATTGGCATTTGCTGAC AAATTTCATATGTGTAGCAATATTTTTAAATTTCTCAGCCATCATTT TAATATCCAATTGCCTTGTAATT CGACAGCTCTACAGAAACAAAGATAATGAAAATTACCCAAATGTGAA AAAGGCTCTCATCAACATACTTT TAGTGACCACGGGCTACATCATATGCTTTGTTCCTTACCACATTGTC CGAATCCCGTATACCCTCAGCCA GACAGAAGTCATAACTGATTGCTCAACCAGGATTTCACTCTTCAAAG CCAAAGAGGCTACACTGCTCCTG GCTGTGTCGAACCTGTGCTTTGATCCTGTCCTGTACTATCACCTCTC AAAAGCATTCCGCTCAAAGGTCA | |
| | | | CTGAGACTTTTGCCTCACCTAAAGAGACCAAGGCTCAGAAAGAAAAA TTAAGATGTGAAAATAATGCATA AAAGACAGGATTTTTGTGCTACCAATTCTGGCCTTACTGGACCATAA AGTTAATTATAGCTTTGAAAGAT AAAAAAAAAAAAAAAAAACAAAAAAAAACTCAGTATGAAAAAATACAG TTAGCTAGCAAATATGGACAGGT TTACTTAGAAATCCTGTTTCTAAATGCAAGTCAAGCTTTATTGTTAG GCTTGCTGCTACTCATTAACCCA AATATTTGTACAAAAAACTAAAGAGTCTCATTGAACGAATGTAAAAT CCTGCAATATCCTTGAAATCCAA AAGAGGTCCATGACATAGACCCAAAGGTATTCATGAGTTATTCATTT AAATGCCTGGAACTGACTTCTTG ATAAAAATATAAAAAATAATTTCCATGTAAGTTACCAGAAAGCCCAC CAGCAACATAATTTTAAAGCCTT TCGGATTACTTTTAAAAAATGCAGCTTACATATAACAACTTGTGCCT ATTTTATTTCTAATCTATCACTT CAAAAGATGGTAATCTTTCAACTCATTATTCCTCCAATTTTTAATGT CGAATTTTTTTCTAACACAATAA CCAAAAGCTTTTATTTATAAAAAGGCTTGAAAAATATAAAAAAAAAA AAAAAAAAAAAAAAAAAAAAAAA AAAAAAAAAAAAAAAAAAAAAAAAAAAAAAAAAAA | |
| 207957_s_at | Protein kinase C, beta 1 | tgatgagctttcctttgatccgga cgacgtaatcactgacattgagat ggtggacgaggg ctggtggcggggacgttgccatgg ccactttggactcttccctgcaaa ttatgtcaagct tctggagtgactagagctcactgt ctactgcaactgtgatttcccatg tccaaagtggct ctgctccacccccctccctattcct gatgcaaatgtctaaccagatgag tttctggacaga | >gi\|89353296\|ref\|NM_001183.4\| Homo sapiens ATPase, H+ transporting, lysosomal accessory protein 1 (ATP6AP1), mRNA | 34.17 |
| | | | GGGGGCAACGGTCACCTGATCTGCGGCTGTCGAGGCCGCTGAGGCAG TGGAGGCTGAGGCTATGATGGCG GCCATGGCGACGGCTCGAGTGCGGATGGGGCCGCGATGCGCCCAGGC GCTCTGGCGCATGCCGTGGCTGC CGGTGTTTTTGTCGTTGGCGGCGGCGGCGGCGGCAGCGGCGGAG CAGCAGGTCCCGCTGGTGCTGTG GTCGAGTGACCGGGACTTGTGGCTCCTGCGGCCGACACTCATGAAG GCCACATCACCAGCGACTTGCAG CTCTCTACCTACTTAGATCCCGCCCTGGAGCTGGGTCCCAGGAATGT | |
| | | cttccctctcctgcttcattaagg gcttggggcagagacagcatgggg aaggaggtccccttccccaagagt cctctctatcctggatgagctcat gaacatttctcttgtgttcctgac tccttcccaatgaacacctctctg ccaccccaagctctgctctcctcc tctgtgagctct gggcttcccagtttgtttacccgg gaaagtacgtctagattgtgtggt ttgcctcattgt gctatttgcccactttccttccct gaagaaatatctgtgaaccttctt tctgttcagtcc ta [Seq Id No 59] | GCTGCTGTTCCTGCAGGACAAGC TGAGCATTGAGGATTTCACAGCATATGGCGGTGTGTTTGGAAACAAG CAGGACAGCGCCTTTTCTAACCT AGAGAATGCCCTGGACCTGGCCCCCTCCTCACTGGTGCTTCCTGCCG TCGACTGGTATGCAGTCAGCACT CTGACCACTTACCTGCAGGAGAAGCTCGGGGCCAGCCCCTTGCATGT GGACCTGGCCACCCTGCGGGAGC TGAAGCTCAATGCCAGCCTCCCTGCTCTGCTGCTCATTCGCCTGCCC TACACAGCCAGCTCTGGTCTGAT GGCACCCAGGGAAGTCCTCACAGGCAACGATGAGGTCATCGGGCAGG TCCTGAGCACACTCAAGTCCGAA GATGTCCCATACACAGCGGCCCTCACAGCGGTCCGCCCTTCCAGGGT GGCCCGTGATGTAGCCGTGGTGG CCGGAGGGCTAGGTCGCCAGCTGCTACAAAAACAGCCAGTATCACCT GTGATCCATCCTCCTGTGAGTTA CAATGACACCGCTCCCCGGATCCTGTTCTGGGCCCAAAACTTCTCTG TGGCGTACAAGGACCAGTGGGAG GACCTGACTCCCCTCACCTTTGGGGTGCAGGAACTCAACCTGACTGG CTCCTTCTGGAATGACTCCTTTG CCAGGCTCTCACTGACCTATGAACGACTCTTTGGTACCACAGTGACA TTCAAGTTCATTCTGGCCAACCG CCTCTACCCAGTGTCTGCCCGGCACTGGTTTACCATGGAGCGCCTCG AAGTCCACAGCAATGGCTCCGTC GCCTACTTCAATGCTTCCCAGGTCACAGGGCCCAGCATCTACTCCTT CCACTGCGAGTATGTCAGCAGCC TGAGCAAGAAGGGTAGTCTCCTCGTGGCCCGCACGCAGCCCTCTCCC TGGCAGATGATGCTTCAGGACTT CCAGATCCAGGCTTTCAACGTAATGGGGGAGCAGTTCTCCTACGCCA GCGACTGTGCCAGCTTCTTCTCC CCCGGCATCTGGATGGGGCTGCTCACCTCCCTGTTCATGCTCTTCAT CTTCACCTATGGCCTGCACATGA TCCTCAGCCTCAAGACCATGGATCGCTTTGATGACCACAAGGGCCCC ACTATTTCTTTGACCCAGATTGT GTGACCCTGTGCCAGTGGGGGGGTTGAGGGTGGGACGGTGTCCGTGT | |
| | | | TGTTGCTTTCCCACCCTGCAGCG CACTGGACTGAAGAGCTTCCCTCTTCCTACTGCAGCATGAACTGCAA GCTCCCCTCAGCCCATCTTGCTC CCTCTTCAGCCCGCTGAGGAGCTTTCTTGGGCTGCCCCCATCTCTCC CAACAAGGTGTACATATTCTGCG TAGATGCTAGACCAACCAGCTTCCCAGGGTTCGTCGCTGTGAGGCGT AAGGGACATGAATTCTAGGGTCT CCTTTCTCCTTATTTATTCTTGTGGCTACATCATCCCTGGCTGTGGA TAGTGCTTTTGTGTAGCAAATGC TCCCTCCTTAAGGTTATAGGGCTCCCTGAGTTTGGGAGTGTGGAAGT ACTACTTAACTGTCTGTCCTGCT TGGCTGTCGTTATCGTTTTCTGGTGATGTTGTGCTAACAATAAGAAG TACACGGGTTTATTTCTGTGGCC TGAGAAGGAAGGGACCTCCACGACAGGTGGGCTGGGTGCGATCGCCG GCTGTTTGGCATGTTCCCACCGG GAGTGCCGGGCAGGAGCATGGGGTGCTTGGTTGTTTCCTTCCTAATA AAATAAACGCGGTCGCCATGCA AAAAAAAAA | 6.11 |
| 208894 _at | major histocompa tibility complex, class II, DR alpha /// major histocompa tibility complex, class II, DR alpha | cgatcaccaatgtacctccagagg taactgtgctcacgaacagccctg tggaactgagagagcccaacgtcc tcatctgtttcatagacaagttca cccca [Seq Id No 60] | >gi\|52426773\|ref\|NM_019111.3\| Homo sapiens major histocompatibility complex, class II, DR alpha (HLA-DRA), mRNA | |
| | | | ACATTCTCTTTTCTTTTATTCTTGTCTGTTCTGCCTCACTCCCGAGC TCTACTGACTCCCAACAGAGCGC CCAAGAAGAAAATGGCCATAAGTGGAGTCCCTGTGCTAGGATTTTTC ATCATAGCTGTGCTGATGAGCGC TCAGGAATCATGGGCTATCAAAGAAGAACATGTGATCATCCAGGCCG AGTTCTATCTGAATCCTGACCAA TCAGGCGAGTTTATGTTTGACTTTGATGGTGATGAGATTTTCCATGT GGATATGGCAAAGAAGGAGACGG TCTGGCGGCTTGAAGAATTTGGACGATTTGCCAGCTTTGAGGCTCAA GGTGCATTGGCCAACATAGCTGT GGACAAAGCCAACCTGGAAATCATGACAAAGCGCTCCAACTATACTC CGATCACCAATGTACCTCCAGAG | |
| | | | GTAACTGTGCTCACGAACAGCCCTGTGGAACTGAGAGAGCCCAACGT CCTCATCTGTTTCATAGACAAGT TCACCCCACCAGTGGTCAATGTCACGTGGCTTCGAAATGGAAAACCT GTCACCACAGGAGTGTCAGAGAC AGTCTTCCTGCCCAGGGAAGACCACCTTTTCCGCAAGTTCCACTATC TCCCCTTCCTGCCCTCAACTGAG GACGTTTACGACTGCAGGGTGGAGCACTGGGGCTTGGATGAGCCTCT TCTCAAGCACTGGGAGTTTGATG CTCCAAGCCCTCTCCCAGAGACTACAGAGAACGTGGTGTGTGCCCTG GGCCTGACTGTGGGTCTGGTGGG CATCATTATTGGGACCATCTTCATCATCAAGGGATTGCGCAAAAGCA ATGCAGCAGAACGCAGGGGGCCT CTGTAAGGCACATGGAGGTGATGGTGTTTCTTAGAGAGAAGATCACT GAAGAAACTTCTGCTTTAATGGC TTTACAAAGCTGGCAATATTACAATCCTTGACCTCAGTGAAAGCAGT CATCTTCAGCATTTTCCAGCCCT ATAGCCACCCCAAGTGTGGATATGCCTCTTCGATTGCTCCGTACTCT AACATCTAGCTGGCTTCCCTGTC TATTGCCTTTTCCTGTATCTATTTTCCTCTATTTCCTATCATTTTAT TATCACCATGCAATGCCTCTGGA ATAAAACATACAGGAGTCTGTCTCTGCTATGGAATGCCCCATGGGGC ATCTCTTGTGTACTTATTGTTTA AGGTTTCCTCAAACTGTGATTTTTCTGAACACAATAAACTATTTTGA TGATCTTGGGTGGAAAAAAAAAA AAAAAAA | |
| 209480 _at | Major histocompa tibility complex, class II, DQ beta 1 | aaaaaaggaaatcgctgcagaatg aaggaatatcccttgaggtgaccc agccaacctgtggccagaaggagg gttgtaccttgaaaagaccactga aagcattttgggtgtcaagtaag ggtgggcagaggaggtagaaaatc aattcaattgtcgcatcattcatg gttctttaatattgatgctcagtg | >gi\|24797068\|ref\|NM_002123.2\| Homo sapiens major histocompatibility complex, class II, DQ beta 1 (HLA-DQB1), mRNA | 12.55 |
| | | | CAGATCCATCAGGTCCGAGCTGTGTTGACTACCACTTTTCCCTTCGT CTCAATTATGTCTTGGAAAAAGG CTTTGCGGATCCCCGGAGGCCTTCGGGCAGCAACTGTGACCTTGATG CTGTCGATGCTGAGCACCCCAGT GGCTGAGGGCAGAGACTCTCCCGAGGATTTCGTGTACCAGTTTAAGG | |
| | /// Major histocompatibility complex, class II, DQ beta 1 | cattggccttagaatatcccagcc tctcttctggtttggtgagtgctg tgtaagtaagcatggtagaattgt ttggagacatatatagtgatcctt ggtcactggtgt ttcaaacattctggaaagtcacat cgatcaagaatatttttt [Seq Id No 61] | GCATGTGCTACTTCACCAACGGG ACAGAGCGCGTGCGTCTTGTGAGCAGAAGCATCTATAACCGAGAAGA GATCGTGCGCTTCGACAGCGACG TGGGGGAGTTCCGGGCGGTGACGCTGCTGGGGCTGCCTGCCGCCGAG TACTGGAACAGCCAGAAGGACAT CCTGGAGAGGAAACGGGCGGCGGTGGACAGGGTGTGCAGACACAACT ACCAGTTGGAGCTCCGCACGACC TTGCAGCGGCGAGTGGAGCCCACAGTGACCATCTCCCCATCCAGGAC AGAGGCCCTCAACCACCACAACC TGCTGGTCTGCTCGGTGACAGATTTCTATCCAGCCCAGATCAAAGTC CGGTGGTTTCGGAATGACCAGGA GGAGACAGCTGGCGTTGTGTCCACCCCCCTTATTAGGAATGGTGACT GGACCTTCCAGATCCTGGTGATG CTGGAAATGACTCCCCAGCGTGGAGACGTCTACACCTGCCACGTGGA GCACCCCAGCCTCCAGAGCCCCA TCACCGTGGAGTGGCGGGCTCAATCTGAATCTGCCCAGAGCAAGATG CTGAGTGGCATTGGAGGCTTCGT GCTGGGGCTGATCTTCCTCGGGCTGGGCCTTATCATCCATCACAGGA GTCAGAAAGGGCTCCTGCACTGA CTCCTGAGACTATTTTAACTGGGATTGGTTATCACTTTTCTGTAACG CCTGCTTGTCCCTGCCCAGAATT CCCAGCTGTCTGTGTCAGCCTGTCCCCCTGAGATCAGAGTCCTACAG TGGCTGTCACGCAGCCACCAGGT CATCTCCTTTCATCCCCACCTTGAGGCGGATGGCTGTGACCCTACTT CCTGCACTGACCCACAGCCTCTG CCTGTGCACGGCCAGCTGCATCTACTCAGGCCCCAAGGGGTTTCTGT TTCTATTCTCTCCTCAGACTGCT CAAGAGAAGCACATGAAAACCATTACCTGACTTTAGAGCTTTTTTAC ATAATTAAACATGATCCTGAGTT | |
| 209613 _s_at | alcohol dehydrogenase IB | gcagatttcttgcttcatatgaca aagcctcaattactaattgtaaaa actgaactattc ccagaatcatgttcaaaaaatctg | >gi\|34577060\|ref\|NM_000668.3\| Homo sapiens alcohol dehydrogenase IB (class I), beta polypeptide (ADH1B), mRNA | 58.89 |
| | | | ATGCACTCAAGCAGAGAAGAAATCCACAAAGACTCACAGTCTGCTGG | |
| | (class I), beta polypeptide | taatttttgctgatcgaaagtgct tcattgactaaa cagtattagtttgtggctataaat gattatttagatgatgactgaaaa tgtgtataaagt aattaaaagtaatatggtggcttt aagtgtagagatgggatggcaaat gctgtgaatgca gaatgtaaaattggtaactaagaa atggcacaaacaccttaagcaata tattttcctagt agatatatatatacacatacatat atacacatatacaaatgtatattt ttgcaaaattgt tttcaatctagaacttttctatta actaccatgtcttaaaatcaagtc tataatcctagc attagtttaatattttgaatatgt aaacacctgtgttaatgctttgtt aatgcttttccc actctcatttgttaatgctttccc actctcgggaaggatttgcatttt gagctttatctc taaatgtgacatgca [Seq Id No 62] | TGGGCAGAGAAGACAGAAACGAC ATGAGCACAGCAGGAAAAGTAATCAAATGCAAAGCAGCTGTGCTATG GGAGGTAAAGAAACCCTTTTCCA TTGAGGATGTGGAGGTTGCACCTCCTAAGGCTTATGAAGTTCGCATT AAGATGGTGGCTGTAGGAATCTG TCACACAGATGACCACGTGGTTAGTGGCAACCTGGTGACCCCCCTTC CTGTGATTTTAGGCCATGAGGCA GCCGGCATCGTGGAGAGTGTTGGAGAAGGGGTGACTACAGTCAAACC AGGTGATAAAGTCATCCCGCTCT TTACTCCTCAGTGTGGAAAATGCAGAGTTTGTAAAAACCCGGAGAGC AACTACTGCTTGAAAAATGATCT AGGCAATCCTCGGGGGACCCTGCAGGATGGCACCAGGAGGTTCACCT GCAGGGGGAAGCCCATTCACCAC TTCCTTGGCACCAGCACCTTCTCCCAGTACACGGTGGTGGATGAGAA TGCAGTGGCCAAAATTGATGCAG CCTCGCCCCTGGAGAAAGTCTGCCTCATTGGCTGTGGATTCTCGACT GGTTATGGGTCTGCAGTTAACGT TGCCAAGGTCACCCCAGGCTCTACCTGTGCTGTGTTTGGCCTGGGAG GGGTCGGCCTATCTGCTGTTATG GGCTGTAAAGCAGCTGGAGCAGCCAGAATCATTGCGGTGGACATCAA CAAGGACAAATTTGCAAAGGCCA AAGAGTTGGGTGCCACTGAATGCATCAACCCTCAAGACTACAAGAAA CCCATCCAGGAAGTGCTAAAGGA AATGACTGATGGAGGTGTGGATTTTTCGTTTGAAGTCATCGGTCGGC TTGACACCATGATGGCTTCCCTG TTATGTTGTCATGAGGCATGTGGCACAAGCGTCATCGTAGGGGTACC TCCTGCTTCCCAGAACCTCTCAA TAAACCCTATGCTGCTACTGACTGGACGCACCTGGAAGGGGGCTGTT TATGGTGGCTTTAAGAGTAAAGA AGGTATCCCAAAACTTGTGGCTGATTTTATGGCTAAGAAGTTTTCAC TGGATGCGTTAATAACCCATGTT TTACCTTTTGAAAAAATAAATGAAGGATTTGACCTGCTTCACTCTGG GAAAAGTATCCGTACCGTCCTGA CGTTTTGAGGCAATAGAGATGCCTTCCCCTGTAGCAGTCTTCAGCCT | |
| | | | CCTCTACCCTACAAGATCTGGAG CAACAGCTAGGAAATATCATTAATTCAGCTCTTCAGAGATGTTATCA ATAAATTACACATGGGGGCTTTC CAAAGAAATGGAAATTGATGGGAAATTATTTTCAGGAAAATTTAAA ATTCAAGTGAGAAGTAAATAAAG TGTTGAACATCAGCTGGGGAATTGAAGCCAACAAACCTTCCTTCTTA ACCATTCTACTGTGTCACCTTTG CCATTGAGGAAAAATATTCCTGTGACTTCTTGCATTTTTGGTATCTT CATAATCTTTAGTCATCGAATCC CAGTGGAGGGGACCCTTTTACTTGCCCTGAACATACACATGCTGGGC CATTGTGATTGAAGTCTTCTAAC TCTGTCTCAGTTTTCACTGTCGACATTTTCCTTTTTCTAATAAAAAT GTACCAAATCCCTGGGGTAAAAG CTAGGGTAAGGTAAAGGATAGACTCACATTTACAAGTAGTGAAGGTC CAAGAGTTCTAAATACAGGAAAT TTCTTAGGAACTCAAATAAAATGCCCCACATTTTACTACAGTAAATG GCAGTGTTTTTATGACTTTTATA CTATTTCTTTATGGTCGATATACAATTGATTTTTTAAAATAATAGCA GATTTCTTGCTTCATATGACAAA GCCTCAATTACTAATTGTAAAAACTGAACTATTCCCAGAATCATGTT CAAAAAATCTGTAATTTTTGCTG ATGAAAGTGCTTCATTGACTAAACAGTATTAGTTTGTGGCTATAAAT GATTATTTAGATGATGACTGAAA ATGTGTATAAAGTAATTAAAAGTAATATGGTGGCTTTAAGTGTAGAG ATGGGATGGCAAATGCTGTGAAT GCAGAATGTAAAATTGGTAACTAAGAAATGGCACAAACACCTTAAGC AATATATTTTCCTAGTAGATATA TATATACACATACATATATACACATATACAAATGTATATTTTTGCAA AATTGTTTTCAATCTAGAACTTT TCTATTAACTACCATGTCTTAAAATCAAGTCTATAATCCTAGCATTA GTTTAATATTTTGAATATGTAAA GACCTGTGTTAATGCTTTGTTAATGCTTTTCCCACTCTCATTTGTTA ATGCTTTCCCACTCTCAGGGGAA GGATTTGCATTTTGAGCTTTATCTCTAAATGTGACATGCAAAGATTA | |
| | | | TTCCTGGTAAAGGAGGTAGCTGT CTCCAAAAATGCTATTGTTGCAATATCTACATTCTATTTCATATTAT GAAAGACCTTAGACATAAAGTAA AATAGTTTATCATTTACTGTGTGATCTTCAGTAAGTCTCTCAGGCTC TCTGAGCTTGTTCATCCTTTGTT TTGAAAAAATTACTCAACCAATCCATTACAGCTTAACCAAGATTAAA TGGGATGATGTTAATGAAAGAGC TTCGCC | |
| 209670 _at | T cell receptor alpha constant /// T cell receptor alpha constant | caactggatcctacccgaatttat gattaagattgctgaagagctgcc aaacactgctgc cacccccctctgttcccttattgct gcttgtcactgcctgacattcacg gcagaggcaagg ctgctgcagcctcccctggctgtg cacattccctcctgctccccagag actgcctccgcc atcccacagatgatggatcttcag tgggttctcttgggctctaggtcc tggagaatgttg tgagggggtttattttttttaata gtgttcataaagaaatacatagta ttcttcttctca agacgtgggggaaattatctcat tatcgaggccctgctatgctgtgt gtctggcgtgt tgtatgtcctgc [Seq Id No 63] | >gi\|36944\|emb\|X02592.1\|HSTCRAR Human mRNA for T-cell receptor alpha chain (TCR-alpha) | 24.26 |
| | | | TTTTGAAACCCTTCAAAGGCAGAGACTTGTCCAGCCTAACCTGCCTG CTGCTCCTAGCTCCTGAGGCTCA GGGCCCTTGGCTTCTGTCCGCTCTGCTCAGGGCCCTCCAGCGTGGCC ACTGCTCAGCCATGCTCCTGCTG CTCGTCCCAGTGCTCGAGGTGATTTTTACCCTGGGAGGAACCAGAGC CCAGTCGGTGACCCAGCTTGGCA GCCACGTCTCTGTCTCTGAAGGAGCCCTGGTTCTGCTGAGGTGCAAC TACTCATCGTCTGTTCCACCATA TCTCTTCTGGTATGTGCAATACCCCAACCAAGGACTCCAGCTTCTCC TGAAGTACACATCAGCGGCCACC CTGGTTAAAGGCATCAACGGTTTTGAGGCTGAATTTAAGAAGAGTGA AACCTCCTTCCACCTGACGAAAC CCTCAGCCCATATGAGCGACGCGGCTGAGTACTTCTGTGCTGTGAGT GATCTCGAACCGAACAGCAGTGC TTCCAAGATAATCTTTGGATCAGGGACCAGACTCAGCATCCGGCCAA ATATCCAGAACCCTGACCCTGCC GTGTACCAGCTGAGAGACTCTAAATCCAGTGACAAGTCTGTCTGCCT ATTCACCGATTTTGATTCTCAAA CAAATGTGTCACAAAGTAAGGATTCTGATGTGTATATCACAGACAAA ACTGTGCTAGACATGAGGTCTAT GGACTTCAAGAGCAACAGTGCTGTGGCCTGGAGCAACAAATCTGACT TTGCATGTGCAAACGCCTTCAAC AACAGCATTATTCCAGAAGACACCTTCTTCCCCAGCCCAGAAAGTTC | |
| | | | CTGTGATGTCAAGCTGGTCGAGA AAAGCTTTGAAACAGATACGAACCTAAACTTTCAAAACCTGTCAGTG ATTGGGTTCCGAATCCTCCTCCT GAAAGTGGCCGGGTTTAATCTGCTCATGACGCTGCGGCTGTGGTCCA GCTGAGATCTGCAAGATTGTAAG ACAGCCTGTGCTCCCTCGCTCCTTCCTCTGCATTGCCCCTCTTCTCC CTCTCCAAACAGAGGGAACTCTC CTACCCCCAAGGAGGTGAAAGCTGCTACCACCTCTGTGCCCCCCCGG TAATGCCACCAACTGGATCCTAC CCGAATTTATGATTAAGATTGCTGAAGAGCTGCCAAACACTGCTGCC ACCCCCTCTGTTCCCTTATTGCT GCTTGTCACTGCCTGACATTCACGGCAGAGGCAAGGCTGCTGCAGCC TCCCCTGGCTGTGCACATTCCCT CCTGCTCCCCAGAGACTGCCTCCGCCATCCCACAGATGATGGATCTT CAGTGGGTTCTCTTGGGCTCTAG GTCCTGGAGAATGTTGTGAGGGGTTTATTTTTTTTAATAGTGTTCA TAAAGAAATACATAGTATTCTTC TTCTCAAGACGTGGGGGAAATTATCTCATTATCGAGGCCCTGCTAT GCTGTGTGTCTGGCGTGTTGTA TGTCCTGCTGCCGATGCCTTCATTAAAATGATTTGGAA | |
| 209795 _at | CD69 antigen (p60, early T-cell activation antigen) | tagtctaattgaatcccttaaact cagggagcatttataaatggcaaa tgcttatgaaac taagatttgtaatatttctctctt tttagagaaatttgccaatttact ttgttatttttc cccaaaaagaatggatgatcgtg tatttatttttttacttcctcagc tgtagacaggtc cttttcgatggtacatatttcttt gcctttataatcttttatacagtg tcttacagagaa aagacataagcaaagactatgagg aatatttgcaagacatagaatagt | >gi\|4502680\|ref\|NM_001781.1\| Homo sapiens CD69 molecule (CD69), mRNA | 30.48 |
| | | | AGACTCAACAAGAGCTCCAGCAAAGACTTTCACTGTAGCTTGACTTG ACCTGAGATTAACTAGGGAATCT TGAGAATAAAGATGAGCTCTGAAAATTGTTTCGTAGCAGAGAACAGC TCTTTGCATCCGGAGAGTGGACA AGAAAATGATGCCACCAGTCCCCATTTCTCAACACGTCATGAAGGGT CCTTCCAAGTTCCTGTCCTGTGT GCTGTAATGAATGTGGTCTTCATCACCATTTTAATCATAGCTCTCAT TGCCTTATCAGTGGGCCAATACA ATTGTCCAGGCCAATACACATTCTCAATGCCATCAGACAGCCATGTT TCTTCATGCTCTGAGGACTGGGT TGGCTACCAGAGGAAATGCTACTTTATTTCTACTGTGAAGAGGAGCT GGACTTCAGCCCAAAATGCTTGT | |
| | | gttggaaaatgt gcaatatgtgatgtggcaaatctc tattaggaaatattctgtaatctt cagacctagaat aatactagtcttataataggtttg tgactttcctaaatcaattctatt acgtgcaatact tcaatacttcat [Seq Id No 64] | TCTGAACATGGTGCTACTCTTGCTGTCATTGATTCTGAAAAGGACAT GAACTTTCTAAAACGATACGCAG GTAGAGAGGAACACTGGGTTGGACTGAAAAAGGAACCTGGTCACCCA TGGAAGTGGTCAAATGGCAAAGA ATTTAACAACTGGTTCAACGTTACAGGGTCTGACAAGTGTGTTTTTC TGAAAAACACAGAGGTCAGCAGC ATGGAATGTGAGAAGAATTTATACTGGATATGTAACAAACCTTACAA ATAATAAGGAAACATGTTCACTT ATTGACTATTATAGAATGGAACTCAAGGAAATCTGTGTCAGTGGATG CTGCTCTGTGGTCCGAAGTCTTC CATAGAGACTTTGTGAAAAAAAATTTATAGTGTCTTGGGAATTTTC TTCCAAACAGAACTATGGAAAAA AAGGAAGAAATTCCAGGAAAATCTGCACTGTGGGCTTTTATTGCCAT GAGCTAGAAGCATCACAGGTTGA CCAATAACCATGCCCAAGAATGAGAAGAATGACTATGCAACCTTTGG ATGCACTTTATATTATTTTGAAT CCAGAAATAATGAAATAACTAGGCGTGGACTTACTATTTATTGCTGA ATGACTACCAACAGTGAGAGCCC TTCATGCATTTGCACTACTGGAAGGAGTTAGATGTTGGTACTAGATA CTGAATGTAAACAAAGGAATTAT GGCTGGTAACATAGGTTTTTAGTCTAATTGAATCCCTTAAACTCAGG GAGCATTTATAAATGGACAAATG CTTATGAAACTAAGATTTGTAATATTTCTCTCTTTTTAGAGAAATTT GCCAATTTACTTTGTTATTTTTC CCCAAAAAGAATGGGATGATCGTGTATTTATTTTTTTACTTCCTCAG CTGTAGACAGGTCCTTTTCGATG GTACATATTTCTTTGCCTTTATAATCTTTTATACAGTGTCTTACAGA GAAAAGACATAAGCAAAGACTAT GAGGAATATTTGCAAGACATAGAATAGTGTTGGAAAATGTGCAATAT GTGATGTGGCAAATCTCTATTAG GAAATATTCTGTAATCTTCAGACCTAGAATAATACTAGTCTTATAAT AGGTTTGTGACTTTCCTAAATCA ATTCTATTACGTGCAATACTTCAATACTTCATTTAAAATATTTTTAT GTGCAATAAAATGTATTTGTTTG | |
| | | | TATTTTGTGTTCAGTACAATTATAAGCTGTTTTTATATATGTGAAAT AAAAGTAGAATAAACACAAAAAA AAAAAAAAAAAAAAAAAAAAAAA | |
| 210038 _at | protein kinase C, theta | aatccattcatcctgattgggcat gaaatccatggtcaagaggacaag tggaaagtgagagggaaggtttgc tagacaccttcgcttgttatcttg tcaagatagaaaagatagtatcat ttcacccttgccagtaaaaacctt tccatccacccattctcagcagac tccagtattggc acagtcactcactgccattctcac actataacaagaaaagaaatgaag tgcataagtctc ctgggaaaagaaccttaacccctt ctcgtgccatgactggtgatttca tgactcataagc ccctccgtaggcatcattcaagat caatggcccatgcatgctgtttgc agca [Seq Id No 65] | >gi\|48255887\|ref\|NM_006257.2\| Homo sapiens protein kinase C, theta (PRKCQ), mRNA | 19.42 |
| | | | AGTCCCCGCGCAGTCCCCGCGCAGTCCCAGCGCCACCGGGCAGCAGC GGCGCCGTGCTCGCTCCAGGGCG CAACCATGTCGCCATTTCTTCGGATTGGCTTGTCCAACTTTGACTGC GGGTCCTGCCAGTCTTGTCAGGG CGAGGCTGTTAACCCTTACTGTGCTGTGCTCGTCAAAGAGTATGTCG AATCAGAGAACGGGCAGATGTAT ATCCAGAAAAAGCCTACCATGTACCCACCCTGGGACAGCACTTTTGA TGCCCATATCAACAAGGGAAGAG TCATGCAGATCATTGTGAAAGGCAAAAACGTGGACCTCATCTCTGAA ACCACCGTGGAGCTCTACTCGCT GGCTGAGAGGTGCAGGAAGAACAACGGGAAGACAGAAATATGGTTAG AGCTGAAACCTCAAGGCCGAATG CTAATGAATGCAAGATACTTTCTGGAAATGAGTGACACAAAGGACAT GAATGAATTTGAGACGGAAGGCT TCTTTGCTTTGCATCAGCGCCGGGGTGCCATCAAGCAGGCAAAGGTC CACCACGTCAAGTGCCACGAGTT CACTGCCACCTTCTTCCCACAGCCCACATTTTGCTCTGTCTGCCACG AGTTTGTCTGGGGCCTGAACAAA CAGGGCTACCAGTGCCGACAATGCAATGCAGCAATTCACAAGAAGTG TATTGATAAAGTTATAGCAAAGT GCACAGGATCAGCTATCAATAGCCGAGAAACCATGTTCCACAAGGAG AGATTCAAAATTGACATGCCACA CAGATTTAAAGTCTACAATTACAAGAGCCCGACCTTCTGTGAACACT GTGGGACCCTGCTGTGGGGACTG GCACGGCAAGGACTCAAGTGTGATGCATGTGGCATGAATGTGCATCA TAGATGCCAGACAAAGGTGGCCA ACCTTTGTGGCATAAACCAGAAGCTAATGGCTGAAGCGCTGGCCATG ATTGAGAGCACTCAACAGGCTCG | |
| | | | CTGCTTAAGAGATACTGAACAGATCTTCAGAGAAGGTCCGGTTGAAA TTGGTCTCCCATGCTCCATCAAA AATGAAGCAAGGCCGCCATGTTTACCGACACCGGGAAAAAGAGAGCC TCAGGGCATTTCCTGGGAGTCTC CGTTGGATGAGGTGGATAAAATGTGCCATCTTCCAGAACCTGAACTG AACAAAGAAAGACCATCTCTGCA GATTAAACTAAAAATTGAGGATTTTATCTTGCACAAAATGTTGGGGA AAGGAAGTTTTGGCAAGGTCTTC CTGGCAGAATTCAAGAAAACCAATCAATTTTTCGCAATAAAGGCCTT AAAGAAAGATGTGGTCTTGATGG ACGATGATGTTGAGTGCACGATGGTAGAGAAGAGAGTTCTTTCCTTG GCCTGGGAGCATCCGTTTCTGAC GCACATGTTTTGTACATTCCAGACCAAGGAAAACCTCTTTTTTGTGA TGGAGTACCTCAACGGAGGGGAC TTAATGTACCACATCCAAAGCTGCCACAAGTTCGACCTTTCCAGAGC GACGTTTTATGCTGCTGAAATCA TTCTTGGTCTGCAGTTCCTTCATTCCAAAGGAATAGTCTACAGGGAC CTGAAGCTAGATAACATCCTGTT AGACAAAGATGGACATATCAAGATCGCGGATTTTGGAATGTGCAAGG AGAACATGTTAGGAGATGCCAAG ACGAATACCTTCTGTGGGACACCTGACTACATCGCCCCAGAGATCTT GCTGGGTCAGAAATACAACCACT CTGTGGACTGGTGGTCCTTCGGGGTTCTCCTTTATGAAATGCTGATT GGTCAGTCGCCTTTCCACGGGCA GGATGAGGAGGAGCTCTTCCACTCCATCCGCATGGACAATCCCTTTT ACCCACGGTGGCTGGAGAAGGAA GCAAAGGACCTTCTGGTGAAGCTCTTCGTGCGAGAACCTGAGAAGAG GCTGGGCGTGAGGGGAGACATCC GCCAGCACCCTTTGTTTCGGGAGATCAACTGGGAGGAACTTGAACGG AAGGAGATTGACCCACCGTTCCG GCCGAAAGTGAAATCACCATTTGACTGCAGCAATTTCGACAAAGAAT TCTTAAACGAGAAGCCCCGGCTG TCATTTGCCGACAGAGCACTGATCAACAGCATGGACCAGAATATGTT CAGGAACTTTTCCTTCATGAACC | |
| | | | CCGGGATGGAGCGGCTGATATCCTGAATCTTGCCCCTCCAGAGACAG GAAAGAATTTGCCTTCTCCCTGG GAACTGGTTCAAGAGACACTGCTTGGGTTCCTTTTTCAACTTGGAAA AAGAAAGAAACACTCAACAATAA AGACTGAGACCCGTTCGCCCCCATGTGACTTTTATCTGTAGCAGAAA CCAAGTCTACTTCACTAATGACG ATGCCGTGTGTCTCGTCTCCTGACATGTCTCACAGACGCTCCTGAAG TTAGGTCATTACTAACCATAGTT ATTTACTTGAAAGATGGGTCTCCGCACTTGGAAAGGTTTCAAGACTT GATACTGCAATAAATTATGGCTC TTCACCTGGGCGCCAACTGCTGATCAATGAAATGCTTGTTGAATCAG GGGCAAACGGAGTACAGACGTCT CAAGACTGAAACGGCCCCATTGCCTGGTCTAGTAGCGGATCTCACTC AGCCGCAGACAAGTAATCACTAA CCCGTTTTATTCTATTCCTATCTGTGGATGTGTAAATGGCTGGGGGG CCAGCCCTGGATAGGTTTTTATG GGAATTCTTTACAATAAACATAGCTTGTAACTTGAGATCTACAAATC CATTCATCCTGATTGGGCATGAA ATCCATGGTCAAGAGGACAAGTGGAAAGTGAGAGGGAAGGTTTGCTA GACACCTTCGCTTGTTATCTTGT CAAGATAGAAAAGATAGTATCATTTCACCCTTGCCAGTAAAAACCTT TCCATCCACCCATTCTCAGCAGA CTCCAGTATTGGCACAGTCACTCACTGCCATTCTCACACTATAACAA GAAAAGAAATGAAGTGCATAAGT CTCCTGGGAAAAGAACCTTAACCCCTTCTCGTGCCATGACTGGTGAT TTCATGACTCATAAGCCCCTCCG TAGGCATCATTCAAGATCAATGGCCCATGCATGCTGTTTGCAGCAGT CAATTGAGTTGAATTAGAATTCC AACCATACATTTTAAAGGTATTTGTGCTGTGTGTATATTTTGATAAA ATGTTGTGACTTCATGGCAAACA GGTGGATGTGTAAAAATGGAATAAAAAAAAAAAAAGAGTCAAAAAAA AAAAAA | |
| 210915 _x_at | T cell receptor beta variable 19 /// T cell receptor beta constant 1 | Aaaggccacactggtgtgcctggc cacaggtatcttccctgaccacgt ggagctgagctggtgggtgaatgg gaaggaggtgcacagtggggtcag cacggacccgcagcccctcaagga gcagcccgccctcaatgactccag atactgcctgagcagccgcctgag ggtctcggccaccttctggcagaa cccccgcaaccacttccgctgtca agtccagttctacgggctctcgga gaatgacgagtggacccaggatag ggccaaacccgtcacccagatcgt cagcgccgaggcctggggtagagc agactgtggctttacctcggtgtc ctaccagcaaggggtcctgtctgc caccatcctctatgagatcctgct agggaaggccaccatgtatgctgt gctggtcagcgcccttgtgttgat ggccatggtcaagagaaaggattt ctgaaggcagccctggaagtggag ttaggagcttctaacccgtcatgg tttcaatacacattcttcttttgc cagc [Seq Id No 66] | >gi\|339011\|gb\|M15564.1\|HUMTCBYZ Human T-cell receptor rearranged beta-chain V-region (V-D-J) mRNA, complete cds | 48.45 |
| | | | GTAAAGCTCCCATCCTGCCCTGACCCTGCCATGGGCACCAGCCTCCT CTGCTGGATGGCCCTGTGTCTCC TGGGGGCAGATCACGCAGATACTGGAGTCTCCCAGAACCCCAGACAC AACATCACAAAGAGGGGACAGAA TGTAACTTTCAGGTGTGATCCAATTTCTGAACACAACCGCCTTTATT GGTACCGACAGACCCTGGGGCAG GGCCCAGAGTTTCTGACTTACTTCCAGAATGAAGCTCAACTAGAAAA ATCAAGGCTGCTCAGTGATCGGT TCTCTGCAGAGAGGCCTAAGGATCTTTCTCCACCTTGGAGATCCAG CGCACAGAGCAGGGGGACTCGGC CATGTATCTCTGTGCCAGCAGCTTAGCAGGGTTGAATCAGCCCCAGC ATTTTGGTGATGGGACTCGACTC TCCATCCTAGAGGACCTGAACAAGGTGTTCCCACCCGAGGTCGCTGT GTTTGAGCCATCAGAAGCAGAGA TCTCCCACACCCAAAAGGCCACACTGGTGTGCCTGGCCACAGGTATC TTCCCTGACCACGTGGAGCTGAG CTGGTGGGTGAATGGGAAGGAGGTGCACAGTGGGGTCAGCACGGACC CGCAGCCCCTCAAGGAGCAGCCC GCCCTCAATGACTCCAGATACTGCCTGAGCAGCCGCCTGAGGGTCTC GGCCACCTTCTGGCAGAACCCCC GCAACCACTTCCGCTGTCAAGTCCAGTTCTACGGGCTCTCGGAGAAT GACGAGTGGACCCAGGATAGGGC CAAACCCGTCACCCAGATCGTCAGCGCCGAGGCCTGGGGTAGAGCAG ACTGTGGCTTTACCTCGGTGTCC TACCAGCAAGGGGTCCTGTCTGCCACCATCCTCTATGAGATCCTGCT AGGGAAGGCCACCATGTATGCTG TGCTGGTCAGCGCCCTTGTGTTGATGGCCATGGTCAAGAGAAAGGAT TTCTGAAGGCAGCCCTGGAAGTG GAGTTAGGAGCTTCTAACCCGTCATGGTTTCAATACACATTCTTCTT TTGCCAGCGCTTCTGAAGAGCTG CTCTCACCTCTCTGCATCCCAATA | |
| 210972 _x_at | T cell receptor alpha locus /// T cell receptor delta variable 2 /// T cell receptor alpha variable 20 /// T cell receptor alpha joining 17 /// T cell receptor alpha constant | ggaacaagacttcaggtcacgctc gatatccagaaccctgaccctgcc gtgtaccagctgagagactctaaa tccagtgacaagtctgtctgccta ttcaccgattttgattctcaaaca aatgtgtcacaaagtaaggattct gatgtgtatatcacagacaaaact gtgctagacatg aggtctatggacttcaagagcaac agtgctgtggcctggagcaacaaa tctgactttgcatgtgcaaacgcc ttcaacaacagcattattccagaa gacaccttcttccccagcccagaa agttcctgtgatgtcaagctggtc gagaaaagctttgaaacagatacg aacctaaacttt caaaacctgtcagtgattgggttc cgaatcctcctcctgaaagtggcc gggtttaatctg ctcatgacgctgcggctgtggtcc agctgagatctgcaagattgtaag acagcctgtgct ccct [Seq Id No 67] | >gi\|338765\|gb\|M15565.1\|HUMTCAYE Human T-cell receptor rearranged alpha-chain V-region (V-D-J) mRNA, complete cds | 26.45 |
| | | | TCCAAAATGAAGGGTCTGTGGAAGGACATGAATAAAGCACAGGAGGT TGAAGTCAGATTTGCAGCTTTCT AGGCAGGAGACAAGACAATCTGCATCTTCACAGGAGGGATGGCCATG CTCCTGGGGGCATCAGTGCTGAT TCTGTGGCTTCAGCCAGACTGGGTAAACAGTCAACAGAAGAATGATG ACCAGCAAGTTAAGCAAAATTCA CCATCCCTGAGCGTCCAGGAAGGAAGAATTTCTATTCTGAACTGTGA CTATACTAACAGCATGTTTGATT ATTTCCTATGGTACAAAAAATACCCTGCTGAAGGTCCTACATTCCTG ATATCTATAAGTTCCATTAAGGA TAAAAATGAAGATGGAAGATTCACTGTCTTCTTAAACAAAAGTGCCA AGCACCTCTCTCTGCACATTGTG CCCTCCCAGCCTGGAGACTCTGCAGTGTACTTCTGTGCAGCAAAGGG GGCCGGCACTGCCAGTAAACTCA CCTTTGGGACTGGAACAAGACTTCAGGTCACGCTCGATATCCAGAAC CCTGACCCTGCCGTGTACCAGCT GAGAGACTCTAAATCCAGTGACAAGTCTGTCTGCCTATTCACCGATT TTGATTCTCAAACAAATGTGTCA CAAAGTAAGGATTCTGATGTGTATATCACAGACAAAACTGTGCTAGA CATGAGGTCTATGGACTTCAAGA GCAACAGTGCTGTGGCCTGGAGCAACAAATCTGACTTTGCATGTGCA AACGCCTTCAACAACAGCATTAT TCCAGAAGACACCTTCTTCCCCAGCCCAGAAAGTTCCTGTGATGTCA AGCTGGTCGAGAAAAGCTTTGAA ACAGATACGAACCTAAACTTTCAAAACCTGTCAGTGATTGGGTTCCG AATCCTCCTCCTGAAAGTGGCCG GGTTTAATCTGCTCATGACGCTGCGGCTGTGGTCCAGCTGAGATCTG CAAGATTGTAAGACAGCCTGTGC TCCCTCGCTCCTTCCTCTGCATTGCCCCTCTTCTCCCTCTCCAAACA GAGGGAACTCTCCCACCCCCAAG GAGGTGAAAGCTGCTACCACCCTCTGTGCC | |
| 211144 _x_at | T cell receptor gamma constant 2 | aaatgatacactactgctgcagct cacaaacacctctgcatattacat gtacctcctcct gctcctcaagagtgtggtctattt tgccatcatcacctgctgtctgct tggaagaacggc tttctgctgcaatggagagaaatc ataacagacggtggcacaaggagg ccatcttttcct catcggttattgtccctagaagcg tcttctgaggatctagttgggctt tctttctgggtt tgggccatttcagttctcatgtgt gtactattctatcattattgtata atggttttcaaa ccagtgggcacacagagaacctca gtctgtaataacaatgaggaatag ccatggcgatctccagcaccaatc tctccatgttttccacagctcctc cagccaacccaaatagcgcctgct atagtgtagacagcctgcggcttc tagccttgtccctctcttagtgtt ctttaatcagat aactgcctggaagcctttcatttt acacgccc [Seq Id No 68] | >gi\|339406\|gb\|M30894.1\|HUMTCRGAD Human T-cell receptor Ti rearranged gamma-chain mRNA V-J-C region, complete cds | 17.38 |
| | | | GAATCAGGAAGACCAGCTCCTCCTACTGTCTTCTGTGTTACGGGATC AGCGTTCCTTGTTGAGTGGGACC TGAGTTTTGAGAGGGTCTTCTGCTCCTCTTGGTCTGGTCCCTTACTT CCAAGAGCCCCAGAGAGGAAGGC ATGCTGTTGGCTCTAGCTCTGCTTCTAGCTTTCCTGCCTCCTGCCAG TCAGAAATCTTCCAACTTGGAAG GGAGAACAAAGTCAGTCACCAGGCCAACTGGGTCATCAGCTGTAATC ACTTGTGATCTTCCTGTAGAAAA TGCCGTCTACACCCACTGGTACCTACACCAGGAGGGGAAGGCCCCAC AGCGTCTTCTGTACTATGACTCC TACAACTCCAGGGTTGTGTTGGAATCAGGAATCAGTCGAGAAAAGTA TCATACTTATGCAAGCACAGGGA AGAGCCTTAAATTTATACTGGAAAATCTAATTGAACGTGACTCTGGG GTCTATTACTGTGCCACCTGGAA GGATTATTATAAGAAACTCTTTGGCAGTGGAACAACACTTGTTGTCA CAGATAAACAACTTGATGCAGAT GTTTCCCCCAAGCCCACTATTTTTCTTCCTTCGATTGCTGAAACAAA ACTCCAGAAGGCTGGAACATATC TTTGTCTTCTTGAGAAATTTTTCCCAGATATTATTAAGATACATTGG CAAGAAAAGAAGAGCAACACGAT TCTGGGATCCCAGGAGGGGAACACCATGAAGACTAACGACACATACA TGAAATTTAGCTGGTTAACGGTG CCAGAAGAGTCACTGGACAAAGAACACAGATGTATCGTCAGACATGA GAATAATAAAAACGGAATTGATC AAGAAATTATCTTTCCTCCAATAAAGACAGATGTCACCACAGTGGAT CCCAAAGACAGTTATTCAAAAGA TGCAAATGATGTCACCACAGTGGATCCCAAATACAATTATTCAAAGG ATGCAAATGATGTCATCACAATG GATCCCAAAGACAATTGGTCAAAAGATGCAAATGATACACTACTGCT GCAGCTCACAAACACCTCTGCAT ATTACATGTACCTCCTCCTGCTCCTCAAGAGTGTGGTCTATTTTGCC | |
| | | | ATCATCACCTGCTGTCTGCTTGG AAGAACGGCTTTCTGCTGCAATGGAGAGAAATCATAACAGACGGTGG CACAAGGAGGCCATCTTTTCCTC ATCGGTTATTGTCCCTAGAAGCGTCTTCTGAGGATCTAGTTGGGCTT TCTTTCTGGGTTTGGGCCATTTC AGTTCTCATGTGTGTACTATTCTATCATTATTGTATAATGGTTTTCA AACCAGTGGGCACACAGAGAACC TCAGTCTGTAATAACAATGAGGAATAGCCATGGCGATCTCCAGCACC AATCTCTCCATGTTTTCCACAGC TCCTCCAGCCAACCCAAATAGCGCCTGCTATAGTGTAGACAGCCTGC GGCTTCTAGCCTTGTCCCTCTCT TAGTGTTCTTTAATCAGATAACTGCCTGGAAGCCTTTCATTTTACAC GCCCTGAAGCAGTCTTCTTTGCT AGTTGAATTATGTGGTGTGTTTTTCCGTAATAAGCAAAATAAATTT | |
| 211796 _s_at | T cell receptor beta variable 21-1 /// T cell receptor beta variable 19 /// T cell receptor beta variable 5-4 /// T cell | gccatcagaagcagagatctccca cacccaaaaggccacactggtgtg cctggccacaggtttctaccccga ccacgtggagctgagctggtgggt gaatgggaaggaggtgcacagtgg ggtcagcacagacccgcagcccct caaggagcagcccgccctcaatga ctccagatactgcctgagcagccg cctgagggtctcggccaccttctg gcagaacccccgcaaccacttccg ctgtcaagtccagttctacggct ctcggagaatgacgagtggaccca ggatagggccaaacctgtcaccca gatcgtcagcgccgaggcctgggg tagagcagactgtggcttcacctc cgagtcttaccagcaaggggtcct gtctgccaccatcctctatgagat cttgctagggaaggccaccttgta tgctgtgctggtcagtgccctcgt gctgatggccatggtcaagagaaa | >gi\|3002924\|gb\|AF043179.1\|AF043179 Homo sapiens T cell receptor beta chain (TCRBV13S1-TCRBJ2S1) mRNA, complete cds | 63.34 |
| | | | ATGAGCATCGGCCTCCTGTGCTGTGCAGCCTTGTCTCTCCTGTGGGC AGGTCCAGTGAATGCTGGTGTCA CTCAGACCCCAAAATTCCAGGTCCTGAAGACAGGACAGAGCATGACA CTGCAGTGTGCCCAGGATATGAA CCATGAATACATGTCCTGGTATCGACAAGACCCAGGCATGGGGCTGA GGCTGATTCATTACTCAGTTGGT GCTGGTATCACTGACCAAGGAGAAGTCCCCAATGGCTACAATGTCTC CAGATCAACCACAGAGGATTTCC CGCTCAGGCTGCTGTCGGCTGCTCCCTCCCAGACATCTGTGTACTTC TGTGCCAGCAGTTTCCCCCGGCA GCCGTCCTACAATGAGCAGTTCTTCGGGCCAGGGACACGGCTCACCG TGCTAGAGGACCTGAAAAACGTG TTCCCACCCGAGGTCGCTGTGTTTGAGCCATCAGAAGCAGAGATCTC CCACACCCAAAAGGCCACACTGG TGTGCCTGGCCACAGGTTTCTACCCCGACCACGTGGAGCTGAGCTGG TGGGTGAATGGGAAGGAGGTGCA CAGTGGGGTCAGCACAGACCCGCAGCCCCTCAAGGAGCAGCCCGCCC | |
| | receptor beta variable 3-1 /// T cell receptor beta constant 1 | gga [Seq Id No 69] | TCAATGACTCCAGATACTGCCTG AGCAGCCGCCTGAGGGTCTCGGCCACCTTCTGGCAGAACCCCCGCAA CCACTTCCGCTGTCAAGTCCAGT TCTACGGGCTCTCGGAGAATGACGAGTGGACCCAGGATAGGGCCAAA CCTGTCACCCAGATCGTCAGCGC CGAGGCCTGGGGTAGAGCAGACTGTGGCTTCACCTCCGAGTCTTACC AGCAAGGGGTCCTGTCTGCCACC ATCCTCTATGAGATCTTGCTAGGGAAGGCCACCTTGTATGCTGTGCT GGTCAGTGCCCTCGTGCTGATGG CCATGGTCAAGAGAAAGGATTCCAGAGGCTGA | |
| 211902 _x_at | T cell receptor alpha locus | gaatcgtttctctgtgaacttcca gaaagcagccaaatccttcagtct caagatctcaga ctcacagctgggggatgccgcgat gtatttctgtgcttataggagtgc atactctggggc tgggagttaccaactcactttcgg gaaggggaccaaactctcggtcat accaaatatccagaaccctgaccc tgccgtgtaccagctgagagactc taaatccagtgacaagtctgtctg cctattcaccgattttgattctca aacaaatgtgtcacaaagtaagga ttctgatgtgta tatcacagacaaaactgtgctaga catgaggtctatggacttcaagag caacagtgctgt ggcctggagcaacaaatctgactt tgcatgtgcaaacgccttcaacaa cagcattattcc agaagacaccttcttccccagccc agaaagttcctgtgatgtcaagct ggtcgagaaaagctttgaaacaga tacgaacctaaactttcaaaacct | >gi\|1100165\|gb\|L34703.1\|HUMTCRAZ Homo sapiens T-cell receptor alpha chain (TCRA) mRNA (HLA-A1, 24; B7, 8; DR 1, 3), complete cds | 20.68 |
| | | | ATGGCATGCCCTGGCTTCCTGTGGGCACTTGTGATCTCCACCTGTCT TGAATTTAGCATGGCTCAGACAG TCACTCAGTCTCAACCAGAGATGTCTGTGCAGGAGGCAGAGACCGTG ACCCTGAGCTGCACATATGACAC CAGTGAGAGTGATTATTATTTATTCTGGTACAAGCAGCCTCCCAGCA GGCAGATGATTCTCGTTATTCGC CAAGAAGCTTATAAGCAACAGAATGCAACAGAGAATCGTTTCTCTGT GAACTTCCAGAAAGCAGCCAAAT CCTTCAGTCTCAAGATCTCAGACTCACAGCTGGGGGATGCCGCGATG TATTTCTGTGCTTATAGGAGTGC ATACTCTGGGGCTGGGAGTTACCAACTCACTTTCGGGAAGGGGACCA AACTCTCGGTCATACCAAATATC CAGAACCCTGACCCTGCCGTGTACCAGCTGAGAGACTCTAAATCCAG TGACAAGTCTGTCTGCCTATTCA CCGATTTTGATTCTCAAACAAATGTGTCACAAAGTAAGGATTCTGAT GTGTATATCACAGACAAAACTGT GCTAGACATGAGGTCTATGGACTTCAAGAGCAACAGTGCTGTGGCCT GGAGCAACAAATCTGACTTTGCA TGTGCAAACGCCTTCAACAACAGCATTATTCCAGAAGACACCTTCTT CCCCAGCCCAGAAAGTTCCTGTG ATGTCAAGCTGGTCGAGAAAAGCTTTGAAACAGATACGAACCTAAAC | |
| | | gtcagtgattgggttccgaatcct cctcctgaaagtggccgggtttaa tctgctcatgacgctgcggttgtg gtcc [Seq Id No 70] | TTTCAAAACCTGTCAGTGATTGG GTTCCGAATCCTCCTCCTGAAAGTGGCCGGGTTTAATCTGCTCATGA CGCTGCGGTTGTGGTCCAGCTGA | |
| 212999 _x_at | Major histocompa tibility complex, class II, DQ beta 1 /// Major histocompa tibility complex, class II, DQ beta 1 | cactgcagaatgaaggaacatccc ttgaggtgacccagccaacctgtg gccagaaggaggnttgtaccttga aaagacactgaaagcattttggng tgtnaagtaagggtgggcagagga ggtagaaaatcaattcaattgtcg catcattcatggttctttaatatt gatgctcagtgcantggcctnaga atatcccagcctctcttctggttt gntgagtgctntntaagtaagcat ggtngaattgtttggggncanata tagtganccttggtcactggtgtt tcaaacattctg gnaagtcacatcnatcaagaatan tttttanttttaagaaagcataac cagcaataaa [Seq Id No 71] | >gi\|24797068\|ref\|NM_002123.2\| Homo sapiens major histocompatibility complex, class II, DQ beta 1 (HLA-DQB1), mRNA | 15.79 |
| | | | CAGATCCATCAGGTCCGAGCTGTGTTGACTACCACTTTTCCCTTCGT CTCAATTATGTCTTGGAAAAAGG CTTTGCGGATCCCGGAGGCCTTCGGGCAGCAACTGTGACCTTGATG CTGTCGATGCTGAGCACCCCAGT GGCTGAGGGCAGAGACTCTCCCGAGGATTTCGTGTACCAGTTTAAGG GCATGTGCTACTTCACCAACGGG ACAGAGCGCGTGCGTCTTGTGAGCAGAAGCATCTATAACCGAGAAGA GATCGTGCGCTTCGACAGCGACG TGGGGGAGTTCCGGGCGGTGACGCTGCTGGGGCTGCCTGCCGCCGAG TACTGGAACAGCCAGAAGGACAT CCTGGAGAGGAAACGGGCGGCGGTGGACAGGGTGTGCAGACACAACT ACCAGTTGGAGCTCCGCACGACC TTGCAGCGGCGAGTGGAGCCCACAGTGACCATCTCCCCATCCAGGAC AGAGGCCCTCAACCACCACAACC TGCTGGTCTGCTCGGTGACAGATTTCTATCCAGCCCAGATCAAAGTC CGGTGGTTTCGGAATGACCAGGA GGAGACAGCTGGCGTTGTGTCCACCCCCTTATTAGGAATGGTGACT GGACCTTCCAGATCCTGGTGATG CTGGAAATGACTCCCCAGCGTGGAGACGTCTACACCTGCCACGTGGA GCACCCCAGCCTCCAGAGCCCCA TCACCGTGGAGTGGCGGGCTCAATCTGAATCTGCCCAGAGCAAGATG CTGAGTGGCATTGGAGGCTTCGT GCTGGGGCTGATCTTCCTCGGGCTGGGCCTTATCATCCATCACAGGA GTCAGAAAGGGCTCCTGCACTGA CTCCTGAGACTATTTTAACTGGGATTGGTTATCACTTTTCTGTAACG | |
| | | | CCTGCTTGTCCCTGCCCAGAATT CCCAGCTGTCTGTGTCAGCCTGTCCCCCTGAGATCAGAGTCCTACAG TGGCTGTCACGCAGCCACCAGGT CATCTCCTTTCATCCCCACCTTGAGGCGGATGGCTGTGACCCTACTT CCTGCACTGACCCACAGCCTCTG CCTGTGCACGGCCAGCTGCATCTACTCAGGCCCCAAGGGGTTTCTGT TTCTATTCTCTCCTCAGACTGCT CAAGAGAAGCACATGAAAACCATTACCTGACTTTAGAGCTTTTTTAC ATAATTAAACATGATCCTGAGTT | |
| 213193 _x_at | T cell receptor beta variable 19 /// T cell receptor beta variable 19 /// T cell receptor beta constant 1 /// T cell receptor beta constant 1 | Tgactccagatactgcctgagcag ccgcctgagggtctcggccacctt ctggcagaacccccgcaaccactt ccgctgtcaagtccagttctacgg gctctcggagaatgacgagtggac ccaggatagggccaaacccgtcac ccagatcgtcagcgccgaggcctg ggtagagcagactgtggctttac ctcggtgtcctaccagcaaggggt cctgtctgccaccatcctctatga gatcctgctagggaaggccacccct gtatgctgtgctggtcagcnccct tgtgttgatggccatggtcaagag aaaggatttctgaaggcagccctg gaagtggagttaggagcttctaac ccgtcatggtttcaatacacattc ttcttttgccagcgcttctgaaga gctgctctcacctctctgcatccc aatagatatccccctatgtgcatg cacacctgcacactcacggctgaa atctccctaacccaggggaccttt agcatgcctaagtga [Seq Id No 72] | >gi\|46184507\|gb\|AL559122.3\|AL559122 AL559122 Homo sapiens T CELLS (JURKAT CELL LINE) COT 10-NORMALIZED Homo sapiens cDNA clone CS0DJ014YE01 5-PRIME, mRNA sequence | 44.75 |
| | | | TGCCTGGCCACAGGCTTCTTCCCCGACCACGTGGAGCTGAGCTGGTG GGTGAATGGGAAGGAGGTGCACA ATGGGGTCAACACAGACCCGCAGCCCCTCAAAGAACAGCCCGCCCTC AATGACTCCAAATAMTGCCTGAG CAGCCGCCTGAGGGTCTCGGCCACCTTCTGGCAGAACCCCCGCAACC ACTTCCGCTGTCAAGTCCAGTTC TACGGGCTCTCGGAGAATGACGAGTGGACCCAGGATAGGGCCAAACC CGTCACCCAAATCGTCAGCGCCG AGGCCTGGGGTARAGCARAMTGTGGCTTTACCTCGGTGTCCTACCAA CAAGGGGTCCTGTCTGCCACCAT CCTCTATGARATCCTGCTAGGGAAGGCCACCCTGTATGCTGTGCTGG TCAGCGCCCTTGTGTTGATGGCC ATGGTCAAGAGAAAGGATTTCTGAAGGCAGCCCTGGAAGTGGAATTA AGAACTTCTAACCCGTCATGGTT TCAATACACATTCTTCTTTTGCCAGCGCTTCTGAAGAGCTGCTCTCA CCTCTCTGCATCCCAATAGATAT CCCCCTATGTGCATGCACACCTGCACACTCACGGCTGAAATCTCCCT AACCCAGGGGGACCTTAGCATGC CTAAGTGACTAAACCAATAAAAATGTTCTGGTCTGGCCTGA | |
| 213539 _at | CD3D antigen, delta polypeptide (TiT3 complex) | gggaacactgctctcagacattac aagactggacctgggaaaacgcat cctggacccacgaggaatatatag gtgtaatgggacagatatatacaa ggacaaagaatctaccgtgcaagt tcattatcgaatgtgccagagctg tgtggagctggatccagccaccgt ggctggcatcattgtcactgatgt cattgccactctgctccttgcttt gggagtcttctgctttgctggaca tgagactggaaggctgtctggggc tgccgacacacaagctctgttgag gaatgaccaggtctatcagcccct ccgagatcgagatgatgctcagta cagccaccttggaggaaactgggc tcggaacaagtgaacctgagactg gtggcttctagaagcagccattac caactgtacct [Seq Id No 73] | >gi\|98985799\|ref\|NM_000732.4\| Homo sapiens CD3d molecule, delta (CD3-TCR complex) (CD3D), transcript variant 1, mRNA | 34.66 |
| | | | AGAGAAGCAGACATCTTCTAGTTCCTCCCCCACTCTCCTCTTTCCGG TACCTGTGAGTCAGCTAGGGGAG GGCAGCTCTCACCCAGGCTGATAGTTCGGTGACCTGGCTTTATCTAC TGGATGAGTTCCGCTGGGAGATG GAACATAGCACGTTTCTCTCTGGCCTGGTACTGGCTACCCTTCTCTC GCAAGTGAGCCCCTTCAAGATAC CTATAGAGGAACTTGAGGACAGAGTGTTTGTGAATTGCAATACCAGC ATCACATGGGTAGAGGGAACGGT GGGAACACTGCTCTCAGACATTACAAGACTGGACCTGGGAAAACGCA TCCTGGACCCACGAGGAATATAT AGGTGTAATGGGACAGATATATACAAGGACAAAGAATCTACCGTGCA AGTTCATTATCGAATGTGCCAGA GCTGTGTGGAGCTGGATCCAGCCACCGTGGCTGGCATCATTGTCACT GATGTCATTGCCACTCTGCTCCT TGCTTTGGGAGTCTTCTGCTTTGCTGGACATGAGACTGGAAGGCTGT CTGGGGCTGCCGACACACAAGCT CTGTTGAGGAATGACCAGGTCTATCAGCCCCTCCGAGATCGAGATGA TGCTCAGTACAGCCACCTTGGAG GAAACTGGGCTCGGAACAAGTGAACCTGAGACTGGTGGCTTCTAGAA GCAGCCATTACCAACTGTACCTT CCCTTCTTGCTCAGCCAATAAATATATCCTCTTTCACTCAGAAAAAA AAAAAAAAAAAAAAAAAAAAAAA A | |
| 215806 _x_at | T cell receptor gamma constant 2 /// T cell | aaatgatacactactgctgcagct cacaaacacctctgcatattacat gtacctcctcct gctcctcaagagtgtggtctattt tgccatcatcacctgctgtctgcn tgnaagaacggc nnnctgctgcaatggagagaantc | >gi\|339168\|gb\|M13231.1\|HUMTCGXH Human T-cell receptor aberrantly rearranged gamma-chain mRNA from cell line HPB-MLT | 19.96 |
| | | | GGCATGCGGTGGGCCCTACTGGTGCTTCTAGCTTTCCTGTCTCCTGC CAGTCAGAAATCTTCCAACTTGG AAGGGAGAACGAAGTCAGTCACCAGGCAGACTGGGTCATCTGCTGAA ATCACTTGCGATCTTACTGTAAC | |
| | receptor gamma variable 9 /// similar to T-cell receptor gamma chain C region PT-gamma-1/2 /// similar to T-cell receptor gamma chain V region PT-gamma-1/2 precursor /// TCR gamma alternate reading frame protein | ataacagacggtggcacaaggagg ccnncntntcctcatcggnnattg tccctagaagcgtcttctgaggat ctagttgggctttctttctgggtt tgggccatttcagttctcatgtgt gtactattctatcattattgtata atggttttcaaa ccagtgggcacacagagaacctca gtctgtaataacaatgaggaatag ccatggcgatctccagcaccaatc tctccatgttttccacagctcctc cagccaacccaaatagcgcctgct atagtgtaganannctgcggcttc tagccttgtccctctcttagtgtt ctttaatcagat aactgcctggaagcctttcatttt acacgccctgaagcagtcttcttt gcta [Seq Id No 74] | AAATACCTTCTACATCCACTGGTACCTACACCAGGAGGGAAGGCCC CACAGCGTCTTCTGTACTATGAC GTCTCCACTGCAAGGGATGTGTTGGAATCAGGACTCAGTCCAGGAAA GTATTATACTCATACACCCAGGA GGTGGAGCTGGATATTGAGACTGCAAAATCTAATTGAAAATGATTCT GGGGTCTATTACTGTGCCACCTG GGACAGGCAAAAATTATTATAAGAAACTCTTTGGCAGTGGAACAACA CTTGTTGTCACAGATAAACAACT TGATGCAGATGTTTCCCCCAAGCCCACTATTTTTCTTCCTTCAATTG CTGAAACAAAACTCCAGAAGGCT GGAACATACCTTTGTCTTCTTGAGAAATTTTTCCCAGATATTATTAA GATACATTGGCAAGAAAAGAAGA GCAACACGATTCTGGGATCCCAGGAGGGGAACACCATGAAGACTAAC GACACATACATGAAATTTAGCTG GTTAACGGTGCCAGAAGAGTCACTGGACAAAGAACACAGATGTATCG TCAGACATGAGAATAATAAAAAC GGAATTGATCAAGAAATTATCTTTCCTCCAATAAAGACAGATGTCAC CACAGTGGATCCCAAAGACAGTT ATTCAAAAGATGCAAATGATGTCATCACAATGGATCCCAAAGACAAT TGGTCAAAAGATGCAAATGATAC ACTACTGCTGCAGCTCACAAACACCTCTGCATATTACATGTACCTCC TCCTGCTCCTCAAGAGTGTGGTC TATTTTGCCATCATCACCTGCTGTCTGCTTGGAAGAACGGCTTTCTG CTGCAATGGAGAGAAATCATAAC AGACGGTGGCACAAGGAGGCCATCTTTTCCTCATCGGTTATTGTCCC TAGAAGCGTCTTCTGAGGATCTA GTTGGGCTTTCTTTCTGGGTTTGGGCCATTTCAGTTCTCATGTGTGT ACTATTCTATCATTATTGTATAA TGGTTTTCAAACCAGTGGGCACACAGAGAACCTCAGTCTGTAATAAC AATGAGGAATAGCCATGGCGATC TCCAGCACCAATCTCTCCATGTTTTCCACAGCTCCTCCAGCCAACCC AAATAGCGCCTGCTATAGTGTAG ACAGCCTGCGGCTTCTAGCCTTGTCCCTCTCTTAGTGTTCTTTAATC AGATAACTGCCTGGAAGCCTTTC | |
| | | | ATTTTACACGCCCTGAAGCAGTCTTCTTTGCTAGTTGAATTATGTGG TGTGTTTTTCCGTAATAAGCAAA ATAAATTTAAAAAAATGAAAA | |
| 216748 _at | pyrin and HIN domain family, member 1 | ccaccctctggatcccaatattga gatcttatcctcaggaatcctca cttagacccctg taacaggttaaatcttcatggtgt tctgtttcctaggaacttctttct tttctactgttt atgacaactgaagttaataagtgt ttatctttcccacctactcaaagt agttccaagatt agggctagtttgtaattctgtgga ccactgtaaacgagggcctagttc agtgtctgcctc atggaagcttccaataaatacct ttg [Seq Id No 75] | >gi\|10437303\|dbj\|AK024890.1\| Homo sapiens cDNA: FLJ21237 fis, clone COL01114 | 10.74 |
| | | | GTCAAAGGAATAATCCCATCTAAAAAGACGAAACAGAAAGAAGTGTA TCCTGCTACACCTGCATGCACCC CAAGCAACCGTCTCACAGCTAAAGGAGCAGAGGAGACTCTTGGACCT CAGGTAAGCTTCAGGAAGAGGAG CAGGCTTCAAGTCTCACAGTGGAAGCTCTGCTGTGGCTGTTCCACTC AATCTGTCCAGCAGGCAGTTATT TCTTCATATGTTTCCCATCAAGTTTCAGATTTATCAAATTACATAAT AATTGATCATCTTTCTGCAAGGC AACAAGTTAAACGCTTTAGTAAACATAATGTAAATATACATAAAATA AATATAATATTTTCATCTCCAAT AGAGAAGGATGTTAACTTGAGAGTCAGATAAAAAAACGTTTGCCTAT GTTTACAAAAGCCTAGTTTCTTA ACTGCAAGTCAGCATATCCCAAAACACAAGTAATTAAGGAATGATGT GTGTTACTTTCTCTGCTCCCTTT TTAAAAATGAAACCATCTATGCCATGTTCTTTCAATTGGCCTGGGGA TGTACTTAAGTTTCCAAGAAAAA CAATTTATATACAATAAATATATTACCTTGTAATGAAAATGTGCTCT GCTTCATTTGACACTGAAAGTAA TTAACAAGAAAAATAAACTACTTGTAGAAAAGAAAAAAACCATCTGA AGAAGAGACTGGAACCAAAAGGA GTAAGATGTCCAAAGAGCAGACTCGGCCTTCCTGCTCTGCAGGAGCC AGCACGTCCACAGCCATGGGCCG TTCCCCACCTCCCCAGACCTCATCATCAGCTCCACCCAACACTTCCT CAACTGAGGTACACTCTTCCTGG TCCCCTTTTGATTCATTTTCTTCAACCCAAAATGTAGGAATCTGATT TCATCTTCTACTGAAAAATGACA TCAATCATCAGCCAGTAAATCAAATGTATAGACTGAGAATTAACTGC ATTTTAATCTTTTGCTTCCACAG | |
| | | | GCATATTTGATGAACTTGACATTATCTCTGACTGCAGGAAGTTTTCT GTCCTGTGCTGTTTGGGGAAGAG ACAGAGAACTGCGGAATCTGGAACTTTCAGCAACAGACTCACTGTCT ACTGCCCCCATCTATTATACACC CATTCCCTTTGCTCACTAATTTGTTCAAGTTTCTCTGACATACACCA TGCTCCTTTTTCCTTTAGGATTT TCACACACCATATTTCTTTCACCTTTAAACTCTTACCTGGCCAACCC TATCCACCCTCTGGATCCCAATA TTGAGATCTTATCCTCAGGGAATCCTCACTTAGACCCCTGTAACAGG TTAAATCTTCATGGTGTTCTGTT TCCTAGGAACTTCTTTCTTTTCTACTGTTTATGACAACTGAAGTTAA TAAGTGTTTATCTTTCCCACCTA CTCAAAGTAGTTCCAAGATTAGGGCTAGTTTGTAATTCTGTGGACCA CTGTAAACGAGGGCCTAGTTCAG TGTCTGCCTCATGGGAAGCTTCCAATAAATACCTTTGCTCAACGAAA AAATGAAAACCCAGTGGCTCACG CCTGTAATCCCAGCACTTTGGGAGGCCGAGGCAGGTGGATTGCCTGA GGTCAGGAGTTTGAGACTAGTCT GGCCAACATGGTGAAACACTATCTCTACTAAAAATACAAAAAAATTA GCTGGGTATGGTGGCTTACGCCT ATAATCCCAGCTACTCAGGAGGCTGAGGCAGGGGAATTGCTTGAACC AGGGAGGTGGAGGTTGCAGTGAG CTGAGATCGCACCACTGCACTCCAGCCTGGGTGACAGAGCGAGACTC CATCTCAAAATAAAAAAGTAAAA AAAAAAAAAAAAAA | |
| 217147 _s_at | T cell receptor associated transmembrane adaptor 1 | tctcctttctcaccaatgggcaat agcccataattgaaataaatttct gattgaaaggta taggaaacattaaaatgcattact aagagaagtaatataattttctta caaagtatttt cccaaagatagctttactatttca aaaattgtcaaattaatgcatgct ccttacaacaaa | >gi\|54607136\|ref\|NM_016388.2\| Homo sapiens T cell receptor associated transmembrane adaptor 1 (TRAT1), mRNA | 24.16 |
| | | | GAGGCACAGATAAAGATAAGTTTTACTGTCATGCTGCTTTTAACATA ACAGAGCAACATCACCTAGGAAA AAAGTTTGTAGGAGGATTTTTAATCCATATATTTGTCTTATGGCTAG ATAAAGATTTCTCTGAAAAAAAG AAGCATGTCAGGAATCTCTGGGTGCCCCTTTTTCCTCTGGGGACTTC TAGCATTGTTGGGCTTGGCTTTG | |
| | | caaatatcaaaaagagtttaggaa ttctactagccagagatagtcact tggagaaacttt ctatatatccttctaaatattttt ctgggcatgctcatgtatgtacat cagttgtttctt tttattttgaaccaaaaatgtggt ttcttttgtacacattacttaaac tttctttccagt caacaatatattgtggatttattt tcactgttatatttaactatatat aaatacgcatat attgtaattttaatgtctgcttag caccccactgataaccaaatcaca g [Seq Id No 76] | GTTATATCACTGATCTTCAATATTTCCCACTATGTGGAAAAGCAACG ACAAGATAAAATGTACAGCTACT CCAGTGACCACACCAGGGTTGATGAGTATTATATTGAAGACACACCA ATTTATGGTAACTTAGATGATAT GATTTCAGAACCAATGGATGAAAATTGCTATGAACAAATGAAAGCCC GACCAGAGAAATCTGTAAATAAG ATGCAGGAAGCCACCCCATCTGCACAGGCAACCAATGAAACACAGAT GTGCTACGCCTCACTTGATCACA GCGTTAAGGGGAAGCGTAGAAAGCCCAGGAAACAGAATACTCATTTC TCAGACAAGGATGGAGATGAGCA ACTACATGCAATAGATGCCAGCGTTTCTAAGACCACCTTAGTAGACA GTTTCTCCCCAGAAAGCCAGGCA GTAGAGGAAAACATTCATGATGATCCCATCAGACTGTTTGGATTGAT CCGTGCTAAGAGAGAACCTATAA ACTAGCTGGACCATGATCTAGTTCAATGATTTGGCTCCTATTGAAGA TGGCTTCTAAGAAAACAAGATGC ACAGAGGACACAGAAGGACTTGGCAGCAGGGTGATGACCTGATCATT TGTTGATGGGATGGTGGCTTACC TCTTATTCACAGCTTACACTTATGCATGCCAAATGTAAGGCCATGAA AATCAGTATTTCAAATAACTTAA AAAATGCTTTACTACTAAAATGTAAAAAATTAATGTGCTCACCTCGG CAGCACATATACTAAAAATTAAT AAGACCCAGCTTGAAAATTGAGCCTGATAACAAGATTACAAATTCAC AATACCTAATACTTAGGGAAATA TAAAAATTTAAGCATGAATGCGTTCTGGAACACGTTAGAAGAAAAAT AAAAGCCAATGAGTTTTTTTTA ATTCTCCTTTCTCACCAATGGGCAATAGCCCATAATTGAAATAAATT TCTGATTGAAAGGTATAGGAAAC ATTAAAATGCATTACTAAGAGAAGTAATATAATTTTCTTACAAAGTA TTTTTCCCAAAGATAGCTTTACT ATTTCAAAAATTGTCAAATTAATGCATGCTCCTTACAACAAACAAAT ATCAAAAAGAGTTTAGGAATTCT ACTAGCCAGAGATAGTCACTTGGAGAAACTTTCTATATATCCTTCTA AATATTTTTCTGGGCATGCTTAT | |
| | | | GTATGTACATCAGTTGTTTCTTTTTATTTTGAACCAAAAATGTGGTT TCTTTTGTACACATTACTTAAAC TTTCTTTCCAGTCAACAATATATTGTGGATTTATTTTCACTGTTATA TTTAACTATATATAAATACGCAT ATATTGTAATTTTAATGTCTGCTTAGCACCCCACTGATAACCAAATC ACAGTTTATTTAAATAATTTTAA TGACTTTTCAAAAACAATTTATTGATGCAAAAAGCAAGGTTGAGATG ACAATGTTTCTTTCAATAATTAA AAAATACTGCTTCAC | |
| 219159 _s_at | SLAM family member 7 | Gagagtggacatttgtcgggaaac tcctaacatatgcccccattctgg agagaacacagagtacgacacaat ccctcacactaatagaacaatcct a [Seq Id No 77] | >gi\|19923571\|ref\|NM_021181.3\| Homo sapiens SLAM family member 7 (SLAMF7), mRNA | 15.84 |
| | | | CTTCCAGAGAGCAATATGGCTGGTTCCCCAACATGCCTCACCCTCAT CTATATCCTTTGGCAGCTCACAG GGTCAGCAGCCTCTGGACCCGTGAAAGAGCTGGTCGGTTCCGTTGGT GGGGCCGTGACTTTCCCCCTGAA GTCCAAAGTAAAGCAAGTTGACTCTATTGTCTGGACCTTCAACACAA CCCCTCTTGTCACCATACAGCCA GAAGGGGGCACTATCATAGTGACCCAAAATCGTAATAGGGAGAGAGT AGACTTCCCAGATGGAGGCTACT CCCTGAAGCTCAGCAAACTGAAGAAGAATGACTCAGGGATCTACTAT GTGGGGATATACAGCTCATCACT CCAGCAGCCCTCCACCCAGGAGTACGTGCTGCATGTCTACGAGCACC TGTCAAAGCCTAAAGTCACCATG GGTCTGCAGAGCAATAAGAATGGCACCTGTGTGACCAATCTGACATG CTGCATGGAACATGGGGAAGAGG ATGTGATTTATACCTGGAAGGCCCTGGGGCAAGCAGCCAATGAGTCC CATAATGGGTCCATCCTCCCCAT CTCCTGGAGATGGGGAGAAAGTGATATGACCTTCATCTGCGTTGCCA GGAACCCTGTCAGCAGAAACTTC TCAAGCCCCATCCTTGCCAGGAAGCTCTGTGAAGGTGCTGCTGATGA CCCAGATTCCTCCATGGTCCTCC TGTGTCTCCTGTTGGTGCCCCTCCTGCTCAGTCTCTTTGTACTGGGG CTATTTCTTTGGTTTCTGAAGAG | |
| | | | AGAGAGACAAGAAGAGTACATTGAAGAGAAGAAGAGAGTGGACATTT GTCGGGAAACTCCTAACATATGC CCCCATTCTGGAGAGAACACAGAGTACGACACAATCCCTCACACTAA TAGAACAATCCTAAAGGAAGATC CAGCAAATACGGTTTACTCCACTGTGGAAATACCGAAAAAGATGGAA AATCCCCACTCACTGCTCACGAT GCCAGACACACCAAGGCTATTTGCCTATGAGAATGTTATCTAGACAG CAGTGCACTCCCCTAAGTCTCTG CTCAAAAAAAAAACAATTCTCGGCCCAAAGAAAACAATCAGAAGAAT TCACTGATTTGACTAGAAACATC AAGGAAGAATGAAGAACGTTGACTTTTTCCAGGATAAATTATCTCT GATGCTTCTTTAGATTTAAGAGT TCATAATTCCATCCACTGCTGAGAAATCTCCTCAAACCCAGAAGGTT TAATCACTTCATCCCAAAAATGG GATTGTGAATGTCAGCAAACCATAAAAAAAGTGCTTAGAAGTATTCC TATAGAAATGTAAATGCAAGGTC ACACATATTAATGACAGCCTGTTGTATTAATGATGGCTCCAGGTCAG TGTCTGGAGTTTCATTCCATCCC AGGGCTTGGATGTAAGGATTATACCAAGAGTCTTGCTACCAGGAGGG CAAGAAGACCAAAACAGACAGAC AAGTCCAGCAGAAGCAGATGCACCTGACAAAAATGGATGTATTAATT GGCTCTATAAACTATGTGCCCAG CACTATGCTGAGCTTACACTAATTGGTCAGACGTGCTGTCTGCCCTC ATGAAATTGGCTCCAAATGAATG AACTACTTTCATGAGCAGTTGTAGCAGGCCTGACCACAGATTCCCAG AGGGCCAGGTGTGGATCCACAGG ACTTGAAGGTCAAAGTTCACAAAGATGAAGAATCAGGGTAGCTGACC ATGTTTGGCAGATACTATAATGG AGACACAGAAGTGTGCATGGCCCAAGGACAAGGACCTCCAGCCAGGC TTCATTTATGCACTTGTGCTGCA AAAGAAAAGTCTAGGTTTTAAGGCTGTGCCAGAACCCATCCCAATAA AGAGACCGAGTCTGAAGTCACAT TGTAAATCTAGTGTAGGAGACTTGGAGTCAGGCAGTGAGACTGGTGG GGCACGGGGGGCAGTGGGTACTT | |
| | | | GTAAACCTTTAAAGATGGTTAATTCATTCAATAGATATTTATTAAGA ACCTATGCGGCCCGGCATGGTGG CTCACACCTGTAATCCCAGCACTTTGGGAGGCCAAGGTGGGTGGGTC ATCTGAGGTCAGGAGTTCAAGAC CAGCCTGGCCAACATGGTGAAACCCCATCTCTACTAAAGATACAAAA ATTTGCTGAGCGTGGTGGTGTGC ACCTGTAATCCCAGCTACTCGAGAGGCCAAGGCATGAGAATCGCTTG AACCTGGGAGGTGGAGGTTGCAG TGAGCTGAGATGGCACCACTGCACTCCGGCCTAGGCAACGAGAGCAA AACTCCAATACAAACAAACAAAC AAACACCTGTGCTAGGTCAGTCTGGCACGTAAGATGAACATCCCTAC CAACACAGAGCTCACCATCTCTT ATACTTAAGTGAAAAACATGGGGAAGGGGAAAGGGGAATGGCTGCTT TTGATATGTTCCCTGACACATAT CTTGAATGGAGACCTCCCTACCAAGTGATGAAAGTGTTGAAAAACTT AATAACAAATGCTTGTTGGGCAA GAATGGGATTGAGGATTATCTTCTCTCAGAAAGGCATTGTGAAGGAA TTGAGCCAGATCTCTCTCCCTAC TGCAAAACCCTATTGTAGTAAAAAAGTCTTCTTTACTATCTTAATAA AACAGATATTGTGAGATTCAAAA AAAAAAAAAAAA | |
| 222838 _at | SLAM family member 7 | aacacctgtgctaggtcagtctgg cacgtaagatgaacatccctacca acacagagctcaccatctcttata cttaagtgaaaaacatggggaagg ggaaaggggaatggctgcttttga tatgttccctgacacatatcttga atggagacctccctaccaagtgat gaaagtgttgaa aaacttaataacaaatgcttgttg ggcaagaatgggattgaggattat cttctctcagaa aggcattgtgaaggaattgagcca | >gi\|19923571\|ref\|NM_021181.3\| Homo sapiens SLAM family member 7 (SLAMF7), mRNA | 22.54 |
| | | | CTTCCAGAGAGCAATATGGCTGGTTCCCCAACATGCCTCACCCTCAT CTATATCCTTTGGCAGCTCACAG GGTCAGCAGCCTCTGGACCCGTGAAAGAGCTGGTCGGTTCCGTTGGT GGGGCCGTGACTTTCCCCCTGAA GTCCAAAGTAAAGCAAGTTGACTCTATTGTCTGGACCTTCAACACAA CCCCTCTTGTCACCATACAGCCA GAAGGGGGCACTATCATAGTGACCCAAAATCGTAATAGGGAGAGAGT AGACTTCCCAGATGGAGGCTACT CCCTGAAGCTCAGCAAACTGAAGAAGAATGACTCAGGGATCTACTAT GTGGGGATATACAGCTCATCACT | |
| | | gatctctctccctactgcaaaacc ctattgtagta [Seq Id No 78] | CCAGCAGCCCTCCACCCAGGAGTACGTGCTGCATGTCTACGAGCACC TGTCAAAGCCTAAAGTCACCATG GGTCTGCAGAGCAATAAGAATGGCACCTGTGTGACCAATCTGACATG CTGCATGGAACATGGGGAAGAGG ATGTGATTTATACCTGGAAGGCCCTGGGGCAAGCAGCCAATGAGTCC CATAATGGGTCCATCCTCCCCAT CTCCTGGAGATGGGGAGAAAGTGATATGACCTTCATCTGCGTTGCCA GGAACCCTGTCAGCAGAAACTTC TCAAGCCCCATCCTTGCCAGGAAGCTCTGTGAAGGTGCTGCTGATGA CCCAGATTCCTCCATGGTCCTCC TGTGTCTCCTGTTGGTGCCCCTCCTGCTCAGTCTCTTTGTACTGGGG CTATTTCTTTGGTTTCTGAAGAG AGAGAGACAAGAAGAGTACATTGAAGAGAAGAAGAGAGTGGACATTT GTCGGGAAACTCCTAACATATGC CCCCATTCTGGAGAGAACACAGAGTACGACACAATCCCTCACACTAA TAGAACAATCCTAAAGGAAGATC CAGCAAATACGGTTTACTCCACTGTGGAAATACCGAAAAAGATGGAA AATCCCCACTCACTGCTCACGAT GCCAGACACACCAAGGCTATTTGCCTATGAGAATGTTATCTAGACAG CAGTGCACTCCCCTAAGTCTCTG CTCAAAAAAAAAACAATTCTCGGCCCAAAGAAAACAATCAGAAGAAT TCACTGATTTGACTAGAAACATC AAGGAAGAATGAAGAACGTTGACTTTTTTCCAGGATAAATTATCTCT GATGCTTCTTTAGATTTAAGAGT TCATAATTCCATCCACTGCTGAGAAATCTCCTCAAACCCAGAAGGTT TAATCACTTCATCCCAAAAATGG GATTGTGAATGTCAGCAAACCATAAAAAAAGTGCTTAGAAGTATTCC TATAGAAATGTAAATGCAAGGTC ACACATATTAATGACAGCCTGTTGTATTAATGATGGCTCCAGGTCAG TGTCTGGAGTTTCATTCCATCCC AGGGCTTGGATGTAAGGATTATACCAAGAGTCTTGCTACCAGGAGGG CAAGAAGACCAAAACAGACAGAC AAGTCCAGCAGAAGCAGATGCACCTGACAAAAATGGATGTATTAATT GGCTCTATAAACTATGTGCCCAG | |
| | | | CACTATGCTGAGCTTACACTAATTGGTCAGACGTGCTGTCTGCCCTC ATGAAATTGGCTCCAAATGAATG AACTACTTTCATGAGCAGTTGTAGCAGGCCTGACCACAGATTCCCAG AGGGCCAGGTGTGGATCCACAGG ACTTGAAGGTCAAAGTTCACAAAGATGAAGAATCAGGGTAGCTGACC ATGTTTGGCAGATACTATAATGG AGACACAGAAGTGTGCATGGCCCAAGGACAAGGACCTCCAGCCAGGC TTCATTTATGCACTTGTGCTGCA AAAGAAAAGTCTAGGTTTTAAGGCTGTGCCAGAACCCATCCCAATAA AGAGACCGAGTCTGAAGTCACAT TGTAAATCTAGTGTAGGAGACTTGGAGTCAGGCAGTGAGACTGGTGG GGCACGGGGGGCAGTGGGTACTT GTAAACCTTTAAAGATGGTTAATTCATTCAATAGATATTTATTAAGA ACCTATGCGGCCCGGCATGGTGG CTCACACCTGTAATCCCAGCACTTTGGGAGGCCAAGGTGGGTGGGTC ATCTGAGGTCAGGAGTTCAAGAC CAGCCTGGCCAACATGGTGAAACCCCATCTCTACTAAAGATACAAAA ATTTGCTGAGCGTGGTGGTGTGC ACCTGTAATCCCAGCTACTCGAGAGGCCAAGGCATGAGAATCGCTTG AACCTGGGAGGTGGAGGTTGCAG TGAGCTGAGATGGCACCACTGCACTCCGGCCTAGGCAACGAGAGCAA AACTCCAATACAAACAAACAAAC AAACACCTGTGCTAGGTCAGTCTGGCACGTAAGATGAACATCCCTAC CAACACAGAGCTCACCATCTCTT ATACTTAAGTGAAAAACATGGGGAAGGGGAAAGGGGAATGGCTGCTT TTGATATGTTCCCTGACACATAT CTTGAATGGAGACCTCCCTACCAAGTGATGAAAGTGTTGAAAAACTT AATAACAAATGCTTGTTGGGCAA GAATGGGATTGAGGATTATCTTCTCTCAGAAAGGCATTGTGAAGGAA TTGAGCCAGATCTCTCTCCCTAC TGCAAAACCCTATTGTAGTAAAAAAGTCTTCTTTACTATCTTAATAA AACAGATATTGTGAGATTCAAAA AAAAAAAAAAAA | |
| 229152 _at | chromosome 4 open reading frame 7 | tgaagaaagttctcctcctgatca cagccatcttggcagtggctgttg gtttcccagtct ctcaagaccaggaacgagaaaaaa gaagtatcagtgacagcgatgaat tagcttcagggtttttttgtgttcc cttacccatatccatttcgcccac ttccaccaattccatttccaagat ttccatggtttagacgtaattttc ctattccaatacctgaatctgccc ctacaactcccc ttcctagcgaaaagtaaacaagaa ggaaaagtcacgataaacctggtc acctgaaattgaaattgagccact tccttgaagaatcaaaattcctgt taataaaagaaaaacaaatgtaat tgaaatagcacacagcattctcta gtcaatatctttagtgatcttctt taata [Seq Id No 79] | >gi\|50428928\|ref\|NM_152997.2\| Homo sapiens chromosome 4 open reading frame 7 (C4orf7), mRNA | 154.0 5 |
| | | | TAAAACAGCTACAATATTCCAGGGCCAGTCACTTGCCATTTCTCATA ACAGCGTCAGAGAGAAAGAACTG ACTGAAACGTTTGAGATGAAGAAAGTTCTCCTCCTGATCACAGCCAT CTTGGCAGTGGCTGTTGGTTTCC CAGTCTCTCAAGACCAGGAACGAGAAAAAAGAAGTATCAGTGACAGC GATGAATTAGCTTCAGGGTTTTT TGTGTTCCCTTACCCATATCCATTTCGCCCACTTCCACCAATTCCAT TTCCAAGATTTCCATGGTTTAGA CGTAATTTTCCTATTCCAATACCTGAATCTGCCCCTACAACTCCCCT TCCTAGCGAAAAGTAAACAAGAA GGAAAAGTCACGATAAACCTGGTCACCTGAAATTGAAATTGAGCCAC TTCCTTGAAGAATCAAAATTCCT GTTAATAAAAGAAAAACAAATGTAATTGAAATAGCACACAGCATTCT CTAGTCAATATCTTTAGTGATCT TCTTTAATAAACTTGAAAGCAAAGATTTTGGTTTCTTAATTTCCACA AA | |
| 236203 _at | Major histocompatibility complex, class II, DQ alpha 1 | atgtcaggtttgtacctaccacat ttaaaatagggacttgaagaatta aacattttatta caaatgaagcacttcatgcacaga ctggcacatagtaagtagtcgata ggtgttaacaat ttgtgttattgttattttctggag tccaactaacaaatcccacagtga atgacatcacag ggatgcaaccaacaagatccagaa tatggaaacttctactagataaac aactccatttct tcagcaacaattcaagagagagag agaagagaagctatacattttaaa | >gi\|52426772\|ref\|NM_002122.3\| Homo sapiens major histocompatibility complex, class II, DQ alpha 1 (HLA-DQA1), mRNA | 38.77 |
| | | | ACAATTACTCTACAGCTCAGAACACCAACTGCTGAGGCTGCCTTGGG AAGAGGATGATCCTAAACAAAGC TCTGCTGCTGGGGGCCCTCGCTCTGACCACCGTGATGAGCCCCTGTG GAGGTGAAGACATTGTGGCTGAC CACGTTGCCTCTTGTGGTGTAAACTTGTACCAGTTTTACGGTCCCTC TGGCCAGTACACCCATGAATTTG ATGGAGATGAGCAGTTCTACGTGGACCTGGAGAGGAAGGAGACTGCC TGGCGGTGGCCTGAGTTCAGCAA ATTTGGAGGTTTTGACCCGCAGGGTGCACTGAGAAACATGGCTGTGG CAAAACACAACTTGAACATCATG ATTAAACGCTACAACTCTACCGCTGCTACCAATGAGGTTCCTGAGGT | |
| | | aggctgaagaaatatatgaaccaa atttgtatgaggcaatcagaaaaa ctgacaccgactgtattaaggaat tatctaattttagtgtggtaatga gattgctgttatgttttctaa [Seq Id No 80] | CACAGTGTTTTCCAAGTCTCCCG TGACACTGGGTCAGCCCAACACCCTCATTTGTCTTGTGGACAACATC TTTCCTCCTGTGGTCAACATCAC ATGGCTGAGCAATGGGCAGTCAGTCACAGAAGGTGTTTCTGAGACCA GCTTCCTCTCCAAGAGTGATCAT TCCTTCTTCAAGATCAGTTACCTCACCTTCCTCCCTTCTGCTGATGA GATTTATGACTGCAAGGTGGAGC ACTGGGGCCTGGACCAGCCTCTTCTGAAACACTGGGAGCCTGAGATT CCAGCCCCTATGTCAGAGCTCAC AGAGACTGTGGTCTGTGCCCTGGGGTTGTCTGTGGGCCTCATGGGCA TTGTGGTGGGCACTGTCTTCATC ATCCAAGGCCTGCGTTCAGTTGGTGCTTCCAGACACCAAGGGCCATT GTGAATCCCATCCTGGAAGGGAA GGTGCATCGCCATCTACAGGAGCAGAAGAATGGACTTGCTAAATGAC CTAGCACTATTCTCTGGCCCGAT TTATCATATCCCTTTTCTCCTCCAAATATTTCTCCTCTCACCTTTTC TCTGGGACTTAAGCTGCTATATC CCCTCAGAGCTCACAAATGCCTTTACATTCTTTCCCTGACCTCCTGA TTTTTTTTTCTTTTCTCAAATG TTACCTACAAAGACATGCCTGGGGTAAGCCACCCGGCTACCTAATTC CTCAGTAACCTCCATCTAAAATC TCCAAGGAAGCAATAAATTCCTTTTATGAGATCTATGTCAAATTTTT CCATCTTTCATCCAGGGCTGACT GAAACTATGGCTAATAATTGGGGTACTCTTATGTTTCAATCCAATTT AACCTCATTTCCCAGATCATTTT TCATGTCCAGTAACACAGAAGCCACCAAGTACAGTATAGCCTGATAA TATGTTGATTTCTTAGCTGACAT TAATATTTCTTGCTTCCTTGTGTTCCCACCCTTGGCACTGCCACCCA CCCCTCAATTCAGGCAACAATGA AATTAATGGATACCGTCTGCCCTTGGCCCAGAATTGTTATAGCAAAA ATTTTAGAACCAAAAAATAAGTC TGTACTAATTTCAATGTGGCTTTTAAAAGTATGACAGAGAAATAAGT TAGGATAAAGGAAATTTGAATCT CA | |
| 236280 _at | Transcribed locus | aaaagatctctcactgggaaaaga aaaagttatgcatttataaagtaa ttaaactggttt tccttgtactttattaatctgaat ctaatggcacttccttacgagggt tttcagatgtgc ttgtagttaatggcaacattatca gaatgactacacagacagtcctac tctgaggagatg actttggaagaaacccatttggaa ctacacaccctgctatgtctgtgg agaaatggaact gcaatcctcaagagtcacacttca tattccttcctttcaagtggttga taaaaggtagtg cttcaagcacaggatttatggaat agttggcaaattaaacaacatgct tttatttttgac tacca [Seq Id No 81] | >gi\|3807951\|gb\|AI225238.1\|AI225238 qx12c04.x1 NCI_CGAP_Lym12 Homo sapiens cDNA clone IMAGE:2001126 3', mRNA sequence | 77.63 |
| | | | AGCGGCCGCCCTTTTTTTTTTTTTTTGAATGTTTTATATTTTTATG TTTTTCTCTCTCACCACATTAGA GATTCACATGTCAAAAAAAAAGTTCAAAGATTCCACTTAAATGGTAG TCAAAATTAAAAGCATGTTGTTT AATTTGCCAACTATTCCATAAATCCTGTGCTTGAAGCACTACCTTTT ATCAACCACTTGAAAGGAAGGAA TATGAAGTGTGACTCTTGAGGATTGCAGTTCCATTTCTCCACAGACA TAGCAGGGTGTGTAGTTCCAAAT GGGTTTCTTCCAAAGTCATCTCCTCAGAGTAGGACTGTCTGAGTAGT CATTCTGATAATGTTGCCATTAA CTACAAGCACATCTGAGAACCCTCGTAAGGAAGTGCCATTAGATTCA GA | |
| 239237 _at | Amphiphysi n (Stiff-Man syndrome with breast cancer 128kDa autoantigen) | ttatcaccattaatccatgccagt tatgtacagttttgcatgtttgtt ttttattttact cttttctctccttcattccctatt cctgttcccccataggtgcccagt ctaatgtatttg atatctgtccttagagcctccgta tctgtgaagactagagtcatgcac ttcatcacattt cagtaacgatgggccgcatgtacc acagtcccatgagatgatagaggt gcagaaaaattcctgtcatctagt | >gi\|51466133\|ref\|XM_499518.1\| PREDICTED: Homo sapiens LOC442534 (LOC442534), mRNA | 25.87 |
| | | | GGCCCAGCCCTAGGAGGGGCAGTCCCACAGCAGGCTCACACCCTGC CCTTCAGAGTTGCAGCAGCTAAT GCTGATGCCAGGACCTTGTCCTGCCAGGAAGCCTCTGATTAGGGCTG TGAACCAACTTCCTTTTCACATC TGCAAGGACTCTGCTCTAAGCTCAAATTTTGCAGTTTCTGCTGTCAT CACAGCTGGGAATGAGAAAGGGA AGGGGCTCCAGTTGGTCTCCCTCTGCCTCTGGAGCACACGTGGAGGA CATGAGGCTTCCGCAGGAACAGC TCTGAAAGCTGAGTTCTCGGGTCCCTGTGAAGGAGGGAAGCTTGCCT GCCTTGGAGCGTGAAAAAGTCTT | |
| | | gacatcgtagccatcataacatnn caacacgactctcatttgtggtga ccctggtgtacacaaacctac [Seq Id No 82] | GCTGGACAGAAGTCTCACGGTTTAAAACTGGAAGGAGAGGATTGA GATCACACTAGAATGCACAGAAA ATTCTT | |
| 244413 _at | dendritic cell-associated lectin-1 | atggctggagatgtagtctacgct gacatcaaaactgttcggacttcc ccgttagaactc gcgtttccacttcagagatctgtt tctttcaacttttctactgtccat aaatcatgtcct gccaaagactggaaggtgcataag ggaaaatgttactggattgctgaa actaagaaatcttggaacaaaagt caaaatgactgtgccataaacaat tcatatctcatggtgattcaagac attactgctatggtgagatttaac atttagaggtgacagcatcccccca cactggcagtga attttttgtgctacaaacttggca aaagtctgtgaaaagaagtttcaa cttcatgtgtta ttaact [Seq Id No 83] | >gi\|40548404\|ref\|NM_172004.2\| Homo sapiens dendritic cell-associated lectin-1 (DCAL1), mRNA | 12.75 |
| | | | ATGGTTAGTAATTTCTTCCATGTCATACAAGTATTCGAGAAATCTGC TACCTTGATTAGTAAGACTGAAC ACATTGGTTTTGTCATTTATTCATGGAGGAAGTCCACCACCCACTTG GGGAGCAGAAGGAAATTTGCCAT CTCAATTTACTTATCAGAAGTTTCTTTGCAGAAATATGATTGTCCCT TCAGTGGGACATCATTTGTGGTC TTCTCTCTCTTTTTGATCTGTGCAATGGCTGGAGATGTAGTCTACGC TGACATCAAAACTGTTCGGACTT CCCCGTTAGAACTCGCGTTTCCACTTCAGAGATCTGTTTCTTTCAAC TTTTCTACTGTCCATAAATCATG TCCTGCCAAAGACTGGAAGGTGCATAAGGAAAATGTTACTGGATTG CTGAAACTAAGAAATCTTGGAAC AAAAGTCAAAATGACTGTGCCATAAACAATTCATATCTCATGGTGAT TCAAGACATTACTGCTATGGTGA GATTTAACATTTAGAGGTGACAGCATCCCCCACACTGGCAGTGAATT TTTTGTGCTACAAACTTGGCAAA AGTCTGTGAAAAGAAGTTTCAACTTCATGTGTTATTAACTATACAAA TATTAGTTGAATGAATTGTTGAA TTACAAAAAAAAAAAAAAA | |
| 34210_ at | CD52 antigen (CAMPATH-1 antigen) | Taatcggctcactataggaatttg cntcgaggccaagattcgnacgag nnngttcaaaagcagctaaaccaa aagaagcctccagacagccctgag atcacctaaaaagctgctaccaag acagccacgaagatcctaccaaaa | >gi\|68342029\|ref\|NM_001803.2\| Homo sapiens CD52 molecule (CD52), mRNA | 31.41 |
| | | | CTCCTGGTTCAAAAGCAGCTAAACCAAAAGAAGCCTCCAGACAGCCC TGAGATCACCTAAAAAGCTGCTA CCAAGACAGCCACGAAGATCCTACCAAAATGAAGCGCTTCCTCTTCC TCCTACTCACCATCAGCCTCCTG | |
| | | tgaagcgcttcctcttcctcctac tcaccatcagcctcctggttatgg tacagatacaaactggactctcag gacaaaacgacaccagccaaacca gcagcccctcagcatccagcagca tgagcggaggcattttcctttttct tcgtggccaatgccataatccacc tcttctgcttcagttgaggtgaca cgtctcagccttagccctgtgccc cctgaaacannnnnnnnnnnnnnn nnnnagagaatcccctccatcttt gggaggggttgatgccagacatca ccaggttgtagaagttgacaggca gtgccatggggncaacagccaaaa tagggggtaatgatgtaggggcc aagcagtgcccagctgggggtcaa taaagttacccttgtacttg [Seq Id No 84] | GTTATGGTACAGATACAAACTGGACTCTCAGGACAAAACGACACCAG CCAAACCAGCAGCCCCTCAGCAT CCAGCAACATAAGCGGAGGCATTTTCCTTTTCTTCGTGGCCAATGCC ATAATCCACCTCTTCTGCTTCAG TTGAGGTGACACGTCTCAGCCTTAGCCCTGTGCCCCCTGAAACAGCT GCCACCATCACTCGCAAGAGAAT CCCCTCCATCTTTGGGAGGGGTTGATGCCAGACATCACCAGGTTGTA GAAGTTGACAGGCAGTGCCATGG GGGCAACAGCCAAAATAGGGGGGTAATGATGTAGGGGCCAAGCAGTG CCCAGCTGGGGGTCAATAAAGTT ACCCTTGTACTTGCAAAAAAAAAAAAAAAAAAAA | |

**TABLE 3A List of 13 probe sets suitable for identify the genes listed in Table 3**

| **Probe Set ID** | **Gene Symbol** | **Gene Title** | **Gene Sequences** |
|---|---|---|---|
| 204661 _at | CD52 | CD52 molecule /// CD52 molecule | Acagccacgaagatcctaccaaaatgaagcgcttcctcttcctcctactcaccatcagcctcctggttatggtac agatacaaactggactctcaggacaaaacgacaccagccaaaccagcagcccctcagcatccagcagcatgagcg gaggcattttcctttcttcgtggccaatgccataatccacctcttctgcttcagttgaggtgacacgtctcagc cttagccctgtgccccctgaaacagctgccaccatcactcgcaagagaatcccctccatctttgggagggttga tgccagacatcaccaggttgtagaagttgacaggcagtgccatgggggcaacagccaaaatagggggtaatgat gtaggggccaagc [Seq Id No 85] |
| 205890 _s_at | UBD | gamma-aminobutyric acid (GABA) B receptor, 1 /// ubiquitin D | Gatcttaaagccacggagaagcctctcatcttatggcattgacaaagagaagaccatccaccttaccctgaaagt ggtgaagcccagtgatgaggagctgcccttgtttcttgtggagtcaggtgatgaggcaaagaggcacctcctcca ggtgcgaaggtccagctcagtggcacaagtgaaagcaatgatcgagactaagacgggtataatccctgagaccca gattgtgacttgcaatggaaagagactggaagatgggaagatgatggcagattacggcatcagaaagggcaactt actcttcctggcatcttattgtattggaggtgaccaccctggggatggggtgttggcaggggtcaaaaagctta tttcttttaatctcttactcaacgaacacatcttctgatgatttcccaaaattaatgagaatgagatgagtagag taagatttgggtgggatgggtaggatgaagtatattgcccaactctatgtttctttga [Seq Id No 86] |
| 206118 _at | STAT4 | signal transducer and activator of transcription 4 | Gctgacatcctgcgagactacaaagttattatggctgaaaacattcctgaaaccctctgaagtacctatatcct gacattcccaaagacaaagccttcggtaaacactacagctctcagccttgcgaagtttcaagaccaacagaaagg ggtgacaaaggttatgttccttctgtttttatccccatctcaacaatccgaagtgattcaacagagccacattct ccatcagaccttcttcccatgtctccaagtgtgtatgcggtgttgagagaaaacctgagtcccacaacaattgaa actgcaatgaagtctccttattctgctgaatgacaggataaactctgacgcaccaagaaaggaagcaaatgaaaa agtttaaagactgttctttgcccaataaccacattttatttcttcagctttgtaaataccaggttctaggaaatg tttgacatctgaagctctcttcacactcccgtggcactcctcaattgggag [Seq Id No 87] |
| 206666 _at | GZMK | granzyme K (granzyme 3; tryptase II) /// granzyme K (granzyme 3; tryptase II) | aaacctctcttagatctggaaccaaatgcaaggttactggctggggagccaccgatccagattcattaagacctt ctgacaccctgcgagaagtcactgttactgtcctaagtcgaaaactttgcaacagccaaagttactacaacggcg acccttttatcaccaaagacatggtctgtgcaggagatgccaaaggccagaaggattcctgtaagggtgactcag gggggccccttgatctgtaaaggtgtcttccacgctatagtctctggaggtcatgaatgtggtgttgccacaaagc ctggaatctacaccctgttaaccaagaaataccagacttggatcaaaagcaaccttgtcccgcctcatacaaatt aagttacaaataattttattggatgcacttgcttctttttcctaatatgctcgcaggttagagttgggtgtaag taaagcagagcacatatggggtccattttgcacttgta [Seq Id No 88] |
| 207651 _at | GPR171 | G protein-coupled receptor 171 | Ttgccttgtaattcgacagctctacagaaacaaagataatgaaaattacccaaatgtgaaaaaggctctcatcaa catactttagtgaccacggctacatcatatgctttgttccttaccacattgtccgaatcccgtataccctcag ccagacagaagtcataactgattgctcaaccaggatttcactcttcaaagccaaagaggctacactgctcctggc tgtgtcgaacctgtgctttgatcctatcctgtactatcacctctcaaaagcattccgctcaaaggtcactgagac ttttgcctcacctaaagagaccaaggctcagaaagaaaaattaagatgtgaaaataatgcataaaagacaggatt ttttgtgctaccaattctggccttactgga [Seq Id No 89] |
| 210038 _at | PRKCQ | protein kinase C, theta | Aatccattcatcctgattgggcatgaaatccatggtcaagaggacaagtggaaagtgagagggaaggtttgctag acaccttcgcttgttatcttgtcaagatagaaaagatagtatcatttcacccttgccagtaaaacctttccatc cacccattctcagcagactccagtattggcacagtcactcactgccattctcacactataacaagaaaagaaatg aagtgcataagtctcctgggaaaagaaccttaaccccttctcgtgccatgactggtgatttcatgactcataagc ccctccgtaggcatcattcaagatcaatggcccatgcatgctgtttgcagca [Seq Id No 90] |
| 210972 _x_at | TRA@ /// TRDV2 /// TRAV20 /// TRAC | T cell receptor alpha locus /// T cell receptor delta variable 2 /// T cell receptor alpha variable 20 /// T cell receptor alpha constant | Ggaacaagacttcaggtcacgctcgatatccagaaccctgaccctgccgtgtaccagctgagagactctaaatcc agtgacaagtctgtctgcctattcaccgattttgattctcaaacaaatgtgtcacaaagtaaggattctgatgtg tatatcacagacaaaactgtgctagacatgaggtctatggacttcaagagcaacagtgctgtggcctggagcaac aaatctgactttgcatgtgcaaacgccttcaacaacagcattattccagaagacaccttcttccccagcccagaa agttcctgtgatgtcaagctggtcgagaaaagctttgaaacagatacgaacctaaactttcaaaacctgtcagtg attgggttccgaatcctcctcctgaaagtggccgggtttaatctgctcatgacgctgcggctgtggtccagctga gatctgcaagattgtaagacagcctgtgctccct [Seq Id No 91] |
| 211144 _x_at | TRGC2 /// TRGV2 /// TRGV9 /// TARP /// LOC642 083 | T cell receptor gamma constant 2 /// T cell receptor gamma variable 2 /// T cell receptor gamma variable 9 /// TCR gamma alternate reading frame protein /// hypothetical protein LOC642083 | Aaatgatacactactgctgcagctcacaaacacctctgcatattacatgtacctcctgctcctcaagagtgt ggtctattttgccatcatcacctgctgtctgcttggaagaacggctttctgctgcaatggagagaaatcataaca gacggtggcacaaggaggccatcttttcctcatcggttattgtccctagaagcgtcttctgaggatctagttggg ctttctttctgggtttgggccatttcagttctcatgtgtgtactattctatcattattgtataatggttttcaaa ccagtgggcacacagagaacctcagtctgtaataacaatgaggaatagccatggcgatctccagcaccaatctct ccatgttttccacagctcctccagccaacccaaatagcgcctgctatagtgtagacagcctgcggcttctagcct tgtccctctcttagtgttctttaatcagataactgcctggaagcctttcattttacacgccc [Seq Id No 92] |
| 211796 _s_at | TRBV21 -1 /// TRBV19 /// TRBV5-4 /// TRBV3-1 /// TRBC1 | T cell receptor beta variable 21-1 /// T cell receptor beta variable 19 /// T cell receptor beta variable 5-4 /// T cell receptor beta variable 3-1 /// T cell receptor beta constant 1 /// similar to T-cell receptor beta chain V region CTL-L17 precursor | gccatcagaagcagagatctcccacacccaaaaggccacactggtgtgcctggccacaggtttctaccccgacca cgtggagctgagctggtgggtgaatggaaggaggtgcacagtggggtcagcacagacccgcagcccctcaagga gcagcccgccctcaatgactccagatactgcctgagcagccgcctgagggtctcggccaccttctggcagaaccc ccgcaaccacttccgctgtcaagtccagttctacgggctctcggagaatgacgagtggacccaggatagggccaa acctgtcacccagatcgtcagcgccgaggcctggggtagagcagactgtggcttcacctccgagtcttaccagca aggggtcctgtctgccaccatcctctatgagatcttgctagggaaggccaccttgtatgctgtgctggtcagtgc cctcgtgctgatggccatggtcaagagaaagga [Seq Id No 93] |
| 213193 _x_at | TRBV19 /// TRBC1 | T cell receptor beta variable 19 /// T cell receptor beta variable 19 /// T cell receptor beta constant 1 /// T cell receptor beta constant 1 | Tgactccagatactgcctgagcagccgcctgaggtctcggccaccttctggcagaacccccgcaaccacttccg ctgtcaagtccagttctacggctctcggagaatgacgagtggacccaggatagggccaaacccgtcacccagat cgtcagcgccgaggcctggggtagagcagactgtggctttacctcggtgtcctaccagcaaggggtcctgtctgc caccatcctctatgagatcctgctagggaaggccaccctgtatgctgtgctggtcagcnccccttgtgttgatggc catggtcaagagaaaggatttctgaaggcagccctggaagtggagttaggagcttctaacccgtcatggtttcaa tacacattcttcttttgccagcgcttctgaagagctgctctcacctctctgcatcccaatagatatcccccctatg tgcatgcacacctgcacactcacggctgaaatctccctaacccaggggggaccttagcatgcctaagtga |
| | | | [Seq Id No 94] |
| 213539 _at | CD3D | CD3d molecule, delta (CD3-TCR complex) | gggaacactgctctcagacattacaagactggacctgggaaaacgcatcctggacccacgaggaatatataggtg taatgggacagatatatacaaggacaaagaatctaccgtgcaagttcattatcgaatgtgccagagctgtgtgga gctggatccagccaccgtggctggcatcattgtcactgatgtcattgccactctgctccttgctttgggagtctt ctgctttgctggacatgagactggaaggctgtctggggctgccgacacacaagctctgttgaggaatgaccaggt ctatcagcccctccgagatcgagatgatgctcagtacagccaccttggaggaaactgggctcggaacaagtgaac ctgagactggtggcttctagaagcagccattaccaactgtacct [Seq Id No 95] |
| 217147 _s_at | TRAT1 | T cell receptor associated transmembrane adaptor 1 | Tctcctttctcaccaatgggcaatagcccataattgaaataaatttctgattgaaaggtataggaaacattaaaa tgcattactaagagaagtaatataattttcttacaaagtatttttcccaaagatagctttactatttcaaaaatt gtcaaattaatgcatgctccttacaacaaacaaatatcaaaaagagtttaggaattctactagccagagatagtc acttggagaaactttctatatatccttctaaatatttttctgggcatgctcatgtatgtacatcagttgtttctt tttattttgaaccaaaaatgtggtttcttttgtacacattacttaaactttctttccagtcaacaatatattgtg gatttattttcactgttatatttaactatatataaatacgcatatattgtaattttaatgtctgcttagcacccc actgataaccaaatcacag [Seq Id No 96] |
| 34210_ at | CD52 | CD52 molecule | Taatcggctcactataggaatttgcntcgaggccaagattcgnacgagnnngttcaaaagcagctaaaccaaaag aagcctccagacagccctgagatcacctaaaaagctgctaccaagacagccacgaagatcctaccaaaatgaagc gcttcctcttcctcctactcaccatcagcctcctggttatggtacagatacaaactggactctcaggacaaaacg acaccagccaaaccagcagcccctcagcatccagcagcatgagcggaggcattttccttttcttcgtggccaatg ccataatccacctcttctgcttcagttgaggtgacacgtctcagccttagccctgtgccccctgaaacannnnnn nnnnnnnnnnnnnagagaatcccctccatctttgggagggggttgatgccagacatcaccaggttgtagaagttga caggcagtgccatggggncaacagccaaaatagggggggtaatgatgtaggggccaagcagtgcccagctggggt caataaagttacccttgtacttg [Seq Id No 97] |

**Table 4A: Table linking probe set id and the gene list of Table 4.**

| **Probe Set ID** | **Gene Symbol** | **Gene Name** |
|---|---|---|
| 218802_at | FLJ20647 | NA (not applicable) |
| 224774_s_at | NAV1 | neuron navigator 1 |
| 207651_at | GPR171 | G protein-coupled receptor 171 |
| 205392_s_at | CCL14 | chemokine (C-C motif) ligand 14 |
| 1555229_a_at | C1S | complement component 1, s subcomponent |
| 209774_x_at | CXCL2 | chemokine (C-X-C motif) ligand 2 |
| 211796_s_at | TRBV3-1 | T cell receptor beta variable 3-1 |
| 210972_x_at | TRDV2 | T cell receptor delta variable 2 |
| 210251 _s_at | RUFY3 | RUN and FYVE domain containing 3 |
| 225502_at | DOCK8 | dedicator of cytokinesis 8 |
| 204224_s_at | GCH1 | GTP cyclohydrolase 1 (dopa-responsive dystonia) |
| 218950_at | CENTD3 | centaurin, delta 3 |
| 218322_s_at | ACSL5 | acyl-CoA synthetase long-chain family member 5 |
| 228094_at | AMICA1 | adhesion molecule, interacts with CXADR antigen 1 |
| 204116_at | IL2RG | interleukin 2 receptor, gamma (severe combined immunodeficiency) |
| 202643_s_at | TNFAIP3 | tumor necrosis factor, alpha-induced protein 3 |
| 209606_at | PSCDBP | pleckstrin homology, Sec7 and coiled-coil domains, binding protein |
| 205225_at | ESR1 | estrogen receptor 1 |
| 213193_x_at | TRBC1 | T cell receptor beta constant 1 |
| 204661_at | CD52 | CD52 antigen (CAMPATH-1 antigen) |
| 216920_s_at | LOC442535 | NA |
| 210915_x_at | TRBV19 | T cell receptor beta variable 19 |
| 226218_at | IL7R | interleukin 7 receptor |
| 209670_at | TRAC | T cell receptor alpha constant |
| 209949_at | NCF2 | neutrophil cytosolic factor 2 (65kDa, chronic granulomatous disease, autosomal 2) |
| 226697_at | LOC92689 | NA |
| 206666_at | GZMK | c("granzyme K (granzyme 3", " tryptase II)") |
| 235831_at | NA | NA |
| 1555630_a_at | RAB34 | RAB34, member RAS oncogene family |
| 207977_s_at | DPT | dermatopontin |
| 1558290_a_at | PVT1 | Pvt1 oncogene homolog, MYC activator (mouse) |
| 215806_x_at | TRGC2 | T cell receptor gamma constant 2 |
| 64064_at | GIMAP5 | GTPase, IMAP family member 5 |
| 34210_at | CD52 | CD52 antigen (CAMPATH-1 antigen) |
| 213539_at | CD3D | CD3d antigen, delta polypeptide (TiT3 complex) |
| 232313_at | TMEM132C | transmembrane protein 132C |
| 201502_s_at | NFKBIA | nuclear factor of kappa light polypeptide gene enhancer in B-cells inhibitor, alpha |
| 211902_x_at | TRA@ | T cell receptor alpha locus |
| 217147_s_at | TRAT1 | T cell receptor associated transmembrane adaptor 1 |
| 211144_x_at | LOC442535 | NA |
| 224710_at | RAB34 | RAB34, member RAS oncogene family |
| 209795_at | CD69 | CD69 antigen (p60, early T-cell activation antigen) |
| 232843_s_at | DOCK8 | dedicator of cytokinesis 8 |
| 218805_at | GIMAP5 | GTPase, IMAP family member 5 |
| 204057_at | IRF8 | interferon regulatory factor 8 |
| 214470_at | KLRB1 | killer cell lectin-like receptor subfamily B, member 1 |
| 236280_at | NA | NA |
| 201474_s_at | ITGA3 | integrin, alpha 3 (antigen CD49C, alpha 3 subunit of VLA-3 receptor) |
| 235421_at | MAP3K8 | mitogen-activated protein kinase kinase kinase 8 |
| 228532_at | C1orf162 | chromosome 1 open reading frame 162 |
| 205890_s_at | UBD | ubiquitin D |
| 209813_x_at | TRGV9 | T cell receptor gamma variable 9 |
| 224772_at | NAV1 | neuron navigator 1 |
| 224451_x_at | ARHGAP9 | Rho GTPase activating protein 9 |
| 226117_at | TIFA | NA |
| 213068_at | DPT | dermatopontin |
| 1569942_at | NA | NA |
| 219243_at | GIMAP4 | GTPase, IMAP family member 4 |
| 202957_at | HCLS1 | hematopoietic cell-specific Lyn substrate 1 |
| 218764_at | PRKCH | protein kinase C, eta |
| 206118_at | STAT4 | signal transducer and activator of transcription 4 |
| 236203_at | HLA-DQA1 | major histocompatibility complex, class II, DQ alpha 1 |
| 206170_at | ADRB2 | adrenergic, beta-2-, receptor, surface |
| 239237_at | NA | NA |
| 214450_at | CTSW | cathepsin W (lymphopain) |
| 201497_x_at | MYH11 | myosin, heavy polypeptide 11, smooth muscle |
| 219777_at | GIMAP6 | GTPase, IMAP family member 6 |
| 211654_x_at | HLA-DQB1 | major histocompatibility complex, class II, DQ beta 1 |
| 205758_at | CD8A | CD8 antigen, alpha polypeptide (p32) |
| 202644_s_at | TNFAIP3 | tumor necrosis factor, alpha-induced protein 3 |
| 1558034_s_at | CP | ceruloplasmin (ferroxidase) |
| 223235_s_at | SMOC2 | SPARC related modular calcium binding 2 |
| 232234_at | C20orf24 | chromosome 20 open reading frame 24 |
| 1559584_a_at | C16orf54 | chromosome 16 open reading frame 54 |
| 205831_at | CD2 | CD2 antigen (p50), sheep red blood cell receptor |
| 203812_at | SLIT3 | slit homolog 3 (Drosophila) |
| 222780_s_at | BAALC | brain and acute leukemia, cytoplasmic |
| 218145_at | TRIB3 | tribbles homolog 3 (Drosophila) |
| 242881_x_at | LOC440160 | NA |
| 1558972_s_at | C6orf190 | chromosome 6 open reading frame 190 |
| 229723_at | TAGAP | T-cell activation GTPase activating protein |
| 235391_at | FAM92A1 | family with sequence similarity 92, member A1 |
| 219938_s_at | PSTPIP2 | proline-serine-threonine phosphatase interacting protein 2 |
| 212587_s_at | PTPRC | protein tyrosine phosphatase, receptor type, C |
| 210982_s_at | HLA-DRA | major histocompatibility complex, class II, DR alpha |
| 211200_s_at | EFCAB2 | EF-hand calcium binding domain 2 |
| 210260_s_at | TNFAIP8 | tumor necrosis factor, alpha-induced protein 8 |
| 228869_at | SLIC1 | NA |
| 205987_at | CD1C | CD1c antigen |
| 213888_s_at | TRAF3IP3 | TRAF3 interacting protein 3 |
| 212671_s_at | HLA-DQA1 | major histocompatibility complex, class II, DQ alpha 1 |
| 242986_at | NAV1 | neuron navigator 1 |
| 212999_x_at | HLA-DQB1 | major histocompatibility complex, class II, DQ beta 1 |
| 212588_at | PTPRC | protein tyrosine phosphatase, receptor type, C |
| 212592_at | IGJ | immunoglobulin J polypeptide, linker protein for immunoglobulin alpha and mu polypeptides |
| 203471_s_at | PLEK | pleckstrin |
| 209671_x_at | TRA@ | T cell receptor alpha locus |
| 228054_at | TMEM44 | transmembrane protein 44 |
| 216191_s_at | TRA@ | T cell receptor alpha locus |
| 205419_at | EBI2 | Epstein-Barr virus induced gene 2 (lymphocyte-specific G protein-coupled receptor) |
| 220330_s_at | SAMSN1 | SAM domain, SH3 domain and nuclear localisation signals, 1 |
| 213008_at | KIAA1794 | KIAA1794 |
| 201425_at | ALDH2 | aldehyde dehydrogenase 2 family (mitochondrial) |
| 1552613_s_at | CDC42SE2 | CDC42 small effector 2 |
| 205696_s_at | GFRA1 | GDNF family receptor alpha 1 |
| 211339_s_at | ITK | IL2-inducible T-cell kinase |
| 208894_at | HLA-DRA | major histocompatibility complex, class II, DR alpha |
| 228071_at | GIMAP7 | GTPase, IMAP family member 7 |
| 222496_s_at | FLJ20273 | NA |
| 206687_s_at | PTPN6 | protein tyrosine phosphatase, non-receptor type 6 |
| 210375_at | PTGER3 | prostaglandin E receptor 3 (subtype EP3) |
| 219440_at | RAI2 | retinoic acid induced 2 |
| 208450_at | LGALS2 | lectin, galactoside-binding, soluble, 2 (galectin 2) |
| 203665_at | HMOX1 | heme oxygenase (decycling) 1 |
| 227995_at | NA | NA |
| 227346_at | ZNFN1A1 | zinc finger protein, subfamily 1A, 1 (Ikaros) |
| 205159_at | CSF2RB | colony stimulating factor 2 receptor, beta, low-affinity (granulocyte-macrophage) |
| 205559_s_at | PCSK5 | proprotein convertase subtilisin/kexin type 5 |
| 212886_at | CCDC69 | coiled-coil domain containing 69 |
| 1552612_at | CDC42SE2 | CDC42 small effector 2 |
| 205488_at | GZMA | granzyme A (granzyme 1, cytotoxic T-lymphocyte-associated serine esterase 3) |
| 217767_at | C3 | complement component 3 |
| 208296_x_at | TNFAIP8 | tumor necrosis factor, alpha-induced protein 8 |
| 223484_at | C15orf48 | chromosome 15 open reading frame 48 |
| 204070_at | RARRES3 | retinoic acid receptor responder (tazarotene induced) 3 |
| 214719_at | LOC283537 | NA |
| 209687_at | CXCL12 | chemokine (C-X-C motif) ligand 12 (stromal cell-derived factor 1) |
| 224773_at | NAV1 | neuron navigator 1 |
| 231882_at | NA | NA |
| 215223_s_at | SOD2 | superoxide dismutase 2, mitochondrial |
| 232617_at | CTSS | cathepsin S |
| 210554_s_at | CTBP2 | C-terminal binding protein 2 |
| 219528_s_at | BCL11B | B-cell CLL/lymphoma 11B (zinc finger protein) |
| 207861_at | CCL22 | chemokine (C-C motif) ligand 22 |
| 222592_s_at | ACSL5 | acyl-CoA synthetase long-chain family member 5 |
| 1554966_a_at | DOC1 | NA |
| 204204_at | SLC31A2 | solute carrier family 31 (copper transporters), member 2 |
| 219926_at | POPDC3 | popeye domain containing 3 |
| 204135_at | DOC1 | NA |
| 217995_at | SQRDL | sulfide quinone reductase-like (yeast) |
| 230233_at | RASGEF1B | RasGEF domain family, member 1B |
| 227265_at | FGL2 | fibrinogen-like 2 |
| 228372_at | C10orf128 | chromosome 10 open reading frame 128 |
| 204912_at | IL10RA | interleukin 10 receptor, alpha |
| 219454_at | EGFL6 | EGF-like-domain, multiple 6 |
| 206295_at | IL18 | interleukin 18 (interferon-gamma-inducing factor) |
| 226219_at | ARHGAP30 | Rho GTPase activating protein 30 |
| 218736_s_at | PALMD | palmdelphin |
| 223322_at | RASSF5 | Ras association (RalGDS/AF-6) domain family 5 |
| 209603_at | GATA3 | GATA binding protein 3 |
| 204687_at | DKFZP5640 0823 | NA |
| 222895_s_at | BCL11B | B-cell CLL/lymphoma 11B (zinc finger protein) |
| 201010_s_at | TXNIP | thioredoxin interacting protein |
| 226818_at | DTX4 | deltex 4 homolog (Drosophila) |
| 208335_s_at | DARC | Duffy blood group, chemokine receptor |
| 213566_at | RNASE6 | ribonuclease, RNase A family, k6 |
| 205685_at | CD86 | CD86 antigen (CD28 antigen ligand 2, B7-2 antigen) |
| 201531_at | ZFP36 | zinc finger protein 36, C3H type, homolog (mouse) |
| 202391_at | BASP1 | brain abundant, membrane attached signal protein 1 |
| 201804_x_at | CKAP1 | cytoskeleton associated protein 1 |
| 206082_at | HCP5 | HLA complex P5 |
| 206204_at | GRB14 | growth factor receptor-bound protein 14 |
| 228563_at | GJA7 | gap junction protein, alpha 7, 45kDa (connexin 45) |
| 219054_at | FLJ14054 | NA |
| 205844_at | VNN1 | vanin 1 |
| 203741_s_at | ADCY7 | adenylate cyclase 7 |
| 223280_x_at | MS4A6A | membrane-spanning 4-domains, subfamily A, member 6A |
| 205624_at | CPA3 | carboxypeptidase A3 (mast cell) |
| 209193_at | PIM1 | pim-1 oncogene |
| 210072_at | CCL19 | chemokine (C-C motif) ligand 19 |
| 226068_at | SYK | spleen tyrosine kinase |
| 216155_at | NAV1 | neuron navigator 1 |
| 205484_at | SIT1 | signaling threshold regulating transmembrane adaptor 1 |
| 228812_at | NA | NA |
| 219368_at | NAP1L2 | nucleosome assembly protein 1-like 2 |
| 206407_s_at | CCL13 | chemokine (C-C motif) ligand 13 |
| 203761_at | SLA | Src-like-adaptor |
| 236295_s_at | NOD3 | NA |
| 206099_at | PRKCH | protein kinase C, eta |
| 217143_s_at | TRD@ | T cell receptor delta locus |
| 218899_s_at | BAALC | brain and acute leukemia, cytoplasmic |
| 229391_s_at | RP1-93H18.5 | NA |
| 219093_at | FLJ20701 | NA |
| 219710_at | SH3TC2 | SH3 domain and tetratricopeptide repeats 2 |
| 206978_at | CCR2 | chemokine (C-C motif) receptor 2 |
| 204655_at | CCL5 | chemokine (C-C motif) ligand 5 |
| 211991_s_at | HLA-DPA1 | major histocompatibility complex, class II, DP alpha 1 |
| 223922_x_at | MS4A6A | membrane-spanning 4-domains, subfamily A, member 6A |
| 208983_s_at | PECAM1 | platelet/endothelial cell adhesion molecule (CD31 antigen) |
| 222108_at | AMIGO2 | adhesion molecule with Ig-like domain 2 |
| 1553102_a_at | CCDC69 | coiled-coil domain containing 69 |
| 221698_s_at | CLEC7A | C-type lectin domain family 7, member A |
| 206637_at | P2RY14 | purinergic receptor P2Y, G-protein coupled, 14 |
| 226459_at | PIK3AP1 | phosphoinositide-3-kinase adaptor protein 1 |
| 209613_s_at | ADH1B | alcohol dehydrogenase IB (class I), beta polypeptide |
| 225802_at | TOP1MT | topoisomerase (DNA) I, mitochondrial |
| 224859_at | CD276 | CD276 antigen |
| 209480_at | HLA-DQB1 | major histocompatibility complex, class II, DQ beta 1 |
| 219213_at | JAM2 | junctional adhesion molecule 2 |
| 208747_s_at | C1S | complement component 1, s subcomponent |
| 224356_x_at | MS4A6A | membrane-spanning 4-domains, subfamily A, member 6A |
| 226625_at | TGFBR3 | transforming growth factor, beta receptor III (betaglycan, 300kDa) |
| 1554240_a_at | ITGAL | c("integrin, alpha L (antigen CD11A (p180), lymphocyte function-associated antigen 1", " alpha polypeptide)") |
| 202948_at | IL1R1 | interleukin 1 receptor, type I |
| 219666_at | MS4A6A | membrane-spanning 4-domains, subfamily A, member 6A |
| 215193_x_at | HLA-DRB1 | major histocompatibility complex, class II, DR beta 1 |
| 232024_at | GIMAP2 | GTPase, IMAP family member 2 |
| 1559263_s_at | ZC3H12D | zinc finger CCCH-type containing 12D |
| 219737_s_at | PCDH9 | protocadherin 9 |
| 222838_at | SLAMF7 | SLAM family member 7 |
| 227983_at | MGC7036 | NA |
| 223809_at | RGS 18 | regulator of G-protein signalling 18 |
| 213831_at | HLA-DQA1 | major histocompatibility complex, class II, DQ alpha 1 |
| 203416_at | CD53 | CD53 antigen |
| 226841_at | MPEG1 | NA |
| 228552_s_at | SSBP4 | single stranded DNA binding protein 4 |
| 231262_at | NA | NA |
| 206898_at | CDH19 | cadherin 19, type 2 |
| 210835_s_at | CTBP2 | C-terminal binding protein 2 |
| 227584_at | NAV1 | neuron navigator 1 |
| 223059_s_at | FAM107B | family with sequence similarity 107, member B |
| 221671_x_at | IGKC | immunoglobulin kappa constant |
| 205786_s_at | ITGAM | integrin, alpha M (complement component 3 receptor 3 subunit) |
| 216194_s_at | CKAP1 | cytoskeleton associated protein 1 |
| 209312_x_at | HLA-DRB1 | major histocompatibility complex, class II, DR beta 1 |
| 235639_at | CDH19 | cadherin 19, type 2 |
| 233562_at | MGC16291 | NA |
| 232476_at | DDEF2 | development and differentiation enhancing factor 2 |
| 202510_s_at | TNFAIP2 | tumor necrosis factor, alpha-induced protein 2 |
| 222484_s_at | CXCL14 | chemokine (C-X-C motif) ligand 14 |
| 207277_at | CD209 | CD209 antigen |
| 204724_s_at | COL9A3 | collagen, type IX, alpha 3 |
| 239196_at | ANKRD22 | ankyrin repeat domain 22 |
| 209734_at | NCKAP1L | NCK-associated protein 1-like |
| 212977_at | CMKOR1 | chemokine orphan receptor 1 |
| 204670_x_at | HLA-DRB5 | major histocompatibility complex, class II, DR beta 5 |
| 208885_at | LCP1 | lymphocyte cytosolic protein 1 (L-plastin) |
| 209612_s_at | ADH1B | alcohol dehydrogenase IB (class I), beta polypeptide |
| 233955_x_at | CXXC5 | CXXC finger 5 |
| 228776_at | GJA7 | gap junction protein, alpha 7, 45kDa (connexin 45) |
| 1553906_s_at | FGD2 | FYVE, RhoGEF and PH domain containing 2 |
| 221760_at | MAN1A1 | mannosidase, alpha, class 1A, member 1 |
| 223361_at | C6orf115 | chromosome 6 open reading frame 115 |
| 229390_at | RP1-93H18.5 | NA |
| 203915_at | CXCL9 | chemokine (C-X-C motif) ligand 9 |
| 223058_at | FAM107B | family with sequence similarity 107, member B |
| 219789_at | NPR3 | natriuretic peptide receptor C/guanylate cyclase C (atrionatriuretic peptide receptor C) |
| 205285_s_at | FYB | FYN binding protein (FYB-120/130) |
| 203868_s_at | VCAM1 | vascular cell adhesion molecule 1 |
| 204236_at | FLI1 | Friend leukemia virus integration 1 |
| 224516_s_at | CXXC5 | CXXC finger 5 |
| 202368_s_at | TRAM2 | translocation associated membrane protein 2 |
| 224795_x_at | IGKC | immunoglobulin kappa constant |
| 235238_at | SHC4 | SHC (Src homology 2 domain containing) family, member 4 |
| 227791_at | SLC9A9 | solute carrier family 9 (sodium/hydrogen exchanger), member 9 |
| 207238_s_at | PTPRC | protein tyrosine phosphatase, receptor type, C |
| 204897_at | PTGER4 | prostaglandin E receptor 4 (subtype EP4) |
| 213975_s_at | LILRB1 | leukocyte immunoglobulin-like receptor, subfamily B (with TM and ITIM domains), member 1 |
| 228964_at | PRDM1 | PR domain containing 1, with ZNF domain |
| 228362_s_at | RP1-93H18.5 | NA |
| 244061_at | ARHGAP15 | Rho GTPase activating protein 15 |
| 1553313_s_at | SLC5A3 | solute carrier family 5 (inositol transporters), member 3 |
| 212538_at | DOCK9 | dedicator of cytokinesis 9 |
| 226043_at | GPSM1 | G-protein signalling modulator 1 (AGS3-like, C. elegans) |
| 1405_i_at | CCL5 | chemokine (C-C motif) ligand 5 |
| 204222_s_at | GLIPR1 | GLI pathogenesis-related 1 (glioma) |
| 221087_s_at | APOL3 | apolipoprotein L, 3 |
| 203932_at | HLA-DMB | major histocompatibility complex, class II, DM beta |
| 225895_at | SYNPO2 | synaptopodin 2 |
| 221651_x_at | NA | NA |
| 231929_at | NA | NA |
| 229543_at | RP1-93H18.5 | NA |
| 211367_s_at | CASP1 | caspase 1, apoptosis-related cysteine peptidase (interleukin 1, beta, convertase) |
| 210038_at | PRKCQ | protein kinase C, theta |
| 205403_at | IL1R2 | interleukin 1 receptor, type II |
| 220066_at | CARD 15 | caspase recruitment domain family, member 15 |
| 1555812_a_at | ARHGDIB | Rho GDP dissociation inhibitor (GDI) beta |
| 208306_x_at | HLA-DRB4 | major histocompatibility complex, class II, DR beta 4 |
| 218854_at | SART2 | squamous cell carcinoma antigen recognized by T cells 2 |
| 203523_at | LSP1 | lymphocyte-specific protein 1 |
| 207992_s_at | AMPD3 | adenosine monophosphate deaminase (isoform E) |
| 228660_x_at | SEMA4F | sema domain, immunoglobulin domain (Ig), transmembrane domain (TM) and short cytoplasmic domain, (semaphorin) 4F |
| 218170_at | ISOC1 | isochorismatase domain containing 1 |
| 1555759_a_at | CCL5 | chemokine (C-C motif) ligand 5 |
| 227253_at | HPS3 | Hermansky-Pudlak syndrome 3 |
| 211990_at | HLA-DPA1 | major histocompatibility complex, class II, DP alpha 1 |
| 212998_x_at | HLA-DQB1 | major histocompatibility complex, class II, DQ beta 1 |
| 204778_x_at | HOXB7 | homeobox B7 |
| 204834_at | FGL2 | fibrinogen-like 2 |
| 205039_s_at | ZNFN1A1 | zinc finger protein, subfamily 1A, 1 (Ikaros) |
| 232543_x_at | ARHGAP9 | Rho GTPase activating protein 9 |
| 209710_at | GATA2 | GATA binding protein 2 |
| 200612_s_at | AP2B1 | adaptor-related protein complex 2, beta 1 subunit |
| 201487_at | CTSC | cathepsin C |
| 201939_at | PLK2 | polo-like kinase 2 (Drosophila) |
| 203547_at | CD4 | CD4 antigen (p55) |
| 228376_at | GGTA1 | glycoprotein, alpha-galactosyltransferase 1 |
| 207574_s_at | GADD45B | growth arrest and DNA-damage-inducible, beta |
| 213560_at | GADD45B | growth arrest and DNA-damage-inducible, beta |
| 219525_at | FLJ10847 | NA |
| 204411_at | KIF21B | kinesin family member 21 B |
| 200953_s_at | CCND2 | cyclin D2 |
| 201859_at | PRG1 | proteoglycan 1, secretory granule |
| 223044_at | SLC40A1 | solute carrier family 40 (iron-regulated transporter), member 1 |
| 213537_at | HLA-DPA1 | major histocompatibility complex, class II, DP alpha 1 |
| 216841_s_at | SOD2 | superoxide dismutase 2, mitochondrial |
| 205081_at | CRIP1 | cysteine-rich protein 1 (intestinal) |
| 226382_at | LOC283070 | NA |
| 219519_s_at | SIGLEC1 | sialic acid binding Ig-like lectin 1, sialoadhesin |
| 1558586_at | ZNF11B | zinc finger protein 11B |
| 217028_at | CXCR4 | chemokine (C-X-C motif) receptor 4 |
| 217478_s_at | HLA-DMA | major histocompatibility complex, class II, DM alpha |
| 204438_at | MRC1 | mannose receptor, C type 1 |
| 211366_x_at | CASP1 | caspase 1, apoptosis-related cysteine peptidase (interleukin 1, beta, convertase) |
| 204249_s_at | LMO2 | LIM domain only 2 (rhombotin-like 1) |
| 221081_s_at | DENND2D | DENN/MADD domain containing 2D |
| 32128_at | CCL18 | chemokine (C-C motif) ligand 18 (pulmonary and activation-regulated) |
| 220005_at | P2RY13 | purinergic receptor P2Y, G-protein coupled, 13 |
| 209924_at | CCL18 | chemokine (C-C motif) ligand 18 (pulmonary and activation-regulated) |
| 242458_at | ANGPTL1 | angiopoietin-like 1 |
| 230391_at | NA | NA |
| 213475_s_at | ITGAL | c("integrin, alpha L (antigen CD11A (p180), lymphocyte function-associated antigen 1", " alpha polypeptide)") |
| 207458_at | C8orf51 | chromosome 8 open reading frame 51 |
| 235306_at | GIMAP8 | GTPase, IMAP family member 8 |
| 227780_s_at | NA | NA |
| 205841_at | JAK2 | Janus kinase 2 (a protein tyrosine kinase) |
| 202687_s_at | TNFSF10 | tumor necrosis factor (ligand) superfamily, member 10 |
| 212067_s_at | C1R | complement component 1, r subcomponent |
| 236908_at | ACPL2 | acid phosphatase-like 2 |
| 223827_at | TNFRS19 | tumor necrosis factor receptor superfamily, member 19 |
| 213652_at | PCSK5 | proprotein convertase subtilisin/kexin type 5 |
| 219631_at | LRP12 | low density lipoprotein-related protein 12 |
| 1557116_at | NA | NA |
| 208981_at | PECAM1 | platelet/endothelial cell adhesion molecule (CD31 antigen) |
| 209685_s_at | PRKCB1 | protein kinase C, beta 1 |
| 238488_at | IPO11 | importin 11 |
| 1568736_s_at | DLGAP1 | discs, large (Drosophila) homolog-associated protein 1 |
| 238439_at | PRKAR2B | protein kinase, cAMP-dependent, regulatory, type II, beta |
| 205027_s_at | MAP3K8 | mitogen-activated protein kinase kinase kinase 8 |
| 211742_s_at | EVI2B | ecotropic viral integration site 2B |
| 202269_x_at | GBP1 | guanylate binding protein 1, interferon-inducible, 67kDa |
| 204533_at | CXCL10 | chemokine (C-X-C motif) ligand 10 |
| 229163_at | CAMK2N1 | calcium/calmodulin-dependent protein kinase II inhibitor 1 |
| 1556579_s_at | MED12L | mediator of RNA polymerase II transcription, subunit 12 homolog (yeast)-like |
| 201566_x_at | ID2 | inhibitor of DNA binding 2, dominant negative helix-loop-helix protein |
| 201220_x_at | CTBP2 | C-terminal binding protein 2 |
| 214677_x_at | IGLJ3 | immunoglobulin lambda joining 3 |
| 235175_at | GBP4 | guanylate binding protein 4 |
| 232001_at | LOC439949 | NA |
| 228427_at | FBXO16 | F-box protein 16 |
| 214617_at | PRF1 | perforin 1 (pore forming protein) |
| 202369_s_at | TRAM2 | translocation associated membrane protein 2 |
| 202625_at | LYN | v-yes-1 Yamaguchi sarcoma viral related oncogene homolog |
| 213618_at | CENTD1 | centaurin, delta 1 |
| 209970_x_at | CASP1 | caspase 1, apoptosis-related cysteine peptidase (interleukin 1, beta, convertase) |
| 218035_s_at | FLJ20273 | NA |
| 206715_at | TFEC | transcription factor EC |
| 1563473_at | PPP1R16B | protein phosphatase 1, regulatory (inhibitor) subunit 16B |
| 204118_at | CD48 | CD48 antigen (B-cell membrane protein) |
| 201137_s_at | HLA-DPB1 | major histocompatibility complex, class II, DP beta 1 |
| 230538_at | SHC4 | SHC (Src homology 2 domain containing) family, member 4 |
| 1568822_at | GTPBP5 | GTP binding protein 5 (putative) |
| 229625_at | GBP5 | guanylate binding protein 5 |
| 212233_at | MAP1B | microtubule-associated protein 1B |
| 209202_s_at | EXTL3 | exostoses (multiple)-like 3 |
| 209083_at | CORO1A | coronin, actin binding protein, 1A |
| 205226_at | PDGFRL | platelet-derived growth factor receptor-like |
| 227640_s_at | RP9 | retinitis pigmentosa 9 (autosomal dominant) |
| 223168_at | RHOU | ras homolog gene family, member U |
| 1553132_a_at | MTAC2D1 | membrane targeting (tandem) C2 domain containing 1 |
| 214038_at | CCL8 | chemokine (C-C motif) ligand 8 |
| 219505_at | CECR1 | cat eye syndrome chromosome region, candidate 1 |
| 214669_x_at | IGKC | immunoglobulin kappa constant |
| 233123_at | SLC40A1 | solute carrier family 40 (iron-regulated transporter), member 1 |
| 209195_s_at | ADCY6 | adenylate cyclase 6 |
| 204846_at | CP | ceruloplasmin (ferroxidase) |
| 204642_at | EDG1 | endothelial differentiation, sphingolipid G-protein-coupled receptor, 1 |
| 239744_at | RGS3 | regulator of G-protein signalling 3 |
| 206545_at | CD28 | CD28 antigen (Tp44) |
| 228339_at | NA | NA |
| 218739_at | ABHD5 | abhydrolase domain containing 5 |
| 224358_s_at | MS4A7 | membrane-spanning 4-domains, subfamily A, member 7 |
| 1559425_at | PRKCH | protein kinase C, eta |
| 231577_s_at | GBP1 | guanylate binding protein 1, interferon-inducible, 67kDa |
| 231032_at | LOC286071 | NA |
| 207655_s_at | BLNK | B-cell linker |
| 242546_at | NA | NA |
| 211066_x_at | PCDHGC3 | protocadherin gamma subfamily C, 3 |
| 216714_at | CCL13 | chemokine (C-C motif) ligand 13 |
| 1562031_at | JAK2 | Janus kinase 2 (a protein tyrosine kinase) |
| 212763_at | CAMSAP1L1 | calmodulin regulated spectrin-associated protein 1-like 1 |
| 205440_s_at | NPY1R | neuropeptide Y receptor Y1 |
| 227458_at | CD274 | CD274 antigen |
| 226303_at | PGM5 | phosphoglucomutase 5 |
| 204613_at | PLCG2 | phospholipase C, gamma 2 (phosphatidylinositol-specific) |
| 202688_at | TNFSF10 | tumor necrosis factor (ligand) superfamily, member 10 |
| 221477_s_at | SOD2 | superoxide dismutase 2, mitochondrial |
| 201236_s_at | BTG2 | BTG family, member 2 |
| 205569_at | LAMP3 | lysosomal-associated membrane protein 3 |
| 215121_x_at | IGLC1 | immunoglobulin lambda constant 1 (Mcg marker) |
| 202255_s_at | SIPA1L1 | signal-induced proliferation-associated 1 like 1 |
| 215051_x_at | AIF1 | allograft inflammatory factor 1 |
| 209138_x_at | IGLC2 | immunoglobulin lambda constant 2 (Kern-Oz- marker) |
| 232311_at | B2M | beta-2-microglobulin |
| 1555756_a_at | CLEC7A | C-type lectin domain family 7, member A |
| 221756_at | MGC17330 | NA |
| 238544_at | IGF1R | insulin-like growth factor 1 receptor |
| 204512_at | HIVEP1 | human immunodeficiency virus type I enhancer binding protein 1 |
| 230728_at | FKBP14 | FK506 binding protein 14, 22 kDa |
| 201720_s_at | LAPTM5 | lysosomal associated multispanning membrane protein 5 |
| 223395_at | ABI3BP | ABI gene family, member 3 (NESH) binding protein |
| 200905_x_at | HLA-E | major histocompatibility complex, class I, E |
| 202207_at | ARL4C | ADP-ribosylation factor-like 4C |
| 207076_s_at | ASS | argininosuccinate synthetase |
| 211368_s_at | CASP1 | caspase 1, apoptosis-related cysteine peptidase (interleukin 1, beta, convertase) |
| 204628_s_at | ITGB3 | integrin, beta 3 (platelet glycoprotein IIIa, antigen CD61) |
| 207540_s_at | SYK | spleen tyrosine kinase |
| 213603_s_at | RAC2 | ras-related C3 botulinum toxin substrate 2 (rho family, small GTP binding protein Rac2) |
| 1557222_at | NA | NA |
| 206804_at | CD3G | CD3g antigen, gamma polypeptide (TiT3 complex) |
| 209542_x_at | IGF1 | insulin-like growth factor 1 (somatomedin C) |
| 228858_at | NA | NA |
| 207843_x_at | CYB5A | cytochrome b5 type A (microsomal) |
| 223924_at | TTC25 | tetratricopeptide repeat domain 25 |
| 1552497_a_at | SLAMF6 | SLAM family member 6 |
| 241701_at | ARHGAP21 | Rho GTPase activating protein 21 |
| 213819_s_at | FLOT1 | flotillin 1 |
| 213095_x_at | AIF1 | allograft inflammatory factor 1 |
| 228153_at | IBRDC2 | IBR domain containing 2 |
| 213007_at | KIAA1794 | KIAA1794 |
| 217525_at | OLFML1 | olfactomedin-like 1 |
| 204220_at | GMFG | glia maturation factor, gamma |
| 203508_at | TNFRSF1B | tumor necrosis factor receptor superfamily, member 1B |
| 217629_at | NA | NA |
| 226659_at | DEF6 | differentially expressed in FDCP 6 homolog (mouse) |
| 200904_at | HLA-E | major histocompatibility complex, class I, E |
| 206571_s_at | MAP4K4 | mitogen-activated protein kinase kinase kinase kinase 4 |
| 232746_at | CMKOR1 | chemokine orphan receptor 1 |
| 1563461_at | NA | NA |
| 204233_s_at | CHKA | choline kinase alpha |
| 226865_at | NA | NA |
| 227361_at | HS3ST3B1 | heparan sulfate (glucosamine) 3-O-sulfotransferase 3B1 |
| 204923_at | CXorf9 | chromosome X open reading frame 9 |
| 204774_at | EVI2A | ecotropic viral integration site 2A |
| 202270_at | GBP1 | guanylate binding protein 1, interferon-inducible, 67kDa |
| 243099_at | NFAM1 | NFAT activating protein with ITAM motif 1 |
| 242874_at | NA | NA |
| 229127_at | ATP5J | ATP synthase, H+ transporting, mitochondrial F0 complex, subunit F6 |
| 224771_at | NAV1 | neuron navigator 1 |
| 215379_x_at | IGLC2 | immunoglobulin lambda constant 2 (Kern-Oz- marker) |
| 222142_at | CYLD | cylindromatosis (turban tumor syndrome) |
| 229367_s_at | GIMAP6 | GTPase, IMAP family member 6 |
| 212713_at | MFAP4 | microfibrillar-associated protein 4 |
| 214023_x_at | TUBB2B | tubulin, beta 2B |
| 203413_at | NELL2 | NEL-like 2 (chicken) |
| 236583_at | NA | NA |
| 212657_s_at | IL1RN | interleukin 1 receptor antagonist |
| 227231_at | KIAA1211 | NA |
| 244023_at | SYK | spleen tyrosine kinase |
| 206134_at | ADAMDEC1 | ADAM-like, decysin 1 |
| 204894_s_at | AOC3 | amine oxidase, copper containing 3 (vascular adhesion protein 1) |
| 204502_at | SAMHD1 | SAM domain and HD domain 1 |
| 218002_s_at | CXCL14 | chemokine (C-X-C motif) ligand 14 |
| 205421_at | SLC22A3 | solute carrier family 22 (extraneuronal monoamine transporter), member 3 |
| 215561_s_at | IL1R1 | interleukin 1 receptor, type I |
| 217138_x_at | IGLV3-25 | immunoglobulin lambda variable 3-25 |
| 1556185_a_at | NA | NA |
| 219681_s_at | RAB11FIP1 | RAB11 family interacting protein 1 (class I) |
| 205251_at | PER2 | period homolog 2 (Drosophila) |
| 224896_s_at | TTL | tubulin tyrosine ligase |
| 209899_s_at | SIAHBP1 | NA |
| 201721_s_at | LAPTM5 | lysosomal associated multispanning membrane protein 5 |
| 241671_x_at | FLJ22536 | NA |
| 218499_at | RP6-213H19.1 | NA |
| 235804_at | NA | NA |
| 207677_s_at | NCF4 | neutrophil cytosolic factor 4, 40kDa |
| 227609_at | EPSTI1 | epithelial stromal interaction 1 (breast) |
| 227035_x_at | LOC441212 | NA |
| 206385_s_at | ANK3 | ankyrin 3, node of Ranvier (ankyrin G) |
| 1565602_at | PCDH9 | protocadherin 9 |
| 228908_s_at | C21orf86 | chromosome 21 open reading frame 86 |
| 223952_x_at | DHRS9 | dehydrogenase/reductase (SDR family) member 9 |
| 38149_at | ARHGAP25 | Rho GTPase activating protein 25 |
| 235688_s_at | TRAF4 | TNF receptor-associated factor 4 |
| 214181_x_at | LST1 | leukocyte specific transcript 1 |
| 222725_s_at | PALMD | palmdelphin |
| 1555852_at | TAP1 | transporter 1, ATP-binding cassette, sub-family B (MDR/TAP) |
| 210319_x_at | MSX2 | msh homeobox homolog 2 (Drosophila) |
| 220485_s_at | SIRPG | signal-regulatory protein gamma |

**Table 5. Primers and probes used for Q-PCR in standard 96 well plates**

| Gene name | Forward primers | Reverse primers | TaqMan Probes |
|---|---|---|---|
| IL7R | TCC-TAC-CAG-CCT-TTG-CCT-CTT | CCA-TAC-TTG-GCT-TTT-CTG-AAG-CA | FAM-CTT-CAA-TGT-GGT-TTC-CAT-GGG-MGB |
| CD3D | GTT-GAG-GAA-TGA-CCA-GGT-CTA-TCA-G | AAG-GTG-GCT-GTA-CTG-AGC-ATC-A | FAM-CCT-CCG-AGA-TCG-AG-MGB |
| CD52 | TCA-GCC-TCC-TGG-TTA-TGG-TAC-AG | TGG-TTT-GGC-TGG-TGT-CGT-T | FAM-CAA-ACT-GGA-CTC-TCA-GGA-C-MGB |
| UBD | TGA-GGA-GCT-GCC-CTT-GTT-TC | CTG-GAG-GAG-GTG-CCT-CTT-TG | FAM-CAT-CAC-CTG-ACT-CCA-CA-MGB |
| GPR171 | GAA-GTC-ATA-ACT-GAT-TGC-TCA-ACC-A | GAC-ACA-GCC-AGG-AGC-AGT-GTA-G | FAM-TCA-CTC-FFC-TTC-GCC-AAA-GA-MGB |
| GMZK | GTC-TGT-GCA-GGA-GAT-GCC-AA | GGG-CCC-CCT-GAG-TCA-CC | FAM_TTA-CAG-GAA-TCC-TTC-TGG-C_MGB |
| PRKCQ | ATC-CAT-GGT-CAA-GAG-GAC-AAG-TGG-A | GAC-AAG-ATA-ACA-AGC-GAA-GGT-GTC | FAM_CAA-ACC-TTC-CCT-CTC-ACT_MGB |
| STAT4 | GTC-CCA-CAA-CAA-TTG-AAA-CTG-C | GTG-CGT-CAG-AGT-TTA-TCC-TGT-CAT | FAM_TGA-AGT-CTC-CTT-ATT-CTG-CT_MGB |
| TRDV2 | GGT-CGA-GAA-AAG-CTT-TGA-AAC-AG | GGA-ACC-CAA-TCA-CTG-ACA-GGT-T | FAM_ACG-AAC-CTA-AAC-TTT-C_MGB |
| TRAT1 | AAG-CCA-CCC-CAT-CTG-CAC | CTG-TGA-TCA-AGT-GAG-GCG-TAG | FAM_CAA-CCA-ATG-AAA-CAC-AGA-TG_MGB |
| TRBV19 | GAG-GCC-TGG-GGT-AGA-GCA-G | AGA-GGA-TGG-TGG-CAG-ACA-GG | FAM_TGT-CCT-ACC-AGC-AAG-GG_MGB |
| CD69 | AAT-CAT-AGC-TCT-CAT-TGC-CTT-ATC-AG | TGT-CTG-ATG-GCA-TTG-AGA-ATG-TG | FAM_CCA-ATA-CAA-TTG-TCC-AGG-OC_MGB |
| INDO1 | CCA-GCA-GAC-TGC-TGG-TGG-A | GGC-ATA-TAT-CTT-CTC-ATG-TCC-TGG-A | FAM_ACA-TGC-TGC-TCA-GTT-C_MGB |
| H3F3A | CAG-CTA-TCG-GTG-CTT-TGC-AG | GAT-AGC-ACA-CAG-GTT-GGT-GTC-TTC | FAM_CAA-GTG-AGG-CCT-ATC-T_MGB |
| CD45R | GAG-ATG-CCT-GAT-GGT-TCA-AGT-AGA-G | TTC-TTC-AAA-CTG-ATT-GTA-TTC-CAC-C | FAM_CAG-TAC-ATC-TTG-ATC-CAT-C_MGB |
| CD45RO | GGC-AAA-GCC-CAA-CAC-CTT-C | CAG-AAG-GGC-TCA-GAG-TGG-TTG-T | FAM_TGC-CTA-CCT-TAA-TGC-C_MGB |
| FoxP3 | CAC CTG GCT GGG AAA ATG G | GGAGCC CTT GTC GGA TGA T | FAM_CAA GGC TTC ATC TGT G_MGB |
| CD20 | CAT-ACA-ATC-TCT-GTT-CTT-GGG-CAT-T | TCA-TTC-TCA-ACG-ATG-CCA-GCT-A | FAM_TCA-GTG-ATG-CTG-ATC-TT_MGB |
| TLR9 | CGC-CAG-ACC-CTC-TGG-AGA-A | GCC-TGC-ACC-AGG-AGA-GAC-A | FAM_TTG-TGC-GGC-AGC-G_MGB |
| ITGB3 | AAG-TCC-ATC-CTG-TATG-TGG-TAG-AAG-AG | GAG-CAG-GAC-CAC-CAG-GAT-GT | FAM_AGT-GTC-CCA-AGG-GC_MGB |
| TLR3 | GGA-AAG-GCT-AGC-AGT-CAT-CCA | GTG-GAG-GAT-GCA-CAC-AGC-AT | FAM_ATG-AGA-CAG-ACT-TTG-CC_MGB |
| TLR4 | CCA-GAA-CTG-CAG-GTG-CTG-G | GGT-GGC-TTA-GGC-TCT-GAT-ATG-C | FAM_CCA-GGT-GTG-AAA-TCC-AGA-CA_MGB |
| TLR7 | Assay-on-demand Hs00152971_m1 (Applied Biosystems) | | |
| TLR10 variant 1 | Assay-on-demand Hs01675179_m1 (Applied Biosystems) | | |
| IRF1 | Assay-on-demand Hs00971965_m1 (Applied Biosystems) | | |

**Table6. Gene included into the TaqMan® Immune Profiling Array**

| **Assay ID** | **Gene Symbol** | **Target Exons** | **NCBI Gene Reference** |
|---|---|---|---|
| Hs00174092_m1 | IL1A | 6 | NM_000575,M15329,X02531,X02851,X56086,CR457414,BC035169,BC013142,BT007014 |
| Hs00174097_m1 | IL1B | 5 | NM_000576,K02770,M15330,M54933,X02532,X56087,AF043335,BC008678,CR407679,BT007213 |
| Hs00174114_m1 | IL2 | 2 | NM_000586,U25676,V00564,X01586,S82692,S77835,S77834,AF228636,BC066255,BC066256,BC066257,B C066254,AY523040,BC070338,A14844 |
| Hs00174117_m1 | IL3 | 2 | NM_000588,M17115,M14743,M20137,BC066274,BC066276,BC066272,BC066273,BC069472 |
| Hs00174122_m1 | IL4 | 3 | NM_172348,NM_000589,M13982,X81851,AF043336,BC066277,BC067514,BC067515,BC070123,AB102862 |
| Hs00174200_m1 | IL5 | 2 | NM_000879,X12705,X04688,BC066282 |
| Hs00174131_m1 | IL6 | 3 | NM_000600,X04403,M14584,M1 18403,M29150,X04430,X04602,M54894,S56892,CR450296,BT019748,BT01 9749,A09363,BC015511 |
| Hs00174202_m1 | IL7 | 2 | NM 000880,J04156,BC047698 |
| Hs00174103_m1 | IL8 | 1 | NM_000584,M17017,M26383,Z11686,Y00787,CR542151,AK131067,BC013615,BT007067 |
| Hs00174125_m1 | IL9 | 3 | NM_000590,M30134,X17543,S63356,BC066284,BC066283,BC066285,BC066286 |
| Hs99999901_s1 | 18S | 1 | X03205 |
| Hs00174086_m1 | IL10 | 3 | NM_000572,M57627,AF043333,AY029171,CR541993 |
| Hs00168405_m1 | IL12A | 2 | NM_000882,M65271,M65291,AF101062,AF180562,BG702253 |
| Hs00233688_m1 | IL12B | 3 | NM_002187,M65272,M65290,AF180563,BC067501,BC067498,BC067499,BC067500,BC067502,BC074723 |
| Hs00174379_m1 | IL13 | 1 | NM_002188,L06801,X69079,BC096141,BC096139 |
| Hs00174106_m1 | IL15 | 1 | NM_000585,U14407 |
| Hs00174383_m1 | IL17 | 2 | NM_002190,Z58820,U32659,BC066251,BC066252,BC066253,BC067504,BC067503,BC067505 |
| Hs00155517_m1 | IL18 | 4 | NM_001562,D49950,U90434,AF077611,AY266351,CR541973,CR542001,AY044641,BC015863,BC007007, |
| Hs00234142_m1 | CCL3 | 1 | NM_002983,M25315,D63785,M23452 ,D00044,X03754,BC071834 |
| Hs00171149_m1 | CCL19 | 1 | NM_006274,AB000887,U77180,U88321,CR456868,BC027968 |
| Hs00234140_m1 | CCL2 | 1 | NM_002982,M24545,M28226,X14768,S69738,S71513,AV733621,BC009716,BT007329 |
| Hs00174575_m1 | CCL5 | 2 | NM_002985,M21121,AF043341,BC008600,AF266753 |
| Hs00174150_m1 | CCR2 | 1 | NM_000647,U03882,BC074751 |
| Hs99999919_m1 | CCR4 | N/A | NM_005508,BC069139,BC071751,BC074935 |
| Hs00152917_m1 | CCR5 | 2 | NM_000579,U54994,BC038398 |
| Hs00171054_m1 | CCR7 | 1 | NM_001838,L31581,L08176,BC035343,AK127810 |
| Hs00171041_m1 | CXCR3 | 1 | NM_001504,X95876 |
| Hs00171042_m1 | CXCL10 | 1 | NM_001565,X02530,BC010954 |
| Hs00171138_m1 | CXCL11 | 1 | NM_005409,Y15220,U59286,AF030514,AF002985,U66096,BC005292,AF352781,BC012532,BT006787 |
| Hs00174164_m1 | CSF1 | 4 | NM_172210,NM_172211,NM_172212,NM_ 000757,U22386,M37435,M76453,M64592,M27087,X05825,BC02 |
| Hs00171266_m1 | CSF2 | 3 | NM_000758,M10663,M11220,AY720441,AF510855 |
| Hs00357085_g1 | CSF3 | 1 | NM_172219,NM 000759,M17706,X03438,X03655,CR541891 |
| Hs00234174_m1 | STAT3 | N/A | NM_139276,AJ012463,L29277,BC014482 |
| Hs00174517_m1 | NFKB2 | 6 | NM_002502,U09609,X61498,X61499,S76638,BC002844,BT009769 |
| Hs00395088_m1 | IKBKB | 8 | NM_001556,AF029684,AF031416,AF080158,AB209090,BX648165 |
| Hs00167894_m1 | CD3E | 1 | NM_000733,X03884,BI910359 |
| Hs00181217_m1 | CD4 | 2 | NM_000616,M12807,BT019791,BT019811,BC025782 |
| Hs00233520_m1 | CD8A | 5 | NM_171827,NM_001768,M12824,M12828,AY039664,AK124156,BC025715,AK097942 |
| Hs00174333_m1 | CD19 | 4 | NM_001770,M21097,X13312,BC006338,AK130657,BC052294 |
| Hs00166229_m1 | IL2RA | 1 | NM_000417,X01057,K03122,AK223313 |
| Hs00174796_m1 | CD28 | 2 | NM_006139,J02988,BC093698 |
| Hs00233552_m1 | CD38 | 1 | NM_001775,D84276,M34461,BC007964,CD688069 |
| Hs00374176_m1 | CD40 | 1 | NM_152854,NM_001250,AJ300189,BC064518,BC012419,BM761221,AK222896 |
| Hs00365634_g1 | PTPRC | 1 | NM_080921,NM_080922,NM_080923,NM_002838,Y00062,BC017863,BC014239 |
| Hs00154355_m1 | CD68 | 6 | NM_001251,S57235,BC015557,BT009923,AK222492,AK222514 |
| Hs00175478_m1 | CD80 | 2 | NM_005191,M27533,AY197777,AY197778,AY081815,BC042665 |
| Hs00199349_m1 | CD86 | 2 | NM_175862,NM_006889,L25259,U04343,CR541844,BC040261 |
| Hs00175480_m1 | CTLA4 | 1 | NM_005214,L15006,BC069566,BC070162,BC074842,BC074893,AY209009,AF414120,AF486806 |
| Hs00163934_m1 | CD40LG | 4 | NM_000074,L07414,X67878,Z15017,X68550,BC071754,BC074950 |
| Hs00219575_m1 | HLA-DRA | 1 | NM_019111,J00194,M60334,M35979,K01171,M60333,CR457013,BC071659,BC032350 |
| Hs99999917_m1 | HLA-DRB1 | N/A | NM_002124,M11161,M33600,X03069,DQ002917 |
| Hs00203436_m1 | TBX21 | 1 | NM_013351,AF093098,AF241243,BC039739,AK223634 |
| Hs00188346_m1 | TNFRSF18 | 2 | NM_148901,NM_148902,NM_004195,AF117297,AF125304,AF241229,BT019531,BT01 9532,AY358877 |
| Hs00359999_m1 | ICOS | 1 | NM_012092,AB023135,AJ277832,BC028006,BC028210 |
| Hs00167248_m1 | NOS2A | 11 | NM_153292,NM_000625,AB022318,U05810,D26525,L24553,X73029,U20141,U31511,375615 |
| Hs00153350_m1 | BCL2 | 1 | NM_000633,M13994,M14745,X06487,BC027258 |
| Hs00169141_m1 | BCL2L1 | 1 | NM_001191,Z23116 |
| Hs00180269_m1 | BAX | 3 | NM_138761,NM_138763,NM_138765,NM_004324,L22473,L22474,U19599,AF247393,AF250190,AJ586909, AJ586910,BM706954,BC014175,AJ417988 |
| Hs00164932_m1 | ICAM1 | 2 | NM_000201,M24283,J03132,X06990,AF340038,AF340039,BC015969,AK130659,BT006854 |
| Hs00174583_m1 | SELP | 1 | NM_003005,M25322,BC068533 |
| Hs00174057_m1 | SELE | 4 | NM_000450,M24736,M30640 |
| Hs00157965_m1 | HMOX1 | 2 | NM_002133,M23041,X06985,CR456505,CR541945,CR541968,BT019785,BC001491 |
| Hs00153133_m1 | PTGS2 | 5 | NM_000963,M90100,L15326,AJ634912,AY462100,BC013734,AY151286 |
| Hs00166915_m1 | REN | 1 | NM_000537,CR536498,BC047752,BC033474 |
| Hs00189742_m1 | LRP2 | 17 | NM_004525,U33837,AY265357,AY265358 |
| Hs00411908_m1 | MYH6 | 23 | NM_002471,D00943 |
| Hs00192564_m1 | RPL3L | 3 | NM_005061,U65581,BC050413 |
| Hs00167927_m1 | CYP 1 A2 | 2 | NM_000761,M55053,Z00036,AF1 82274,BC067425,BC067427,BC067424,BC067429,BC067426,BC067428 |
| Hs00167982_m1 | CYP7A1 | 3 | NM_000780,X56088,M93133 |
| Hs00174143_m1 | IFNG | 1 | NM_000619,X13274,V00543,X87308,BC070256,AY255837,AY255839,AY044154,AF506749 |
| Hs00169473_m1 | PRF1 | 1 | NM_005041,M28393,X13224,BC063043,BC047695 |

**Table 7AGeomean ratio between responder and non-responder groups**

| **Gene** | **Ratio** | **LL** | **UL** |
|---|---|---|---|
| CCL5 | 7.83 | 3.09 | 19.84 |
| FASLG | 7.57 | 2.77 | 20.70 |
| GNLY | 8.82 | 3.61 | 21.56 |
| GZMB | 12.15 | 4.38 | 33.71 |
| PRF1 | 11.07 | 4.85 | 25.28 |
| IFNG | 21.19 | 5.36 | 83.86 |
| ICOS | 13.44 | 3.44 | 52.51 |
| TBX21 | 13.93 | 3.63 | 53.41 |
| CD8A | 11.60 | 4.22 | 31.90 |
| CD3E | 12.78 | 3.55 | 46.04 |
| CXCL11 | 4.71 | 1.44 | 15.35 |
| CXCL10 | 7.39 | 2.56 | 21.39 |
| CXCR3 | 16.96 | 3.10 | 92.90 |
| CD20 | 9.46 | 1.35 | 66.42 |
| FOXP3 | 5.57 | 2.58 | 12.04 |
| INDO | 13.62 | 2.89 | 64.20 |
| CD45Ro | 4.02 | 1.84 | 8.79 |
| CD45R | 4.92 | 1.90 | 12.77 |
| CD69 | 5.84 | 2.00 | 17.06 |
| TRBV19 | 11.15 | 3.56 | 34.94 |
| TRAT1 | 9.67 | 2.77 | 33.80 |
| TRDV2 | 7.05 | 2.79 | 17.82 |
| STAT4 | 3.41 | 1.75 | 6.65 |
| PRKCQ | 7.41 | 3.24 | 16.94 |
| GMZK | 9.20 | 2.60 | 32.49 |
| GPR171 | 5.25 | 2.47 | 11.17 |
| UBD | 14.68 | 3.95 | 54.65 |
| CD52 | 6.17 | 1.96 | 19.41 |
| CD3D | 10.03 | 3.07 | 32.77 |
| IL7R | 6.49 | 2.08 | 20.26 |
| IRF1 | 5.54 | 2.83 | 10.83 |
| TLR7 | 3.44 | 1.50 | 7.89 |

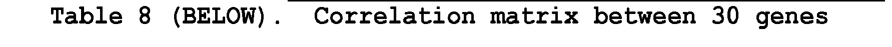

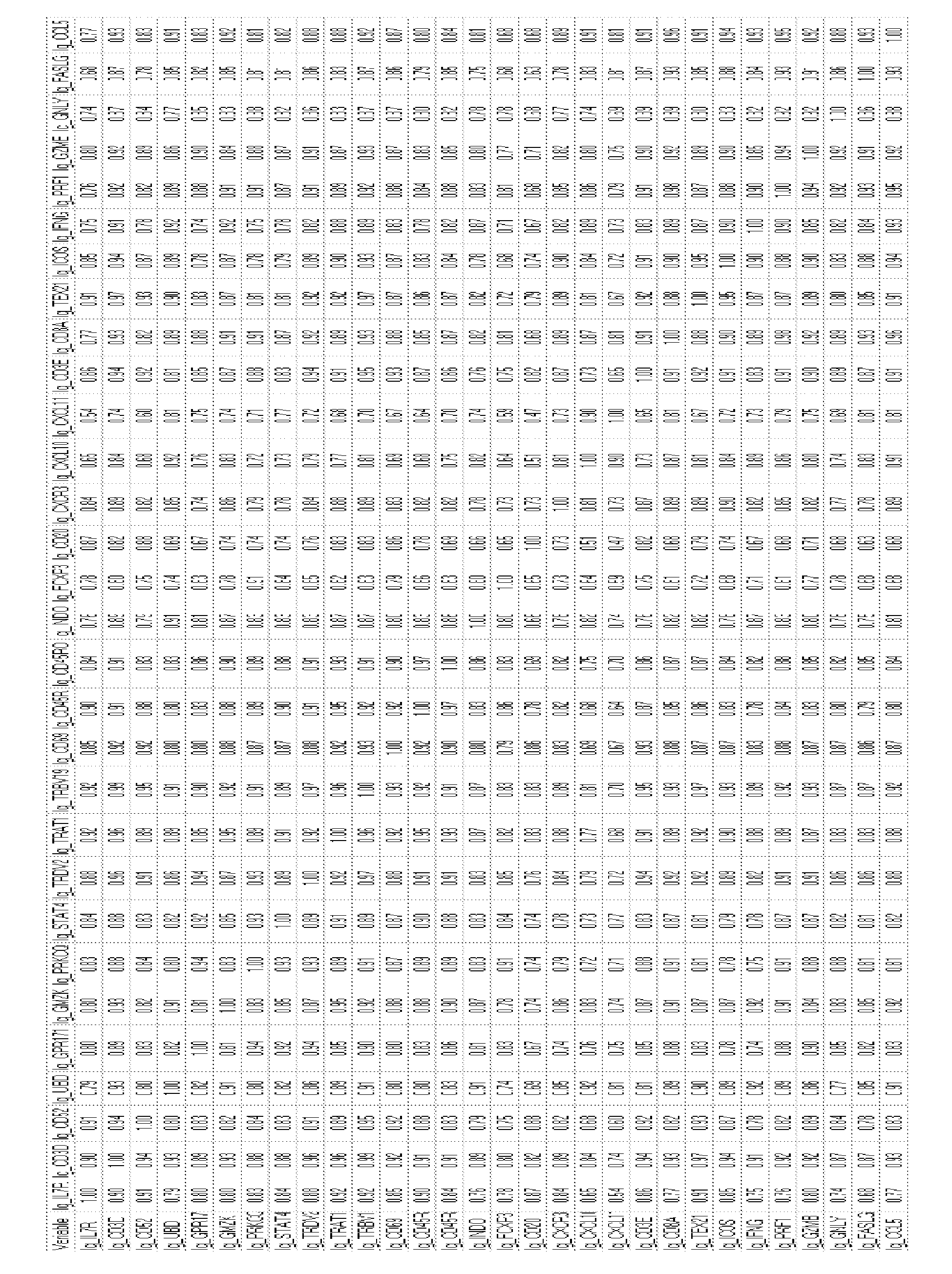

**Table 9A 18 best models obtained by logistic regression.**

| **Predictor** | **R2 (%)** | **Chi-square Score** |
|---|---|---|
| PRF1 | 55 | 16 |
| IRF1 | 50 | 14 |
| GZMB | 46 | 14 |
| GNLY | 45 | 14 |
| CD8A | 46 | 14 |
| PRKCQ | 45 | 14 |
| FOXP3 | 42 | 13 |
| IFNG | 52 | 13 |
| CCL5 | 45 | 13 |
| GPR171 | 41 | 12 |
| TRBV19 | 44 | 11 |
| CD3E | 39 | 11 |
| TBX21 | 44 | 11 |
| FASLG | 39 | 11 |
| CXCL10 | 35 | 10 |
| ICOS | 39 | 10 |
| CXCR3 | 35 | 9 |
| CXCL11 | 22 | 6 |

**Table10 Percentage of correct classification calculated using the logistic regression model for some genes.**

| **Model** | **Percentage of patients correctly classified** |
|---|---|
| PRF1 | 86,6% |
| IRF1 | 89,7% |
| GZMB | 82,8% |
| GNLY | 82,8% |
| CD8A | 82,8% |
| PRKCQ | 89,7% |
| FOXP3 | 86,2% |
| IFNG | 89,7% |
| CCL5 | 79,3% |
| GPR171 | 82,8% |
| TRBV19 | 86,2% |
| CDE3 | 75,9% |
| TBX21 | 82,8% |
| FASLG | 75,9% |
| CXCL10 | 75,9% |
| ICOS | 75,9% |
| CXR3 | 75,9% |
| CXCL11 | 72,4% |

**TABLE 11A**

| | | | **Patient Identity Number** | | | | | | | | | |
|---|---|---|---|---|---|---|---|---|---|---|---|---|
| **Probe Set** | **Gene Title** | **Gene Symbol** | **14** | **52** | **66** | **37** | **16** | **13** | **55** | **56** | **5** | **60** |
| **1555759 _a_at** | chemokine (C-C motif) ligand 5 | CCL5 | 3.148 | 3.204 | 3.208 | 3.096 | 3.055 | 3.049 | 3.096 | 3.208 | 2.664 | 2.835 |
| **1555834 _at** | Ubiquitin carboxyl-terminal esterase L1 (ubiquitin thiolesterase) | UCHL1 | 7.911 | 2.4 | 2.324 | 5.169 | 5.06 | 2.401 | 3.561 | 2.627 | 4.868 | 2.401 |
| **1558290 _a_at** | Pvt1 oncogene homolog, MYC activator (mouse) /// LOC441378 | PVT1 /// LOC44 1378 | 10.849 | 10.579 | 11.312 | 10.154 | 9.815 | 10.752 | 11.192 | 9.514 | 10.132 | 8.752 |
| **1569942 _at** | CDNA clone IMAGE:479638 8 | - | 5.295 | 3.843 | 4.837 | 5.187 | 3.748 | 5.656 | 3.518 | 6.042 | 6.463 | 4.378 |
| **204661_ at** | CD52 antigen (CAMPATH-1 antigen) /// CD52 antigen (CAMPATH-1 antigen) | CD52 | 5.673 | 5.506 | 8.56 | 5.104 | 5.814 | 5.755 | 4.087 | 4.785 | 3.839 | 4.29 |
| **205890_ s_at** | ubiquitin D | UBD | 4.731 | 7.003 | 9.226 | 4.138 | 3.687 | 8.103 | 3.242 | 6.126 | 3.231 | 7.706 |
| **206118_ at** | signal transducer and activator of transcription 4 | STAT4 | 4.336 | 3.291 | 4.142 | 2.396 | 3.235 | 3.291 | 2.382 | 2.439 | 2.663 | 2.948 |
| **206666_ at** | granzyme K (granzyme 3; tryptase II) /// granzyme K (granzyme 3; tryptase II) | GZMK | 6.187 | 5.357 | 7.079 | 2.659 | 2.571 | 5.033 | 2.225 | 3.383 | 2.567 | 3.104 |
| **207072_ at** | interleukin 18 receptor accessory protein | IL18R AP | 2.225 | 2.237 | 2.232 | 2.232 | 2.231 | 2.232 | 2.227 | 2.237 | 2.227 | 2.237 |
| **207651_ at** | G protein-coupled receptor 171 | GPR17 1 | 3.961 | 7.086 | 4.363 | 4.635 | 3.271 | 5.499 | 3.426 | 4.679 | 3.42 | 4.672 |
| **209606_ at** | pleckstrin homology, Sec7 and coiled-coil domains, binding protein /// pleckstrin homology, Sec7 and coiled-coil domains, binding protein | PSCDB P | 4.553 | 5.031 | 5.888 | 4.679 | 3.194 | 3.996 | 3.036 | 3.285 | 3.692 | 3.473 |
| **209949_ at** | neutrophil cytosolic factor 2 (65kDa, chronic granulomatous disease, autosomal 2) | NCF2 | 6.058 | 6.184 | 3.286 | 6.305 | 4.585 | 3.866 | 4.672 | 3.294 | 4.457 | 3.802 |
| **210038_at** | protein kinase C, theta | PRKC Q | 2.362 | 2.18 | 2.415 | 2.178 | 2.364 | 2.358 | 2.577 | 2.181 | 2.179 | 2.191 |
| **210629_ x_at** | leukocyte specific transcript 1 | LST1 | 5.649 | 6.559 | 6.72 | 5.368 | 5.367 | 3.893 | 3.484 | 6.134 | 4.775 | 5.283 |
| **210972_ x_at** | T cell receptor alpha locus /// T cell receptor delta variable 2 /// T cell receptor alpha variable 20 /// T cell receptor alpha joining 17 /// T cell receptor alpha constant | TRA@ /// TRDV2 /// TRAV2 0 /// TRAJ1 7 /// TRAC | 4.292 | 6.159 | 7.116 | 3.791 | 4.059 | 6.091 | 5.234 | 5.057 | 4.291 | 4.842 |
| **211144_ x_at** | T cell receptor gamma constant 2 | TRGC2 | 2.887 | 2.261 | 4.452 | 2.257 | 2.193 | 3.203 | 2.347 | 2.453 | 2.332 | 2.196 |
| **211796_ s_at** | T cell receptor beta variable 21-1 /// T cell receptor beta variable 19 /// T cell receptor beta variable 5-4 /// T cell receptor beta variable 3-1 //// T cell receptor beta constant 1 | TRBV2 1-1 /// TRBV1 9 /// TRBV5 -4 /// TRBV3 -1 /// TRBC1 | 3.275 | 3.556 | 7.359 | 2.877 | 2.424 | 5.177 | 2.521 | 2.728 | 2.297 | 2.909 |
| **212671_ s_at** | major histocompatibilit y complex, class II, DQ alpha 1 /// major histocompatibilit y complex, class II, DQ alpha 2 | HLA-DQA1 /// HLA-DQA2 | 8.1 | 7.479 | 6.553 | 8.223 | 6.574 | 7.356 | 7.347 | 5.884 | 6.068 | 5.558 |
| **213193_ x_at** | T cell receptor beta variable 19 /// T cell receptor beta variable 19 /// T cell receptor beta constant 1 /// T cell receptor beta constant 1 | TRBV1 9 /// TRBC1 | 4.185 | 3.787 | 7.027 | 3.011 | 2.962 | 4.53 | 3.096 | 3.192 | 2.829 | 3.058 |
| **213539_ at** | CD3D antigen, delta polypeptide (TiT3 complex) | CD3D | 3.179 | 4.399 | 7.52 | 3.352 | 3.182 | 5.87 | 2.8 | 3.183 | 3.178 | 3.626 |
| **213560_ at** | Growth arrest and DNA-damage-inducible, beta | GADD 45B | 4.967 | 5.948 | 6.218 | 5.528 | 4.673 | 5.367 | 5.49 | 3.67 | 5.735 | 5.048 |
| **216261_ at** | integrin, beta 3 (platelet glycoprotein IIIa, antigen CD61) | ITGB3 | 8.21 | 2.588 | 4.394 | 3.536 | 6.237 | 4.486 | 3.088 | 6.729 | 6.77 | 2.606 |
| **217147_ s_at** | T cell receptor associated transmembrane adaptor 1 | TRAT1 | 3.914 | 4.251 | 5.453 | 2.871 | 2.292 | 4.283 | 2.327 | 2.887 | 2.277 | 2.308 |
| **217478_ s_at** | major histocompatibilit y complex, class II, DM alpha | HLA-DMA | 8.989 | 8.655 | 8.599 | 9.516 | 8.621 | 7.051 | 8.181 | 8.129 | 7.41 | 8.04 |
| **218950_ at** | centaurin, delta 3 | CENT D3 | 9.212 | 7.139 | 8.794 | 8.51 | 8.32 | 7.438 | 9.077 | 8.075 | 9.012 | 7.142 |
| **220330_ s_at** | SAM domain, SH3 domain and nuclear localisation signals, 1 | SAMS N1 | 4.712 | 4.228 | 5.117 | 4.641 | 4.804 | 3.557 | 2.702 | 4.246 | 3.492 | 3.828 |
| **222934_ s_at** | C-type lectin domain family 4, member E | CLEC4 E | 5.382 | 4.219 | 2.931 | 2.753 | 2.511 | 3.812 | 4.494 | 2.512 | 3.917 | 3.417 |
| **223827_ at** | tumor necrosis factor receptor superfamily, member 19 | TNFRS F19 | 4.16 | 3.985 | 5.98 | 2.86 | 5.91 | 3.239 | 4.301 | 6.268 | 5.029 | 4.931 |
| **224771_ at** | neuron navigator 1 | NAV1 | 7.893 | 6.773 | 7.352 | 7.998 | 8.385 | 7.571 | 7.077 | 7.277 | 6.956 | 7.304 |
| **224772_ at** | neuron navigator 1 | NAV1 | 10.827 | 9.319 | 9.458 | 9.799 | 10.771 | 10.304 | 9.47 | 9.943 | 9.752 | 9.998 |
| **224773_ at** | neuron navigator 1 | NAV1 | 8.959 | 7.241 | 7.742 | 9.332 | 9.582 | 8.051 | 8.427 | 8.479 | 7.795 | 7.564 |
| **224774_ s_at** | neuron navigator 1 | NAV1 | 7.288 | 6.204 | 7.292 | 8.109 | 7.683 | 6.486 | 7.056 | 5.947 | 6.406 | 6.181 |
| **227145_ at** | lysyl oxidase-like 4 | LOXL4 | 11.368 | 8.235 | 10.93 | 12.565 | 12.636 | 12.563 | 7.768 | 11.858 | 12.092 | 11.625 |
| **227584_ at** | Neuron navigator 1 | NAV1 | 11.735 | 10.222 | 10.601 | 10.533 | 11.778 | 11.041 | 10.121 | 11.247 | 10.732 | 11.003 |
| **227812_ at** | tumor necrosis factor receptor superfamily, member 19 | TNFRS F19 | 11.9 | 11.787 | 12.618 | 10.566 | 12.747 | 11.729 | 11.115 | 12.911 | 12.125 | 11.837 |
| **229723_ at** | T-cell activation GTPase activating protein | TAGA P | 5.224 | 4.874 | 5.527 | 5.091 | 2.501 | 3.01 | 4.398 | 2.891 | 3.472 | 3.425 |
| **232024_ at** | GTPase, IMAP family member 2 | GIMAP 2 | 8.489 | 7.321 | 7.275 | 6.411 | 7.83 | 6.861 | 6.317 | 8.172 | 7.42 | 7.119 |
| **238544_ at** | Insulin-like growth factor 1 receptor | IGF1R | 6.79 | 8.031 | 9.43 | 5.969 | 7.331 | 7.827 | 8.887 | 6.322 | 9.011 | 7.444 |
| **242886_ at** | Chromosome 17 open reading frame 63 | C17orf 63 | 10.066 | 10.766 | 9.654 | 8.976 | 9.813 | 9.864 | 10.039 | 9.839 | 8.819 | 9.808 |
| **242986_ at** | Neuron navigator 1 | NAV1 | 9.936 | 9.155 | 9.341 | 10.292 | 10.071 | 9.41 | 9.098 | 9.552 | 8.465 | 8.663 |
| **34210_at** | CD52 antigen (CAMPATH-1 antigen) | CD52 | 6.58 | 6.581 | 9.446 | 5.799 | 6.518 | 6.798 | 4.246 | 6.008 | 3.469 | 5.289 |

**TABLE 11B**

| | | | **Patient Identity Number** | | | | | | | | | | | |
|---|---|---|---|---|---|---|---|---|---|---|---|---|---|---|
| **Probe Set** | **Gene Title** | **Gene Symbol** | **75** | **2** | **65** | **20** | **10** | **19** | **23** | **26** | **27** | **38** | **8** | **67** |
| **155575 9_a_at** | chemokine (C-C motif) ligand 5 | CCL5 | 4.665 | 5.498 | 7.109 | 3.207 | 5.121 | 3.656 | 4.159 | 4.664 | 5.256 | 5.411 | 3.034 | 3.11 |
| **155583 4_at** | Ubiquitin carboxyl-terminal esterase L1 (ubiquitin thiolesterase) | UCHL1 | 2.329 | 2.36 | 2.4 | 2.399 | 2.358 | 2.398 | 2.463 | 2.401 | 2.4 | 2.4 | 5.041 | 2.401 |
| **155829 0_a_at** | Pvt1 oncogene homolog, MYC activator (mouse) /// LOC441378 | PVT1 /// LOC44 1378 | 8.59 | 7.403 | 8.63 | 8.588 | 9.536 | 8.46 | 9.384 | 9.409 | 8.504 | 8.57 | 10.616 | 9.901 |
| **156994 2_at** | CDNA clone IMAGE:4796 388 | - | 3.605 | 3.324 | 4.066 | 2.849 | 4.189 | 2.198 | 3.972 | 3.61 | 3.392 | 2.944 | 5.832 | 4.382 |
| **204661 _at** | CD52 antigen (CAMPATH-1 antigen) /// CD52 antigen (CAMPATH-1 antigen) | CD52 | 8.008 | 8.301 | 9.702 | 8.979 | 9.415 | 6.144 | 8.556 | 8.487 | 11.34 | 10.289 | 4.082 | 8.696 |
| **205890_s_at** | ubiquitin D | UBD | 11.687 | 10.159 | 11.582 | 7.52 | 9.692 | 8.088 | 9.801 | 12.384 | 10.657 | 10.131 | 3.178 | 9.54 |
| **206118 _at** | signal transducer and activator of transcription 4 | STAT4 | 5.794 | 5.481 | 4.044 | 5.157 | 6.026 | 5.775 | 6.731 | 5.845 | 8.349 | 6.709 | 2.383 | 3.09 |
| **206666 _at** | granzyme K (granzyme 3; tryptase II) /// granzyme K (granzyme 3; tryptase II) | GZMK | 7.615 | 9.057 | 9.338 | 6.513 | 8.013 | 7.564 | 8.532 | 8.921 | 8.337 | 9.328 | 2.305 | 6.371 |
| **207072 _at** | interleukin 18 receptor accessory protein | IL18R AP | 4.76 | 2.358 | 2.233 | 3.154 | 2.235 | 2.264 | 2.512 | 2.239 | 3.2 | 4.126 | 2.222 | 2.238 |
| **207651 _at** | G protein-coupled receptor 171 | GPR17 1 | 8.491 | 8.969 | 8.37 | 6.884 | 8.501 | 8.037 | 8.578 | 10.668 | 10.834 | 9.404 | 3.046 | 6.328 |
| **209606 _at** | pleckstrin homology, Sec7 and coiled-coil domains, binding protein /// pleckstrin homology, Sec7 and coiled-coil domains, binding protein | PSCDB P | 6.808 | 6.788 | 8.373 | 6.524 | 7.997 | 7.366 | 7.301 | 7.101 | 10.455 | 7.959 | 2.232 | 7.231 |
| **209949 _at** | neutrophil cytosolic factor 2 (65kDa, chronic granulomatou s disease, autosomal 2) | NCF2 | 6.928 | 6.845 | 7.973 | 5.572 | 5.428 | 6.643 | 7.499 | 6.281 | 6.48 | 7.213 | 3.405 | 7.449 |
| **210038 _at** | protein kinase C, theta | PRKC Q | 4.981 | 4.768 | 2.406 | 2.836 | 6.907 | 4.108 | 5.51 | 5.272 | 8.218 | 4.73 | 2.179 | 2.301 |
| **210629 _x_at** | leukocyte specific transcript 1 | LST1 | 7.078 | 6.711 | 6.926 | 5.831 | 6.424 | 6.69 | 7.323 | 6.834 | 7.058 | 8.828 | 3.779 | 7.287 |
| **210972 _x_at** | T cell receptor alpha locus /// T cell receptor delta variable 2 /// T cell receptor alpha variable 20 /// T cell receptor alpha joining 17 /// T cell receptor alpha constant | TRA@ /// TRDV2 /// TRAV2 0 /// TRAJ1 7 /// TRAC | 7.913 | 8.072 | 11.25 | 6.451 | 10.646 | 7.331 | 8.326 | 9.727 | 11.544 | 8.3 | 4.292 | 6.411 |
| **211144 _x_at** | T cell receptor gamma constant 2 | TRGC2 | 6.096 | 4.892 | 6.456 | 2.624 | 6.163 | 2.689 | 6.279 | 7.941 | 7.365 | 6.31 | 2.189 | 2.645 |
| **211796 _s_at** | T cell receptor beta variable 21-1 /// T cell receptor beta variable 19 /// T cell receptor beta variable 5-4 /// T cell receptor beta variable 3-1 /// T cell receptor beta constant 1 | TRBV2 1-1 /// TRBV1 9 /// TRBV5 -4 /// TRBV3 -1 /// TRBC1 | 7.924 | 8.11 | 10.708 | 6.84 | 9.737 | 6.456 | 8.211 | 9.022 | 11.111 | 8.316 | 2.293 | 5.742 |
| **212671 _s_at** | major histocompati bility complex, class II, DQ alpha 1 /// major histocompatibility complex, class II, DQ alpha 2 | HLA-DQA1 /// HLA-DQA2 | 10.798 | 9.601 | 11.257 | 9.336 | 8.87 | 9.39 | 9.435 | 10.008 | 10.974 | 10.757 | 2.679 | 7.61 |
| **213193 _x_at** | T cell receptor beta variable 19 /// T cell receptor beta variable 19 /// T cell receptor beta constant 1 /// T cell receptor beta constant 1 | TRBV1 9 /// TRBC1 | 7.553 | 7.871 | 10.191 | 6.704 | 9.248 | 5.828 | 7.936 | 8.266 | 11.152 | 7.722 | 2.83 | 5.472 |
| **213539 _at** | CD3D antigen, delta polypeptide (TiT3 complex) | CD3D | 8.208 | 8.038 | 9.64 | 7.257 | 8.387 | 6.635 | 8.381 | 9.075 | 10.519 | 8.841 | 2.311 | 6.078 |
| **213560 _at** | Growth arrest and DNA-damage-inducible, beta | GADD 45B | 7.243 | 5.936 | 6.794 | 7.57 | 6.389 | 7.236 | 7.105 | 6.776 | 7.114 | 6.774 | 3.402 | 7.123 |
| **216261 _at** | integrin, beta 3 (platelet glycoprotein IIIa, antigen CD61) | ITGB3 | 2.545 | 2.691 | 2.651 | 2.592 | 2.59 | 2.553 | 2.609 | 4.561 | 2.622 | 2.557 | 6.05 | 2.602 |
| **217147 _s_at** | T cell receptor associated transmembra ne adaptor 1 | TRAT1 | 6.748 | 6.139 | 8.028 | 5.044 | 7.377 | 7.56 | 6.512 | 7.612 | 9.246 | 8.077 | 2.281 | 3.002 |
| **217478 _s_at** | major histocompati bility complex, class II, DM alpha | HLA-DMA | 10.873 | 10.838 | 11.541 | 9.389 | 10.193 | 9.475 | 10.475 | 9.998 | 11.266 | 10.161 | 7.109 | 9.33 |
| **218950 _at** | centaurin, delta 3 | CENT D3 | 7.911 | 7.983 | 5.552 | 6.866 | 7.456 | 5.891 | 7.35 | 5.522 | 5.804 | 6.245 | 8.04 | 8.202 |
| **220330 _s_at** | SAM domain, SH3 domain and nuclear localisation signals, 1 | SAMS N1 | 7.494 | 6.244 | 4.271 | 5.243 | 7.151 | 6.458 | 7.429 | 8.583 | 8.421 | 7.405 | 2.325 | 6.719 |
| **222934 _s_at** | C-type lectin domain family 4, member E | CLEC4 E | 6.912 | 6.523 | 6.984 | 5.384 | 4.588 | 6.678 | 6.941 | 4.589 | 4.731 | 5.877 | 3.019 | 7.666 |
| **223827 _at** | tumor necrosis factor receptor superfamily, member 19 | TNFRS F19 | 3.043 | 3.308 | 2.721 | 2.855 | 3.48 | 2.8 | 2.854 | 2.977 | 2.796 | 3.43 | 4.669 | 2.996 |
| **224771 _at** | neuron navigator 1 | NAV1 | 6.378 | 7.607 | 6.624 | 6.24 | 6.483 | 4.678 | 6.467 | 6.537 | 3.882 | 5.375 | 6.798 | 5.73 |
| **224772 at** | neuron navigator 1 | NAV1 | 9.184 | 10.073 | 8.059 | 7.897 | 8.937 | 8.214 | 8.963 | 9.472 | 7.529 | 7.742 | 9.418 | 8.412 |
| **224773 at** | neuron navigator 1 | NAV1 | 7.427 | 7.781 | 5.878 | 5.855 | 6.881 | 5.668 | 6.94 | 7.344 | 4.953 | 7.035 | 7.352 | 6.473 |
| **224774 s at** | neuron navigator 1 | NAV1 | 5.706 | 7.087 | 5.432 | 5.724 | 5.939 | 3.907 | 6.432 | 6.166 | 5.262 | 5.989 | 6.48 | 5.911 |
| **227145 at** | lysyl oxidase-like 4 | LOXL4 | 3.699 | 7.798 | 3.703 | 4.953 | 8.792 | 7.205 | 9.531 | 9.214 | 9.691 | 3.702 | 11.838 | 8.604 |
| **227584 at** | Neuron navigator 1 | NAV1 | 10.236 | 10.828 | 8.45 | 9.758 | 9.512 | 8.8 | 10.254 | 10.798 | 8.769 | 9.367 | 10.568 | 9.564 |
| **227812 _at** | tumor necrosis factor receptor superfamily, member 19 | TNFRS F19 | 9.962 | 9.478 | 9.706 | 8.939 | 11.568 | 11.39 | 9.998 | 10.948 | 9.083 | 10.541 | 12.176 | 10.575 |
| **229723 _at** | T-cell activation GTPase activating protein | TAGA P | 6.398 | 6.009 | 9.043 | 7.032 | 8.532 | 5.424 | 7.084 | 6.786 | 10.798 | 7.081 | 3.21 | 5.293 |
| **232024 _at** | GTPase, IMAP family member 2 | GIMAP 2 | 9.137 | 9.127 | 8.794 | 7.948 | 9.093 | 9.673 | 10.09 | 9.763 | 10.696 | 9.891 | 6.523 | 8.294 |
| **238544 _at** | Insulin-like growth factor 1 receptor | IGF1R | 7.023 | 6.644 | 6.255 | 4.359 | 6.899 | 5.442 | 6.574 | 4.359 | 5.264 | 3.677 | 9.187 | 5.445 |
| **242886 _at** | Chromosome 17 open reading frame 63 | C17orf 63 | 9.338 | 10.004 | 7.65 | 7.978 | 8.744 | 5.974 | 8.959 | 9.284 | 6.208 | 7.589 | 9.344 | 7.335 |
| **242986 at** | Neuron navigator 1 | NAV1 | 8.777 | 8.689 | 8.154 | 6.863 | 8.461 | 5.136 | 8.727 | 8.749 | 4.83 | 5.92 | 8.296 | 6.756 |
| **34210_ at** | CD52 antigen (CAMPATH-1 antigen) | CD52 | 9.102 | 9.324 | 10.652 | 10.24 | 10.103 | 7.423 | 9.382 | 9.344 | 11.944 | 10.812 | 2.854 | 9.448 |

**Table 14 Correlation of Patient Identity Numbers and Clinical Outcome after MAGE Immunotherapy**

| | **Progressive Disease** | **Stable Disease** | **Mixed Responders** | **Responders** | **Too Early** |
|---|---|---|---|---|---|
| **Patient ID No.** | 160 | 19 | 20 | 38 | 138 |
| | 30 | 77 | 67 | 136 | 91 |
| | 8 | 127 | 2 | 75 | 124 |
| | 109 | 65 | 11 | 120 | 150 |
| | 60 | 119 | 53 | 27 | 163 |
| | 55 | 23 | 59 | 118 | 122 |
| | 100 | 145 | 96 | | |
| | 37 | | 10 | | |
| | 52 | | 26/1 | | |
| | 81 | | | | |
| | 14 | | | | |
| | 66 | | | | |
| | 106 | | | | |
| | 133 | | | | |
| | 129 | | | | |
| | 74 | | | | |
| | 113 | | | | |
| | 36 | | | | |
| | 9 | | | | |
| | 155 | | | | |
| | 15 | | | | |
| | 5 | | | | |
| | 56 | | | | |
| | 16 | | | | |
| | 13 | | | | |
| | 98 | | | | |
| | 28 | | | | |
| | 108 | | | | |
| | 71 | | | | |
| | 145 | | | | |
| | 121 | | | | |
| | 80 | | | | |
| | 132 | | | | |
| | 3 | | | | |
| | 85 | | | | |
| | 135 | | | | |
| | 134 | | | | |
| | 82 | | | | |
| | 154 | | | | |
| | 39 | | | | |

Appendix A : R object of class nnet for neural network clinical outcome prediction of patients.

$n
[1] 489   2   1
$nunits
[1] 493
$nconn
  [1]   0   0   0   0   0   0   0   0   0   0   0   0   0   0   0   0   0   0
 [19]   0   0   0   0   0   0   0   0   0   0   0   0   0   0   0   0   0   0
 [37]   0   0   0   0   0   0   0   0   0   0   0   0   0   0   0   0   0   0
 [55]   0   0   0   0   0   0   0   0   0   0   0   0   0   0   0   0   0   0
 [73]   0   0   0   0   0   0   0   0   0   0   0   0   0   0   0   0   0   0
 [91]   0   0   0   0   0   0   0   0   0   0   0   0   0   0   0   0   0   0
[109]   0   0   0   0   0   0   0   0   0   0   0   0   0   0   0   0   0   0
[127]   0   0   0   0   0   0   0   0   0   0   0   0   0   0   0   0   0   0
[145]   0   0   0   0   0   0   0   0   0   0   0   0   0   0   0   0   0   0
[163]   0   0   0   0   0   0   0   0   0   0   0   0   0   0   0   0   0   0
[181]   0   0   0   0   0   0   0   0   0   0   0   0   0   0   0   0   0   0
[199]   0   0   0   0   0   0   0   0   0   0   0   0   0   0   0   0   0   0
[217]   0   0   0   0   0   0   0   0   0   0   0   0   0   0   0   0   0   0
[235]   0   0   0   0   0   0   0   0   0   0   0   0   0   0   0   0   0   0
[253]   0   0   0   0   0   0   0   0   0   0   0   0   0   0   0   0   0   0
[271]   0   0   0   0   0   0   0   0   0   0   0   0   0   0   0   0   0   0
[289]   0   0   0   0   0   0   0   0   0   0   0   0   0   0   0   0   0   0
[307]   0   0   0   0   0   0   0   0   0   0   0   0   0   0   0   0   0   0
[325]   0   0   0   0   0   0   0   0   0   0   0   0   0   0   0   0   0   0
[343]   0   0   0   0   0   0   0   0   0   0   0   0   0   0   0   0   0   0
[361]   0   0   0   0   0   0   0   0   0   0   0   0   0   0   0   0   0   0
[379]   0   0   0   0   0   0   0   0   0   0   0   0   0   0   0   0   0   0
[397]   0   0   0   0   0   0   0   0   0   0   0   0   0   0   0   0   0   0

[415]   0   0   0   0   0   0   0   0   0   0   0   0   0   0   0   0   0   0
[433]   0   0   0   0   0   0   0   0   0   0   0   0   0   0   0   0   0   0
[451]   0   0   0   0   0   0   0   0   0   0   0   0   0   0   0   0   0   0
[469]   0   0   0   0   0   0   0   0   0   0   0   0   0   0   0   0   0   0
[487]   0   0   0   0   0 490 980 983

$conn
  [1]   0   1   2   3   4   5   6   7   8   9  10  11  12  13  14  15  16  17
 [19]  18  19  20  21  22  23  24  25  26  27  28  29  30  31  32  33  34  35
 [37]  36  37  38  39  40  41  42  43  44  45  46  47  48  49  50  51  52  53
 [55]  54  55  56  57  58  59  60  61  62  63  64  65  66  67  68  69  70  71
 [73]  72  73  74  75  76  77  78  79  80  81  82  83  84  85  86  87  88  89
 [91]  90  91  92  93  94  95  96  97  98  99 100 101 102 103 104 105 106 107
[109] 108 109 110 111 112 113 114 115 116 117 118 119 120 121 122 123 124 125
[127] 126 127 128 129 130 131 132 133 134 135 136 137 138 139 140 141 142 143
[145] 144 145 146 147 148 149 150 151 152 153 154 155 156 157 158 159 160 161
[163] 162 163 164 165 166 167 168 169 170 171 172 173 174 175 176 177 178 179
[181] 180 181 182 183 184 185 186 187 188 189 190 191 192 193 194 195 196 197
[199] 198 199 200 201 202 203 204 205 206 207 208 209 210 211 212 213 214 215
[217] 216 217 218 219 220 221 222 223 224 225 226 227 228 229 230 231 232 233
[235] 234 235 236 237 238 239 240 241 242 243 244 245 246 247 248 249 250 251
[253] 252 253 254 255 256 257 258 259 260 261 262 263 264 265 266 267 268 269
[271] 270 271 272 273 274 275 276 277 278 279 280 281 282 283 284 285 286 287
[289] 288 289 290 291 292 293 294 295 296 297 298 299 300 301 302 303 304 305
[307] 306 307 308 309 310 311 312 313 314 315 316 317 318 319 320 321 322 323
[325] 324 325 326 327 328 329 330 331 332 333 334 335 336 337 338 339 340 341
[343] 342 343 344 345 346 347 348 349 350 351 352 353 354 355 356 357 358 359
[361] 360 361 362 363 364 365 366 367 368 369 370 371 372 373 374 375 376 377
[379] 378 379 380 381 382 383 384 385 386 387 388 389 390 391 392 393 394 395
[397] 396 397 398 399 400 401 402 403 404 405 406 407 408 409 410 411 412 413
[415] 414 415 416 417 418 419 420 421 422 423 424 425 426 427 428 429 430 431

[433] 432 433 434 435 436 437 438 439 440 441 442 443 444 445 446 447 448 449
[451] 450 451 452 453 454 455 456 457 458 459 460 461 462 463 464 465 466 467
[469] 468 469 470 471 472 473 474 475 476 477 478 479 480 481 482 483 484 485
[487] 486 487 488 489   0   1   2   3   4   5   6   7   8   9  10  11  12  13
[505]  14  15  16  17  18  19  20  21  22  23  24  25  26  27  28  29  30  31
[523]  32  33  34  35  36  37  38  39  40  41  42  43  44  45  46  47  48  49
[541]  50  51  52  53  54  55  56  57  58  59  60  61  62  63  64  65  66  67
[559]  68  69  70  71  72  73  74  75  76  77  78  79  80  81  82  83  84  85
[577]  86  87  88  89  90  91  92  93  94  95  96  97  98  99 100 101 102 103
[595] 104 105 106 107 108 109 110 111 112 113 114 115 116 117 118 119 120 121
[613] 122 123 124 125 126 127 128 129 130 131 132 133 134 135 136 137 138 139
[631] 140 141 142 143 144 145 146 147 148 149 150 151 152 153 154 155 156 157
[649] 158 159 160 161 162 163 164 165 166 167 168 169 170 171 172 173 174 175
[667] 176 177 178 179 180 181 182 183 184 185 186 187 188 189 190 191 192 193
[685] 194 195 196 197 198 199 200 201 202 203 204 205 206 207 208 209 210 211
[703] 212 213 214 215 216 217 218 219 220 221 222 223 224 225 226 227 228 229
[721] 230 231 232 233 234 235 236 237 238 239 240 241 242 243 244 245 246 247
[739] 248 249 250 251 252 253 254 255 256 257 258 259 260 261 262 263 264 265
[757] 266 267 268 269 270 271 272 273 274 275 276 277 278 279 280 281 282 283
[775] 284 285 286 287 288 289 290 291 292 293 294 295 296 297 298 299 300 301
[793] 302 303 304 305 306 307 308 309 310 311 312 313 314 315 316 317 318 319
[811] 320 321 322 323 324 325 326 327 328 329 330 331 332 333 334 335 336 337
[829] 338 339 340 341 342 343 344 345 346 347 348 349 350 351 352 353 354 355
[847] 356 357 358 359 360 361 362 363 364 365 366 367 368 369 370 371 372 373
[865] 374 375 376 377 378 379 380 381 382 383 384 385 386 387 388 389 390 391
[883] 392 393 394 395 396 397 398 399 400 401 402 403 404 405 406 407 408 409
[901] 410 411 412 413 414 415 416 417 418 419 420 421 422 423 424 425 426 427
[919] 428 429 430 431 432 433 434 435 436 437 438 439 440 441 442 443 444 445
[937] 446 447 448 449 450 451 452 453 454 455 456 457 458 459 460 461 462 463
[955] 464 465 466 467 468 469 470 471 472 473 474 475 476 477 478 479 480 481
[973] 482 483 484 485 486 487 488 489   0 490 491

$nsunits
[1] 493
$decay
[1] 0
$entropy
[1] TRUE
$softmax
[1] FALSE
$censored
[1] FALSE
$value
[1] 9.930496e-05

$wts
  [1] -7.822649e-01 -2.605320e-01 -3.445762e-01  1.193835e+00  7.980130e-01
  [6] -1.454517e-01 -3.852555e-01 -6.038561e-01  3.628980e-01  1.214178e-01
 [11]  6.302635e-01  1.163077e+00 -1.348927e+00  4.313002e-01 -3.439181e-01
 [16]  1.354545e+00 -5.330816e-01  9.006249e-04 -3.553966e-01 -7.807184e-01
 [21] -2.792791e-01 -3.170041e-01 -7.652292e-01  7.708915e-02 -8.561736e-01
 [26]  5.142947e-01 -2.484503e-01  9.726332e-01  1.778460e-01 -2.21F09e+00
 [31]  1.533794e-01 -9.241165e-01 -1.623889e+00 -1.673846e-01 -3.942404e-01
 [36]  4.839469e-02 -6.068823e-01 -7.231734e-01  2.776973e-01  9.486888e-01
 [41] -3.606490e-01 -9.814695e-02 -4.978396e-01  6.743036e-01 -6.715348e-01
 [46]  3.743046e-01  1.127027e+00  2.424162e-01 -2.472467e-01  5.103072e-01
 [51] -2.249262e-01  9.216276e-02 -7.811338e-02  6.543984e-02 -9.300402e-01
 [56] -1.723861e-01  8.450912e-01 -1.112637e-01 -1.340432e+00 -4.579130e-01
 [61] -2.000303e-01 -6.724820e-01  3.185803e-02 -8.755603e-01 -1.432133e-01
 [66]  1.734187e-01 -9.095931e-01 -5.779322e-01 -6.239654e-01  5.350169e-01
 [71]  2.660352e-02  7.575969e-01 -5.857182e-01  7.047356e-01  2.619782e-01
 [76]  7.365027e-02  1.085127e+00 -6.649458e-01  1.526824e-02  9.521713e-01
 [81]  2.953514e-01 -5.496499e-02 -7.349885e-01  4.766398e-01 -4.030412e-01

[86]  8.509923e-01 -2.896527e-02 -4.922158e-02 -1.075633e+00  2.852051e-01
 [91] -2.040135e-01  7.087471e-01 -7.626716e-01  4.932172e-02 -1.068290e+00
 [96]  4.525142e-01 -3.426858e-01 -5.564159e-01  4.206087e-01  3.702788e-01
[101] -8.482293e-01  2.445519e-01 -2.423795e-01 -7.502546e-01 -2.448160e-01
[106] -1.029814e-01 -4.534969e-01 -5.680071e-01 -2.278669e-01 -4.914741e-01
[111] -2.408882e-02 -1.122049e+00  1.637105e+00 -8.674111e-01 -3.993925e-01
[116]  6.766219e-01 -2.590604e-01 -1.128037e-01 -5.454970e-01  3.117549e-01
[121]  6.807225e-01  1.767702e+00  1.749150e+00  1.576723e+00  1.000372e+00
[126] -3.135498e-01 -3.461115e-01 -1.577623e-01 -3.688724e-03 -5.169024e-01
[131]  5.209675e-01  6.239938e-01 -6.562843e-01  3.318594e-01  1.073695e+00
[136]  3.852917e-01  7.445331e-01  6.147421e-01  3.486045e-02  2.328786e-01
[141]  5.865109e-01  8.789772e-01  2.735659e-01  2.179924e-01  6.546454e-01
[146] -7.425786e-02  2.332181e-01  2.026860e-02  1.920527e+00 -2.199749e-01
[151] -2.861057e-01 -6.113327e-01 -7.327236e-01  7.630948e-01 -6.725150e-01
[156]  8.195715e-01  1.354855e-01  7.557001e-01 -6.311882e-01  4.028033e-01
[161] -1.095942e-01  2.675720e-02  9.191105e-02 -5.412179e-01 -4.676564e-01
[166] -7.538073e-02 -8.129017e-01 -4.018241e-01 -2.996730e-01 -5.965576e-01
[171]  9.892726e-01  5.105776e-01  1.052146e+00 -2.957608e-01 -4.917327e-01
[176]  2.512329e-01 -1.016831e-01  1.099494e-02 -6.844977e-01  5.180294e-01
[181]  5.463099e-02 -5.062577e-01  5.281003e-01 -5.333424e-01  9.727653e-01
[186] -8.865765e-01  5.137428e-01 -1.480626e-01 -1.719326e-01  7.033034e-01
[191]  5.484301e-01  4.860635e-01 -4.432808e-01  6.295423e-01  6.296338e-01
[196]  3.257622e-01  5.230624e-01  3.651660e-01  7.889078e-01 -1.381056e-02
[201]  1.092450e-01  9.362120e-01 -9.812814e-02  5.535681e-01  1.927306e-01
[206]  4.132113e-01  2.483396e-01 -4.509187e-01 -5.662541e-01  6.316726e-01
[211] -1.081482e+00 -2.642237e-01  5.756106e-01  7.014808e-01  8.240274e-02
[216]  1.157531e-01  2.011787e-01  6.522527e-01  1.072262e-01  7.789481e-01
[221] -3.298577e-02 -2.177002e+00 -5.493206e-01 -3.840635e-01 -5.162878e-01
[226] -3.685617e-01  2.763670e-01 -4.768905e-01  2.604606e-01  3.470977e-01
[231]  1.882645e-01 -1.529664e-01 -1.141422e+00 -1.192761e+00  9.653415e-01
[236] -5.616165e-02  7.073548e-01  1.663138e-01 -1.724516e-02  5.466189e-01

[241]  8.215863e-02 -2.863819e-01 -7.682638e-01  6.907757e-01  2.953517e-01
[246] -8.597825e-02 -1.237122e-01 -1.141088e+00  7.686872e-01  1.278818e-01
[251]  1.363887e-01  2.012764e-01 -2.660433e-01 -2.356637e-01 -1.108908e+00
[256]  4.767741e-01 -3.796921e-01 -8.998346e-01  4.556543e-01 -1.754178e-01
[261]  1.102156e+00 -6.089921e-01  4.790648e-01 -1.378465e+00 -7.794537e-01
[266] -4.038758e-01  1.344525e+00 -4.043755e-01 -4.395272e-01  6.362795e-01
[271]  1.834397e-01  8.358359e-03 -3.857196e-01  7.268706e-01 -7.168161e-01
[276]  4.306795e-01  7.706614e-01  6.513772e-02 -2.245757e-01 -1.063308e+00
[281]  1.798277e-01 -1.752740e-01  2.278172e-01  2.965010e-01  5.452244e-01
[286]  1.543681e-01  1.661905e-01 -2.996078e-01  1.187087e-01  1.133628e-01
[291]  9.718658e-01  4.520898e-01  9.167133e-01 -3.433412e-01  3.579116e-01
[296]  6.023363e-03  2.989546e-02 -4.734359e-01  5.843214e-01 -3.799736e-01
[301]  5.842987e-01  1.525014e+00  1.118956e-01  4.651440e-01  5.483678e-01
[306] -3.747111e-02 -6.338612e-01  1.383897e-01  1.423111e-01  8.514850e-01
[311]  6.421267e-01  1.666450e-01 -2.689546e-01 -1.752850e-01  6.560046e-02
[316]  3.302397e-01  5.434177e-02  7.720145e-01 -2.185973e-01 -1.400281e-01
[321] -3.805316e-01  5.617241e-01 -7.327657e-01 -9.889267e-01 -1.877597e-01
[326] -4.126207e-01 -1.432952e+00 -2.641545e-01 -1.279892e-01  3.308045e-01
[331]  2.947318e-01  3.080860e-01  4.368988e-01 -7.796344e-01  6.472218e-01
[336]  3.216706e-01  3.220898e-01 -7.642426e-01  7.559186e-01 -7.224366e-02
[341]  8.057934e-01 -8.280142e-01  5.811389e-01  8.082749e-01 -1.739542e-01
[346]  1.878581e-01 -9.195490e-01  4.122718e-01  4.458733e-01 -5.164353e-01
[351] -2.987246e-01 -2.953580e-02 -1.027130e+00  1.380701e+00  1.015581e+00
[356]  7.511154e-01 -1.295990e-01 -1.289274e-01  2.716158e-01  4.902457e-01
[361] -8.143006e-01  2.466899e-01 -7.051082e-01 -1.421216e+00  7.574016e-01
[366]  1.062493e+00  3.120192e-01 -8.555016e-01 -2.710965e-01  5.270817e-01
[371] -2.772449e-02 -1.164226e+00 -5.875827e-01  4.967017e-01 -9.446788e-02
[376]  4.286175e-01  6.268223e-01 -3.435110e-01  1.025191e+00 -6.801600e-01
[381] -3.224292e-02 -3.193044e-01 -3.950073e-01 -6.430585e-01 -4.542940e-01
[386] -8.485502e-01  7.760473e-01  3.202145e-01  6.633763e-01 -5.801942e-01
[391]  1.306814e-01  1.299666e-01  3.046116e-01 -1.684422e-01 -6.295904e-01

[396]  1.565646e-01 -1.529663e-01 -1.148021e-02 -4.255510e-01 -4.986614e-01
[401] -2.557943e-01 -6.037794e-01 -1.125332e-01  7.177677e-01 -5.615997e-01
[406]  3.587169e-02  8.140494e-01 -6.603336e-01  5.388208e-02  2.157824e-01
[411]  8.007642e-01  1.088418e+00  7.859298e-01 -5.836605e-01 -3.394254e-01
[416] -7.943000e-01  5.089051e-01  2.668172e-01 -9.942403e-01 -6.977250e-01
[421]  5.418000e-02  9.294052e-01 -5.541002e-01 -2.663888e-01  1.582470e-01
[426] -9.034341e-01  1.796377e-01 -1.666522e+00 -6.447704e-01 -1.460522e-01
[431]  4.064656e-01  4.535141e-01 -8.493838e-02  4.404440e-01 -6.813737e-01
[436]  4.246934e-01  4.738893e-01  5.388799e-01  5.893005e-02  8.046329e-01
[441] -1.312353e-01  6.065425e-01  4.344549e-01 -1.432887e-01  6.266481e-01
[446]  2.491106e-01  5.628406e-01  7.156338e-01 -3.341744e-01 -7.083861e-01
[451] -3.816457e-01  7.333444e-01  8.614763e-01  1.158857e+00  4.593186e-01
[456] -1.059777e-01 -2.820036e-01  5.517071e-01 -3.863232e-01 -1.729483e+00
[461] -1.887161e-01  3.810323e-01  7.033161e-02  4.999075e-01  1.066108e-01
[466] -1.682395e-01  2.324000e-02 -1.079890e-01 -9.324107e-02 -1.789810e-02
[471] -1.151141e+00  1.302142e-01 -4.902189e-01 -9.327821e-03  2.853960e-02
[476]  3.681996e-01 -5.675777e-02 -1.121354e+00  9.055091e-01 -3.077752e-01
[481]  1.150414e-01  2.903319e-01 -1.978837e-01 -2.513171e-01 -6.335767e-01
[486]  1.883908e-01  7.978107e-01  5.193154e-01  4.594379e-01 -5.630128e-01
[491] -2.558691e-01  1.399098e-01  2.716841e-01 -1.029870e+00 -3.337377e-01
[496] -1.639137e-01  1.084488e+00 -2.009724e-01  2.988744e-02 -9.373952e-03
[501]  7.188562e-02  1.568801e-01  1.110666e+00 -6.429716e-01 -9.851071e-02
[506] -3.954610e-01  6.241091e-01  4.466790e-01 -3.418489e-01  1.129400e+00
[511]  7.873880e-02  3.685607e-01  1.312878e+00  3.921632e-01 -9.862630e-01
[516]  7.303611e-01  8.164471e-01 -7.706864e-01  1.701961e-01  9.772512e-01
[521]  3.345849e-01  5.069341e-01  9.739133e-01  1.608824e+00  1.145627e+00
[526]  5.074447e-01  2.034436e-01  1.470176e-01 -1.600484e-01 -2.150473e-01
[531]  1.377926e-01  6.227566e-01  4.094106e-01  4.259897e-01  1.865411e-01
[536] -4.727419e-01 -2.291728e-01  1.015483e+00  1.073489e+00 -2.136211e-01
[541] -8.307180e-01 -2.981026e-01  1.194316e+00 -1.318526e+00  1.129897e+00
[546] -6.792031e-02 -1.136511e-01  1.129797e+00  1.283430e+00  1.225369e+00

[551] -4.935841e-01 -5.978041e-01  6.994883e-01 -5.000395e-01 -5.917541e-02
[556]  8.643770e-01 -8.313903e-02 -7.645437e-01  4.585311e-01  2.760396e-01
[561] -9.048995e-01  2.270971e-01 -5.491731e-01  1.485625e-01  5.379235e-01
[566]  2.400513e-01 -1.398254e+00  2.149663e-01 -9.872386e-01 -2.349650e-01
[571] -1.134452e+00  1.084613e+00 -5.258544e-01 -2.309454e-01 -1.118639e+00
[576] -6.754324e-01 -1.105271e+00 -3.729548e-01  4.167448e-01 -6.998005e-02
[581] -6.067353e-01 -8.627442e-01 -2.747273e-01 -6.861895e-01 -8.294093e-01
[586] -7.157550e-01 -1.543888e+00  8.807905e-01  1.449671e-01  6.694883e-01
[591]  8.768986e-01 -3.354555e-01 -3.288934e-01 -6.713428e-02 -8.535772e-01
[596]  3.177712e-01  2.302577e-01  6.388607e-01 -8.468490e-01 -6.823332e-01
[601] -4.824133e-01 -1.152830e+00 -1.296166e-01  3.876039e-01 -1.264702e-01
[606] -6.234923e-01  7.556986e-01 -1.079503e+00  7.462365e-01 -7.156638e-01
[611] -6.597890e-01 -9.319472e-01  7.275191e-01 -1.206812e+00  1.462996e-01
[616]  3.163475e-01 -4.897914e-01  9.590103e-01  4.964603e-01 -2.036444e-01
[621]  5.536274e-01 -8.859709e-01  4.360544e-01 -1.174303e+00 -7.004479e-01
[626] -9.104478e-02  2.520933e-01  1.406452e-01 -5.811733e-01  1.005106e+00
[631] -8.395171e-01  2.836866e-01 -6.667517e-01  7.589487e-01  9.250607e-01
[636]  2.226882e-01 -9.617627e-01  7.764753e-01 -2.592188e-01 -3.870735e-01
[641]  3.652488e-01 -2.089410e+00 -8.639070e-01  6.041561e-02 -1.249116e-01
[646] -1.589578e-01 -9.288991e-01 -1.438925e-01 -1.584532e-01  4.032568e-01
[651] -4.542904e-01  4.196369e-01  3.925464e-02 -7.026149e-01 -4.894067e-01
[656]  1.472926e-01  5.125774e-01 -7.921361e-01 -6.512886e-01  6.268859e-01
[661] -8.082803e-01 -1.230153e-01 -1.259874e+00 -7.360178e-01 -4.800681e-01
[666] -9.807421e-02  3.650259e-01 -1.110299e+00 -6.785503e-02  8.200076e-02
[671] -7.377264e-01  9.628769e-01 -6.621068e-01 -1.064977e+00  1.635347e-01
[676]  3.436851e-01 -4.931667e-01  1.110185e+00 -5.205865e-01  6.614455e-01
[681] -2.341954e-01 -3.812965e-01 -3.107048e-01 -5.694353e-01  2.589693e-01
[686] -5.235213e-01  1.573854e-01 -4.416297e-01  2.271200e-01 -9.274423e-01
[691]  2.991815e-01 -2.355672e-01 -6.642878e-01 -2.300048e-01  3.206771e-01
[696] -2.170961e+00 -5.213502e-01 -1.148505e+00 -6.783888e-01 -4.686891e-04
[701]  7.177564e-01 -3.765768e-02 -1.415048e-02 -2.595464e-01  2.996030e-01

[706] -1.233281e-01 -3.676282e-01 -3.679718e-01 -9.210808e-01  4.052164e-01
[711]  4.585852e-02  1.312361e+00  5.048614e-01 -1.809305e+00 -1.721751e+00
[716] -1.669053e+00  1.401519e-01 -8.288580e-01  1.470533e-01 -1.315543e+00
[721]  6.430333e-02 -7.649410e-01  9.157078e-01  3.640731e-01 -1.306945e-02
[726]  9.203552e-02 -6.416202e-01 -6.434372e-01 -7.309035e-01  6.760630e-01
[731]  5.793554e-01  3.266417e-01 -5.467879e-01  1.875603e-02  6.823768e-01
[736]  6.605321e-01 -2.192790e-01  6.458484e-01  1.256230e-01 -3.446176e-01
[741] -7.349580e-01 -3.794139e-01 -5.742886e-01 -7.069700e-01  2.605697e-01
[746] -2.759157e+00 -3.153237e-01  6.614816e-01 -1.399808e+00  2.537040e-01
[751] -2.566458e-01 -9.300803e-01 -3.258002e-01  1.847266e+00 -8.318504e-01
[756]  8.032399e-01 -7.391342e-02 -1.458280e-01  1.475066e-01 -2.016509e-01
[761] -1.116992e+00  2.565951e-01 -1.417568e+00 -4.075312e-01 -4.624426e-01
[766] -3.892320e-01  9.233114e-02  8.039994e-01  1.708351e-01  3.140843e-01
[771]  1.558756e-01  4.926293e-01 -1.257969e+00 -7.845624e-01  1.556651e-01
[776] -1.152795e+00 -1.463677e+00 -8.108185e-01 -8.317870e-02 -1.456545e-01
[781]  6.954507e-01 -8.047871e-01 -8.898140e-01 -1.254371e+00 -6.563698e-01
[786]  3.967049e-01  9.335104e-01  1.299258e-02 -2.453240e-01 -5.841076e-01
[791] -1.658272e-01 -1.326827e+00  8.844022e-01 -6.606743e-01  4.137678e-01
[796]  7.360620e-02 -1.241175e+00  1.066117e+00 -1.086576e-01  7.812635e-01
[801] -5.793264e-01  3.664636e-02  5.461605e-01 -1.468411e-01 -1.200992e+00
[806]  7.664790e-02 -9.648972e-01  8.464096e-02  8.781079e-01 -1.329100e-01
[811] -3.140853e-01 -3.603208e-01  5.330240e-01  9.776989e-01  1.754023e-01
[816] -3.932540e-02  1.381857e+00  4.967358e-01  2.445305e-01  2.033007e-02
[821] -1.660898e+00 -3.826148e-01 -1.350548e-01  2.055229e-01 -4.826541e-01
[826] -3.437897e-02 -1.131522e+00 -2.924096e-01  1.078663e+00 -3.618922e-01
[831] -3.795694e-01  7.062471e-01  1.818627e-01  3.360420e-02 -5.509515e-01
[836]  2.820342e-01 -5.169129e-01 -7.298595e-01  6.831575e-02 -4.718518e-01
[841] -1.177961e-01  3.261549e-01  9.243626e-01 -9.539287e-01 -8.574709e-01
[846]  8.404623e-01 -1.477904e+00  7.161732e-01 -7.213226e-02 -1.072695e+00
[851]  1.099245e+00  3.907082e-02  4.707593e-01 -1.037259e+00 -6.337726e-01
[856] -6.489972e-01 -8.105587e-01 -1.132535e-01  5.618367e-01  1.120736e+00

[861] -1.589420e-01 -4.736779e-01 -3.492589e-01 -5.292510e-01  7.552729e-02
[866]  6.245302e-01 -1.599877e+00  1.961020e+00 -5.986310e-01  7.907619e-01
[871]  9.258619e-01  4.602164e-01 -4.456243e-01  2.256970e-01  1.180578e+00
[876] -2.202471e-01 -1.595068e+00 -7.650998e-01  1.068712e-01  3.886734e-03
[881]  3.158598e-02 -7.733971e-01  1.905452e-01 -6.009153e-01  2.211837e-01
[886] -5.134809e-02 -8.393086e-01 -1.087550e+00 -3.173469e-01 -1.101492e+00
[891]  4.972408e-01  1.799967e-01 -2.447001e-01 -8.741257e-01  2.992654e-01
[896] -9.245988e-01  9.044085e-02  9.133224e-01  7.446155e-01 -7.193918e-01
[901]  9.205514e-02 -1.402019e+00  4.976776e-01 -6.959568e-01  4.945441e-01
[906]  1.286614e-01 -8.543565e-01 -3.603456e-02  4.664471e-01 -7.580873e-03
[911] -3.060679e-01 -6.700308e-01  4.941296e-04 -8.978372e-01 -6.954622e-01
[916] -3.736479e-01  1.754756e-02  1.567201e+00  8.573695e-01  3.966870e-01
[921] -2.215584e-02 -1.169949e-01  4.257213e-01 -2.346597e-01  7.421774e-01
[926] -5.966420e-01  5.751260e-02 -2.472787e-01  2.334021e-01  1.300946e+00
[931]  2.341594e-01  1.049020e+00 -3.269379e-01 -1.269805e-01 -6.880527e-01
[936] -3.229197e-01  7.076868e-01 -3.403887e-01  1.676770e-01 -7.620830e-02
[941]  3.558033e-01  1.459493e-01 -6.244181e-01  1.770295e-01 -1.216517e-01
[946]  7.426668e-01 -1.385593e-01 -6.277839e-01 -1.879113e-01  9.488544e-02
[951]  2.351063e-01  1.073347e+00 -4.874189e-01  3.801474e-01 -6.690750e-01
[956] -6.047573e-01 -3.134005e-01 -6.000130e-01 -3.197655e-01  6.631834e-01
[961] -6.356578e-01 -5.904780e-01  1.097918e+00  6.007569e-01 -2.505695e+00
[966] -5.697962e-01  5.674896e-01  4.245279e-01 -1.060281e-01 -1.130024e-01
[971]  1.595858e+00  1.516974e-01 -3.702457e-02 -1.700967e-01 -3.641777e-01
[976] -1.604025e-01 -1.447675e+00 -1.008405e+00  3.365701e-01  2.348416e-01
[981] -1.189562e+01  2.427842e+01 -2.275821e+01

$fitted.values
         [,1]

1 9.999958e-01
2 0.000000e+00
3 0.000000e+00
4 0.000000e+00
5 0.000000e+00
6 9.999958e-01
7 9.999958e-01
8 9.999958e-01
9 9.999958e-01
10 9.999958e-01
11 9.999958e-01
12 0.000000e+00
13 9.999958e-01
14 0.000000e+00
15 0.000000e+00
16 0.000000e+00
17 9.999958e-01
18 6.820198e-06
19 6.825022e-06
20 3.118923e-05
21 0.000000e+00
22 0.000000e+00
23 9.999958e-01
24 0.000000e+00
25 0.000000e+00
26 9.999958e-01
27 0.000000e+00
28 9.999958e-01
29 9.999958e-01
30 0.000000e+00
31 0.000000e+00

$residuals
              [,1]
1    4.189989e-06
2    0.000000e+00
3    0.000000e+00
4    0.000000e+00
5    0.000000e+00
6    4.189989e-06
7    4.189989e-06
8    4.189989e-06
9    4.189989e-06
10   4.189989e-06
11   4.189989e-06
12   0.000000e+00
13   4.189989e-06
14   0.000000e+00
15   0.000000e+00
16   0.000000e+00
17   4.189989e-06
18  -6.820198e-06
19  -6.825022e-06
20  -3.118923e-05
21   0.000000e+00
22   0.000000e+00
23   4.189989e-06
24   0.000000e+00
25   0.000000e+00
26   4.189989e-06
27   0.000000e+00
28   4.189989e-06
29   4.189989e-06
30   0.000000e+00
31   0.000000e+00

$lev
[1] "NR" "R"


$call
nnet.formula(formula = f ~ .,
data = data, MaxNWts = 10000,
maxit = 250,
    size = 2, skip = FALSE,
rang = 0.7, decay = 0, Hess =
FALSE,
    trace = TRUE, abstol = 1e-
04, reltol = 1e-08, metric =
"euclidean",
    subset = training)

Appendix B: R object of class lda for linear discriminant analysis clinical outcome prediction of patients.
$prior
NR R
0.5862069 0.4137931
$counts
NR R
17 12

$scaling
LD1
X1405_i_at | 5.944209e-03
X1552497_a_at | -1.489159e-03
X1552612_at | 1.928010e-03
X1552613_s_at | -3.948620e-03
X1553102_a_at | 2.035336e-02
X1553132_a_at | -1.048751e-02
X1553313_s_at | -3.034153e-02
X1553906_s_at | -1.834813e-02
X1554240_a_at | 1.365655e-02
X1554966_a_at | 3.759930e-03
X1555229_a_at | 8.797787e-02
X1555630_a_at | -2.791031e-02
X1555756_a_at | 1.654466e-02
X1555759_a_at | 1.036814e-03
X1555812_a_at | 1.278876e-02
X1555852_at | -3.512472e-03
X1556185_a_at | 3.025315e-02
X1556579_s_at | 4.497852e-02
X1557116_at | 4.611121e-03
X1557222_at | 2.410949e-02
X1558034_s_at | 2.790575e-02
X1558290_a_at | -1.523617e-02
X1558586_at | -4.151460e-02
X1558972_s_at | 5.561221e-04
X1559263_s_at | 7.463183e-03
X1559425_at | 1.864440e-03
X1559584_a_at | -5.820026e-03
X1562031_at | 9.246741e-03
X1563461_at | -2.064194e-02
X1563473_at | 4.011858e-03
X1565602_at | -4.884858e-02
X1568736_s_at | -2.773483e-02
X1568822_at | 2.099681e-03
X1569942_at | 6.739294e-03
X200612_s_at | -6.365853e-02
X200904_at | 6.387716e-03
X200905_x_at | 5.294827e-03
X200953_s_at | -8.671791e-03
X201010_s_at | 4.474642e-03

COLUMN 2
X213007_at | -3.352605e-02
X213008_at | -4.189798e-03
X213068_at | 2.658290e-02
X213095_x_at | 1.421934e-02
X213193_x_at | 2.914348e-03
X213475_s_at | -4.914118e-04
X213537_at | -8.357035e-03
X213539_at | 7.487953e-03
X213560_at | -4.020759e-03
X213566_at | 5.845149e-03
X213603_s_at | -5.088264e-03
X213618_at | 1.305106e-02
X213652_at | 8.548287e-03
X213819_s_at | 8.196495e-03
X213831_at | 1.631173e-02
X213888_s_at | 8.134177e-05
X213975_s_at | -2.095655e-02
X214023_x_at | -2.237865e-02
X214038_at | 2.389479e-02
X214181_x_at | -8.976012e-03
X214450_at | 1.788553e-02
X214470_at | 2.213217e-02
X214617_at | 1.563590e-02
X214669_x_at | 1.972879e-03
X214677_x_at | 3.809399e-03
X214719_at | 1.439531e-03
X215051_x_at | 1.072135e-02
X215121_x_at | 4.118073e-03
X215193_x_at | 2.105556e-04
X215223_s_at | 1.530709e-02
X215379_x_at | 7.064224e-03
X215561_s_at | -2.385977e-02
X215806_x_at | 1.767858e-02
X216155_at | -3.705145e-02
X216191_s_at | 4.747897e-02
X216194_s_at | -4.006836e-02
X216714_at | 2.405266e-02
X216841_s_at | 8.716417e-03
X216920_s_at | 3.911396e-03
X217028_at | 5.128469e-03
X217138_x_at | 9.357220e-03

X201137_s_at | -1.506315e-02 | X217143_s_at | 7.546655e-03
X201220_x_at | -2.667959e-02 | X217147_s_at | -1.958024e-02
X201236_s_at | 1.404961e-02 | X217478_s_at | -1.327997e-02
X201425_at | 1.554231e-02 | X217525_at | -1.717356e-02
X201474_s_at | -6.107372e-03 | X217629_at | 7.854937e-03
X201487_at | 2.472156e-02 | X217767_at | -5.238491e-03
X201497_x_at | 7.254710e-04 | X217995_at | -6.809023e-03
X201502_s_at | 5.696526e-03 | X218002_s_at | 9.869321e-03
X201531_at | 1.148431e-02 | X218035_s_at | 1.245016e-03
X201566_x_at | 1.794849e-02 | X218145_at | -3.291834e-02
X201720_s_at | 1.570380e-03 | X218170_at | -7.323517e-03
X201721_s_at | -9.613928e-03 | X218322_s_at | -8.250237e-03
X201804_x_at | -2.053567e-02 | X218499_at | 5.140903e-04
X201859_at | -1.376084e-04 | X218736_s_at | 2.138520e-02
X201939_at | -3.369784e-02 | X218739_at | 2.714745e-03
X202207_at | -1.196899e-02 | X218764_at | -9.158772e-03
X202255_s_at | -4.340999e-02 | X218802_at | 5.152377e-02
X202269_x_at | -1.833048e-02 | X218805_at | -2.574003e-03
X202270_at | -8.686638e-03 | X218854_at | 3.989147e-03
X202368_s_at | -2.233262e-02 | X218899_s_at | -3.411423e-02
X202369_s_at | -1.278489e-02 | X218950_at | -4.336176e-02
X202391_at | -5.586253e-03 | X219054_at | 4.975575e-03
X202510_s_at | 1.200980e-02 | X219093_at | 2.082412e-02
X202625_at | 1.449689e-02 | X219213_at | -4.341432e-03
X202643_s_at | 2.057522e-02 | X219243_at | 2.233332e-03
X202644_s_at | 2.044322e-02 | X219368_at | -1.134073e-03
X202687_s_at | 1.939420e-03 | X219440_at | 2.580567e-02
X202688_at | 8.690145e-04 | X219454_at | 3.065280e-02
X202948_at | -1.822099e-02 | X219505_at | 5.933108e-03
X202957_at | -1.143226e-02 | X219519_s_at | 2.660637e-02
X203413_at | -3.803468e-02 | X219525_at | 4.320312e-03
X203416_at | -1.189864e-02 | X219528_s_at | 1.066797e-02
X203471_s_at | 1.553784e-02 | X219631_at | -2.616546e-02
X203508_at | 9.486448e-03 | X219666_at | 1.182519e-02
X203523_at | 5.575011e-03 | X219681_s_at | 8.705075e-03
X203547_at | 9.218142e-03 | X219710_at | -1.046131e-02
X203665_at | 3.487226e-02 | X219737_s_at | -6.473357e-02
X203741_s_at | 2.161880e-02 | X219777_at | -6.023843e-03
X203761_at | 8.568780e-03 | X219789_at | 8.550214e-03
X203812_at | 4.104549e-02 | X219926_at | -8.456673e-03
X203868_s_at | -3.218808e-02 | X219938_s_at | 1.596546e-02
X203915_at | 1.356669e-02 | X220005_at | 6.859902e-03
X203932_at | -1.767768e-03 | X220066_at | 7.525339e-03
X204057_at | 1.199848e-02 | X220330_s_at | 3.751801e-03
X204070_at | -8.013273e-04 | X220485_s_at | 2.962480e-03
X204116_at | 6.190984e-03 | X221081_s_at | -4.394394e-03
X204118_at | 1.625516e-02 | X221087_s_at | 8.161216e-03
X204135_at | 4.612466e-03 | X221477_s_at | 1.240372e-02
X204204_at | 3.177067e-02 | X221651_x_at | 1.118009e-02
X204220_at | -6.573462e-03 | X221671_x_at | 5.247343e-03
X204222_s_at | 8.871953e-03 | X221698_s_at | -1.058842e-02
X204224_s_at | -4.997460e-03 | X221756_at | -1.209965e-02

X204233_s_at -1.586361e-02 | X221760_at -1.764419e-02
X204236_at 5.467904e-03 | X222108_at 4.026916e-02
X204249_s_at -1.313280e-02 | X222142_at -3.508958e-02
X204411_at 6.579659e-03 | X222484_s_at -8.096999e-03
X204438_at 2.031749e-02 | X222496_s_at -4.133924e-03
X204502_at 6.392044e-03 | X222592_s_at 1.511240e-02
X204512_at -1.534429e-05 | X222725_s_at 2.808005e-02
X204533_at 2.287973e-02 | X222780_s_at -2.679162e-02
X204613_at 5.458783e-03 | X222838_at 4.550716e-03
X204628_s_at -3.028829e-02 | X222895_s_at -2.841100e-02
X204642_at -3.867430e-03 | X223044_at 5.255602e-02
X204655_at -5.765680e-03 | X223058_at 9.381819e-03
X204661_at 2.636612e-02 | X223059_s_at 8.539393e-03
X204670_x_at -1.182992e-02 | X223168_at -4.973917e-03
X204687_at 3.953856e-02 | X223235_s_at 4.953002e-04
X204724_s_at -1.982230e-02 | X223280_x_at 6.016285e-03
X204774_at -1.596679e-02 | X223322_at -1.188607e-02
X204778_x_at -2.436425e-02 | X223361_at -5.418802e-03
X204834_at 4.664052e-02 | X223395_at 1.411881e-02
X204846_at 2.224653e-02 | X223484_at 1.743558e-02
X204894_s_at 4.442071e-03 | X223809_at 2.119400e-02
X204897_at -2.250415e-02 | X223827_at -1.852331e-02
X204912_at 2.155492e-03 | X223922_x_at 8.293530e-03
X204923_at -9.860185e-03 | X223924_at -2.212120e-02
X205027_s_at 3.406286e-03 | X223952_x_at 3.606709e-02
X205039_s_at 1.453129e-03 | X224356_x_at 4.977366e-03
X205081_at 2.008363e-02 | X224358_s_at -9.225797e-04
X205159_at 4.374158e-03 | X224451_x_at -4.781080e-03
X205225_at -8.687131e-03 | X224516_s_at -1.494258e-02
X205226_at -4.151219e-02 | X224710_at -2.279989e-02
X205251_at 2.071759e-02 | X224771_at -1.243246e-02
X205285_s_at 9.469306e-03 | X224772_at -4.059490e-02
X205392_s_at 1.236199e-02 | X224773_at -7.626004e-03
X205403_at 1.344662e-02 | X224774_s_at -1.743111e-02
X205419_at -6.256056e-03 | X224795_x_at 5.385788e-03
X205421_at 1.955389e-02 | X224859_at -1.540871e-02
X205440_s_at 2.903482e-02 | X224896_s_at -4.228341e-03
X205484_at -1.026029e-02 | X225502_at 3.460051e-03
X205488_at 2.109358e-02 | X225802_at -2.887415e-02
X205559_s_at -2.837597e-02 | X225895_at 4.741893e-03
X205569_at 1.407987e-02 | X226043_at 1.277772e-02
X205624_at 2.531892e-02 | X226068_at 5.224876e-03
X205685_at 4.266186e-02 | X226117_at 2.490399e-02
X205696_s_at -7.143024e-03 | X226218_at -1.341873e-02
X205758_at 9.290501e-03 | X226219_at 4.175131e-03
X205786_s_at -7.188176e-03 | X226303_at 4.358878e-02
X205831_at 8.970703e-03 | X226382_at 6.264514e-04
X205841_at -1.024458e-02 | X226459_at 4.497883e-03
X205844_at 2.406034e-02 | X226625_at 4.213244e-02
X205890_s_at 1.526584e-02 | X226659_at -4.064464e-03
X205987_at 5.099598e-03 | X226697_at -5.986636e-02
X206082_at -2.365415e-02 | X226818_at 2.581249e-02

X206099_at | 4.205302e-02 | X226841_at | 1.092228e-02
X206118_at | 1.153657e-02 | X226865_at | -2.335927e-02
X206134_at | -4.474477e-03 | X227035_x_at | 8.655280e-03
X206170_at | -1.785743e-02 | X227231_at | -1.692593e-02
X206204_at | 1.969866e-02 | X227253_at | 1.018350e-02
X206295_at | 1.528790e-02 | X227265_at | 1.360205e-02
X206385_s_at | -1.655446e-02 | X227346_at | -1.295157e-02
X206407_s_at | 3.592881e-02 | X227361_at | -5.811243e-03
X206545_at | 1.272019e-02 | X227458_at | 1.893847e-02
X206571_s_at | -3.009919e-02 | X227584_at | -2.425646e-02
X206637_at | 7.423640e-03 | X227609_at | 3.029790e-02
X206666_at | 1.204182e-02 | X227640_s_at | -5.262288e-03
X206687_s_at | -1.762981e-02 | X227780_s_at | 1.333635e-02
X206715_at | 1.116688e-02 | X227791_at | 1.583168e-02
X206804_at | 1.234660e-02 | X227983_at | -1.027572e-02
X206898_at | -3.907050e-02 | X227995_at | -1.414606e-02
X206978_at | -4.979548e-03 | X228054_at | -1.428632e-03
X207076_s_at | 1.863378e-02 | X228071_at | -8.103128e-03
X207238_s_at | -5.780355e-03 | X228094_at | 9.305227e-03
X207277_at | 2.125419e-02 | X228153_at | 1.840560e-02
X207458_at | 2.748393e-02 | X228339_at | 1.115556e-02
X207540_s_at | -1.283397e-02 | X228362_s_at | 1.505306e-02
X207574_s_at | -2.431727e-03 | X228372_at | -1.233323e-02
X207651_at | 2.066787e-02 | X228376_at | 3.005129e-03
X207655_s_at | 5.405012e-04 | X228427_at | 1.441232e-02
X207677_s_at | 2.230816e-02 | X228532_at | -1.984789e-02
X207843_x_at | 3.315503e-02 | X228552_s_at | 1.488213e-02
X207861_at | 4.377097e-02 | X228563_at | 5.478160e-03
X207977_s_at | 1.145723e-02 | X228660_x_at | -3.321971e-03
X207992_s_at | 6.265461e-03 | X228776_at | 1.735059e-02
X208296_x_at | -2.224775e-03 | X228812_at | 2.966052e-02
X208306_x_at | -5.132684e-03 | X228858_at | 8.798795e-03
X208335_s_at | 3.329359e-03 | X228869_at | 1.576960e-02
X208450_at | 3.811124e-02 | X228908_s_at | 1.715903e-02
X208747_s_at | -7.912263e-03 | X228964_at | 1.578864e-02
X208885_at | -1.288364e-02 | X229127_at | 2.437139e-02
X208894_at | 7.896947e-03 | X229163_at | -1.306446e-03
X208981_at | -1.015968e-02 | X229367_s_at | 8.285081e-03
X208983_s_at | -3.056594e-02 | X229390_at | 9.455414e-03
X209083_at | 4.483265e-04 | X229391_s_at | 2.152998e-02
X209138_x_at | 7.183442e-03 | X229543_at | 1.337527e-02
X209193_at | 1.740572e-02 | X229625_at | 1.390034e-02
X209195_s_at | -2.237887e-02 | X229723_at | 1.284379e-02
X209202_s_at | 8.930806e-03 | X230233_at | 6.517225e-03
X209312_x_at | -5.814581e-03 | X230391_at | -1.165179e-02
X209480_at | 1.848455e-02 | X230538_at | -4.922326e-02
X209542_x_at | 4.971766e-03 | X230728_at | -3.921071e-02
X209603_at | 3.004576e-02 | X231032_at | 1.220460e-02
X209606_at | 5.064024e-03 | X231262_at | 8.401904e-03
X209612_s_at | 2.595736e-02 | X231577_s_at | -4.352510e-03
X209613_s_at | 1.417618e-02 | X231882_at | -1.597680e-02
X209670_at | -1.636542e-02 | X231929_at | -7.190517e-03

X209671_x_at   2.518135e-03
X209685_s_at  -9.113030e-03
X209687_at    -1.842487e-02
X209710_at     5.230642e-03
X209734_at    -5.196811e-03
X209774_x_at   2.951233e-02
X209795_at    -6.624457e-03
X209813_x_at   2.371992e-02
X209899_s_at   7.998200e-04
X209924_at     3.375216e-02
X209949_at     3.781916e-02
X209970_x_at   2.564418e-02
X210038_at     6.929319e-03
X210072_at     1.293525e-02
X210251_s_at   1.756383e-02
X210260_s_at  -1.082042e-03
X210319_x_at  -1.726655e-02
X210375_at     2.483769e-03
X210554_s_at  -2.077789e-02
X210835_s_at  -7.455480e-03
X210915_x_at   3.172003e-03
X210972_x_at   6.080266e-03
X210982_s_at   9.635875e-03
X211066_x_at  -1.164103e-02
X211144_x_at   4.992137e-02
X211200_s_at  -4.031722e-02
X211339_s_at  -8.002311e-03
X211366_x_at   3.593000e-02
X211367_s_at   6.231728e-02
X211368_s_at   2.045696e-02
X211654_x_at   2.874123e-02
X211742_s_at  -1.282275e-02
X211796_s_at   6.744624e-03
X211902_x_at   1.350420e-03
X211990_at    -1.552756e-02
X211991_s_at  -2.759634e-03
X212067_s_at  -9.461685e-03
X212233_at     8.400376e-03
X212538_at     7.226797e-03
X212587_s_at  -5.979921e-03
X212588_at    -7.824565e-04
X212592_at     3.516261e-03
X212657_s_at   2.978857e-02
X212671_s_at  -2.602076e-02
X212713_at     3.233683e-02
X212763_at    -3.275778e-03
X212886_at     3.683799e-03
X212977_at     9.877644e-03
X212998_x_at   5.090511e-03
X212999_x_at   3.695828e-02
Continued in column 2
$lev
X232001_at    -2.527809e-02
X232024_at     1.562502e-02
X232234_at     1.979601e-02
X232311_at    -5.811767e-04
X232313_at     3.438576e-02
X232476_at    -1.156007e-02
X232543_x_at  -6.890048e-03
X232617_at     2.120826e-03
X232746_at    -1.032038e-02
X232843_s_at  -1.582714e-03
X233123_at     3.841746e-02
X233562_at    -6.149035e-02
X233955_x_at  -2.411061e-02
X235175_at    -8.822903e-03
X235238_at    -4.455537e-02
X235306_at    -1.163963e-02
X235391_at     9.692727e-03
X235421_at    -7.303012e-04
X235639_at    -3.367098e-02
X235688_s_at  -1.717805e-03
X235804_at    -1.564079e-03
X235831_at     2.675921e-03
X236203_at    -8.187551e-03
X236280_at     6.197497e-03
X236295_s_at   4.606321e-03
X236583_at     2.311931e-03
X236908_at    -2.109408e-02
X238439_at     3.286334e-02
X238488_at     2.932061e-02
X238544_at     1.183049e-03
X239196_at     4.987304e-02
X239237_at     3.319884e-02
X239744_at    -1.276710e-02
X241671_x_at  -6.586756e-03
X241701_at    -8.761918e-03
X242458_at    -1.194306e-02
X242546_at     4.355118e-03
X242874_at     5.607680e-03
X242881_x_at  -6.062508e-03
X242986_at     2.749620e-03
X243099_at     1.186699e-02
X244023_at    -1.018460e-02
X244061_at     1.229564e-03
X32128_at      2.935196e-02
X34210_at      3.776762e-03
X38149_at      4.937711e-04
X64064_at     -3.336735e-03

[1] "NR" "R"
$svd
[1] 7.995862
$N
[1] 29
$call
lda(formula = f ~ ., data = datatr)

Appendix C: R object of class naiveBayes for naïve Bayes clinical outcome prediction of patients.

$apriori
Y
NR  R
17 12
tablestables$X1405_i_at
    X1405_i_at
Y            [,1]       [,2]
  NR -0.1639258 0.5851902
  R   0.6748740 0.6631486
tables$X1552497_a_at
    X1552497_a_at
Y            [,1]       [,2]
  NR -0.3029996 0.6800071
  R   0.6607734 0.8223674
tables$X1552612_at
    X1552612_at
Y            [,1]       [,2]
  NR -0.2523340 0.5485271
  R   0.4450904 0.5434438
tables$X1552613_s_at
    X1552613_s_at
Y            [,1]       [,2]
  NR -0.2165343 0.5894093
  R   0.4800677 0.4432806
tables$X1553102_a_at
    X1553102_a_at
Y            [,1]       [,2]
  NR -0.2474064 0.5834113
  R   0.7071397 0.7121750
tables$X1553132_a_at
    X1553132_a_at
Y            [,1]       [,2]
  NR -0.1377405 0.3946696
  R   0.4961300 0.4509575
tables$X1553313_s_at
    X1553313_s_at
Y            [,1]       [,2]
  NR  0.2393526 0.6100254
  R  -0.3838373 0.3940848
tables$X1553906_s_at
    X1553906_s_at

Column 2
Y            [,1]       [,2]
  NR -0.3674984 0.5888660
  R   0.3219144 0.5336377
tables$X213539_at
    X213539_at
Y            [,1]       [,2]
  NR -0.3813796 0.5040301
  R   0.3415072 0.2577433
tables$X213560_at
    X213560_at
Y            [,1]       [,2]
  NR -0.5055000 0.7230271
  R   0.2766383 0.2303920
tables$X213566_at
    X213566_at
Y            [,1]       [,2]
  NR -0.2311022 0.5691307
  R   0.3719339 0.4376776
tables$X213603_s_at
    X213603_s_at
Y            [,1]       [,2]
  NR -0.3061774 0.6126919
  R   0.3508772 0.5262723
tables$X213618_at
    X213618_at
Y            [,1]      [,2]
  NR -0.3695683 0.681454
  R   0.4212985 0.554157
tables$X213652_at
    X213652_at
Y            [,1]       [,2]
  NR -0.2586966 0.4605599
  R   0.3270410 0.8177604
tables$X213819_s_at
    X213819_s_at
Y             [,1]       [,2]
  NR  0.00122895 0.3972336
  R  -0.64986825 0.9085849
tables$X213831_at
    X213831_at

Y            [,1]      [,2]
  NR -0.3915645 0.6474232
  R   0.2882052 0.3076821
tables$X1554240_a_at
    X1554240_a_at
Y            [,1]      [,2]
  NR 0.01780307 0.4865912
  R  0.88377535 0.8029798
tables$X1554966_a_at
    X1554966_a_at
Y            [,1]      [,2]
  NR -0.1357018 0.3678133
  R   0.6662706 0.6833596
tables$X1555229_a_at
    X1555229_a_at
Y            [,1]      [,2]
  NR -0.2263265 0.3882668
  R   0.5467680 0.4361597
tables$X1555630_a_at
    X1555630_a_at
Y            [,1]      [,2]
  NR  0.2005512 0.3578418
  R  -0.6088957 0.7683101
tables$X1555756_a_at
    X1555756_a_at
Y            [,1]      [,2]
  NR -0.2186897 0.5247142
  R   0.4061700 0.5187056
tables$X1555759_a_at
    X1555759_a_at
Y            [,1]      [,2]
  NR 0.08037061 0.5249017
  R  0.85078348 0.7360755
tables$X1555812_a_at
    X1555812_a_at
Y            [,1]      [,2]
  NR -0.4493812 0.8437685
  R   0.4087372 0.6145611
tables$X1555852_at
    X1555852_at
Y            [,1]      [,2]
  NR -0.3431720 0.6409197
  R   0.3060830 0.4160286
tables$X1556185_a_at
    X1556185_a_at
Y            [,1]      [,2]
  NR -0.3542416 0.4573215
  R   0.3876607 0.8113095
tables$X1556579_s_at
    X1556579_s_at
Y             [,1]      [,2]
  NR -0.08046679 0.2880584

Y            [,1]      [,2]
  NR -0.3533186 0.4215363
  R   0.1563120 0.3886529
tables$X213888_s_at
    X213888_s_at
Y            [,1]      [,2]
  NR -0.2086168 0.5349552
  R   0.4303498 0.4269851
tables$X213975_s_at
    X213975_s_at
Y            [,1]      [,2]
  NR -0.4990363 0.6803409
  R   0.1878550 0.2710180
tables$X214023_x_at
    X214023_x_at
Y            [,1]      [,2]
  NR  0.1848739 0.7174998
  R  -0.4325008 0.4810479
tables$X214038_at
    X214038_at
Y            [,1]      [,2]
  NR -0.3255701 0.6706608
  R   0.3599967 0.6048520
tables$X214181_x_at
    X214181_x_at
Y            [,1]      [,2]
  NR -0.3415893 0.6522400
  R   0.2853699 0.2763122
tables$X214450_at
    X214450_at
Y            [,1]      [,2]
  NR -0.1562932 0.2807857
  R   0.8434281 0.8613678
tables$X214470_at
    X214470_at
Y            [,1]      [,2]
  NR -0.2839688 0.4885156
  R   0.4212807 0.3750679
tables$X214617_at
    X214617_at
Y            [,1]      [,2]
  NR -0.3487562 0.4518071
  R   0.2125207 0.4242197
tables$X214669_x_at
    X214669_x_at
Y            [,1]      [,2]
  NR -0.1915274 0.4981106
  R   0.3359622 0.4156267
tables$X214677_x_at
    X214677_x_at
Y            [,1]      [,2]
  NR -0.2087490 0.4787112

R   0.58371780 0.7445854
tables$X1557116_at
   X1557116_at
Y           [,1]      [,2]
  NR -0.4470538 0.6892889
  R   0.3267151 0.4124078
tables$X1557222_at
   X1557222_at
Y            [,1]     [,2]
  NR -0.04818824 0.402835
  R   0.74360000 0.863980
tables$X1558034_s_at
   X1558034_s_at
Y           [,1]      [,2]
  NR -0.2487822 0.4220782
  R   0.4252406 0.4675114
tables$X1558290_a_at
   X1558290_a_at
Y           [,1]      [,2]
  NR  0.3179399 0.5555339
  R  -0.4890204 0.4786095
tables$X1558586_at
   X1558586_at
Y           [,1]      [,2]
  NR -0.2298781 0.4177707
  R   0.3950435 0.5416909
tables$X1558972_s_at
   X1558972_s_at
Y           [,1]      [,2]
  NR -0.2797971 0.5418070
  R   0.4745703 0.5335784
tables$X1559263_s_at
   X1559263_s_at
Y           [,1]      [,2]
  NR -0.3833393 0.5703645
  R   0.3494365 0.4676936
tables$X1559425_at
   X1559425_at
Y           [,1]      [,2]
  NR -0.2287316 0.5560199
  R   0.4282525 0.4558128
tables$X1559584_a_at
   X1559584_a_at
Y           [,1]      [,2]
  NR -0.4622881 0.5846900
  R   0.3021721 0.4028788
tables$X1562031_at
   X1562031_at
Y           [,1]      [,2]
  NR -0.3551523 0.4862453
  R   0.1736934 0.4872335
tables$X1563461_at
  R   0.3128333 0.3867513
tables$X214719_at
   X214719_at
Y           [,1]      [,2]
  NR -0.4511937 0.6070570
  R   0.2711287 0.3505487
tables$X215051_x_at
   X215051_x_at
Y           [,1]      [,2]
  NR -0.3597073 0.8514825
  R   0.4054302 0.3092948
tables$X215121_x_at
   X215121_x_at
Y           [,1]      [,2]
  NR -0.1634545 0.4642640
  R   0.3410072 0.4014670
tables$X215193_x_at
   X215193_x_at
Y           [,1]      [,2]
  NR -0.2667421 0.7134086
  R   0.4777644 0.4030902
tables$X215223_s_at
   X215223_s_at
Y           [,1]      [,2]
  NR -0.3883071 0.4910665
  R   0.2565276 0.4364856
tables$X215379_x_at
   X215379_x_at
Y            [,1]      [,2]
  NR -0.09709331 0.4452099
  R   0.39542823 0.4092484
tables$X215561_s_at
   X215561_s_at
Y           [,1]      [,2]
  NR -0.2711632 0.4077480
  R   0.7072055 0.9320206
tables$X215806_x_at
   X215806_x_at
Y             [,1]      [,2]
  NR -0.007773052 0.3665788
  R   0.661454102 0.4785205
tables$X216155_at
   X216155_at
Y           [,1]      [,2]
  NR  0.5119447 0.6151164
  R  -0.1726881 0.3347342
tables$X216191_s_at
   X216191_s_at
Y            [,1]      [,2]
  NR -0.01479148 0.4433394
  R   0.67109688 0.5079869
tables$X216194_s_at

X1563461_at
Y            [,1]       [,2]
  NR  0.3566529 0.5512415
  R  -0.2706966 0.6822506
tables$X1563473_at
    X1563473_at
Y            [,1]       [,2]
  NR -0.4074615 0.5896901
  R   0.2254661 0.4096997
tables$X1565602_at
    X1565602_at
Y            [,1]       [,2]
  NR  0.4490349 0.6371381
  R  -0.1986003 0.5670284
tables$X1568736_s_at
    X1568736_s_at
Y            [,1]       [,2]
  NR  0.6111740 0.9655418
  R  -0.1669456 0.3464934
tables$X1568822_at
    X1568822_at
Y            [,1]       [,2]
  NR  0.4611639 0.6061123
  R  -0.1089934 0.3960131
tables$X1569942_at
    X1569942_at
Y            [,1]       [,2]
  NR  0.4441859 0.7007245
  R  -0.2886340 0.3295043
tables$X200612_s_at
    X200612_s_at
Y            [,1]       [,2]
  NR  0.2054340 0.4467337
  R  -0.4553481 0.5048914
tables$X200904_at
    X200904_at
Y            [,1]       [,2]
  NR -0.2660950 0.4738856
  R   0.3014091 0.4032104
tables$X200905_x_at
    X200905_x_at
Y            [,1]       [,2]
  NR -0.1327891 0.5236164
  R   0.4735567 0.3948498
tables$X200953_s_at
    X200953_s_at
Y            [,1]       [,2]
  NR -0.2814847 0.8261769
  R   0.5757425 0.6362976
tables$X201010_s_at
    X201010_s_at
Y            [,1]       [,2]
    X216194_s_at
Y            [,1]       [,2]
  NR  0.1641682 0.3935104
  R  -0.3667475 0.4613841
tables$X216714_at
    X216714_at
Y            [,1]       [,2]
  NR -0.1657215 0.6600958
  R   0.5669961 0.9931878
tables$X216841_s_at
    X216841_s_at
Y            [,1]       [,2]
  NR -0.2378813 0.7063683
  R   0.5838156 0.5499012
tables$X216920_s_at
    X216920_s_at
Y            [,1]       [,2]
  NR -0.3626922 0.5129860
  R   0.4424685 0.4026252
tables$X217028_at
    X217028_at
Y            [,1]       [,2]
  NR -0.3936253 0.7242766
  R   0.2813510 0.3842826
tables$X217138_x_at
    X217138_x_at
Y            [,1]       [,2]
  NR 0.06341764 0.4690319
  R  0.61978484 0.5049407
tables$X217143_s_at
    X217143_s_at
Y            [,1]       [,2]
  NR -0.1709594 0.6329777
  R   0.6395395 0.5675738
tables$X217147_s_at
    X217147_s_at
Y            [,1]       [,2]
  NR -0.1919006 0.3907915
  R   0.4594408 0.3635919
tables$X217478_s_at
    X217478_s_at
Y            [,1]       [,2]
  NR -0.3453566 0.7688595
  R   0.5037971 0.4475461
tables$X217525_at
    X217525_at
Y            [,1]       [,2]
  NR -0.2639411 0.8866455
  R   0.6075298 0.5960824
tables$X217629_at
    X217629_at
Y            [,1]      [,2]

NR -0.3613038 0.7929993
R 0.4944130 0.2677128
tables$X201137_s_at
X201137_s_at
Y [,1] [,2]
NR -0.3646008 0.7012958
R 0.3957589 0.4949475
tables$X201220_x_at
X201220_x_at
Y [,1] [,2]
NR 0.08672472 0.5177988
R -0.48143253 0.5513205
tables$X201236_s_at
X201236_s_at
Y [,1] [,2]
NR -0.1198028 0.5613972
R 0.5331348 0.5322952
tables$X201425_at
X201425_at
Y [,1] [,2]
NR -0.5116596 0.7193710
R 0.4964073 0.5304284
tables$X201474_s_at
X201474_s_at
Y [,1] [,2]
NR 0.6028068 0.6299064
R -0.2457276 0.3007111
tables$X201487_at
X201487_at
Y [,1] [,2]
NR -0.4225210 0.8252371
R 0.3171429 0.4591150
tables$X201497_x_at
X201497_x_at
Y [,1] [,2]
NR -0.3200855 0.4908815
R 2.5010094 2.6712499
tables$X201502_s_at
X201502_s_at
Y [,1] [,2]
NR -0.3308824 0.4722939
R 0.5454167 0.3159327
tables$X201531_at
X201531_at
Y [,1] [,2]
NR -0.1938551 0.3744374
R 0.5269050 0.5074891
tables$X201566_x_at
X201566_x_at
Y [,1] [,2]
NR -0.2981010 0.6780610
R 0.3509438 0.5014504
NR -0.1348654 0.671148
R 0.8011176 1.134701
tables$X217767_at
X217767_at
Y [,1] [,2]
NR -0.4316702 0.6383017
R 0.3296425 0.3962954
tables$X217995_at
X217995_at
Y [,1] [,2]
NR -0.6500190 1.0622844
R 0.3656452 0.3596701
tables$X218002_s_at
X218002_s_at
Y [,1] [,2]
NR -0.2371548 0.4549764
R 0.3590465 0.5928193
tables$X218035_s_at
X218035_s_at
Y [,1] [,2]
NR -0.3792486 0.6876336
R 0.3685478 0.4296116
tables$X218145_at
X218145_at
Y [,1] [,2]
NR 0.5407030 0.7961511
R -0.5330237 0.7914334
tables$X218170_at
X218170_at
Y [,1] [,2]
NR -0.2778944 0.5053423
R 0.5047588 0.6555875
tables$X218322_s_at
X218322_s_at
Y [,1] [,2]
NR -0.4368137 0.5136086
R 0.3249243 0.2575257
tables$X218499_at
X218499_at
Y [,1] [,2]
NR -0.2574311 0.6676495
R 0.4022312 0.4066238
tables$X218736_s_at
X218736_s_at
Y [,1] [,2]
NR -0.03615144 0.4561428
R 1.08677338 1.1597104
tables$X218739_at
X218739_at
Y [,1] [,2]
NR -0.02247695 0.4787563
R 0.98329866 1.2407971

tables$X201720_s_at
    X201720_s_at
Y           [,1]      [,2]
  NR -0.1977420 0.6818082
  R   0.5749931 0.7157990
tables$X201721_s_at
    X201721_s_at
Y           [,1]      [,2]
  NR -0.1677046 0.5133205
  R   0.4231327 0.5084300
tables$X201804_x_at
    X201804_x_at
Y           [,1]      [,2]
  NR  0.1992091 0.4851767
  R  -0.4765795 0.5915133
tables$X201859_at
    X201859_at
Y           [,1]      [,2]
  NR -0.4216528 0.7283696
  R   0.2486708 0.3163434
tables$X201939_at
    X201939_at
Y           [,1]      [,2]
  NR  0.2953508 0.5226393
  R  -0.3592031 0.5614863
tables$X202207_at
    X202207_at
Y           [,1]      [,2]
  NR -0.4088858 0.6063797
  R   0.2159392 0.2923256
tables$X202255_s_at
    X202255_s_at
Y           [,1]      [,2]
  NR -0.5135289 0.8447912
  R   0.3161547 0.6241535
tables$X202269_x_at
    X202269_x_at
Y           [,1]      [,2]
  NR -0.4070967 0.6699657
  R   0.3070278 0.3134680
tables$X202270_at
    X202270_at
Y           [,1]      [,2]
  NR -0.3598381 0.7402801
  R   0.4664866 0.4311290
tables$X202368_s_at
    X202368_s_at
Y           [,1]      [,2]
  NR  0.4828523 0.6622996
  R  -0.3498387 0.7337426
tables$X202369_s_at
    X202369_s_at
tables$X218764_at
    X218764_at
Y           [,1]      [,2]
  NR -0.4099779 0.4830029
  R   0.3567869 0.4038910
tables$X218802_at
    X218802_at
Y           [,1]      [,2]
  NR -0.3430126 0.4453773
  R   0.5970457 0.3951568
tables$X218805_at
    X218805_at
Y           [,1]      [,2]
  NR -0.3671355 0.5708859
  R   0.4517210 0.2930704
tables$X218854_at
    X218854_at
Y           [,1]      [,2]
  NR -0.3745559 0.7096822
  R   0.4739352 0.5603528
tables$X218899_s_at
    X218899_s_at
Y           [,1]      [,2]
  NR  0.3671446 0.5909345
  R  -0.5393210 0.4381099
tables$X218950_at
    X218950_at
Y           [,1]      [,2]
  NR  0.2691863 0.4573251
  R  -0.6111111 0.6474936
tables$X219054_at
    X219054_at
Y           [,1]     [,2]
  NR 0.01472793 0.584855
  R  2.49050475 2.683467
tables$X219093_at
    X219093_at
Y           [,1]      [,2]
  NR -0.2971580 0.4688604
  R   0.5384298 0.6366817
tables$X219213_at
    X219213_at
Y           [,1]      [,2]
  NR -0.1731958 0.4047427
  R   0.6423430 0.7730134
tables$X219243_at
    X219243_at
Y           [,1]      [,2]
  NR -0.5195758 0.7578959
  R   0.4000750 0.3801539
tables$X219368_at
    X219368_at

Y            [,1]      [,2]
  NR  0.3894707 0.7051767
  R  -0.4762401 0.5968765
tables$X202391_at
    X202391_at
Y            [,1]      [,2]
  NR -0.5342313 1.0124766
  R   0.5933123 0.6491928
tables$X202510_s_at
    X202510_s_at
Y            [,1]      [,2]
  NR -0.6015966 0.9255712
  R   0.4919973 0.6463912
tables$X202625_at
    X202625_at
Y            [,1]      [,2]
  NR -0.2142036 0.7804199
  R   0.6620458 0.7148131
tables$X202643_s_at
    X202643_s_at
Y            [,1]      [,2]
  NR -0.4832904 0.5361050
  R   0.3792320 0.4385239
tables$X202644_s_at
    X202644_s_at
Y            [,1]      [,2]
  NR -0.3513719 0.5742499
  R   0.4307245 0.3838438
tables$X202687_s_at
    X202687_s_at
Y            [,1]      [,2]
  NR -0.4279648 0.7540136
  R   0.3504315 0.4045005
tables$X202688_at
    X202688_at
Y            [,1]      [,2]
  NR -0.4163941 0.8163225
  R   0.4027076 0.4386812
tables$X202948_at
    X202948_at
Y            [,1]      [,2]
  NR -0.5154460 0.7699102
  R   0.4056092 0.4764355
tables$X202957_at
    X202957_at
Y            [,1]      [,2]
  NR -0.3735948 0.5616205
  R   0.4472934 0.5131898
tables$X203413_at
    X203413_at
Y            [,1]      [,2]
  NR -0.3383329 0.5941084
Y            [,1]      [,2]
  NR -0.1679820 0.3075502
  R   0.6324772 0.6637538
tables$X219440_at
    X219440_at
Y            [,1]      [,2]
  NR 0.08196161 0.2856868
  R  0.87878388 0.7545222
tables$X219454_at
    X219454_at
Y            [,1]      [,2]
  NR -0.1553019 0.3324367
  R   0.6028235 0.7768835
tables$X219505_at
    X219505_at
Y           [,1]      [,2]
  NR -0.335471 0.5884701
  R   0.331203 0.4762600
tables$X219519_s_at
    X219519_s_at
Y            [,1]      [,2]
  NR -0.3029785 0.6574268
  R   0.4245899 0.6733826
tables$X219525_at
    X219525_at
Y            [,1]      [,2]
  NR -0.4849566 0.6164310
  R   0.2295082 0.3984913
tables$X219528_s_at
    X219528_s_at
Y            [,1]      [,2]
  NR -0.2597881 0.4319096
  R   0.4928448 0.5142826
tables$X219631_at
    X219631_at
Y            [,1]      [,2]
  NR  0.5705786 0.6831084
  R  -0.2784402 0.3830630
tables$X219666_at
    X219666_at
Y            [,1]      [,2]
  NR -0.5430705 0.7930909
  R   0.2839697 0.3631652
tables$X219681_s_at
    X219681_s_at
Y           [,1]      [,2]
  NR -0.417274 0.7733850
  R   0.376309 0.5736019
tables$X219710_at
    X219710_at
Y            [,1]      [,2]
  NR  0.2636804 0.4736957

R 0.3196087 0.6292591
tables$X203416_at
X203416_at
Y [,1] [,2]
NR -0.3141917 0.6552882
R 0.4391612 0.4251811
tables$X203471_s_at
X203471_s_at
Y [,1] [,2]
NR -0.2041767 0.5987281
R 0.6153344 0.5620431
tables$X203508_at
X203508_at
Y [,1] [,2]
NR -0.2695595 0.5179795
R 0.2812243 0.4853358
tables$X203523_at
X203523_at
Y [,1] [,2]
NR -0.3791594 0.8958684
R 0.8117565 0.9246945
tables$X203547_at
X203547_at
Y [,1] [,2]
NR -0.008562598 0.4462166
R 0.663436693 0.5733365
tables$X203665_at
X203665_at
Y [,1] [,2]
NR -0.2522801 0.5533137
R 0.6428083 0.7340506
tables$X203741_s_at
X203741_s_at
Y [,1] [,2]
NR -0.1850309 0.5721364
R 0.6581712 0.6185694
tables$X203761_at
X203761_at
Y [,1] [,2]
NR -0.1852189 0.5956581
R 0.5255720 0.4369002
tables$X203812_at
X203812_at
Y [,1] [,2]
NR 0.0587172 0.3136796
R 1.0676683 0.8555980
tables$X203868_s_at
X203868_s_at
Y [,1] [,2]
NR -0.3688355 0.6726738
R 0.2602381 0.3965858
tables$X203915_at
R -0.3241903 0.5279440
tables$X219737_s_at
X219737_s_at
Y [,1] [,2]
NR 0.3596773 0.5146777
R -0.2409581 0.4405703
tables$X219777_at
X219777_at
Y [,1] [,2]
NR -0.3965677 0.5974954
R 0.5026195 0.4178151
tables$X219789_at
X219789_at
Y [,1] [,2]
NR 0.0625228 0.3929864
R 1.5326986 1.6918348
tables$X219926_at
X219926_at
Y [,1] [,2]
NR 0.2428123 0.5989463
R -0.6869766 0.9798973
tables$X219938_s_at
X219938_s_at
Y [,1] [,2]
NR -0.4681105 0.5841168
R 0.3093149 0.2386032
tables$X220005_at
X220005_at
Y [,1] [,2]
NR -0.09036238 0.5172305
R 0.62083607 0.5403729
tables$X220066_at
X220066_at
Y [,1] [,2]
NR -0.2813542 0.4958868
R 0.2894245 0.2549095
tables$X220330_s_at
X220330_s_at
Y [,1] [,2]
NR -0.1838030 0.5516329
R 0.4155977 0.3566931
tables$X220485_s_at
X220485_s_at
Y [,1] [,2]
NR -0.2516505 0.989380
R 1.2092181 1.358517
tables$X221081_s_at
X221081_s_at
Y [,1] [,2]
NR -0.3406222 0.5260212
R 0.2204497 0.3080250
tables$X221087_s_at

X203915_at
Y [,1] [,2]
NR -0.4061025 0.5236050
R 0.2301446 0.2415020
tables$X203932_at
X203932_at
Y [,1] [,2]
NR -0.2494244 0.6622825
R 0.4463418 0.3757829
tables$X204057_at
X204057_at
Y [,1] [,2]
NR -0.3274287 0.5906957
R 0.4847696 0.4283121
tables$X204070_at
X204070_at
Y [,1] [,2]
NR -0.2719985 0.6097243
R 0.4394442 0.3954333
tables$X204116_at
X204116_at
Y [,1] [,2]
NR -0.2653059 0.4657288
R 0.5320249 0.3827573
tables$X204118_at
X204118_at
Y [,1] [,2]
NR -0.2188632 0.5465435
R 0.4397206 0.4841528
tables$X204135_at
X204135_at
Y [,1] [,2]
NR -0.2511983 0.3620796
R 0.5582305 0.6587871
tables$X204204_at
X204204_at
Y [,1] [,2]
NR -0.2476304 0.5677727
R 0.5469376 0.5015591
tables$X204220_at
X204220_at
Y [,1] [,2]
NR -0.4512334 0.9464671
R 0.4470878 0.4911261
tables$X204222_s_at
X204222_s_at
Y [,1] [,2]
NR -0.2858410 0.8009875
R 0.4771543 0.4557632
tables$X204224_s_at
X204224_s_at
Y [,1] [,2]
X221087_s_at
Y [,1] [,2]
NR -0.3965314 0.6050086
R 0.2956204 0.3544964
tables$X221477_s_at
X221477_s_at
Y [,1] [,2]
NR -0.3833003 0.7680959
R 0.4128233 0.5675033
tables$X221651_x_at
X221651_x_at
Y [,1] [,2]
NR -0.3022085 0.4778491
R 0.2004842 0.2922928
tables$X221671_x_at
X221671_x_at
Y [,1] [,2]
NR -0.3000931 0.4932356
R 0.2268629 0.2801547
tables$X221698_s_at
X221698_s_at
Y [,1] [,2]
NR -0.6376038 0.9130990
R 0.3423824 0.3837021
tables$X221756_at
X221756_at
Y [,1] [,2]
NR -0.1597059 0.4810294
R 0.7144792 0.8100807
tables$X221760_at
X221760_at
Y [,1] [,2]
NR -0.3895238 0.6667009
R 0.2589523 0.4120362
tables$X222108_at
X222108_at
Y [,1] [,2]
NR -0.3578270 0.4644883
R 0.3207759 0.3326270
tables$X222142_at
X222142_at
Y [,1] [,2]
NR -0.1876780 0.4933581
R 0.3824561 0.4368395
tables$X222484_s_at
X222484_s_at
Y [,1] [,2]
NR -0.4729730 0.6937840
R 0.3761529 0.5801888
tables$X222496_s_at
X222496_s_at
Y [,1] [,2]

NR -0.4034291 0.533261
R 0.4591664 0.324186
tables$X204233_s_at
X204233_s_at
Y [,1] [,2]
NR 0.1725203 0.526541
R -0.4473613 0.498801
tables$X204236_at
X204236_at
Y [,1] [,2]
NR -0.3405471 0.7438102
R 0.4577420 0.4830432
tables$X204249_s_at
X204249_s_at
Y [,1] [,2]
NR -0.2439368 0.6736956
R 0.5798690 0.5938803
tables$X204411_at
X204411_at
Y [,1] [,2]
NR -0.1561360 0.5046013
R 0.5348875 0.6639757
tables$X204438_at
X204438_at
Y [,1] [,2]
NR -0.2155729 0.8013524
R 0.5238707 0.5465740
tables$X204502_at
X204502_at
Y [,1] [,2]
NR -0.3163975 0.5660414
R 0.3122723 0.3372872
tables$X204512_at
X204512_at
Y [,1] [,2]
NR -0.3823994 0.7656201
R 0.3405082 0.3302137
tables$X204533_at
X204533_at
Y [,1] [,2]
NR -0.3432882 0.5411550
R 0.2820025 0.3529044
tables$X204613_at
X204613_at
Y [,1] [,2]
NR -0.2921141 0.5365989
R 0.3433918 0.4634024
tables$X204628_s_at
X204628_s_at
Y [,1] [,2]
NR 0.2412563 0.5057318
R -0.4238494 0.4487938
NR -0.4144878 0.6266733
R 0.2909259 0.2895369
tables$X222592_s_at
X222592_s_at
Y [,1] [,2]
NR -0.2780972 0.5068942
R 0.4106129 0.2951602
tables$X222725_s_at
X222725_s_at
Y [,1] [,2]
NR -0.004398634 0.5694045
R 1.150669643 1.2645302
tables$X222780_s_at
X222780_s_at
Y [,1] [,2]
NR 0.4139560 0.5398394
R -0.4224571 0.2983037
tables$X222838_at
X222838_at
Y [,1] [,2]
NR -0.2978748 0.5457765
R 0.2590990 0.2508078
tables$X222895_s_at
X222895_s_at
Y [,1] [,2]
NR -0.4561043 0.5248071
R 0.1985346 0.3539992
tables$X223044_at
X223044_at
Y [,1] [,2]
NR -0.4249287 0.7273378
R 0.3345815 0.5282742
tables$X223058_at
X223058_at
Y [,1] [,2]
NR -0.5976291 0.7565737
R 0.2836632 0.4557419
tables$X223059_s_at
X223059_s_at
Y [,1] [,2]
NR -0.2707661 0.6996713
R 0.5245500 0.4391394
tables$X223168_at
X223168_at
Y [,1] [,2]
NR -0.3429218 0.5205996
R 0.2543322 0.3823076
tables$X223235_s_at
X223235_s_at
Y [,1] [,2]
NR -0.2858518 0.4718234
R 0.6748075 0.7147878

tables$X204642_at
    X204642_at
Y            [,1]      [,2]
  NR -0.3125616 0.7411993
  R   0.6952398 1.0691429
tables$X204655_at
    X204655_at
Y            [,1]      [,2]
  NR -0.2716510 0.6208783
  R   0.5579874 0.5183030
tables$X204661_at
    X204661_at
Y            [,1]      [,2]
  NR -0.1558546 0.4835795
  R   0.5868508 0.3065469
tables$X204670_x_at
    X204670_x_at
Y            [,1]      [,2]
  NR -0.3190668 0.6529019
  R   0.4161121 0.3104711
tables$X204687_at
    X204687_at
Y           [,1]      [,2]
  NR 0.1167838 0.3453266
  R  1.0001048 0.8753104
tables$X204724_s_at
    X204724_s_at
Y            [,1]      [,2]
  NR  0.5223107 0.6822646
  R  -0.1768374 0.3008967
tables$X204774_at
    X204774_at
Y            [,1]      [,2]
  NR -0.3545849 0.6395887
  R   0.1938959 0.3271488
tables$X204778_x_at
    X204778_x_at
Y            [,1]      [,2]
  NR  0.3141006 0.5671928
  R  -0.4937885 0.7083350
tables$X204834_at
    X204834_at
Y            [,1]      [,2]
  NR -0.2829585 0.5026786
  R   0.2222514 0.2582449
tables$X204846_at
    X204846_at
Y           [,1]      [,2]
  NR 0.1129389 0.4384144
  R  0.7672991 0.7249123
tables$X204894_s_at
    X204894_s_at
tables$X223280_x_at
    X223280_x_at
Y            [,1]      [,2]
  NR -0.5988057 0.8558921
  R   0.2763766 0.4726699
tables$X223322_at
    X223322_at
Y            [,1]      [,2]
  NR -0.5307109 0.7623257
  R   0.4863893 0.5655322
tables$X223361_at
    X223361_at
Y            [,1]      [,2]
  NR -0.2076597 0.5924435
  R   0.3858025 0.5723073
tables$X223395_at
    X223395_at
Y            [,1]      [,2]
  NR -0.2616053 0.5452143
  R   0.4342364 0.5337485
tables$X223484_at
    X223484_at
Y            [,1]      [,2]
  NR -0.2440592 0.3975480
  R   0.4224246 0.4741438
tables$X223809_at
    X223809_at
Y            [,1]      [,2]
  NR -0.2342077 0.5645847
  R   0.3558237 0.3616038
tables$X223827_at
    X223827_at
Y             [,1]      [,2]
  NR  0.60366334 0.8225446
  R  -0.06966146 0.1454527
tables$X223922_x_at
    X223922_x_at
Y            [,1]      [,2]
  NR -0.4981749 0.7014087
  R   0.2234232 0.3896602
tables$X223924_at
    X223924_at
Y            [,1]      [,2]
  NR  0.4849658 0.6301636
  R  -0.1459645 0.4704890
tables$X223952_x_at
    X223952_x_at
Y           [,1]      [,2]
  NR 0.1264371 0.4236437
  R  0.8628858 0.9031651
tables$X224356_x_at
    X224356_x_at

Y              [,1]      [,2]
  NR -0.04169668 0.5285664
  R   0.90340136 1.0066307
tables$X204897_at
    X204897_at
Y             [,1]      [,2]
  NR -0.5753379 0.7174762
  R   0.2097906 0.3253104
tables$X204912_at
    X204912_at
Y             [,1]      [,2]
  NR -0.1581854 0.4616605
  R   0.4406020 0.3673491
tables$X204923_at
    X204923_at
Y             [,1]      [,2]
  NR -0.1810564 0.5020173
  R   0.4762799 0.5956602
tables$X205027_s_at
    X205027_s_at
Y             [,1]      [,2]
  NR -0.2115740 0.8053652
  R   0.9541627 0.9478824
tables$X205039_s_at
    X205039_s_at
Y              [,1]      [,2]
  NR -0.07447284 0.6105666
  R   0.75225499 0.7306959
tables$X205081_at
    X205081_at
Y             [,1]      [,2]
  NR -0.4320239 0.6844631
  R   0.4515831 0.6544651
tables$X205159_at
    X205159_at
Y             [,1]      [,2]
  NR -0.3955032 0.7064307
  R   0.4559497 0.4821926
tables$X205225_at
    X205225_at
Y             [,1]      [,2]
  NR -0.4166425 0.5776734
  R   0.4113626 0.4147703
tables$X205226_at
    X205226_at
Y              [,1]      [,2]
  NR -0.07833695 0.4854712
  R   0.44132335 0.7107620
tables$X205251_at
    X205251_at
Y             [,1]      [,2]
  NR -0.3110220 0.6899898
Y             [,1]      [,2]
  NR -0.5122008 0.7621637
  R   0.2604697 0.4291461
tables$X224358_s_at
    X224358_s_at
Y             [,1]      [,2]
  NR -0.4371837 0.8408400
  R   0.3218548 0.5126749
tables$X224451_x_at
    X224451_x_at
Y             [,1]      [,2]
  NR -0.2604966 0.5285926
  R   0.4760714 0.4279992
tables$X224516_s_at
    X224516_s_at
Y             [,1]      [,2]
  NR  0.2968854 0.6541436
  R  -0.6889220 0.9012319
tables$X224710_at
    X224710_at
Y             [,1]      [,2]
  NR  0.2230392 0.3643719
  R  -0.7978563 0.9064005
tables$X224771_at
    X224771_at
Y             [,1]      [,2]
  NR  0.4329412 0.8903073
  R  -0.4421469 0.6725526
tables$X224772_at
    X224772_at
Y             [,1]      [,2]
  NR  0.1916542 0.4865887
  R  -0.5224768 0.4540955
tables$X224773_at
    X224773_at
Y             [,1]      [,2]
  NR  0.3157972 0.5857999
  R  -0.4979135 0.4238849
tables$X224774_s_at
    X224774_s_at
Y             [,1]      [,2]
  NR  0.5703417 0.6486541
  R  -0.5030708 0.5503160
tables$X224795_x_at
    X224795_x_at
Y             [,1]      [,2]
  NR -0.3054091 0.4872416
  R   0.2135411 0.2854565
tables$X224859_at
    X224859_at
Y             [,1]      [,2]
  NR  0.4830222 0.7137397

R 0.5272399 0.6253671
tables$X205285_s_at
X205285_s_at
Y [,1] [,2]
NR -0.04432586 0.5000114
R 0.80925537 0.9021956
tables$X205392_s_at
X205392_s_at
Y [,1] [,2]
NR -0.4220336 0.4956664
R 0.4657780 0.3693635
tables$X205403_at
X205403_at
Y [,1] [,2]
NR -0.2005647 0.4806835
R 0.5966667 0.7091052
tables$X205419_at
X205419_at
Y [,1] [,2]
NR -0.2813820 0.5751165
R 0.5056242 0.4918400
tables$X205421_at
X205421_at
Y [,1] [,2]
NR 0.07840435 0.3868954
R 0.86698828 0.8643222
tables$X205440_s_at
X205440_s_at
Y [,1] [,2]
NR -0.3108277 0.5223075
R 0.3749281 0.5730182
tables$X205484_at
X205484_at
Y [,1] [,2]
NR -0.2739962 0.5607660
R 0.4613413 0.4462779
tables$X205488_at
X205488_at
Y [,1] [,2]
NR -0.211302 0.5156343
R 0.451222 0.3725271
tables$X205559_s_at
X205559_s_at
Y [,1] [,2]
NR -0.1967785 0.4797350
R 0.4925489 0.5104237
tables$X205569_at
X205569_at
Y [,1] [,2]
NR -0.3112147 0.5708862
R 0.3356277 0.4533386
tables$X205624_at
R -0.3679703 0.6853105
tables$X224896_s_at
X224896_s_at
Y [,1] [,2]
NR 0.1816808 0.7977231
R -0.6408326 0.5850013
tables$X225502_at
X225502_at
Y [,1] [,2]
NR -0.3910820 0.6073778
R 0.4852237 0.4201402
tables$X225802_at
X225802_at
Y [,1] [,2]
NR 0.1344681 0.5702449
R -0.4950925 0.5294767
tables$X225895_at
X225895_at
Y [,1] [,2]
NR -0.3277170 0.7566241
R 0.6412089 0.7479682
tables$X226043_at
X226043_at
Y [,1] [,2]
NR 0.1973572 0.6004289
R -0.3655970 0.4868833
tables$X226068_at
X226068_at
Y [,1] [,2]
NR -0.3490564 0.6019387
R 0.4335378 0.4792696
tables$X226117_at
X226117_at
Y [,1] [,2]
NR -0.2837194 0.5233255
R 0.4253069 0.3317653
tables$X226218_at
X226218_at
Y [,1] [,2]
NR -0.3859418 0.6090051
R 0.4404194 0.3778062
tables$X226219_at
X226219_at
Y [,1] [,2]
NR -0.2943873 0.5216637
R 0.4004941 0.4131372
tables$X226303_at
X226303_at
Y [,1] [,2]
NR -0.04068867 0.4037450
R 0.73077236 0.8465357
tables$X226382_at

X205624_at
Y [,1] [,2]
NR 0.005427652 0.4382683
R 0.763112333 0.7396355
tables$X205685_at
X205685_at
Y [,1] [,2]
NR -0.1946623 0.5288449
R 0.4467601 0.4223863
tables$X205696_s_at
X205696_s_at
Y [,1] [,2]
NR -0.01091462 0.3565887
R 0.77207850 0.7974577
tables$X205758_at
X205758_at
Y [,1] [,2]
NR -0.2386930 0.4171697
R 0.4248193 0.3603297
tables$X205786_s_at
X205786_s_at
Y [,1] [,2]
NR -0.4968271 0.8934640
R 0.4149078 0.5488123
tables$X205831_at
X205831_at
Y [,1] [,2]
NR 0.04447927 0.4259325
R 0.69838627 0.4833483
tables$X205841_at
X205841_at
Y [,1] [,2]
NR -0.5900546 0.7313311
R 0.3136276 0.5142651
tables$X205844_at
X205844_at
Y [,1] [,2]
NR -0.1423775 0.4883837
R 0.4235432 0.4438472
tables$X205890_s_at
X205890_s_at
Y [,1] [,2]
NR -0.3511421 0.4822404
R 0.3120630 0.2244957
tables$X205987_at
X205987_at
Y [,1] [,2]
NR -0.09413415 0.3978789
R 0.59504052 0.5335613
tables$X206082_at
X206082_at
Y [,1] [,2]
X226382_at
Y [,1] [,2]
NR -0.3657410 0.4652645
R 0.2469253 0.4366802
tables$X226459_at
X226459_at
Y [,1] [,2]
NR -0.3680501 0.7660787
R 0.5040254 0.5894990
tables$X226625_at
X226625_at
Y [,1] [,2]
NR -0.3757333 0.7664846
R 0.5166961 0.4684207
tables$X226659_at
X226659_at
Y [,1] [,2]
NR -0.4911180 0.6832209
R 0.2612643 0.5381065
tables$X226697_at
X226697_at
Y [,1] [,2]
NR 0.1958473 0.3711059
R -0.5652399 0.4049380
tables$X226818_at
X226818_at
Y [,1] [,2]
NR -0.2318967 0.6119576
R 0.4987669 0.3954684
tables$X226841_at
X226841_at
Y [,1] [,2]
NR -0.2979441 0.7024396
R 0.5392790 0.4413953
tables$X226865_at
X226865_at
Y [,1] [,2]
NR -0.1559129 0.5471301
R 0.5041605 0.5099762
tables$X227035_x_at
X227035_x_at
Y [,1] [,2]
NR 0.2005227 0.4921465
R -0.3809524 0.4152160
tables$X227231_at
X227231_at
Y [,1] [,2]
NR 0.2353572 0.7279505
R -0.6509830 0.8181286
tables$X227253_at
X227253_at
Y [,1] [,2]

NR -0.4540809 0.6785699
R 0.4716106 0.5496119
tables$X206099_at
X206099_at
Y [,1] [,2]
NR -0.0531401 0.3438675
R 0.5295345 0.5201337
tables$X206118_at
X206118_at
Y [,1] [,2]
NR -0.2013207 0.4883106
R 0.5011802 0.4695632
tables$X206134_at
X206134_at
Y [,1] [,2]
NR -0.2474317 0.6355258
R 0.3651382 0.4198818
tables$X206170_at
X206170_at
Y [,1] [,2]
NR -0.4274713 0.5078651
R 0.3104491 0.3419569
tables$X206204_at
X206204_at
Y [,1] [,2]
NR -0.02057820 0.2205198
R 0.94769151 0.9338282
tables$X206295_at
X206295_at
Y [,1] [,2]
NR -0.4222879 0.5539909
R 0.2869025 0.3697476
tables$X206385_s_at
X206385_s_at
Y [,1] [,2]
NR -0.1448051 0.4343064
R 0.5421084 0.6951307
tables$X206407_s_at
X206407_s_at
Y [,1] [,2]
NR -0.0965811 0.4714271
R 0.5253937 0.6785424
tables$X206545_at
X206545_at
Y [,1] [,2]
NR -0.2348002 0.6434845
R 0.3898586 0.5979256
tables$X206571_s_at
X206571_s_at
Y [,1] [,2]
NR 0.2964052 0.5266454
R -0.5021825 0.7307312
NR 0.03671536 0.4958005
R 1.03309945 1.0290932
tables$X227265_at
X227265_at
Y [,1] [,2]
NR -0.3512747 0.6026026
R 0.3283856 0.3401296
tables$X227346_at
X227346_at
Y [,1] [,2]
NR -0.3365397 0.7439603
R 0.6189716 0.5315020
tables$X227361_at
X227361_at
Y [,1] [,2]
NR -0.2481734 0.4900125
R 0.2745731 0.3724106
tables$X227458_at
X227458_at
Y [,1] [,2]
NR -0.1210425 0.5832677
R 0.5061539 0.5297024
tables$X227584_at
X227584_at
Y [,1] [,2]
NR 0.1614340 0.6512560
R -0.6792229 0.5867806
tables$X227609_at
X227609_at
Y [,1] [,2]
NR -0.2162779 0.6215554
R 0.4224651 0.4449701
tables$X227640_s_at
X227640_s_at
Y [,1] [,2]
NR 0.2476183 0.5414889
R -0.3764071 0.4938285
tables$X227780_s_at
X227780_s_at
Y [,1] [,2]
NR -0.3434434 0.6237700
R 0.4192693 0.6791114
tables$X227791_at
X227791_at
Y [,1] [,2]
NR -0.2588648 0.5252067
R 0.4328271 0.4393930
tables$X227983_at
X227983_at
Y [,1] [,2]
NR -0.3232334 0.6182386
R 0.3661930 0.5316841

tables$X206637_at
    X206637_at
Y            [,1]      [,2]
  NR -0.4929224 0.8293232
  R   0.5561408 0.7415308
tables$X206666_at
    X206666_at
Y            [,1]      [,2]
  NR -0.3815276 0.4518748
  R   0.3020703 0.1922297
tables$X206687_s_at
    X206687_s_at
Y            [,1]      [,2]
  NR -0.3592461 0.6018756
  R   0.3975221 0.5022615
tables$X206715_at
    X206715_at
Y            [,1]      [,2]
  NR -0.2541760 0.6982257
  R   0.4082547 0.3259496
tables$X206804_at
    X206804_at
Y           [,1]      [,2]
  NR 0.1482064 0.5068781
  R  0.9166047 0.7147839
tables$X206898_at
    X206898_at
Y            [,1]      [,2]
  NR  0.1056709 0.4682096
  R  -0.5435822 0.5520821
tables$X206978_at
    X206978_at
Y            [,1]      [,2]
  NR -0.3496069 0.5339659
  R   0.2843234 0.3287630
tables$X207076_s_at
    X207076_s_at
Y             [,1]      [,2]
  NR -0.02361779 0.5694192
  R   1.31170006 1.6322450
tables$X207238_s_at
    X207238_s_at
Y            [,1]      [,2]
  NR -0.2571791 0.6182402
  R   0.5089045 0.4856678
tables$X207277_at
    X207277_at
Y            [,1]      [,2]
  NR -0.3525872 0.7509863
  R   0.4181711 0.4255746
tables$X207458_at
    X207458_at
tables$X227995_at
    X227995_at
Y            [,1]      [,2]
  NR -0.1696409 0.3982272
  R   0.4873422 0.5629170
tables$X228054_at
    X228054_at
Y            [,1]      [,2]
  NR  0.1996287 0.5784055
  R  -0.4191671 0.4113732
tables$X228071_at
    X228071_at
Y            [,1]      [,2]
  NR -0.5040346 0.7357555
  R   0.4529897 0.4488694
tables$X228094_at
    X228094_at
Y            [,1]      [,2]
  NR -0.5037012 0.7256858
  R   0.5085461 0.4165603
tables$X228153_at
    X228153_at
Y            [,1]      [,2]
  NR -0.3116782 0.6938566
  R   0.3443284 0.3237526
tables$X228339_at
    X228339_at
Y            [,1]      [,2]
  NR -0.2039593 0.7344429
  R   0.5985043 0.7672987
tables$X228362_s_at
    X228362_s_at
Y            [,1]      [,2]
  NR -0.3778104 0.5378346
  R   0.2342191 0.2730511
tables$X228372_at
    X228372_at
Y            [,1]      [,2]
  NR -0.4350057 0.7819102
  R   0.4561920 0.3911495
tables$X228376_at
    X228376_at
Y            [,1]      [,2]
  NR -0.3453361 0.7260925
  R   0.5292299 0.5379290
tables$X228427_at
    X228427_at
Y            [,1]      [,2]
  NR  0.1593717 0.7055373
  R  -0.6106282 0.8316641
tables$X228532_at
    X228532_at

Y              [,1]       [,2]
  NR  0.2419331 0.5735746
  R  -0.3710235 0.4350871
tables$X207540_s_at
    X207540_s_at
Y              [,1]       [,2]
  NR -0.2692932 0.5488522
  R   0.3711915 0.4966252
tables$X207574_s_at
    X207574_s_at
Y              [,1]       [,2]
  NR -0.3958191 0.7713632
  R   0.4298086 0.3017609
tables$X207651_at
    X207651_at
Y              [,1]       [,2]
  NR -0.3101854 0.4462963
  R   0.5275557 0.2883934
tables$X207655_s_at
    X207655_s_at
Y              [,1]       [,2]
  NR -0.2656028 0.6249668
  R   0.4038824 0.4984622
tables$X207677_s_at
    X207677_s_at
Y              [,1]       [,2]
  NR -0.1067724 0.6536107
  R   0.6251422 0.4735116
tables$X207843_x_at
    X207843_x_at
Y              [,1]       [,2]
  NR -0.1980640 0.4840094
  R   0.6923162 1.1190386
tables$X207861_at
    X207861_at
Y              [,1]       [,2]
  NR -0.1260775 0.3508021
  R   0.6778354 0.7911460
tables$X207977_s_at
    X207977_s_at
Y               [,1]       [,2]
  NR -0.02630196 0.3452417
  R   0.72601762 0.5798353
tables$X207992_s_at
    X207992_s_at
Y              [,1]       [,2]
  NR -0.2962782 0.6213732
  R   0.4065298 0.3562586
tables$X208296_x_at
    X208296_x_at
Y              [,1]       [,2]
  NR -0.3202962 0.6726712
Y              [,1]       [,2]
  NR -0.3794859 0.6481241
  R   0.5000793 0.4684207
tables$X228552_s_at
    X228552_s_at
Y              [,1]       [,2]
  NR  0.1167790 0.4798513
  R  -0.5450925 0.7268180
tables$X228563_at
    X228563_at
Y              [,1]       [,2]
  NR  0.4236616 0.5619516
  R  -0.3316479 0.5347978
tables$X228660_x_at
    X228660_x_at
Y              [,1]       [,2]
  NR  0.5804684 0.7378901
  R  -0.1713584 0.4483440
tables$X228776_at
    X228776_at
Y               [,1]       [,2]
  NR  0.06752481 0.5251283
  R  -0.80628566 0.8423271
tables$X228812_at
    X228812_at
Y              [,1]       [,2]
  NR -0.3127907 0.6313677
  R   0.5074128 0.6516242
tables$X228858_at
    X228858_at
Y              [,1]       [,2]
  NR -0.2190644 0.7355751
  R   0.8241261 0.8754638
tables$X228869_at
    X228869_at
Y              [,1]       [,2]
  NR -0.3255735 0.5223796
  R   0.3751149 0.4806706
tables$X228908_s_at
    X228908_s_at
Y              [,1]       [,2]
  NR  0.3383439 0.4459491
  R  -0.2935832 0.7121557
tables$X228964_at
    X228964_at
Y              [,1]       [,2]
  NR -0.3612173 0.5362295
  R   0.2810782 0.4326503
tables$X229127_at
    X229127_at
Y              [,1]       [,2]
  NR -0.4082796 0.5831644

R 0.4715979 0.3630417
tables$X208306_x_at
X208306_x_at
Y [,1] [,2]
NR -0.3404445 0.6697022
R 0.3560108 0.2684112
tables$X208335_s_at
X208335_s_at
Y [,1] [,2]
NR -0.6832978 0.8622729
R 0.3887120 0.6307942
tables$X208450_at
X208450_at
Y [,1] [,2]
NR 0.05646932 0.6161496
R 0.97349919 0.7211533
tables$X208747_s_at
X208747_s_at
Y [,1] [,2]
NR -0.7269106 1.215719
R 0.6146325 0.602124
tables$X208885_at
X208885_at
Y [,1] [,2]
NR -0.4878552 0.6970401
R 0.3000394 0.4132369
tables$X208894_at
X208894_at
Y [,1] [,2]
NR -0.2782715 0.5325159
R 0.3660963 0.3734728
tables$X208981_at
X208981_at
Y [,1] [,2]
NR -0.3974853 0.7581136
R 0.3901547 0.3829096
tables$X208983_s_at
X208983_s_at
Y [,1] [,2]
NR -0.3682743 0.6238660
R 0.3346874 0.3924348
tables$X209083_at
X209083_at
Y [,1] [,2]
NR 0.1258308 0.6751235
R 0.9235844 0.8069453
tables$X209138_x_at
X209138_x_at
Y [,1] [,2]
NR -0.1402324 0.4308172
R 0.3394879 0.3946554
tables$X209193_at
R 0.3339318 0.8204140
tables$X229163_at
X229163_at
Y [,1] [,2]
NR 0.4273831 0.7589842
R -0.2717283 0.5013583
tables$X229367_s_at
X229367_s_at
Y [,1] [,2]
NR -0.05987742 0.3928434
R 0.56349206 0.5126817
tables$X229390_at
X229390_at
Y [,1] [,2]
NR -0.4894938 0.6210411
R 0.1935497 0.2963602
tables$X229391_s_at
X229391_s_at
Y [,1] [,2]
NR -0.4084651 0.5373347
R 0.2172787 0.2684421
tables$X229543_at
X229543_at
Y [,1] [,2]
NR -0.3788834 0.5874714
R 0.2662277 0.2904183
tables$X229625_at
X229625_at
Y [,1] [,2]
NR -0.3005955 0.6601697
R 0.5530654 0.4811529
tables$X229723_at
X229723_at
Y [,1] [,2]
NR -0.2920168 0.5053402
R 0.4696342 0.4191172
tables$X230233_at
X230233_at
Y [,1] [,2]
NR -0.4143786 0.5555824
R 0.3424073 0.3923358
tables$X230391_at
X230391_at
Y [,1] [,2]
NR -0.3501254 0.8265678
R 0.4900276 0.4781532
tables$X230538_at
X230538_at
Y [,1] [,2]
NR 0.1276822 0.3932100
R -0.5735748 0.7224135
tables$X230728_at

X209193_at
Y [,1] [,2]
NR -0.2565556 0.5468921
R 0.4873494 0.4649883
tables$X209195_s_at
X209195_s_at
Y [,1] [,2]
NR 0.1800756 0.4677919
R -0.4026223 0.3673557
tables$X209202_s_at
X209202_s_at
Y [,1] [,2]
NR 0.1964649 0.7379767
R -0.5653081 0.5741860
tables$X209312_x_at
X209312_x_at
Y [,1] [,2]
NR -0.2928795 0.6652902
R 0.4275265 0.3568548
tables$X209480_at
X209480_at
Y [,1] [,2]
NR -0.2841051 0.4380402
R 0.2693738 0.4024957
tables$X209542_x_at
X209542_x_at
Y [,1] [,2]
NR -0.2363103 0.6011656
R 0.4349808 0.5810748
tables$X209603_at
X209603_at
Y [,1] [,2]
NR -0.4656699 0.4521237
R 0.3621330 0.6118526
tables$X209606_at
X209606_at
Y [,1] [,2]
NR -0.2611256 0.5441526
R 0.5627781 0.3290949
tables$X209612_s_at
X209612_s_at
Y [,1] [,2]
NR -0.2482860 0.4645433
R 0.3891742 0.5803480
tables$X209613_s_at
X209613_s_at
Y [,1] [,2]
NR -0.3246706 0.4195264
R 0.2137530 0.4363348
tables$X209670_at
X209670_at
Y [,1] [,2]
X230728_at
Y [,1] [,2]
NR 0.3244249 0.4668566
R -0.2743950 0.5139070
tables$X231032_at
X231032_at
Y [,1] [,2]
NR 0.43019858 0.5207535
R -0.04588641 0.3560523
tables$X231262_at
X231262_at
Y [,1] [,2]
NR 0.05461486 0.2495836
R 1.32806434 1.1081493
tables$X231577_s_at
X231577_s_at
Y [,1] [,2]
NR -0.3279081 0.7462895
R 0.4819051 0.4270797
tables$X231882_at
X231882_at
Y [,1] [,2]
NR 0.1390358 0.4492620
R -0.4280975 0.4211427
tables$X231929_at
X231929_at
Y [,1] [,2]
NR -0.4149811 0.6028339
R 0.3275499 0.4633949
tables$X232001_at
X232001_at
Y [,1] [,2]
NR -0.4082551 0.5622408
R 0.2120889 0.4452512
tables$X232024_at
X232024_at
Y [,1] [,2]
NR -0.2767578 0.5975732
R 0.4329087 0.3644723
tables$X232234_at
X232234_at
Y [,1] [,2]
NR -0.2485230 0.5169943
R 0.5378690 0.4919297
tables$X232311_at
X232311_at
Y [,1] [,2]
NR -0.2207241 0.5017714
R 0.4996502 0.4849287
tables$X232313_at
X232313_at
Y [,1] [,2]

NR -0.3904387 0.5757881
R 0.4960604 0.4286288
tables$X209671_x_at
X209671_x_at
Y [,1] [,2]
NR 0.005489902 0.4958616
R 0.946682909 0.6669064
tables$X209685_s_at
X209685_s_at
Y [,1] [,2]
NR -0.3380362 0.6105057
R 0.3675781 0.4181149
tables$X209687_at
X209687_at
Y [,1] [,2]
NR -0.5180091 0.5954837
R 0.3297076 0.5807315
tables$X209710_at
X209710_at
Y [,1] [,2]
NR -0.2868007 0.3941463
R 0.4039716 0.7769142
tables$X209734_at
X209734_at
Y [,1] [,2]
NR -0.2771824 0.5584141
R 0.3480342 0.4269202
tables$X209774_x_at
X209774_x_at
Y [,1] [,2]
NR -0.5154483 0.5084051
R 0.3327482 0.2052511
tables$X209795_at
X209795_at
Y [,1] [,2]
NR -0.5014384 0.5485146
R 0.2126023 0.2563472
tables$X209813_x_at
X209813_x_at
Y [,1] [,2]
NR -0.02446552 0.4178348
R 0.66828816 0.5665816
tables$X209899_s_at
X209899_s_at
Y [,1] [,2]
NR 0.2607015 0.6973904
R -0.4270344 0.5920830
tables$X209924_at
X209924_at
Y [,1] [,2]
NR -0.1412661 0.5234151
R 0.3997205 0.5359769

NR 0.09690295 0.2976671
R 0.96479280 0.7382041
tables$X232476_at
X232476_at
Y [,1] [,2]
NR 0.1978897 0.4874151
R -0.3000000 0.4272541
tables$X232543_x_at
X232543_x_at
Y [,1] [,2]
NR -0.1276883 0.6158742
R 0.6695534 0.5964269
tables$X232617_at
X232617_at
Y [,1] [,2]
NR -0.2190263 0.6769132
R 0.5988644 0.4031826
tables$X232746_at
X232746_at
Y [,1] [,2]
NR -0.3742172 0.5931449
R 0.3407643 0.5742058
tables$X232843_s_at
X232843_s_at
Y [,1] [,2]
NR -0.3175189 0.5332408
R 0.3950617 0.3666760
tables$X233123_at
X233123_at
Y [,1] [,2]
NR -0.2015742 0.5531279
R 0.4654343 0.7103440
tables$X233562_at
X233562_at
Y [,1] [,2]
NR 0.2792368 0.4159995
R -0.2877796 0.4229988
tables$X233955_x_at
X233955_x_at
Y [,1] [,2]
NR 0.3290657 0.6359343
R -0.3166667 0.5295163
tables$X235175_at
X235175_at
Y [,1] [,2]
NR -0.1311223 0.4904278
R 0.4533666 0.4676313
tables$X235238_at
X235238_at
Y [,1] [,2]
NR 0.1248063 0.3770484
R -0.5772758 0.7499223

tables$X209949_at
    X209949_at
Y            [,1]      [,2]
  NR -0.2882600 0.4631077
  R   0.4047160 0.3303257
tables$X209970_x_at
    X209970_x_at
Y            [,1]      [,2]
  NR -0.4439942 0.6955252
  R   0.1102440 0.2548926
tables$X210038_at
    X210038_at
Y           [,1]      [,2]
  NR 0.1317110 0.4768708
  R  0.8494560 0.7295487
tables$X210072_at
    X210072_at
Y            [,1]      [,2]
  NR 0.01156063 0.3737049
  R  0.57481931 0.4627490
tables$X210251_s_at
    X210251_s_at
Y            [,1]      [,2]
  NR  0.2240660 0.4879510
  R  -0.6597691 0.6547051
tables$X210260_s_at
    X210260_s_at
Y            [,1]      [,2]
  NR -0.3640910 0.6337100
  R   0.4487179 0.3720418
tables$X210319_x_at
    X210319_x_at
Y             [,1]      [,2]
  NR -0.08072798 0.5440481
  R   0.83296501 1.2085067
tables$X210375_at
    X210375_at
Y            [,1]      [,2]
  NR -0.0859139 0.2736614
  R   0.9924649 1.0291749
tables$X210554_s_at
    X210554_s_at
Y            [,1]      [,2]
  NR  0.1522882 0.5489999
  R  -0.5083098 0.6130962
tables$X210835_s_at
    X210835_s_at
Y            [,1]      [,2]
  NR  0.2332468 0.5454729
  R  -0.4438314 0.6253821
tables$X210915_x_at
    X210915_x_at
tables$X235306_at
    X235306_at
Y            [,1]     [,2]
  NR -0.5666624 1.047734
  R   0.4955309 0.534125
tables$X235391_at
    X235391_at
Y            [,1]      [,2]
  NR  0.3239007 0.6545010
  R  -0.5514259 0.6035657
tables$X235421_at
    X235421_at
Y            [,1]      [,2]
  NR -0.2094653 0.4583538
  R   0.4672043 0.4014682
tables$X235639_at
    X235639_at
Y            [,1]      [,2]
  NR  0.3234035 0.5491068
  R  -0.3795961 0.6104110
tables$X235688_s_at
    X235688_s_at
Y             [,1]      [,2]
  NR  0.01982982 0.5208595
  R  -0.55915444 0.5353062
tables$X235804_at
    X235804_at
Y            [,1]      [,2]
  NR  0.2009344 0.5886169
  R  -0.4162556 0.4684402
tables$X235831_at
    X235831_at
Y             [,1]      [,2]
  NR -0.08120476 0.4915252
  R   1.14904526 1.1198369
tables$X236203_at
    X236203_at
Y             [,1]      [,2]
  NR 0.005259016 0.3895193
  R  0.676842217 0.4524154
tables$X236280_at
    X236280_at
Y            [,1]      [,2]
  NR -0.1220235 0.4998530
  R   0.5788924 0.4002499
tables$X236295_s_at
    X236295_s_at
Y            [,1]      [,2]
  NR -0.1165942 0.5645451
  R   0.6163840 0.5662135
tables$X236583_at
    X236583_at

Y            [,1]      [,2]
  NR -0.2269420 0.466748
  R   0.5087007 0.360080
tables$X210972_x_at
    X210972_x_at
Y            [,1]       [,2]
  NR -0.2866590 0.4272028
  R   0.5598691 0.4527992
tables$X210982_s_at
    X210982_s_at
Y            [,1]       [,2]
  NR -0.3512586 0.5571462
  R   0.3419340 0.3732178
tables$X211066_x_at
    X211066_x_at
Y            [,1]       [,2]
  NR  0.2422814 0.6284310
  R  -0.5086901 0.7771297
tables$X211144_x_at
    X211144_x_at
Y            [,1]       [,2]
  NR 0.05087736 0.3119915
  R  0.70598903 0.4822038
tables$X211200_s_at
    X211200_s_at
Y            [,1]       [,2]
  NR  0.2580184 0.5530421
  R  -0.4922700 0.5506593
tables$X211339_s_at
    X211339_s_at
Y            [,1]       [,2]
  NR -0.2296132 0.5247178
  R   0.5767409 0.5988969
tables$X211366_x_at
    X211366_x_at
Y            [,1]       [,2]
  NR -0.3681938 0.6327750
  R   0.1432735 0.2631491
tables$X211367_s_at
    X211367_s_at
Y            [,1]       [,2]
  NR -0.1751026 0.5723239
  R   0.3320629 0.2562800
tables$X211368_s_at
    X211368_s_at
Y             [,1]       [,2]
  NR -0.51886132 0.7515452
  R   0.07358956 0.2643141
tables$X211654_x_at
    X211654_x_at
Y            [,1]       [,2]
  NR -0.3850022 0.5573621
Y            [,1]       [,2]
  NR 0.02541689 0.5675783
  R  0.82576116 0.7584443
tables$X236908_at
    X236908_at
Y            [,1]       [,2]
  NR  0.5408046 0.8859905
  R  -0.3849473 0.3825884
tables$X238439_at
    X238439_at
Y             [,1]       [,2]
  NR -0.08639162 0.4532391
  R   0.64076976 0.7133665
tables$X238488_at
    X238488_at
Y            [,1]       [,2]
  NR -0.2588720 0.5056425
  R   0.4974834 0.7836170
tables$X238544_at
    X238544_at
Y            [,1]       [,2]
  NR  0.2824277 0.7145403
  R  -0.4079449 0.4677199
tables$X239196_at
    X239196_at
Y            [,1]      [,2]
  NR 0.04478165 0.456084
  R  0.92699218 0.784260
tables$X239237_at
    X239237_at
Y             [,1]       [,2]
  NR -0.06887682 0.4538298
  R   0.56173192 0.5087772
tables$X239744_at
    X239744_at
Y            [,1]       [,2]
  NR -0.1939947 0.4873477
  R   0.3861305 0.5051036
tables$X241671_x_at
    X241671_x_at
Y            [,1]       [,2]
  NR  0.3853780 0.6714183
  R  -0.2467927 0.4002054
tables$X241701_at
    X241701_at
Y            [,1]       [,2]
  NR  0.3603183 0.8440397
  R  -0.4068724 0.5295527
tables$X242458_at
    X242458_at
Y            [,1]       [,2]
  NR -0.2312046 0.7607719

R   0.3719847 0.3143733
tables$X211742_s_at
    X211742_s_at
Y            [,1]      [,2]
  NR -0.2540869 0.6521022
  R   0.4880575 0.5506316
tables$X211796_s_at
    X211796_s_at
Y            [,1]      [,2]
  NR -0.3093201 0.4536705
  R   0.4624019 0.3008702
tables$X211902_x_at
    X211902_x_at
Y            [,1]      [,2]
  NR -0.2792212 0.5535743
  R   0.6836665 0.6034058
tables$X211990_at
    X211990_at
Y            [,1]      [,2]
  NR -0.4590253 0.8582217
  R   0.4251869 0.4814630
tables$X211991_s_at
    X211991_s_at
Y            [,1]      [,2]
  NR -0.2092869 0.6666829
  R   0.5537245 0.3967729
tables$X212067_s_at
    X212067_s_at
Y            [,1]      [,2]
  NR -0.4803278 0.7242299
  R   0.3558230 0.6889846
tables$X212233_at
    X212233_at
Y            [,1]      [,2]
  NR  0.1442229 0.5131552
  R  -0.4879059 0.7214358
tables$X212538_at
    X212538_at
Y            [,1]      [,2]
  NR -0.3849016 0.7522011
  R   0.6516464 0.6995619
tables$X212587_s_at
    X212587_s_at
Y            [,1]      [,2]
  NR -0.3531934 0.5890246
  R   0.3718118 0.3431055
tables$X212588_at
    X212588_at
Y            [,1]      [,2]
  NR -0.5662804 0.8162279
  R   0.4008046 0.4444447
tables$X212592_at
  R   0.5057350 0.7076187
tables$X242546_at
    X242546_at
Y            [,1]      [,2]
  NR  0.1097238 0.4715672
  R  -0.4309505 0.5259991
tables$X242874_at
    X242874_at
Y            [,1]      [,2]
  NR -0.2219024 0.3807166
  R   0.5644292 0.7338815
tables$X242881_x_at
    X242881_x_at
Y             [,1]      [,2]
  NR  0.08543868 0.3930728
  R  -0.63018680 0.5655045
tables$X242986_at
    X242986_at
Y            [,1]      [,2]
  NR  0.2371634 0.5409865
  R  -0.6643602 0.7457416
tables$X243099_at
    X243099_at
Y            [,1]      [,2]
  NR 0.01903757 0.6357632
  R  0.80481642 0.8461258
tables$X244023_at
    X244023_at
Y            [,1]      [,2]
  NR -0.3068381 0.6806949
  R   0.4666732 0.5605710
tables$X244061_at
    X244061_at
Y            [,1]      [,2]
  NR -0.2379335 0.5919363
  R   0.5615362 0.6126814
tables$X32128_at
    X32128_at
Y             [,1]      [,2]
  NR -0.04592188 0.6445335
  R   0.60003775 0.6856519
tables$X34210_at
    X34210_at
Y            [,1]      [,2]
  NR -0.3404304 0.6066166
  R   0.4975685 0.2978426
tables$X38149_at
    X38149_at
Y            [,1]      [,2]
  NR -0.4040241 0.6171240
  R   0.2427490 0.4577025
tables$X64064_at

X212592_at
Y [,1] [,2]
NR -0.3409693 0.4849486
R 0.2387676 0.2127384
tables$X212657_s_at
X212657_s_at
Y [,1] [,2]
NR -0.3419394 0.6083771
R 0.2570024 0.4217927

tables$X212671_s_at
X212671_s_at
Y [,1] [,2]
NR -0.5140866 0.8193929
R 0.5074176 0.3776582
tables$X212713_at
X212713_at
Y [,1] [,2]
NR 0.03767197 0.3268940
R 0.74731430 0.8347694
tables$X212763_at
X212763_at
Y [,1] [,2]
NR 0.2888164 0.8845972
R -0.6529463 0.9707572
tables$X212886_at
X212886_at
Y [,1] [,2]
NR -0.3765371 0.7892555
R 0.6201350 0.6611937
tables$X212977_at
X212977_at
Y [,1] [,2]
NR -0.4688782 0.8767720
R 0.5293928 0.6187406
tables$X212998_x_at
X212998_x_at
Y [,1] [,2]
NR -0.2035514 0.6457962
R 0.5296660 0.3118830
tables$X212999_x_at
X212999_x_at
Y [,1] [,2]
NR -0.2290011 0.4910183
R 0.4331640 0.3718106
tables$X213007_at
X213007_at
Y [,1] [,2]
NR 0.2632353 0.6401111
R -0.3996142 0.4037179
tables$X213008_at
X213008_at

X64064_at
Y [,1] [,2]
NR -0.4613187 0.6447816
R 0.4548881 0.3392856

levels
[1] "NR" "R"
call
naiveBayes.default(x = X, y = Y,
laplace = laplace)

Y           [,1]      [,2]
  NR  0.4761995 0.7015473
  R  -0.4947303 0.8235263
tables$X213068_at
    X213068_at
Y           [,1]      [,2]
  NR -0.1758656 0.4044001
  R   0.4193472 0.3911686
tables$X213095_x_at
    X213095_x_at
Y           [,1]      [,2]
  NR -0.3502315 0.8054069
  R   0.3616843 0.3443190
tables$X213193_x_at
    X213193_x_at
Y           [,1]      [,2]
  NR -0.2370856 0.4636341
  R   0.5058580 0.3505763
tables$X213475_s_at
    X213475_s_at
Y           [,1]      [,2]
  NR -0.2137215 0.4617051
  R   0.4040370 0.4908631
tables$X213537_at
    X213537_at
CONTINUED COLUMN 2

**Nucleotide sequence encoding fusion protein of Lipoprotein D fragment, Mage3 fragment, and histidine tail, and its respective amino acid sequence (SEQ ID NO: 34 and 35).**

atg gat cca aaa act tta gcc ctt tct tta tta gca gct ggc gta cta        48
Met Asp Pro Lys Thr Leu Ala Leu Ser Leu Leu Ala Ala Gly Val Leu
1               5                   10                  15

gca ggt tgt agc agc cat tca tca aat atg gcg aat acc caa atg aaa        96
Ala Gly Cys Ser Ser His Ser Ser Asn Met Ala Asn Thr Gln Met Lys
            20                  25                  30

tca gac aaa atc att att gct cac cgt ggt gct agc ggt tat tta cca       144
Ser Asp Lys Ile Ile Ile Ala His Arg Gly Ala Ser Gly Tyr Leu Pro
        35                  40                  45

gag cat acg tta gaa tct aaa gca ctt gcg ttt gca caa cag gct gat       192
Glu His Thr Leu Glu Ser Lys Ala Leu Ala Phe Ala Gln Gln Ala Asp
    50                  55                  60

tat tta gag caa gat tta gca atg act aag gat ggt cgt tta gtg gtt       240
Tyr Leu Glu Gln Asp Leu Ala Met Thr Lys Asp Gly Arg Leu Val Val
65                  70                  75                  80

att cac gat cac ttt tta gat ggc ttg act gat gtt gcg aaa aaa ttc       288
Ile His Asp His Phe Leu Asp Gly Leu Thr Asp Val Ala Lys Lys Phe
                85                  90                  95

cca cat cgt cat cgt aaa gat ggc cgt tac tat gtc atc gac ttt acc       336
Pro His Arg His Arg Lys Asp Gly Arg Tyr Tyr Val Ile Asp Phe Thr
            100                 105                 110

tta aaa gaa att caa agt tta gaa atg aca gaa aac ttt gaa acc atg       384
Leu Lys Glu Ile Gln Ser Leu Glu Met Thr Glu Asn Phe Glu Thr Met
        115                 120                 125

gat ctg gaa cag cgt agt cag cac tgc aag cct gaa gaa ggc ctt gag       432
Asp Leu Glu Gln Arg Ser Gln His Cys Lys Pro Glu Glu Gly Leu Glu
    130                 135                 140

gcc cga gga gag gcc ctg ggc ctg gtg ggt gcg cag gct cct gct act       480

Ala Arg Gly Glu Ala Leu Gly Leu Val Gly Ala Gln Ala Pro Ala Thr
145                 150                 155                 160

gag gag cag gag gct gcc tcc tcc tct tct act cta gtt gaa gtc acc      528
Glu Glu Gln Glu Ala Ala Ser Ser Ser Ser Thr Leu Val Glu Val Thr
                165                 170                 175

ctg ggg gag gtg cct gct gcc gag tca cca gat cct ccc cag agt cct      576
Leu Gly Glu Val Pro Ala Ala Glu Ser Pro Asp Pro Pro Gln Ser Pro
            180                 185                 190

cag gga gcc tcc agc ctc ccc act acc atg aac tac cct ctc tgg agc      624
Gln Gly Ala Ser Ser Leu Pro Thr Thr Met Asn Tyr Pro Leu Trp Ser
        195                 200                 205

caa tcc tat gag gac tcc agc aac caa gaa gag gag ggg cca agc acc      672
Gln Ser Tyr Glu Asp Ser Ser Asn Gln Glu Glu Glu Gly Pro Ser Thr
    210                 215                 220

ttc cct gac ctg gag tcc gag ttc caa gca gca ctc agt agg aag gtg      720
Phe Pro Asp Leu Glu Ser Glu Phe Gln Ala Ala Leu Ser Arg Lys Val
225                 230                 235                 240

gcc gaa ttg gtt cat ttt ctg ctc ctc aag tat cga gcc agg gag ccg      768
Ala Glu Leu Val His Phe Leu Leu Leu Lys Tyr Arg Ala Arg Glu Pro
                245                 250                 255

gtc aca aag gca gaa atg ctg ggg agt gtc gtc gga aat tgg cag tat      816
Val Thr Lys Ala Glu Met Leu Gly Ser Val Val Gly Asn Trp Gln Tyr
            260                 265                 270

ttc ttt cct gtg atc ttc agc aaa gct tcc agt tcc ttg cag ctg gtc      864
Phe Phe Pro Val Ile Phe Ser Lys Ala Ser Ser Ser Leu Gln Leu Val
        275                 280                 285

ttt ggc atc gag ctg atg gaa gtg gac ccc atc ggc cac ttg tac atc      912
Phe Gly Ile Glu Leu Met Glu Val Asp Pro Ile Gly His Leu Tyr Ile
    290                 295                 300

ttt gcc acc tgc ctg ggc ctc tcc tac gat ggc ctg ctg ggt gac aat      960
Phe Ala Thr Cys Leu Gly Leu Ser Tyr Asp Gly Leu Leu Gly Asp Asn
305                 310                 315                 320

cag atc atg ccc aag gca ggc ctc ctg ata atc gtc ctg gcc ata atc     1008
Gln Ile Met Pro Lys Ala Gly Leu Leu Ile Ile Val Leu Ala Ile Ile
                325                 330                 335

gca aga gag ggc gac tgt gcc cct gag gag aaa atc tgg gag gag ctg     1056
Ala Arg Glu Gly Asp Cys Ala Pro Glu Glu Lys Ile Trp Glu Glu Leu
            340                 345                 350

agt gtg tta gag gtg ttt gag ggg agg gaa gac agt atc ttg ggg gat     1104
Ser Val Leu Glu Val Phe Glu Gly Arg Glu Asp Ser Ile Leu Gly Asp
        355                 360                 365

ccc aag aag ctg ctc acc caa cat ttc gtg cag gaa aac tac ctg gag     1152
Pro Lys Lys Leu Leu Thr Gln His Phe Val Gln Glu Asn Tyr Leu Glu
    370                 375                 380

tac cgg cag gtc ccc ggc agt gat cct gca tgt tat gaa ttc ctg tgg     1200
Tyr Arg Gln Val Pro Gly Ser Asp Pro Ala Cys Tyr Glu Phe Leu Trp
385                 390                 395                 400

ggt cca agg gcc ctc gtt gaa acc agc tat gtg aaa gtc ctg cac cat     1248
Gly Pro Arg Ala Leu Val Glu Thr Ser Tyr Val Lys Val Leu His His
                405                 410                 415

atg gta aag atc agt gga gga cct cac att tcc tac cca ccc ctg cat     1296
Met Val Lys Ile Ser Gly Gly Pro His Ile Ser Tyr Pro Pro Leu His
            420                 425                 430

gag tgg gtt ttg aga gag ggg gaa gag ggc ggt cat cac cat cac cat     1344
Glu Trp Val Leu Arg Glu Gly Glu Glu Gly Gly His His His His His
        435                 440                 445

cac cat taa                                                         1353
His His
    450

### Preferred embodiments

The invention also relates to the following numbered preferred embodiments:
1. A gene profile, from a patient derived sample, which is indicative of an increased likelihood that the patient will be a responder or a non-responder to an immunotherapy, wherein the profile comprises differential regulation of at least one immune activation gene.
2. A gene profile according to embodiment 1, wherein the immunotherapy is a cancer immunotherapy.
3. A gene profile according to embodiment 2, wherein the cancer immunotherapy is Mage antigen specific cancer immunotherapy.
4. A gene profile according to any one of embodiments 1 to 3, wherein the at least one immune activation gene is selected from the gene list in Table 1, 2, 3, 4, 7, 9, 11, 12 and/or 13.
5. A gene profile according to any one of embodiments 1 to 4, wherein the at least one immune gene is 5 or more genes.
6. A gene profile according to embodiment 4, wherein the at one gene is at least 10% of the genes listed (such as 40, 50, 60 or 70% of the genes listed) in Table 1, 2, 3, 4, 7, 9, 11, 12 and/or 13.
7. A gene profile according to embodiment 6, wherein the at least one gene is at least in Table 1, 2, 3, 4, 7, 9, 11, 12 and/or 13.
8. A gene profile according to embodiment 7, wherein the at least one gene is 80, 90 or 100% of the genes listed in Table 1, 2, 3, 4, 7, 9, 11, 12 and/or 13.
9. A gene profile according to any one of embodiments 1 to 8, wherein the at least one gene(s) is upregulated.
10. A gene profile according to embodiment 9, wherein the profile is indicative of a responder.
11. Use of a profile as defined in any one of embodiments 1 to 10 for the identification of a responder or a non-responder to immunotherapy.
12. A method of identifying a profile as embodimented in any one of embodiments 1 to 10 comprising the steps:
   a) analyzing a patient derived sample for differential expression of one or more immune response genes, and
   b) characterizing the patient from which the sample was derived as a responder or a non-responder based on the results of step (a),
      wherein the characterization is optionally performed by reference or comparison to a standard.
13. A method according to embodiment 12, wherein the standard in a sample with a known clinical outcome.
14. A method according to embodiment 12, wherein the comparison is performed using an alogorithm.
15. Use of a probe for the identification of differential expression of at least one product of an immune activation gene for establishing if the profile of embodiments 1 to 10 is present in a patient derived sample.
16. Use according to embodiments 15, wherein the differential expression is upregulation.
17. Use of a microarray kit for the identification of differential expression of at least one product of an immune activation gene for establishing if the profile of embodiments 1 to 10 is present in a patient derived sample.
18. Use according to embodiment 17, wherein in the differential expressionis upregulation.
19. A microarray comprising one or more probes suitable for the detection of differential expression of one or more immune activations genes.
20. A microarray according to embodiment 19, wherein the immune activation genes are selected from the group comprising the list of genes in Table 1, 2, 3, 4, 7, 9, 11, 12 and/or 13.
21. A diagnostic kit comprising at least one component for performing an analysis on a patient derived sample to identify a profile according to any one of embodiments 1 to 11, the results of which may be used to designate a patient from which the sample was derived as a responder or non-responder to immunotherapy.
22. A method of treating a patient comprising administering a therapeutically effect amount of an appropriate immunotherapy, after first characterising the patient as responder thereto based differential activation of at least one immune activation gene.
23. A method according to embodiment 22, wherein the therapy is cancer immunotherapy.
24. A method according to embodiment 23, wherein the therapy is Mage cancer immunotherapy.
25. A method according to any one of embodiments 22 to 24, wherein the patient is characterised as a responder based on a profile as defined in any one of embodiments 1 to 10.
26. A method according to any one of embodiments 22 to 25, wherein the immunotherapy comprises and antigen and an appropriate adjuvant.
27. A method according to any one of embodiments 21 to 25 wherein the at least one immune activation gene is upregulated.
28. A method of inducing a responder gene profile in a patient characterised as a non-responder comprising the step of stimulating a systemic immune or inflammatory response in the patient.
29. A method according to embodiment 28, wherein the systemic response is induced by radiotherapy or administering effective amount of interferon
30. A method according to embodiment 28 or 29, wherein after optionally analysising the patient to identify a gene profile as embodimented in one one of embodiments 1 to 10 and characterising the patient as a responder, administering an effective amount of an appropriate immunotherapy, for example an appropriate cancer immunotherapy, such as a Mage immunotherapy.

## Claims

1. A method of identifying a responder or non-responder patient to cancer immunotherapy comprising the steps:
a) analyzing a patient derived sample for differential expression of one or more immune activation genes in comparison to its normal expression, and
b) characterizing the patient from which the sample was derived as a responder or a non-responder based on the results of step (a),
wherein one of the one or more immune activation genes is ICOS .

2. The method of claim 1, wherein the characterization is performed by reference or comparison to a standard.

3. The method according to claim 1 or claim 2, wherein the standard is a sample with a known clinical outcome.

4. The method according to any one of claims 1 to 3, wherein the comparison is performed using an algorithm.

5. A method of inducing a responder gene profile in a patient initially characterised as a non-responder to a cancer immunotherapy according to the method of any one of claims 1 to 4, comprising the steps of stimulating a systemic immune or inflammatory response in the patient by radiotherapy or by administering effective amount of interferon.

6. Use of a probe for the identification of differential expression of ICOS for the characterisation of a responder or a non-responder to cancer immunotherapy.

7. A cancer immunotherapy for use in a method of treating a patient, in which the patient is first characterised as a responder using the method of any one of claims 1 to 4.

8. A cancer immunotherapy for use in a method of treating a patient, wherein the patient has been characterized as a responder based on the differential expression of one more immune activation genes in comparison to its normal expression, wherein one of the one or more immune activation genes is ICOS.

9. A cancer immunotherapy according to claim 7 or 8, wherein the immunotherapy comprises an antigen and an appropriate adjuvant.

10. A method according to claim 1 to 5, a use of claim 6 or a cancer immunotherapy according to any one of claims 7 to 9, wherein the cancer immunotherapy is MAGE antigen specific cancer immunotherapy.
